(19) Europäisches Patentamt
European Patent Office
Office européen des brevets

(11) EP 3 511 718 A1

(12) EUROPEAN PATENT APPLICATION

(43) Date of publication:
17.07.2019 Bulletin 2019/29

(51) Int Cl.:
*G01N 33/74* (2006.01) *G01N 33/68* (2006.01)

(21) Application number: 18204426.3

(22) Date of filing: 29.11.2013

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 30.11.2012 EP 12195182
07.12.2012 EP 12196177

(62) Document number(s) of the earlier application(s) in accordance with Art. 76 EPC:
13814848.1 / 2 926 142

(71) Applicant: F. Hoffmann-La Roche AG
4070 Basel (CH)

(72) Inventors:
- BELOUSOV, Anton
82377 Penzberg (DE)
- BIANCHINI, Giampaolo
24125 Bergamo (IT)
- GIANNI, Luca
20122 Milano (IT)
- THOMAS, Marlene
79595 Rümmingen (DE)

(74) Representative: Vossius & Partner
Patentanwälte Rechtsanwälte mbB
Siebertstrasse 3
81675 München (DE)

Remarks:
•This application was filed on 05-11-2018 as a divisional application to the application mentioned under INID code 62.
•Claims filed after the date o freceipt of the divisional application (Rule 68(4) EPC).

# (54) PD-L1 INHIBITOR

(57) The present invention relates to means and methods for determining whether a patient is in need of a PD-L1 inhibitor cotherapy. A patient is determined to be in need of the PD-L1 inhibitor cotherapy if a low or absent ER expression level and an expression level of programmed death ligand 1 (PD-L1) that is increased in comparison to a control is measured in vitro in a sample from the patient. The patient is undergoing therapy comprising a modulator of the HER2/neu (ErbB2) signaling pathway (like Trastuzumab) and a chemotherapeutic agent (like dodetaxel) or such a therapy is contemplated for the patient. Also provided herein are means and methods for treating a cancer in a cancer patient for whom therapy comprising a modulator of the HER2/neu (ErbB2) signaling pathway (like Trastuzumab) and a chemotherapeutic agent (like dodetaxel) is contemplated, wherein the patient is to receive PD-L1 inhibitor cotherapy.

EP 3 511 718 A1

SEQUENCE LISTING

```
<110> F. Hoffmann-La Roche AG

<120> Identification of patients in need of PD-L1 inhibitor cotherapy

<130> U2912 EP/1 S3

<150> EP12 19 5182.6
<151> 2012-11-30
<150> EP12 19 6177.5
<151> 2012-12-07

<160> 51

<170> BiSSAP 1.2

<210> 1
<211> 10
<212> PRT
<213> Artificial Sequence

<220>
<223> HVR-H1 sequence

<220>
<221> VARIANT
<222> 6
<223> Xaa is D or G

<400> 1
Gly Phe Thr Phe Ser Xaa Ser Trp Ile His
1               5                   10

<210> 2
<211> 18
<212> PRT
<213> Artificial Sequence

<220>
<223> HVR-H2 sequence

<220>
<221> VARIANT
<222> 4
<223> Xaa is S or L

<220>
<221> VARIANT
<222> 10
<223> Xaa is T or S

<400> 2
Ala Trp Ile Xaa Pro Tyr Gly Gly Ser Xaa Tyr Tyr Ala Asp Ser Val
1               5                   10                  15
Lys Gly


<210> 3
<211> 9
<212> PRT
<213> Artificial Sequence
```

<220>
<223> HVR-H3 sequence

<400> 3
Arg His Trp Pro Gly Gly Phe Asp Tyr
1               5

<210> 4
<211> 25
<212> PRT
<213> Artificial Sequence

<220>
<223> HC-FR1 sequence

<400> 4
Glu Val Gln Leu Val Glu Ser Gly Gly Gly Leu Val Gln Pro Gly Gly
1               5                   10                  15
Ser Leu Arg Leu Ser Cys Ala Ala Ser
            20                  25

<210> 5
<211> 13
<212> PRT
<213> Artificial Sequence

<220>
<223> HC-FR2 sequence

<400> 5
Trp Val Arg Gln Ala Pro Gly Lys Gly Leu Glu Trp Val
1               5                   10

<210> 6
<211> 32
<212> PRT
<213> Artificial Sequence

<220>
<223> HC-FR3 sequence

<400> 6
Arg Phe Thr Ile Ser Ala Asp Thr Ser Lys Asn Thr Ala Tyr Leu Gln
1               5                   10                  15
Met Asn Ser Leu Arg Ala Glu Asp Thr Ala Val Tyr Tyr Cys Ala Arg
            20                  25                  30

<210> 7
<211> 11
<212> PRT
<213> Artificial Sequence

<220>
<223> HC-FR4 sequence

<400> 7
Trp Gly Gln Gly Thr Leu Val Thr Val Ser Ala
1               5                   10

<210> 8
<211> 11

```
<212> PRT
<213> Artificial Sequence

<220>
<223> HVR-L1 sequence

<220>
<221> VARIANT
<222> 5
<223> Xaa is D or V

<220>
<221> VARIANT
<222> 6
<223> Xaa is V or I

<220>
<221> VARIANT
<222> 7
<223> Xaa is S or N

<220>
<221> VARIANT
<222> 9
<223> Xaa is A or F

<220>
<221> VARIANT
<222> 10
<223> Xaa is V or L

<400> 8
Arg Ala Ser Gln Xaa Xaa Xaa Thr Xaa Xaa Ala
1               5                   10

<210> 9
<211> 7
<212> PRT
<213> Artificial Sequence

<220>
<223> HVR-L2 sequence

<220>
<221> VARIANT
<222> 4
<223> Xaa is F or T

<220>
<221> VARIANT
<222> 6
<223> Xaa is Y or A

<400> 9
Ser Ala Ser Xaa Leu Xaa Ser
1               5

<210> 10
<211> 9
<212> PRT
<213> Artificial Sequence

<220>
```

<223> HVR-L3 sequence

<220>
<221> VARIANT
<222> 3
<223> Xaa is Y, G, F or S

<220>
<221> VARIANT
<222> 4
<223> Xaa is L, Y, F or W

<220>
<221> VARIANT
<222> 5
<223> Xaa is Y, N, A, T, G, F or I

<220>
<221> VARIANT
<222> 6
<223> Xaa is H, V, P, T or I

<220>
<221> VARIANT
<222> 8
<223> Xaa is A, W, R, P or T

<400> 10
```
Gln Gln Xaa Xaa Xaa Xaa Pro Xaa Thr
1               5
```

<210> 11
<211> 23
<212> PRT
<213> Artificial Sequence

<220>
<223> LC-FR1 sequence

<400> 11
```
Asp Ile Gln Met Thr Gln Ser Pro Ser Ser Leu Ser Ala Ser Val Gly
1               5                   10                  15
Asp Arg Val Thr Ile Thr Cys
            20
```

<210> 12
<211> 15
<212> PRT
<213> Artificial Sequence

<220>
<223> LC-FR2 sequence

<400> 12
```
Trp Tyr Gln Gln Lys Pro Gly Lys Ala Pro Lys Leu Leu Ile Tyr
1               5                   10                  15
```

<210> 13
<211> 32
<212> PRT
<213> Artificial Sequence

<220>

<223> LC-FR3 sequence

<400> 13
Gly Val Pro Ser Arg Phe Ser Gly Ser Gly Ser Gly Thr Asp Phe Thr
1               5                   10                  15
Leu Thr Ile Ser Ser Leu Gln Pro Glu Asp Phe Ala Thr Tyr Tyr Cys
            20                  25                  30

<210> 14
<211> 11
<212> PRT
<213> Artificial Sequence

<220>
<223> LC-FR4 sequence

<400> 14
Phe Gly Gln Gly Thr Lys Val Glu Ile Lys Arg
1               5                   10

<210> 15
<211> 10
<212> PRT
<213> Artificial Sequence

<220>
<223> heavy chain

<400> 15
Gly Phe Thr Phe Ser Asp Ser Trp Ile His
1               5                   10

<210> 16
<211> 18
<212> PRT
<213> Artificial Sequence

<220>
<223> heavy chain

<400> 16
Ala Trp Ile Ser Pro Tyr Gly Gly Ser Thr Tyr Tyr Ala Asp Ser Val
1               5                   10                  15
Lys Gly

<210> 17
<211> 11
<212> PRT
<213> Artificial Sequence

<220>
<223> light chain

<400> 17
Arg Ala Ser Gln Asp Val Ser Thr Ala Val Ala
1               5                   10

<210> 18
<211> 7
<212> PRT

<213> Artificial Sequence

<220>
<223> light chain

<400> 18
Ser Ala Ser Phe Leu Tyr Ser
1               5

<210> 19
<211> 9
<212> PRT
<213> Artificial Sequence

<220>
<223> light chain

<400> 19
Gln Gln Tyr Leu Tyr His Pro Ala Thr
1               5

<210> 20
<211> 118
<212> PRT
<213> Artificial Sequence

<220>
<223> heavy chain

<400> 20
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Ser
            20                  25                  30
Trp Ile His Trp Val Arg Gln Ala Pro Gly Lys Gly Leu Glu Trp Val
        35                  40                  45
Ala Trp Ile Ser Pro Tyr Gly Gly Ser Thr Tyr Tyr Ala Asp Ser Val
    50                  55                  60
Lys Gly Arg Phe Thr Ile Ser Ala Asp Thr Ser Lys Asn Thr Ala Tyr
65                  70                  75                  80
Leu Gln Met Asn Ser Leu Arg Ala Glu Asp Thr Ala Val Tyr Tyr Cys
                85                  90                  95
Ala Arg Arg His Trp Pro Gly Gly Phe Asp Tyr Trp Gly Gln Gly Thr
            100                 105                 110
Leu Val Thr Val Ser Ala
        115

<210> 21
<211> 108
<212> PRT
<213> Artificial Sequence

<220>
<223> light chain

<400> 21
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Val Ser Thr Ala
            20                  25                  30
Val Ala Trp Tyr Gln Gln Lys Pro Gly Lys Ala Pro Lys Leu Leu Ile
        35                  40                  45
Tyr Ser Ala Ser Phe Leu Tyr Ser Gly Val Pro Ser Arg Phe Ser Gly

```
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Leu Tyr His Pro Ala
                85                  90                  95
Thr Phe Gly Gln Gly Thr Lys Val Glu Ile Lys Arg
            100                 105
```

<210> 22
<211> 195
<212> PRT
<213> Homo sapiens

<400> 22

```
Thr Gln Val Cys Thr Gly Thr Asp Met Lys Leu Arg Leu Pro Ala Ser
1               5                   10                  15
Pro Glu Thr His Leu Asp Met Leu Arg His Leu Tyr Gln Gly Cys Gln
            20                  25                  30
Val Val Gln Gly Asn Leu Glu Leu Thr Tyr Leu Pro Thr Asn Ala Ser
        35                  40                  45
Leu Ser Phe Leu Gln Asp Ile Gln Glu Val Gln Gly Tyr Val Leu Ile
    50                  55                  60
Ala His Asn Gln Val Arg Gln Val Pro Leu Gln Arg Leu Arg Ile Val
65                  70                  75                  80
Arg Gly Thr Gln Leu Phe Glu Asp Asn Tyr Ala Leu Ala Val Leu Asp
                85                  90                  95
Asn Gly Asp Pro Leu Asn Asn Thr Thr Pro Val Thr Gly Ala Ser Pro
            100                 105                 110
Gly Gly Leu Arg Glu Leu Gln Leu Arg Ser Leu Thr Glu Ile Leu Lys
        115                 120                 125
Gly Gly Val Leu Ile Gln Arg Asn Pro Gln Leu Cys Tyr Gln Asp Thr
    130                 135                 140
Ile Leu Trp Lys Asp Ile Phe His Lys Asn Asn Gln Leu Ala Leu Thr
145                 150                 155                 160
Leu Ile Asp Thr Asn Arg Ser Arg Ala Cys His Pro Cys Ser Pro Met
                165                 170                 175
Cys Lys Gly Ser Arg Cys Trp Gly Glu Ser Ser Glu Asp Cys Gln Ser
            180                 185                 190
Leu Thr Arg
        195
```

<210> 23
<211> 124
<212> PRT
<213> Homo sapiens

<400> 23

```
Thr Val Cys Ala Gly Gly Cys Ala Arg Cys Lys Gly Pro Leu Pro Thr
1               5                   10                  15
Asp Cys Cys His Glu Gln Cys Ala Ala Gly Cys Thr Gly Pro Lys His
            20                  25                  30
Ser Asp Cys Leu Ala Cys Leu His Phe Asn His Ser Gly Ile Cys Glu
        35                  40                  45
Leu His Cys Pro Ala Leu Val Thr Tyr Asn Thr Asp Thr Phe Glu Ser
    50                  55                  60
Met Pro Asn Pro Glu Gly Arg Tyr Thr Phe Gly Ala Ser Cys Val Thr
65                  70                  75                  80
Ala Cys Pro Tyr Asn Tyr Leu Ser Thr Asp Val Gly Ser Cys Thr Leu
                85                  90                  95
Val Cys Pro Leu His Asn Gln Glu Val Thr Ala Glu Asp Gly Thr Gln
            100                 105                 110
```

```
Arg Cys Glu Lys Cys Ser Lys Pro Cys Ala Arg Val
        115                 120

<210> 24
<211> 169
<212> PRT
<213> Homo sapiens


<400> 24
Cys Tyr Gly Leu Gly Met Glu His Leu Arg Glu Val Arg Ala Val Thr
1               5                   10                  15
Ser Ala Asn Ile Gln Glu Phe Ala Gly Cys Lys Lys Ile Phe Gly Ser
            20                  25                  30
Leu Ala Phe Leu Pro Glu Ser Phe Asp Gly Asp Pro Ala Ser Asn Thr
        35                  40                  45
Ala Pro Leu Gln Pro Glu Gln Leu Gln Val Phe Glu Thr Leu Glu Glu
    50                  55                  60
Ile Thr Gly Tyr Leu Tyr Ile Ser Ala Trp Pro Asp Ser Leu Pro Asp
65                  70                  75                  80
Leu Ser Val Phe Gln Asn Leu Gln Val Ile Arg Gly Arg Ile Leu His
                85                  90                  95
Asn Gly Ala Tyr Ser Leu Thr Leu Gln Gly Leu Gly Ile Ser Trp Leu
            100                 105                 110
Gly Leu Arg Ser Leu Arg Glu Leu Gly Ser Gly Leu Ala Leu Ile His
        115                 120                 125
His Asn Thr His Leu Cys Phe Val His Thr Val Pro Trp Asp Gln Leu
    130                 135                 140
Phe Arg Asn Pro His Gln Ala Leu Leu His Thr Ala Asn Arg Pro Glu
145                 150                 155                 160
Asp Glu Cys Val Gly Glu Gly Leu Ala
                165

<210> 25
<211> 142
<212> PRT
<213> Homo sapiens


<400> 25
Cys His Gln Leu Cys Ala Arg Gly His Cys Trp Gly Pro Gly Pro Thr
1               5                   10                  15
Gln Cys Val Asn Cys Ser Gln Phe Leu Arg Gly Gln Glu Cys Val Glu
            20                  25                  30
Glu Cys Arg Val Leu Gln Gly Leu Pro Arg Glu Tyr Val Asn Ala Arg
        35                  40                  45
His Cys Leu Pro Cys His Pro Glu Cys Gln Pro Gln Asn Gly Ser Val
    50                  55                  60
Thr Cys Phe Gly Pro Glu Ala Asp Gln Cys Val Ala Cys Ala His Tyr
65                  70                  75                  80
Lys Asp Pro Pro Phe Cys Val Ala Arg Cys Pro Ser Gly Val Lys Pro
                85                  90                  95
Asp Leu Ser Tyr Met Pro Ile Trp Lys Phe Pro Asp Glu Glu Gly Ala
            100                 105                 110
Cys Gln Pro Cys Pro Ile Asn Cys Thr His Ser Cys Val Asp Leu Asp
        115                 120                 125
Asp Lys Gly Cys Pro Ala Glu Gln Arg Ala Ser Pro Leu Thr
    130                 135                 140

<210> 26
<211> 107
<212> PRT
<213> Mus musculus
```

<400> 26
Asp Thr Val Met Thr Gln Ser His Lys Ile Met Ser Thr Ser Val Gly
1               5                   10                  15
Asp Arg Val Ser Ile Thr Cys Lys Ala Ser Gln Asp Val Ser Ile Gly
            20                  25                  30
Val Ala Trp Tyr Gln Gln Arg Pro Gly Gln Ser Pro Lys Leu Leu Ile
        35                  40                  45
Tyr Ser Ala Ser Tyr Arg Tyr Thr Gly Val Pro Asp Arg Phe Thr Gly
    50                  55                  60
Ser Gly Ser Gly Thr Asp Phe Thr Phe Thr Ile Ser Ser Val Gln Ala
65                  70                  75                  80
Glu Asp Leu Ala Val Tyr Tyr Cys Gln Gln Tyr Tyr Ile Tyr Pro Tyr
                85                  90                  95
Thr Phe Gly Gly Gly Thr Lys Leu Glu Ile Lys
            100                 105

<210> 27
<211> 119
<212> PRT
<213> Mus musculus

<400> 27
Glu Val Gln Leu Gln Gln Ser Gly Pro Glu Leu Val Lys Pro Gly Thr
1               5                   10                  15
Ser Val Lys Ile Ser Cys Lys Ala Ser Gly Phe Thr Phe Thr Asp Tyr
            20                  25                  30
Thr Met Asp Trp Val Lys Gln Ser His Gly Lys Ser Leu Glu Trp Ile
        35                  40                  45
Gly Asp Val Asn Pro Asn Ser Gly Gly Ser Ile Tyr Asn Gln Arg Phe
    50                  55                  60
Lys Gly Lys Ala Ser Leu Thr Val Asp Arg Ser Ser Arg Ile Val Tyr
65                  70                  75                  80
Met Glu Leu Arg Ser Leu Thr Phe Glu Asp Thr Ala Val Tyr Tyr Cys
                85                  90                  95
Ala Arg Asn Leu Gly Pro Ser Phe Tyr Phe Asp Tyr Trp Gly Gln Gly
            100                 105                 110
Thr Thr Leu Thr Val Ser Ser
        115

<210> 28
<211> 107
<212> PRT
<213> Artificial Sequence

<220>
<223> Humanized 574 Variable Light Chain

<400> 28
Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Asp Val Ser Ile Gly
            20                  25                  30
Val Ala Trp Tyr Gln Gln Lys Pro Gly Lys Ala Pro Lys Leu Leu Ile
        35                  40                  45
Tyr Ser Ala Ser Tyr Arg Tyr Thr Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Tyr Ile Tyr Pro Tyr
                85                  90                  95

```
Thr Phe Gly Gln Gly Thr Lys Val Glu Ile Lys
            100                 105

<210> 29
<211> 119
<212> PRT
<213> Artificial Sequence

<220>
<223> Humanized 574 Variable Heavy Chain

<400> 29
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Thr Asp Tyr
            20                  25                  30
Thr Met Asp Trp Val Arg Gln Ala Pro Gly Lys Gly Leu Glu Trp Val
        35                  40                  45
Ala Asp Val Asn Pro Asn Ser Gly Gly Ser Ile Tyr Asn Gln Arg Phe
    50                  55                  60
Lys Gly Arg Phe Thr Leu Ser Val Asp Arg Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asn Leu Gly Pro Ser Phe Tyr Phe Asp Tyr Trp Gly Gln Gly
            100                 105                 110
Thr Leu Val Thr Val Ser Ser
        115

<210> 30
<211> 107
<212> PRT
<213> Artificial Sequence

<220>
<223> Consensus framework Hum kappa1 Variable Light Chain

<400> 30
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Ser Ile Ser Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Ser Leu Glu Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Asn Ser Leu Pro Trp
                85                  90                  95
Thr Phe Gly Gln Gly Thr Lys Val Glu Ile Lys
            100                 105

<210> 31
<211> 119
<212> PRT
<213> Artificial Sequence

<220>
<223> Consensus framework Hum kappa1 Variable Heavy Chain

<400> 31
Glu Val Gln Leu Val Glu Ser Gly Gly Gly Leu Val Gln Pro Gly Gly
```

```
1               5                   10                  15
Ser Leu Arg Leu Ser Cys Ala Ala Ser Gly Phe Thr Phe Ser Ser Tyr
            20                  25                  30
Ala Met Ser Trp Val Arg Gln Ala Pro Gly Lys Gly Leu Glu Trp Val
        35                  40                  45
Ala Val Ile Ser Gly Asp Gly Gly Ser Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Gly Arg Val Gly Tyr Ser Leu Tyr Asp Tyr Trp Gly Gln Gly
            100                 105                 110
Thr Leu Val Thr Val Ser Ser
        115
```

<210> 32
<211> 214
<212> PRT
<213> Artificial Sequence

<220>
<223> Pertuzumab light chain

<400> 32

```
Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Asp Val Ser Ile Gly
            20                  25                  30
Val Ala Trp Tyr Gln Gln Lys Pro Gly Lys Ala Pro Lys Leu Leu Ile
        35                  40                  45
Tyr Ser Ala Ser Tyr Arg Tyr Thr Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Tyr Ile Tyr Pro Tyr
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
```

<210> 33
<211> 448
<212> PRT
<213> Artificial Sequence

<220>
<223> Pertuzumab heavy chain

<400> 33

Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Thr Asp Tyr
            20                  25                  30
Thr Met Asp Trp Val Arg Gln Ala Pro Gly Lys Gly Leu Glu Trp Val
        35                  40                  45
Ala Asp Val Asn Pro Asn Ser Gly Gly Ser Ile Tyr Asn Gln Arg Phe
    50                  55                  60
Lys Gly Arg Phe Thr Leu Ser Val Asp Arg Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asn Leu Gly Pro Ser Phe Tyr Phe Asp Tyr Trp Gly Gln Gly
            100                 105                 110
Thr Leu Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Lys
    210                 215                 220
Thr His Thr Cys Pro Pro Cys Pro Ala Pro Glu Leu Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Glu Glu Met Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445

<210> 34
<211> 214
<212> PRT
<213> Artificial Sequence

<220>
<223> Trastuzumab light chain

<400> 34
```
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Val Asn Thr Ala
            20                  25                  30
Val Ala Trp Tyr Gln Gln Lys Pro Gly Lys Ala Pro Lys Leu Leu Ile
        35                  40                  45
Tyr Ser Ala Ser Phe Leu Tyr Ser Gly Val Pro Ser Arg Phe Ser Gly
    50                  55                  60
Ser Arg Ser Gly Thr Asp Phe Thr Leu Thr Ile Ser Ser Leu Gln Pro
65                  70                  75                  80
Glu Asp Phe Ala Thr Tyr Tyr Cys Gln Gln His Tyr Thr Thr Pro Pro
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
```

<210> 35
<211> 449
<212> PRT
<213> Artificial Sequence

<220>
<223> Trastuzumab heavy chain

<400> 35
```
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Asn Ile Lys Asp Thr
            20                  25                  30
Tyr Ile His Trp Val Arg Gln Ala Pro Gly Lys Gly Leu Glu Trp Val
        35                  40                  45
Ala Arg Ile Tyr Pro Thr Asn Gly Tyr Thr Arg Tyr Ala Asp Ser Val
    50                  55                  60
Lys Gly Arg Phe Thr Ile Ser Ala Asp Thr Ser Lys Asn Thr Ala Tyr
65                  70                  75                  80
Leu Gln Met Asn Ser Leu Arg Ala Glu Asp Thr Ala Val Tyr Tyr Cys
                85                  90                  95
Ser Arg Trp Gly Gly Asp Gly Phe Tyr Ala Met Asp Tyr Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
```

Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Glu Glu Met Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly

<210> 36
<211> 217
<212> PRT
<213> Artificial Sequence

<220>
<223> variant Pertuzumab light chain sequence

<400> 36
Val His Ser Asp Ile Gln Met Thr Gln Ser Pro Ser Ser Leu Ser Ala
1 5 10 15
Ser Val Gly Asp Arg Val Thr Ile Thr Cys Lys Ala Ser Gln Asp Val
20 25 30
Ser Ile Gly Val Ala Trp Tyr Gln Gln Lys Pro Gly Lys Ala Pro Lys
35 40 45
Leu Leu Ile Tyr Ser Ala Ser Tyr Arg Tyr Thr Gly Val Pro Ser Arg
50 55 60
Phe Ser Gly Ser Gly Ser Gly Thr Asp Phe Thr Leu Thr Ile Ser Ser
65 70 75 80
Leu Gln Pro Glu Asp Phe Ala Thr Tyr Tyr Cys Gln Gln Tyr Tyr Ile
85 90 95
Tyr Pro Tyr Thr Phe Gly Gln Gly Thr Lys Val Glu Ile Lys Arg Thr
100 105 110
Val Ala Ala Pro Ser Val Phe Ile Phe Pro Pro Ser Asp Glu Gln Leu

```
        115                 120                 125
Lys Ser Gly Thr Ala Ser Val Val Cys Leu Leu Asn Asn Phe Tyr Pro
    130                 135                 140
Arg Glu Ala Lys Val Gln Trp Lys Val Asp Asn Ala Leu Gln Ser Gly
145                 150                 155                 160
Asn Ser Gln Glu Ser Val Thr Glu Gln Asp Ser Lys Asp Ser Thr Tyr
                165                 170                 175
Ser Leu Ser Ser Thr Leu Thr Leu Ser Lys Ala Asp Tyr Glu Lys His
            180                 185                 190
Lys Val Tyr Ala Cys Glu Val Thr His Gln Gly Leu Ser Ser Pro Val
        195                 200                 205
Thr Lys Ser Phe Asn Arg Gly Glu Cys
    210                 215

<210> 37
<211> 449
<212> PRT
<213> Artificial Sequence

<220>
<223> variant Pertuzumab heavy chain sequence

<400> 37
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Thr Asp Tyr
            20                  25                  30
Thr Met Asp Trp Val Arg Gln Ala Pro Gly Lys Gly Leu Glu Trp Val
        35                  40                  45
Ala Asp Val Asn Pro Asn Ser Gly Gly Ser Ile Tyr Asn Gln Arg Phe
    50                  55                  60
Lys Gly Arg Phe Thr Leu Ser Val Asp Arg Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asn Leu Gly Pro Ser Phe Tyr Phe Asp Tyr Trp Gly Gln Gly
            100                 105                 110
Thr Leu Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Lys
    210                 215                 220
Thr His Thr Cys Pro Pro Cys Pro Ala Pro Glu Leu Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
```

```
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Glu Glu Met Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys
```

<210> 38
<211> 100190
<212> DNA
<213> Homo sapiens

<220>
<221> source
<222> 1..100190
<223> /mol_type="unassigned DNA"
/organism="Homo sapiens"

<400> 38

```
agtccacagc tgtcactaat cggggtaagc cttgttgtat ttgtgcgtgt gggtggcatt      60

ctcaatgaga actagcttca cttgtcattt gagtgaaatc tacaacccga ggcggctagt     120

gctcccgcac tactgggatc tgagatcttc ggagatgact gtcgcccgca gtacggagcc     180

agcagaagtc cgacccttcc tgggaatggg ctgtaccgag aggtccgact agccccaggg     240

ttttagtgag ggggcagtgg aactcagcga gggactgaga gcttcacagc atgcacgagt     300

ttgatgccag agaaaaagtc gggagataaa ggagccgcgt gtcactaaat tgccgtcgca     360

gccgcagcca ctcaagtgcc ggacttgtga gtactctgcg tctccagtcc tcggacagaa     420

gttggagaac tctcttggag aactccccga gttaggagac gagatctcct aacaattact     480

actttttctt gcgctcccca cttgccgctc gctgggacaa acgacagcca cagttcccct     540

gacgacagga tggaggccaa gggcaggagc tgaccagcgc cgccctcccc cgcccccgac     600

ccaggaggtg gagatccctc cggtccagcc acattcaaca cccactttct cctccctctg     660

cccctatatt cccgaaaccc cctcctcctt ccctttcccc tcctcctgga gacgggggag     720

gagaaaaggg gagtccagtc gtcatgactg agctgaaggc aaagggtccc cgggctcccc     780

acgtggcggg cggcccgccc tcccccgagg tcggatcccc actgctgtgt cgcccagccg     840

caggtccgtt cccggggagc cagacctcgg acaccttgcc tgaagtttcg gccataccta     900

tctccctgga cggctactc ttccctcggc cctgccaggg acaggacccc tccgacgaaa      960
```

agacgcagga ccagcagtcg ctgtcggacg tggagggcgc atattccaga gctgaagcta 1020

caaggggtgc tggaggcagc agttctagtc cccagaaaa ggacagcgga ctgctggaca 1080

gtgtcttgga cactctgttg gcgccctcag gtcccgggca gagccaaccc agccctcccg 1140

cctgcgaggt caccagctct tggtgcctgt ttggccccga acttcccgaa gatccaccgg 1200

ctgcccccgc cacccagcgg gtgttgtccc cgctcatgag ccggtccggg tgcaaggttg 1260

gagacagctc cgggacggca gctgcccata aagtgctgcc ccggggcctg tcaccagccc 1320

ggcagctgct gctcccggcc tctgagagcc ctcactggtc cggggcccca gtgaagccgt 1380

ctccgcaggc cgctgcggtg gaggttgagg aggaggatgg ctctgagtcc gaggagtctg 1440

cgggtccgct tctgaagggc aaacctcggg ctctgggtgg cgcggcggct ggaggaggag 1500

ccgcggctgt cccgccgggg gcggcagcag gaggcgtcgc cctggtcccc aaggaagatt 1560

cccgcttctc agcgcccagg gtcgccctgg tggagcagga cgcgccgatg gcgcccgggc 1620

gctccccgct ggccaccacg gtgatggatt tcatccacgt gcctatcctg cctctcaatc 1680

acgccttatt ggcagcccgc actcggcagc tgctggaaga cgaaagttac gacggcgggg 1740

ccggggctgc cagcgccttt gccccgccgc ggagttcacc ctgtgcctcg tccaccccgg 1800

tcgctgtagg cgacttcccc gactgcgcgt acccgcccga cgccgagccc aaggacgacg 1860

cgtaccctct ctatagcgac ttccagccgc ccgctctaaa gataaaggag gaggaggaag 1920

gcgcggaggc ctccgcgcgc tccccgcgtt cctaccttgt ggccggtgcc aaccccgcag 1980

ccttcccgga tttcccgttg gggccaccgc ccccgctgcc gccgcgagcg accccatcca 2040

gacccgggga agcggcggtg acggccgcac ccgccagtgc ctcagtctcg tctgcgtcct 2100

cctcggggtc gaccctggag tgcatcctgt acaaagcgga gggcgcgccg ccccagcagg 2160

gcccgttcgc gccgccgccc tgcaaggcgc cgggcgcgag cggctgcctg ctcccgcggg 2220

acggcctgcc ctccacctcc gcctctgccg ccgccgccgg ggcggccccc gcgctctacc 2280

ctgcactcgg cctcaacggg ctcccgcagc tcggctacca ggccgccgtg ctcaaggagg 2340

gcctgccgca ggtctacccg ccctatctca actacctgag gtgagggccc gggacggggc 2400

acgcccagcg cgtccgggag tagcggttcc gttggcggcg gcggccgcca accctcagcc 2460

ccagccccag cgcaccgctg cgctccccgg ggcggccgga gagggtgggc agcgggacac 2520

agcacagggg cagttgcctc ccttcttctt ccctcctctc ctcactcttg gggacacgaa 2580

ggtgggcgca gaatatacta tttttggggc gtgcctccct gaaagctgtt tttttgtttg 2640

ttttttaact ttccgaatct tccagattcc gaagcagaac caaccccgat ttaaaacgtg 2700

cagcgtcaca ctaggtccgc tgtagcccag tggggcagaa agtgcgcggc gagttggggg 2760

ctttatgaaa tgcttctttc ttagaagaag gacgtttacc aggagtgctt gtcttggaga 2820

ggagttaagg caccgttccc ccgggagggg tgggacttga gaggtggccg gccagaaccg 2880

aaagcagcac catcttaggg atttgaacac ttcagtggct cagttttctt aagaatctca 2940

agattaaaat taagttcacg tgggaaatgt ttaaactgtg gatttaaacg cctgtcactg 3000

cattgcaccg ttttcttatt attgcttgct attcactaca attttttta tatacaggtt 3060

taaaaaacac tactttgcat actgaagtaa tggaatgtaa aaaaagaatg ctctgtttgg 3120

aatcttatgt tgtgaatagg caaaacagtg tcagtgtatt ggacaatact ttaaaatgac 3180

aaacatatac ttgcttaagt aagcaatgat tacagggttg tgttttaaaa actcaaaacc 3240

aaaacattgc aaagtaccat cgaactttta aagccaaacc atatttgttt tgacccagca 3300

tacagacagg aaggacataa catttcattt gtcaaagact aaattgtttc tatataaaga 3360

gttttgtaga aagatttcct tttaaccgac tttaactttc taggacataa tattatacac 3420

taattattgt tcttttatat tggtgctact gatgaatggc taatcatttg caagtatggt 3480

gaatccagtt acggatagtc tattaccaag tttagtttgc atgtctttca agtgtatata 3540

tacagttctg tttttaaaat ctcctttcac cctgttaata ctggtttaag aaacctttag 3600

tattagatag tggtgcactt aaaaataaat ggagtacttt gttttgcatt tcaaggccgg 3660

attcagaagc cagccagagc ccacaataca gcttcgagtc attacctcag aagatttgtt 3720

taatctgtgg ggatgaagca tcaggctgtc attatggtgt ccttacctgt gggagctgta 3780

aggtcttctt taagagggca atggaaggta ggctcctttc ccctgatcct ttattattgg 3840

tttaattgta aatggagacc atctaatatt gtatagattt caattattcc ttgtttctta 3900

taagaaatgg tgatatttcc atataattta aaatatatga tgacatttta acaatatgtt 3960

tttatttatg atactcaaaa tggaatgtgg ttgggtacta taattgcata ctctttgact 4020

aacactttca gtattagaca taagtcataa aaatcttgag gacagtgcta ctattgttcc 4080

ttaactgctt agctttgagg aaacagcttt gttctaatag tacttttata tatctattat 4140

gtaggtatat gtgtatgcag tactttaaaa ttttgattaa aagaaaaatg gtagttgaca 4200

catatgtaca tgtatgcgta tacatatttg tacatacacg aacatatatc acaacatgta 4260

tatgatgcag ttttctacat gatactgtct tttgactaca tgaatattta tgtaatattt 4320

acaaagaagt aattctaaac aaatttttga attctctttt tgttcagtat atttttgtaa 4380

gtgtataaga ggacaggatt aaacagttta aaataaaaaa cctggacatc acagtaacat 4440

aatttctaaa gaagtatttt gctttaagta aaactttcat gtttttaaac tcattgaact 4500

tacatgctta atgatttcag atttacttgc atagtgtttc agattttaac tttcaaagaa 4560

aatatttttg aatttctttc tacctaaagt ctaagcagcc aaacatcttt acatttgaag 4620

ataaaaatac attgaaagat ttcatatttt aataccagca ataaatgtat tctataacta 4680

tgtaaaatga agcttaggat tccctctgga gtgctgagat cacacctaga caaggaacca 4740

aggatctgaa tgttggcttt ttgtttcctg ttctgaggat ttttgtttgt ttgtttgttt 4800
caacagccct ctccttacaa gcagaaaagc aggtagtagg aaatttcact ttaagggagc 4860
tttcaaagga gttcttcata acaaatattt gctttgtata tttttagaac atgatttttt 4920
ctcacaaaag atgaatgtat tttactgatg ttgaacatat tcagctttag gggtttgat 4980
tgcattttaa actaattgag gcagtgttaa aagtggtact tgagaaaata gggcaactga 5040
tagtggctct tacccattga cattatttat ttacagttac agttggaagt tctttgtgtg 5100
gaaaagtcag ttttccaatg ggtaattgga gttaccattt ttatctgact ttggttctgg 5160
tttcttaaac attgccttct gcattaatat gatttcctct cttcttaaag tctcctaagg 5220
atagtataat tttataagtg aatgactccc ttagaatcct cttaagccca atttgtccta 5280
ccccagctcc ttcttttgaa agataagcaa atctcaaaga cataaatatg agtttccaaa 5340
ggtcacaaaa ctagttagta gctgattgtt agtaaacata ggttaaatat ttttacattg 5400
cagcgcttgt aaatcagaga tgatatgcaa aagtagatat ataaactgtt tgattcacag 5460
aagttatttc ataaagtgca tatatagaac aaagagcacc ctaactaaaa tacaaatgct 5520
ttctcgtcat tttgttagaa tagcatccaa aactgtagac gaagtctttc caaatgtact 5580
cttagaaagc atttgttgga ctccggctgt tggcatggtc ctatagtctt gagtactaga 5640
agtgaagcac ctttatttag cagtaattac aaagagttac ttaagattga tgcagataaa 5700
tcattcatga aactagaaca agattatgaa ctacattagt aagttccttc attcagcaat 5760
ttatgccaaa gatacacttt ccctgacttc acttttctct gccttgagat aaaatgagga 5820
taacagtggc tatttcttag ggttgctata aagattaaat gagctgatac ttgtaaagta 5880
tgtaaaagaa ggcctgacat attatcagtt tccattgaca tttctacttt caaggaactt 5940
gtaatatagt tagggaggta acatatgcac ataaaacatc taaataaaga ttctcagtaa 6000
atgcccaagt aagcaattct gtaatgtata tgagatctgt gtggtttgtg agtttttgta 6060
tttggacaga gcgaggtggt tatgggttga aatatgtata ttcttgaatg atgaagaagt 6120
ctacatggaa gatatgaaca tttgattagt aaaggacaaa taagctttct aggcattgag 6180
gagagcttta agtgtataca gtcaaagaag agtgaagaaa ttaagattac actgactaag 6240
cattgaatgt tcacattagg aaattgagag agttaaagtt tgagaaacta gattgctagt 6300
gtttgggtga atttggagaa tcggtaattt aaggcaagag aatatagaga atgttctagg 6360
agttttcagg aatgagaaag ataagtagaa ggacttatat caggttcaaa atcttcaata 6420
aagcaatgct gcgtgatgag ctggttcaag gtggcgctgt gtgtggatgc caatatggcc 6480
agaagtttaa agtaaacagg caacaatatg gatactaatg ttgccaagga tgagaatgaa 6540
aatgatggca ttataaaatg ctttctctgt gccagtgact atttcagtgc tttgcaggtc 6600
ttaacttatt tagttgttat aatgttggta ttattattat tactcccact ttacaaatga 6660

ggaaactaag acctgtagat gttaagctgt cttaatacgc ttaggaaatg gtaaagtcag 6720
gattcaaatc caggtggtat gaatccagaa tcccggccct tgaccactgt gcttcctttc 6780
tcataatagg aaatgcagtc aaagaaaaac aatagagggt tagaagaaaa gatgtgagcc 6840
aagtgatgaa acatctagga aggtaaaagt gaatcctaaa ggagaatgca agagcagggg 6900
taatgagaag tgtgtgatat aaaaggatgg atcatattaa cttcacattt agggcagcaa 6960
taagaagaca taagcaggag ccaacagcca gtttcttcac ctccctccca tgttaggaga 7020
gggaggtgat aaagcctata aagtcattct ggatgactgg gtttccctgg atatgagaca 7080
gaagagagaa gagataaaga aataagatac cactcaggaa atgggagaaa ggagttggga 7140
aaaatgattt cttttaagct aattgaactg tttaggatat agtattggcc aaaaaccagt 7200
tggttggata gcccattgca ttcctttacc aagacttgta ggtttggagt gaaccatgaa 7260
aggaccagga attgacttaa tgccttccaa agagaggaag taatcatgac ctgccagtcc 7320
tacaaatgca gactactaac ctggtatgat gaaggaaagg actatttctc aatggcttat 7380
ctttgccagt acacagtaac cagcccagtg ctaggaatat actaggcatc cagtagataa 7440
ctgctacatg atccagtcat cataactgat aacgccacac ttttattttt tggatgcttt 7500
actcagtgac agcatttgtt gtgaatacat ttggtgtaat atcattaaac acatgttata 7560
atacaattga aatgtattac ttagaagaca ctaagctaag taggtattga aggattttca 7620
attgtattgc atattatgct cacttttttt tttttttttt tgagacggag tctcgctgtt 7680
gcccatgctg gagtgcagtg gtgtgatctc ggctcactgc aagctccgcc tcccgggttc 7740
atgccattct cctgccttag cctcccgagt agctgggact acgggtgccc cccaccacgc 7800
ctggctaatt ttttgtattt tttagtagag atggggtttc accatggtct caatctccta 7860
acctcatgat ccgcctgcct cggcctccca aagtgctggg attacaggcg tgagccacca 7920
cacctggcct gtcctcattc ttttattcat atattaattg ttcatgaagt aattacctta 7980
attacattag ttcatgtatt tattgagtac ctgccatgcg ccaggcacta tgttaggtac 8040
tggtgaaacc acagtaaaac gagagacctt gctggctgtt gactaaagtt tatagtgtgg 8100
tggtggagag agacatttta cctatttgtg tacacatgac taattgcaca tgtggtaagt 8160
gattcgttta tgcaatgttt ggacaactag agaattgacc cctctgtcag ataatgggga 8220
aagtttttca gacctagaca tcacaataaa tagactgcag aggaaactag acagaaatga 8280
aatactttta tgaataaagt gtttctttca aaagttgggt atacttggtg tcataggcta 8340
gtaatgaaaa ctggtttggt agcatagttc tccttgatac agcatcagaa agagaaagat 8400
tgaacatcca atttttcatc agcaacttct gtgatttatt tgttctatat ttagagctct 8460
gtaatgcttt ctccatcttt tgtagtgcac agatcatcca gcaatctctt ctactataat 8520

ctatttgaat ttgaataata tttgggctct gagaagatat tttgccaata atcttattta 8580
tattcattta atctccaata gagtctgctc tatagcagct ttcacattac cctaaaaata 8640
aatatgtagt aattcactag attttatttc aaaactctat atccaatttc ttttcaagct 8700
gaggtctctc agtttactat ttagcatgga taatgataga ctggttttaa gcaccacttt 8760
acattagggt tcattgaaat ctctactgta ctgaaaaaga aatggttaaa aagatagcat 8820
ttggtgtcat ctgttcatat ttggttattt agagctccca gatttttaat actctttcag 8880
aacatgtact ttaataccac tacaagagcc agaggagaag cagtggtatt agccatgcgt 8940
gctagtgcta ataactgctc ctgttctggc cactgaatca ttgtacttta attactcaag 9000
taacacaaaa atccatctcc tttaaaaaat gaaaatgtta tcaataaggc ttaagtcctc 9060
ttgatcaata cctctaatct gttcccttgc ctaccatctt tctatacctc tacctcagtt 9120
tttcccatgt gtatgtgtgt gtggaatatg ttctctgggg tatgttaata tataaatggt 9180
attatatatc ataatttatg catcatgtta ttttgataca aaaatgactt gaagctctat 9240
tgttcatata tgtagttcca acttgttttt ttaaccactg cttaatattt cataatatga 9300
aaaatcatta tatttaggaa ttactaatta cactattact tgagtaaacc ctttttgtac 9360
atttctcctt tggtacatgt cttgcttctc tttccagaat atattgaagt attttgatac 9420
agttctaaat gaactagatg attagctttc tacctgctgc taagatgatt tgattcacta 9480
atttattcac ttcagataca tgtattgatc ctacatttaa aagttgcttg tgctgggact 9540
gaagatgaaa atgtatgcag accctgtctt tgacatgcac aaaaccaaga agaagaaaac 9600
aaaacagaac aaacctgtgg tacaactagt gaacatgaga ataccttgat acaagttatg 9660
ccagagccgt ggccaacaca ccgtagtatt cagttagaca catggagaag aatggcagtt 9720
tattctgcta acatatcttg atgttgtgca agaggcttat tcatttaata gtcacttaag 9780
tcgccttcac tataacccag gcactgtttt aactgcttca ctggattagc tcaattaatc 9840
cttgtaagag tcctttgagg tggaggttct gttagtatct atattttaca gatgggtgta 9900
aagagaggtt tctactattc aggaggtctg actctaactt ctgtatctta aattcctgct 9960
tctgtgctgt tttcctagac tgttttgtat ttgagcaaat attaattact ttatactttt 10020
tcagataaat acatggtctc ttataggaga ctggttttca aattgtgctc cttaactcag 10080
tcaagttcct tcttctattc agaacagctc tgtatttctt tatttggttt atatttccac 10140
ttgagatttt tattgggaca aaggattctc agccaacctt tttttaaaaa gcactccttt 10200
ataacaggaa acccttagat ctcaatcaag tatttattcg agtgactcct aagaagtttt 10260
ttggctctat ttgctggtat ctttttggca tttgtgcaaa gacgagatga ccatttgtga 10320
tcaagattta aagtccaact gctcactctt ctaacagcat ggccacatta ttagccgaaa 10380
atcacatctg agttatagag cttttgcttt tgtcagaaaa aaaacaaagt aactccaggg 10440

aatcttttat cagtcacttc tttaaaggat tgattgaaat aattgacact taagacagac 10500
actcaaaaat aggcacacta atcatttaca caagggattt ccaacccta ggatgtggac 10560
tggtaccaca gccagtccat ggtccggtgc gggcccatgg cctgttagga agtgggccac 10620
aaagcaggag gtgagcagtg gccaagcgag aattacagac tgagctccgc ctccctgtca 10680
catcagcagc agccttagat tctcataaga gtgagaaccc tattgtgaat tctgcatgca 10740
agggatctag gttgcgcact tcttatgaga atctaatacc tgatgatctg tggtagaaca 10800
atttcatcca gaaaccatcc cacaccatcc atggaaaaat tgtattccac aaaactggtc 10860
cctgatacca aaaaggttga ggactgctgg tttacatctt ggtttttaaa ctccattgtc 10920
atttaagaac atcagaaagt cacatactct gtatatcttc cctatctacc taatttgagg 10980
agaggccgat gaacacaata tccttcttgt ggctagatgg ccctcatata attaactata 11040
taatgcatac ttcatataat ctatcatcaa gacatttttt tctcctctga tgagatatta 11100
tgctttttaa taagtgatat gtaaatagcc tattgggttt tccttttat tttaactcct 11160
gcaaaacaag tacattttaa tgctcaattt taaaaaatta acaggttttg agctttattt 11220
ttaactgcta ctacagcgtc ttgtgttgtg tactttatat gacattttaa ggaatcataa 11280
ctttttcttt aaagcatagt atttactagt aatcattatt tctttaggaa aaaaatgcaa 11340
atacttttca tgctatttac tcttctataa ccaagtgatt tatttatgag atagaataca 11400
catgtagtga gtatggaccg tgagctcaaa acagtgctgt agcatttgta cgaaatataa 11460
aaattaaagg gtaaaaataa tggttatcat ttattgagtg tttactaaat tcaggtactc 11520
ttctaatgtc tctttgttta ctaactcatt taactcttgc aacaaaccta tgagacaggt 11580
tctatttttc tcattttaca gataagattt gttcagttag tcaacagtag agcagaactc 11640
cactcctgga gtcaatgccc ttaacttcca ctggatattc ctttgcagga aaagatacag 11700
tctttgattt caaggagcct gcagtgtgtg tgtggaagtg agtgattaac agacaacact 11760
gcaaatttag tgtttgatta aattgtgtgt tgcagactta gggtgctata agtgaactag 11820
agaggaaagt accaagagtt gtcattactg attgcctata tataatagaa accatgctaa 11880
atgctttata tttaatattt ctattcttca caacaatcct aaaaagtaga atttaccatt 11940
tccacttcat atgtaagcaa agactcagaa gttagttaac ttaccccaat taatagtagt 12000
actggcagag atgtgtctgc agtagttaga aaaggtagtt tattgagcta gtaagattta 12060
ttcaaagcct ttaaaaacag agacagtaat taagaaacgt ttatttttga tagttttttt 12120
aatataataa tattgtgact cctggcgtat tatacacaag aaaaatttca ggtgggtcaa 12180
caacttaatt tttttttat ttgaagaaaa gaaacctaaa agtatggaaa gatgatggag 12240
gtatgtattt atagtcttgg gatggaaaac tttctaaatt tgatataaaa tccagaattc 12300

aaaaacaaat ttgctccatt tgctatgtaa tctcaaattt cttatataag gcaaaaaatt 12360
cataaagtca aagacaacaa caaattagga aactgtaaca tatatgtaaa tcaaaaggaa 12420
attattaccc atatacaaaa tattcttaca aatcaagaaa aagacaaaca ggaagaaaaa 12480
tgaacaaaaa atattaagac agttcacaaa gtacagccca cagaagtatg aagaaggctc 12540
aactcactaa taagcattac tgattaaaat aataatggga atttcagcaa tttgagtact 12600
ggaataataa tgaataacaa ataatgacca ttggagaatc tgaagatagt ttggtattgt 12660
caattaaaat atgcgttact ttggcccaac aattctactt ctagaaactc atccttacag 12720
aactctagaa tacctgtagg tggatgttta ttgcagtatt acattattta taatactgag 12780
aaactgaata cagcccaaat gtctgttaaa aagagattag caaattgtgg tatacccttg 12840
caatgaaaag agtgagttag ctgtctatac tacagatgtc aagaagtctc taaaatattt 12900
gattttatga aaaagcaagg aacaatatca ttctgggtaa aaaattgtac ccatttgatc 12960
ttgctaggta tagagagcta tctaaaagaa aataactaag tgcttactgt caggatgaga 13020
gacttccaat ttctgatgac atgttttggt cttttttttt tttttttttt ttttgaggtg 13080
gaatctagct ctgtcaccca gcctggagtg cagtggtttg acctcagctc actgcaacct 13140
gtgcctccct ggttcaagtg attctcctgc ctcagcctcc cgaatagctg ggattacagg 13200
tgcccaccac catgcccagc taattttttt atttttagta gagacagggt ttcaccgtgt 13260
tgaccagatt ggtctcgaac tcctgacctc aggtgatcct cccgccttgg cctcccaaag 13320
tgttgggatt acaggcatga gccactgcac ccagcttcat tctttaagca agtatttact 13380
gaaggggctg caattacaca ccaacttggt gcctgacttt cctccacact ggccccgcct 13440
agtctcagct agcctctgct gccctaatgg tgtccagtga ccatatgtgc cacttttcac 13500
cttcaggtac cctagaatgt tgaaagagac taacaaagaa tgaaacttga agaatattga 13560
agcaaaagct tgaggttact ctgtatcatg aattaaggaa tccttcctga ctagatggag 13620
ccaagaatgg acacaggccc acaattcctc acacacaatc tcaatattca aaaagcactg 13680
caaacaaaaa ttttgttgta gtcacttgtc aacaatacct aacctgacgt gaatttgttt 13740
ggcagcaaaa tctgatctga aatcacatga ggaaatcttg tagtctatgt aaatattcgt 13800
acattttgct gcataaatat aatgttttat taagtgttgc tctattttat ctttctaaaa 13860
tacaaacaat tttgaatttt gaaacacaat tggcccaata catttcagct aagagatttt 13920
ggacatgtac tcagaaatga tttatagact atgttgagag tggtgcctgc tggctttttg 13980
caatgtagca gccctgaaaa tttttataaa caattcttat catttattct tatctttttt 14040
ttttgagagg gagtccccgc tctgtagccc aggctgaagt gcagtggcac aatctcagct 14100
cactgcactc tgcctcctgg gttcaagcga ttctcctgcc tcagcctccc gagtagctgg 14160
gattacaggc atgtaccacc acacacacta atttttgtag ttttagtaga gatggggttt 14220

caccatgttg gccaggttgc tcttgaactc ctgacctgaa gtgatcctcc caccttggca 14280
tcccaaagtg ctgggattac aggcgtgagc aacagcgccc agcctattct tatcattttt 14340
aataaattct tagtaccatg ctgggttcta cagggagcta taaaagataa ataagccata 14400
ttacctaact tcatatagtt ttcgttcaat catggagttt atcccatatt caagtataat 14460
ataggtaata tataaatcaa atgtgttttt gatttcaagg ggaaaaagat catatttagc 14520
tagggagacc aaaatgtgtt tttatggaaa ggatagcatg ctagatgggc tgctgagaat 14580
gcatgaaatt tttgatagca agaattgtaa tggcttttgt aaacaaaaac tacagactga 14640
gcagaaggac aatttggcta aagcatggaa catggtcaga atcccttacg attatatata 14700
aattatttgt gtttttttcta ggataattct tttcatgagg ttttgaaaga gatcagtgac 14760
cccttattcc cacccccaaa aaagttagct taccactgct ttaaaggaat ggtgaggatt 14820
attataaaac agaaggtcaa gaaaatgtgg aatgcacaat ggggtgttta caattaattg 14880
agtaaagagt gagaagcccc taaatatttt ttgggcagtg tagtggcata ttttaatatt 14940
taagaaaatt agtatatcag cagtatattg gaaagattaa tgtcagaaag gtaagggaca 15000
aggaaaacag tctggggatg gtgcactaag gacctgagcc agggttgtgg agagaggaat 15060
gggttcaatg ggtagattgg aaccaattga gaagggtgat gaaagcgaca cagagaattg 15120
tgggggtggg ggacgtcacc ttcagaaata ggaagtacat gatcatttta ctcagtaaag 15180
gagtgatgtt gaaagtagta tttaggaagg attatcctaa aagcaccatg cagaatatat 15240
ttgtacagag attagatagc ttgaagtcag agagaacagc taggaaactg gtaacacaga 15300
ttctgagaag tagtttaatt tcaaattgtt tgtattatat tctacataat gtcctcagca 15360
ggatattgaa ataatataat ttttgtgcac aaaataactt atgagaattg agatgttctg 15420
gtttgtcact tcatttagtc gtctgcttat ttattaattt ataccaaaaa taatgccgtt 15480
ttgtgcctgg atttgtgtta aatattgaga ctcaaaaatg gatgagccaa gttcataact 15540
taggagaaag gcacataaac acgttgcaga aatgcagtct ggtaatatct atgagaggtg 15600
tgagctgaga attgtggaag tataggacag gaagatctaa ccctggtgtt taaatgcggg 15660
gtatggaagg agaaagtctg aatcctgact aagtattaga ctaaaaatat taaatcttaa 15720
aaattattgt aatttcaaag tttgtggcaa ctttttttgta attaacagtg gagaccacaa 15780
tggtttatct tctttaagca gagcacttca gtgagtgttt ctttgtgcaa aggcccatcc 15840
agaggccact ttggatgggg gggcggatga aagtatgaag aactgaacct ctctatgtac 15900
tattttaata gaaattaggg aattgtaggc tagcagctgg ggcaaggagt aggaaataga 15960
aagggcctaa caactgagta ggaagcaagg gctcaaggag agtagatgcc tgtgagaatt 16020
tccctattgc aggcaacaat ccataaatcc ctaggctggt agtgcctgat agcaaacttg 16080

gaattgttta ctatctcatg ttttaattac ctccagctac ctcccaaccc ttaagtaaat 16140
ttttgctgtt tacattttcc tttataaaag gatatgcatt tcagattgct gacctttcct 16200
atgatgacaa gtgtctcagt agtatgatac ttagaagata tataggccgg gtgcaatggc 16260
tcacgcctgt aatcccagca ctctggaagg ccgaggtggg cagatcacga ggtcaggaga 16320
tcgtgaccat cctggctaac acggtgaaag aaaccccgtc tctactaaaa atacaaaaaa 16380
aaaaaatagc caggcatggt ggtgggtgcc tgtagtccca gctactcagg aggctgaggc 16440
aggagaatgg catgaaccta ggaggtggag cttacagtga gtggagatca caccactgca 16500
ctccagcctg ggtgacagag caagactctg ggaaaaaaaa aaagaagata tttagcagaa 16560
ggtacagtta atctgcccct tcaagttaaa gataatgttc atgatagaaa aaaggaggta 16620
acttctgttt gaaagacact gttgttttgt agatagcatt ttgaaaacaa gtctttgggg 16680
atgattccac tgttatttga ttttgttgtg aaggtattat aagagatcaa aattttatag 16740
tttcatacat aaacattttg aaataagatt ttctaacttg ctaaaaaatc ttccaaaaaa 16800
attcagttag gttctgaaca catttcataa tagtggcatt tattgaacac atatgtcagg 16860
aacttggcta aatgtgttat attaaacaca aagattttaa atcacttgca cagataaata 16920
gtaaataaaa tggtagagct gagattggaa cccacagaac cttctctctt atttgttaat 16980
attacctctt attcattatc tagtgtctct ttgtcctaag tatttgtaat gaaaattgat 17040
tgacatcagg gagagtagat acttgctagc tgaatttctg ggtaaaaaga agcttttcat 17100
aactggtatg tggaattgaa agtgaatatt atgactagat aatctgccct gcagacagtc 17160
ccctcattct ccatggcttc tgggatggct gtcttccacc ttctcttctc agcctgccag 17220
cactcccctg tcctcacttt cagctaacag ccttgcttcc taattttcgg aaaaaaactt 17280
tctgaaattg ctaccaccat ctgtatggac agaagtggcc tctgaactat aatcccattt 17340
ttctggggtt accagagctc tgcagttctc aagtccagcc ctctctcctt gaccactaga 17400
tccagattac tcctgctgac tcaaggacag gctctaccaa tcctcccgca acatttaaat 17460
catttttccc cttctctact ggataattcc tgtcagccta caacatgctc ttccttttct 17520
catcttaaaa tataaacaaa ctacttttgt tgatttcact tactcttcag ccaccaatca 17580
tttttcttct tttacagcaa aactcttcta acacattttc ttctcccatt cactcttaaa 17640
cctaacccaa tcagttgttg acctctgtca ctccaccaaa acttctcttg tcaaggttac 17700
catgttacta aatcttatgg gcaattgtca gtcccatgtt acttgactca ttggtagcat 17760
ttgacaattg atcactccac cttcctgcaa atactttatt cgcttggttt tcaagacacg 17820
accctctctt gattttcctc actagctccc cttcaaactg ttcttctcct ttagctttta 17880
atatgagagg gtccagtgct gagtattctg agtatttggt cctcttccct tctgtttata 17940
tacttacttc cttggttatc taatccagtc tccaaactgt ggataaacca cccaaaatcc 18000

aatcacttcc aaatttctat ctccagccca gacctttccc ctgaactcca gactgaatat 18060
ctcaaaccaa cacatcccaa agttggctcc tgatctactt agggttttct ctggtaagcc 18120
agtgttcttt tggtcctcaa gctatctgcc tcagaatcac ctgggtactg gtttgagatg 18180
atttctgcac ctcgccccag gtaagccaag tcaaaatatc taagaaaggg acccagaaat 18240
ggcatttcag ctagctgttt ggttgtttct taaagttctg cttggctcag aggggagcca 18300
gatacaaagc tgagaggtga aatggttggc tttgtttttg agaacattaa ctctagaagc 18360
aatggggaat agaggcagaa ggtcatgcca gggagatcag acctaagatt atttaaaata 18420
ctctttgatc gagatctggg ggccttcagt cttatgctga gaaaacacta cttttcattc 18480
cctaaagcag tgaacctgaa atatagaagg caggcaggct gtggccccta ccaagaatgc 18540
tcctttctct aggaagaatt ctaaaaaatg tgagaggcta gggagggagg ttggagactt 18600
ccacttttttg tgtccttact atataccagg cactatagta gacacattat atgtatttgt 18660
tataggcata ccttagagat atcgcaggtt ctgttctaga caactacaat aaagcaaata 18720
ttgcaataaa gcaagtcaca caaatttttt gctttctggt atctaaaaaa gttatgttta 18780
cactatactg cagtctgtta agtgtgcagt agctttatgt ctaaataaac aatgtacata 18840
ccctgattga aaatacttta ttgctaaaat tgctaatgat cacatgagcc ttaagggagt 18900
tgtaatgttt ttgctggtag agggtcttgc cttgatgtga atggtcattg acttatcgag 18960
gtagtgattg ctggaggttg gggtgtctgt ggcaatttct ttaaataaga caacagtgaa 19020
gtttaccata tccatagact gttcctttca caaaagatta ctctgtagta tgcaatgctg 19080
tgttttaccc acagtagagc ttctttgaaa attggagtca cttttctcaa aacctgctgc 19140
tgcactatca cctaagttta tataatatcc taaatcctaa atcttttgtt gtcatttcaa 19200
caatgctcac agcgtcttca ccaggagcag attgaatctc aagaaaccgc tttctttgct 19260
catccatgag acaagacccc tcatgtgttc aaattttatg agattttagc aattcagtca 19320
catctttgaa ctccacttct aattccagtt cttttgctat tcctaccaca tatgcagtta 19380
cttcctccat gaagtcttga atccctcaaa gtcatctata agggtttgaa tcaacttctt 19440
ccaaactgct gttaatgctg atgctttgac tttctccgat taaccacaaa tgttcttaat 19500
gacatctaaa atgatgaacg ctttctaaaa gattttcaat gtactttgcc cagattcatc 19560
agaggaatta caatctatgg cagctgtagc ctaataaaat gagtttctta agtcataagg 19620
cttgaaagtc agaattactc agtgatccat gggctgcaga atagatgttg tgttagcagg 19680
catgaataca ttaaccttct tgtacatttc catcagagct catgggaatt ttttttcta 19740
agcagtaggt ctcaacagtg ggcttaaagt agttagtgga ccatgctgta aatagatgtg 19800
ctgtcactca ggctttgttg ttccatttct agaacacaag cagagtagat ttagcataat 19860

tcttaagggc cttaggaact tcagaatgat aaatgaggat tggctttaac ttcaaatgcc 19920
cagcttcatt agcccctaac aagagagtca gcctttcctt tgaagctttg aagccaggca 19980
ttgacttctc ctatctagct ctgaaagttc taaatggcat ctttttccaa gagaagactg 20040
ttttatctac attgaaaatc tgttgtttag tgtagctacc ttcatccatg ggcttagcta 20100
gatcttttgg ataacttgct gcagcttcta catcagcact tgctgcttca ccttgtactt 20160
ttgtgttatg ggaatggctt ctttccttta acctcataat ccagcttctg ttttcaaact 20220
tttcttttac agctttcatc cctctctcac ccttgataga attgaagaga attatggcct 20280
tgctctggat taggtttggc tgaagggaat gttgtggctg gcgtgatcct ctatccagac 20340
cattaagcgt ttctccatat cagctataag gctttttttg ttttgttttt ttgctttctt 20400
atcattcatg tgttcactgg agtagcactt tttaacttcc ttcaagaaca tttcctttgc 20460
atttacaact tggctaactg gtgcaagagg cctagctttt ggcctgtctt ggcttttgac 20520
attcctccct cacgaagctt aatctttcta gcttttaatt taaggtgaca catatgcaac 20580
ccttcctttc acttgaacat ttagaggcca ttgtagggtt gttaattggc ctgatttcaa 20640
tattgttgtg acacagggaa taggaaatca tgaagaaaga gacaaggaca gaatgccaat 20700
tggtggagca gtctgaacac acacatttat cagttaagtt tattgtctta catgggcaag 20760
gtttgtggca cccaaaaaca attacagtaa taacatcaaa gatctctgat catagatcac 20820
catagcagag atgataatga aaaagtttga aatattttga gaattactaa aacgtgacag 20880
agacaggagg tgaacatgtg ctgttggaaa aatgatgccc atagtcttgc tggatgcagg 20940
gctgccacaa accctgaatt tgtaaaacat acaatatttg tgaagtacaa ttaagcaaag 21000
cgcaataaga tacgatatgc ttgtatctcc cttaattttc agctaagagc tgtttgagaa 21060
gttactgcca tctccttttt tcagatataa gaattaaaac aaggaaggaa gttaactttc 21120
ctaaagatac ctagccagtt agtaatggag ctggtaacca aacccagttc tggcagactc 21180
caaaaattaa accgctttca ctaaagcaca ccactgggca ctgtgtttga agcacgagat 21240
gatattagca actgattgaa gatttattga aaagacttaa aactcttatt gtagtcaagt 21300
cgtttaaaaa tattttttac ttttaaaatt tattattatt tagagacagg ctggagtgca 21360
gtggcatgat catagctcac tgcagccttg aactcctggg ctgaagtgat actcccacct 21420
caacctccag atattttttt aatagacaag gctttgcttt gttgcaggct ggtctggaac 21480
tcctggcctc aagcaatcct cccaaagtgc tggagtttca ggcataaacc accttgccta 21540
ggctcatttt tttttttttt tttttagacg gagtctcatt ctgtctccca ggagtgtaat 21600
ggcacaatct ctgctcactg caacctctgc ctcccaggtt caagtgactc tcctgcctca 21660
gcctccagag tagctgggat tacaggtgcc caccaccaag cccacctaat ttttttattt 21720
ttagtagaga cagggtttca ctgtgttggc taggctggtc tcaaactcct gaccttaagt 21780

gatctgcctg cctcagcctc ccaaagtgct gggattacag gcgtgagcca ctgcacctgg 21840
ccagttcaaa tcttatatat tttggtgcct agacttaaat aacatttagg aaaatgttaa 21900
ttaattatcc atatagttct acctttttct ttcttttttt tttttttttac aaagccagtt 21960
tcaagtaaga ctggggagat tctagaaagt tataacagaa tcttaagtca cttgatatgc 22020
ctaaacagaa tttgaagaaa taatgaagtt gaacacattg ataaacttag gatttctgta 22080
atcaggaaga aaaaactaaa cttcatagat atgagggaag gaattaacca tttgcatgta 22140
tgtaactgtt ataatcctac atcagagtag cacaaggaca tgaacaactt tgttgacaaa 22200
tagttttgta attttttaat gttttttatg tagctgtctt aaactccttt ctcaatagta 22260
tgtgaggtca aatgattgtg gtttcttttt aaaattttgc atgtaatgtg tagttcctta 22320
cattctctaa atgcttactg tttagctaac tatgttatta tggatgtcaa ttataactaa 22380
gtacttcata caatttcacg ttttataagt tagaagatat gtgaataatt agggcatttg 22440
gaagagttcc agagagattt ttttatttag aaagagagga gcagaacagc ttcaggaaag 22500
atagatgaaa agctgattca ttcaatttaa gatagttgaa gggaagaaaa aagaacacat 22560
tatttagaat gatgacatga agaacttaat ttcctttttt tacttatact ttaagttctg 22620
gggtacatgt gcagaacgtg cagttttgtt acatagatat acacgtgcca tggtggtttg 22680
ctgcacccgt caacccgtca cctacattag gtatttctcc taatgctatc tctcccctag 22740
ccccccatcc cctgacaggc cccaggtatgt aatgttcccc tccctaagtc catgtgttct 22800
cattgctcaa ctcccactta tcagtgagac aatgcggtgt ttggtttttct gttcttgtgt 22860
tagcttgctg atgatggttt ccagcttcat ccatgtccct gcgaagaaca tgaactcatt 22920
ctttttttatg gctgcatagt attccatggt gtatacgtgc cacatttgct taatccagtc 22980
tgtcattgat ggacatttgg cttggttcca agtctttgct attgtgaata gtgcttcagt 23040
aaacatatgt gtgcctgtgt ctttatagta gaatgattta taatcctctg ggtttatacc 23100
cagtaatggg attgctgggt caaatggtaa ttctagttct agatccttga ggaattgctg 23160
cactgtcttc cacaatggtt gaactaattt acactcccac caacagtgta aaagctttcc 23220
tatttctcca catcctctcc agcatctgtt gtttcctgac tttttaatgg ttgccattcc 23280
aactgccatg agatggtatc ttattgtgat tttgatttgc atttctctaa tgactagtga 23340
tgatgagcat tttttcatgt ctgtcggctg cataaatgtc tttttttgag aagtgtctgc 23400
tcatatcctt tgcccaattt atgaggttgt tttttccttg taaatttaag ttccttctag 23460
attctggata ttagtccttt gtcagatgga tagattgcaa aaattttctt ccattctgta 23520
ggttgcctgt tccctctgat gatagtttct tttgctgtgc agaagttctt tagtttaatt 23580
agatcccatt tgtcaatttt ggcttttgtt gccattgctt ttggtgtttt agacatgaag 23640

tctctgccta cacctatctc ctgaatagtg ttgcctaggt tttcttctag gatttttatg 23700

gtttgcagtc ttacatttaa gtctttaatc catcttgagt taatttttgt ataaggtgtt 23760

aggaaggggt ccagtttcag ttttctgctt atggctagcc agttttccca acaccattta 23820

ttaaacaggg aatcctttcc ccattgcttg tttgagtcaa gtttgtcaaa gatcagatgg 23880

ttgtatatgt gtggtgttat ttctgaggtc tcttttctgt ccattggtct atatatccgt 23940

tttggtacca gtgccatgct gttttggtta ctgcagcctt gtagtatagt ttgaagtcag 24000

gtagtgtgat gcctccagct ttgttctttt tgcttaggat tgtcttggct atgtgggctc 24060

ttttttggtt ccatatgaag tttaaagtag ctttttccaa ttctgtgaag aaagtcagtg 24120

gtagcttgat ggggataaca ttgaatgtat aaattacttt gggcagtatg gccattttca 24180

ctacattgat tcttcctacc catgagcatg gaatgttttt ccatttgttt gtgtcctctc 24240

tgattttctt gagcagtggt ttgtagttct ccttgaaaag gtccctaaaa cttcatttct 24300

aaaaaaaaaa aaaaaaaagg aatatgctac caaataggat tctttaaatc aattgtctcc 24360

tccttcagaa aacatccaga acctgacctc ttatcaccac ctccactgac cactttggtt 24420

taagccactc ttattcccct tcggatgtat tgcagtggtc tcctgacagt ctcatttcta 24480

tccttacctt cctagattcc agaggttgag agtctgccct cgacttagaa gccattgtga 24540

tactgttaaa atataagtca gatcatgtta gccgtctgct ccagtgtctc cagtggtacc 24600

atatctttta gagaaaaatc aaatttctta cagtggcctt caaggcccta cagcatctgc 24660

actctgctgc actctgattg tactatctac tactcttctc cctggcttag ccagctctag 24720

ccttactcac atcctcaaac aatccctgtc atctcttagc atcttagcaa tttcttcttt 24780

cctctctaat tcctttcccc aacactcccc acttctccaa cccattccag cttccagctt 24840

ccagcttcca tgagtatcct gaaacataac aaactttggt tactgccagt gtctcactta 24900

acacattccc ccactacagt gttgtcaagt tgtttcattt aacaccagta acatttaata 24960

tcagtaatag ccagattcta tcttaggaaa aatatgccta aaatttattt gctgaaaaaa 25020

aaagaaaaga aaaatttgga taataatctg ctttgagtta ttacaaacac agcagtatct 25080

gagaagcaag actgagtgtc ataaggaaac aaccagatta aataaaagca tagctggatg 25140

agataagaaa atactgcagt gtgatccaaa atgcagtaac agagttaaaa tccatattgg 25200

tcgtcataaa gagcaggaaa taacaccaat tatgtagagt tcacatttaa ggagttactt 25260

cggaatgaag agaaattggt aaactggtga aaattacaca ctatgattta ttgtttttat 25320

tatgtaccag tcattgtggt aacatttcat ctaatttagc tcacttaatc catataacag 25380

ccttataaaa tgtttatctc tatattatat gtgggaaaaa ctaagatttt gattaaacaa 25440

ttttatcatg ctgcagaaag tgacagaact gaatttgaat gaaagtctat ttgactctaa 25500

agcatatggc cttatcacta tgctgttgga caacagatac gatgaataga gagcacatat 25560

gcaacccata aatatccagt gattctgaag aagaaactaa aaaacaaggc agcaaaagta 25620
ttaattgatt atagaaaatg actcttcctt atgtatcttt tttacgagat catttctaca 25680
ttgtgtcacc agcacctaga acaatgttgg cacatagtag atattcaaga aatatttgtt 25740
tattgaactg tcataattac tgttaagcat ttattctgtg tctgttatgg tgctatagat 25800
tttatataca ttattttacc ctcaaagcaa cttttaaact gttattttat agatgaggat 25860
aatgtagtgc caagaagtta aatgatctgc tcaaggttat atagctaata ataggtggta 25920
gaacacgtat aacagaagta agtttagcag ttaaataaaa attttacttt ttagatcaaa 25980
aggactcact gcatcccagg taaatgtaat tttaaaaagc taaaactttg caatattggt 26040
aattttttaa aatttttcat gagaaggaaa aacaccctat atgcatttaa gtaggattaa 26100
taaaactggt taactactaa gacataaaaa ttgggttgac attagatttc ctctttgagt 26160
actaaatgct gagattggca aaaattccat caggttgagt tgctattctt gggtgaagat 26220
aacatatatt cttatatatg tgagaggtca ggaatatgcc cagtctttac cgaagtactt 26280
gtttgtctga tggttggact aatttgggat tcctgtagtg tcctgtagtt cagggtgaag 26340
tttagtaaca tgcgggggta agagacagga ggtgctattc tctgccctgc tgctcaccag 26400
tgtgctgtat ccctcctcag gatcaccctt aaggtctcag atgctcacac ctggtgttca 26460
gtgcccagtg gtctgttctc tagccagtat attctgtgtc gtcttagatt ccagtccaaa 26520
gactatttag atctgctcca tccttgggaa tttccacgca ctttattatt tcttctaggg 26580
caggggtccc caacctagaa caagcctacg atttgtgggt cataccatac tgactttcac 26640
catttcactc cttgaactat tgaacataca ttatcatttc tcctgccatt ttactcacct 26700
gctgctcacc tcctgctgtg aatcaggcca cacagcagga ggtgagcagc aggtgagtga 26760
gcattactgc ctgagctgca cctcccatca gaccagtggt ggcattagat tctcatagca 26820
gcgcaaaccc tattgcaaac tgcgcgtgcc agggatctag gttgtgctcc ttatgagaat 26880
ctaactaatg cctgataatc tgaagtggaa caatttcatc ctgaaatcac cgcttccccc 26940
aacccccccc acccctcact gcccccagtc cgtggaaaat tcgtctttca tgaaacctgt 27000
ccctggtgca aaaaagatta gggactgctg ttctagagaa attaaatgtt tttatgtcta 27060
ctttgttaca aggttacatg aaataaacca gttaaatgct tagtatattg gtctctaata 27120
tactcaataa gattttcctt ctctccttca ccccacacct ttttccttca cttttatcaa 27180
aaatccttgg atcttcatga agatatttta aaacctactg atttgttatt gtccagctgt 27240
ctttcacact ctgctagata gtcactcttc attccacttt acatgaccat cccctgactt 27300
actaacgtga aaaactggtc tgaacctttg tctcattctg catgcatctt cagaatatgc 27360
tggattccag aaaacataca aaaaacttta aggaatagta cagcgaatat ctgtaatcaa 27420

caatttgaag gtaagttgca gacatcatga catttcacct cttaaggctc caaaattcat 27480
gtcctaagga caagaacatc ttccattaca accacaatat tatcacccca aaattttaac 27540
attgataaaa caattctaat attcagctag tattaaaatt tcttcagttt ttcccaaaat 27600
gttttgtata gaatgttttt ttattcagga tccagtcagg aattgtgtat tttacttggt 27660
tagcaactct gtttagtttt ctttaatcta aaaagatctc ccaccttttt ttgtctttca 27720
ggacattggc atttttttgg gagtctaggc cagccgtttt gtagaaagtt ccataatctg 27780
gctttgtctg atttttcccc cacataatta tatgccagtt aaacgttttg acaagaatcc 27840
tacacaggcc aacggttctc agacactgat ctcatgaccc ccttatgtgc ttaaaaattg 27900
ctacaagccc aaagagcttc tgtatacgtg aattctctct ctcagtattt actgtgttag 27960
aaattaaaac tcataatgtt ttaaaacttt ttattagctc atttaaaaaa cagcaaaccc 28020
attagatact aatttttcaa accaaaaaat ttaatgacaa gaatgacatt gttttacatt 28080
tttataagtc attttaatgt ctatcttaat agaagacagc tggattctca tatctggatc 28140
tacagtcaat ctgttccaat atgttgtttt ggtggaagca tgggaaggaa atgtggcctc 28200
acacagatat gtagttggaa aatggaggag tattttaatt gtcttttcag acttgacaag 28260
tggtggccat ttcattagga attgcaaaag gatgattctc tatttctatt atcccttctg 28320
attttatcag ctataaatat ttttaatagt tctctctctc ttccaagagg caatctggta 28380
atcttaaaat acacttaata atggaaagcc taacagatac ttattattta cttttaattt 28440
tcagttttca aagttgttaa gtttgtgccc tagtaatctc cagtggtttc cagtgagttc 28500
ctcttgtttc tttcattctt tctcttctta catatcatta tgtactcatg ttttttaaaa 28560
tatatattca atgtgtttta attacttgaa ccagttcttt ttgatgttca agtggtttta 28620
attttggttg gtgtgaatta tcagtggtca atgaaacaca attttattag ccctcagtaa 28680
aagcaaatca tctgtcacag caaagcatcc caggctcaac ttgcacattt cctgccctag 28740
tcctggatca gtcatttccc caaggagctc tggttcgttt tagatgaaaa ttatattaca 28800
tgtgaaaatc tgaattcctg ctggagttgt ctttactttt gggcattttc agtgaagaga 28860
cctagaaaat acctcttttt gaaaaagtaa ggggatcatg agtttatacc atgatttctc 28920
aacctcacac tattgaccta ttgggccaca taattgttgc aggggggggat gtcctgtgca 28980
ttatgggatg ttattagcct ttttggcctc tctgcaataa tacctaccct ctagttgcat 29040
aaaccaaaat tgtctccaga tattgtgaaa tgtcctggtg aggaagggag caaaattgcc 29100
tctagttgag aattactggt ttatgtttgt atttctagca aaatcaaaat aattactagt 29160
tgttttatct gtatgtattg tgtgtgtgtg tgtgtgtgtg tgtgtgtgga ttcaaaatac 29220
tagtatcagc attattacct ctaacaacaa aacctgcttt gtagactagt tcttccatct 29280
cctgcataaa atcttaggcc agtcccaaag attttaatca gaagaacaat ctcattccac 29340

taaccgtatt agtctgttct catgctgctg tgaagaaata cctaagactg ggtaatttat 29400

aaaagaaaga ggtttaattg actcacagtt ccacatggct gggaaggcct caggaaactt 29460

acaatcatgg cccaagggga agtaaacatg tccttcttta caaggcagca ggagagagaa 29520

tgagtgccag taggggaaat gctagactct tataaaacca tcagatctca tgagaattca 29580

ctgattgatc acactgctca tgatgatcac gagaagagca tgggggggaaa ccaccccccat 29640

gattcaatta cctcccactg agttcctccc acaacatggg gggattatgg gattacaatt 29700

caagatgagg tttgggtggt gacacaaagc caaaccatat cagtaatcaa aatgcccagg 29760

gaagcattat gccaaattta caaatcctct tctaagttta gatttcttct ttgccccaat 29820

tcagcactca ctgctttgag cttgaagcct aaagggaaga acaaataaga tctgcttctt 29880

ctctgtttat gctgcctaca ctcacccaat gacagctgat tttgaccctt ccagggccaa 29940

cgtgtgggaa gagaagttag agagcacagt tttacacaac aagtaggaat tttagtgtca 30000

ctggggttat ctgtgtttgg tgggtgatta caggctgctc ttcctctagt gggatacttt 30060

ggtgagattc atggagatac tttactgaga gcctctaact gtaatcctgt aatgctgtaa 30120

tgcagtagac acctctcttc tctctggaca tgaccgtgac cctctgcttc tggacttccg 30180

ctgactgacc ctacacagat tctgttggat cacaatactc tctaggttgt cctcttagct 30240

ggagccagtt tttaaaagaa agaaactttt attgataaat attttcagaa aagcactcaa 30300

agatgtagag cttatcagta ggtaaatgaa acgatgttat tatcacccag accaaaaaat 30360

ttaaagatca ttaggttccc agaagctcca ttttgcccct tccaatcatt ccccaaaagt 30420

aaccgccatc ccaatttcta acatgataga ttggtttgaa ctattttaga actttatatt 30480

aatggaatca gatagtattt ttctatgtct gcttatttta cacaacatta tacttatgta 30540

gttcatccac attttgcatg gagctagaca tttattctca ttgctgtata ataatgcatt 30600

gaataaataa aaccatttgt ttacacatta tgctgtaggt gatcatttgg gttgttttca 30660

gattgaggct attatgaaca ctgctgctat aaatattctt gtacaaatct tttatttttt 30720

gcatgtggac acacctgtcc ctacagataa gcttttcatt tgcctctcca aggaccctgg 30780

caatttcact ggtcctgtac cagtctttgt tccttggccc aggttcctat accatgtgat 30840

agtgaaaatt cacctcccat taaaggccaa tattttattt tgtttatgtt gtttttggca 30900

taaatgattt ttgtcctcac cctgaatttt ggacagattc ctggctgcct ggcattttct 30960

gtgtagtgtt ttctagccct gttttcattg ctaaggcagt cttttaaggt tctacatttt 31020

tatgcatata gttcagccct gccactctct tccattggga ctgaagtaat atatcccatc 31080

ctctcatggg ctttaaaccc catttcttat cttttaggca tttcttatat ctgtatctga 31140

aactttttg gatcactgca gagtccagtt aagtttgttc ctccagcttt catttctttt 31200

ttcttttctg gcttatagag atttacctac cttttttttcc agactgaaat taagcaacat 31260
gcttctatta tattttatcc agtatttctg tgtttgtggt agaaagattg ccctcatatg 31320
gaccagctca tgttgcttca agcccgtgac ttgtgggtca taccatactg actttcacta 31380
tttcattcct taaactactg aacatatatt atttcctgcc atttgactat gtattttccc 31440
cttcttttca aaaatgccat gaaaaattgt gaactctctc agacctaagt gaactatgtt 31500
cacttgtgct catattgaga agcaacacaa acaatatagg tctcttattt tacaggtaag 31560
tccctttctt gtttctaata aagatatttt atttcctaat aaagatattt tatttccttt 31620
aaagtggaat tttgtcatct aaaatttttg taagtcacta ggcaaataaa atgatctaat 31680
cacattttga tagtgctttt attttcacag agtatttttt attaagagta acacttgggg 31740
ctgggcacgg tggctcacgc ctgtaatctc agcacttcag gaggccgagg caggaggatc 31800
acaaggtcag gagatcgaga ccatcctggc taacacggtg aaaccccatc tctactaaaa 31860
atacaaaaaa ttagccagac gtggtggtgg gcgcctgtag ttccagctac tcgggaggct 31920
gaggcagggg aatggcatga atccaagagg cagagcttgc agtgagctga gatggctcca 31980
ccgcactcca gcctgagcga cggagcgaga ctccgctcaa aaaaaaaaaa aaaaagagta 32040
acacttggaa ataatcttgt tagacttcac atttaattat ggtctattac tataaaaagg 32100
tgagaaatta ctgaataaaa agcattctgt aagaaaaaaa gttaaaaggt ggtaaaaaat 32160
cagaagcaag tgactattta ctatatatga caataagtga catatgtctt atactgttga 32220
gtataactat atttacaaat ataagcgaaa caataggtat aattgtatat acacctatct 32280
aaaaaatatt tatcaagctg tattttacac aagagttaaa agtatcaaac tgagtagttc 32340
ctactcttat agaacctagt agctatagac acaaagtata caataggaat acattgaatg 32400
tgcagttggt acacagaaga aatgtctaaa gcctttatga gagcagtcag gtaagaccag 32460
atgaggggag cagttaagcc aaggcttgaa agacgagtga gattttgtta agttgataaa 32520
ttggaaggaa gggaggaaag tcatagaggc atgagagagt ttgggggtaa tccaaggatg 32580
ttagtactct ggagcataaa gaacatggga aagagtatgt gattagaaag aaaagacata 32640
acagtttgaa ctcacccccct gctttcccca ccctaaaata gatcagtttt gcaaggagaa 32700
agaagcatag tgctctatta agctccccag gtttcaacag gggcagatga tgcatatatt 32760
aagggagaga gctagatagt ggctcctagt ggggtgaact cccctattta tcattatgcc 32820
atgtaacctg tttgtggtgg cttatagctt ataagtgttc ctaaatgtct tataactcct 32880
ggtggtgata atgtaggtaa acagcaaaga tggcagcaaa tggaatgctc tgagtggttt 32940
aatatagtga cgtgttttac acttgttgta gaatcacaat gttatgttgg actgaggaga 33000
agaaataata cgcaatttga gccatcaacc tacggtttat gagtgagtga gtggatctag 33060
acagggatgt gtttctcaaa accagaatat aaactgtggg ctgttgtctg aagaaaatgg 33120

tagcaactga tgttatgtta gattttcatc tttttctaga tttccaggaa aatgttgtga 33180
cagggctaat gcacttgtgc ctgacctttc ttcacctgca tatacagcca ggtacctgaa 33240
gtagcgagtg gctcccactg cattcattgc attcttggtg gctctgagtc ctggccatgc 33300
attatggttc agaggcaatg cctgggcccc atgccaaatt cccttagtcg gctctagttg 33360
gaactggctg gctacaacat ttctttaagc caaaacattg cttgagaaga taaaccttta 33420
actgtactcc ccatattttg tttgcatgat aatgcatcta ctttgcagtt ttgatggctc 33480
atcacttaat gttaaagatg atgaaaagtt agagtgaatt gagcttccag atttctttaa 33540
gccattccag cctgtcacag gcagtctaca atgatatgag tcgtgccaac ccctcaaggt 33600
cgtgacttca tgccagtgcc cttaattatt aaaaattatc cttccaactc aaactttaaa 33660
tattggacat ctaagtcatt ttttcttcca aacttataag aattgcattc tgtttggtaa 33720
ctataatttc cttatttata ctcatgttta aatttatact tatgtttaaa tttatttggt 33780
acttatcatc agtctcttca taccagactt cctacctcat acattgtctg ctataacgta 33840
tctgttgttg actggattac atcttttgga agagcatact ggggtcttat ttcccaattt 33900
catgcaagtt tgagaatatc tttaagttgc ctttaaaact gaataacttg actagatata 33960
gacttcttgg accacatcta gaaaaaatct aactatattt gagatttaga tgtgtgataa 34020
tcataattat aatagacatt tgttaagtac ttgctatgtg ccagactatt ctaagctctt 34080
acatgagctt gtgtattcct tagatccaat ccaaggtact aatgttatct tcattttaat 34140
tgccaaatgt catctagcta ctaagtgatg agattgacat tcacacttaa acagtaactt 34200
tctatactac ctctcaagat aattaaccta aaatgtttta actgtgtgac cttgagcatg 34260
ttatttaact tctagatgcc tcagttaact ctgtataaaa tgagaatatt aatacctcca 34320
aaggttgctt tgatgatgaa atgagttaat atatgtcaat agctcataac aatgcctggc 34380
acatagtaag tgcttaataa ttgtctgttg ttcttttatt atttttattt gtggatagta 34440
agttgcagat ctgagctttg aacaagcagt ctggcctctg taactttgct cttcggtact 34500
acaatgtagt gtcagcaatt aagatggcat tttaatttag tgggggaaaa catggattag 34560
tcagtaaata gtgttgggat atagtagata ctcaatactt gttgaatgaa tgagagtttt 34620
ataaaagtat aaatacatta acttaaatat taacaaatag atattaaata ccatttagat 34680
ttgtatagac agtttggaaa tactttagtc tgttccactg caataaaaaa ataccttaga 34740
ctgggtagtt tataaataca gaaatatttt ctgatggttc taaaggctga gaagtccaag 34800
atcaaggtgg cacagattcc atgtctggta aaagcttgct gtcttcttcc aagatggcgc 34860
cccttgctgc atcctcacat ggcagaaagg ataaaaagga ccaaattcac tccctcaagc 34920
ccttttaaaa gggtactgat tccatccatg gaggcagaga ccccacctct taatattgtc 34980

acattgggga ttaagtttca atatgaattt tgaaggggac acaaacattc aaaccatagc 35040
aagaaggaag caaaaatgag aatagatgca tgattatcaa attgtaggtt attttctgtt 35100
ttgaatttcc ttacgttctt atataatttc tctaataata caattcatga tataatttgt 35160
acagataacc acttagttgt attttgtata tattgagttt gaggtaccta gaaatatgac 35220
tggaaggtca gagctcatat tgagattttg ggaccatcag aagataatga gacagactac 35280
ccacaaagtg tgtgcagtgg cacaccacat ataaggaata agcaaagtaa gaggagttga 35340
caaagggaat ttaaaaggaa gtgctggaaa gacaggtcaa gagtcagaaa agagaggccc 35400
caaaatatga atcaatggga caggtgggga aagaaattac ctgaagcaaa tgattcaggt 35460
caggaaagca ttcacagcat ttaggaatga aaaaatattt gatgatgtta agccatttta 35520
taagaatagt gaatatacaa gtcaaattac cacataggaa taaaggaagt taggtagaaa 35580
cagcaaatgt agatgatgtt ttcgacaact ttgaggacca aaaaaagggg aatgtattca 35640
gggagaatct agagtgagct agggttcctt tttttggtgc tggaactttt ttggatggaa 35700
aaataatgag agagcaggac atcactggac tggagagact aaagacttta ggagagaaag 35760
gaggaaattg atggagcaag acttgaaagc agataggaag gaattgtttg aaggacaaaa 35820
gcaaaaacca ttggtaagga ggactctttg agacactgaa taaggagatg atatcgtagg 35880
ccattttaga aatgtaagtg agacagatca gcagcatgcc agcttctggt gtttagatca 35940
gacctttttc ctgcgtattt atgaccaagg agcagacagt aggacagcct ctcttcaaac 36000
aattacatct gcgtgctcta tttctcgaag tctgttttct gtatagagaa aaccttggtg 36060
ggcgtggtgg ctcatgcctg taatcccagc actttgggag gccaaggcgg gcagatcacc 36120
tgaggttggg agttcgagac cagcctgacc aacatgcaga aaccctgtct ctactaaaaa 36180
tacaaaatta cccaggcgtg gtggtgcatg cctgtaattc cagctactca gaggctgagg 36240
caggaaagtt ccttgaaccc aggaggtgga cgttgtggtg agccgagact gtgcctttgc 36300
tttccaggct gggcaacaag agcgaaactc cgtctgaaag aaaaagaata gaaaatcttg 36360
ctattttctg ttctctcttc ttgtgagata tcagtatagg gtagaaatag atgatcaact 36420
gatagactga cttacacaca ccttgctctc acagtcactc ctatagccta aatatataac 36480
ttgtggtaaa aaaaaaaaaa attagagtca aatcagaaag tagtatgcag tacactctgc 36540
taacctcctg taggactggc actatgtgca caaaagtcag caaagatatg aggcttcaca 36600
gttacccttt attaggtgaa ggcttaattc ctgggactct aaacaagaga tgaacactat 36660
gtgagtgtac atgaacataa gtttatagag gcatgcatct ttaagaaaat atttgtgctt 36720
ttccagaaat gcatgaggtc aggaaccttc ttgatatcag ctgtcacata tataggcctt 36780
ttggcatttt tgccctttgc cgtttaggct aatcctttcc cctgcttgat taagtacatg 36840
ttttaggtac ctctgagttc ctttactacc aactttggcc agaagactct gattttaatc 36900

ttaactaatg taaaatacta caggaaaatc ccatgatctt ttaaaattat gtgtcatgct 36960
tcagcagtat aagtaggctg aagtaggctg atgatattaa aagactcaca gtctgtttta 37020
aagaaattta ttgggaaaaa aagagagtgt caaaaatagt aaattaacgt ttacatgttt 37080
aagctgaatt tttttatttt tgttaaatct aaataaatta gattgttccg tgccttatta 37140
acatatttgt ggtttaaaaa tcattattga atattaaatt catgtgccag acctttttaa 37200
aggcatccat agtttcctct acacaaagtg accccagggt gatgtgcgat gtgtattgtc 37260
tatacttcta taaagtttct cctctttatt gagtacttct atctgtttta agttcttcct 37320
ttatcattat ttctgttcaa ggtattttct aatgtgtagt caaaataagg caggtattaa 37380
attcagtaca ataagtgatc ttttttacat gctcttccat tgattcctta aagagagaac 37440
cagctctggg atttttccaa tagaactaag catttagatt ttttttttaaa aggtcttttc 37500
cagaggcctc ttcaattctt tagtctgtat ctgttttctt gaaggcatca atgtcagaaa 37560
gaaaggtttt actagagttt gaccacctgg tacaactaat gcttatcaga aacgtgtagc 37620
taatcttgaa atgacatcta aaggcaatct gatactgaca ccattaagaa gataaacaga 37680
aatctcttgc aaaagatttt taaaggagcc ctaggattga ttcttcagct atgctcacct 37740
aatttaggct cctaaaggca caggtaaagt actgagcagt tttatagctt caccaacatt 37800
cagctgtgcc aggcaggaag ctggctgagt catgcacttg ctgatgtgta tctagtctct 37860
atttcagttg tcactgcata gaatttccag tgaatcttag atattgtaaa taattttgac 37920
tcattttgtt tttttagggc agcacaacta cttatgtgct ggaagaaatg actgcatcgt 37980
tgataaaatc cgcagaaaaa actgcccagc atgtcgcctt agaaagtgct gtcaggctgg 38040
catggtcctt ggaggtaatt ctgatgtttt catcaataat atactgtgta atctttatac 38100
tataaaactg tgtgtcagaa aattgcaatt tcttattcat ctttgttttt gtttttttctg 38160
tcttgattat tggcagtgac tctgcacatg tgagtgtctg aatgaagcaa ccaataccat 38220
tccattaact ttacagtttt ctagcacatt gaggtctgtt aagagaagaa aactcaattc 38280
atctatattg cctataagta gaggtaatgg gcaatgtact gacctgtcat taagtaacat 38340
gtgtgggagt ctaggctttg ctgcccatct cctgtgtgac cacagctctt cctgggcatc 38400
agtctactta tctgtaaaac gagagcatta acattccttt cagctcaaaa aattgactct 38460
gcaaattggt caggtacatt gcttactagc ttctccaagt gattgattaa agcaagatta 38520
ctgtaactgg ggtaggaatc catttgatga agagttatat taataattcc cttttcccat 38580
atttttatta gaatctctaa aactttcagt aatatagcac acatttaaaa aattaatgtc 38640
agatagtgac agatagtttg aatgaaaaca tagcagagtg agggagatca agcgagccac 38700
agttatgcaa gacacccgta ctcccttgaa ttttttcact tagccacatc ctgcacaaca 38760

ctgtctggga atcgtagctt ttacatctag atataacttt ttaaaaacat aataaaaata 38820
ttacataatt gttaaagcta aaaatgtttt ttcagtgcac taactataaa cagcttgtgt 38880
tccatggacg ttgtgtgtat tttcctttgg gaaatgctat atttagacca atgcttgtca 38940
agtttaaagc gtatacaact gtcacctcat ttgtttttgc aaatgtgctg gaccaaaatt 39000
aatcacccag gtaacatact gggcacttta cagtaattca tgtaattaga tcacttatag 39060
aattttataa tttcttgatt aaagatttgt tttcctaagt taagggcagt gattaggttg 39120
acagtagtgc ctggcttaag gaggtgctca gtaactttct attaaatgaa taaaaccaca 39180
acctcagcag attaaaattg tttttgagtc ccacacctca attaccacct ctttgtattt 39240
ctgtttcaac tcagaagtct taaggaacct cctaaatttt agaaaataca actttttcac 39300
aaaatcacac atctagtcaa tgtcggagcc agaagaaaga cctgaaggta ttctgaccct 39360
ggcgcagcca ttgttttctc cttggcatgc ttgccttaga aagtagcaga gcatctaatg 39420
ctgacactta gattccatta agatctttaa tcttgagtgt tttcccaatg actttaaaag 39480
actcttcaat tttatcaaag aatgaaaatg gcggagttct ggatagaacc ttcagaatac 39540
tcctggggat cagcaatata acctagagaa atttatgtta tcttttaata ttctagggct 39600
ttatttattt atttttgaga cagagtctca ctctgtcgcc caggctggag tgcaatggtg 39660
caatcttggc tcactgcaac ctctgcctcc caggttccag cgattctcct gccacaacct 39720
cccaagtagc tggtatcata ggcacccgcc attacgccca gctaattttt gtattttagt 39780
agagatgggg tttcaccatg ttggccaggc tggttttgaa ctcctgacct caggtgatct 39840
gcccacctcg gccttctaaa gtgctgggat tacaggcgtg agccactgca cccggccttt 39900
agcatattat tcaagcatct gtaatccttt atcttctcta accaaactgc ttgttacttt 39960
agcctgttat tcctatgctt cccaagccat attctacggg cttcacagct attgctgcat 40020
tttcatacct tttatattac ttatttgttt aactctactc cctccatctc tccctccttt 40080
ccgtccctcc ctccctccct tccttcttct ttccttcctc cctcttctcc ctcaatcgct 40140
ttctaatttg caccccatcc ctttctttca gtaactgtat atcgagccct ttctagttac 40200
cagatactat tctaagttct gcgcatatag cagcaaagaa aatggacaaa attcctgccc 40260
tcatagatct tacactgtgg gaggacgagc aacaaatgta catgtcaaat aatgataagt 40320
gctgtggaaa gatattaagc agagttagtg gggattatca ggaagcagac tcactactaa 40380
actcattagt ctaatatttt tgttttgctt agcctcttcc taagcaataa tatctctgaa 40440
ataatggatt tgatatgcca gttataattt ttctaaaaga cattaatata cattcttaac 40500
tatttaaata tatattcgat gtaccaccta aaaactgctt gtataccata aatgattatg 40560
tgttaaactc tgggaaagag tgtattagac cattttttc cagctatcgc agctctattt 40620
attttttcag cttttatttt agcttcagtg ggtacatgtg caggtttgtt acctgggtat 40680

attgtgtgac actaatgttt gaggtacaaa tgatcccatc acctaggtac tgaggtactg 40740
agcatagtac tcaatagttt ttcaaccctt tcttcttctt ccttcctcct ctagtagtcc 40800
tcagtttcca ttgttatgct catgaatacc caatgtctag ctcccactta taagtgagaa 40860
catgcagtat ttggtttttt gttcctgtgt taatttgctt agggtcttgg cctacagcta 40920
catccatgct gctgcaaagg atgtgacttc gttcttttca tggctgcata atattccatg 40980
gtgtatatat gccacatttt ctttgtccaa tccactatta atgggtgcct cagttaattc 41040
catgtctttg cttttgtgag tagtgctgtg atgaacatgt gagtgcatgt gtctttctag 41100
tagaacaatt tattttcttt tggctatata tccagtaatg ggattgctgg tctaatggta 41160
gttctgtttt aagttctttg agggccggga gcggtggctc atgcctgtaa tcccagcact 41220
ttgggaggcc aacgcaggtg gatcatgagt tcaggagatg gagaccatcc tggctaacac 41280
agtgaaaccc cgtctctact aaaaacacaa aaaaattagc caggcatggt ggcaggcgcc 41340
tgtagtccca cctacttggg aggctgaggc aggagaatgg catgaacctg ggaggcggag 41400
cttgcagtga gctgagttcg cgccactgca ctccagcctg ggcgacagag cgagactgtc 41460
tcaaaagaaa aaaaaatgtc ttttgagaag tgttcaaacc aatttctacc gtggcagaac 41520
taatttacat tcccaccaac agtatataag tattcccttt tttccacagc ctcaccagca 41580
tctattaggt tttgagtttt ttgagttttc ttttttgaga cagagtctca ctctgtcacc 41640
cagactggag tgctgtggca caattatggc tcactgcagc ctcaatctcc tgggctcaag 41700
tgatcttcct acctcagcat cctgagtagc tgagactaca ggcacgcacc accacacctg 41760
gcaaactttt tatttatttt ttttttgtaa cgataaggtc tcactttgtt acccagtcag 41820
gtctcaaact tctgggctca agtgatcctc ccaaagtgct ggaattacag gtgtgagcca 41880
ccatgcctgg catgagtttt taataatagt cattctgatt ggtgtaagat ggtatgtcat 41940
tgtcgctttg atttgcatat cttaatgatt agtgatatgg agcattttaa atgtttgttg 42000
gctgcttgta tgtctccttt tgagaaatgt ctgttcaagt cttttgccca tttttttggt 42060
gagatttttt ttttttgctt gttcaactgt ttaagttcct tatagattct ggatattaga 42120
catttgttgg aggcatagtt tgtgaacatg ttctcctatt ctgttggttg tctgttaact 42180
ccattggtag tttattttgc tgtgcagaaa ctctttagtt gaattaggtc tcacttgtta 42240
atttttgttt ttgtttcaat tgcttttgag aacttagtta gaaattattt cccaaggctg 42300
atatccagaa tcatatttcc tagattttct tttaggattc ctatggtttg aggtgttata 42360
tttaaatctt taatctacct tgaggtaatt tttgtatatg gtgaaatgta ggggtccaat 42420
ttcattattc tgcatgtgac taggtaggta tcccagcacc atttattgaa taaggaatcc 42480
tttctgcatt gcttattttt gtcaacattg tcaaagatca gatggctgta ggtgtgcacc 42540

tttatttctg ggttctatat tctgttccat tggtctatgt gtctgctctc gtgccagtat 42600
catgctgttt tggttactgt agccttacag tatagtttgg aattgggtaa tgtgatgcct 42660
ctgacttttc tttttgttta ggactgctgt agttatttgg gctcttttga ttttatataa 42720
attttagaat agctttttct agttctggaa aaaatgtcgt tggtagtttg ataggaataa 42780
cattgaatct gtaaattgct ttggacagta tggccatttt aacaatactg attcttctaa 42840
tccatgaaca tggaatgttt ttacatgtgt tagtgtcatc tgtgatttct tttagcaatg 42900
ttatgtagtt atccatgtag aaatctttca tctgcttagt tatatgtata cctaggtatt 42960
ttgtgtgtgt gtgtggctat tttaaatggg attactttct tgatttggct ctcaccttga 43020
atgctatagt tgtatagaaa tggtactaat ttttacatgt tcattttgtg tcctgaaact 43080
ttactgaagt catttatcag ttttaggagc cttttggtgg agtctttagg gttttctagt 43140
tataaaatca tgtaatatgc aagagagata gtttgacttc ttttttttcat gtttgaatgc 43200
cttttatttc cttctcttgc ctgactgctc tggctagcac ttccagtgtt aacaggagtg 43260
gtgagagtga gcatccttaa ctttttccag atctcaagga aaattcttcc agcttttgcc 43320
cattcagtct gatattggtt gtgagtttgt cattttggtc tcttattgtt tttaggtata 43380
tttctttgtt gcctagtttc ttgagggttt ttatcatgaa gtggtattgc attttatcga 43440
aagctgtttc tgtgtctatt gagatgatca tatggtttta tttttaattc ttctcatgtg 43500
gtaaatcata tttattgatt tgcatatatt gagctaacct tccataccag gagtgaagcc 43560
aacttgatcg tggtaaatta actttgatgt ttgatttggt ttgctagtgt tttgttgaga 43620
atttttgtgt ctgtgtttat cagagatatt gtcctatagt tttcctttct catggtatct 43680
cttacaggtt ttggtatcag ggtgatactg gcttcagaaa acgagttagg gaggagtctt 43740
tccttctcga ttttttgaaa tagtttcagt agaattggta ccagctctac tttgtgcatc 43800
tggtagaatt tagctgtgaa tctctctggc cagggctttc tttttggttg gtaagttttt 43860
tattactaat tccatttgga acccaatatt ggtctgttca gtgctttagt tttttcctga 43920
tttaaacttg ggacattgtg tgttttcagg aatttattca tttcctctag atattgtagt 43980
ttgtgtgtgt agaggtgttc acaatagtta tctaaggatc tttcatattt ctgtgggatc 44040
agttgtgatg tcacctttgt catttctgtt tgtgtttatt tggatcttct ctcttttttt 44100
ctttgttaat gtagctagca gtctatcaat cttatttatc ctttaaaaaa ataacttttg 44160
gttttgttga ttctttgtat agatttttgg gtctcaattt catttggttc tgctctttag 44220
ttcgtccttt tcttctgcta gctttaggga tagtttgttc ttgttttttt tttttttttt 44280
tttttttag ttcctctagg tgtgatgtta ggtcattaat ttgagatctt tctaactttt 44340
tgaggtaggc atttagagct gtaaactctc ctcttaatat tgtttttgct acattccaga 44400
gattttggta tgttgtgtct gttttcattt attttaattt ttttttattt ctgccttgat 44460

tttattgttt actcaaagtt atccagagca aactttttaa tttccatgta attgtgtggt 44520
ttgtacagat cttcttgatg ttggtttctc tttgtattcc actgtgacct gacagtatga 44580
ctggcatgat tttgactttt taaaatgtac tgagtcttgg tttatggcca aggatgtggt 44640
gaatcttgga gtatatgttc tgtgtacaga tgagaagaat ttacgttctg tggttgatgg 44700
gtggattatt ctgtagatgt ttattagatc caattggaca agcattgagt ttaagtccag 44760
aatttctttg ttagtcttct gccttgatga tctaatgcta tcagtccccc actattgttg 44820
tgtggctttc taggtcctta gaagtccttg ttttatgaat ctgggtgtgc cagtgttgag 44880
ggcgtatgta tttaggatag ttaaatcttc ttattgaact ctttatcgtt atgcccttat 44940
ttttcctttt ttactgttgt taaagtctgt tttatctaat ctaagaatag caacccctgc 45000
tctttttgt tttctgtttg catgctacat ctttctccaa ccctttactt tgagcctatg 45060
gttgtcatta catgtgagat gggtttcttg aatacagaag atggatgagt ctggttttcc 45120
tagctggttt gccactctgt gacttttttt ttttttaga tggagtctta ctctgatgcc 45180
caggctggag tgcagtggcg caatctcagc tcactgcaac ctccacctcc caggttcaag 45240
caattctcct gtctcagcct ccggagtagc tgggattata tgcacctgcc accacggctg 45300
gctaattttt gtatttttag tagagatggg gttttacctt gttggtcagg ctggtcttga 45360
actcctggtc tcaggtgatc cacccgcctc agcctcccaa agttctggga ttacaggtgt 45420
gagccactgt gcctggaccc actctgtgac ttttaaatgg gacattgtat tagtccattc 45480
ttgtgctgct ataaagtact acctgagact gggtaattta tgaagaaatg acatttaaat 45540
gactcatagt tctacaagct taacaggaag catagctagg caacgtcagg aaacttacaa 45600
tcaaggcaga aggtgaaggg gaagcaagaa tatcttacca tggcagagta ggagaggtgg 45660
gtcaggggag tgccacacac ttttaaatca ttatatcttg ggagaaccca ctcactatca 45720
tgacaacagc atgggggaaa tttgcctcca tgatccaata acctcctacc aggttcctcc 45780
ctcaacattg ggaattacaa ttcaacgtga gatttggatg gggatacaga gccaaaccat 45840
atcattctgc ccctggcccc tcccaaagct tatgtccttc tcagttttca aaacacaatc 45900
atgccttccc aacagtcccc caaagtctta actcattcca gcattaaccc aaaagtccaa 45960
gaccaaagtc tcgtctgaga caaggcaagt cccttccacc tatgatcttg taatatcaaa 46020
aacaagttag ttacttccaa gatacaatag gggtatgggc attgggtaaa tactcccatt 46080
ttaaatggga gtaactggcc aaaacaaagg agatacaggc cccaagcaag ttcaaaaccc 46140
agcagggcag tcattaaata ttaaagctcc aaaataatct cttttgtctc catgcctcac 46200
atccagagca tactgatgca agggatgggc tcccaaggcc ttcagcagct ccaccctgtg 46260
gctctacagg atacagcccc cacagctgct tttacaggct ggcattgaat gcctgcaggt 46320

attccaggca cacaatgcaa gctgttgatg gatctacgat tctgaggtct ggttgatggt 46380
ggtcctcttc tcacagctcc actaggcagt gccccagtgg gaactctgtg caggggctcc 46440
aaccccacat ttccctttca cactgtccta gtagaggttc tccatgaggt ctctgcgctt 46500
gaagcagact tctgcctgga catccaggca tttccataca tcctctgaaa tctaggtgga 46560
ggtttccaaa cctcaactct tgccttcttc atacccgcag gcccaatacc atgtggaagc 46620
caccagggct ttgggtttgc atcctctgaa gccctggccc aagctgtacc ttggcccctt 46680
ttagcaacag ctggagctgg agcagctgga atgcagggca ccatgtccca aggctgcaca 46740
gagtagctag gccctgggcc tggccctcaa aaccattaat ccctcttagg cttctgtgcc 46800
tgtgatggga ggggctgctg tgaaggtctc tgaaatgccc tggagacatt ttccccattg 46860
tcttgactgt taacatttga ctcctcttta ctaatgcaaa tttctgcagc aggcttgaat 46920
tgctccccag gaaatgtttt tttgtctctc tcttttctac cacatggcca ggctacaaat 46980
attccaatat attttttgct ttgcttcact tttaaatgta agttccaggt tcagataatc 47040
tctttttttca tgcatatgag catacatgtt tagaaacagc caggtcacat acatctggaa 47100
tgctttgttg cttagaaatt ttttccacca gataccccaa atcatctctc tcaagttcaa 47160
agcttcacag atctctagga caggggcaaa atgccaccag tctttttgct aaagcatagc 47220
aagagtgacc tttactccag ttcccaataa gttcctcact ccatctgaga ccacctaagc 47280
ctagactcat tgtccatgtc actatcagca ttttggtcac aaccattcaa caagtctcca 47340
ggaagttcca aacattccca tatctttctg tcttctgagc cctgcaaact gttccaacct 47400
ctgcccatta ctcagttcca aagtcactcc cacattttca ggtatcttct taactgtgct 47460
ccactctccc agtaccaatt ttctgtatta gctcattctc atactgttat aaagaactac 47520
ctgagactgg gtaatttatg aagaaaagaa ctttaactga ctcacagttc tgcaggctta 47580
acaggaagca tagctaggag gtctcaggaa acttacaatc atggtggaag gcaaatggga 47640
agcaagcacg tcttaccagg gcagagcagc agagagagag agagagagtg agaggggaag 47700
tgccaccact tttaaaccat catatctcct gagagctcac tcattatcac aacaacagta 47760
tggggggaaat gtgcccccat gatccaatca cctcccatca ggtccctccc caaacattaa 47820
gaattaaaat tcaacgtgag atttgggtga ggacacagaa ccaaaccata tcaggcattt 47880
agaccattta cgttcaaggt taatttttat atgtgaggtt ttgatccctt gtttagttgt 47940
tagctgtttg ctttgttgtt tctattgtgt ttttgctttg tagggtgtgc aggctatgtg 48000
cttaagtgtg tttatgtgga agcaggtatg gttcttttgc ttccatgttt agaactccca 48060
tgtaaggatc tcttgtaagg atggtctagt ggtaacaaat tcctttagca cttgattgcc 48120
tggaaaagat tttatttgtc ctgcacttat gaagcatagt ttggcaggat gtgaaattct 48180
tggttgaaat ttcttttaag aacgctgaaa acaggccccc aatctctcct agcttgcaaa 48240

cctctgctga gaaatctgtt attatcctga tagagttccc tttttatgtg atctgcactt 48300
ttctctggct gcctttaaga ttttttctgt aatattgcct ttgaacattc tggtgactat 48360
ataccttggt gatttttttt tttttgagat ggagccttac tgtgtcacac aggctggagt 48420
gcagtggtga gatcttggct cactgcaacc tccgccctcc aagttccatg gattctcctg 48480
cctcaacctc ccgagtagct gggattacag gtgcctgcca ccgtgtctgg ctaatttttt 48540
gtatttttag tagagtcagg gtttcaccgt cttggccagg ctggtcttga actcctcacc 48600
tcatgatcca cctgcctcag cctcccaaag tgctgggatt acagacatga gtcaccacgc 48660
ccagccagtg atgttcatgt ttgtggtgtc tcacagatgt tctctggatt tctagctctc 48720
tagcaagatt aggaaagttt tcttgaatta ttccctcaac tatattttcc agtttgtttt 48780
ctttttctcc tactttctga ggaatgccaa taattcatag ggtttttttg ctttatataa 48840
tcttatattt ctcaaaaaca gctcatttaa aataattttt ttctttattt ttatcagatt 48900
gggttagttc acaagaccag ccttcaagct ttaaaatttt ttcttctgct tgatccagtc 48960
tattgataac gaatttcaaa atataattga aggctttaag tgagtttttc aattccagaa 49020
gctactattg attttttttta agatgttcat gtctttctta atttcctaga ttgctttaga 49080
agtttatgtt ggttttcaac cttgtctttg attacaatga acttccttgc aatccatgct 49140
ttgaattatt tatgtgtcat ttctgagttt ccatcttggt tagggaccat tgctggaaag 49200
ctagtgcaat cccttagtga tgtcactgca atcagatttt tcattatgcc agaattcttg 49260
cactggtttc ttctcatctg gagacattgg cacttcaaat ttttgtactt attttcatgt 49320
gggtagtagt ttttcttttt tttttctttc cctataatgg tattattatt attattttaa 49380
taattttttt tgtttccctt ttaccccttt tctagggctt gtgcctctaa agaatgctgg 49440
gtaggatctt ttcactttga ttctacagcc ctatgtgctt ctttcagcag gttttatact 49500
gggctctgca ggtcatccca caggcccata ggcggcacgt ataggtaaga gctggctgtg 49560
accaatgtgg ctggatatat acttgatcct tgtttccgag caaaagctct ccattgtctt 49620
aggcaatagg ctgattctga ttcatagaat gcacagtagt tggaactccc tgctcagccc 49680
caaggagaag ggaaccacaa agggcaagtt tggaccgagc aggtccacct gcgtcccctg 49740
atggcaagca caagcaccag tgccaaggaa gaatccagtg ggtggccacc aagcacccag 49800
agttgtacac agacctgaag cttggaaacc tcctcagctg caaattctct gcatgggagg 49860
catcctaagc tcctgattca ggagagtggg tgctccagat gcctggagat ctgctttggc 49920
atggaataga gaggggcccc ctgcaccaag atctctacac aggaggggta aatgacaatt 49980
atatatagtg ctgcaggaaa catgtgaatg cagataatat cttcaatata ccagttttct 50040
ttcttttggc tatatgccca acagtaggat tgctgcatca cgtggtagtt ctgtttttag 50100

ttttaggaac ctccatactg ttttccatag ttacagtact aatttagatt actaccaaca 50160

gtgtacgaga atttctcttt ctccatatcc tcaccagcat ttattttttg tctttttagt 50220

aacagccatt ttaactggag tgagataata tcttattgtg gttttatttg catttccctg 50280

atgattagtg atgttgagca ttttttcata tatctgttgg catttgtatg tcttctttca 50340

agaaatttct gttcattttg cctattttta aatgggatta cttttttttt ttgctattga 50400

gctgagttcc ttatatattc tgataattaa ctccttgtca gatgaatagt tttcaaatat 50460

tttctctcat tctttgtctc ttcacattgt taactatttc ctttgctgtg cggaagcttt 50520

ttagcttgat gaaattccat ttgtcaattt ttgccttgat tgcctatgcc ttgaggtctt 50580

actacaaaaa tatttaccca aaagtcttaa agcagttccc caaagttttc ttgtagtagt 50640

tttgtagttt cagatcttac acttaagtct ttaatcgatt ttgattttat tttcgtatgt 50700

ggtgagtgat agtgatctag ttctgttttc tgcatatagg tagccagttt tcccagcagc 50760

atttattgaa gagattgccc tttccccaat gtatatccat ggcacctttg tcaaaaatga 50820

gttgactgtt aatgcatggc tttatttctg ggttctctat tctgttccat tggtctttgt 50880

gtctgttttt ataccaatat catgctgtta tggttaagaa ttctcatttc tgacatagag 50940

accatttaaa taacaaaaga taaaacatga gagaatatta gaaagctttt aaaagattta 51000

ataaaatctt acagtagaat aatagaggta ataaatatgt tttgacatgt catgacatag 51060

attttgttag gagcagtttg ttcttatctg cttccgtggc tatgttttga ttataaaaat 51120

tatgttttga gttatttgat ttcttcgttg catatgattc ttgtctttaa tgaatcactt 51180

ccctaccttg aacaggtcac agtgaacttt tgctttagtt cactgctgta cagcactgag 51240

tgggaagata gttacaccct taaggatgat aatatatctt ctactttagg tgttctgctt 51300

ccatcttctt tcttctagaa aacattttg tttaatgact cacaaaaaaa tatctgaaat 51360

gaatacagct ttatattttg cagttgtaaa aatgctaaac tagtataagg taattagctc 51420

tttcaaaaat aagtcgcttt ctacataaaa gaatgattaa atgaatagtt atcttctttg 51480

aaatttttta aatccacagt tacaggtttt atgggttttt cttttttct attattttct 51540

tttttggtag caagccttat cttcacattt taaaaggttg tgttagaaag ccagtttctc 51600

tccttctccc cctacctcct cctccaccta gattcaacca cttcttatgg caaagagaat 51660

aactttctgg gctagagagt atgggttgtg tagtaaagat ttttgttgtt tttttgtttt 51720

ctcctcctac acttgacagc caacaaggct acaagtcttc tttggaagaa ctgcaaaatt 51780

cttgaattta ggacacagtg gtatcttttc tagaagtaca gagtcctgct ctgtgccttt 51840

aataccctaa gaacaaaaat cattttttcct ctttaaggca agatctactg tgggtgatgg 51900

ctaccaccta cctctctaga cctcacagaa tcttcttctt aatgggcatc tctttttcca 51960

cccaaatcct ggcctttgaa tcagcatctc tcagagacag cacctctcac attgctcccc 52020

aagttcactg cattggagcc tgcagccaac tgatgtcctt ctcatcagtc tggttgttta 52080
ccctaaacac catctcacac atgctagctg gggatggtcc tcagactatt cattctcatt 52140
tctttgctta aaattttct gtgtttgtat gggatgtctt ttatggtttg ccaactagaa 52200
cttttcagta gtatctacaa gcatttatta ttttctagac tcagtaaaaa gttagaattg 52260
aggggttcaa atatctcctc cattgtagtt gactttattt gaatgattaa gacatataca 52320
tttaaaaaaa tcaattctag tatagttttg gtttttggaa aatacctgat ttcactttat 52380
atttcctgtt gtctagcatc tttgttttct ttgaagtata tacaaaggca cactgtgttt 52440
tgatctggaa attggaggtg aatcctgaaa aatggaaggc aagcactatc agtgctataa 52500
aggattgtta aagtgtagtg ccagtggact gatcccagca ctgcgcagtg gtcattatca 52560
ttttggtaat ctgaagtaga agcatgcctc tagacatcct tccataattt tatataggta 52620
gtgtaaggat gactgtctat aaattcacct gaactacaga gagacttctg atataataaa 52680
agaaaaatac agagattgtt caggatggga atacaaacac taggaaactg gaaaagcaaa 52740
ttaaaacttc tgaattaata gaaaaagtta aataggaaag agaaaataaa gggaaacttg 52800
accaaggtag caaagaaaga aaatggaaaa gaggtaatta tgaggcagta taaaacaaac 52860
tctgtcgcaa aaaaaaaaaa aaaaaaaaaa aaaattccat ttgtagtttt aaaaaaatag 52920
taaagatata gaaaaattag tagaaaatac aaaaatctgt attcttcttt tatcagaatt 52980
aaataattat gcctgcccta ttttaccaaa ttattttatt aaaacatgct aaagaagttt 53040
ataagctttt cttctccctg ctcccattct ctataggatt tttgctgaga tatttcaaaa 53100
tttttatttt taggttatat ttaagctttc acttctaatc tctctcaaaa acctttcata 53160
tcacttgtca taaaaatttc ttctcatagt atcatcatcc agattcagtc acatatacat 53220
caagaataat tgtgcaatac tgtttcctag actcttcatc ttatcttttc aatgcatgat 53280
atttaaataa gccatacttt ttaatgtttt caattttttta atttatattt atttatttca 53340
aatttttat tatattttaa gttctgggat acacgtgcag aacgtgaagg tttgttacat 53400
aggtatacac gtgccatgat gttttgctgc acccatcaac ccatcatcta cattaggtat 53460
ttcttctaat gttatccctc ccttagcccc ccaccccccg acagatccct gtgtgtgaca 53520
ttcccctccc tgtgtccatg tgttctcatt gttcacctcc cacttatgag agaacatgca 53580
gtgtttggtt ttctgttctt gtgttagttt gctctgaatg atggtttcca gcttcatcca 53640
tgtccctgca aaggacatga actcatccct tttgatagct caatgttatt gcatggtgta 53700
tatgtgccac attttcttta tccagtctat cattgatggg catttgggtt ggctccaaat 53760
ctttactatt gtgaatagtg ctgcagtaaa catacgtgtg catgtgtctt tatggtacaa 53820
tgatttataa tcctttgggt acatacccag taacgggatt gctgggtcaa atggtatttc 53880

tggttctaga tccttgagga atcaccacac tgtcttctac aatggttgaa ctaatttaca 53940

ctgccaccaa cagcgtaaaa gctttcctat ttctccacat cttttccggc atctattgtt 54000

tcctgagttt ttaatggtca tcattctaac tggcatgaaa tggcatctca ttgtggtatt 54060

gatttgcatt tcgaccagtg atgatgagct ttttttcat gtttattgcc cacataaatg 54120

tcttcttttg agaagtgtct gttcatatcc tttgcccact ttttgatggg gttgtttttt 54180

gtttttttt ttgtaaattt gtttaaattc tttgtagctt cttgatagta gtcctttgtt 54240

agatggataa attgcagaaa ttttctccca ttctgtaggt tgcctgttca ctctgatgat 54300

agtttctttt gctgtgcaga aggtctttag tttaattaga tcccatttgt caattttgtc 54360

ttttgttgcc attgcttttg gtgttttagt catgaagtct ttgcccatgc ctatgtcctg 54420

aatgatattg cccaggtttt cctctagggt ttttatggtt ttcagtctta catttaagtc 54480

tttgatctat cttgagttaa tttttgtata aggtgtaagg aaggggtcca ctttcaattt 54540

tctgtatatg gctagccagt ttccccaaca ccatttatta aatagggaat cctttcccca 54600

ttgcttttt ggtcagattt gtcaaagatc agatggtaga tgtgtggcat tatttctgag 54660

gcctctgttc tgcttcattg gtctatatat ctgttttggt accagtacca tgctgttttg 54720

gctactgtag ccttgtagta tagtttgaag tcagctagcc tgatgcctcc agctttgttc 54780

tttttgctta ggattgtctt ggttatatgg gctctttttt ggttccatag gaaatttaaa 54840

gtagtttttt ctaattctat gaagaaagtc aatgttagct tgttggggat agcattgaat 54900

ctataaatta ctttggtcag tatggccatt ttcatgatat tgattcttct gatccatgag 54960

catagagtgt ttttccattt gtgtcctctc tgatttcctt gagcgttgat ttgtagttct 55020

ccttgaaggg gtccttcaca tttctcgtga cttgtattcc tagctatttt actccttttg 55080

tagcaattgt gaatgggagt tcagtcatgt ttgggctctc tgtttgtcta ttattggtgt 55140

ataggaatgc ctgtgatttt tgcacattta ttttgtatcc tgagacttca ctgaagttgc 55200

ttatcagctt aaggagattt tgggctgaga tgatggagtt ttctaaatat acaatcatgt 55260

cctctacaaa aagacacagt ttgacttcct ctcttcctat ttgaatacgc tttatttctt 55320

tctcttgcct gattgccctg gccagaactt ccactactat gtttaatagc agtggtggga 55380

aaggacatcc ctgtcttgtg ctggttttca aagggaatgc tttcagcttt tgcctattca 55440

ttatgatatt cgctgtgggt ttgccataaa tagctcttat tattttgaga tacggtccat 55500

caatacctag cttattgaga gtttttagca tgaaggggtg ttgaattttg ttaaaggcct 55560

tttctgcatc tattgagata atcatgtggt ttttgtcatt ggttctgttt atgtgatgga 55620

ttacgtttat taatgtgcgt atgttgatcc aggctttcat ctcagggatg aaactgacct 55680

gatcatggtg gataagcttt ttgatgtgct gctggatttg gattgccagt attttattga 55740

ggatttttgc atcaatattc atcagggata ttggcctgaa attttctttt ttgttgtttc 55800

tctgccgggt tttggtgtca ggatgatacc aaataaaatg agttagggag gattccctct 55860
ttttctattg tttggaatag ttttagaaag aatggtgcca gctcctcctt gtatctctgg 55920
tagaattcag ctatgaatct gtctggtcct ggggtttttt tggttggtag gctattaatt 55980
actgcctcaa tttcagaact tgttattggt ctattcagga attcaacttc ttcctggttt 56040
catcttggga gggtgtatgt gtccaggaat gtatgcattt cttctagatt ttctagttta 56100
ttttcataga ggtgtttata gtattctctg atggtagttt gtatttctgt gggatcactg 56160
gtgatatccc ctttatcatt ttttattgtg tctacttgat tcttctctcc cttctttatt 56220
agtctggcta gtggtctatc tactttgtta atcttttcaa aaaaccagct cctggattca 56280
ttgatttttt ttttttaagg gtttttcatg tctctatctc cttcggttct gctctggtct 56340
tagttatttc ttgtcctctg ctagcttttg aatttgtttg ctcttgcttc gctagttctt 56400
ttaattgtga tattagcgtg tcaattttag atcattccca ctttctcctg tggacattta 56460
gtgctataaa tttccctcta aacaaacact gctttagctg tgtcccagag attctggtat 56520
gttgtgtctt tgttctcatt ggtttcaaat aacttattta tttctgcctt aatttcatta 56580
tttatgcagt aatcattcag gagcaggttg ttcagtttcc atgtagttgt gcagttttga 56640
gtgagtttct taatcctgag ttctaatttg attgcactgt ggtctgagaa actgtttgtt 56700
attatttcca ttctttcaca tttgctgagg agtgttttac ttccaattat gtggtcaatt 56760
ttagaataag tgcaatgtgg tgctgagaag aaggtatatt ctgttgattt ggggtagaga 56820
gttctgtaga tgtctcttag gtccacttgg tccagagcct gagttcaagt cctggatatc 56880
cttattaatt ttctgtcttg ttgatctgtc taatattgac agtagggtgt taaaagtctc 56940
ccccattaat gtgtgggtgt ctaagtctct tcataggcct ctaagaactt gctttatgaa 57000
tctgaatgct cctgtattga gtacgtattt ttaggatagt cagctcttct tgttgcattg 57060
atccctttac cattatgtaa tggccttctt tgtcttttga tctttgttgg tttaaagtct 57120
gttttatcag agactaggat tgcaacccct gccttttttta gctttccatt tgcttggtaa 57180
atattcctcc atccctttat tttgagcata ttgtgtctct gcctgtgaga tgggtctcct 57240
gaatatagca caccaatggg tcttgactct ttatccagtt tgctggtctg tgtcttttaa 57300
ttggggcatt tagcccattt acatttaagg gtaatagtat tatgtgtgaa tttgatcctg 57360
tcattatgat gctagctggt tattttgccc attagttgat gcagtttctt cctagcatcg 57420
atggtcttta caatttggca tgtttttgca gtggctggta ccggttgttc ctttccatgt 57480
ttagtgcttc cttcaggagc tcttgtaagg caggcctcgt ggtgacaaaa tctctcagct 57540
tttgcttgtc tgtaaaggag tttatttctc ctttgcttat gaagcttagt ttggctggat 57600
atgaaattct gggttgaaaa ttcttttctt tagaaatgtt gaatattggc ctccactctc 57660

ttctggcatg tagggtttca gcagagagat ctgctgttag tctgatgggc ttccctttgt 57720

gggtaacccg actttctctt tggctgccct taacgttttt tccttcattt caatcttggt 57780

gaatctgaca gttatgtgtc ttggggttgc tcttctcgag gagtatcttt gtggtgttct 57840

ctgtatttcc tgaatttgaa tgttggcctg tcttgctagg ttggggaagt tctcctggat 57900

actatcccga agagtgtttt ccaacttggt tccattctcc ccgtcacttt caggtacacc 57960

aatcaaacgt aagtttggtc ttttcacata gtcccatatt tcttggaggc tttgtttgtt 58020

ccttttcatt ctttattctc taatcttgta ttcatgcttt atttcattca gttgatcttc 58080

aatctctgat atcctttctt ctgcttgata aatttggctg ctgatatttg tgtatgcttc 58140

acacagttct tgtgctgtgt ttttcagctc catcaggtca tttatgttct tctctaaaat 58200

ggttattcta ggtagcaatt cctctaacct tttttcaagg ttcttagctt ccttgcattg 58260

ggttagaaca tgctccatta actcagagga gtttgtcatt acccgcctcc tgaagcctac 58320

ttctgtcaat ttgtcaaact cattctccgt ccagttttat tcccttgctg gtgaggagtt 58380

gtgatctttt ggaggagaag aggcattctg atttttggaa ttttcagcct ttttgcactg 58440

gtttttcctc atcttcatgg atttatctac ctttggtctt tgatgttggt gaccttcaga 58500

tggggttttt gtgtggacat ccttttttgtt gatgttgatg ctattccttt ctgtttgtta 58560

gttttccttc taacagtcag gcccctctgc tgcaggtctg ctggagtttg ctggaggtcc 58620

actccagacc ctgtttgcct gggtatcact agcggactct gcagtacagc aaagattgct 58680

gcctgttcct tcctctggaa tcttcatccc agcagggcac ccaccagatt gccagccaga 58740

gctctcctgt gtgaggtgtc tgtcgacccc tgctgggagg tatctcccag tcaggaggca 58800

caggggtcag ggacccactt gaggaggcag tttgtccttt agcagagctt gagcactgcg 58860

ctgggagatc cgctggtctc ttcagagcca gcaggcagga acgtttaagt ctgctgaagc 58920

tgcgcccaca gccacccctt cccccaggtt ctctgtccca gggagatggg agttttatgt 58980

ataagcccct gactggggct gctgcctttc tttcagagat gccctgccca gagaggagtc 59040

tagagaggca gtctggctac agcagctttg ccatgctgca gtgggctctg cccagtttga 59100

agttccaggt ggttttgctt acactgtgag gggaaaactg cctactcaag cctcagtaat 59160

ggtggacgcc cctcccacca ccatgctcca gcgtcccagg tcgacttcac actgctgtgc 59220

tggcagcgag aatttcaagc cagtgaatct caacttactg ggctctgtgg ggatggtatc 59280

tgctgagcta gatcacttgg ctccctggct tcagcccccc ttccagggga gtgaatggtt 59340

ctgtctcact ggcattccag gtgccactgg ggtatgaaaa aaaactcctg cagctagttc 59400

attgtctgcc caaatggctg cccagttttg tgcttgaaac ccagggccct ggtgatttag 59460

gcacccaagg gaatctcctg gtctgtgggt tgcaaagacc atggggaaag catagtatct 59520

gggccggaac acattgttcc ttacagcaca gtcactcacg gcttcccttg gctaagggag 59580

ggagtttccc cactctttgt acttcccggg taaggcaatg ccccaccctg cttcagctcg 59640
ctctccatgg gctgtagcta ctgtctagcc agtcccaatg agatgaggtg ggtacctcag 59700
ttgaaaatgc agaaatcacc caccttctgt gttgatcgcg ttgggagcta cagaccggag 59760
ctattcctat tcagccatct tgccagccag caaccactct actttttttt tttttaattt 59820
aagttctggg atacatgtac agaatatgca ggtttgttac atagatatac atgtgccatg 59880
gtggtttgct gcacccaaca atccatcatc tacattagat gtttctacta gtgctatccc 59940
tcccctagct ccccacccct caacaggccc tggtgtgtga tgttcctctc cctgtgtcca 60000
tgtgttctca ttgttcacct cccacttatg agagaatatg cagtgtttgg ttttctgttc 60060
ttgtgttaat ttactaagaa tggtttccag cttcatccat gtccctgcaa aagacatgaa 60120
ctcatccctt ttgatggctg catagtattc catggtgtat atgtgccaca ttttctttat 60180
ccagtctctc actgatgagg atttgggttg gctccaagtc tttgctattg tgaatactgc 60240
tgcagtaaac atacatgtgc atgtgtctta tggtacaatg atttataatc ctttgggtat 60300
atacccagta atgggattac tgggtcaaat ggcatttctg gttctagatc tttgaggaat 60360
catcacactg tcttctacag tggttgaact aatttacact cccaccaaca gtgtaaaagc 60420
tttcctattt ctccacatcc tctccagcat ctgttgtttc ctgacttttt aatgatcgcc 60480
attctaactg gtgtgagatg gtatctcatt gtggttttga tttgcatttc tctcatgacc 60540
agtgaagatg agcttttttc catgtttttt ggccacataa atgtcttctt ttgagaagtg 60600
tctattcata cccttcaccc actttttgat gttttttttt tcttgtaaat ttgtttaagt 60660
tccctgtaga ctctggatat tagccctttg tcagatggat aaattgcaga gattttctcc 60720
cattctatag gttgcctgtt cactccgatg atagtttctt ttgctgtgca gaaggtcttt 60780
agtttaatta gatcccattt gtcaattttg tcttttgttg ccattgcttt tggtgtttta 60840
gtcatgaagt ctttgcccat gcctatgtcc tgaatggtat tgcctaggtt ttcttccaag 60900
gtttttatgg ttttaggtct tatgtttaag tctttagtcc atcttgagtt aatttttgta 60960
ttaggtgtag ggaaggggtc cactttcagt tttctgcata tggctagcca gtttccccaa 61020
caccatttat taaataggga atcctttccc cattgttttt tttggtcagg tttgtcaaag 61080
atcagatggt tgtagatttg tgacgttact tctgaggcct ctgttctgtt ctattgttct 61140
atatatctgt ttgggtacca gtaccatgct gttttagtta ctgtagagtt gaagtatagt 61200
ttgaagtcag gtagcatgat acctccagct ttgttctttt gacttaggat gtcttggcta 61260
tacaggctct tttttggttc catatgaaat ttaaagtaat ttttttctaa ttttgtgaag 61320
aaagtcaatg ttagcttgat ggagctatta ttggatctat aaattacttt ggtcactatg 61380
gccattttca aaatatacat tcttcctatc tatgaatatg gaatgttttt ccatttgttt 61440

gtgccctgat ttccttgagc agtgttttgt agttctcctt gaagaggccc ttcacatccc 61500
ttgtaagttg tattcctggg catttaattc tctttgtagc aattgtgaat gggagttcac 61560
tcatgatttg gctctctgtt tgtctattat tggtgtatag gaatgactgt gatttttgca 61620
cattgatttt gtatcctaag aataagccat actttttaat atattttatt cataaaaaaa 61680
tgcacataaa gcaaatgaca tatagaaaca atatgattaa catccacctc tggactcagt 61740
tggcttaaga aaatatgaat attatcacta cacttgaaat tgattgcata tttcttcctt 61800
atcacattcc tctttatctt tctccagaaa cctctatatc tttaatttgg agttggtcat 61860
tttcttatat tattctgtag gatttttaa tatctatgta tttctcacta atagattttt 61920
tagttttgag atgttttttt ctactccata gaaatgctat gtatatttat gcaatttgat 61980
cttactgcct aatattatat ttgtgttttt tattttgatt tgtattattc tggtttgttt 62040
tttcctacac tgtatgattt cattgtatga gtatctccca tttatttatt ctcatgtatt 62100
gacatgtact tccacttgat atttgctgtg ctttacctac agttcaattg tgaacattat 62160
tttacatgta ttctggtggt tcatgtgtga gagtttctgt aggtaacctg cacacctggg 62220
catgaaattt ctgactgtaa tacatgctca tgctgggctt tactagataa tgctagattg 62280
tttttaaagt agtagtagtt tacactccat ggcagctctt cattgttttg tgtgttcctg 62340
tattctgtgt ccttgccgtt ttaggtagat gatgtctcat gtggtcttac tttgcatttc 62400
tctggttaat aatgaggtca gtcatattgt tgtattttgt catctatata tttcttcctc 62460
tgtgaaatat ctgttcatgc cttttctttc tgatttatag gaattctttg tgtattttgg 62520
gtatatatgt tataaatatc tataggatag ataaaaacaa gctatctgat aaggcgatat 62580
gcagaaatat aaattaggtg aggaagcaag ccatgcaaat atccaaggaa aaaatcattt 62640
catgcagagg aacttgtcag tgtaagaccc caaggcagaa acacacttca agggcaagat 62700
gggcatcaat atgggtggag catgagggag aatagaagag agaccatcta tgcaagggta 62760
agttgggcct tgtagagctt gatatgaatt ttaacttcat tctaagtagg atcaggagtc 62820
actgaaaata tcaagcaaga atgacatagt ctgattaagt ctttaaagtt gtatagaaaa 62880
tagtctacaa aaagtgaaag ctgggaaacc agccagcata tgttacaata gtccaactgt 62940
gattgtgggg acataaatta tggtgcaagc agtggaaaca ggtatttgga tttgggttat 63000
attttcatgg ttatccacta ggatttgctg atgtattaga tatgaggtga gaaaataagt 63060
gaggtatcag taatgactct agagtttggg gctagagcag caggtgtgta aatgaagtta 63120
caaacctaga agatgcatgt ttggaccaat gcatcaaagt tctgctctaa acatattaat 63180
ttgaggggtc tgttagaaat acaagtggag atgctgagaa tacagttgca tagttgagtc 63240
tagagtttct tggaaaagct gggtctagga ctgtaaattt ggtaatcatt aatgtctaga 63300
tgatattaga gacatagaac tagatgcaga cacctagaaa gagcatagat atctaagaga 63360

agtctgagga ctgagcttgg tgtactcaaa gatttagaag ttgagaagat gaaaaagagc 63420
tagacagaga gactgaaaga gtgactacaa gaatgaaata tgtggaagcc aaatgaataa 63480
gagcgttaaa agaagaagga aacgatcaat gtgttaagta cttacccggg ttaagaccag 63540
gactgagaat tggccattgt tttttgacat ttagaaatct ttgaaggcct tgccaagaat 63600
aatttggttg ggataaggag aggaatgacc tgactggaga aggttcaagg gagaattgga 63660
ggagaggaaa tgaagagagt gatatttctc cacggaatac attatgtgtg agagggacta 63720
ttagaggatg cagacaagag ggcaaatgat ttcagagaaa agtccctgag tgggtgagaa 63780
ggactaagat ttggtattga aatgtcagga gtatgaattg taacaggagg gaaggcagag 63840
tacaggggta gccatactgt agattggcca caaagaaaat attctccaaa tggagcttct 63900
ttccccctaa gcctccccca aatcattacc tctgcttctg attcaatccc tcaaaacctc 63960
atccatcagc ttgaatgaga gacaactatt cagccaccat gtccatccgc tttcttatcc 64020
ccccttgctc acccccagcg tctcagccca aatcttccct ccttccagcc aacaaagctc 64080
ccatgcctgt catctcctga tgtcaatgca gcgggcaggg gagtggggga ccactgaccc 64140
cacatgccta ttctctgctt agattaccca aaataccagg tgtcgttatt tttcagaggt 64200
gcaaaaatat aatacaaagt cattctaaac tgtcactgaa ttcagagtac tttttgccat 64260
tgtctagtca ctgaattcag agtacttttt gccattgtct tattttctca atcaacaaat 64320
gctaagagta caattatatg aagttctaca gatttatttg ttacttgcat tacttagtaa 64380
tggttcttat gcataaaggt taggaataac ttctttgaac aaatatccta tataaaaaaa 64440
caacaaagtg gtttttaaaa atcacattgc tgttcacatt ccctttttag tactctcttc 64500
tcatattctt ttttaagcta tgttaggtca aaacaactac tttaataact tagcaaatat 64560
ctatcaggag tccactgtgt agttagcagt ttataaagct tcagatctac attagtgagt 64620
gaaatggacc tagcccctat ccttacaggg cagcaagcct ggtggaggaa gctatgttct 64680
ctgaaggcaa caaaaacagt catgcgtcat ttaagagtag ggatacattt tgagaaatgt 64740
gtcatgaagt agtcaatttc atcattgaac atcatagaat gcatttacgc agacttagat 64800
ggtatagcct actacacacc taggctgttt ggtgtagccc attgctcctg ggctacaaac 64860
cgatacagca tgttactctg ttgaattttg taggcaactg taatgcaatg gtaagtattt 64920
gtgtacctaa acatatctaa agatagagac agtacagtaa aaaatactat cttatgaaaa 64980
cactgttgta tatgtggtct gtcattgact gaaacaatat tatgtagtgc aaaccataca 65040
gcaacagata ataaagacaa gatattcaga gtgggagatg gaaaagacaa taatgtattc 65100
cttcattcat ttagcagaca tgtattgcca aaagacactg gaaaaaataa ataaaggtga 65160
caaacaggtg gtgcctttgt tgaagattat agtcatggac agggaaactt tagccctctt 65220

cttaacatta tcttccctta gacacaaaca cacacacaca caattcctgg catatgctat 65280

atactccata actgttcctt cccttccctt tgtgactcat tccttccccc agaaagctca 65340

tttgatctct gcaaataatt ttgaatcccc tttgtttttt aaccatggca attgttttgt 65400

tttcccatct cctacaagtt ttagctcaac tcgttggggc tgtttttttt tttttttttt 65460

tttttttttt tttttttttt ttgaggtgga gtttctccct tgtcacctgg gctggagtgc 65520

aatggcatga tcttgactca ctgcaacctc tgcctcctgg gttcaagtga ttttcctgcc 65580

tcagcctctg gagtaactgg gattacaggt gcccaccacc acacccagct aatttttgta 65640

tttttagtag agacggggtt tcaccatgtt ggccaggctg gtctcgaact tctgacctca 65700

ggtaatctgc ccacctcggc ctctcaaagt gctagaacta caggcatgag ctcaccatgc 65760

ctggccattc cttggggctt tatagaacaa gtataatcct ttttctttat gacaataccc 65820

tgagttcaga aatctcagag acaaatgttt acagagacca ggtaaataag tagctagagt 65880

gacaagactt aagaaaagag accattggcg taccaccagt ttgtggaggg aactggaaaa 65940

actggaatac acatgcccca tccaaaagca accattgcaa ctaaacttta acagattgtt 66000

gccacctaag taattcacgg atggtctcat aattctggtc agcattgtct gagccaaaca 66060

aaatgtatct atgggcatga tcagatacta gagccagcag attgcaacct ctgcttagat 66120

aattgcaggt atcagccttc ccttggctaa acagctactt catactgata agtagccctt 66180

gcctggcaca aagcaggtgg ggctgaatcc agcctgatat cacatcacca caactttctc 66240

taattctcct caaggcgtct gtgaactacc agcagcccaa ataagcatcc ttttctcctt 66300

caatcatttc tcataaagca gattctactc atctcaccat ccatcttttt cccctaacat 66360

atcagaattc atcaaagtag aaactccaga aatcatccag aaatacaaat gtgatctgac 66420

cagcacgggt ataaactttc tctttcctta atttagaaaa aggattatta attttgagtc 66480

ttagatctca taaaagtaga gctggattga ttttaaggga tcaatgaacc tactgaaatt 66540

gtataaaaag attttgtggg gctggcaaac aaatgctttt cttttaacat attcttaaaa 66600

gaacaaaacc tcaaagaaga tttaacaacc actatagact aagtttcaat taatgtagtc 66660

tgtagcatta gattttggaa ggctgttcta tcagaatatg gaccaatact gagcgtagtc 66720

gctactaaaa ctctttgtct ttttattcat atcttctcct aaaaagctcc atgacccctg 66780

cagaatactg gtataattac ccacacctca cccacccagt gattgttgat gtaactaatt 66840

atttaatcca agtctgaggc ctaaattttt tttaatcaag agttagttac atgttttaat 66900

ttattttttg taattttaat atctaacact tttattctat ttcattcgat tgatgctaaa 66960

atgcataatg ctatgattct aagacttagg aaaaataata ctatactggg atatggagtt 67020

tatatatatt tacatgaatt tctttttttt cttctctgta ggtcgaaaat ttaaaaagtt 67080

caataaagtc agagttgtga gagcactgga tgctgttgct ctcccacagc cagtgggcgt 67140

tccaaatgaa agccaagccc taagccagag attcactttt tcaccaggtc aagacataca 67200
gttgattcca ccactgatca acctgttaat gagcattgaa ccagatgtga tctatgcagg 67260
acatgacaac acaaaacctg acacctccag ttctttgctg acaagtctta atcaactagg 67320
cgagaggcaa cttctttcag tagtcaagtg gtctaaatca ttgccaggta ataataattt 67380
ttatatacag catgtaataa aaaatatact atgtttgttg tattagtaca ttgttaaata 67440
aaacactcta tgcagtaaat taactacaat ataaaacagt atgtgatagt atgtgtttga 67500
gcagttcaaa atatttgtct aggaccctaa tatgtgttac agaaatataa aaaattagag 67560
aacacactga gtcttactga tgagtcagaa attgcataca ttaaacttat gctaataaga 67620
aatagcttag gtttatgtgt tcaaccacag atattagagt agctcagaaa tgataaatgg 67680
accatttttt ataaatgccc tatttattct aacccttaat tcaaccaacc tcatttggtt 67740
gaaaataaaa ctgaatgtat aaagtcatat aacaaatcaa agtagtattc agagtacagt 67800
aaggtgtcaa aagtaaatta agaaaacaaa attccctaac gtaaattact ttaaaaaata 67860
atagcacaca gagaaaagaa tctctagtca ctattcctct aatttatacc caaagagagt 67920
atatcgccca gcaacagaga gaacttggat tatatcatca cagaagaatt tcagtcactt 67980
tacttatcag ctatttttgc tccctcagca cagctctgta ggagccaagt acttgaaaga 68040
ataacaagga tgaaattctc acttggttaa atcttagact gcagagccag gagaatacat 68100
gtatagcaat tgtttgtgaa ggcattactc ctataattag aaatagaact ttttaaaaaa 68160
ctacattctc ttcacatcag taataccctt atactgctat agacaaatta tttttctaat 68220
atctgatcaa gaattaaaca tattatgtcc ttttagtccc caagtaaatt caatggcact 68280
tttagaaaaa gaatttcttc ccaattctcc ttaatataat tctcagttat tattctacct 68340
aaaaggtttg aaatacaata gatgaattgt tactgaaaat tcaattacat gaaacagaaa 68400
gaaaaataga tacctgtttg ttgaatcttt caaaaaatac atattaagaa aagttaatag 68460
aattcagtat tctagtggag atgaagtaat tttatagaaa aatcaaaatg cccagattct 68520
tactggcttc agtctcacca aaaggcctta aacagacaat aattttttga acattctaaa 68580
tgtttaagat ttatcacata gtggttatca aacttgacag agctttaata aataaaaata 68640
acccaatagc attgatgaac tttttctgtt taaaaatgag tgtttaaaca tgtgtttaat 68700
ttttctccat tgttctatta tttggaaaat atgatttctt tggtttttgt tgatatagtt 68760
tatattcctt ttgtttgcaa ttttggaggt tctttactat ttaaaaagat tctagcatca 68820
gctggtaacg ttagaaaata accagttata cttgaaaaga tgacttattc actttggata 68880
aaagctaagt gaccttttta accggaaacc gttatctatc aggacttttt gacccccaca 68940
caaccacaaa aaggaatgag agaaaactct tacattgaat ttggtggcaa ctttttttaat 69000

gctgcaaaat gtaatttttg tgtagtaaat cagaataatg gcaacactag tattacccat 69060
actgtttcca ttaattgcaa acagggaaac attgagatta tcaaagtctc ttgtggcctg 69120
tggactttta aagccatatt ttcatggtct gcattttggg aatagcacta ttgaatgaat 69180
tatgaaaatg gtcagtgagc tagagtgact atttgacctc tgagaaagga cttggatgga 69240
cacatgtata gaaagtactt tagtattatt agctacttaa aactgatttc aattgacttg 69300
aaataagctt acagttttta ttcacttatt cactcaataa atgtctatta tgtacctgct 69360
gtgttcaata tctaataatt atacagtggt ttagaaagaa aagccacaag gctgggctca 69420
tggtgtggat gcattttggt acagcacatg ctagcatctg gtcatcagtt ttgcatgtaa 69480
aagaaattta tttcgttcat tcactcatgt gtaataaaac taataagtct gtggaagaca 69540
gacttttgtt aagtgatcct cactaattag ccagaatgtc aactaacttg cttatttaca 69600
gtgagacaca cacacacaca cgcacacaca cacaatataa atatacataa tacttatgtt 69660
gctaggagaa tatattaaca gatcagatca ttctttcaca ataggaaact gtatttggaa 69720
agtcacatta agatctgatg cagaatgacc atcataacaa aggtgacagc gtttatgaag 69780
acacctaggc atatatttgc aggtgcccta agaattctta ttgcctggta gggctttctc 69840
atttcttctt tgtctacact cttccattat cttggatctg ttataataat ggtagtttat 69900
atttcttcaa aaatatttca gatatatcct tttgtttctt ttcttttttt tctttttttt 69960
tttttttttt tgagagaggg tctcactcta ttgcccaggc tggaccacag tggcatgatc 70020
ttggctcatt gcagcctgga cctcttggtc tcaagttatc ctaccacctc agcctcctga 70080
tgagctggta gctgggacta caggcatgcg ccaccttgct tggctaaatt ttttttattt 70140
ttagttgaaa ttgggtttaa ctgttttacc caggctggtc ttgaactcct ggactcaagt 70200
gatctgcctg cctcagcctc ccaaagtgct ggaattacaa acatgactca ccgcaccccca 70260
cccctttac tttaatagag acttgcaaga tgctctagcc ttatgtcagc acgtttataa 70320
accatctctt aggatacatt gcaaatgagt gattttgcat gggcttttat ctgtagcctt 70380
agtgggcaat attttcttat ttgaaatgta gagcaacacg atatttgaat ttttttaatg 70440
aacaagacat attccctcta ttaaggaact ctcaatccag tagcagagag aagataccca 70500
aacaactata gtgttatgtg aaagtgatat aagatacaga tataatgaaa gtggggacag 70560
aagaacattt ttggcttcaa ggactaaaga agccttcaga gacaaaaatg tcagttaaac 70620
tgcatcttac aggagaaaca agactttgat aaatcatgat aagagaaagc attctagata 70680
gaaggaacag tatggctgaa ataagaaaag catgagatag gtttgctgaa taatgcatta 70740
aaccagcata ttaatggttc tcaacctttt caagtgtggg gggtccctta gtaacatcaa 70800
aaattttata gtctccccat agggtagtaa ttaaagcttt tagtagctat ggattgagaa 70860
aacacatttg tacatacagt ccctcggaca ctgctacagt aagtactggc cttcgggaga 70920

tttaatcacc caaatggtaa gaaaccattg aaaagaaatt tattttgaac tgaataatgg 70980
aaagtaaact tagaggtgta agttataggc tgatcaagga gggtcttaaa aattactgta 71040
gatgttagag tttaaattaa aagtactaat tcacaattcg gtgtcacctt taaattgtcc 71100
tcatcctgta catcaatacg ttgcagtgaa attgatgtga cagaggggt atgagggaaa 71160
cacttccgtt gcataaattt tgtcttgata tttcagttaa cttagtgaaa actccattta 71220
ggtcaatgct ttgaattttt aaaccttgca ttttttagga catactttat tctttgtgtc 71280
atttcaccag actacttgca attatcagta tcagaatatt agaacagtat tttgcctcat 71340
ttttctgctt ttgaagcgtg ccaaaacaac ttttgagtca gtgagtcatt gcatcttgac 71400
agaggatttt ttctcttcct tcccgaatca gaagactaaa ttaaattatt gctctccaga 71460
aaaaaatgat gagagagaaa tgagagtagc atactgagag taaaggcctt aataaatgtg 71520
ttgataaaga ctaaaccatg gtttttctca caccctattc cactgaaatt atgattgcca 71580
gtggcaacaa aaatttttac ttgtcaaatt agatgaatat ttccagccct taatgtgttt 71640
cacctacctc tgggactatt ggcaaagtaa ctatttcttg ctaagactct tgcttaggta 71700
tttgtcactt cccttccagg tagatatgtt tctctcccta tgtacatttc ccgactcctg 71760
tgtggatttg ttcttccacc cacactataa atttgtttgt gttctgggtt ctatcttttc 71820
cagtcatttc tcactctaca cagtctgcat aagctcattc atagtcttgt cttcaactac 71880
cactatattt cgatgactcc taaatacata atattagcca gaccctctac aaaactactg 71940
aactttcaac tgcttattac acagctccac ccagatgact tctaagtcag catgaccccc 72000
taaacctaag gccttttcca gtatcttgaa atggcacctc agcaagaatt tttggtatca 72060
tccttggctc agtttctcct tcacccctta cccaataaat tgtcaaattc tatcaactcc 72120
acttattaaa tatatgattc attatacatg ctacttctgt tgctacttcc ctgacttagt 72180
ttggtgctgg tcttttttta cttggattca tgttgcagcc tcctgactga ttctattacc 72240
atcaatttca cgtggtgccc aaatgatcct ctattctacc cccagaatca tttttctaaa 72300
acaattttaa tcaatcatat tttccatttc tttttccaaa tagcatccaa actccatggc 72360
ctcaatatcc aagggctttc tgtgatctgg ctcctgaatt tgcagtgtta atctctcatc 72420
attcctcttc acacgactct aagatctagt tccctaaatg ctgtttgctc aaattctagc 72480
cttcttctct tgcccatatt atttttccta cctggcagcc catctctttt cccacgtctt 72540
cctttgactg acctgtaact atctttcagg attcgcatta cttgtcactt gctctgggaa 72600
gatttctctg agccttccta gactgggtga ggaaaccctg taccctccct tgtctccttt 72660
gtgcttgtca accataacac tatgactctg agttacaatt gtgttctact tttctatctg 72720
tctcacaagc ctatagtgtt aaaggcagaa acagcatctt tctaaccttg atcctaattg 72780

caagtataat gcttcctccc aaatagatgg ttaaagaata tttatagaaa tttaaattac 72840

agaattgatt tgcaaagaac tgtactacct tagattatta gacattaact tcttattcag 72900

cataaatcat caaacatacg ttggtgatta tacttaatca tattgaagct tctttgtgct 72960

tctcaacatt gatacttgtg tatgaaagtt gttgctttgt gtcagaaggt gctgagatat 73020

ttaatgctct gccattgttg tagattatct gtttcaatta catgacattt tttacagaac 73080

caaaacctac atattttatc aaaactctgg gagtaagatt ggggaactta gtagtactgt 73140

catacaggcc atcttttttt tttttttttt tcttgagata ggctctcact ctgtcaccca 73200

ggctggagtg cagtggcatg attgtagctc acggcagctt agaactcctg ggctcaagcc 73260

atcgtcctgc ttcagcctcc ccagtaactt cagcctcccc agcactacag gtgtgcacca 73320

ccacacctgg acaagctttt taaattggtt tgtagagact gggtcttggc tggtctcaaa 73380

ttcctggcct caagtgactc tcctgcctcg cctcccaaag cactgggact acgggcatga 73440

gccaccacac ccagccccag gccatctctt cactatatac tttctgttaa ttctttaagc 73500

taaatttgga cagctgtaac agggcttttg tcagaaatta tcaggtcaag tcacttaaaa 73560

ggaaaagtgt tttttttttt aacatttgtt aaacacaatt agtgataatc catcacttct 73620

ccatgtgaag gtttttcatt ttggtcatct caaatgtact aagtttacct gatcattatc 73680

atcactggga atttaatctc tgtgttccca tctttgtttc agcataaagt aatgaatgat 73740

attacagtta ataaggaaaa aaatagcatt tctatactag gcatgttcaa gtcaatttaa 73800

cattgtacat ttatcacaga taagcactgt aaaaggtgga gtaaataaag gttattttct 73860

gagttctatt ccattctgtc aaacaaacag aaatatagtt tctgctataa atattggtaa 73920

ttttataaat ttaaagttat tgtaataaga tttgccttta atgttgtcat aaaataaaag 73980

taagcctgga tttctagtcc cagaatgcaa aaacaaagaa aaaagcaaaa agacgaaaac 74040

aaaaactaag agtaggataa agtgaatgta aacatgtaaa tgtagacagg atctgttttt 74100

ggataaagaa caatttaatt cttacataat tttgtaaaat gtttaaatgt ttaaaactac 74160

tcaaaggtta acataaaaca aatgcctgaa gtgaaaaatt acagcatagt taagtcaaat 74220

atttaaatat tataaaatac tttttttaga aagttataaa gttcaccaag aaagatatag 74280

agaataataa atgtatttgt ggagattatt gtgaacactt ggttaatgtt ttaatacatc 74340

tgattaatga aaacgaactt tctatgagcc tttttttttt tttttttttt tttttttttg 74400

agatggagtc tcgctgtgtc accaggctgg agtgcagtgg cacgttcttg gctcactgca 74460

acctcagcct cctgggttca aaccattctc ctgcctcatc ctcccaagta gctgatgaga 74520

ctacaggcat acaccaccac acctagctaa tttttgtatt tttagtagag acagggtttt 74580

accatgctgg ccaagatggt ctcaatctct tgaccttgtg atccacccac ctcggcctcc 74640

caaagtgctg ggattacagg cgtgagccac tgcgcccagc ccattttttt gtattatatg 74700

aaatatataa tttgttatat ataacaacac acataaaata tatattctgg aaaatatata 74760

ttccagaaca aacacataat ttatctttaa aatttttca ccattatttt ttatacttca 74820

gttctatctt atatgttcta taatatataa aatgcctcct aacactttt tttatggcat 74880

tgtgttttag tattagagac ttcccattaa actgactggt ggatttcata ctagtattac 74940

tccaatgagc aattctgttg cattgataga ccaataataa atatctccat agtgttctac 75000

agtgtgcata attatcccat ttgtctcata gcaactcact gagttagtta tcattgtgaa 75060

ttgtgatttt tacttatgaa gaaatttaga ttcaatgata ataagtgatg tttccaaagt 75120

ggctgttagt aagtggtaga gccaggactg gaacttgggt cttctgtcct gaatgccttc 75180

caatttgttg agacagatca gcaataggga ggtttatatg tgcctccctt tagaaaggct 75240

tccttttcaa aaattaagac aattcattga tctcttgtca aatgattcta aattttatca 75300

caaaattacc catttttata gtctcttaaa tatttgactt acaagcagca tttatgtatt 75360

agatttattg tattgagctt tataattttc ttaaaaagtt attatgtaga cctatacttg 75420

aagacacgtt caagtcatat gcaaattaaa aaatgataaa acttgcatac caaaaatttt 75480

tttggctgct tagttagaga gtctttctgc acctgagtaa accaggattc ttggatgcca 75540

gtattaaaaa taaactatgt taattataaa acacacaagc aacaacaaaa aaccctgact 75600

tttaaaaaca aaagaaaaag aaaaaaaata atttactgag aggacaacag gggttcactg 75660

aatagaagct agaggacaag cttagaatat gggcagaagc caaggcacat ctgaagggct 75720

aaaaagcaag catcacaaat cctcccattg gttctcatta tctggacacc accaacatca 75780

agaataaata gtcatatctt tctctttgct ttagtatttt ctgattcaaa ttcctgttag 75840

tatatgttcc atgggccaaa cctatgcccc aagcccaaag aaggaggagg tttacccagc 75900

tttctccgtg ggaaagtagg aaggtaaggc atttgctctc gcaaaaacta caaccaatgg 75960

ggaactctgc ccacacagag aaaggaagga ggggaggaag gaaggaggga aggaaagaag 76020

gaaagcagga aggtattagg gagcagtgag ggaggaggag gaaaaagagg aaaggaagaa 76080

agagagggaa ggaagaaagg agggagggag gaagggagtg agagaagaaa ggagggagaa 76140

ggggaagaaa gaaaggaggg aaaaggggag ggaagaaagg aggaagaagg ggagggaaga 76200

aaggagggag aaggggaggg aagaaaggga gaaggggagg gaagaaaggg agaaggggag 76260

ggaagaaagg gagaagggga gggaagaaag gagggagaag gggagggagg aaaggtggga 76320

aggagaaaag gttagacatt gaagagcatc ccttctcagt aaaaggtaaa tgctcactat 76380

ggcatctcta attatgaata tgcatttgag tttttggtct tattgaggct aaggagttct 76440

aagaagagag tagcaatttt tctactattc acatgaagat gggacgatct gaagtgtatg 76500

aagagaagag ccttcttttt tgctacctac cttgacatga ttaacattgt caggaaataa 76560

ctttcttttg ctcttgatag ctaatgaatt aagttttgct ttggttttac ttttgaaaca 76620
gattatttgt aagaacatca gtggattttt agagaaaata gatgttttta aaacagcttt 76680
atagaaagtg gttgatgttc tcagcaaaac attgtctcca tacctttaat tttaaaaatt 76740
ctccattttc atcatttcag ttatactcag atacaaataa ctttcaaatt tatattaata 76800
tttttagccc atatatctcc ccatacttct aattcatatg tgcagctgat accaggatgt 76860
tgacccctga atcctatcca aaactatttt ctccctagca agtttcttct cctttatttt 76920
atctcagcaa tgctgttatc atcatctacc cttcaagaca aatactgaaa gatgtatttg 76980
attccttctt cttcccatct accggcaatc agttgacaaa tgtcttttat ttttcccttt 77040
aaatattttt ctgatgcact ctctcctctt aattccaatg tgttgacttg acttacttca 77100
ggcttttatc atctctcacc tgggctttga cagtatctcc caactgtcct ccctaactcc 77160
agacttattc ccttgaatcc acctaccaca tagctaagaa tgatccatct acaatgcaag 77220
gctatgtcac tcccattgct ccttaataga cttgcaagac cttggatgat ctgacccagc 77280
ctacatctct aaacacatct ttgcttctcc cacctttaag ttactcattg ttgaactggt 77340
gatcttccct tgagaattgg gaagacatgg agagacttgt gatgattaag tcaagattca 77400
tttgcccttt ccctgccatc cttctgccac caacaccatg gaacagccta aaagaggatg 77460
aaagaccaaa gcattcacac catacccccc aatacacaca cacacacaca cacacacaca 77520
cacacacaca cacacacaca cacagattct aaatctctta agccacacgg aagtttaaaa 77580
caagactgga ttaagtttca gagaacgaaa gtgacctgat gactgtggaa tctacccgac 77640
atagttaagg gttgttaaaa gaaaattaga tataacacaa ctgaaagcag tggttttaca 77700
aagtaagacc agtttatgtt taaatcccaa agaactgaga tttaacgaac tgattatatt 77760
ccattcaggc attatctctc tttttttcta gaaagttttt cctgacctcc ctcccgattg 77820
actgatacac tcccttttaa ccttctctac cccagcactc atgtgcttgt ttctcctgtt 77880
gagttgtgag ctgcttttga ggctaggatt gactcttact catcttcatt tttatacagt 77940
gcctggcacc caaatggttc ttattaaatg ggtgttgcat gaatgagtga tggatgatgg 78000
attcacttag caaaatccca gaaatgctaa atcacagtga ctaatttcaa gtgacttcca 78060
aagttgctga tattttaggc tgtattattt attaatgttt tctgattttc agtgtaaaat 78120
atgttgggat tgaaaacatt ttattaccct acgcatacct attttcagat ttatatgtat 78180
tttcaacatt tttgagataa ttcagtcatt tttatggtca ctgtattttt aaatttgttt 78240
tgtaggtttt cgaaacttac atattgatga ccagataact ctcattcagt attcttggat 78300
gagcttaatg gtgtttggtc taggatggag atcctacaaa cacgtcagtg ggcagatgct 78360
gtattttgca cctgatctaa tactaaatga gtaagtagta acttttgttg tttttgttat 78420
tttaagtgta catgtaggat aattttgaaa gttatatttc aatagatgat cacatattta 78480

gtgttcttga tatgatgaca ttactgtcat tttacggtaa ttttatattt ggagcttttt 78540

cctcttgttt caaaatatag ttatatcaaa tccaattttc ttacacatca agaatgtatc 78600

aagaaatata tttaaagata aacttaaatg tttcattaat caatttaaaa tttcatagct 78660

tggtactaat gacaataata aatgagaaat atttttgtta ctgacttaca ttcaaaaagt 78720

aaaaacatct ttcttcatac atgacaaatt agactgctaa tttatttaaa aaattgaaag 78780

ataaatattt caatttgctt tatatttaaa taattttaat taattttcta cctacaacct 78840

ttaactgaat aagaatcttt tcatggtaat atttatattg ttctagtggt aaatgctgac 78900

ttagaagtat ataactcaca cttatataag tctttacagt ttttagataa ttttcatgtt 78960

tcattacatt ttatccaaca attccttgag ataagtaagg aatcagcatt cttcattgta 79020

cagtttagaa aactgagtct caggagaatt atataatctg ccaaataatt tgaatgacag 79080

tgagattcct tatctctttt tagccaaaat ctagttttca ttattcttgt tagcttcatt 79140

tgactaatgt ggacattata aaacaacaca aggttgggaa aatgccttaa ttaaattttc 79200

tcagccttca gcacttgtgt ttaactttgt caattaatta atgcttgcca atgtcctctt 79260

ttactctttt ctgtatcatt gtatatttcc ctagaaaagt ttaaaatatg gtaagctttg 79320

tgaactgact tctcctattt aattgcacac aaaattatat aatttttcat agaaaataaa 79380

ttgtaagtaa ataaaacatt tctgagcatt cttcagcttc acattctcaa ctcctacacc 79440

tctgctgtgt tttcttggtt taggtattta aagtgtgata ataaaagtga gaaacaatgt 79500

taatcttggt cactgtgtat ttaatataat tttctagttt taagatttct taacctcaaa 79560

aattcagtat tgtgaaaagt ttagaagatg tttaaaattc caaatattgg gtttgtttat 79620

taaaagtagt agtcattttc aataatatgt aaggtaatta ccaaaaccat attccctgat 79680

aaattaccta aactggctga gacactattt tttttcctgt tgctttttct tttgtgtatt 79740

gtgtgtgatg caagacagcg gatgaaagaa tcatcattct attcattatg ccttaccatg 79800

tggcagatcc cacaggagtt tgtcaagctt caagttagcc aagaagagtt cctctgtatg 79860

aaagtattgt tacttcttaa tacaagtgag tgagttcaag taacttaatg caagatatct 79920

agtttcttaa ttcattagaa aagttgcaaa caatatgatt atatagttat gtatgaggta 79980

gacgtcttgg attataagta taaagaagaa atacccaata tattgttata gacattaata 80040

aaattactgg attttttcat cttttaccta ccatataata ctaaaatagc ctcatcaatt 80100

tctttttatt ttagataaaa tgatttaatt actctttcat attcacctaa ttcagtaata 80160

taaactacag tcatgtgcca cataataata ttttggtcaa caacagacca catatacaat 80220

ggttccctaa gatgatagtg gagctgaaaa attatcatcg cctagtcctg ttatagccac 80280

cataacatca tagcacaatg cactaatcac atgttcgtgg tgatactaat gtaaacaaac 80340

ctactgtgct gccagtcata taaagtatag cacatacaat tatgtacagt acatagtact 80400
tggtaaagat aataaaaaac tcttactggt ttatgtattt actgtagtat acttttctat 80460
tgagagtcac tacttctact ttttttaagt taaccgcaaa acagcctcag gcaggtctgt 80520
cagagggatt ccagaaaagg gcatacgtca ccctgggaga tgacaactcc gtgtgtgtta 80580
ctgccccctg aagaccttcc agtgagataa gatgttgggg tggatgacag tgatattgat 80640
gattctgact ctgtgtaggt ctaggctaat gtgtgttttg ggtattagtt tttttaaaaa 80700
aagtttaagc aaaaaagaaa atatttaaaa atagataaag tttatagaat aaagatatag 80760
agaaagaaag ccaggcacgg tggcatgtgc ctgtagtccc cactacttgg gaggctgagg 80820
tgagaggatc acttgagccc aggagtttta agtccagcct aagcaacatg tgagaacttg 80880
tctctaaaaa aagaaagaaa gagaactgta ccttgtgttt ttgtttttaa ctaaatgtta 80940
ttacaaaaga gtcaaaaagt taaaaaacta gagtttataa agtaaaaaag ttacggtaag 81000
gtaaagctaa tttattataa aagaaataaa aatattttta taaatgtagt gtagcctaag 81060
tgtacagtgt ttataaaggc tatcgtagtg tatagtaatg tcctaggcct tcacattctg 81120
tcaccactca ctcgctgact cacgcagagc aacttccagt cctgcaagtt ccattcatgt 81180
taagtcccct atgcaagtgt cccattcgta tcttttatat tggattttta ctatgcctct 81240
tctgtgttta gatacaaaac acttaccatt gtgttacagc tgcctacagt attcagtaat 81300
cacatgctgt acaggttcat agcctaggaa taaggcctag gtgtatagta gtctatacca 81360
tctaggtttg tgtgagtaca ctctgtgata atcacacagt gatgaaatca cataaggaca 81420
catttctcag aatatatccc catcattaag tgatacacga ctgcaattaa tattggataa 81480
gcacaaacca tactctcctc taccaccact ctcaccccat ccccaaagta tttactatat 81540
aaaatacata attttgttta gaattatttt tatcaagata aattgttaaa acatgtaact 81600
ataaagatta gcatcttaat ataatagtgg tttctttatc actgacacag tccagtgagt 81660
tagcaagaaa cacccaggct ccttctagcc agtggcttca ccccttccta gtggcttgca 81720
aacctctatt ggacatgact agaaatgagc actgagtgag agagattttg aaggctagat 81780
ctgaaactat catacatcac tttcacccac attccaaggc acttaagctg caggaaaagc 81840
tgggaaatac agtctagcta taggcccaga agaaagagga aattatgttt tataaagcat 81900
tgcattgatc accagaagga caggcaaata tccgttccac tttttcttat atccaaggaa 81960
catacttacc tctgcctcag tggatcccca agggtctggg tgggcaaagt tttaaaaaaa 82020
agaaaaagca gctgctttga cccttgcata ggcaggatca gccctcaaat gtacaaactg 82080
atgcttttgt gattcattgt gatttctatg acaaccacag ggagctaaga cctcaggccg 82140
agggtgagcc taaggtcttt cttggcaaca tctttacagg gacttctttc taaggtccca 82200
aatctcaaat ggctaggggg tgcagatcag gagggaaggg caggagagat aaaccctgaa 82260

atggaaggct tcctcataga atttatgcaa agacagggat gaatacataa ccatcttggg 82320
aaccctattt ggacaaggaa tttcaaggct aatttggtga cagagcatca attgaacaaa 82380
ggatatttac gtggaaccgg cccagagagt caactggaat acatttaata atacagtaat 82440
tagctcctgc ccttctttgc atgaaggaat gaatcctata gcacttatgt cacagatagt 82500
cctagatctt ccaaactggc ttatatcctt aacataattc tctatttatg gataggcaac 82560
attatgctgg ggaattaaac aacaatggaa ttaatcaaca gggaggcttt gctcattgtt 82620
gtcacttaga aacttaggcg atcactagag cagtggtaag aaaaggaagt gagattcata 82680
caacttccac tcacatacca tagagagtaa gatttttagc catttgccac ttcacaaaaa 82740
ggaagaaaaa gaaactattt aatgaatagt acttacgctt tccacaaatt ctaatcaaga 82800
ttctaatgag atttggggag aagagggtga acttgacaaa cctactgaaa atccatcttt 82860
aaaactagaa gaataaatag ataagcatta taaaaatatg cagggaatta gaaagctgct 82920
gttaattaat acagcatggg agaagagata tactgaactt ttgggcaaat cagacaagta 82980
tatattagaa tataaatagt atagtggaaa ccacaaatca atgagtctga aacaaataaa 83040
tgttaagaaa aatagtttta tatttggaaa aaaactgttt ggatatgtag ctcaaattat 83100
atccttgaat aacagataat tgagttaaat gtaaacactc aaataattaa aaaacaagct 83160
agaagaaata gaaataatct tcaatctcaa ataccttttta taaaataaat gcaaggagaa 83220
gattggcaaa gtaaaacttt gataatttgg tagttatgaa cttaaacctc tcatttgcca 83280
ttaaatatga aaatataaat ctccaggaat agacagtagg ttgaacaaac atttttacat 83340
tgttctctcc aaaattacac taaaggccag atgtagtcgg tcacatttct aatcacactt 83400
tgggagggca agctgggcag atggcttgaa cccaggagtt tgagaccagc ctaggcaact 83460
tggaaaaccc cgtctctaaa aaaaaaaaaa aaaaaaaaaa aaaaaaaaaa aaaaaaaaaa 83520
attagccagg cgtagtggca agtgcctgta gtcccaggta gccgggaaac tgaggtggga 83580
ggatcacctc aacctaggaa gtcgaggctg cagtgagcca tgattgtgcc actgtactcc 83640
tgcctgggtg attgagtgag accatgtctc aaaataaaaa taaataaata aataaataaa 83700
aataaataaa ttacactaaa atcttagtaa agggatttac tatgaaaaat taagaaaaaa 83760
tgattttaca ttcatgttta cagctgaggg aaatataggg attggttggt aaccttaaag 83820
agagctgtga aagtttgaaa tgactgctat ttgaagtagt ccagaaaatt agtacagcct 83880
ctatggaaca ccatatggag atttctcaaa aaactataaa tataactacc attctatcta 83940
gcaatcccac cactggatat ctacccaaag gaaaagaaat cattatgtca aaaagacacc 84000
tgcactcttg tttattacag cagtattcac tatagcaaag ataggaaatc aatccaagtg 84060
tgtatcaaca gataatagga taaagaaaat gtgatgtact cacacatata catacataca 84120

cacacacaca cacacacaca cacacacaaa atggaatagt attcagcaat caaaaagaat 84180
gaaagtgtgt cttttgcagc aacatggatg gaacaggaag tcattatctt aagtgaaaca 84240
attcagagac aaagtcagat accacatgtt ctcgcttata agtgggagct aaataatgca 84300
tgtacatggg catagagtat agaataattg acagtggtgc cttagaaggc tggaaaggta 84360
ggaggggggt gagacattac ctaatggata caatgtgcat tattgggtga tggttatacc 84420
aaaagctcag atttcaccac tatgcaatat attcatgtaa caaaactgca cagatggtca 84480
ttaaatttat acaaatttaa taaaaagagg ttctagatat acatatagat gtacatacta 84540
gagttaagtt ttcaattgac aaaaatttgc atagatatac tgaattgcca gaattactct 84600
tcagagtctc ctttatatac aagatcttga aaactcaaaa ccaaagaatt aacttatttg 84660
tttttcataa gtttgtatct ctggccttct tttctagggg aaatggaaac caagttttga 84720
ttggggtgca ggggataaat acaggcttaa gaaatagtaa ttttcctccc tgttccctga 84780
gatacaacaa tattggaatt aggccaaggc tgggcatggt ggctcaagcc cgtaattcca 84840
gcactttggg aagctgaggt gggcgaatca cttgaggtga ggagttcaag accagcctgg 84900
ccaatatagt gaaaccccat ctctaccaaa aagacaaaaa ttagtcggac atgatggcac 84960
acacttgtaa tcctagctac tcaggaggct gaggcgggag aattgcttga acatgggagg 85020
cggaggttgc agtgacctga gattgtgcca ctgaactcca gcctgggcga cagagcaaga 85080
ccctgtgtca aaaaaaaaaa aaaaaaaaaa aaagcaatta ggccaactaa caatcttgga 85140
atggcctctc agtgttccag taaaagaaag agttgcaagt ctgaggtaga aatgattaag 85200
cttaattagg aaggcatgtc aaaagttgat ataggttgaa aactaggtct cctgagccaa 85260
gcaactagcc aagtttcaaa tacaaagtaa gagttcttga aggactttaa aagtgccatt 85320
ccagtgaaca catgaatgat aagcaaaacc gccttattgc tgatatggag aaagttttag 85380
tggtctgaat agaagatcaa accagccaca atattccctt aagcctaagc ctcatccaga 85440
ggaaggccct gattctcctc aatactagga aggcaaagag atgtaaggaa ggagcagaaa 85500
aaagtttgaa gctagcagaa gttggttcat gagatttaag aagccatttc cataacaaag 85560
tataaggtga agcagcaagt gctgatgcag aagctgcagc aagttaccca gaagatctag 85620
gtaagattat cgctacttat gaaagtagct gtgctaaaaa acaggttttc agggtagata 85680
aaacagcttt ccactggaag aagatgtcat ctaggacttt catagctaca gagaacaagt 85740
caatgtctgt cttcaaagct tcaaatgaca ggttgactct cttgttaggg gctaatgaag 85800
ctggtgactt gaagctgaag ctggtgactt gaagctgaag ctaatgctca tttatcattc 85860
tgaagatcct aaggccttta agaattacgc tcaatctgtt ctgcctgtgg tttatgaatg 85920
gaacaacaaa gctggatgac agcacatcta tttatggcat ggtttactga atattttgac 85980
ccactattga gacttactgc tcagaaaaga atactacttt caaatattat tgctcattga 86040

caatgcacct agtcagccaa gagctcttat ggatatatac aaggagatta atgttttcat 86100
ttctgctaac acaacattca ttctgtagtt catagatcaa agagtaattt tccactttca 86160
tgtcttatta tttaagaaat atattttgta aagctgtggc tgccatagat agtgtttcct 86220
ctgatggatc tgggtaaagt aaattgaaaa ccttctagaa aggattcacc tttctagatg 86280
ccattaagaa cattcgtgat tcatgggaga aggtgaaaat agcaacatta acaagagttt 86340
ggaagaagtt tatttcatcc ctcttgcatg actttgaggg tgttcaaaac ttcagtggag 86400
gaaataacta cagatatgtg aaaagagcaa gagaactaaa attagaagta aagcctgaag 86460
acatgactga attgaagagg tttcgtggta aaacttgaaa gatgaggagt tgtttcttat 86520
ggatgagcaa agaaagtggt ttcttgagtt ggaatctact tctagtgaag atgctatgaa 86580
gattattgaa gtgacaacaa aggatttcgg agattacata aacttagttg ataatgcagc 86640
agcaggactt aagaggattg actccaatta tgaaagaagt tctattgtgg gtaaattgcc 86700
atcaaactgc tagcattgca tgctacagaa aaattattca caaaagagtc aactgatgca 86760
gcaaacttca ttgttgcctt attttgagaa actgtcatag ccaccccaac cttcagcaac 86820
caccaccctg gccaatcagc agccatcaac atttagacaa caccctccaa cagcaaaaag 86880
attttgactc agggaagact cagatgattg ttaccatttt ctagcaataa agtgtttttt 86940
agttaaggtg tgttcacttt ttaaataatg ctgttgcaca cttaatacat tctagtatag 87000
tgtaaatata acttctacgt ggactgggaa acaaaaagat gcatgtgatt cactttgttg 87060
caatatttcc ttcattgccg tggtctggaa ccaaaccggc tatatctcca agatatgcct 87120
gtgtaacaaa gtggctggat ataagataaa tatttaaata ttaatacctt tctcccatat 87180
cagcaggaag gcattagaaa acttaatgaa gcaattttta aaattacaac aaaaagataa 87240
atcatctagg aataatctta atggcaaata cttgagattt atataaagaa gacaaaagtt 87300
tactaagaga tccaagtatt cattaaaaag aagggaaaaa aaagatccag attacatgca 87360
gctgtaaact aagtcaaacc aagtatttaa gaagcatgta gaataaggcc aggttctctt 87420
ttgagcaaat gctaagttaa atgcacaatc cattgataca ggtctaaaat aggtcagctg 87480
aagtgttctt taatatacct attaattctt atgttcttcc agtgaactag aagtttatat 87540
gtcctgatat tgatagtgat atcaataaca ctatctcttt cctatataag gtgttttttt 87600
tctagagtca tgtatcagaa gaaatcatta tatagctcag aaatatatgt tataaattgt 87660
taatgtcatc tttttataca caactgccac ttttaattgt cttcttaaca gttcctttgg 87720
aagggctacg aagtcaaacc cagtttgagg agatgaggtc aagctacatt agagagctca 87780
tcaaggcaat tggtttgagg caaaaaggag ttgtgtcgag ctcacagcgt ttctatcaac 87840
ttacaaaact tcttgataac ttgcatgatg taagtatttg gttgattcca gaatatcaat 87900

gattattctc tgaatttcta taacttttta aatgttacat gtaaatttta ctttgtatga 87960
ttttctcaga ttaatacttg tatgttaaaa gtgtttggat catgctgctc agacttttta 88020
ttgtgttttt ttttcatttt caatatagac cactcaaata ttcttttaca agtatttgta 88080
taatgtgagg gtaatttaat agctgataga aattatggta catttcctga atatgttcta 88140
aaatatttgt gcctaaaatt ataaaagatt ttatatatac agtaaaaaca aaggtatgta 88200
tgtgaaaaat aagactcatg ccattttcag tggaattata aataacattg ctgatgggtg 88260
ggcaatcagg caatacatag caaaagttaa aatttgcatg cttttcaagt ttctattttt 88320
taggatttat cttaaagaaa taattagaca aatgtaatat acatgcaagg atgttcacca 88380
tagcagaggg ggaaaaaaga aaagcaaaca agaaatgccc aacaccagag gtggattact 88440
aagacaagat atctgtggtt tgtcaagata tatgaaatac aagcttttat tgctactatg 88500
ttcagatcac attaaaatga cagtgaaggc ataaaaaagc tatccactct ccaggtcaac 88560
ttttgaggtc ttactcctga atacgaacag acagccaaga ttatgagcta tttgagtaaa 88620
gcctctaaaa tgaaaggcag aaagcaaaat aaaaagaaac ttgaaggaaa taaacaccag 88680
tgcagagaac agaagaaaac ttctaacatc ctcagagaaa taagaatgat acggtatcca 88740
tgacatgaga acagaaaaca tttttaaaac agacatttag caagaaaata cttttggata 88800
attaaatgaa gaaaggcaga aattgaaaat tcaatgaaag ggctgaaatg gaaaggaaaa 88860
tctgaggaaa aataaaagat caaaacatgg aaaattagaa agaagagtta tagtaaattt 88920
agtattcagg agattctagc ctccaattaa taggaatgtc cagaagaaaa aatagagaaa 88980
atcaaggaga gaaatttgtg taagaaatcg caaaagttgc tggaactgaa acatacaagt 89040
tttctaacaa aaagggtccg gtgtagaggc actgcacaat tgaactaagg tacattattg 89100
taaaattgta ccaggccaga aatgaagaga atattttaaa gtataccagt gatgaaagaa 89160
acaggtcaca tagacgttat caggattcag aatgatgttg gacttctctg cagcaacact 89220
ggaagccaga agacagtgga gcagtgcctt cagaatttga tggaaaagaa aatcttcaaa 89280
gaatttcata cctagccaaa ccatcaaaca agtacaatgg cagcatgaaa gtattttcca 89340
acttgcaagg tctcctagag tttacctcag atacaacttt ctcaagaact gctacaggat 89400
gtcttataac aaaacatggg attaagccaa agatccaccc caacagagat gcaaagtgat 89460
gtcccagaac aataactgtg cagagcagta gttagtctag atgggagcag taatacaaag 89520
ggttctagga taccagctcc aggaaaaata aatggaactg agagattacc actaagtatg 89580
actgtaattg aggtgatttt tacaattctg ttgcagaatt tgactatggc tttgagacaa 89640
atatagagaa aacaaagcaa acaagaacac gaggtgattc acttttgcaa aataaaaata 89700
agtcgtaaga atggagatgc aatccagcag aaaaccataa aaaaatcttt atgatataaa 89760
tgctggatat tgactttagc taaaaattga gccaaaagct ctctctgggg agaagagaac 89820

atgtatgtag ttgaggaaga gagtgtctag tataagagag cttcattttc ttcatttcct 89880

atagtagaaa ctaaaactaa ctttagtgga aatatagaaa tatcaaaaga cacaggctac 89940

aaaaagttga aggtagttgt gtctagggag ctggaatagg gatgggaaga agtctatcag 90000

gaaactgcta ggctctattg atgctgctgc tgctgctgtt tttgttttta taatcatgtg 90060

gtactacttg actttcaaca ttatacacat gaattacttt gataaaaatt aaacgatgtt 90120

ggattattgg cataggaaga accaatatgt tgttatataa aacagctaga ttagcaaata 90180

gaatttatga aacatgtgta atattgtgtg tatgcttggg tatgtctgta tacatgtata 90240

aaaagaaaaa aaaacttgag taatacacca aaatgttcac acagcttatc ctgtagaatt 90300

gtgaataact ttcatttctt taaataaatc tttccataat atcagaatgt tctatcatga 90360

gtatataaca ttttacatat tattctagaa aatggtggga gttattttca ctatggtaag 90420

aaaaaaacac cttgagaagt atttatatta taaatagtta gaaaaataaa ttgccatgtt 90480

tgaatagcat atgaatttat tatttttatt acatgttttc tactcatttg ttaaaccaac 90540

agcttgtcaa acaacttcat ctgtactgct tgaatacatt tatccagtcc cgggcactga 90600

gtgttgaatt tccagaaatg atgtctgaag ttattgctgc acaattaccc aagatattgg 90660

cagggatggt gaaacccctt ctctttcata aaaagtgaat gtcatctttt tcttttaaag 90720

aattaaattt tgtggtatgt ctttttgttt tggtcaggat tatgaggtct tgagttttta 90780

taatgttctt ctgaaagcct tacatttata acatcatagt gtgtaaattt aaaagaaaaa 90840

ttgtgaggtt ctaattattt tcttttataa agtataatta gaatgtttaa ctgttttgtt 90900

tacccatatt ttcttgaaga atttacaaga ttgaaaaagt actaaaattg ttaaagtaaa 90960

ctatcttatc catattattt cataccatgt aggtgaggat ttttaacttt tgcatctaac 91020

aaatcatcga cttaagagaa aaaatcttac atgtaataac acaaagctat tatatgttat 91080

ttctaggtaa ctccctttgt gtcaattata tttccaaaaa tgaaccttta aaatggtatg 91140

caaaattttg tctatatata tttgtgtgag gaggaaattc ataactttcc tcagattttc 91200

aaaagtattt ttaatgcaaa aaatgtagaa agagtttaaa accactaaaa tagattgatg 91260

ttcttcaaac taggcaaaac aactcatatg ttaagaccat tttccagatt ggaaacacaa 91320

atctcttagg aagttaataa gtagattcat atcattatgc aaatagtatt gtgggttttg 91380

taggttttta aaataacctt ttttggggag agaattgtcc tctaatgagg tattgcgagt 91440

ggacataaga aatcagaaga ttatggccta actgtactcc ttaccaactg tggcatgctg 91500

aaagttagtc actcttactg attctcaatt ctctcacctt tgaaagtagt aaaatatctt 91560

tcctgccaat tgctcctttg ggtcagagct tattaacatc ttttcaaatc aaaggaaaga 91620

agaaagggag aggaggagga gggaggtatc aattcacata cctttctcct ctttatcctc 91680

cactatcatg aattcatatt atgtttcagc catgcaaatc tttttaccat gaaatttctt 91740

ccagaatttt cccccttga cacaaattcc atgcatgttt caaccttcga gactcagcca 91800

aatgtcattt ctgtaaaatc ttccctgagt cttccaagca gtaatttgcc ttctcctaga 91860

gtttacctgc cattttgtgc acatttgagt tacagtagca tgttatttta caattgtgac 91920

tctcctggga gtctgggagc catataaagt ggtcaatagt gtttgctgac tgagagttga 91980

atgacatttt ctctctgtct tggtattact gtagatttcg atcattcttt ggttacattt 92040

ctgcatattt ctgtacccat gactttatca ctttcttctc ccatgcttta tctccatcaa 92100

ttatcttcat tacttttaaa ttttccacct ttgcttccta ctttgtgaga tctctccctt 92160

tactgactat aacatagaag aatagaagtg tattttatgt gtcttaagga caatacttta 92220

gattccttgt tctaagtttt taaactgaat gaatggaata ttatttctct ccctaagcaa 92280

aattccacaa aacaattatt tcttatgttt atgtagcctt aaattgtttt gtactgtaaa 92340

cctcagcata aaaactttct tcatttctaa tttcattcaa caaatattga ttgaatacct 92400

ggtattagca caagaaaaat gtgctaataa gccttatgag aatttggagc tgaagaaaga 92460

catataactc aggaaagtta cagtccagta gtaggtataa attacagtgc ctgataaata 92520

ggcattttaa tatttgtaca ctcaacgtat actaggtagg tgcaaaacat ttacatataa 92580

ttttactgat acccatgcag cacaaaggta ctaactttaa atattaaata acacctttat 92640

gtgtcagtaa ttcatttgca ttaaatctta ttgaaaaggc tttcaatata ttttccccac 92700

aaatgtcatc ccaagaaaaa agtattttta acatctccca aatataatag ttacaggaaa 92760

tctacctctg tgagagtgac acctctcaga atgaactgtg tgacacaaga aaatgaatgt 92820

aggtctatcc aaaaaaaacc ccaagaaaca aaaacaatat tattagccct ttatgcttaa 92880

gtgatggact cagggaacag ttgatgttgt gatcatttta ttatctgatt cttgttactt 92940

tgaattaaac caatattttg atgatataaa tcatttccac cagcatatat ttaatttcca 93000

taataacttt aaaattttct aatttcactc aactatgagg gaatagaatg tggtggccac 93060

aggtttggct tttgttaaaa tgtttgatat cttcgatgtt gatctctgtc tgcaatgtag 93120

atgtctaaac actaggattt aatatttaag gctaagcttt aaaaataaag taccttttta 93180

aaaagaatat ggcttcacca aatggaaaat acctaatttc taaatctttt tctctacaaa 93240

gtcctatcta ctaatgtctc cattactatt tagtcatcat aaccattatc ttcattttac 93300

atgtcgtgtt ctttctggta gctctaaaat gacactaaat cataagaaga caggttacat 93360

atcaggaaat acttgaaggt tactgaaata gattcttgag ttaatgaaaa tattttctgt 93420

aaaaaggttt gaaaagccat ttgagtctaa agcattatac ctccattatc agtagttatg 93480

tgacaattgt gtgtgtgttt aatgtttaaa gatgtggcac tttttaataa ggcaatgcta 93540

tgctattttt tcccatttaa cattaagata atttattgct atacagatga tatggaaata 93600

tgatgaacaa tatttttttt gccaaaacta tgccttgtaa gtagccatgg aatgtcaacc 93660
tgtaacttaa attatccaca gatagtcatg tgtttgatga tgggcactgt ggagataact 93720
gacataggac tgtgcccccc ttctctgcca cttactagct ggatgagatt aagcaagtca 93780
tttaactgct ctgattaaac ctgcctttcc caagtgcttt gtaatgaata gaaatggaaa 93840
ccaaaaaaaa cgtatacagg ccttcagaaa tagtaattgc tactattttg ttttcattaa 93900
gccatagttc tggctataat tttatcaaac tcaccagcta tattctacag tgaaagcagg 93960
attctagaaa gtctcactgt tttatttatg tcaccatgtg ctatgatata tttggttgaa 94020
ttcatttgaa attagggctg gaagtattca agtaatttct tctgctgaaa aaatacagtg 94080
ttttgagttt agggcctgtt ttatcaaagt tctaaagagc ctatcactct tccattgtag 94140
acattttaaa ataatgacac tgattttaac atttttaagt gtctttttag aacagagagc 94200
ctgactagaa cacagcccct ccaaaaaccc atgctcaaat tatttttact atggcagcaa 94260
ttccacaaaa gggaacaatg ggtttagaaa ttacaatgaa gtcatcaacc caaaaaacat 94320
ccctatccct aagaaggtta tgatataaaa tgcccacaag aaatctatgt ctgctttaat 94380
ctgtctttta ttgctttgga aggatggcta ttacattttt agtttttgct gtgaatacct 94440
gagcagtttc tctcatccat acttatcctt cacacatcag aagtcaggat agaatatgaa 94500
tcattttaaa aacttttaca actccagagc catgtgcata agaagcattc aaaacttgcc 94560
aaaacataca ttttttttca aatttaaaga tactctattt ttgtattcaa tagctcaaca 94620
actgtggtcc ccactgataa agtgaagtgg acaaggagac aagtaatggc ataagtttgt 94680
ttttcccaaa gtatgcctgt tcaatagcca ttggatgtgg gaaatttcta catctcttaa 94740
aattttacag aaaatacata gccagatagt ctagcaaaag ttcaccaagt cctaaattgc 94800
ttatccttac ttcactaagt catgaaatca ttttaatgaa aagaacatca cctaggtttt 94860
gtggtttctt tttttcttat tcatggctga gtgaaaacaa caatctctgt ttctccctag 94920
catctgtgga ctatttaatg taccattatt ccacactcta tggtccttac taaatacaaa 94980
attgaacaaa aagcagtaaa acaactgact cttcacccat attataaaat ataatccaag 95040
ccagattagt caacatccat aagatgaatc caagctgaac tgggcctaga ttattgagtt 95100
caggttggat cacatcccta tttattaata aacttaggaa agaaggcctt acagaccatc 95160
agttagctgg agctaataga acctacactt ctaaagttcg gcctagaatc aatgtggcct 95220
taaaagctga aaagaagcag gaaagaacag ttttcttcaa taatttgtcc accctgtcac 95280
tggagaaaat ttaagaattt gggggtgttg gtagtaagtt aaacacagca gctgttcatg 95340
gcagaaatta ttcaatacat accttctctg aatatcctat aaccaaagca aagaaaaaca 95400
ccaaggggtt tgttctcctc cttggagttg acctcattcc aaggcagagc tcaggtcaca 95460

ggcacagggg ctgcgcccaa gcttgtccgc agccttatgc agctgtggag tctggaagac 95520
tgttgcagga ctgctggcct agtcccagaa tgtcagcctc attttcgatt tactggctct 95580
tgttgctgta tgtcatgctg accttattgt taaacacagg tttgtttgct ttttttccac 95640
tcatggagac atgggagagg cattattttt aagctggttg aaagctttaa ccgataaagc 95700
atttttagag aaatgtgaat caggcagcta agaaagcata ctctgtccat tacggtaaag 95760
aaaatgcaca gattattaac tctgcagtgt ggcattagtg tcctggtcaa tattcggata 95820
gatatgaata aaatatttaa atggtattgt aaatagtttt caggacatat gctatagctt 95880
atttttatta tcttttgaaa ttgctcttaa tacatcaaat cctgatgtat tcaatttatc 95940
agatataaat tattctaaat gaagcccagt taaatgtttt tgtcttgtca gttatatgtt 96000
aagtttctga tctctttgtc tatgacgttt actaatctgc atttttactg ttatgaatta 96060
ttttagacag cagtggtttc aagcttttttg ccactaaaaa taccttttat tttctcctcc 96120
cccagaaaag tctatacctt gaagtatcta tccaccaaac tgtacttcta ttaagaaata 96180
gttattgtgt tttcttaatg ttttgttatt caaagacata tcaatgaaag ctgctgagca 96240
gcatgaataa caattatatc cacacagatt tgatatattt tgtgcagcct taacttgata 96300
gtataaaatg tcattgcttt ttaaataata gttagtcaat ggacttctat catagctttc 96360
ctaaactagg ttaagatcca gagctttggg gtcataatat attacataca attaagttat 96420
ctttttctaa gggctttaaa attcatgaga ataaccaaaa aaggtatgtg gagagttaat 96480
acaaacatac catattcttg ttgaaacaga gatgtggctc tgcttgttct ccataaggta 96540
gaaatacttt ccagaatttg cctaaactag taagccctga atttgctatg attagggata 96600
ggaagagatt ttcacatggc agactttaga attcttcact ttagccagta aagtatctcc 96660
ttttgatctt agtattctgt gtattttaac ttttctgagt tgtgcatgtt tataagaaaa 96720
atcagcacaa agggtttaag ttaaagcctt tttactgaaa tttgaaagaa acagaagaaa 96780
atatcaaagt tctttgtatt ttgagaggat taaatatgat ttacaaaagt tacatggagg 96840
gctctctaaa acattaaatt aattattttt tgttgaaaag tcttacttta ggcatcattt 96900
tattcctcag caactagctg tgaagccttt actgtgctgt atgccagtca ctctgctaga 96960
ttgtggagat taccagtgtt cccgtcttct ccgagcttag agttggatgg ggaataaaga 97020
caggtaaaca gatagctaca atattgtact gtgaatgctt atgctggagg aagtacaggg 97080
aactattgga gcacctaaga ggagcaccta ccttgaattt aggggttagc agaggcatcc 97140
tgaaaaaagt caaagctaag ccacaatcta taagcagttt aggaattagc agaacgtgcg 97200
tggtgaggag atgccaaagg caagaagaga agagtattcc aaacaggagg gattccaaag 97260
agagaagagt atcccaaaca acatttgcac aaacctgatg gggagagaga atgtggggtg 97320
gggatggatg atgagactga agaagaaagc caggtctaga taatcagtgg ccttgtacac 97380

catgttaaag agtgtagact tgattctgtt gtaaacagga aagcagcaca attcatatga 97440
atattttaga agactcccac tggaatatgg agaataaagt tggagatgac taatcctgga 97500
agcagggaga acatttttga ggaagttgca ctattttggt gaaaatgatg atcataaaca 97560
tgaagaattg taggtgatca tgacctcctc tctaattttc cagaagggtt ttggaagata 97620
taacatagga acattgacag gactgacgaa aggagatgaa atacaccata taaattgtca 97680
aacacaaggc cagatgtcta attattttgc ttatgtgttg aaattacaaa tttttcatca 97740
ggaaaccaaa aactacaaaa cttagttttc ccaagtccca gaattctatc tgtccaaaca 97800
atctgtacca ctccacctat atccctacct ttgcatgtct gtccaacctc aaagtccagg 97860
tctatacaca cgggtaagac tagagcagtt caagtttcag aaaatgagaa agaggaactg 97920
agttgtgctg aacccataca aaataaacac attctttgta tagattcttg gaacctcgag 97980
aggaattcac ctaactcata ggtatttgat ggtatgaatc catggctggg ctcggctttt 98040
aaaaagcctt atctgggatt ccttctatgg aaccaagttc catcaaagcc catttaaaag 98100
cctacattaa aaacaaaatt cttgctgcat tgtatacaaa taatgatgtc atgatcaaat 98160
aatcagatgc cattatcaag tggaattaca aaatggtata cccactccaa aaaaaaaaaa 98220
aaagctaaat tctcagtaga acattgtgac ttcatgagcc ctccacagcc ttggagctga 98280
ggagggagca ctggtgagca gtaggttgaa gagaaaactt ggcgcttaat aatctatcca 98340
tgtttttttca tctaaaagag ccttcttttt ggattacctt attcaatttc catcaaggaa 98400
attgttagtt ccactaacca gacagcagct gggaaggcag aagcttactg tatgtacatg 98460
gtagctgtgg gaaggaggtt tctttctcca ggtcctcact ggccatacac cagtcccttg 98520
ttagttatgc ctggtcatag acccccgttg ctatcatctc atatttaagt ctttggcttg 98580
tgaatttatc tattctttca gcttcagcac tgcagagtgc tgggactttg ctaacttcca 98640
tttcttgctg gcttagcaca ttcctcatag gcccagctct tttctcatct ggccctgctg 98700
tggagtcacc ttgccccttc aggagagcca tggcttacca ctgcctgcta agcctccact 98760
cagctgccac cacactaaat ccaagcttct ctaagatgtt gcagacttta caggcaagca 98820
taaaaggctt gatcttcctg gacttccctt tacttgtctg aatctcacct ccttcaactt 98880
tcagtctcag aatgtaggca tttgtcctct ttgccctaca tcttccttct tctgaatcat 98940
gaaagcctct cacttcctct tgctatgtgc tggaggcttc tgtcaggttt tagaatgagt 99000
tctcatctag tcctagtagc ttttgatgct taagtccacc ttttaaggat acctttgaga 99060
tttagaccat gtttttcgct tgagaaagcc ctaatctcca gacttgcctt tctgtggatt 99120
tcaaagacca actgaggaag tcaaaagctg aatgttgact ttctttgaac atttccgcta 99180
taacaattcc aattctcctc agagcaatat gcctgcctcc aactgaccag gagaaaggtc 99240

cagtgccaaa gagaaaaaca caaagattaa ttatttcagt tgagcacata ctttcaaagt 99300

ggtttgggta ttcatatgag gttttctgtc aagagggtga gactcttcat ctatccatgt 99360

gtgcctgaca gttctcctgg cactggctgg taacagatgc aaaactgtaa aaattaagtg 99420

atcatgtatt ttaacgatat catcacatac ttattttcta tgtaatgttt taaatttccc 99480

ctaacatact ttgactgttt tgcacatggt agatattcac attttttgt gttgaagttg 99540

atgcaatctt caaagttatc taccccgttg cttattagta aaactagtgt taatacttgg 99600

caagagatgc agggaatctt tctcatgact cacgccctat ttagttatta atgctactac 99660

cctattttga gtaagtagta ggtccctaag tacattgtcc agagttatac ttttaaagat 99720

atttagcccc atatacttct tgaatctaaa gtcatacacc ttgctcctca tttctgagtg 99780

ggaaagacat ttgagagtat gttgacaatt gttctgaagg tttttgccaa gaaggtgaaa 99840

ctgtcctttc atctgtgtat gcctggggct gggtccctgg cagtgatggg gtgacaatgc 99900

aaagctgtaa aaactaggtg ctagtgggca cctaatatca tcatcatata cttattttca 99960

agctaatatg caaaatccca tctctgtttt taaactaagt gtagatttca gagaaaatat 100020

tttgtggttc acataagaaa acagtctact cagcttgaca agtgttttat gttaaattgg 100080

ctggtggttt gaaatgaatc atcttcacat aatgttttct ttaaaaatat tgtgaattta 100140

actctaattc ttgttattct gtgtgataat aaagaataaa ctaatttcta 100190

<210> 39
<211> 933
<212> PRT
<213> Homo sapiens

<400> 39
Met Thr Glu Leu Lys Ala Lys Gly Pro Arg Ala Pro His Val Ala Gly
1 5 10 15
Gly Pro Pro Ser Pro Glu Val Gly Ser Pro Leu Leu Cys Arg Pro Ala
20 25 30
Ala Gly Pro Phe Pro Gly Ser Gln Thr Ser Asp Thr Leu Pro Glu Val
35 40 45
Ser Ala Ile Pro Ile Ser Leu Asp Gly Leu Leu Phe Pro Arg Pro Cys
50 55 60
Gln Gly Gln Asp Pro Ser Asp Glu Lys Thr Gln Asp Gln Gln Ser Leu
65 70 75 80
Ser Asp Val Glu Gly Ala Tyr Ser Arg Ala Glu Ala Thr Arg Gly Ala
85 90 95
Gly Gly Ser Ser Ser Ser Pro Pro Glu Lys Asp Ser Gly Leu Leu Asp
100 105 110
Ser Val Leu Asp Thr Leu Leu Ala Pro Ser Gly Pro Gly Gln Ser Gln
115 120 125
Pro Ser Pro Pro Ala Cys Glu Val Thr Ser Ser Trp Cys Leu Phe Gly
130 135 140
Pro Glu Leu Pro Glu Asp Pro Pro Ala Ala Pro Ala Thr Gln Arg Val
145 150 155 160
Leu Ser Pro Leu Met Ser Arg Ser Gly Cys Lys Val Gly Asp Ser Ser
165 170 175
Gly Thr Ala Ala Ala His Lys Val Leu Pro Arg Gly Leu Ser Pro Ala

```
                180                 185                 190
Arg Gln Leu Leu Leu Pro Ala Ser Glu Ser Pro His Trp Ser Gly Ala
            195                 200                 205
Pro Val Lys Pro Ser Pro Gln Ala Ala Ala Val Glu Val Glu Glu Glu
    210                 215                 220
Asp Gly Ser Glu Ser Glu Glu Ser Ala Gly Pro Leu Leu Lys Gly Lys
225                 230                 235                 240
Pro Arg Ala Leu Gly Gly Ala Ala Ala Gly Gly Gly Ala Ala Ala Val
                245                 250                 255
Pro Pro Gly Ala Ala Ala Gly Gly Val Ala Leu Val Pro Lys Glu Asp
            260                 265                 270
Ser Arg Phe Ser Ala Pro Arg Val Ala Leu Val Glu Gln Asp Ala Pro
        275                 280                 285
Met Ala Pro Gly Arg Ser Pro Leu Ala Thr Thr Val Met Asp Phe Ile
    290                 295                 300
His Val Pro Ile Leu Pro Leu Asn His Ala Leu Leu Ala Ala Arg Thr
305                 310                 315                 320
Arg Gln Leu Leu Glu Asp Glu Ser Tyr Asp Gly Gly Ala Gly Ala Ala
                325                 330                 335
Ser Ala Phe Ala Pro Pro Arg Ser Ser Pro Cys Ala Ser Ser Thr Pro
            340                 345                 350
Val Ala Val Gly Asp Phe Pro Asp Cys Ala Tyr Pro Pro Asp Ala Glu
        355                 360                 365
Pro Lys Asp Asp Ala Tyr Pro Leu Tyr Ser Asp Phe Gln Pro Pro Ala
    370                 375                 380
Leu Lys Ile Lys Glu Glu Glu Glu Gly Ala Glu Ala Ser Ala Arg Ser
385                 390                 395                 400
Pro Arg Ser Tyr Leu Val Ala Gly Ala Asn Pro Ala Ala Phe Pro Asp
                405                 410                 415
Phe Pro Leu Gly Pro Pro Pro Pro Leu Pro Pro Arg Ala Thr Pro Ser
            420                 425                 430
Arg Pro Gly Glu Ala Ala Val Thr Ala Ala Pro Ala Ser Ala Ser Val
        435                 440                 445
Ser Ser Ala Ser Ser Ser Gly Ser Thr Leu Glu Cys Ile Leu Tyr Lys
    450                 455                 460
Ala Glu Gly Ala Pro Pro Gln Gln Gly Pro Phe Ala Pro Pro Pro Cys
465                 470                 475                 480
Lys Ala Pro Gly Ala Ser Gly Cys Leu Leu Pro Arg Asp Gly Leu Pro
                485                 490                 495
Ser Thr Ser Ala Ser Ala Ala Ala Ala Gly Ala Ala Pro Ala Leu Tyr
            500                 505                 510
Pro Ala Leu Gly Leu Asn Gly Leu Pro Gln Leu Gly Tyr Gln Ala Ala
        515                 520                 525
Val Leu Lys Glu Gly Leu Pro Gln Val Tyr Pro Pro Tyr Leu Asn Tyr
    530                 535                 540
Leu Arg Pro Asp Ser Glu Ala Ser Gln Ser Pro Gln Tyr Ser Phe Glu
545                 550                 555                 560
Ser Leu Pro Gln Lys Ile Cys Leu Ile Cys Gly Asp Glu Ala Ser Gly
                565                 570                 575
Cys His Tyr Gly Val Leu Thr Cys Gly Ser Cys Lys Val Phe Phe Lys
            580                 585                 590
Arg Ala Met Glu Gly Gln His Asn Tyr Leu Cys Ala Gly Arg Asn Asp
        595                 600                 605
Cys Ile Val Asp Lys Ile Arg Arg Lys Asn Cys Pro Ala Cys Arg Leu
    610                 615                 620
Arg Lys Cys Cys Gln Ala Gly Met Val Leu Gly Gly Arg Lys Phe Lys
625                 630                 635                 640
Lys Phe Asn Lys Val Arg Val Val Arg Ala Leu Asp Ala Val Ala Leu
                645                 650                 655
Pro Gln Pro Val Gly Val Pro Asn Glu Ser Gln Ala Leu Ser Gln Arg
            660                 665                 670
Phe Thr Phe Ser Pro Gly Gln Asp Ile Gln Leu Ile Pro Pro Leu Ile
        675                 680                 685
```

Asn Leu Leu Met Ser Ile Glu Pro Asp Val Ile Tyr Ala Gly His Asp
    690                 695                 700
Asn Thr Lys Pro Asp Thr Ser Ser Ser Leu Leu Thr Ser Leu Asn Gln
705                 710                 715                 720
Leu Gly Glu Arg Gln Leu Leu Ser Val Val Lys Trp Ser Lys Ser Leu
                725                 730                 735
Pro Gly Phe Arg Asn Leu His Ile Asp Asp Gln Ile Thr Leu Ile Gln
            740                 745                 750
Tyr Ser Trp Met Ser Leu Met Val Phe Gly Leu Gly Trp Arg Ser Tyr
        755                 760                 765
Lys His Val Ser Gly Gln Met Leu Tyr Phe Ala Pro Asp Leu Ile Leu
    770                 775                 780
Asn Glu Gln Arg Met Lys Glu Ser Ser Phe Tyr Ser Leu Cys Leu Thr
785                 790                 795                 800
Met Trp Gln Ile Pro Gln Glu Phe Val Lys Leu Gln Val Ser Gln Glu
                805                 810                 815
Glu Phe Leu Cys Met Lys Val Leu Leu Leu Leu Asn Thr Ile Pro Leu
            820                 825                 830
Glu Gly Leu Arg Ser Gln Thr Gln Phe Glu Glu Met Arg Ser Ser Tyr
        835                 840                 845
Ile Arg Glu Leu Ile Lys Ala Ile Gly Leu Arg Gln Lys Gly Val Val
    850                 855                 860
Ser Ser Ser Gln Arg Phe Tyr Gln Leu Thr Lys Leu Leu Asp Asn Leu
865                 870                 875                 880
His Asp Leu Val Lys Gln Leu His Leu Tyr Cys Leu Asn Thr Phe Ile
                885                 890                 895
Gln Ser Arg Ala Leu Ser Val Glu Phe Pro Glu Met Met Ser Glu Val
            900                 905                 910
Ile Ala Ala Gln Leu Pro Lys Ile Leu Ala Gly Met Val Lys Pro Leu
        915                 920                 925
Leu Phe His Lys Lys
    930

<210> 40
<211> 6455
<212> DNA
<213> Homo sapiens

<220>
<221> source
<222> 1..6455
<223> /mol_type="unassigned DNA"
      /organism="Homo sapiens"

<400> 40
gagttgtgcc tggagtgatg tttaagccaa tgtcagggca aggcaacagt ccctggccgt 60

cctccagcac ctttgtaatg catatgagct cgggagacca gtacttaaag ttggaggccc 120

gggagcccag gagctggcgg agggcgttcg tcctgggact gcacttgctc ccgtcgggtc 180

gcccggcttc accggacccg caggctcccg gggcagggcc ggggccagag ctcgcgtgtc 240

ggcgggacat gcgctgcgtc gcctctaacc tcgggctgtg ctctttttcc aggtggcccg 300

ccggtttctg agccttctgc cctgcgggga cacggtctgc accctgcccg cggccacgga 360

ccatgaccat gaccctccac accaaagcat ctgggatggc cctactgcat cagatccaag 420

ggaacgagct ggagcccctg aaccgtccgc agctcaagat ccccctggag cggcccctgg 480

gcgaggtgta cctggacagc agcaagcccg ccgtgtacaa ctaccccgag ggcgccgcct 540

acgagttcaa cgccgcggcc gccgccaacg cgcaggtcta cggtcagacc ggcctcccct 600
acggccccgg gtctgaggct gcggcgttcg gctccaacgg cctggggggt ttcccccac 660
tcaacagcgt gtctccgagc ccgctgatgc tactgcaccc gccgccgcag ctgtcgcctt 720
tcctgcagcc ccacggccag caggtgccct actacctgga gaacgagccc agcggctaca 780
cggtgcgcga ggccggcccg ccggcattct acaggccaaa ttcagataat cgacgccagg 840
gtggcagaga aagattggcc agtaccaatg acaagggaag tatggctatg gaatctgcca 900
aggagactcg ctactgtgca gtgtgcaatg actatgcttc aggctaccat tatggagtct 960
ggtcctgtga gggctgcaag gccttcttca agagaagtat tcaaggacat aacgactata 1020
tgtgtccagc caccaaccag tgcaccattg ataaaaacag gaggaagagc tgccaggcct 1080
gccggctccg taaatgctac gaagtgggaa tgatgaaagg tgggatacga aaagaccgaa 1140
gaggagggag aatgttgaaa cacaagcgcc agagagatga tggggagggc aggggtgaag 1200
tggggtctgc tggagacatg agagctgcca acctttggcc aagcccgctc atgatcaaac 1260
gctctaagaa gaacagcctg gccttgtccc tgacggccga ccagatggtc agtgccttgt 1320
tggatgctga gccccccgata ctctattccg agtatgatcc taccagaccc ttcagtgaag 1380
cttcgatgat gggcttactg accaacctgg cagacaggga gctggttcac atgatcaact 1440
gggcgaagag ggtgccaggc tttgtggatt tgaccctcca tgatcaggtc caccttctag 1500
aatgtgcctg gctagagatc ctgatgattg gtctcgtctg gcgctccatg gagcacccag 1560
ggaagctact gtttgctcct aacttgctct tggacaggaa ccagggaaaa tgtgtagagg 1620
gcatggtgga gatcttcgac atgctgctgg ctacatcatc tcggttccgc atgatgaatc 1680
tgcagggaga ggagtttgtg tgcctcaaat ctattatttt gcttaattct ggagtgtaca 1740
catttctgtc cagcaccctg aagtctctgg aagagaagga ccatatccac cgagtcctgg 1800
acaagatcac agacactttg atccacctga tggccaaggc aggcctgacc ctgcagcagc 1860
agcaccagcg gctggcccag ctcctcctca tcctctccca catcaggcac atgagtaaca 1920
aaggcatgga gcatctgtac agcatgaagt gcaagaacgt ggtgcccctc tatgacctgc 1980
tgctggagat gctggacgcc caccgcctac atgcgcccac tagccgtgga ggggcatccg 2040
tggaggagac ggaccaaagc cacttggcca ctgcgggctc tacttcatcg cattccttgc 2100
aaaagtatta catcacgggg gaggcagagg gtttccctgc cacggtctga gagctccctg 2160
gctcccacac ggttcagata atccctgctg cattttaccc tcatcatgca ccactttagc 2220
caaattctgt ctcctgcata cactccggca tgcatccaac accaatggct ttctagatga 2280
gtggccattc atttgcttgc tcagttctta gtggcacatc ttctgtcttc tgttgggaac 2340
agccaaaggg attccaaggc taaatctttg taacagctct ctttccccct tgctatgtta 2400
ctaagcgtga ggattcccgt agctcttcac agctgaactc agtctatggg ttggggctca 2460

gataactctg tgcatttaag ctacttgtag agacccaggc ctggagagta gacattttgc 2520
ctctgataag cactttttaa atggctctaa gaataagcca cagcaaagaa tttaaagtgg 2580
ctcctttaat tggtgacttg gagaaagcta ggtcaagggt ttattatagc accctcttgt 2640
attcctatgg caatgcatcc ttttatgaaa gtggtacacc ttaaagcttt tatatgactg 2700
tagcagagta tctggtgatt gtcaattcat tccccctata ggaatacaag gggcacacag 2760
ggaaggcaga tcccctagtt ggcaagacta ttttaacttg atacactgca gattcagatg 2820
tgctgaaagc tctgcctctg gctttccggt catgggttcc agttaattca tgcctcccat 2880
ggacctatgg agagcagcaa gttgatctta gttaagtctc cctatatgag ggataagttc 2940
ctgatttttg tttttatttt tgtgttacaa aagaaagccc tccctccctg aacttgcagt 3000
aaggtcagct tcaggacctg ttccagtggg cactgtactt ggatcttccc ggcgtgtgtg 3060
tgccttacac aggggtgaac tgttcactgt ggtgatgcat gatgagggta aatggtagtt 3120
gaaaggagca ggggccctgg tgttgcattt agccctgggg catggagctg aacagtactt 3180
gtgcaggatt gttgtggcta ctagagaaca agagggaaag tagggcagaa actggataca 3240
gttctgaggc acagccagac ttgctcaggg tggccctgcc acaggctgca gctacctagg 3300
aacattcctt gcagaccccg cattgccctt tgggggtgcc ctgggatccc tggggtagtc 3360
cagctcttct tcatttccca gcgtggccct ggttggaaga agcagctgtc acagctgctg 3420
tagacagctg tgttcctaca attggcccag caccctgggg cacgggagaa gggtggggac 3480
cgttgctgtc actactcagg ctgactgggg cctggtcaga ttacgtatgc ccttggtggt 3540
ttagagataa tccaaaatca gggtttggtt tggggaagaa aatcctcccc cttcctcccc 3600
cgccccgttc cctaccgcct ccactcctgc cagctcattt ccttcaattt cctttgacct 3660
ataggctaaa aaagaaaggc tcattccagc cacagggcag ccttccctgg gcctttgctt 3720
ctctagcaca attatgggtt acttcctttt tcttaacaaa aaagaatgtt tgatttcctc 3780
tgggtgacct tattgtctgt aattgaaacc ctattgagag gtgatgtctg tgttagccaa 3840
tgacccaggt gagctgctcg ggcttctctt ggtatgtctt gtttggaaaa gtggatttca 3900
ttcatttctg attgtccagt taagtgatca ccaaaggact gagaatctgg gagggcaaaa 3960
aaaaaaaaaa agtttttatg tgcacttaaa tttggggaca attttatgta tctgtgttaa 4020
ggatatgttt aagaacataa ttcttttgtt gctgtttgtt taagaagcac cttagtttgt 4080
ttaagaagca ccttatatag tataatatat atttttttga aattacattg cttgtttatc 4140
agacaattga atgtagtaat tctgttctgg atttaatttg actgggttaa catgcaaaaa 4200
ccaaggaaaa atatttagtt tttttttttt tttttgtata cttttcaagc taccttgtca 4260
tgtatacagt catttatgcc taaagcctgg tgattattca tttaaatgaa gatcacattt 4320

catatcaact tttgtatcca cagtagacaa aatagcacta atccagatgc ctattgttgg 4380
atactgaatg acagacaatc ttatgtagca aagattatgc ctgaaaagga aaattattca 4440
gggcagctaa ttttgctttt accaaaatat cagtagtaat atttttggac agtagctaat 4500
gggtcagtgg gttcttttta atgtttatac ttagattttc ttttaaaaaa attaaaataa 4560
aacaaaaaaa aatttctagg actagacgat gtaataccag ctaaagccaa acaattatac 4620
agtggaaggt tttacattat tcatccaatg tgtttctatt catgttaaga tactactaca 4680
tttgaagtgg gcagagaaca tcagatgatt gaaatgttcg cccaggggtc tccagcaact 4740
ttggaaatct ctttgtattt ttacttgaag tgccactaat ggacagcaga tattttctgg 4800
ctgatgttgg tattgggtgt aggaacatga tttaaaaaaa aactcttgcc tctgctttcc 4860
cccactctga ggcaagttaa aatgtaaaag atgtgattta tctggggggc tcaggtatgg 4920
tggggaagtg gattcaggaa tctggggaat ggcaaatata ttaagaagag tattgaaagt 4980
atttggagga aaatggttaa ttctgggtgt gcaccagggt tcagtagagt ccacttctgc 5040
cctggagacc acaaatcaac tagctccatt tacagccatt tctaaaatgg cagcttcagt 5100
tctagagaag aaagaacaac atcagcagta aagtccatgg aatagctagt ggtctgtgtt 5160
tcttttcgcc attgcctagc ttgccgtaat gattctataa tgccatcatg cagcaattat 5220
gagaggctag gtcatccaaa gagaagaccc tatcaatgta ggttgcaaaa tctaacccct 5280
aaggaagtgc agtctttgat ttgatttccc tagtaacctt gcagatatgt ttaaccaagc 5340
catagcccat gccttttgag ggctgaacaa ataagggact tactgataat ttacttttga 5400
tcacattaag gtgttctcac cttgaaatct tatacactga aatggccatt gatttaggcc 5460
actggcttag agtactcctt cccctgcatg acactgatta caaatacttt cctattcata 5520
ctttccaatt atgagatgga ctgtgggtac tgggagtgat cactaacacc atagtaatgt 5580
ctaatattca caggcagatc tgcttgggga agctagttat gtgaaaggca aatagagtca 5640
tacagtagct caaaaggcaa ccataattct ctttggtgca ggtcttggga gcgtgatcta 5700
gattacactg caccattccc aagttaatcc cctgaaaact tactctcaac tggagcaaat 5760
gaactttggt cccaaatatc catcttttca gtagcgttaa ttatgctctg tttccaactg 5820
catttccttt ccaattgaat taaagtgtgg cctcgttttt agtcatttaa aattgttttc 5880
taagtaattg ctgcctctat tatggcactt caattttgca ctgtcttttg agattcaaga 5940
aaaatttcta ttcttttttt tgcatccaat tgtgcctgaa cttttaaaat atgtaaatgc 6000
tgccatgttc caaacccatc gtcagtgtgt gtgtttagag ctgtgcaccc tagaaacaac 6060
atattgtccc atgagcaggt gcctgagaca cagacccctt tgcattcaca gagaggtcat 6120
tggttataga gacttgaatt aataagtgac attatgccag tttctgttct ctcacaggtg 6180
ataaacaatg ctttttgtgc actacatact cttcagtgta gagctcttgt tttatgggaa 6240

aaggctcaaa tgccaaattg tgtttgatgg attaatatgc ccttttgccg atgcatacta 6300

ttactgatgt gactcggttt tgtcgcagct ttgctttgtt taatgaaaca cacttgtaaa 6360

cctcttttgc actttgaaaa agaatccagc gggatgctcg agcacctgta aacaattttc 6420

tcaacctatt tgatgttcaa ataaagaatt aaact 6455

<210> 41
<211> 412779
<212> DNA
<213> Homo sapiens

<220>
<221> source
<222> 1..412779
<223> /mol_type="unassigned DNA"
/organism="Homo sapiens"

<400> 41
atggtcataa cagcctcctg tctaccgact cagaacggat tttaccaaaa ctgaaaatgc 60

aggctccatg ctcagaagct ctttaacagg ctcgaaaggt ccatgctcct ttctcctgcc 120

cattctatag cataagaaga cagtctctga gtgataatct tctcttcaag taggtactcc 180

tatttcttct caatttattt tttccttttt gatataatgt gctactgttt acaagcatat 240

tgtaacttca gagcttacct ctcatcttta aaaaatgttc atttttttgt ctttctgctc 300

caaggatatt ttgcaaagtt actggcaagt attcctggga tgataaaatg tgaaatctaa 360

acttggtaca gtggaaattc acttctagaa taatatttag ctgaggcaga gggcaatccg 420

actacccttt tcttagtaca gcacacacag gctgcctgtc tgttccagat aacataaatg 480

tattggatct agcactagct aggagacact gtattgttga actgtgttag aattttataa 540

gctcttaatt ggacaaatct cagagtagca tgaacacact acctgttttc tgaatctttg 600

gagccataac ttacgtgagt ttgaactaag cgatgtgaat aagccattat ttgtttccta 660

aaggcagtca agttttctga aaagctacac atttagcagc aaaagaacga gcccctctgt 720

cttgaaatgg gcctctgatt ttaagcaagc tcttttgagt cctggtgtcc cattttctca 780

gttccttttt gcctcacaat ggcacataca taatgactcc accacatata gcagtgggct 840

actcgggtaa tgatgtggca gtcacaagac agggcagaat actttcattt tggttagagg 900

aatgccacat gtcttaggaa atgctcgctg aactgtaagt ttcatacttt gtcaagaaga 960

taaccagtat tctctcaaac aagtccgtag gagccaacat gattaaaaga ttttaaagca 1020

atttactcga tagaagggtt gggcttaatc aggacttgtg atcatggcaa tggttcctgc 1080

ttaaaggtgc cagattttta atgcctttat gtgccagatc taataggctt acagagcaac 1140

tccatgtata tgaggttgct gggaaactca tctggttttg aatgtggtat atacatattt 1200

taatattgag agtaaacagg aagaccaggt agaagtattt gaactgaatt ctgaaacgta 1260

cagagaactt aaattggatg agaatgtttg agaaccatgg atggttcagg gtccatttgt 1320
ataccataaa atcccttcct atttctcaaa agcaaatatt ttctttgatt ttgttaatat 1380
cctgtgatga ctgcccacta ggccttggaa tgcatgcaga taatgctgta gcagttggat 1440
aaaatactct agaatgtcag attttcaagg aaattacaaa taaaaacaat gtaggtaaaa 1500
gatacgacat gtaggaaaaa agcaaaattt tccttaagaa tacaagcgtc gtatttacga 1560
gtagaagatt tttcttggac atttggagat acgtgtttca ttttattgct tgtgacagtc 1620
aaggaaagga ataaagctct tgtgtcttaa acttgcagca agaacaaagt ccaactatct 1680
ttcgtttctg ataactctgt gtctttttgt tcaatattgg gtttagagct agctaccatc 1740
taccttccct tcaaacactc actctaaacc tccacacagt gtaagtattt acatcaggca 1800
tctgctaaac attttcacat ggttatctgc aggtcttctc tgagtctgtc tgcattcatt 1860
tatcctagtt gctcagaaca atcgcttggg ctgacggcct ggtggttgag tttactttgc 1920
tttgaatagg gaacagctgt gctccaggac tgcagattgg ctattcggaa aaaagggcaa 1980
ggaagagagt gacccagctc tgggtagacc atccacactg taaaaaaaaa agtcatagca 2040
tagtcttcat ctatggcagt ggagatatgg tcctgtccat ccacctctat caggagaggg 2100
tctccattgt taatctccga gaaagaggcg ccatctgtga aacctcctgc atattgtcta 2160
atgaagtgct attttgaaag gcgtgcactg tacaggtaag agtgatgatg gtgtgcctgc 2220
cttctgggca acatctcagt agacttcctc tgaatcattc ccactggacc caacagcaaa 2280
agcgtacaca tcattgagtt ttatcgttct atctaaggcg tataacattt ttctctctga 2340
aagtctgtga ttctttcgga gaatgaaggg gcttacccta tccatctgcc cctaaaaata 2400
ttcgctggaa agctgatacc tctgctcagg ttttccacag cagatcactc ttcagtaaga 2460
attctgatat taagtagttt aattcagatc catggaaaat gactgagtac cagccatagc 2520
tcccaccact ctctagctgt gcaatttaga ggcagattat ttaacctcat gccttgaatt 2580
tctcatctgc aaagtgggag tactaacagt agctaccaga tatattatat ggaataaatg 2640
acattaagcc actcattaaa ctccttgcag tttccaagca ttagaaactc agtatgagga 2700
agttatttct attctaagcc tagcacagtg cctggtactc aatacatgtt ggttgaatca 2760
gtgaaataat tcaaggtcag caccaaagct gctaggaatt tatgaacatc tgatcataac 2820
tggaaacttg atctaaaaag caaagggcag tcaatccaat caactgagcg taccatctgt 2880
tgaaatgctg ctgcttctgt aatgagtata aagtgttgga agagaaagtc caggaacctc 2940
catcattctt ccatcattcc tctcctttgt gactatcttt gtgatgagaa gggtaacaaa 3000
aaaatcttgc tgagatgagc gtgtcaaaaa cgtgttacaa atgcttcaca ttccctttca 3060
catcaacaga agcatgttgc ttcattgttg ggcaatgtct tagtccatac acacatagag 3120

ctctatgctg atttttttt gagatggagt ctcgctcttt caccgaggct ggagtgcagt 3180

ggtgctttct cagctcactg caacctccgc ctcccaggtt caagcaattc tcctgcctca 3240

gcctccagag tagctgggat tacaggcatg tgccaccatg gccagctaat tttcatattt 3300

ttagtagaga cggggtttta ccatgttggc caggctggtc tcaaactcct gaccttaagt 3360

gatccactcg ccttggcctc ccaaactgct gggattacag acgtcagcca gcatacccgg 3420

ttgtatgctg atgttctaat tcaatgtgat accaaagacc tgagatagtc tctcccactc 3480

tggccccata acatatgtcc acgaggtggt aataataaca actatagtag cacctaaggt 3540

tggggcagct cttactttgt gcgatgcttt ttatagtgtt attacgtgtg attctcacag 3600

caaccccagg tggtgaacaa cgttatgatt cctgttgtac aaatgaggaa actaaggctt 3660

tgcaaagcta ggtaacatgc ccaatattac acagcttcaa aagtgacagc cctaggactt 3720

gaagataaac tcatctaatt ccaaagctca tgcttttagc cattacttga gacagtatta 3780

acttttaaag tttgtaatca atatgaattt ggccttggga aagcaggtta agcatctggg 3840

gttgatggga gataacatta caccctctct tagcctcagc aacttcatct gtaaaatggg 3900

aataattaca tccgagtcac agaagttttg tggctcttca tgaggattaa ataaagtaat 3960

gcatgtaaaa gagttttgta caaagttcac ttttataaaa tgcaagttgt ggccgggatt 4020

ggtggttcac gcctataatc ccagcacttt gggggctga ggtgggtgga tcacctgagg 4080

tcaagagttc gtgaccagcc tggccaacac ggtgaaaccc gtctccacta aaaatacaaa 4140

aattagccag gagtggtggc atgggcctgt aatctcagct acttgggagg ctgagactgg 4200

agaatcgctt gaacccgcga agtggaggtt gcagtgagcc gagattgtgc cattgaactc 4260

ccacctgggc aacagagtga aactgcatct aaaaaaagac aaaaaaaaa aaagtaagtt 4320

gttatatgca atgcataaat tattacttag ttccatgtaa aatcttccca ctaagtgaat 4380

gagggttcac ctggctgtag tgctatgaga tagatatgag gggcagattg gtttatgctt 4440

ctcaaaaaca gaaagtgtgc cagggtggaa gtgtgggtgg ggagtctccg cctcccagcc 4500

atgtggcaaa gctggaatgt gagtacagca gcagtatgga tgcggttttg aggggatggt 4560

ggtaatcctc ttctggcggc accccctccag tattgtggga tgctctctga tttcttttga 4620

gaagacaagt agctaggagc ttccctagcc tttctgttgt aaaaaccatc aagatccctg 4680

ttgaatgcat acctggagct tggtttccct aagcacagac tttaataact tcatttggat 4740

ttagtctcct atttaaagct gccacccact ctcaattttt tgggttttcc tactaagaat 4800

ggatataaca tgggcagtct tccagttctc ctttcttgct gccttgaaga caacacacag 4860

gccaatcaca aggaggcaga gacaggcccc aacaagttga caatcctaga gagcttagtg 4920

tgagtagact tgctgaggtt cctgactttt gctggaatag gagagtgcca ctggcttttt 4980

gacatttctt tttccaactg tttccttgtc aaaatgacca gcagctcagc tcccctaaac 5040

atacctcctc ccctagattg gttcagagga agccatcaag gtccttttgc aaacggatga 5100

tctgcatttt tgagatcctt ctttcctctg ctgttcatag aaatggtctc attggaataa 5160

ttccttttgg aacgttacta aggacaccaa gaaatcaaca agaaaatttg agtgtatctg 5220

acagaagaaa tttggctttt gtactctaat aattatttat tagaagcaat aactggtcag 5280

aatttatttg cttaaaacca tgtaaagaaa ggtgcttaat aaagataatt gcatcacata 5340

tagtaatccg ttttagtatc tttcacctta aaactatgtg acaaataaag acacaaattg 5400

ctctttcttt ctaataagca attttggaaa ttccttattg ggaaattcca aattttacaa 5460

aatttacaaa atttaaaatt ttggaaatcg tacctgcatc aagttttctg aaagaatatt 5520

taaggattaa ggttacttag aatccaatat atgcatagtt taatttaact catattgtta 5580

aattcttttt ctaattttat ttaagaaatg agtaatattg acaagggcct tgctgtggtt 5640

tattatggct tgtcctagag tctctattcc cagctagatt aaaagtgcct cagggctggg 5700

catggtggct catgtctgta atcccaatat tttgggaggc tgaggccagt gaatcagttg 5760

aggccaggag tttgagacca gcctggccaa catagtgaaa tgctatctct actaaaaata 5820

caaaaaatta gccggacatg gtggtgcatg cctgtaatac cagctacttg ggaggctgag 5880

gcaggagatt cgcttgaacc tgggaggcgg aggttgcagt gagctgagat aacaccactg 5940

cactccagcc tgggagacat agagagactc catctcaaat aaataaataa ataaataaat 6000

aaataaataa ataaataaat aaataagtag tgcctcgggt gcatagatgg ggttgttcag 6060

ttttctaccc tgtgccttgg gaagtgcctg gtacacactg gtgtttggca cataattgta 6120

gagtgagatt gagcctggca tgttccttga tccctagaca atctctatca ggtgtgatca 6180

gtaaggcaca taatatgata tgtgactatt acagagtagc acactacaat atgtgcctaa 6240

agccataaca tcaactaata caattcagca gagtagaatg tgaagagtgc ttgcagagtg 6300

ctgagcacca cggtgaggaa gtgtaaacag aagaaggtat ttctgggttg gtgcaggatg 6360

gttagggata gctttgggta ggaagcgact cttgaccatg accttaaagg acaggtaatg 6420

tttacatggg aaaatacaag gaaaaggagg tgcataatga aaggcatggg ggaggtggtt 6480

ttgggaaacc atcagtctga cagaaacata gttaagaaaa gtgcaaggtg gtggtaggag 6540

gtaatttaga agtggagagt cagatgtcct attaaggagc ttgaacttta tcttgtaggc 6600

attaaagatg ttggtgtagg attggaatga aataaagact taaccttaga aaaatactgt 6660

gtggattgaa gttttcagat gaagagtggg tacatcaatt cggaggtgag ggaaatagtt 6720

caagggaggt gtcacaccat taaaaaaaat cttttaggac ttgttttttg caatcatgat 6780

tgtttatggt tgtaaagatt ttgtttcaga aatgattggt ggagaaagaa gtgtattgag 6840

aatcaaaaga tgggactgtg ccactgatgt gcgcagtgag aagctgcaag cttggtgccc 6900

catcttccta actgtaaaat gggcataaca actaccagac agccgtcaaa acattatact 6960

agtgatacaa tgaagactaa gtgatataat tttgaaaggc agtatgcctt atatgtgtaa 7020

aatgctgttt acttggaata tttttatagt aaaattattt tctttggaca agatatagat 7080

gcagaagaaa atgatcatat ttctgtgaga ttaggattca gaccatttga attttttata 7140

tttattaaat aggtacttta tgttgggtac tgaataagtc ttttattttt aacacctcat 7200

ttccctcaca aaatggatgg catctttgga ctaggggaaa tgtgtggtcc ctgcagctgt 7260

agtattttat gattctatag cttttgcctt tagcaaactt ctcttagaat agtgatttaa 7320

gggctgggtg tggtggctta cacctgtaat cccagcactt tgggaggctg aggcaggcag 7380

atcacctgag gtcaggcatt cgagaccagc ctggccaaca tggtgaaacc ccttctttac 7440

taaaaataaa aaattagccc cgtgtggcag catgcgcctg tagtcccagc tactagggag 7500

gctgaggcaa gagaatcgct tgaaccggga ggtggaggtt gcagtgagcc aatattgtac 7560

cactgcactc cagcctgggc gacagaacat ttaatgctgg tggctcacac ctgtaatcct 7620

agcactttgg gaggccgagg tgggtgaatt gcttttgctc aggagtttga gagcagtctg 7680

ggcaacatgg cgaaacccca tctctataaa aaatacaaaa attagccagg tgtggtggtg 7740

cacgcctgta gtcccagcta cttgagaggc tgaggtgaga ggatgactta agcccaggag 7800

atccaggctg cagtgagctg acatagcgcc actgcacact gcattccagc ctgggcaaca 7860

gagcaagatc ttgtcttaaa aaaaaaaaaa agtgacttaa gatactgaca gtataaacac 7920

aaccaaaccc atgggcatct gtgctgggaa taaaagacag acatgttttc attgcactga 7980

tttaggattc tgcttggaaa ggaaaactgt gatctctcct gcagttgcct aaaatgcttt 8040

acagttatgg gaatggaaaa gctaaattct gtgatttgtg ggtagaggga aggaggacga 8100

gtgtcctttc tgatttcctc tttttccttc tcttccaagg ggaaataaaa ggctagagca 8160

acaatttaaa aagaaaaggc aaggagctac tgggggtaga gtcgggaggg aaaaaggcca 8220

aacccactaa ataatttcac ttcagttaca gtaaatctca aatgatgatg ctacactcct 8280

gagaatgttc catggtggaa atgcctgggc tttgaatttt gagcagcgaa gtaccaggag 8340

agggtgacat gataacattc aacaggaaga acactgctct tgtccgtttg acggtcctat 8400

tcctccggat cactcaatct tcacaaatca agcatatagc tccttcacat gtcatttgtg 8460

aagggaaaga ctcctaggaa aatgttttct aggaaaggga aaaaagcaga aagcaaaggt 8520

ctcctgctta ctacagcatt cgtgttgcat aagcagtatt aatgtagtgg agtattaatc 8580

acttagaaca gttcacgtat tattcctatc tgggctctgc agggtccttt cttcacttct 8640

ttctcgacat ttggattctg tccattttac tcctccctct gcacttggaa tagaagtgaa 8700

atttgttgtg ccaattctct gcttgttaga agccatggta atgtttagta ggggaaaaga 8760

tttgtactgc tacatagaag gaggttttgg gattatttaa gactttactt tgttgatggg 8820

attcctagaa tctactatta ctgggtatac tagacaagtt tgtagattta caaaagtgtg 8880

gataacatgg gttgcacttg atttctttat ccagctcttt ggttctaaat ttattgccaa 8940

ttttatttat caaaactatt ctcagcggag tagtattttc tgtggacaca aggaaacatc 9000

tgtgagctta aaccttagag gcagatagcc agttagaaca tttggcatat gaagcttaga 9060

tagcagaaga gaaaaattaa ccaaggcaac cctcaaaaat attgagaagg caactaagaa 9120

aaatttctta tcactgacag actgcagtat tgagttcgtg ttaatgaaga aatgtcagaa 9180

tacataatga atcaataagg agtgtcatat tatggcaagt ttcaatgttg gtgtcaatct 9240

cgactgccag gatttatggt agattatata atggaatcct gagttctgag agatcaaagg 9300

aaaatgctgg ctctttcttc atccattctc ttaggtcaag cctatgcagc aagaagccaa 9360

gttgagaatt gagcaattat ctgaagtggg cagtggagac ggccaaacca gataatatat 9420

aaccagaaag tcaccgtgga gggaaaatgt ggactgaaaa taggataagg ggtggaggta 9480

gaatgtcaag ccatttggtc agtgtccaca gtccaaattt actgaataac agccaattcc 9540

ttacttcatg tggttattta gagcgagact agaggacaag tgaaaaaaaa aaaaaagagt 9600

tcattcctaa ggttgttgca tgtttgtgca aaatttcttc aaatctctgt tattatctgt 9660

tacatgtcta attctgagta cttccagaga ttgcatttca ttactttaga ttagtgggtt 9720

gaagtggtgg aggaagccca tataatctga cttacatgga gaaaacaagt ctttttttat 9780

agtttagttg agtgttatat ttaattagcc ttttttttccc tccataatgc atgcttatga 9840

attttccact taaaattctg aggctctgac ctttatcatt tcctcatgga gcaatttctg 9900

tacctcctta actttagtag aacatagcaa tgaaatatac aagtccccta gaatagaatt 9960

tgtgtgaatt tggttttcta aaaacacaac taccaaagtc tgaggaccag tgtggtattt 10020

ttttcttcta tttgatatgg aaaccataaa ctacttactt tgagcagatg tttcccagtc 10080

ctaaaataca gctatcagtc cactggcccc tataaatctg ttggagtgca cctgctaatt 10140

ctgaagcctc cctaatacaa tgttaaactt tcctttttttt tttttttttt tttttgagaa 10200

aaaaattgta aattaggttc actgttgctt ggaattagga aaaaataaaa acacaggcat 10260

actaatattt cagatggaaa aacaaagtta agaattcaac aagttttttt cccccactct 10320

atcatgtctt gcacatttaa agctgttata caacatttta aaaacaaacc aaaaaaaccc 10380

agggcctttt tccaacagct taggctgatg acccacagta aaaaagtgcc ccatgggata 10440

ttcttaaagc accccaaggg agtctattga aataacttaa agtaaaaatt cagaagaatt 10500

ttacatgtaa ttaaaaatta agaacgttgt tttataagca atagggaaaa gctgtattaa 10560

gttgcattgc tcaatttctc acttgctctg tgcagtgact gctttttcaa ccatgtaaca 10620

acgtctgaat cttcgtggta aaatcatacc tatcacagcc acagcaggtt ttgttctgct 10680

gctgcacatg tgatttgaga tactgtgggc tgggagtttt ttttttttta atttctaatg 10740
gcaaaatgga tctatagaaa tggaagtcat ctgtaatctc agcactttga gaggctgagg 10800
tgggtggatc accggaggtc aggaattgga gaccagccca gccaacatgg tgaaacccat 10860
ctctactaaa aagataaaaa attagctggc gggcgtggtg gtgtgcgcct gtagtcccag 10920
ctacttggga ggctgaggca gaagaaatgc ttgaacccag gaggtggagg ttgcagtgag 10980
ccgagaccgc accgttgcac tccaatctgg gtggcaagag cgacactaca tctcaaaaaa 11040
aaaaaaaaaa aaaaaaagaa gaagaagaaa tggaagtcat cccgtgggcc tgagttggtg 11100
tagtggatta tggcctgtgc gtgaatgaag aaatacttgc caatggcatc agtggtaact 11160
agcttaagcc ctactcagct ttgtaaaata atgtaatcaa ggaatttgat ctgaacaggt 11220
aagccaaaca ttgattcttc agtgcctatt gataagtgag actacttttc tttttaacag 11280
ccttatttca cttaagtggg gagtcaaact agctttaatt aaggaaatct gtagaaatca 11340
cccacatctc cctttccttc tctgttaaaa aaacaaaagg aagaagaaaa ctaggaagga 11400
gtaagcacaa agatctcttc acattctccg ggactgcggt accaaatatc agcacagcac 11460
ttcttgaaaa aggatgtaga ttttaatctg aactttgaac catcactgag gtatgtgtga 11520
acatactagt ttcctctttc tctctcctga ctttgtccgt gaattgataa gatctaattt 11580
ggtcatcagt ttggagaacg atttttcatt taatttcttt cattatcaag tgtgtattgt 11640
caggggctta gcagtacacc tactatctga tgggcactct acatgcgttg cttaggttga 11700
aattaaggat acaatctgtg cactaccacc atttaaaaaa atccccaagt ctactgtgtg 11760
ggtgaggttt ctttgcatag tatcagagag gttaaccaat ttatttacat actcaatagt 11820
ccactctaag tagggaaaat ccaagctttt tttttcttaa gaaagagctt ttcattcttt 11880
tttccctcag agctttacca tagtttccag caagtgaaaa catttcttga tttgaatttc 11940
acaacctcct tctcctcttg ccagttgtat ggtgctaata ggaagcaaaa tgtgagtgtg 12000
cttttaattt aaccctaaaa taggggaata tcttcagaat ttagacttta catattcagt 12060
gacctaccta aaaatagaga catagttcta accttctcaa tcattaagaa acatttctat 12120
tgaatttaat tattcaaaac aacaggtagc agtttcacaa gggaaaagac aagccatgcc 12180
acttgtattt gtggtcacca agtaattaca ttattttttta tttaaaatta agataaaata 12240
acttactgac tgtcaagtcc atttctcatt tcctccatag aacattttaa aactagttca 12300
caaacctctt gaaaagtcat gcctctagaa agtgcaaatt gtcaccattg cctagaagcc 12360
aacttgagag gctgtgtatc tatgcagcat tttgcaaatc cagtagcagg catgtgatat 12420
aaaagattaa atgcacccct gatttgtgaa tactatccta gccttcaaaa tgttcaaaag 12480
cacaggaagt ctgcactttt aaaaatataa cctgcccaaa ttgcaaggga ggggttgggt 12540
gtaggaccta ggtctgtctg ccaatcagct tgagatgcca gtggacgaaa cctgacttca 12600

ctaaaaacca gactggcaaa tattactcag cgtggtaatt tcctaattgt agaactggcc 12660
tgtttggtga agtatttcag gaaaattttt agctggacct tttctaccct actgcataag 12720
aaatgaaggt ttctaggcaa agactttact tagtgactaa ccaaaaatgg taaataaagt 12780
caccacttcc aggcttagct actgtcctca aaacagctgc aggactggtc aggccagtct 12840
tctgaggctg gaggtgcatg tggaaccccc agagccttgt ctgcaagggc atggctgact 12900
gcaggctgtt tagaagcacc ccgccccgtg aaactcctct ggcttaggaa ttttaaacag 12960
ttctagttct aatctctctg ctcagagtct gagcagctgt gaatgagctc tcctgtgaat 13020
cacagaaatg ttaatgggtg agctgctcta gcttcttact tccaagtgga aggcgcctgt 13080
agctctttgc cagcttgatg cagtgagttt gattcctctt ttgggcagca gggagagata 13140
atgaaggagg gggaaaaaag attttaatat agaaacgagt cttccatctg gctgaggctg 13200
aacaaagtag aagaaccagt gcaagtcact gggggtatgg atctcatacc attttcagca 13260
tatacctccc tttctctctt tttgattcta cacatgtcag tccctgagta gtcttgcatt 13320
gctttcccca ctttgaagtc ttagatccaa aactttgatt ccagaaaggt ctaaggtgct 13380
gttgctgaga ttttgaaaga gacctgagac aaactgttgg gattctagac gctattgcgc 13440
atctgcctgg gtaactgtgt cacttaggtt gggtcaccag aataattctc ctccattttt 13500
gctgcctttc caatttataa acaacacaga acatcagtgg ttctcccata atcaaatata 13560
tttggctcta ttaatattaa aatttgatct tgagtagaaa gattctaagg gctaaaaaat 13620
aaaagatgta aaatgtatgt tgataggtta gtgtagaagc cacccatggg gttgccccca 13680
ctcccactct ttcatatttt ttttaagaaa caacactcaa gttacaaagt cgattctttt 13740
tttgttttgt tttatttgag actgagtctc actctgtcac cacccaggct ggagtgcagt 13800
ggtgcgatct tggctcactg caacctctgc ctccccggtt caagcgattt ttcctgcctc 13860
agcctcccaa gtagctggga ttacaggtgc ccgccaccac acccagctaa tttttgtatt 13920
tttagtagag atagagacag ggtttcacta tgtttcacca tgttgaccag gctggtcttg 13980
aactcctgac ctcaggtgat ccacccgcct cagcctccca aagtgttggg attacaggcg 14040
tgagccaacg cccctggcca caaaagtcaa ttcttaactt aaaacataaa agttccattt 14100
cttttatggt ctacttagtc atgtctggag tcagtgagct cagtcaagta gtttgcagaa 14160
tgacagttgt gatacctatt catttaccca ccagattatt ttctgctctc catggcactt 14220
tatagaaaac agtttattcg gtcattacaa cagctctagg aggggtttc attaaaccca 14280
ctttctagga aagcgaacta aggcttagaa aaatcaagta atttgctaaa gactacccag 14340
ctggcaatga tatgacagga catgaactta ggacaataac tcaaggtctt ggtccctgta 14400
aatatgggtt tccaggttat gtatgcaggc agagggctta gatcctgaag tttccttcag 14460

aaacttcagg atctaagccc tctatatagg agcagtataa aaaggaagga tctcttccca 14520
tagggagctt gtcatccatt gaatgaggaa ctcagacttc cactgtgaaa tgatatttgt 14580
atccccattg tgtgcgagat atggggactc tgatcatcat aagagcacat ttttcctgcc 14640
tataaatgat acttaataga gctgacagat tatatatatc tggaacactt ttacctagca 14700
gaaagacaac aggctaggat tagctgacta ataattttat ttatcaagct caattcccat 14760
gctgacttct cttcctctag tgaaaatgtg tcatcagatt tggtcccaaa ctggagaggt 14820
gaaaggatcc tttgaccagt ataatagcca tgccatcagt tttgctttcc taattaagtt 14880
ttatatgggt taatgatcta tatcattatt ttggggtttt ctaaacacat atgaactaaa 14940
gatggaaaag atgatggaaa agatggaaag aagatgactc agatgtacaa aggcttgcag 15000
agagaaggat aagccaaaaa attggtaaat cacacatgaa taatcaagaa ctgactacaa 15060
tataataata tgtgtccatt tccactaatg aataagctgc cattatgtct cttcattctc 15120
tatttaggac aaattacttc tctgctgtat ttccattctt acaggtacct atctgctgtg 15180
atttttccca gttttgactt tatcttgttg ctttaatctt gtctctaagt aagctgcaac 15240
ccagtaaata tattccagta ttgtttattg taattcctgc ctttatgtca ggtggcaaga 15300
ggaagggact ggttttatta atttatcagt ctgggtgtgt gctaagagga tccttagtac 15360
atgctttgat tatggtagat tgagtctaaa agattcagct ggtttattcc atttattttg 15420
gtctgtctac ctaacaaggt cacacagtgg attatactag ttttctatgt gtcatattgt 15480
tggcctgaca cataatagca aataagaggt accagaattt tctgtacagt ctgaagctgt 15540
gtgtgtacaa caaccagtga aaaatcttag ttatttggac ttttgggttc catgatgcca 15600
gagaaagcca gacaccagca gctggaatca gctaagacct aagaccctgc atgcacatat 15660
agatgtttac attttcttcc tattgcgaca ttcagccatt gctttggcgc ctatttcaat 15720
aatatattgc tgctcatcaa agtggaaata atgttttgtt actagctgag tattgaaaag 15780
ctcttcatag ttttgtgatt ttgactcagc tccaacatgc aggacccatt tcttcaactg 15840
actttggcaa caggagagat tgactagtgg gcttgaaagt gatccactgc tgtttctgtt 15900
taactttctt caatcctact gggtcttggg aagaagaggt agcgggcatc cttgtttgcc 15960
tcaaccagga agcacaaggc catcccaggg cggggcagga gagaggtggg agggaagaag 16020
caggctccac agggccattg tttaccttgt tggcgggtca ggtcttctca gggtagggtt 16080
cctgggataa gagcaaagct tattggtttt ccttgctgtg ccacgagagc acaccagatc 16140
acccctgcag ccttgcttgc cttcccacag cctgcccttg cctagggttt cagctgatat 16200
cctttctact caggaggaga aaccacagaa cacatggagg aagtgtttcc agtgttaaga 16260
ctttagacca aactatagaa atctgttcta cctggaaacc tgaagaaata aatcatgact 16320
gctattcagt agaagtaaaa taaaaaaaca gctttactgg tttggaatca taggaaggct 16380

ttctgtatag cctctctgag agctgcctat tggaaggatt tgtcctcaaa acgtgaggat 16440
ttgtggtgtt ccagggttta tatgacactg gcaggatagt ttgctagagg gctgtgcttc 16500
gacctttatc caagggatgt aagccgtgtg tttaggttag tgagcgtcta gcacaaagct 16560
ctaatgttga aggagcattg gaggcaggct gtgcctctga acatgtagaa ttgactagaa 16620
atgccaatgt tcatttcaaa atgaaatcat tatctttctt atgcttccat ttaaaaatga 16680
ttattacatt tttgcatatg gttgaatgca tattaatttt agcaagttta gaaaagagat 16740
aagcataaag acatacataa aaatcactta ttctcatcag ctatagataa tcactaacaa 16800
tattttagga cattttcttt tcatcttttt cttagcaata tgtatgtgtg tgggtataca 16860
tatttaagtg tgtatatatg taaattaaaa aacataaaat attcccacaa tataattaat 16920
aaaaatctaa gatacaacac aatttccgtt tgtaatatgt agtgtatata tgtatatata 16980
cacattatat gtatatataa aatacatgca taaaatagat acacaggtat atattttgca 17040
aattgaaatg atgttgtatc ttgtagattt tatttaacat tagaatttat ttacactact 17100
taggtgtttt aacaaggatt gtggtccacc atttatttcc ttaagagtaa ttccaaaaag 17160
tggaaataga atgtcaaggg gcaaagttct ttataaagtc cttaaggagt attgccagat 17220
atattttttt ttacttcctt ttaaaacatg attgtattgt agaagttctc tttaaaaatt 17280
ccaagcaaaa atttttcaaa atcccatcta gaaatcaaat catgatttta tttggctatg 17340
cctcttgtag tccttgtcta tgaacttatt ctattattaa atataatagg taatttccac 17400
taatttgaca gatagaattt tattttttac attttctctt gttacagtgc catgcatatt 17460
tctattatgt tacagagatc ttggggggtct tttttcctcc ctttttcctt tctcttttct 17520
tgttctgtac cccttcacag aaaccaacaa atacaggttg atgtgtatcc tctactcttt 17580
tctccatgct cacacaaccc aatatttaca catgcactga tttttcaggg caatggtcct 17640
tctttatcat aaattgtggg ataaggttat atacataata tatgtgtgtg tgtattactc 17700
tgaatcttgg ttttgtcact ttgcaatatg tcttaaagat tctgataaat cttatagatc 17760
tatctcattc ttttcagtag ttgcatgcag tataaattta ctataactta ttcaagtaat 17820
ctgctttcag ctgatactct gttgtgaaca gttttcaaca tataaaaaag tgctgtcatt 17880
acaaatattc ttagatatat attcttagta attgggggtt ttattgttat ggtgtaagtc 17940
ccaggagtag aattgctggt ttaaaaggta tgtgtatttt tacatttcta ttggtttttt 18000
catattattt tctaaaaata ttatagcaaa tcacagtccc actagtgatg ttggagagta 18060
tcattttcct cactagtact cctatttccc tgctttctaa tctttcctga tctaatgagt 18120
aagcattgtt aattttcttt tcattttctt gacttctaat gagcttaagc atcttttcac 18180
attattgttg ttcctttgga ttttcttatc tgtcaattac caatggatat ccttgaccta 18240

tttctccctt atatggttgc ttttctctta ttaaaaattt gtaaaagttc tttgactatt 18300
acagatttta aaaactggca gataaagttg tatgatttat tgtgtataat atgatatttt 18360
gagatataca tatatcttga tatatcaata tcgagatata tatatcacac gttgtggaat 18420
ggctaaatat agctaattaa catgtgcgtt acctcatgaa gttatcattt ttgtggtgag 18480
aacacttaaa acctctcagc atttttcaag aatataatat attgttatta actatagtca 18540
ccatgttgta cagtggatct cttgcactta cttttccgat ttaactgaag ttttgcatcc 18600
tttgaccaat gtctttccaa ccctcacctc ccacccctct gcccgacact gccccagccc 18660
cggtaaccac cattctactc tctatgtcta tgagatcaac ctttttagac tctacatata 18720
agtgagatct tttggtattt gtctttctgt gcctggctta tttcacttaa tataatgtgc 18780
tccaggttca tccgtgttgt tgcaaatgac aatatttttc tctttttta aggctgaatg 18840
gtattccact gtgaacatat accatatttt ctttatccat tcatctgttg atggatactt 18900
agattgattc tacatcttgg ctattatgaa taatgctgta ataaacatgg gagtgcagat 18960
atggctttga catactgaat acattcgctt tgggcatata cccagtagtg ggactgctga 19020
gtcattcggt aattgtatga ttactgtttt ccatagtggc tgtactaaca taccttccca 19080
ctaacagtgt gcaagggttc tctttactcc gcatccttgc caacactttt aatcttttgt 19140
ccttttcata atacccattc taacaggtgt gagatgatat ctctttgtgg ttttaatttc 19200
cattttctga tgattatagt gatgctgagc atttttttcta tagattttttt ttttttggtc 19260
agtggattgc taaatcttac cccgtcaata atttctcctt tgattttact tttataattt 19320
tctgctacaa aattaaaaaa taattgtatg aaataaaatt tttcttccta tacgtattct 19380
gaatttccta tcttacttca aagagtcttc ccacctccta ttttctaaaa ttttcttcta 19440
aaaatttcta tgttttattt tattattttt aatatttagt attttttaca tctggaattt 19500
ttttatataa aagtggaagg ctcaactgca tctcttttta tataaagaac gaattgttta 19560
aacatcacat gttaaataat cttttctcca ataaattaaa ttatattagc atcaatttaa 19620
agattttttt tctctaaacc actaatctat ttatttgttg ccaggcctga gacgtattgc 19680
tttgattata atagctgtat agtctctttt aacatttggc aagtttgatg ctccctactc 19740
attaatatta tttataatgt tagcctggtt ttcctttttt tttttcagtc caaataggat 19800
ttagtcagaa gaaagatacg tggattacat ttttaaaata ctgatcaaaa tgaagatgct 19860
ccaaccgtat aaatggcaga tgaaatagac tttaaagtaa aaaatattta tcacacaata 19920
tatcagaaaa atataacaaa cccgaaccaa caaacatcag caacgtagct ccaaaatatt 19980
agcttgaaac atgaaattgc caatagttga ccactttttg acctacaaaa gcaacaattt 20040
atataaagaa aaggtcaata aaattatggt aaattgaatt ttttttttatt attacacttt 20100
aagttctgtg atacatgtgc agaacgtgca ggtttgttac acaggtatac acatgccatg 20160

gtggtttgct gcacccatca acccgtcatc tacattaagt atttctccta ttgctatccc 20220

tcccctagcc ccccaccctc tgacaggccc cagtgtgtga tgttcccctc cctgtgtcct 20280

taggttccca cttatgagtg tggcgtttgg tttgatgttc ctgtgttagt ttgctgagaa 20340

tgatgattgc cagcttcatc catgtcccta caaaggacat gaactcatcc tttttaatgg 20400

ctgcatagta ttccatggtg tatatgtgcc acattttctt aatccagtct atcattgatg 20460

ggcatttggg ttggttccaa gtctttgcta ttgtgaataa tgctgcaata aacatacatg 20520

tgcatgtgtc tttatagtag aatgacttat aatcctttgg gtatataccc agtaatggga 20580

ttgctgggtc aaacggtatt tctagttcta gatccttgag aaattgccac actgtcttcc 20640

acaatggttg aactaattta cactcccacc aacagtgtaa aagcattctt atttctccac 20700

atcctcccca gcatctgttg tttcctgact tttttttttt ttttgagatg gagtctcact 20760

ctgttgccca ggctggagtg cagtggtgca atcttggctc actgcaagct ccacctcccg 20820

ggttcatgcc attctcctgc ttcagcctcc caagtagctg ggactacagg cgcccgccat 20880

catgcccagc taattttttg tatttttagt agagacgggg tttcactgtg ttagccagga 20940

tggtctcgat cttctgacct cgtgatccac ctgccttggc ctcccaaagt gctaggatta 21000

caggcgtgag ccaccgcacc tggcctgttt ccagactttt taatgatcac cattctaact 21060

ggtgtgagat ggtatctcat tgtggttttg atttgcattt ctctaatgac cagtgatgat 21120

gagctttttt tcatatgttt gttggccgca taaatgactt cctttgagaa gtgtctgttc 21180

atatccttca cccacttttt gatggggttg tttgttttct tgtatatttg tttaagttct 21240

tgtagatatt agccctttgt cagatggaga gattacgaaa tttttccccc attctgtagg 21300

ttgcctgttc atgctgatga tagtttcttt tgctatgcag aagctgttta gtttaattag 21360

atcccattcg tcaattttgc cttttgttgc cattgctttt ggtgttttag tcatgaagtc 21420

tttgtccatg cctgtgtcct aaatggtatt gcgttggttt tcttctaggg tttttatggt 21480

ttcgggtctt acatttaagt ttttaatctt gagttaattt ttgtataagg tgtaaggaag 21540

ggatccagtt tcagttttct gcatatggct cgccagtttt cccatcacca tttattaaat 21600

agggaatcct ttccccattg cttgtttttg tcaggtttgc caaagatcag atggttgtag 21660

atgtgtggcg ttatttctga ggcctctgtt ctgttccact ggtttatata tctgttttgg 21720

taccagtacc atgctgtttt ggttaccgta gccttgtagt atagtttgaa gttaggtagc 21780

atgatccctc cagctttctt ttagcatagg attttcttgg ctatacgggc tttttttttgg 21840

ttccatatga aatttaaagt agttttttct aattctatga agaaagtcaa tggtagcctg 21900

atggggatag cattgaatct attaattact tttggcagtg tggccatttt catgatattg 21960

attcttccta tccaagagca tcgaatgttt ttccatttct ttgtgtcctc tcgtttttcc 22020

ttgagcagtg gtttgtagtt ctccttgaag aggtccttca cttcccttgt aagttgtctt 22080
cctacatgtt ttattctctt tgtagcaatt gtgaatggaa gtgcactcat gatttggctc 22140
tctgtttgtt attactgtat aggaattctt gtgatttttg cacattgatt ttgtatcctg 22200
agactttgct gaagttgctt ttcagattaa ggagattttg ggttgagatg atggggtttt 22260
ctaaatatac aatcatgtca tctgcaaaca gagacaattt aacttcctct cttcctattt 22320
gaataccctc aatttctttc ttttgcctga ttgccctggc cagaacttcc aatactatgt 22380
tgaataggag tggtgaaaga ggatatcctt gtctggtgct ggttttcaaa gggaattctt 22440
ccagcttttg cccattcagt atagtattag ctgtgggctt gtcatgaata tctcttatta 22500
ttttgaggta tgttccatca atgcctactt tgttgagagt ttttagcacg aaggggtgtt 22560
gaattttatt gaaggccttt ttttcatcta ttgagataat catgtggttt ttgtcattgg 22620
ttctgtttat gtgatggatt acatttactg atttgtgtat gttgaaccag ccttgcatcc 22680
cagggatgaa gctgacttga ttgtggtgga caagcttttt gatgtgctgc tgggttcagt 22740
ttgccagtat tttatcgagg atttttgcat caatgttcat cagagatatt ggcctgaaat 22800
tttctttttt tgttgtgtct ctgccaagtt ttggtatcag gatgatgctg gcctcataaa 22860
atgagttagg gaggagtccc tctttttcta ttgtttggaa taatttcaga aggaatggta 22920
ccagctcctc tttgtacctc tggtagaatt cggatgtgaa tccatcttgt cctgggcttt 22980
ttttggttgg taggctatta attcctgcct caatttcaga acttgttatt ggtctgttca 23040
gggatttgac ttcttcctgg tttagtcttg ggagggcgta tgtgtccagg aatttatcca 23100
tttcttctgg attttctagt ttatttgcgc agaggtgttt atagtattct ctgatggtag 23160
tttgtatttc tgtgggattg ctggtgatat tccctttatc atattttagt gtgtctattt 23220
gatttttctc ttttcttctt tattagtctg gctagcagtc tatctatttt gttaatcttt 23280
tcaaaaaacc agctcctgtg ttcattgatt ttttttgaa gtttattttg tgtctctgtc 23340
tccttcagtt ctgctctgat cttagttatt tcttgtcttc tgctagcttt tgaatgtgtt 23400
tgctcttgct tctctagttc ttttaattgt gatgttaggg tgtcagtttg agatctttcc 23460
tgctttctct tgtgggcatt tagtgctata aatttccctc taaacactgc tttagctgtg 23520
tcccagagat tctggtatgt tctgtctttg ttctcattgg tttcaaagaa cttatttatt 23580
tctgccttaa ttctgtcatt tacacagtag tcattcagga gcaggttatt cactttccat 23640
gtagttgtgc agttttgagt gagtttctta atcctgagtt ctaatttgat tgcagtgtgg 23700
tctgagagac tgttaggatt tccattcttt tgcatttgct gaggagtgtt ttacttctaa 23760
ttatgtggtc aattttagaa taagtgtgat gtggtgctaa gaagaatgta tgttctcttg 23820
gtttggggtg gagacttcta tagatgtcta ttaggtctgc ttggtccaga ggtgagttca 23880
agtcctgaat atccttgtta attttctgtc tcattgatct gtttaatatt gacagtgggg 23940

tgttaaagtc tcccgctatt attgtgtgag aatctaagtc tctttgtagg tctctaagaa 24000
cttgctttat gaatctgggt gctgctgtat tgggtgcata tatatttagg atagttagct 24060
cttctcgttg cattgatacc tttaccatta tgtaatgccc ttctttgtct tttttgatct 24120
ttgttggttt aaagtctgtt ttatcagaga ctaggactgc aacccctgct tttttttgc 24180
tctccatttg cttggtaaat cttccgccat tcctttattt tgagcctatg tgtatctttg 24240
catgtgatat gggtctcctg aatacagcac accaatgggt cttgactctt tatccagttt 24300
gccagtctgt gtcttttaac tggggcattt aacctgttta catttaagat taatattttt 24360
atgtgtgaat ttgatcctgt cattatgatg ctagctggtt attttgccca ttagttgatg 24420
cagtttctcg tagtgttgat ggtctttaca atttggtatg tttttgcagt ggttggtacc 24480
agttttacct ttccatattt agtgtttctt ttaggagctc ttgtaaggca ggcctggtgg 24540
tgagaaaatc tctcagcatt tgcttgtctg taaaggatct tatttctctt tcacttatga 24600
agcttagttt ggctggatat gaaattctag gttaaaaatt cttttcttta agaatgttga 24660
atattggccc ccactctctt ctggcttgca aggtttctgc agagtgatcc actgttagtc 24720
tgatgggctt ccctttgtgg gtaacacgac ctttctctct ggctgcactt aacatttttt 24780
ccttcatttc aaccttggtg aatctgacaa ttatgtgtct tggggttgct cttctcgaga 24840
gtatctttgt gatgttctct gtatttcctg aatttgaatg ttggcctgtc ttgctaggtt 24900
ggggaagttc tcctggataa tatcctgaaa agtgttttcc aacttggttc cattctttct 24960
gtcactttca ggtacatcat caaatatagg tttggtcttt tcacatagtc ccatatttct 25020
tggaggcttt gttcattcct tttcactctt tcttctctaa tcttgtcttc atgctttatt 25080
tcaagttgat cttcaatctc tgatatcctt tcttttgctt gattgattca gctattgata 25140
cttgtgtatg cttcataaag ttcttgtgct gtgtttctca gctccatcag gtcatttatg 25200
ttctgctcta aactggttat tgtagttagc aattcctcta tcctttttc aaggttctta 25260
gcttccttgc cttgggttag aacatgcttc ttcagctcag aggagtttgt tattactcac 25320
cttctgaagc cggcttctgt caattcatca aactcattct ccatccaatt ttgttccctt 25380
gctggcaagg agttgtgatc ctttggagga gaagaggtgt tttggttttt ggaattgtca 25440
gcctttttgt actggtttat tctcatcttc atggatttat ctacctttgg tctttgatgt 25500
tggtgacctt tggatggggt ttctgtgtgg atgtcctttt tgttgatatt aatgctattc 25560
ctttctgttt gttagttttc cttctaacag tcaggcccct ctgctgcagg tctgctggag 25620
tttgctggag gtctactcca aaccctgttt gtctgggtat ggaggctgca aaacagcaaa 25680
gattgctgcc tgttccttcc tctggaagct ttgtcccaga ggggcaccca ccagatgcca 25740
gccagagctc tcctgtatga ggtgtctgtc aacccctgct gggaggtgtc tcccagtcag 25800

gaggcacagg agtctgggac ccacttgagc aggtagtctg tcccttagca gagctcaaat 25860
attgtgctgg gggatccgtt tctctcttca gagccagcag gcaggaatgt ttaaatctgc 25920
tgaagctgtg ctcacagccg ccgcttcccc caggtgctct gtcccaggga gatgggagtt 25980
ttatctataa gcccctgact gggactgctg cctttctttc agtgatgccc ttcccagaga 26040
gcaggaatct agagagacag tctggctaca gcagctttgc tgagctgcga tgtgcttcac 26100
ccagtttgaa cttcctggtg gctttgttta cactgtgaca ggaaaactgc ctactcaagc 26160
ctcagtaatg gcggatgccc ctccccccac caagcttgag catcccaggt caagttctga 26220
ctgctgtgct ggcagcgaga atttcaagct catggatctt agcttgctgg gctctgtggg 26280
ggtgggatcc actgagctag aacattggct ccctggcttc agccccccttt tctggggagt 26340
gaatggttct gtctcgctgg cattccagga gccactaggg tatgaaaaac aaaaactcct 26400
gtagctagtt cggtgtctga ccaaatggct gcccagtttt tgcttgaaac ccagggccct 26460
ggaggcatag gcacccaagg gagtctcctg gtctgcaccc aagggaatct cctggtctgt 26520
gtgtttcaaa gaccatggga aaagtgtagt atctgggcag gagtgcaccg ttccttaggg 26580
cacagtccct cagggcttcc cttggctagg ggagggagtt ccctgacccc ttgggattcc 26640
caggtgaggc gatgccccac cctgctttgg ctcaccctct gtgggctgca cccactgtct 26700
aaccagtccc aatgagatga gctgggtacc tcagttggaa atgcagaaat cacctgtctt 26760
ctgcgttaat ctcactggga gctgcagact ggagctgttc ctatttggcc atcttgcccc 26820
ttggtctggt tttcaacctt cagtcttcca tatttatttt gaatgaattt tctgcagctt 26880
tattaaaaaa gaatagaaac aagtaagagc aaagcatagt tggtttatgg ttgtaataaa 26940
cttatatgta tgtcctaatg atttgacagc tttgtgatag tatcttgtaa tccaagtaca 27000
tgatatattt ctacattatt tagatcttgt gttaggtttt tccataagat taattttctt 27060
catattggct ttcttgctat gtttcttttt agcataaagc taatccagga aaaggaataa 27120
aactggaagg aaaaaatgtc ttaaaccatt aatattggct gaccccggtg gtagggttat 27180
gaatgaaaca tttttgcttc ttccactttt ttatattttc caaattgtcc atattaatca 27240
tgtatgattt ttataataat aaattaatga aatttaagaa ttagataatt gattgaattg 27300
gataattgaa ttaataaaat ttaagaatga gaataggaat aattgtgctt tgaaaagtca 27360
tatacacaag agttttagat ggaattcaat actaaattta tatgctatac taaatcagta 27420
attctcaagg agcaaaggtg ctggggagtg tggaggagct ctttgttggt gttgcacata 27480
caatttaaga caacattttc cttattcctc accattttaa ccatatcacc taaccagcct 27540
ctggtagcac tcaatagaca tctgatgaat gaatgaataa gtgaatgaaa acattgtgac 27600
aaaatggtat aacattttgt atttgaaaaa tatatgaaaa tctattcttt tcaaatataa 27660
aatgggaaaa taaaattcaa taaaaatatc ttggttggtg agaatacaca aaagatatac 27720

cttcttgtct atgaattagt aataagaaat tgtcttgagg aagtcaacta catctggaag 27780
gttctctctg gacaaggagc ataagtgaaa aacagtgcac taaattataa ccaagagttg 27840
caacttacca ttttaatgct tcagcacagg cacagagact aacatttact aagcaaacaa 27900
gaagtcttgg caagttaatt aaataatgaa ttatttttgg tgaccaagga gttgggcttc 27960
tcattttaaa accatgcatg agatttttcc ctttctaccc attactaaaa tatcgtatta 28020
gtgtgaaaaa ttataccagg actgggagaa aaagaaatca catctgtcct tgacaatggg 28080
ctgaatgaag aggtgaagga gtggttttac tatctaagtg actgaaaaat aggttatggt 28140
gccccagcaa atccttgttg tttgctgaca attagtgatg tctgtttaaa tcatgcagtt 28200
ttattaggcc ttaaaaatat cttttagtta gtttcaattt catcaaggga aaaggagagc 28260
agagtagagt ggaactctgc tcagagtccg gttggaagcc ttcaactttg ttctccctgt 28320
tttcagatga gtgtctattt gtggctctac cttttctctt tcttctctta ccatgacact 28380
cccctcttct ctcttatctt agctgttctt ttcttcttcc gcatattagg cagtgggaga 28440
aacaccctaa atcagttgga ggtgaggaaa gaagaaaagc accccctatct tggtagttcc 28500
tcattcttcc cttcgtcacg tccatgtcat caggctctgc cttcatctgc gatctggata 28560
caatccaatt tatagcatcc attcaaccaa aaaatattga gctcttacta tgtcaaagta 28620
gtgttctgga tgctcggctc catcagtaaa caacctagac taacatcttg ccgttgttta 28680
catctcgtgg tgtagagttt acaccttgtg gtctagaatt tacatcttgt ggtggaggca 28740
ggcagatgat taacaataaa cttaagtagg tctagatgtc aaaaggtggt aaatgctaca 28800
ggaaagagca aaggtcgagc caggtaaggc agattggctg tgtgggtgtg gaggaagggt 28860
gcaagagtac tgtcaagata agccttcctg agaacatctg aaccaggaca tgaaggacat 28920
gaggaagtga gacagtcgct atccagggaa ggaatttcca ggcagaagaa agagccagtg 28980
ttacgcctta aggtgggagc atttctgcga gactggagaa tcactggagt ccattgtact 29040
tggagcccag caagggagaa aagattggag gactcaggag gtgctggggc aggtgaactg 29100
ctttttgatt ggggagttca gagggtaatg ctttggataa atgtaactga aaactttctc 29160
agaagtgttt tcatactatc tctacaattc attttcatgt gaaaacttaa ttggcgagca 29220
gattaatatg gtgatcttcc cttagatcac taggaaaatc ttgttttatg aatttttctt 29280
ccctttttcat tcactagcag agaaaagagt tgtaaaggag ccgcagaaaa ttatagtagg 29340
tttctcccta ctactgagct actgagactg gaagatgctc acgttagcaa ctgagtatat 29400
ttatgtattt ccctttaatg tttgaagggt caggaatatt tgacttagga tactagtctg 29460
tttagaaaca tgattcaact agctacatga ctaaactagc agctgtcaga gagaatgcaa 29520
ggtcaaaact tagtagttga taataataaa gaaagggcag agtgttatga aatgttatga 29580

tctgggtaat ccattggctc acttttttt gtcagcctaa ggttacaaga tgataaatta 29640

agttgctaat attctatatc taccatttac ttttccttta tcatgtgtct ggcaatatgc 29700

tgagtgttct acctctagca ttttattcaa actgtgcaat ctgcctgctg aaacaagtat 29760

cattaacttc atttcctaag gtgagaaaat tgaggcccag agaggttaaa taagtttagg 29820

atcatatgca tagctggtaa gtactatact ttaatatccc tttgccttca tgctttgctc 29880

tcactccatg tttaaatgag cagagagact tatctttaaa cagatatata gcaagtagta 29940

ttttccaaat gaattccaca agatgctcca ttgcaaaagt tcaaataagt ttagaaaaat 30000

gctgcaaact ttgtgcttct taagagattt acagtgcaga ttagcacatt aaagcctttg 30060

ggaaatttgt attagaaaaa cctttattaa cttatttacc ccaattttc caagcttttt 30120

gatcacgaaa ctctttaaaa aacaacaagt ataattgctt ttatagtatt ctttgggata 30180

cacttggaga agtgttacat gaaaagatat gattggataa atttaaatat aggaagaatt 30240

atttaatcat tttttggaat aaaacaccaa ttttaggttg tctaacatac atttgagcca 30300

agacatgaag gacatgagga cataatttac ttttgtcctt gtaaggacat aatgcacttt 30360

tagtggtttg cattgtttat tttcttttga atttcatgtt actttataaa ggtaattatt 30420

taatcaaacc atttgttaag tgtcagacac tatggtaggt gctggtaata taaagagaat 30480

atgtaaacat caacacaaca tatctcctag tatgggctta cagtacagtg agagacacaa 30540

acaaataatt tgtaggattg aaataagtct tacatttgcc acatcaataa agcactatgg 30600

aaacaaaggc acaagaactt aactttgtcc aatgaagtct ttcaggagga ggtgatattg 30660

gagctgaatt ttaaaggatt catagatgtt tatctgaagg actaatgcaa catgttggag 30720

gtgagtcttc aggaaagagg caacaagaac catttgggta acaagatagg acttggcaag 30780

ttaggggagt cgtaagtggt tcattacggc tagaatccag ggctctctgg agagattggg 30840

agtacacaga tcactcaagg gtttggtatg aactgtcagg ctactaagac agaaataaca 30900

aaaatcagat gtgggtttag aaagagcatt ttggctgtaa tacagaacat gatggataat 30960

tgatggagcg agattcctgg ggagatagaa gaggataggg ccatgtaagt acatctgctg 31020

gtattttaaa attagaaaaa gaaatttttg gtagcaaaat cagaacagac ccaccaactc 31080

tttgcaaatc atgggactag aatgtttgga aaggaagatg ctaacgattt tcattcattt 31140

gtttatcatc tatgttgagt gtcttcttgg gcctaggcag catgcccaaa gctgggagaa 31200

taagatactg tatgagatgc tagaaagatc cccacctctc ctttccagaa attcagtata 31260

tcagactaat tggtttacag ttgtgaccat gggcttttgg catagctgtt caacaagatt 31320

tcattctttt tcaaaagcaa ttcattcatc caccaaatat ttgttaaaca aatgtacgtt 31380

gcattgtagt gggtcctcag ggagctggat gtacatgcca aatatgtttc attttatttg 31440

aagcaagtcc acagacaagt aaactagtca agtgatacca ggcaaagtga tttgctttcc 31500

aacagatggt gacagattaa tttcctaaga gactgatctg ccccagaggc actgggaaag 31560
tcttgttagg gaggtagatt atagtcagga ccttggaaaa ttgttgggaa taattgaggt 31620
caggaagggt atacttaggt ggatggggac agcccctgaa cgagggatca gtccttttgg 31680
agtgagcacg gaatattcag gtgcctggga ggagtgaagg cttgtgcttc agcactgatt 31740
gtgtgcaccg atggtggaac atacctgcct gctcagaagc tttggcttct ctctgtaggt 31800
gagagacagc cattagaagt ccttgaacag ggaagaaaca cggtgaaagc aaggatttca 31860
ttgagaagtc cctcatggaa gaagaagatt acattggaaa gagcctggct tagggaggga 31920
gcagtgacaa tcagagaaaa aggcagatag gggcaaagat actggaagaa gaggaaaaac 31980
tgtccaaagc tttgtccttg tgtgggaaag ctggtgctgt tggcgtgaaa gaggaagcaa 32040
gaacatgtgc actgacacgg ctgagagtct ccaaaaccat tgtctttcct taaaacagta 32100
atgatcacaa gcagcttaca actttgcata tagccactgg ggcaaaatcc tattaaaagc 32160
taattcatta tctgagccag tgcatgataa tgcgatgtaa aggcgtgttg ggaggcagat 32220
gatcagaact tttaagcaaa ctaaatttca taacatttat tagaagcctg tctctatttc 32280
cagaaaacgc ctataaatgt ggctgtaatg taggctacag gtcatcacac tgaagtagaa 32340
gcaatgtaca atacctgaaa ggttagagaa gttcagggtc tgggcttata aaaaacttca 32400
ttattcattt gaaagatatt gttttgtatc ccccaattgt ggttttcaat gtatataaaa 32460
gcaggatatc atactttttg atctatggat ttggaagggg ataatggtgt ggtgggcact 32520
gagcatatgg aattatttaa ttagagagat gccctttaga ttactataaa atataaccag 32580
aatctcctgt ttagacttct aaaatctact tctttgaaaa cactactaaa atgaccacaa 32640
agacctgtcc tcacaagtgg gagctattaa tcaatagtgt gtacacatgg acatagagag 32700
cagaataata aacactggag actctgaggg gtgggagggg aagagggagg tgagggatga 32760
gaaattactt gatgggtaca atctacacta ttctggtgat tgatttctct ctgtttcctc 32820
ttcagaaact gagaggaaat ttagtttcct cagtcaccac tcctccaaac tcctgcgtgg 32880
gtgatacaag acttgctgtc atcttgaggc agaaggggtt aggggatttc cccattgaac 32940
ctgatagcct tcatttcatt tctgtcttct cagggaaaca tttacccctc tttactaaat 33000
ggaagaaggt ttgtaaacta ggagggtatc tatggatctg attgctcctt caccactctg 33060
ctatatatcc atgtaacaaa acagcgcttg tggccggata cagtggctca cacctgtaat 33120
cccagcactt tgggaggccg aggtggtcgg atcacctgag gtcaggagtt cgagaccagt 33180
ctggccaata cggtgaaacc ctgtctctac taaaaataca aaaattagct gggcgtggtg 33240
gagcattcct gtaatcctta gctactcggg aggctgaggc aggagaatcg cttgaacccg 33300
ggaggccgag gttgcagtga gccgagatca tgccactgca ctccagcctg ggtgacagag 33360

caagactcca tcgtgggaaa acaaacaaac aaaagaacag tgctggtacc ccctaaatct 33420

ataaaatttt ctaaaaaata aaatgacaat aaaggaatga aaaaggcata ggtacaagca 33480

gatgggatag gacactaaag caagctggag taatactgga attctagaag gtagaaggct 33540

tagcagagca gttcaggtgg aacccacgcc tgtgggggca ggggcatgag cagtgagccc 33600

atctaactac gggaccccag ataggcttgg gatctagaag cgacagatgc ctctaaaggc 33660

tgggggtagg ctgggactga aagcagaatt ggtgtaaagt ctttaaaagg agcaattaga 33720

tccacaaata ccttcctagt ttctaattat tctaccgctt aggagagagg ggtaaatgac 33780

tccctgaaaa cagagaaatg aagtgaaggc tatcatgtta aatggggaca acacctagcc 33840

cctccttcct caagactgac aggaaagctc ctacaaccca cgaaggagtt tggaggggtg 33900

ttgtctgagg aaactaataa gtccaagaga aagaaccttc agaaacggac atttaaagga 33960

ttaggtcccc acccgattac cctagagata cacatacaga aatgtgattg gacagtcaag 34020

gatcaccgta tgatgcttct aacctgaaca atagacacct agtcacacac ccaaacaatc 34080

acacacacac atacacacac acacacacac acttacttca cttctgctgg aggaatttac 34140

aagatgaaga acatcttgta tctcttttgg gctactgttt gtgaaaggaa tggttgcatg 34200

tgctcttggc taagccaact ccatcctgaa gacagatttt tgtgaggtgg gggaaattcg 34260

cccagcgtta atagtgttag tggttgtcct gggtccatcc tggtctgtat tcctgctttt 34320

tcattttcag atttaaatcc agagttatta cagatggtaa catctgatgc caatttatgg 34380

atctttttca taattcacct gctaggaatc tcaggtaacc tataaaatat gcttttattt 34440

ggtcatttta agagttacgt ttctgaattt caggtatttt atcctgtatt gtcagcacat 34500

taaattaggg aacacattca ttgacattct atgtattttc ctttgtattt caaagtcaga 34560

gtcaagtatt taaaaagata agatctttca ttttgtgtgt gatcccacgg aatactttct 34620

actgtagatt tattaaaatt ctcccacctc tggcctctga ttaaggaatt gcagagtatt 34680

ctctatgaca actcattaaa cctattattc cccccagggc ggtttagtat atcactaaat 34740

atacttttag tgatatttgt tgattggagc atagcttttg tgtttgccaa acatcattga 34800

tgtgttttga ggtcatgtgg ttgtgggggg caggggggtgg ttgtaaatgt aatagtgtcc 34860

ctctagtatt gtaatacttc aaggacatag aatttatttt ttaaagtctt aaatcttttt 34920

gtttgttttt ctaggaaatc ttgagtctat gttctttgat ttgagactat agagtttctt 34980

ggtaaagtct ccaattattg gttttctgag taaattcttg agaattggtg aggctcccag 35040

gattattgtt tgtcatcctg ttctaattaa tagagaaaaa gcatataaat cttagatgtc 35100

ttagatctgg gactattgca tctacttggc ttgagccaaa gtgtcatcta atttttatcc 35160

agtaattgtg gcaacatatt gcttccaaaa caaatcacat ttgtaaatgc atattgagca 35220

ttttctacat gaagggcatt accagggagt gaaagaaata taaggtctga attttgtcct 35280

ctgggagaga acattctagt gggagagatg agttgtgaaa agttaaataa caaatattta 35340
tagatggtca atataggaac ttgcaaatat tattaagatt aattacatgt gaaaaattgt 35400
tattgaattc aaggcaaaag aagtgaactt aaactgagtt gcacaggaag aactcataaa 35460
aaggtgagct tgtgttggat gagtggattt gggcagataa agaaagacag gagcaaaaat 35520
aaagaggtgg aaaaccacag gggtgtttga tgtatgcagg gcacggaaat cagttgtatg 35580
gtggggggta ccttcaactt aaaacctctg taccagttag ggtgcttgag ttgtaagaag 35640
ccgaaaccaa ttctggctca tttaggcgga gaaagaattt acaatccatg actaggatat 35700
atccctccat ctatttaatc cctgtttaac ttttctttat tttttatagt ttgtatagtt 35760
ttctatgcag aagacttgca catctttcgt tacatttgtt cataagtatt tgacactttt 35820
atgttattat taaagatttt cttttttttt ttttgagacg gagtctcact ctgtcgccca 35880
ggctggagtg cagtggcgca atctcggctc actgcaagct ccgcctgccg ggttcacacc 35940
attctcctgc ctcagcctcc cgagtagctg ggactacagg cgcctgccac cacttccggc 36000
taatttttgt gtgtgtgttt ttagtagaga cggggtttca ccgtattagc caggatggtc 36060
ttgatctcct gatttcgtga tccaaccgcc tcggcctccc aaagtgctgg gattacaggc 36120
gcaagccaca gcgcccggtc aagagtttta aaaaaatttc agtttctaac tgttgacact 36180
acatagaaat acaatagatt tttgcatatt gtccatgtat ctgccaaact tgctaattta 36240
acttattaat tataataatt ttatctatgg attcttttgg attttccaaa tatacaacat 36300
gccatatatg agtagtgaca tttttatttc ttcttcctag ctccgtaact ttattctatt 36360
ttcttgacct actgcattga ctaggatcct tcactacaat gggaagaaaa agtgatggtg 36420
ggcattcttt tctcactcct gatcgcaggg cagcatttaa cttttcacca ttatgaatga 36480
tgtttgctct acagatttct gtagaaccat ttatcagatt caggtagtta atcctaactt 36540
ggctaacagc aattaaaaaa atgaagtaat ataaaaatta agaaattaat atgctgctaa 36600
tgatatgttg ttgagtaaca taaatatacc aatttttcta tatctactga gatgatcata 36660
tgaatattgt tctttattta ctatggtgaa ttgcattcat taattttctt ttcttttttt 36720
ttttgagaca gagtcttgct ctgtcaccca ggctggagtg cagtggtgca gtctgagttc 36780
actgtaacct tcacctcctg ggtccaagtg attctcctgc ctcagtctcc cgagtagctg 36840
ggattatagg tgtgcaccat cacgcccggc taatttttat atttttagta gagatggggt 36900
ttccccatgt tggccaggct gatcttgaac tcctgacgtc aagtgatctg cctgtctcgg 36960
cctcccaaag tgctgggatt acaggtgtga gccactatgc ctggctggat tcattaattt 37020
tcaaatgcat tttaaaattt ctgaactaaa tccaacttga ttataatata ctatcttttt 37080
ttaagggaaa agtagatgaa tcagactcag caaaaatagg agccagaata gcaactgata 37140

cggtttgtct atattcccac ccaaatctca tcttgaattg taatctccat aaccccact 37200
tgtctaggga gagatctggt gggaggtgat gggatcatgg tggcagtttc cctcttgctg 37260
ttctcgtgat agtgaattct catgagatct gatagtttta taagggcctc ttccccttc 37320
gctactcact ctgtctctca cctgccacca tgtaagacgt gccttggcta ctcctttgcc 37380
atctgccata attatctgag atctccccag ccatttggaa ctgtgagtca gttaaacctc 37440
ttttctttat aaattaccca gtctcaggca gttcttcata gcagcgtgaa aattaactaa 37500
tacagcaacc atggcagggg aaggccaagc accttgactg agtatatcca atgggagagg 37560
agattttttt ctcacagcca aatcaggaca ctcacagctt tgtgtttaaa agcttgggct 37620
ctggactcag gctgcctggt ttgaagtgcc agtcctccct cttatgtggc aggggacctt 37680
agctctgagt atccttctct ataaaggagg atgatgttct atttacccaa taagattcta 37740
aggattaaat gatacattaa agtgcacagg tcagatcact ctcagcccat agaagtaatt 37800
ataattccag ctattattat tgttatttgt gaatatgaga aagaagaaag ttcactctga 37860
catcctgtaa atggaaaatt caactgaaca ttgaaaaatg tttaaatctg tctatcagaa 37920
agtttttcat caaaagaaaa cactgagtag tctagtgttg tagcttgatc caaaaccttg 37980
aataattttc aaactcattt atgatatctg aaatattcaa tgacttatac ctttgaattt 38040
tttggcagct ttgtggaggt atagctgaca tataagaaac tgcagatatt caaaatgtac 38100
agttttataa gttttgatgt atgtatacaa ccattaaacc actaccacaa taacataagg 38160
aacatacctg ccccccatcc acacatttga tgcgcctcct tttaaaattc tctttcctac 38220
cctccacccc tcactgttta ccaaaaagaa tgtctgatgg tgccctgggc agtcccactc 38280
tgcctagaaa acaccataga aaaagacagc aaaagtgctt tctcactctg gagttctact 38340
tgattttaat ggacatagat tagtgtgaac cacataaacc agtactaaaa tacaggattt 38400
gatctagaaa aggtgatgct aaaaatgcat gtagataaaa cctaattttt ttcagacacc 38460
aaaatgaaaa attattagta tgccatgaca catgaacact tttatattcc acaaccactg 38520
tgctctcctt ttggtgttca tttgcaatgg atggaatgtt tatgtccctt ccaaatttgt 38580
atgttgaaat gctaactctc aagatgatgg tattaggagg agaggctgtt gagaggtgat 38640
taggccatga gggtggaacc ctcaagaaaa ggattagtgc cctcagcaaa gaggcctagg 38700
agagctagct tgctcttccc agcatttagg gacacagcaa gagggtgccg tctgtgaact 38760
agaaagcagg ccctcaccag ataccaaatt tgccaatgcc ttggccttgg acttcccagc 38820
ctccagaacc atgagaagta aatttctgtt gtttataagc cacctagtct atagtgtgtt 38880
ttgttttagc aggctgaatg gactaaggca ttatttattg tgagttctat tcaccatgga 38940
gcatatgtgg cctatgtgtc cctggcactg aggcaggaac tgagcagagt acgcagggga 39000
attaataatg cttggaccta tctttaaaac acctagagtt tagtaggaat caactggaag 39060

tagtagggta aaggaaagta ggtgactttt gttgagccct gccatgtgct agagactgtg 39120
ctaaagtgat ttacccacat tatcttattt gaacctcatg gcaagcctgt gtggtaggtc 39180
ctattcctct atttacagat ggggaaactg aagctcagag acattaagta atttgcccaa 39240
ggttatagaa ttatcagcaa caaagctgaa agtggcaaag ggctctttct agtcaggagg 39300
aattaagaga gcttgggtgc agtggctcac atctgtaatc ctagcatttt gggaggccaa 39360
ggtgggagga tcagttgagc ccaggagttt gagatcagcc tgggcaacat agtgagactt 39420
cgtctgtaca gaaaaaaaga aaaaagaaaa aaagagcttg ggatcgtgcc tcctgtgact 39480
cagcatcacc ttagtcttgg tctgcctcca ttctgaattg tgccctaaag ctagtcattg 39540
cttccttctt ggtttctgct acaagtttct gcctctccca ctggcccatt tccggggttc 39600
ctgttcctgt tatgcacatc aatccctaga tttctgaggt cccagagggt gggcttctgc 39660
ttagtccctg tttgtcttcc cagggctagg catcctggtc cttagccctg gcactggggc 39720
ttctctccct gctgccttcg accccccggg gctgaccatc tgctgctcta tccctgcatc 39780
ctgccagctt tcacatcccc tgtctcgagc tctgcttgcc ctttactctt gaggggaact 39840
ccctgcctcc acctgttgga ctggtctggt cccttcactg gtcctgccct ttctgaccat 39900
cttctactct ggtctggcct cccattttcc agagctcatc tgtctccatg tgtgtcctgc 39960
ttctcagagg gtcttgagtt ccagcttttc caggtctggc tggtgccatg gccaaaacct 40020
gtcccacctc tcccattcga gttcctcact agctcctttt tgacagttct ttcttttttg 40080
gggtctagct ttggccttgc tgcatgaatc attaacacat aaatatgtgt cttcacaatt 40140
aattgtttcc atttggtgtt tgctctagtc aaggaggatg gagagggagc aacatcttgt 40200
actgaatgtc ttgacatcac taggaactag actgcttgtg cttcccgggc atgctgaggt 40260
ctcaagaata aagagtatcc ctgaagacat tcttatcagt cttccctggt gaaacattca 40320
tcctaatttt tccttttagc tttgagacca cttttgcatg atttttaata tgtcattaaa 40380
ttaaatagat ataattttct ctgtctagcc aagctgctgt tagaagaatc tttgcataca 40440
actaatcata aaaaagacat tcttcttgcc tctttggaaa ggagcctgtg tcacctaaga 40500
gtgaggagta cactcattct tttgtgcctt gttcatctgc tcaggggatt attgattagc 40560
agatagaatg tgggtgcagg ctggacgcag tggccatgtc tgtaatccag cactttggga 40620
ggccgagatg ggtggattac ttgaggtcag gagtttgaga ccagcttgtc caacatatag 40680
tgaaaccccc gtgtctacta aaaatataaa attagctggg cgtggtggtg catgcctgta 40740
gtcccagcta cttgggaagc tgaggcagga gaatcgcttg aactccggag gtggaggttg 40800
cagtgagctg agatcgcgcc actgtacacc agcctaggtg acacagtgag actccatctc 40860
tcaagaaaaa aaaaaaagaa atgtgggggc agataagttt tttgggggt ttgcttttaa 40920

ttctttggaa gaactgggct tggctggctg ggcacctaga gcaatgtgga catgggcagc 40980
tgcttgtccc atggatgcca gggacagagt cacaagagag acggttagaa atggtgccag 41040
gttctcgctg cctcgtgcca ggtcagctcc caacaaagcc tttgtacaag acacatataa 41100
accctcagag agtttatgct aacccagtgt cttggccatg gcttactcat gatggaaaac 41160
tgatcatatt ttattctggc cacatggaat aatagacatg taatcactat gagatttgag 41220
ttgcagggtc tttggtttct aataaccttt ataattttct ccctcatcag ggagacagaa 41280
taaggtgttt acattactac tttcattttg caagaataag atcgaggtta aatctacagt 41340
tatgtcttga tagactgcag cagatttctg catcagtaga aagtgctgtt ttcccactaa 41400
tcagaagaag aaagtgctag ccataatgat atgggtccac tgtgaagtag aagacaagaa 41460
acagagcagg acgtggaatt gggagcaatg agaaaaggtg cctagagttg ctcttggcac 41520
cctgcacctt agatcaccct tattaactgc ctgctgttta ctctgaaaat ctttctgtca 41580
tttttgagag ataaaatctg aaaatgcagt ttcttcaagt tagaaatgat aatttttata 41640
atagagttta agaaagccat gcttcttttc tcttctatga gaacattttt atttcaatta 41700
taaaattgcc actataaata gccagtcaag ttcatagggc atgagtctca tcaggcggat 41760
aatttacatt atataaaaaa agtatgagtt atttaactaa actaaaaaag caaccagtat 41820
cttgtggtca catactgtat gtgatccctt ttacttgcaa ggtaaaaagt ggtttcataa 41880
ccttgccaat ataattccaa agaactaagg acttagtgat cgttgctaat tgaagttatt 41940
ctgtttatga gcaagcaaca ctatttcttt gatgaaaacc agaaaggatt tacgtgtcgg 42000
taaatagggt tcctttatgt ttgtgtatgt gtgtgtgttt gtgtgtgtgt gtgtgtgtgt 42060
gtgattttat gaggtttgtc tcattcttct atgctagtga gtgttgatta gttggattgc 42120
cttttcagtt gacctattgc tttttaagag catttttgga tatagtccct taacccagaa 42180
ttaacttcta tgataaaaac gctttttggg ttgtcagaga ggctaatagg ggatatatgg 42240
gcagggagtt ttttaccagc cgtcagatct catcttgcag actgtaacta ctgtaaccat 42300
gccccatgaa gggtagtaca aatcatgcta ttgaaattga gagagatggc tgccttttgg 42360
acaggaagag aactagaagt ttttgacttt gtgaatctaa cctctgcagc tgcatccccc 42420
agggctgctg tccacatacc ctgtgctcca ctccctctca tgatttctct caccaccgtc 42480
attctttgtt tctcttgttc ctttatcctg aagctctcaa cctatgaacc tgtgaaaagc 42540
ccagataaca gtaacagatt acatttattt atggaacatt tattatagat ggtgtcattt 42600
tctaagtact ttaaccatat tgattccttt aatctttgca atcatgcagt gaaacaggtg 42660
attctcatac tcccatttta cagttgagga aactgaggca ctgaggggtg aaatgacttg 42720
tctgggacca cacagtacgt agtcgagcta gtatttggac ccagactgtc taactcaaga 42780
gcccaagtta ttggccacca tactctatag cacctctaac ccagatcaaa tatacacagc 42840

accatcacat agtgaaatta atcagtgtca tgtccattcc ctctgcttaa ttgtatgttt 42900

cttgtgatga ggaacataag ccatctccaa tgcctggggc cacacatagc agatgcccta 42960

taactatcat cattgtcatc gtcattctta tcttcctctt cctcctcatc ataatctact 43020

acatataagg caaatttgtg ggtcaggcac acttcacata aattagattt aattctcaca 43080

acaatcttac gaaatttgtg ttgtccccag tttatagttg aaaagacctt gcatttagga 43140

agtggtaaaa tgggaatttg cgaccctgcc atttggttcc agaatccttc ctcctaacca 43200

ctctatacca tatgcttaat taagaaaaaa agaaacaaat aggtgagaaa aaacaatgat 43260

gtgaagaatt ttgctgcccc cagtattagg atttttttc ttcttcttgc tagactctag 43320

agggcccaga tcctgtggag ctgtctctct gaagggaagg gtcaggctgt tcaccctgat 43380

tttcctggag tcacagataa acttctgccc tccaccggat gcctctatct tacacaatta 43440

ttttccttta caaatttggt tgatgacata ttcacttggg ataattgggt tgtccctcct 43500

actttgaaac tgtgagagag ttcactcaat ttccaactgc gtatgaagct cttgagtcag 43560

atttttttgc ctgtaacacc tcagctcaca ccagtgcaga aatggaagtt ttaattggct 43620

gtaggattga agggttggtt tggtgcatgg tggtatttta agaaaggcaa agttttattt 43680

aaatgaggaa tattctccct gggtttttttg agttagctac tgggccatgc aacaggcctg 43740

gctgcttgtg agaaaatgag aagggtgatc aagtacaaga agatgcagcc aaagaagtgg 43800

acacaatcca cgtcaagttc catcttcttt gtggcagcca tgattctgaa ggtcactgaa 43860

gggtattttt ccctgagctg tttttggagc ctgtgccact ggcttcaaat gctgggtctt 43920

ggccacctca gttgctataa aatatggtta tgagtgcttc cgcttcccag catctagctg 43980

acaaagcccc acttgtgcct tgctcagccc cacttcattg ctcctgccct ctctggggag 44040

agggactcac cacctgggtg acaaggttag ggcttcttgc tgcacttttt ggcatcttgc 44100

tgctccctgt ataagggcaa agctaacctt ttttttttct tttcattgag acagtctctc 44160

tctgtcaccc aggctggagt gcagtggtgc gatctaggct cactgcagcc tctgcctcct 44220

gggttcaagt gattctcctg cctcagcctc ccgagtagct gggattacag gcttgaacca 44280

ccatgccagg ctaatttttg tattttagta gagatggggt ttcatcatgt tggccaggct 44340

agtctggaac tcctgacctc aaggaatctg cctgcctcgg ccttccaaag tgctggcatt 44400

actggcatga aacaccaccc cacctccaac cttcaaaaga tttaaaaaaa tgtgaaaact 44460

gaaatgaaga ctagatacta atgaaacatt ggactcatta tgactttgtt aagtcatttt 44520

gattttatta aaatcaaaca ttaataaagc agactgtgaa taaataaatt atattctttg 44580

gccatttctt cattagacaa ggatgctatc tttcatggct gtgttattgt ggggatggct 44640

gatagctcat agaaccaatc taacatccac agattttttt catagttctg gactaggttt 44700

cttagcataa tgcgttgttg tggtcctcat ggtctgttgt tctctcttgc caaccttgtt 44760
tcatcagtcc cctgttgtcc tgcagatttt tgaagcattg gaatcctagc aacagatttc 44820
tcatttaagt aggatggtcc tgatcactag tctcccacag agagttggta aaaagttttg 44880
ttcttcttat cacccacaga acttccctga aacatttttt gttgtgcttg ctcagaaacc 44940
ataagcttag tagaaaaatg caccttgctt gtgaatacac aatcaggtgt ccaaatgaga 45000
tggtcccctc ctagaagata atatttcgga agtcttgcct cagtgggagg tacttggttg 45060
tttgtttttg tttttttttt catttcaata gctttagggg tacaagtggc ttttggttac 45120
atgcatgaat tgtatagtgc tgaagtctgg gattttagtt tacccatcac tcgagtagtg 45180
tactctgtac ccagggtcct tggattttat gggcttgttt ttgtttgctt ttatgttaag 45240
atatgaataa tatgatatgc tttggctctg tgtccccact caagtcttat ctcgaattgt 45300
aatccccata atctccacac gtcaagggag gtacctggtg ggaggtgatt ggatcatggg 45360
gggcaattcc cccatgctgt tgtcataata gtgagtgagt tatcatgacg tctgatgttt 45420
ttataagtgt ttgaccattc caccttcaca cgctgtcact ctcacctgcc gccatgtaag 45480
acatgcctct tccccttctg ccataattgt aagtttcctg aggccacccc agccatgtgg 45540
aactgttgag tcaattaaac ctctttcctt tataaattac ccagtctcgg gtatttcttt 45600
atagcagtgt gaaaacagac tagcatataa tagaaggctc tttacctttt taaacttgga 45660
aggccaaatt aatactgtcc tttccattat aagaatcttc agattaaata ttttctcatt 45720
cattgtccct cagatcatat acatttgctt tttaactcta agttacatgt aataaacaat 45780
gaaattgtat tgtggatcat aaacttgagt tttaaaaaga tttttctcct aattttaaaa 45840
gtaataaaaa caataaatac ttactgtatt ccaggtagtg tttttttttt tttttgagat 45900
ggagtttcac tctgtagccc aagctgtagt gtggtggcgt gatcttggct cactgcaacc 45960
tccacttccc ggcctcaagc aattctcctg cctcagcctc cttggtagct gggactacag 46020
gcacaagcca ccaagcctgg ctaattgttt tgtactttag tctctactaa acagggtttc 46080
cccatgttgc ccagggtggt ctcgaactcc tgagctcagg tgatccaccc accttggcct 46140
cctaaagtgc tggtattaca ggcgtgagcc accgcgcctg gcccaggtag tgttctaagg 46200
ggcatgcttc cattaattca tggaattcac aataaccctt tgttgcagta actattatta 46260
tccccacttt acagatagga aagcggtaca gtgactgggg cagagagagg ctctgtgact 46320
ttgtgcaagc agcaatgtag gcatttgaat ttcggcacgc tggctcctga gtccattcta 46380
ctaaaaattg tgctgtattt aatgcagaga atttcaaaaa catgagataa aaaataagag 46440
aagaatagca tcaattttcc catcattcaa aggtatctgt taaaaggaaa catttaaaac 46500
ttagctatat atactttctg aatttgtatg tacatataaa cttataaaaa atgaaaatgc 46560
taattgtttt cacacgtact gtttggtagc ctccctttgg tatgttccca gaccaaaaat 46620

tattattctt ccccatgctt tttttcagct acataatgtt ctattttatg gaagtacatc 46680
atttccataa ttgaatttct tattaagaca tattatttgc aaatctgcaa caaatgtgca 46740
tgttattttt agacgtatgt ttggttattt ctttacgata gagtcctaga atcaccggct 46800
gaggcaaggg agatgcacat ttgaagcctc tttattcatc ttgccaattg tcctcaagaa 46860
acattgtttc aagtcccatt aatgcaagca atgaacaaaa gtttttattt ttctggatac 46920
tggatgcctc tggcttattt ttctttctat tctgtttctt taatagacaa aacaaaagaa 46980
aaagtttctc atatcaatgt gcattttttt gcgatttgca aaaagttcaa aaattaacaa 47040
aatttatcta ttccttgagt aaatgtatgt ggttcaagat gtcaaagaaa caaagtattg 47100
tgcagtggaa aatgagtgca ccttcagcac ctgcctccca gacatccatc agtaaccctc 47160
tcaggaggcg accactgtta ccatttcctt gactagcctt cctagaataa ttatgcatat 47220
acaagcaaag aagggtataa gggtacacac acaaataagc atactacata tattgttttt 47280
gcccttgtgt ttttcaccta acaatatatt ttggaaatta ttccatatta ataatcaaat 47340
aatgatatta ttatttttga tgcctaccta ctattgcatt ataagggaat tataagggaa 47400
gggttgcaaa tattaacatc tgttaattag ctttggatat ttcttctttt gcaaatactt 47460
gccagtgttc tttgagcagt cttagaattg tggttttaat gttttgttat tgatgatgat 47520
ttgtaagtct ttgctgtgaa ataagatgac cgaccattgg tattaaatga tgtaaatttt 47580
atttcacatt tcattgttgt cttttcaatt ttgttttctg gtggtaggct tattttgttg 47640
ttgtttgttt tgttttggtg tggctatgga agtctttttg ggcaatcaaa tctatcaatc 47700
attttgattt gatttgtgaa tgattttcct ggattttcct ggatgatcag aggctagtgt 47760
tgtctctttg tttacttaga ttcttttgac aagggctttg gctattgtga atagtgctag 47820
tatgaacatg gatgtgcaaa tatctcttcg agaccctgct ttcaattatt ttgggtatat 47880
gtccagaagt ggaattgttg gatcatatgg tagttctatt tttaaatttc tcggaaactg 47940
ccatactctt tctcataaca gctgcaccat ttcacaatcc caccaacagt gcacaaggat 48000
tccaatttct ccacaccctc accaatgctc gctatttttt gggtgttatt tttgacagta 48060
gcccatccta ataggcatga ggatcaattg ttctgaacat aacatttctg tcagggtttt 48120
taaaaagttt ccaaaatgac atccctccag ctccacatat tctataagag caatatcata 48180
aattagaagc tgtctatgaa aacttgtagt tggaagatgt ttgttttctg tgggttagtt 48240
acatttcaca tatgcctcat ttgtgaaata atttctaatg tgcatgagct ggaattcaat 48300
gcctgtagtc aaagtaggct taattttgaa ttggagttga ttaaaatgaa atgactaatt 48360
agctttatat tgacttggga gttgtcccct aaggaaggca ttgggacaat gttgatactc 48420
tgattacctc tattcctacc tctgttagct taaaatatta gtactgcatt cagcacccag 48480

caagaatttc tagtatctga aggcagcagg atacttgcag ccattttcaa accatggaat 48540
atggatgatt ggtagagact tctctatctt gagtcccttc taatatttca tgctttatct 48600
tattcataag aaggggcttc actaagtagg actgtcttgt cttgaccact aggggatcaa 48660
ggttaagttt cttagtcttg gtaattgtca gatttctagt ctttaaaata gggataatgt 48720
catggagtac ctatgtcaga gttgcaagga ccaaacgagc taaggcatga aaagcattta 48780
gcaaggtccc tggcaagtaa gggctaaata aacatggtgt ttcttgatgt tatgtcttgt 48840
aaccattgta ataatctcag tcaatcagat ttatatcagg gtacaggttc ttttctgggg 48900
aatttttcta gcaccaggta ttttctgttt atcttctatg tggatggctt ttccttattt 48960
cttctcattg acaccaaggg attccaagtc ccattctgat tgtgtattgt caaagtgaaa 49020
aacagctgtg gccagcagag ccatgactat aaccattttc aaagtcctgt gtgtaacaaa 49080
cctcaaggat tgatttttca ctaagccttt ctccttgact gtcaaatgac aagcaagggt 49140
atgcagatga taaccccgaa ccccttttgat cccatttcca aaatatggcc atgccataga 49200
tgaaggcaaa ttgttttatt tcatcttgtt ggccccattg tgtatgctct aagttcattt 49260
tggacttctg tctttactga tagcaatttc tatttctttc agacagaagc cccttctttc 49320
ataccttttta gttgcatttc ttagcattct ggccgcagtc ttctactaca tggtccttat 49380
ttgtcatctc ttccctttct tcctctctcc atccctttttt tccattattc atccagcaat 49440
atttattgag aaccaaccat gtgaggggca gcacactgga cattgtcttg gcttcctctg 49500
tcactgcaga tttaatggat cactttagac ttatgcctgt attattgcca gggttcccct 49560
atgtatattg aaacaaatag tctctgggga tctttccatt ttctgccact gattttttct 49620
ttcttttctt tttttttgag atagagtctt actagctctg tcacccagcc tggagtgcag 49680
tggtgcgacc tctgctcact gcaacctatg cttcctgggt tcaagtgatt cttttgcctc 49740
agcctcctga gtagctggga ttacaggagc gcaccaccat gcctggctaa tttttttttt 49800
tttttttttt ttgtattttt agtatataca ggtttttctc atggtggcca ggctggtctc 49860
aaactcctga cctcaagtga tctacctgcc tcggcctccc aaagtgctgg gattacaggc 49920
gtgagccacc acacctggcc attttctgtc tcttctgatt gggctgtgta tctgagctgt 49980
gtcctgggcc actgtgccat cccacagctg acaaaatggc tctcacctcc aggaaactct 50040
tacctcagca ttttttcctc tgtcttgagc ttttcttttg ttctctggga atagtcctct 50100
tactttcttg gagcttcttt agttattttt tgatcaattt tttttacaca gttttttgta 50160
acggagcaaa atattttcag aacttgtttc tggcatgtgt gtgtctgtct catctatctg 50220
cctgtgtcta tgttaaccgt tctttgaatc tgatgcccaa tattctattt gattctgttg 50280
attctgaaaa tttcttttta gtcattggtt tagcatttct acctcttctc cttaaaccct 50340
aggattttct ttaaaaagtg ttgggactga tgttctgttc ttcagattgg cgaacatggc 50400

tcaaagacca ctagtgttta agcagcagca gcttttctat attctgcttg ctgtgggcac 50460
ttgtgatgtc ccactgtggc tgccatcagc agagatgtgg atcctggtgg tggctgtgtc 50520
tgcctcctgg ccagccagct tcctttatag ggaagaaatg cttccgcttc ttactggggt 50580
gtgagaggcg ggcccgtcag tcactgctgc tccctagcac tcatagcaat gtccattttc 50640
tatcctttgt gccctgatga taatctttct ggtatcataa ctgtcttctc ctccccttcc 50700
ctgtctcctc ttcctgcttt ttttcttctt cctccttctc ctcctcactc cagtatcaag 50760
gcttagctta aatgtgttgc actataaagg cctcatattt taaaaatata attcattcag 50820
tcctcctcta tgcctaagct tatttctttg ttatggcact tttcatttca tagtatagct 50880
ctctaccgag gagcgcatac gggataaggg cagagtatga gtatttttttg agcccaaaaa 50940
ataatagtca tttttattgg tatagcttca agcatagttc atttacatga tttatgtttt 51000
aggaaatttc aagtgcagaa aaattgccca gcagttagag atgattgggt tatcaatatg 51060
ctccctgttt tttggtgtat tttttccagc tttattgagg tataacttac aaataacatt 51120
gtatatgttt aagatgtaca acttgatttt ttcaaaatta attatttttt tgaagtaaat 51180
tttattgtgt atacttaagg tgtacaacat gacgtgatga gatatatata gataataaaa 51240
tgattactac agtgaggcaa actaacgtat ccatcatctc acgttgttat ccaacactaa 51300
aatctacgca tggagcaaaa atgcggaata cagtacaata tgattaacta tagttctcat 51360
gttgtagatt aaatctctag acttgttcat cctatatctg ctactttata gcctttgatc 51420
tacatcttct cattttcttc ccttccccca accctcccca taaccactgt gttatgttct 51480
atttccgtat atttgactta ataaaagatt ccatatataa atgagatgat gcaatttttt 51540
tctgagtctt tgtttatttc acttagcaca ataacctcca ccaggctcat ccatgttgtg 51600
gcaaatggca agatctcatt cgtttttaag gctgaataat attccatttg tccatttgtc 51660
catgaattgt cacttaggtt gtttccatat cttggctgct gtgaatagtg ctgcaatgaa 51720
cacgggggtg cagatatctt tacgaggtgg tgatttcatt cctttggata tatattcaga 51780
agagggtttt ctggatcaca gagtaattct attcttaatt ttttgagaaa atttcatact 51840
gttttccgta atggctgcag caatctacac tgccaccaac agggtacagg ttcccttttt 51900
ctccacacgc tcaccaacac ttgctatttc ttgtcttttt ggtaatagaa gggcagggca 51960
tcattaatat tgtttccctt acatgtgctg aacaaacaca gcagagcagt taccagcgct 52020
aactctggaa ctggcttgct catgctcatc tcttgtccct gccagttcct aactgcggaa 52080
acttgggcaa gttgcttaac ctctctgtgc cttcctttgt tacttggagc tgataaaaat 52140
aatgcttaga acagttactg ccatataaaa agtgctatta agtgtttgct attatgtgtt 52200
gaattcagct taatagaatc atcatcagga tcaccagcaa tttattgagt gacagtgtgt 52260

gttttattca ttgatcgtca ttacacttag aatagggtcg gggatatagc aggcattcaa 52320

tcaatatttt tgagggaatg aattaatgaa tgggctagat gttatatata caaagaaata 52380

tgtctaattt ctttccttca ggtctcacag atggttgaga atacaagatg gagataagac 52440

gaaaattgaa tggtgcttaa tatgaagccc ttcaaggctg cctctttcag aatcatttag 52500

gaggcttatt aaaattgcag atttgctagc caaactctcc tcgatctcag ttccatagat 52560

ctggacgtcg gtccaaaact ctacagtgcc aagaaagctc cccaggggct tccggtgaat 52620

tcccaagaac agccaggact gggatcgcag gccaggcagt aaatgataca ggagatgaga 52680

ggagggagag ctctggggc tgacactgtt gggaaggcct ggtgggaggg gaggtacctg 52740

caagggcatg tagggcttgg gcatggggtg gacttgatcg tgggggctta gactgcagta 52800

gagacattct ggtcacattt gcctgcccat gagctccttc cgtctcctga tgtgcctcag 52860

caatttcatg tgcaaagtac acaagagcca tttgttggaa agtacctaaa tcactctatt 52920

tactgagtgc tttacttgca cttataaatc taaaacaaat tttcaagcaa ataaacaagt 52980

gtgtggatat ggagcagaaa taccactcat ggcatcctga acttggccct ctgactcagt 53040

aattagtatc ctaaaactga gttcaagcat cacattttat tttcaggttt tacatacttc 53100

atagaacttg gtctctgatg ttacagagat cacagtaaca gtttacctta tctccagaat 53160

ttctaacttc tcaatggtgg catctctcat tagaacaggt agtcaccaaa tagttccaca 53220

caaagactct cccaatgtgt catttattaa aacaaacaaa cagatttgat atacaattaa 53280

tataccacaa aatcacccat ctaaaatgta cagtctggta gtttttaaca tattcagaat 53340

tctgcagctt tcaccacgaa ttaatcgtag agtattttca tcaccccaga gccacccctc 53400

cctaccttta gtcacttccc atttccccgg cccctgataa tcactcatct gctttctgtc 53460

tctgtggagt actattctgg aaatttcata taaatggaat gatacaacat gtggcctttt 53520

gtgactgact tcttttactt agcatactgt tttcgaggtt catctatatt gcagcatgtg 53580

tcagcacctc atttcttttt gtgctgaaca ctattctact atataaatat acttcatttt 53640

ctttatccac tcattagctt gtacacaact ggatttccac tttttggcta tgtgcaaagt 53700

ttctgtatgc atgcatgttc tcatttctct tgggtaccca cttaggactg gaattcctgg 53760

gacatgtgct aactgtattt atctaacttt ttgagaaatc ttcaaaatgg ttcccaaagc 53820

agctgcacta ttttatatgc ctaccagcaa tgcatgaggg ttcaatttct ccacatcctc 53880

accgacattt gttattgtct ttgattatag ccatcccagt gtgtgtgaag tagtatcttg 53940

ttatagtttt gatttgcatt tccctaatga taaatgacat aaagcatctt ttcaggcata 54000

ttggccattt gcatatcttc cttgagaaaa tgtatgttca attttttttcc atttaaaaat 54060

ttgatgattt taaattttat tgttgagttg taaggattcg ttatatattc tggatagtag 54120

acccttatca aatatatgat ttgtaaatat tttctcccat tttgtgaatc ttttcacttt 54180

cttgatagtg tccttggatg cacaaaagtt tttagttgtg ttgaaaaaca gtttgtatat 54240
cttttccttt ggttacttgt gctttatgtg catatctaag aaactgtttc taatcctggg 54300
tcaggagact tacaactctg ttttcctcta agagttttat agtttcagct cttatattta 54360
ggtctgattc atttagaatt aatgacttgt atatggtgtg aggtagaggt tcaaatctat 54420
tcttctgcat gtggctatcc agttgtccga gcactaaatg tttagtccat tttcatcctt 54480
ccattttgtc ttgtccaggc tgttgaagta gcctcctcac agctctccct gctcttagct 54540
cttctccacc agagtccatc tcccaccata ctaccagtta ttcatcttat acacagatat 54600
gattatgtcc tttccttaat aaaaaaactt tattctttcc ttagtttcta taaaatagtt 54660
caaatattca acataaaatt ccaggtccta tctagcatga cctcaaccca cctcctaatt 54720
ttactgtgtt ccttctcccc acttgctacc ccatcccagc catccttgac ttcttagctg 54780
gtctctttct ggcctgagtg agacatttgg cctggatata tatgcatggt atggctttgg 54840
ttgcttactg tggcattggt gcttatggga tagccattga ctttaattaa tggggtgtta 54900
tcagcacatt tatcctattg aaatgactag aggaacccat aagaaaccac attttcagga 54960
ctagtaatct attttagcat catttggaag acaacaaact ttcattttat gggtagacta 55020
acattcacaa attatgacat atatcatata atcataatca ttaagttata ttttaaataa 55080
tcaaccatcc accatcccaa actctaagac tttgttggta agttttgtat gtcacaggtt 55140
catatttttc acattctgac acctacattc aaggcctcaa actttaccag agacttttta 55200
tcattgaggt aaaactagaa gtcattaatt taaaaaatta tattattttt aattatggta 55260
aaaacataaa atatacattt taccatcttg atttttttaaa gcagtttact agtgttaagt 55320
atattcaaca gatctccaga gcttttcatc ttgtaaaact gaaactgtaa cccatttacc 55380
aacaactcct tccccatttc cttctcctcc caaggcctgg caaccaccat tattctttct 55440
gtttctgtga attttactac tttagatacc tcatataaat ggagtcatat ggtatttagc 55500
acaaggtcct caaggttcat ccatgttgca gcacatgaca gggttttctt ccttttcagg 55560
gctgaataat actccatttt gtctctatat cacatttttt tgtttatcca ttgatctgtt 55620
ggtagacatt tgtgttgctt ccacttcttg gctgttgtaa ataatgctgc tgtgaacaca 55680
ggtgtgcaaa tctctttgag gactctgctt tcagttcttt gggatatata cacagaagtg 55740
tatataggat taggattgca ggatcagatg ataattattg ttttaatttt tcaacttatc 55800
ttctcgaatg agaacactaa attaaatttg tggggtttgt gtcattttag acatagctca 55860
cgaagaagat ggtaacttta aattgtccct gcaacaatga tgatgggctt cagtgattgt 55920
cttaaatgag tcatcattat ttttgtgttt tataaccaac cctatgcatc tgaacacaaa 55980
agtcaaacct ttttaatacc tcaggtgtat tttacaccaa aatacaggga aaaggcatca 56040

atcaaagctg cttaacagct gatatgatag tgattacatg tgatatggta gttgagactg 56100
aaatgctatt tgtaatacaa agattatctt aactgagtct gatttgaggt gaaaaaaggt 56160
actaattagg gtgacaatat gaatttgatt taactttaaa gtattatgag agaaaacata 56220
ctatgtcaca actcttttat ataacgaact gggatttagg tatggagtgg gtagaatagt 56280
ggtcaagagc agggattctg gagtcaagtg tttggcacca atcctggctc tggccattgc 56340
cagctgcatg agtgccactg ggcaagcttc tcacagcacc agatttttca tctgtaaaat 56400
acggttaata atggattgtg gtggcattac atgaattaat atacgcaaca tgccggaatg 56460
acatctggca gggaggaaaa gccatataag attttgtaat tgattttcta gcacaagcta 56520
tccattctta ggttgttgta tcttccttgt gtcatcccac atatcaatat tgtcatctgc 56580
aaaaaatata cacgtatgtc tcttttaact aatgatttta aaatgtggac taatcaatga 56640
tctaaaatcc cccttccttt tagtcaatta gtagttcata aattgaagat gtgatggata 56700
gaacatttcg tacccagaaa gctcttagat tgtgaatgct gagggaacac tgggagattc 56760
agtgagtgca gcacaaatac aatctaggtg actggaatag tttctaatga atgaatcact 56820
gaataagcag atgggttgtg gaaggcaaac catgagtaaa ttttttcttt tactacagaa 56880
atatttctga aaggtaaata cccaggttta tgatgataga ccttctttaa actcatgcta 56940
tttttctttc tgggttttgt tgtcaacaca atctcatctg ctctttgcaa ccattacctc 57000
tcagaatggg tatgaagatc aggtgcaacc atgtatgtga taatgcttct aaaaactatt 57060
ttttttccag ttttattgaa atatattttg cataaaaata taaaatccac ccatttaaag 57120
tacaaagctc agtggttttc agtacattca cagagttgtg cgatcattgc cacaattaat 57180
tcctttctgt ctccatggaa tctaattttg aacattttat ataaatagaa caatacaata 57240
tgcggtcttt tgcgtctggc ttatttcatt tagtatagtg ttttcaaggg tcatttatat 57300
tgcagcatgt atcagtactt cattcttttt attgccaaat aagacttcat tgtatggata 57360
tgtactacat tttattttct cattcatgag ttgatgaaca tttgggtttc cacttttggc 57420
tattacaaat aaaattgcta tgaacgtttg tgtgtgagtt tttgtgtgga cacacatctt 57480
taattctctt gagtatatgc ctaatagaga aatttctgag tcatatggta accctatatt 57540
tagcacttcc aggaactgcc aaatggctgt acgattttac attccaacca gcaatgtgtg 57600
agggctccaa tttccctaca ctgtcatgta cacttgctat tatctgtttt gtcataatgg 57660
gtatgaagtg gtaaaactac tgaaaaaagt gacatattca tggagtaaaa gtaaaattga 57720
aacatgcaga aagtattctt caggcccaga cagcccccca gaattacagt ttttttttcta 57780
gagtccttga aagttgtcta tgcacatatg acgtatccct agatatgtat ttgcaagaac 57840
acatgtagga gtatactatt ttgtatcatt ctttatcact taataatata cctaagagca 57900
attgctatag aacaggttga cctgcctcat tctttttact cttagcagag tatttttttgt 57960

gacgatgtac cttcgttcgt tttaacagct tttctaatga agggcatttt ggttggtttt 58020
gctcttataa gtaatgccat gcgcatccac aagacataca attgttcttg agtacatttg 58080
tgaagatatt cacaggaaaa aacaagttat actgctatat accaccatca acaggggatg 58140
agagtgccta ttagcccaca atctttccag cttaacactg tgtgttatca gcctttccca 58200
tcttggccaa tgtggtaggt aaaaaatagt tcagtaaaag ttgatttgac ctataagggg 58260
caaatcaagt ttatgttata aagcttgaag ttcaacattg aagttgaact ccagataata 58320
tggttaaagc tttcaaattt ctgagaaata tgcaggcata gagattgtat ctaaatttgc 58380
cttaatacat agtatatcca tttgctaatt gaagaaggtg attggttcat ttatttattc 58440
aactggcatt tattcagcat ttatcgtatg ctaggtgaac tttaagtgct agagagaagg 58500
atgaataaga aacatacatt gcttacaagt aatatatagt ctaataaggg ggacatatat 58560
atataaatca ataagcataa tggacatgaa atagaggcaa gtattataca ataagctgtg 58620
tatattaggc cataggctaa gctactgtga aaaaaaagga ccctcaaata cttgggaaat 58680
tgcttttggg aaaagacaaa aaaaaaaaaa aaagcttgtg gaaaagggtg aaaagtggac 58740
atgagagtaa ggagagtaag ccatttttaa aataaaattt tattttaact tcgattgtca 58800
tatggtaagt gtacatattc atgggataaa aatgaaaggt tttgacacat gtgtgcattg 58860
tgtaatgatg aaatcagagt aattagcaaa tccatcatct caaacattta tcatttcttt 58920
gtggtaaagc attcaaaatc ctctcttcta gttagtttga aatatacgat gcattattgt 58980
tagccacagt caccctgctg tgcaatggaa caccagaact gattcctctc acctaactgc 59040
aactttgtac cagttgatca acctctcccc atagccattt cctcttaagc aagaaacatg 59100
gaaattgtac acattacttc tgaaaataca ctggcaagcc acttctagtt gcagggtaga 59160
cttggaaaat gaagtctaac tgggtgaccc tgtgcctcca cattattctg gaggaagggg 59220
atcacggagc tagggtcccc agtctgggag gctggtagag aggcagctcg ctgtgctact 59280
ctgatatttt tagctccgtg ggggaaaaag aactgctaga cagagctctg tcaacacaga 59340
tctgacaatc tcattcagaa acaagttaca gagacctcaa gtgattaata aacaagatta 59400
ttctatagaa catcttatgg tgacataatc aatatgattt caacactcag ctaataaatg 59460
ctatgaaata caatttatgt aaggtgttaa ttgacatgtc aggttttaaa tacaaaagga 59520
atgagggagg gagacatgaa tatagatggg tgttatcaga gaatgacact tagatacatt 59580
tcatattgtg tcatgtaaac taatacctac tttttgggat atttcttcat ggtttagttt 59640
ttaatgtaga ttcaagagtt ttatatattc aaactcttca tgaaaaaaat tcagctctgg 59700
ttatgtacat ccacatttac aggaattgaa tttttctttt tgtacacttg tgagtatatt 59760
ttttcctatg gcaaacgttt tccagaaagc aaaagttatt tgtgttactg tccctgataa 59820

tcgatgctgt gtaataaatc gttctcagca atggcagttt ggacactgat ggacagttgg 59880
tgactggtaa tttgtacagt atgtttagtg ggacggcggg caagggggaa aagtggcatt 59940
tggttcctca gtcccagatt tgggtcaatg ttctcataat actggtgagc aaggagccta 60000
attctttagg aattttaaaa ggtttctttt agtgaagaac atttcccagg tgtctaatga 60060
aaaattataa atgaccaggt ataattttgc caagctttcc aacatagttt tcctttaggg 60120
tgagaaacaa tcctttgatt ttcttgaagt agaagtctct tctcgaatgc taacagtaaa 60180
acaagacctt ttcagacaga ttttgtattt tgccatcaaa tgtgcaattt ttgaagatac 60240
gggacttgag aatcaggtaa acttcgttct gaaaaccagg cttaatttta tgactgtgat 60300
tagaaagttg aaaaaaaaat cacgttctca caaatgaaaa caaaagtcag agttcaaact 60360
gtttatcctg tttctgtaag cttttgtttc agaaggccaa ggattatttg aggagaacct 60420
ggaaaattaa ctcaaatgga ggaaaaaaat ggcgtttatg caaaatgcag caaaagcaga 60480
cagccttgga gaactgaagc ttaaatagcg taagagtctg agtactagtc ttcagatact 60540
taaaacagca tttgatagac aagttagata tttaaggaaa catttaatca gcttggcatc 60600
agcattagtg gcttcaattt ttggctgagc ctgactttgc catatgatcc tagacaaatt 60660
tgcctaaaat gggtgatatt aacttcttta gggaaaggct ctgtactggg atttttttga 60720
ggcctatgat ttatggggtc aagctataaa acaggaggaa acacatgctc atttgtataa 60780
tttttacata tatttggtcc ataattgtac agctaggcaa ttcatttatt atctgtttga 60840
gagtaaaagt ttgattattt taatgttact acttctggtt ttttctcttc tcacatgttc 60900
ccagtgactc ctttctgata acccaactta tcatcataga acgcatgtaa tgctgagaat 60960
aagacacagg gtcttggaaa atgaatgaca gcaatggtgc tccgatggca gttttgtcag 61020
actttgaatg gtttatgaaa tcactgttga taaaagtgaa caccttctac tgaagggaga 61080
aatttagggg ggaaaaatcc caaatagaag gagttaatat ccaaacctgg agacttacct 61140
ggtaaggttc acttaactgg taaaatgtga tccaatttaa acaaagtatt tttagttttc 61200
tcagaacaaa catcctacat aaacacaaaa aatgatatga gacatagata taacttggtt 61260
cacaatattt tccaaaacta taatgtacca gccagttggt acagcacacc aggagagaag 61320
atcattatta atgtgctaat agcagcattt tattttgaaa cccactctgc atggttacag 61380
ggctcaaaac aacatattct aacaggaaga tacattaccg aaatatttta atgagaatat 61440
ttaatatgca ttgagaggtc cgcattttct tgcagagacc ttgtaggtag ctctttgaga 61500
tttctgtctc tatgcattta agtgaaggag ttggttgggt attttagttg gcaaattttg 61560
cagacatgta gctttggtag tggagaggta atagtaccat gccctgcgtg ctggcgagga 61620
agccccacag caacagtggc ttttagcagc taccagattt gctaaaagca gccatgtcca 61680
attagcagta agtgccatgc acctgcagtt actaggaatt gaacctcttt tgaggctgaa 61740

tcttaatgta gccttttaaa aaaatagcaa aaatcttact catactctga gataataaag 61800
aaaaattagc aatggcaaaa tggacgcact ctgaaatgta ttcttaataa tgatttagaa 61860
tatggggtaa atgtagaggc aatgacacat ttaaactgca ttatttttaa atactgtttt 61920
atcagtttac ttcctaattt tgaattcaat tttcatatct atactgcaac tgcttttttt 61980
tttttttttt agaaatctat aatattgctg caggctccct atgtatgttt ccataatttt 62040
ctgcaaagtg ttttcaccag aataaaaaaa aattacagtt caaaattgca aaactgtaga 62100
aaaaatatgc tctttgactt cttttctatg tgtcaaattc accacaatgg aaaggactac 62160
actatagaat taaaacgtta ttttcaacag atagtactta ttttaacatg tgttcagcat 62220
ttaaaaatat ttaatattct tttctaaaaa tgcttacatt cagaaactat ttatgagggt 62280
tctgagcagt atgatgtttc tttctctgat ttactgcttt ttctctttta gaggatattg 62340
taggagaaaa acatttgtta agcaatttcc agaactacta ggttctaata agaccaatag 62400
gctaataatt catttcattg caactggagt gtgtactttt cctttattct cagtcataat 62460
tttttaaaag agccaagaac accaaatcat caaatctaat gttgaacatg tagtaacttt 62520
tatttgctga tcatactctg agattcacca taacgaataa atcataagta ttaaaatttc 62580
agctttttaa catacccact acactgctag cctggtaaca cagtcaccaa ctacacagct 62640
tcttcaactg ttgacgtgct ttaaagctag gattatggta tcctgccaaa aagatcctac 62700
aaactgtcac tattttggtt tgtcctgcca gccattgaga agtagacaat ttctatatac 62760
acaaaccagt ttggaaaatt tatgcaaata ggaagttatt ctcaaaatgg gttatttgat 62820
tgcatttttt tttttttttt ttgagacaag gtctgactct cgcccaggct ggagtgcagt 62880
ggcgtgatct tggctcactg caacctccac ctccgtggtt caagcgattc tcctgcctca 62940
atctcctgag tagctgggaa tacaggtgca tgccaccact cctggctaat tttgtatttt 63000
ttagtagagg caaggtttca ccgtgttgac tagggtggtc tcaaactcct ggcctcaagt 63060
gatccaccca cgttggcctc ccaaagtgct gggattacag gcataagcca ccgtgcccag 63120
cctgattgtg ttttaatgta ttggctccca accagtagca acagtttggg tgcacagata 63180
ttgccgtagt gtttctattc aatgtgtcaa attatttagg taaaagtttg gcttaacttt 63240
gttgacatgg aatttctata tcctccagtt tctggtttaa ctggtttatg tggaaatgaa 63300
tggaaacaca tggttaagtt ccccagcctc cagcttctct cttcctctta cctatatttg 63360
tacttgccca cttttcctct tgtgggcctg ttcctagaat ccattatgtt ctgtgggtga 63420
catcagcctg ctactgcggg aagaaataga attttatggt gcccaagata aaatcgacta 63480
tgatgatgca gaatgaagac ctggagagga gtcagagatt gcaggggata aaaagagaga 63540
aatcagagat gctcccacaa acagagaaaa ccttcagaag cagcaggaag gacagagtaa 63600

atggagaaca aaaactcaag tcaaaatagt caatggaaat aaacagtttt gaattccagt 63660
tataatttgg aaaagaagca aacgtttatt ttaaattaag aaaaaagtcg cttataattt 63720
cctgaagttt agaaaagtga aaaatagtta aacctcctgt tgttagctaa gaagtgtcta 63780
ttattataag tttgttgctg gagagaacat aaaaatctct atctattgct attttttttt 63840
tttttgcata agggatggat aaaagagtgt ttattttatt tagcctttgg cctgtaaagt 63900
aagatttatg aaacaaagga caatcggaaa tgataagaat tttttcctaa tttctaatta 63960
tttagctaaa ccttgattga tttctctcag gctagtgccc atattacagc agcagtcagc 64020
cttcatttat cttacctggg accactgcta aatccgatgt cacttttcag tttttatatt 64080
atgtgaccat ttagccacat ttcacctact tgatcattcc ctccatcttg agacctccct 64140
cgacccggct tccaacatac tttgccctcc tggtttttct ttctctgttg gctcactttc 64200
tcagtctcct ctgctggttg ctccattttt ccacaatctc tcaaaactgg tgctcctcag 64260
attaagtcct tggacttctc tccttagatt gacatctgga gtcatggctt caaacaccac 64320
ctatcactcc ctttctcctt gaactctaga cgtgtccatc caactgctac gtgacatctc 64380
cactgggatt tctaagaact ttgccaaact taacatgtct aaaaccaaat tctgggctgg 64440
gggtggtggc tcacgcctgt aatcccagca ctttaggagg ctgaggtggg cagactactt 64500
gagcccagga gttcgagacc agtctggcca atagagaaaa gctgtctcta caaaaaatac 64560
aacacttagc tggacgtgat ggtgcatgcc tgtagtccca gttactgggg aggctgaggc 64620
gggaggattg cttgagcctg agaagttgat tctgcagtaa gctatgatta caccactaca 64680
ttccagcctg ggtgacagag tgagaccctg cctcaaaaaa aaaaaaacaa aaccaaaacc 64740
aaaaccaaaa aaaaaaagca aagagcaaaa cacaaaaaac atattccaaa tctccccttt 64800
aaacctcctc ttcttacaat gagtcctatc ttgatttatg gcagatcctt ccttccagtt 64860
ctcaagccaa acaccatggg gtcattcttg actcctctct tccatgggct catctaaaca 64920
tcagagtatc ctgttggcta tctggaattt aaccacttcc tatcactgcc attgctaccc 64980
acctgctctt agttatcacc agagttccct ggagtgttgg agccgccgca gagctggttt 65040
ccctgctcct gcctttcctg cccatgctct gtcctgccag tcagctctgt cctttaaaaa 65100
tggaaatcag gttacctcat gactcttttc tttctttttc ttttcttttt ttttttacag 65160
aatcttgctc tgtcatccag cctggagagc agtggcatga tctctactca ctgcaacttc 65220
cgcctcccag attcaagtga ttctcctgtc tcagcctctt aagtagctgg gattataggt 65280
gccagccacc atgcccagct actttttgta tttttagtag cgaaggggtt tcgccatgtt 65340
ggccaggctg gtcttgaact cctgacctca ggtgatctgc ccgcttcagc ctcccgaagt 65400
gctgggatta caggtgtgag ccaccgcgcc cagccatgac tcttttcaaa aactccccat 65460
cttctccact tccattcagt aacagtcaga tctgcatggc cccctcctga ccttctgatg 65520

tcatgttctt tgctacccct tgagtgcatt gaacatgcct ctgacccaga acttttctgt 65580
ttgctatttc ctcaagtgca tatcttgttc cttcattttc ttcaggtgat tattaaaatt 65640
tcacttccat gagtccttcc ctggccccca tatttaacac tgcatcttcc tccatcttct 65700
ttccctcctc cccatgtaca ttttactccc ttactaaatt ttttttccta tctcactttc 65760
ttttcttttt gagacagagt attactctgt cacttaggct ggagcgcagt ggcgcaatct 65820
tggctcactg caacctccac ctcctgggtt caagcaattc tcctgcctcg gcctcccgag 65880
tagctgggat tagaggcgcg tgccaccatg cctggctaat tttgtatttt ttagtagaga 65940
cagggtttca tcacgttggt cgggctggtc tccaactcct gaccttggct tcccaaagtg 66000
ctggtattac tttctaacct cctgtgtatg ctacctattt atttgtctgt ctctctacac 66060
tagaatataa gctctatgag ggtagacttc tttgttttgt ccaatgctct atttccaata 66120
tttataaccg tactggccgg taggtactgt caattatagt ttttgaataa attgagagta 66180
acaaattcta actggtgaaa ataaattaaa tgggccgggc gtgatggctc acgcctgtaa 66240
tcccagcact ttgggaggcc gaggcgggca gatcacttga ggtcaggagt tcaagaccag 66300
cctgggcaac atggtgaaac cccgtctcta ttaaaaatac aaaaattagc tgggtgtggt 66360
ggtgcatgcc tgtaatccca gctactcggg aggctgaggc acaagaatcg cttgaaccct 66420
ggaggcgaag gttgcagtga gccggcattg agccactgca ctccagcctg ggcgacagag 66480
cgagactctg tctcaaaata aataaataaa ataaaaatta aaaagaaaat aaatgatctc 66540
acaggatatc atttcttgat tggaggagga gagaaaaatg tgtatgtgtg agggtacaga 66600
gagagttcct ttgcattagt gagggttcac atcagaactg tctaacctcc ctactcagtg 66660
ggtcctgtta cctctctcct gaatccaata atactctggt gcacaccttt tactggtact 66720
gaggctattt taacgttcca ttaaaaaaaa aaagaaaaca tttaaaatac aaaaacaagg 66780
ctgtactact ttaactctga ccttagaaga ctctcagttt tataactgtt tagagaaaat 66840
gtggaaatgt ttgctggccc caatcttttt attactcatc tctataaaga ggcttttaag 66900
gacatattaa aaagtttcca tctctcttgt ctcttgcctt tcttctcatc ttcctttctt 66960
gtaccccaaa tggctgaaag caagaccctg agagtctgga agaaatatat tctggtcaga 67020
gcagactgag ctggagctgg cctagaacct tggatcctga cctcagaatt gctgctcaaa 67080
ttactgctct ctgaaagagt gagctcatgc atgtttatta ttactgtgaa caaatttagc 67140
tgttgtttca cccaaacatt gaagttctcg cttggacccc tttgcttgtt tgtgcttttg 67200
caaatgtgtt ttctcatgtg ctactagaac caagatgaaa gcttcagttc tcagaacagg 67260
tgccacgacc gaggagacaa aacaagagct atagaggggt gtggtgtgtg aaactgagaa 67320
agcaggtagt tttcagaaa aactgagagc caggaatgac ttcatccatt tcctgtaata 67380

aggcaatggt ctgaccttga ggaacctatt agagtgtgct tctaaacaga ggccatttcg 67440
aggctgaatg tacgggagta tttacctttg actgttttct gaagcaaggt attttataaa 67500
ctgtaaagtg gcttatacaa ttttaggaat ggaaagtttt gcaaagtctc caaaatccct 67560
gggagttttg aatgtgcagg ccatatgaat tctttgtagt ttcatggtct tgctgttcag 67620
cattctttag agtcacctgc atggtagaag tttagtagga atttgggtag ttatgggctg 67680
gtttctttgt tttcccaaat agaggtagca aaatggatgc taagaagcaa attactttta 67740
ctattttaga tgcctctggg gacatcctgt cataatgcaa cttaaaatat actcgtctcc 67800
tcagttcctt tttatgtatt tatttaatag tcttcaacca atttgttctt cattagctgt 67860
tctacaatac ttcaagagaa gtcatgataa tgatgcattt aggatcagaa aaatatagca 67920
tgtgtgagtt cctggggact ttagagaata tctagcccag tctttcattt tatagaagag 67980
gaaggccagg gatggtggct catgcctgta atcccagcac tttgggaggc caaagtgggc 68040
ggatcacctg aggtcaggag ttcgagacca gcctggccaa catggtgaaa ccgcatctct 68100
actaaaacta caaaaattag gcaggcctgg tggtgggtgc ctgtaatccc agctactcag 68160
gaggctgagg caggagaatt gcttgaacca ggcagaggtt gcagtgagcc aatatcatgc 68220
cactgcactc cagcctgggc aacagagtga gactctgtct caaattaaaa aaaaaaaaaa 68280
aagaagaagg agaggaagct gggggtgagg acaggcagtg actcacccag ggtctcacac 68340
tgatttgctg gcagagttaa gcatgaaaag gtgcctctcc tagtctcttt ccagtgttct 68400
tgtcatcaca atgagttaaa ataaaaatta ctagcaactt gggagtaata acaataaact 68460
tctcaaaatc tactacatgg tctagaaaac caaacataag gccggacgca gtggctcacg 68520
cctgtaatcc cagcactttg ggaggccaag gcaggcggat caggaggtca agagatcaag 68580
acaatcctgg ccaacatggt gacactctgt ctctactaaa accacagaaa ttagctgggt 68640
gtggtggtgc gtgcctgtag tcccagctac tcaagaggct gaggcaggag aattgcttga 68700
acacgggagg cagaggttgc agtgagccga gatcatgcca ctgcactcca gcatggcgac 68760
tggactctgt ctcaaaaaaa aaaaaaaaaa aaaagaaaac caaacataaa accagaatat 68820
tcctgtgagt tccattacat aggacaatat ggtcctgctg atctattaaa attatgttta 68880
taaattagag gctcttcatt gggagatata cttaatgcta gatgacgagt tagtgggtgc 68940
agcgcaccag catggcacat gtatacatat gtaactaacc tgcacattgt gcacatgtac 69000
cctaaaactt aaagtataat aataataata ataaaaaaag aataacagga aaaataatta 69060
gaggctcttt actgtattta aggtgttcaa ataattgaag ctagtttagt atctccttag 69120
ccttccccag catgccctgc agctttgacg ctgcagaaca ccagttggtg gctgatattt 69180
agatgatgga aatcttggac acattttcat ttttgataaa atgacatggc actatggtta 69240
cattttacaa atatttttgc ggatgtatat caggtggcat acccaagaag gaacagacct 69300

tcctcagggg tccttcttag gaaagtacag ctttgcttct ctgatcatca gaattggatc 69360
cttcaggtat taggttggtg caaaagtaac tgcggttttt gccattcaaa gtaatggcac 69420
ctgaaagggt tggtaaaatt cagaatatta taaaatcagt aaaagcttaa tcaaatttgc 69480
ctaaatctat cctacagatt agtctatgca tggtttatta aacaacctaa ctgaaacaca 69540
catgcatatg tgtgagtaag ctgacatcag ggatcacctt atcatggaaa atctagggaa 69600
ggttggctca atcttcaatt tgtgaagacc aattccccta ccacgtccat atatacatat 69660
tcccaggaga cttgtttctt cagggctctc tttggttctc agctcttcct ttctgaatta 69720
gtgggtcact ttaacctccc ttgggtcaca gtcgtgttcc acactgggtg ggtccagagg 69780
tagaactcat tgcctttctt tgaatcccac acaagaccac ccaacaaggc attgtagttg 69840
gtggaaaact ccattgaaga aagggtctat attgcaacaa acaagcatct agaaatagaa 69900
tattaaatat ccctcctctt aaaaatccat tgtccaattc agacttcagc ataatgtaat 69960
cttctgtctt cttttctctg tgtttccatc ttgagagcca tcctattatc tgtgtccttt 70020
ctttgttgtc ttaggaagaa tattttggct tatcctttgt tttgttctct ccttcccgga 70080
cctattatgg taaatgtgga atttgcagat aggcttattt gcgctcccca gcctgctccc 70140
ttaatgctct ctggtaagac acttctttga gtcctccgag gcgagttgct gccccccagc 70200
acctgctctt ccccatcgca acccatgaca gtaaacgcac tgcacatact tatcccacag 70260
actgctgtga gaactagatg gaggctatgt gtgaagctgc aagctcttgg taaagtgcta 70320
cagaaattgg tagacactgt tactatgata agatgttcag aaaataatgg atcgattaaa 70380
gtttcactta tctattaaaa gataaaaaac tctatgattg agaccttttt ggaatattgg 70440
aaaatatttt tgtgtgctta ttagcatact gatatgtttc agtacacatt tcatggattt 70500
atggctaagg atgttacttt caagaaggta actatacatc gctctctact agcatgctgt 70560
tctgttcctg agaaatagtg gctgggcgaa gggtatgcaa acatttctta atatctcatc 70620
caataccatt ttcccactac taattattca acacatggaa atgtaggtca aagcattaaa 70680
aaaaaaaact agaacagcaa taccttggtg gttggcaggt gtttatttaa aatatttacg 70740
ttttagcctt attttttaat acttcttttt catatatatt agatatttaa ctaaagaaaa 70800
taataatcat tctcatggaa tactttaaaa tagtagaaac aatcatgaat agaaatgaag 70860
atttaggcct atactaccct aggctctgtc attcttattc ctaaccattt gcataggaaa 70920
tttagattca cccacttctc tttcttagaa taagattcac aggtttttgg gcatattccc 70980
attgcatgcc tgactgggga ggtgttcaaa tccctgcttt tctactcctc agccctgtga 71040
ccctgagcaa tttctgtggc ttggtttcct catctgtaaa ataggatatt aatagaacat 71100
atccctttaa cgttgcagtg atcacattaa ttagttgatg caaaacagtc catctagaac 71160

agtaagccct caataaatgc tagctatcat tatcatttca tagccagccc caccatggat 71220
gggtcatgtg acctgaagca aatttaaatt ctctggattt cagttatctc tcattgaagg 71280
aattaggctc catcagtggt tctcaaactc aagtgagtat cagaatcaca gggagggtgt 71340
gttatgacac tgaaggccgg gccccaccct cagaatttct tattcagtag atccaggagg 71400
gagtgcaata atttgtattt ctaaaaagct cccaagtgaa gttgatggct gatctggaga 71460
cacaccttga aattcttctg cttactctaa acaaaacgtg ctttttttatc ttgagtgcac 71520
atcagaaccc tctggggagg ttaaaagtcc cagtgaaatc acaacctctg cagatggtaa 71580
ccagatctta gtggctttta aagttctcca ggtgcttcca ttgagcagct aaggttgaca 71640
acacttctaa caccgctttc aggtgaaaaa gaactatcat tctaacttaa atgaaggaga 71700
aaatgaattc atcattctga caccgttatc tattctttca tgcatgggct tatcctatga 71760
gaaataaaca tttactaagg tgtatgcatg tttttagaac atccattggg aagcatgttg 71820
tacagcttgt agtaaaacag catggtgcat gctgaaactg ttataggctt tgcaagttat 71880
tttttataaa ttagttttgc tggtgtgctg tttgattatt ggagagtggt gtgaacatgt 71940
ttctatgcat cccagggggt tgctgtcacc tttaaggggc ctctttaaat cccagctctt 72000
tcatttacaa attctatgac cttggatgag tttcttcact tttctgtgcc acaagttctc 72060
agtaaaatgg agataattat agaacttact tcatagagct ttgagaatca tacaggtaaa 72120
gttattttac gtaattagca ctcaaaagtg accattaata atctaacgtt ttaaaatata 72180
ttaataggcc agatgcagtg gctcacacct gtaatttcag cactttggga ggctgaagtg 72240
ggtggatcac ttgaggtcag gggttcaaag ccagcctggc caacatggcg aaactccgtc 72300
cctactaaaa ctgtaaaaat tagctgggca tagtggcgtg tgtctgtagt cccagctatt 72360
caggaggctg aggcaggaaa atcgcagtga gccgagatca cgccactgca ctctagcctg 72420
ggtgacagag tgggactcca tctcaaaaac aaacaaacaa acaaaaatta atagtttaaa 72480
tatttttaat atatttcaat atataaagat gtaattttta ttttaaatat attaaaatag 72540
ataagtttaa aatacataaa gtaaatatat aaaattaata tgttaaaatg tttactgaaa 72600
gtttacattt ttctctgtat tgctaagagc aatttattgc cttatatgtt gtgtaatatc 72660
tagaaaacac acctatgtaa tttccaaatt ataaccacct tggctcctgg acaagtagga 72720
ctgtgtctga tttttttttt tgcatccttg tcactacctg ccactctgag atcacttgtt 72780
gtctttccat atataatcac ctctaggtac acttctttca ttatttctac gcatcctctc 72840
cactttgctg cattcttgtg tgcattcttt tctcccacgt tggaaagaaa tatgcatatt 72900
gagcgattcc aacccccaaa ggcaaaaatt actagccagt gttgaaaatg aaagcaagga 72960
attttttggt tattgttgct gtaacttttt gatgtgcgta catatgtgcg tctttttttt 73020
ccccctaggg gtggaatggg gtggtgttag tcagggatta ctgacaggac ggtgttgcct 73080

actgtgttgg agagactaag actggggaaa gattctccaa ggctgggaaa tgcagacgtc 73140
cagtcttgga aaacagtttg tcgttggtcg ttatgtcaga agaggagtgt attcctggaa 73200
gaaggcccac aaaatatacc cagagcccag gtgcctttct aagacgaatc aacattcact 73260
cacaagttca gctaatatat gacagaaata caagaatgaa caagactcct gacttaagct 73320
tacagctgat gggaaacttg ggaactggac agagaattaa aatacaacgt ggttaggtac 73380
aacttacctg cgccagagga gtgacggaag gccttggacg gggagtgatg cctggaggat 73440
gacagggcga caggtaagtt agggggctgg gaaggggaag ggcggccctg tgggtaaagg 73500
gaaggaggtt aaagtcagca gggaaacagg cagttccctg ttgctggagc aaagcgcggg 73560
ggtgtggctc tcctgtctct ggctttgctg tcaacaggcg agattggcaa gtagggcgac 73620
caaccggcct ggtttgctcg ggaccggggt ttcctggaag gtgggacttt caatgctaaa 73680
actgggacag tcctgggcaa acaaatcagt acggttggcc accctatctt tgtgtgggca 73740
tctgctaact gagtcatggg cctgtctggt ttatttattt attttttttc tgcctgagta 73800
acagaagaat gtgttaaaca tatggagtat ggaagaagaa ggagagggga gataggattt 73860
ttatgttata gattggaaga agacatcctt gatgaaggag atgtttttac tttggagaag 73920
gactgcctca gccgcgaaag accaaggggt ggcaccacag ggagaagtct gcactgcgct 73980
tgtcaggacg gccagtgggg ggctgctgag cgcagggagt cagtcctcgc aatgaagata 74040
tagtctcggg gagataatgc tggaaatcga tggaagaagt ggaacaggac acaaatttag 74100
tggaatttct ttacaatgtg tgcttcttgt caccagttcg ttaaccatga taaaaacggt 74160
tatgttgtta cattcatcaa gatccaaatt ttctaccatt ttaagatgtc tatcagtcac 74220
aatgatgatc actgaagacc gaacgagatc atttcctcta aaaattatgt tacatttatc 74280
cataatggca tcagtgtctg ttacacgttt gctttaattt tgaggtcaaa tacgacattt 74340
gtaaatctag cacttcatgt gattctgtaa gcactaaaca gctaaacata tttacttctt 74400
ttttttgaat caattagaac agtgctgtac aatagaaaaa tagaacagtg ctgtacaatg 74460
gaaacagtgc tgtacaacag aaatctgtca gccacacgtg tcgtttaaac ttttctagta 74520
atcacattaa aattgtaaaa agaagccaat aaaagtaatt ttaataatat attttgtttg 74580
acataatata gctaaatatc aattttgaca tgttctaaat ataacaatta ttaatgaaaa 74640
atattacatt cttttggctt tgtactaaac atttgaaatg tggtgtacgt tttcactta 74700
tagcacacgc aattcagatg ctacattttt attaggaata tttaatctgt agatagatat 74760
cgtaaaattt acatttgaaa aaaatagatt cagacaccta agttgttaca agcatactta 74820
aaagttttca atgactgaat tgagtatcag ttttaaaatt taaatcaatg aaattaatta 74880
aaatgaaatg aaaatttaga aactattcct cagtcacgct ggctacattt caagtgcttc 74940

atagccacat gaaagctgta ttggacagca aagaagtaga atatatttgg aataaaaatt 75000
ttaaagtgga cacattgtgt tactctcgtt agccatgcta ttgctatttt ttttcctata 75060
gctaattaaa accttaagat ccagtaggtt ctccaccttt ttttaaagca ttagttccat 75120
gtcgaccctg tagatggcag cactttcttc ctaaaactac atggaggagt tgcctgggct 75180
tgtcactcag attctggcac ttttcataga aagagtctga attatctgga aaattctttg 75240
gtaacatagg tcagaatctt ttcagctcta ttgttattct gcacagatgg ctgttgctta 75300
tgaaaacaat ctctcagcct ctagtccagg atattactca ttcctcagtt caagaaactc 75360
tagggtgaga ggagaaaggg gttcaattac agaactgtta tcaaaatgcg ttggtttatg 75420
cacatccgtg ttttggacat ggtgattcca agagactctt aataaaactt ttcaaagtag 75480
atgagagaca gtttttccct cacatgctgt ggcaatatta atctatgttt tcatgttcca 75540
ctggactttg taattgaatt tttaaggaatg catacagggc ttcatattta tatataaaat 75600
atccatatcc agtgttgaaa gaaattaaca ataaaatatg tacctgtata aaatttgtga 75660
tttttgaagc acccctctct tcctcttcgc attgcatttg tggtaggaga acatgagaca 75720
aggaacgagg tactaaggac aaagaaggag cgattcagag gtggatttcc tgaagaccaa 75780
caacattttt gcatagctcg tgaggactct ctgatactaa actccaataa atgatggttt 75840
ggtgttttga ttgcttttga ttgacattta aaaattacag gagactgaga taggaggatt 75900
gcttgagcct gggaagcgga ggttgcagtg agccaagatt gcaccactgc actccaacct 75960
gggtgatcga gtgagacccc atttcaaaac aaacaaaaaa ataaaaaata aaaaaaaaca 76020
aataaaaatc acacgcctct ttttttcact caatctgttt tccaaataaa atatcaagat 76080
tctatttgaa ttttaataat gattttgctg agttttatag cttgaatact atggcaagta 76140
taatgtctaa aatgctgtga ttttgactta agaaaaaatt tacagttttc ttaatatact 76200
ctttagttct tttaaactct aatacatgaa atggattgct aatgagggta ggaaggggga 76260
aagactgggg agaaaaatag ctaacttttc tagcgataga tacatgtcag aagctgtagt 76320
aggttctttt gcatgtgttt acttacatta ccttctgtaa tctgcattgc aattttttca 76380
tagacaagga aacctagact tagagaagta caatacctgt catctcgtaa atgacagata 76440
tagttgagca aatctactga aatggtttag cttcttatct ttccagacaa cagataagga 76500
cttattctat atgaagacat ctgagaaatg aaaatatggt ttcaggttca tagaatcttt 76560
tacaccacgt atttctggtt ctctttcttc tattcagatc attctttcat ctgtttcttt 76620
tgtcttcttt tccaaaatgt agcaattgcc ccaaattcag tacttgtctc tcttcttaac 76680
aatcacatcc aatcccatgg cctcaaccat cttcccttgc atataatttc cacatttaca 76740
actttattct cttgattact caggataaga taattgctga acaaataaca gcatatatct 76800
cagcttaaga gagtaaaagt ttatttttct ctcatgcaaa ttctattgca gatattcccc 76860

acctccttcc ctaggttgtc ctcaaaatgc agtgactcag ggaactcctc tttctgtctt 76920
aacctatttt aggtcttaga cccctttggc agtctggtga agccaataaa tctcctctca 76980
ggacagtttc taaatgtgat aaaagaaaaa aaagatatag tgacagatct aataattaaa 77040
attcaaagtt aatttcaaat tagtgatagg taaatatatt tcaagatgtc tgcactgaat 77100
acagcgtgat aggtaatata tatgatttat cttggtgaca aagacattgg tattgctaac 77160
actattatag ctgccatctg tattcacaaa gggagatgtc aaattccagt taaaagaaag 77220
attttcttaa cccaagttca taggacttcc tgaatggtac agatgctaag gtctagtggc 77280
tccacaacta ccaaagcctt cttagtgttc tctgctggat cttatgcatc caattgactg 77340
acaagcaaag aacctgcaga ataataggag acatttgttg ggtggggaga gcaaacctag 77400
aaggggcata taccccttac aattatagct ctatgcgcta gaattagtta tatcattcct 77460
cctagatatg aggggactag gcactgtact tcagctgtgt gtccaggaaa aaggtaaggt 77520
atggggaatg tggagcaata attatttgaa gcaagaatca ccagtggatg ctaaaattcg 77580
tgagggaaaa caggaccaga aacaggatat ttttaaagtc tccaagtacc tccacatagg 77640
atacttatta attgcaaagg gaaaatagta actttacaga gaagaagaaa tctggcagac 77700
actaccatat ccaagtgatc aaaggtgaca tcagtagtgg gacacattaa catcatgtgc 77760
ttctgatatg atacacaaag gagaaacaac ctcattcttg tgatgttcct gccaaaaatc 77820
cataacctga aagaagcatc acaaaatccc caattgaggg gcattccaca aaatagctgg 77880
ctagaactct tcaaaattgt caaggtcatg aacagttcca gaccaaagga ggctaaaaga 77940
gacaggatgc ctcaatgcaa gtataatcct gaattgggtc ttgggtcaga aaatgggcat 78000
tagtaagaca attggcaaaa tttgaataag gtcaatagat ttgaaaataa tactgtacct 78060
atgtgaattt cctgactttg atcaaagcac tggggttaag ttaaaacata agatgttgtt 78120
tttggaaata catgctgcta tatgcaactt actctcaaac atctcagaaa aaaaaataga 78180
taagttcata gatagataga tgacaggata ataaagcaag tgtgataaaa tgttagcatt 78240
tggggaatgt aggtaaaagt atatgggaat attttgtacc attttctcaa cctttccttt 78300
aactcgtaaa ttacttaaaa gaaaagcaat aaccctggct cttataaaac taaggaaaat 78360
ggtccatcct ttcctgtgcc ttgtgttttc ctgctggtca aagaaccttt ggtaatgaca 78420
atctgagttg gtcactgata ttctagtaaa ttcagaatat aacttcctgg gctatgtaga 78480
aaaatatagc tcttcatatt ttaattcatt ttcctgacag cacgtttctg tgcttttatg 78540
tatggatact gcctagtaat gtgcacacca gtcctgcaga tcctggaaca ttttattatc 78600
ttgatattga cttacgtatt taggtagatc tgaatagcag aattgtcagt cgtttcacca 78660
agacattatc gggaaatctg gacttggaag tctacttctg ccaggcatct ctccacgctg 78720

gcttctgggc cacagaagaa aagatctaat caaactcatt tattaacaaa ggcttataat 78780
attgaatagc agcttccagg aatctaagaa aatgttcact tttatttctg actaaggagg 78840
aactattata gtctttttat accagttaga agtgatgact tggtattaaa cttattctaa 78900
ttctcaatag ccctgatctc gactctgacc acatgcgcgc acaccctccc ccaaccctgt 78960
aagtgtgatt atggcaaggg gaaagttaaa gtggagtgaa tggtgagtcg tggaatgaca 79020
gagagcattt agcatttgta attgacagag ttggtgattg actgaatgga cactgaatat 79080
ctaggctaaa agtaattgaa tgtggatggt gtagtgagat ggagaacact agcaggaaga 79140
tagactgggc tacgcagggg ctagatcatg tgttcagtct gggatatact aagtttgagg 79200
tttctgtaaa atgtccatgt gaagatgtcc aattgggagt tggatattta tttatgtttc 79260
cattcccatc aatcgataag cttcagagga aataagagtg taagattttt ttgaacaaca 79320
ggaaaggaag attgtagtaa gatatgatgg cataatggta tatggaagag ttttaaataa 79380
aagttagcca ggcatggtgg cacgcgtctg cagtcccggc tactcgggaa actgaggcag 79440
gagaatcgct tgaatccggg aggtgaaggt tgcagtgagc caagatcgca ccactgcatt 79500
ccggcctggg cgacagagtg agactccatc tcaaaaaaaa aaaaaaaggg atgaagaggg 79560
atgaggagaa ctcaagttat attagcctga atggaataga tgagtggaga gatgggcatg 79620
ataggtgagt taagaaggaa attctttgcc tcagcaacca actcagatgc tttgtgaatc 79680
taaattatgt gttgacattc tagtagacct agacctggga taattcactt cagcattatg 79740
taatcttaat ttttcattta taaataagaa aatattatgc aatacttgcc atagttttct 79800
aaaagctgaa ctcagaatgt taatgaaatt aaaatgggaa agctattttg cttagaagat 79860
attttgttat aaacatttct taaatgaaga ggctctagaa atatttattt tatagaattt 79920
aaaatatagc catcactaat aaaggcaggc ataattccaa cgatttcata acacatcagt 79980
tattaaatct aattaatata taaatgtaac taatatgtaa tttaatataa ccctaattca 80040
atgtttaacc aactgaagct ggttaaacta tgaagaagaa tcttcaaatt gaccatttct 80100
ttccaagcgg taaaggtgat acgggatttt gtctatacat atagagcaaa ggagaattaa 80160
gccaagattt agaattttag gcaaaaagtg tttatagtat cattaagtgg ttacatatgt 80220
aattttactg ttataaatat gactgagtca attttttttt caaagatttt gtcacaatgg 80280
gaggcacaag gagtttagac agatgaagac tctttctaga aatctaccta atctatcaca 80340
ttagacctgg agataattgg tttgacaatt tcagttgttt ctcagcaaaa aaaataagaa 80400
actaaaagtg ttcagatggc agagaaaaaa gtttgaagag gagagaaaga agaacaaaaa 80460
gaaaaaggaa acagtagaca tataaagaaa gcacctaaac cagtgagatg ccccagctat 80520
gcaccagcag catggtggat tacagtagag tttttctaca gaactattgt aaatcactgg 80580
aaaatagtgt ccaacttatt tgcttaaaat tttcagttaa tgtgctccag cagcattctg 80640

tagacaacga ctctcactct ccccttatta tggaggcaga gctgtttctc tttgtcccgt 80700
tctctagcct gtgatatact ttttattgct caagagctat agaattgtat ccatgcctat 80760
tgttgtcctc ctgatgggaa ttcatgtaat tgaaagcgtt tatcctctct tagtagaaaa 80820
attgggcact tggaggcaat gaaaatcccc cacctttgtc cacggcactg gaaatactct 80880
tcagtctgct ttgttctaaa gtttctactt ttcccagtat aacctgttga aaatttagtc 80940
cttcatgaga aatacggctg gaatttcttg tggcaggctg gccgccggtg ttcatgtagt 81000
tacacctttt gttgctattt caccactgat caaaaaataa aattgtattt tttcaaaaga 81060
aacatggaat gacatgaaga aagtccttaa tatatcatgg agtgaaaaac agcaagttgc 81120
catgcaaatg tcaaatagat cctatatttt taaataagca actgcatatg tatcatttaa 81180
atatatatgt ttagatattt gctaaattat gaaccgcagt taccccctggg gagtgaggtt 81240
aggaaaagta aagtgagatt ttcacttttt acattatact ttttacttta cccacttctg 81300
tattgtttga atctttaaaa tgaacaagtg ttattttgta attaaaaaaa acaacaactg 81360
acaaataact atggggagta agaaattact tggaatggaa tatgtttaaa taattaattt 81420
gttgatacac tgcttgacta ttattttctt ttgctaaatc aaaactcccc tgcttttctc 81480
aggaggttgt tttgaaaaga agggtaaatg tttctaatga aatgaacagc tcaaccttcc 81540
cacctcctgc ctaaatgcct cattttggtt ttgttactaa caaatgcaac acgcaaggct 81600
ttatggtaaa gcaagcaatg tgggtttcta acagggcagt acacacccgt agttgctggg 81660
aaaccacttt agtaatggtg gatttattga attggaacag gaatggagat ttggaacaac 81720
aggaagtgag gtacctgtag ggtctcagt tgtcctgggc actgtgacat ggcacccaaa 81780
tgtcatgcaa gagtggccct tccaacatat gcaccaatct gaatctctcc aaagtttctt 81840
gattacacca taaaacaatg aagagaacag cattgccaat aacttcagta ataacttttg 81900
catcatatta caaagggtgg tgggtggcac tttgtgtggt catgggtatg tccaaaagaa 81960
gggagtggtg gctagactca gttctccttt agattttaag gcaaaattta gcaaactcta 82020
tggcagattc ttcaactatt tgtcagacag acattgtcta cttttctggg gatttatgat 82080
tgaggcccct tttgagggga gctttgagtg cagtgaggcc agatagaagg gccaagaagg 82140
atgggagcag ctgcatgcta gtgagcaatt caaagttgga ttgtgctgac tggaaaatcc 82200
cggttagcaa atcaactatc acagacttca tattcttttc agatgtgcag cctttataac 82260
atatttgtaa atgctaaagg actgctccac agggaggtta caaagagaaa agctctgggt 82320
ggttgtaaaa tggacaaatt gtaaagcctt actagtagtc ttaccattcc tctcagctca 82380
taaaaaagtc ctcctcttca tttgactatg cctcaaatct actgcttctt ggattattaa 82440
acactctttt cctccaggat cttaaaatct ctctgttata agttgaattg tgtccccctc 82500

aaaaatgata tgttgaggtc ttaaacccca gtatcctaga atgtggcctt atttggaggt 82560
ggggtcttta cagggttaat ggaattaaaa caaggccact agggttggcc agtccttttt 82620
ataaggaatc cagtatgact ggtgtcctta ttaaaaggga aaatttggac agagacacac 82680
acatagggag aaagacgtga tgatgaaggc agagtggggg gtgatgcttc tataagccaa 82740
cgaaccccaa agattgccag caaacccccca gaagctacag agaagcatgc aacagatttt 82800
ctatcacagc cctcaaacag aaccaaccct gccaatgctt tgattttgga cttttagccc 82860
ctggaactgt gagacaatac atttttgatg tttaagccac ccactttgcg gtgctttgtt 82920
ccagcaacct tgggaaatga ttacactttc tcatctgtga tcacagtgag gccttgtcac 82980
aatcttcact cattccttct atagaaagct gaaagcctat ggacaaggat aattgccttg 83040
gctgacgcat atgtttgaag gcagctgctg gcaggcaacc ggagacttta ccctggtcct 83100
agtggagagg aagtctatag gaggaggcag ggagccttcc tggctggcaa cccagtgact 83160
caggttgttt tcttactcca aggatctctg tgcaagtagg aatcctgaca tcttctcttt 83220
ttcacttgtt tatgagaccc tgcttgttgt agagtaaaga gcagaactta agaatggaat 83280
ctaatcacct tgaaagaata atctctcatc tcctctggtg gcaaaggatt gggatgtgga 83340
ggttatagtt ggaaaattga ttcctaggtc aatgtctgtg ggtgaatttc tctttgaatt 83400
gttatcagca atgttatggt gttcggtggc tttcaacgaa ataaacacac ctgacatcct 83460
tagtaaagag aaagctttct gaaataagag tgagggacag actgggacat gatgctttgc 83520
atgaaatagg tgttcagtaa gtatttgtag agttggaaga agttagatta aacagggcta 83580
ttgtatggca cattgtcatt agggagttag agagggcttc tggaaaggtt tggtcagttt 83640
tgacttacct tgcaataaat ttggactata ataaccattt cactagtttg taaaaggtac 83700
tggcaaccaa agatggggaa tgccactcaa tctcacttgt tctactttca aaagcttgct 83760
ttggtcacat tgaaaggttc catttggtga agtatagcat atcacagaaa acaatggagt 83820
ttgggagttc gggaacaaat ggttgttttg tgataatgaa taactttgac atttcgtttg 83880
aagttaagtt atgttgtttg aacttttctg tccctgaaca aggcatcgta tgccaagaac 83940
aggtaacagt ttctggatca ctggggactc attagtcatg gatggttagt ggtactacat 84000
tgtggtcttc cttcaatatt gaaagctgta agaagcttgt gacaaattct taccttggcc 84060
ctcacacagt actggtaggg ctgggatgtt attgcttttg tccaccaact tccaactgta 84120
gaagtctagg atgatcacag cagttagaga tgatccattt agatagtcta tattaaggga 84180
aagtatcctt catccatggt gttgaacatt cgtaggatta aaatgaaaga agccaaaacc 84240
tttctagatc tttattggta tatcaacaga attagctgat taggtatgca gaaaaaactt 84300
gggacttgaa gacaccatta aataaataac tgtacataac catacattaa ataactgtaa 84360
caccattata taaataacta actataaata aagagctcta cattcaaact gcttttcact 84420

gcatttgtca gatttcactt ttaatataat tttagttgac ttatccctca tcttacctta 84480

agaagataat gagttgaggt ggatcttgag ccagtgttta gtccaatata gccctttgac 84540

ttggggaaag atcaaacttc gaaatttata tgcatgactc ctctctgggc aaggactgaa 84600

gtggcaggag aggtgaaaga aggaatcagg acaaaaagta gatgctaaaa ggaaaacagt 84660

ctgtcccgtg gagaggaagt acccaaagaa acagaagaac tccatgatgg agagtaatac 84720

aagttgaata tcccttatcc aaaatgcttg cgaccagatt gttttggctt tgggattatt 84780

tcagattttg gaatattttc atacccgctc agtatcccta atctgaaaat ccaaaatctg 84840

aaatgcttca gtgagcattt cctttcagtg tcatcttggc cctcaaaaag ttttggattt 84900

tggagcattt tgaattttgg atttttggat tagggatact caacctgtac tagcgtgtta 84960

aaacagtcct ggccaggcgc ggtggctgac acctgtaatc ccagcacttt gggaagccga 85020

agcgggcgga tcacaagatc agaagatcga gaccatcctg gctaacacgg tgaaaccctg 85080

tctctactaa aaatacaaaa aaattagctg tgccgtggcg ggcgcctgta gtcccagcta 85140

ctcgggaggc tgaggcagga gaatggcgtg aacccgggag gcggagcttg cagtgagccg 85200

agatcgcgcc actgcactcc agcctgggtg acaaatcgag actccgtctc aaaaaaaaaa 85260

aaaaaaatcc aaaaaaccca aaacagtcct agaggctaca gcctagagca gtgggcaaag 85320

agaagcagaa tctacagaag atattgttca aggcctgcta tgctaggtct cagatagcac 85380

cagaactggg cagggcaaga aacgtcaggc ccaggggtct ggtttaacca gaagaggtgc 85440

tgaattttag gatctgaata gcaagggttg tggtcaggaa gtcagtttca tgtaagaatg 85500

aaggtgagcg gatcttaaat ccctgaaagg aggcagagag aagaatatag gaaatgggtg 85560

gataattttt gttgcgagta aaaatgttca ggactttgga ggcaggaaga gcaggtaaag 85620

caagttcgag gaaagaggct gggtcagagg ggatctgaaa gcaggagtca gaaacatcca 85680

ttggaggcct ggagaacata aatgggaggt gaatagactg gggacatagt gcctgggtgg 85740

aaagaagctg gctcgagtcc gaaaccacag gctgaaatat taggaaaaag agacccagga 85800

attcagttca gaataggagg gatttgtggg gcaagaccag tggttcttcc agacatttta 85860

tcagagataa caaagcagag gctgtctgtt ctctgccctt ggtgtgagaa gaaaaggact 85920

gacaggagtg cttggcagct caagccagtt atggagctgt gggtgctagt ggctattaac 85980

taccagggaa ttagctctgc tgaatgagtc ctcacgcaga ggcagggata gaacgttgca 86040

ttcagaagac tggccacgga ggccggctgg ggtggctcac gcctgtaatc ccagcacttt 86100

gggagaccaa ggcaggagga tcatctgagg tcaggagttc gagatcagcc tggtcaacat 86160

ggtgaaacca tgcctctact aaaaatacaa aaattagcca ggcttggtgg cgggtgcctg 86220

taatcccaac tactttagag gatgagagag gagaatcgct tgaacccggg aggcagaggt 86280

tgcagagagc cgagatcacg ccactgcact ccagcctggg caacaaagag tgaaactccg 86340
tcttaaaaaa aaaaaaaagg aaaagaaaag aaaagaagag tggccacaga ttggcttggc 86400
tggagaaggc attttttat agggagatat acgtgagttt gacgggatgg gctagcaggc 86460
ggacattcag gttttatggg ataggcaaaa aggtacaaat ggtatttggg gggatgtgaa 86520
tggcatctga gaaaggttga tccaggcttg tgtggaagct ggctctgagc ataaaatgcc 86580
agagaaaggc ttgtacaact ctgaggtctt ttcttttctt ttcttttctt tttttttttg 86640
agacagagtc ttgctcttgt cgcccagcct agagtgtagt agtgtgatct cggctcactg 86700
caacctctgc ctccctggtt caagcgattc tcctgcctca ccctcctgat tacctgggat 86760
tacaggcacc tgccaccaag cctggctaat tttttgtatt tttagtagag acacggtttt 86820
accatgttgc ccagggtggt cttgaattcc tgacctcaag tgatctgccc acctcagcct 86880
cccaaagtgc tgagattaca ggtgtgagcc accgcacctg gcctattttc tattttttaa 86940
ccagtcccaa gtaagggtat tggttagggt tgaggcaatg gaaatctgaa agaaagggtg 87000
agttaggtat ttcactggac acatgtggag aatgttggtg atttaatgca gagggaattg 87060
tggttaaaaa ggttaatgta agaagggtca tgtggggact ggaaatcaca atagagaaac 87120
agaggccaga atgctgaact cataggaagt gggtgaccat gggtgattaa aatgtacaat 87180
agggctgggt atagtggctc atgcctgtaa tcctagcact ttgggggact gaggcagttg 87240
gatcacctga ggtcaggagt tcgagaccag cgtggccaac atggggaaat cccaacccta 87300
ctaaaaatac aaaaattagc cgggcacggt ggtgcatgcc tgtaacccca gctacgtggg 87360
aggctgaggc aggagaatcg cttgaaccca ggaggcagag ggtgcagtga gccgagactg 87420
cgccactgca cttcagcctg gggaacaaga gcgaaactct gtctcaaaaa taaataaata 87480
aataaataaa aataaaaaat taaaatgtac aatagagtgt gtaaaaccag gaaatctaca 87540
tcttgacact taactaccca agacgcaatt ggttcctcgt ctgattttta aaagaagttt 87600
ccactttctt cggaaaatca cgacagtcct tgttctcaat ttctttgttt ttaagaagag 87660
aaatgttgta tgatttcact tatataaact acctagaata ggcaaattca cagggataga 87720
aaggtggaat ggaggtgacc aaaggtggaa aggaaggagg attgttcaat gggtacaaca 87780
tttctgtttg ggatgatgaa gaatttctgg agatggacaa tggtgatggc tgcacaacac 87840
tgggaatgtg cttaatgcca ttacttaaaa atggctaaaa atcatgcatt ttatgttatg 87900
tatatttaca actttttttt tttttttga gacagtgtct tgctctgtca tccaggctgg 87960
atggcggtgg catgatctcg gctcattgca acctccacct cccaggttct agcaatgctc 88020
ctgcctcagc ctccccagta gctgggatta caggtgtgtg ccaccatgcc tggctaattt 88080
ttgtattttg agtagagatg gggttttgcc atgttggcca ggctggtctc gaactcctaa 88140
cctcaagtga tctgcccacc tcagcctccc aaagtgctgg gattacaggt gtgagccacc 88200

atacctgcca tatttacaac ttttaaatca taggaaagaa accactaatg ctttgacaac 88260
tgtgagattt tgaatctcaa ctgtgagatt atggtttgtg gaaatttctt tatgtgatta 88320
aaaaagatac tttttttgcc aaaaaatttg ttttcgctgt catgcaagtt ctcctgggta 88380
agcacctttg gtcttgtttt gtgcatagca tacagagcag caattgtata ttgctaaaga 88440
aagcctgttt tttttttctt gttaatttat ttaagttcct tgtagattct ggatattacc 88500
ctttgtcaga tggatggatt acaaaatttt tctcccattc tgtaggttgc ctgttcactc 88560
tgatgatagt ttcttttgct gtgcagaagc tctttagttt aattagatcc catttgtcaa 88620
ttttggcttt tgttgccatt gcttttgtgt tttagtcatg aagtctttgc ctatgcttat 88680
gtcctgaatg gtattgccta ggttttcttc tagggttttt atggttttag gtcttatgtt 88740
taaatcttta atctgtcttg agttaatttt tgtataaggt ataaggaagg ggtccagttt 88800
cagttttctg catatggcta gccagttttc ccaataccat ttattaaacg gggaatcctt 88860
tccccataag gatatgaaca gacacttttc aaaagaagac atttatgcag ccaacaaaca 88920
tatgaaaaaa acctcattat cactggtcat tagagaaatg aaaatcaaaa ccacaatgag 88980
atactatctc acactagtca gaatggttgt tattaaaaag taaaaatata acaggtactg 89040
gtgaagttgc agagaaatag gaacacatat actgttggtg ggagtgtaaa ttatttcaac 89100
cattgtggaa gacagtgtgg cgattcctca aggatctaga actagaaatg ctatttgacc 89160
cagcaatccc attactgggt atatacccaa aggattataa atcattctac tataaagaca 89220
catgcacacg tatgtttatt gcagcactat tcacaatagc aaagacttgg aaccaaccca 89280
aatgtccatc aatgtttgac cgaataaaga aaatgtggcg cgtgaacccg ggaggcggag 89340
cttgcagtga gccgagatcc cgccactgca ctccaccctg ggcgacagag cgagactccg 89400
tctcaaaaaa aaaaaaaaaa aaaaaggaaa tgtggcacat atacaccatg gaatactatg 89460
cagccataaa aaagaatgag tttatgtcct ttgcagggac atggatgaag ctggaaacca 89520
tcattctcag caaactaaca cagaaacaga aaaccaaaca ctgcatgttc tcactcacaa 89580
gtgggtgagt tccataatga gtgggtgagt tccacaagtt ccacaagttt cacaagtggg 89640
tgagttccac aagttctata atgagaacat atttgcacag ggaggggaac atcacacacc 89700
agggcctgtc ggggggttgg gggtcaaggg gaagaatagc attaggagaa atatctaatg 89760
tagatgacga gttgatgggt gcagcaaacc accatgacac atgtatacct atgtaacgaa 89820
cctgcacatt ctgcacatgt atcccagaac ttaaggtata ataaaaaaga aaaagaaaaa 89880
gaaaaaaaac cctgcctttt gctttggcag tcatcattcc ttccatcttc tcatgttttc 89940
ttatttaact cagtagttct caattgacag cacaaaagaa ttactcagga gagttcaaaa 90000
aacacctatg gctgactccc ccaagattct gatttccttg gtgtggggta gcctgggcat 90060

gaatattatt taaaaactcc tctcatgatt ctaaggtggt agccaggatt gaaaaatgct 90120

ggactttcaa gtttttgttt tctttctttt cttccaaaaa ggtgaagccc tgacttggga 90180

agaattcaaa tgcgagttag gcttatgttt gatgtcactg attcctaaag atcagtccat 90240

tgattctcct cattctcagg agagcatttg gtgtcaaaat cacagcccaa aactcttacc 90300

ctagttcagc atctaactct cttcagtcct gaaactgttt ttgtcactct gtccatataa 90360

tcacagtgtt gtagaaattt ccagctaaac attgtctcaa actttttgta cctagtttta 90420

aatcaaaatg tagtacaaaa ttattaaata caacgggctt taaaatattg agattttggt 90480

gatctttttt gtagaatgca aatactaatg aaactaattt tttcttttgc tttcaaacta 90540

tacatttctt ctaaaacttc tttcaaagta gaatttcata agtggaagaa cccttgaaga 90600

ttattttaat agaattcctt aattttagag atgaggaaac tgagactcaa atatattaat 90660

taatttatct aagtgtctta gtccattttg tgctgctata agagagcata cttgagactg 90720

ggtaatttat attgaacaga aatttattgg ctcacgattc tggaggttgg aaagtctgat 90780

atcaaggtgc tggcatctgg caagggcctt ctggatgtgt ctacatggtg gaaggcggaa 90840

gggccaagag gtaaaatgtg gctgaatttg tgcttctatt atggcatgaa tcccatccat 90900

gaaggtggag gcctcatggc ctaatcattt ctcacaggca ccacctttta atactgttac 90960

aatggcaatt acatttcaac atgagctttg gaggagacaa aaattcaaac catagcactg 91020

aagttataca actagttaag aagagagaac ctgagactga aaccctgatg ttttgactcc 91080

caatccagaa tgtttgatcc cctacatccc accgcttcgc tgtttccatt cctttctcct 91140

gttttcatct attttcacta agagggcaag atatttgcta actgctacac acccaaattg 91200

tataccaggc aggtcagaat gactctaaca ttttatttaa tgaggcagaa taaaatatct 91260

tcctactaaa cagtgacaag tgtttgtttt aaaaaaaagt attgtgcaca aaaaaaaaaa 91320

ctactttcag cataggctat attttacaaa ataaatttac agtgggaacc acagagctat 91380

ttagagtgtc tggtataaat gattttattg ttactctgct tttttgaaaa agaagtgtgt 91440

gaagcagagg taagtagtac tgaggaaata ataactaaaa tactaaggac gagatgcaca 91500

tgggaaaagt tcttcattaa ctgtaacgtg gtggagaaat gtatgttcta gacaggtcaa 91560

tcatggaatt agaaaaagtt gggatcttgg attgttgagt tcaaatcctt agttttaaaa 91620

ctgaagactt caaattttga ttgactacag atcaatcaac aacctgttgg agttggaaac 91680

ttgaatctga ttctccatcc ttgtttggga tatgaaaaaa aggaagacat ttaagagtat 91740

ttttttattt atgaaactaa tgtatcatca tgtaaaaata tgaaatagaa agtgttaaaa 91800

attataaaga aacaagaatg tgtttttata atctcatttt cttactttac gtaagatggt 91860

aagtctattt tggaattaat tgtttttaag aatagtatta taattcaatg ttagataaag 91920

atttatttaa ccctttcccc tttaacaagt gtttgggttt tttaagtttg cttccaagtt 91980

ttcggtaaat gatgcttcaa tggatatctc tgtttgttat cttagtatgt atcttagatt 92040

aattcctcaa gataaatatt taaaggtatg gtatatgtaa tattcttgat acatacttcc 92100

atatttccct ccagaaatgt gtgcacctat ttaaatgtgt gtggaggtgc acattttaac 92160

attcctcatt attctggctg ccttctataa atcaagctac cctggtatta tggctctcct 92220

ccagaaatct aatcatttaa acacatgaag tgaatgtgtg aatggacaat taagtgctgg 92280

ccaaatgtgg aaaaatgtta agtgtaaatc aaaggcaact gaaacgttaa atacaccttc 92340

cattcctccg ccacatccat gctctttccc atactccaca tctgctcact aaaattggaa 92400

ataaactgta atgaaccaga aatgtctgac ttactggtgt gtttaggtga gttttattga 92460

aacactgact gaccccacca ctctaatacc atcaccttaa tgtttgtatc ttagattaat 92520

tcctcaagat aaatatttaa acgtatggta tatgtaatat tattgataca tacttccata 92580

gttccagcaa ccacttgtca tcctgtgtcc cccaaggaca ctgcaatgac tgtcacagtg 92640

acttgtgctc ggttgaggag caagtggtgg ctgaaatcca gccccagtca gctccccaaa 92700

ccaaaccact tttacacagt gtctatgtgt aaagttggtt tgctttgggg agattgatcc 92760

ccataaggga tcagcaccag ccctgtgtct atctcttctt ctcctgggga gatgttggct 92820

attttgctag ctggagaagg gcacgcaatt tgttgtcagg agacaggttc ttaagttcca 92880

ttggtacatg cagggcaaga caggcagagg gaatctgtca ctgctacagg cacttcataa 92940

gcccaggtat agacgataat tgaaatggat tgatttcata gtagtctaat acagagtggt 93000

gctacaatct ctgtatgtgc ctatttagaa aataacttca tatttaattc ttcactttac 93060

ataaaagata tgtgtattaa atacaccttt tattttagaa cagttttaga ttttagattt 93120

aaattgggaa gacagtagag atttcctatc taacccacac ccagctcccg tattagtaac 93180

taacatcttg cattagtatg gtatattaca attaatgaac caatactgtt attttattat 93240

taacttaatt catgctttat tccagttccc ttagtttcta cccaatgtcc gttttctatt 93300

ccagtatccc atccagaata ccacattaca ttctgttgcc atgagtttct cagattttcc 93360

atgtttttga tgaccttgac agttttgagg aatactggtc aagcgttttg tagaatgtac 93420

ctcaactgag atttgtctca tgtttttctc atgattaact tggtattaca ggtgttttga 93480

gaggaagacc acagaaatga aatgccattc ttattacatc atatcaagca tatattctaa 93540

caatatgatt tatgacaact aatgttgacc ttggccaact ggctggggta gtatttgtct 93600

agtttctcct ctgtaaagtc cttccttacc ccctttccac actgtactct ttgaaggaag 93660

tcactctgca cagctcacaa tgaaggagtg gggagctatg cttcccctct ttgagagttg 93720

tgtatgtaca caaattattt ggaattcttc tgcatgggag atttgtctat tctcccctat 93780

ttatttattt attgaattat ttatatcagt atagcttcat ggatatttat tttagacttt 93840

gggctaaaaa acaatactac tttattttat tgttcaaatt gttccagctt ctagaagtta 93900
tatttttata gagaagatat gtttttaaat ataaaaacac tttaaaaaaa tgaagtcaac 93960
aggataaaat atttttaaat tttccttaat gggtgttctt tgtaccatgt ttccatcaaa 94020
taacattctg attgaaatct aaaaattagt gtttgtattt taagtgctaa gatcagagaa 94080
aaagtcacaa gtttcccaga ctgagttagg caacatgtga ctcagagtta taaggaatac 94140
ctttcccatt cctgcattgc aaccagtgtt acagttacag aagtgactag tgtaacttcc 94200
cctcctgggt tccagctgcc aggacttact ttagttcttt taacatatcc ttcccaccaa 94260
agataagttt tttgaacagt ttccccatga gtgctccccc caccccaact tgtgcctttt 94320
attcatgtct ttataatatt tattggttgg ctggtgcaat tgcagaggtt ggcaagtctg 94380
aaatttgtag agcaggccag caggctggaa actcaggcag gagttaatgc tacattctag 94440
ggacattttt ttcttctctg gaaacttcag tttttgctga caagcttttc aactgattgg 94500
gcaaggccca cccatgttat gaggggggaac tgtacaatca gttgtcattt aagccaatca 94560
gtcaactgat ggttggtgtc aaccatatct tcagaatacc ttcactacaa catctagact 94620
catgtttgat taaataactg ggtgccataa ccttggcaag ttgacacctg aaattggcca 94680
tcacagtgac caagacagaa agtgtaatga gcaggaaagc accccagtga caggacagtg 94740
aaagatgagc ctggagacgg ggagtgggag gagtgctttg acagaatggt caagagagat 94800
ggcatctggg tggagatatg aaaagatgta aaagaaggga aggaaccagt tctgccaaga 94860
atttatactt ctacaatttc ccagatgata ttgatgctgt tgccctggag cccatacttt 94920
gagaaccaca gagtaagggt atacctctgt tattatccaa tgaatcctga gaaagcccat 94980
taatatctca gtgtgaccat tttttctcaa aattcctacc tcacatgctc tgaggggctg 95040
gcagcttttc tgggaaggta aaatctcaag agggaggctt tgaatgattc caggacaaag 95100
tgaagataac atactaccgc aggtaaagtc taggtttact tggaggtggc tcaaaacaca 95160
aatcctgtca attttaatga cacatgaggc catgactctg ggtcgtcact ctgggcaagc 95220
cattgaaccc attagaacct cagttttccc atctgtacaa aaagaggtga taatgggtaa 95280
gcaatcctgg actgcattgt ggtagtgcca tcaagcaatg agatttgtca ggaaggaagt 95340
gtgattcaag tacaggcact ttgttatctg atgcctgtgt gttgagtgtg aaggaggggt 95400
taattattcc tctattcatg tcctttctat tctcaaaatt tcttatgtgg gaggaaaatc 95460
ttggtgcggt aaagatcaca gccaccctgg ggtcagacag aggactgtgt caagcttggt 95520
aagcgtcacc attcaggtga gtgggaaaga gactgaaccc cattaaacca gatgacctcc 95580
aaggccctgc ccgccccaac atctgagggc tgcctggctt gtctgcacag ggtgctcaca 95640
gtactcctga ctttgacata attttaacat taaagtcaca aggatgccct ggatcacaac 95700
tgctggagaa gagatggtag tgggatttgt ttcccaggag actttctgat tctagtgggg 95760

ccaggaccaa cctccaaaat aagaggtctt tgcaactgct gcgagcctcc tggcacctct 95820
accctctagc gagaggctgc ctctcctgcc ccaccccggt ttcaagcacg ctgcagggca 95880
ggaactcact gtgctggcaa aggtgagctg gagacctggc acggcataaa gctttttttaa 95940
ctgacccttt ttaatttcat cttccctgga cttaatctag agagtcattg atgaaacaaa 96000
catgccattt tctcccgatt tcacgctttt aaatgtcaac aacaaacaaa cgtttatata 96060
cacaaatgtt gctgaaggag acttttggct ttagacaagg gtaaaaactg aacctcttag 96120
tgtgactttg gttgattttt taaaaaatct gtaatttgac atataaagaa ttattaagac 96180
tttttttttt tttctggtag gcattgaatg tgtccttgaa aagaaaaaaa atatgctcag 96240
tttcaatctt ctgtctatgg gtagctagtt atcccagcct tagttattga ataaggagtc 96300
ttttcaccat tgcttatttt attttacttt ttgagatggg gtcttgctct gtcacccagg 96360
ctggagtgca gtggcacgat tttggctcac tgcaacctcc gcctcccagg ttcaagcgat 96420
tcccctgcct cagtctccca agtagtttgg attacacatg tgcaccacca cacctggcta 96480
atttttgtat tttgatagag acagggtttc accaagttgg ccaggctggt cgtgaactcc 96540
tgacctcagt tgatccaccc cacttggcct cccaaagtac tgggattaca ggcatgagcc 96600
accgtgcctg gctgcattgc ttatttttgt cagctttgtc aaagatcaga tagttgtagg 96660
tgtgtggtct tatttctggg ctctctattc tgttccatca gtctatgtgt ctgtttgtgt 96720
atcagtgcca tgttgttttg gttattgtag ccctgtagta tggtttgaag ttggataaca 96780
tgatgcttcc agctttgttc tttttgctta gattgtcttg gctatttggg atcttttttt 96840
ggttccatat gaattttaaa atagtttttt tctagttctg tgaagtatgt cactggtagt 96900
ttgataggaa tagcattgaa tctataagtt gctttgagca gtatggccct tttattgata 96960
ttgattcttc ctattcatga gcatggaatg ttttttccat ttgtttgtgt catctctgat 97020
ttatttgagc agggttttgt agttcttctt gtagagaatt tcacctcccg ggttagctgt 97080
attcctaggt gttttatttc ttttgtggca attgtgaatg ggattgtgtt cctgatttgg 97140
ctctttgctt gactattttt ggtgtatagg aaggctaagt gatttctgta tgttgatttt 97200
gtgtcctgag agtttgctga agttgtttat cagctgaagg agcttttggg ccaacactat 97260
ggggttttct aaatataggg acatgtcatc tgcaaatagg gatagtttga ctacctctct 97320
ttctatttgg atgtgctata tttctttctc ttgcctgatt gctctggcca ggacttctaa 97380
tactatgttg aataggagtg gtgagagagg gcatcatttt cttgtaactg gacccccttcc 97440
ttacaccata tataaaaact aactcaagat ggattaaaga cttaaatgaa aagcccaaaa 97500
ctctaagaac cctggaagac tactgaggca ataccatctt agacatagga atgggcaaaa 97560
atttcatgat gaagatgaca aaagcaattg caacaaaagc agaaattgag aaatgagatc 97620

taattatact aaagagcttc tatataacaa ataaaactat caacagagta aacagacaac 97680

ctacagaatg ggagaaaatt tttgcaaact atgcgtctaa ccaaggtcta ctatccagca 97740

tctataagga acttaaattt acaagaaaaa aacaacctca ttaaaaagca ggcaaagggc 97800

atgaacagac acttttcaac agaagacata catgtggcca acaagcatat gaaaaaagct 97860

cagcatcact gatcattaga gaaatgcaaa tcaaaaccac aatgagatac catctcacac 97920

caatcagagt ggttgttatt aaaaagtcaa aatataatag atgctggtga agttgcagag 97980

aaatgggaac acttatataa tgttggtggg agtgtaaatt agttcaatca ttgtgaaaaa 98040

cagtatgatg attcctcaaa gacctaaaaa tacaactacc attcaaccta gcaattccat 98100

gactgggtat atacccaaat ggatataaat tgttctatca taacgacaca tacatgcata 98160

tgttcattgc agcactattc acaatagcaa agacatggaa tcaatctaaa tgcccattga 98220

tggtagactg gataaagaaa atgtggtaca tatacatgat agaatactat gcagccacca 98280

aaaagaatga gatcatgtcc ttttcaggaa catggaagga gctggaggcc attatcctta 98340

gcaaactaat gcaggaacag aaaacctaat actgcatgtt ctcacttgta agtgggagct 98400

acatgattag aattcatgga cacatagaga ggaataacag acactggggc ctatcagagg 98460

gtggagggtg gaaggaggga gaggatcagg aaaaataact aatgggtact aggcttaata 98520

cctgggtgat gaaataatct gtacaacaaa cccccatgac acaagtttac ctatgaaaca 98580

aacctgcacg tgtacccctg aacttaaaat aaaagttaaa aaaaaacccc aaaaggtgaa 98640

gtgttggtga ggatgtggag aaaagggaac ccttattaca caattagtgg gaatgtaaat 98700

tagtgcagcc attttggaaa atagccaagg aaaatttttt tattgagtca gatgatgtta 98760

ccttacagca atagcttcca aaggggatgt cctgaatagt agaacttctc tttacatttt 98820

caaacctctt tttactttct gattttgagt ttcataatga acatgtatca atacaatagc 98880

acactatgta ttttataaat gaacaaatat gggaaaaata agatatgttc actttcctgg 98940

ctgaccctag ttagtctaaa ctatcagtta taggatccat ttgttctaaa tattgaaggc 99000

attgttgggg gcagtgggga gggtgaatga tagacgcaaa ttccttgaag gaatagaaat 99060

gtgactacta tgctgaaaag caggacccat ggagtataaa tagagtattc actgtgcttt 99120

tacatttgtt tccctggagg aaacgtaaaa caaatctcaa gactttctgg tcatttctaa 99180

gtcataaacg gccactagtc actaatttat ataaccagcc caatgtaaat atcaaacgtg 99240

tgagtttcat ttcatctcac aactcctccc ctttcccagt tctggcccca tacaagcccg 99300

ctgcctgcag tgtaatggag gaagatggtt tctttttcct tccctcccat ttatttgtct 99360

ttgtgcatct taaagccagc ctgaatttct actccctaca aggttgaagt gggtgggtca 99420

ggaggtgggg agagagctaa ggaacgttgg cgtgagttgt tgttgtgttt gagtctggaa 99480

ggtgttggga gctgcccccc ctcactcagg gcctcattta tgggctcttc ggataacccc 99540

catgctctag cactgggatc ttgccctgtg gatccctcca tgcaggtggc accacaccct 99600
ctggctggtt gtgatgggcc ttctcagctt tgagaattag caacacactt cctattgaag 99660
tcatctgtcc ctgtgggtag cccacttggg ctggatgcaa actgaacctg cactgattct 99720
ctcttgcatg tggcccacat gagctccatt ttcctccgca aactttgggt gcacagcccc 99780
acgacgacac agacagcttt tcttgcgtgc caccaaagtg gtgggccaga gttctcttat 99840
ccttaagatt ttcaggtgtc accacgtcca ccaattcttg gcagacatga atcaatactt 99900
ccaagggttt tggtgaagcc cttttccctg ggctgcagat gagggcagcc atgtcctgtc 99960
tcttccacct gttgtggaag gggttcatgg caaacatctt cgaacatcct ctaatttccc 100020
aattcttgat cctttcgtat gcttgatgtg tgagagagaa gagagaagag agaaacgctt 100080
cttgctgctt tttttttttt taatcccaca tggtgacctt agactttact ttggaatgtg 100140
ccatttaata tctggggacc tcagcccaac acagggacta aatagctctg ttttaacatt 100200
ttgtttatat acacacattc atttgaccaa atgaacatgt tccagctttt ttaacccttg 100260
cccagcaaat cacttaattc agttaataaa aagactaaaa taatgaggcc aaccttattg 100320
gccacgaaca tctgttgggt attcctaaat tgatccctga gttaatctcc acctttaagg 100380
agtttataat tttatttggc aaattgatgt tataatgaaa ttcctgggca tgaaaaaaac 100440
ctaactatgt aatgttggat agtgtgagtt ttcccaaaag taccagtata agcaataaat 100500
gctataactg atcatgaatt gtttacagtg attgagtaga atcattgcat ctggtcatga 100560
atagaaagag cacctcccag acggctggtc tgctgccagg tgtgcagttt taggggtctc 100620
cacactcatt ctattcacat cttttgactt tatcagctgt gtggtggatc tgtggttaca 100680
ggctgatgtg gtttggctgt gacctcgctc aaatctcatc ttgaattgta gctcccataa 100740
ttcccacgtg tcatgggagg gatccagtcg gaggtaattg aatcacgggg gcgggtctct 100800
cccgtgctgt tctcgtggta atgaataagt ctcacgagat ctgatggttt tataaatggg 100860
agttctcttt cacgagctct cttgcctgcc gccatgtaag aagcgccttt gctcttcctt 100920
tgtcttctgc catgattgtg aggccttccc agccatgtgg aactgtgagt ccattaaacc 100980
tctttccttt ataaattacc cagtcttgga tatgtcttta ttagcagtgt gaaaatggac 101040
taatacatga gccacattgt tacagagttt ctgaaggtca ttaagagaag tccatgctgt 101100
gggctgagct gggactcaag aatctcaaag gagagccagt gcaatcaaca cgaagggccc 101160
atcttgaact cctaaggcag ggcagagctg ggtcttatgg agacatgtgg ctttcaggta 101220
atccagtgag cacctgtgtt ttcctcctat aattcttgag gaatggatat tgttctatat 101280
ttcagatagt ataataataa atacctggaa aatactgtat gaccccaaac aacaggtaaa 101340
atgtcaaaaa aaacctttta tctacacaaa gttagacaac aaattcaaat aatcttatcc 101400

gtttattcat tctcttccaa tacaatcatg tatcatttaa tgatggggat gagtttggag 101460
gaaggtgttg ttaggagatt ttgtcatggt gcaaacatca tcaagtatac ttatgccaac 101520
ctcagtggta tagcctacta cacatctagg cttggatagt acagcctgtt gctcctaggc 101580
tataaacctg tacagcatgt tactgtcctg aatactgcgg caactataaa ataatggtaa 101640
gtatttgtat atctaaacac acttcaacat agaaaaggca cagtgaaaat atggtataag 101700
agataaaaaa tggtccacct gtacaaggca cttgccagaa tggagcttgc aggactggaa 101760
gttgctctgg gagagtcagc gaatggctga tgagtgaatg tgaaggccta gggcattatt 101820
gtatgctact gtaaacttta taaacactgt gcacttagga tacactaaat ttattaaaaa 101880
tttttctttc ttcaatcaat aataagtgaa cattagctta ctgtattttt ttttttactt 101940
tataaacttt aatttttttt tagagatagg gtcttgctct gttggccagg ctggagtgca 102000
gtggcacaat catagttcac tacaacattg aactcctagg cttaagcaat cctcacacct 102060
cagcctcctg ggtagctggt actgcagacg tacactactg cacccagcta atttttaaat 102120
tttttgtagc gactgggttc tgctatgttg accaggctgg tcttgaactc ctgactcaag 102180
caatcctcct gcctcagcct cccagcatgc tgggattata ggtgtgagct actgcacttg 102240
gcctaaactc taatttttaa aatcttcttg actgttttat aataacactt agtttgaaac 102300
acaaacacat tgtacagctg tacaaaaatg ttttctttct ttacatcctt attctataag 102360
ctttttttctg tttaagtttt taaaaatgtt ttagtctgag tgcgggggct cacgcctgta 102420
atcctagcac tttgggaggc cgaggcgggc agatcatgag gtcaggagat cgagaccatc 102480
ctggctaaca cggtgaaacc ctgtctctac taaaaataca aaaaaattag ccgggcatgg 102540
tggtgtgcac ctgcagtccc agctactcgg gcggctgagg caggagaatg gcatgaaccc 102600
aggaggcgga gcttgcagtg agctgagttt gcaccactgc actccagcct gggcgacaga 102660
acgagactct atctcaaaaa aaaaaaaaaa agttttaaac ctttttaaaa aatcaaagtc 102720
acagacacat gcattagcct aggcctaccc agggtcagga tcatcaatgt cactgtcttc 102780
cccttccaca tcttgtccca ctggaaagtc ctcagggaca gtaacacccc tggagctgtc 102840
atctcctctg ataataatat gggcttctgg aagacctcca gaaggacctg cctcctgcct 102900
aatgctgttt tacaggtaat atttttttct agtagaagga gtacactaaa ataatgataa 102960
aaactgtagt aaagtaaata tataaattag tcatatagtc atttattatc ataatcatta 103020
tgtactgtac ataattgtat tgccagactt ttatacaact ggtggcacaa taggtttgct 103080
tacaccagca tcaccacaca cgtgagtaat gtgttgtgct gtgacattag gacagctaca 103140
atgtcaggag gcaataggaa tttttcagct ccattataac cttacaagac cactgtcata 103200
tctgcagtac aaaacatcat tatgtggtgc atgactatat ttactgagca ccaaatatgt 103260
gcccaggcag tgtgctaggg gctggaagtg acctcgaagt ctagtaaaaa aaccacaaca 103320

gtaaaacaat atgtgtaatt caaagtgaca tatcctgtaa tagctggttg cctacctgct 103380
ccccagagag aaattaaacc tgcttgctta acttattaat tttaaattca tgtacttgca 103440
atataaaaac atattttaaa acatattagg agaataggat gtcttctgta gtgataggct 103500
atttaatgtt ctcttcactg tgttctaaaa aaatcgtgat cgagtgtaac aaaatgggga 103560
agggagggtt agcataaact ctcctttgat gtctatgctg tggatcagaa aactcatgaa 103620
tatgttaaca actggatttt ctcctaatat gaaaacaact ctttggcttc ttttgcaaat 103680
gtgaaaagtt aaccataaat ataagacctt ttctttccac taaaactagg gcacaatcat 103740
tgttttcctc ttattggagt ccaaatgggt ttccataatc ttcagatcta gtagttgtat 103800
ttatcgatga cataaatgga aagactaaaa gataaagtta agaaaaattc agtggtttat 103860
ggcttcaact ctactgataa ttttgtatat aaagggatcc taaattagac tgtacacaca 103920
tttgccattt gagttccaag cattttagca atagatcaaa agatcttaat gcctgatagt 103980
tgaaagatta cttccaaatt ttttttattt ttgtgaaagc caaagtcctt ttctcaacat 104040
gacagtcaca attttgtcaa caatctaccc gttatttaca aaggtttaaa atctgataat 104100
agatatttac ttggactata caatgtctca gtgggagcaa tagatggttt acaatgggtc 104160
tgaaaatgtt gcaaatagtc aaaactggca ttgcaacaat tcatttccaa aaacacacta 104220
gactcgtgag tttttggttt ttaagtttat atagcttgct tctggtgtta acgttctcca 104280
cacctaaggt gccaacatgc atggctactc tttgtcccca gagaaacatt gctaatttgt 104340
accaaatttc aatgatgtgt caagcaaact gtcattttgg aatgatagta agcacgcctt 104400
caacaacagc tctttatttc gtccaaaacc tttgggccac acaattctga cctttatcac 104460
actctcagta cctttcattc attattagct tgtgcttctg gggccccggg aattgtcaac 104520
tatggtcaaa ataatggagc aaaatagcaa gaagaacttg cgtttcctat atcattgcat 104580
ttaatctttg caacaactct ttgaggtgga aattgcccca attttataga ttcagcttaa 104640
agaccttgat ttgtccaaga tcacacaacc actaggaggt ataatgttca aataaaaatg 104700
tctgaaaaga gctgggtgtg gtgggtcatg cctgtaatcc cagcacgttg ggaggctgag 104760
gtggggggat cacctgaggt caggagtttg aaaccagcct gggcaacatg gcgaaacccc 104820
gcctctacta aaaatacaaa aattagctgg gtgtggtggt gcatacatgt aatcccagct 104880
acttgggaga ctgaggcacg agaattgaat gaaccctgtg aggcagaggt tgcagtgagc 104940
tgaaatcgca ccactgcact ccagcccagg cggcagagtg agactccatc tcaaaaaaaa 105000
aaaaaaaaaa aaatctggaa agatagatta cttggcctca atcatagtaa aaaagtgtca 105060
gttaaaactg catggagaga tactattttg aaaagcatca aaaagttcga agatccatca 105120
tatttgtaag gctatgggag tttaaattgt acaggcgtta tgaaggcatt tggttacatc 105180

taccaaaatt aacaccgcac atatttactc atgtgcaaat tgacttatgt acaaatttac 105240
taattgtagc attgttgata attgtaaaat attaggaaca cacctaattg ccattaatgg 105300
ggaactggta aaataaacta gaattcctct gtgcatggaa tactatgcag tcatcaaaaa 105360
tgacgaagat gtggccgggt gcggtggctc acgcctgtaa tccagcactt tgggaggccg 105420
aggtgggcgg atcatgaggt caggagatca aaatcatcct ggctaacaca gtgaaacccc 105480
atctcttcta aaaataccaa aaaaaaaaaa ccaaaacaca attagccggg catggtggcg 105540
ggtgcctgta gtcccagcta ctcgggaggc tgaggcagga gaatggcgtg aacccaggag 105600
gcggagcttg cagtgagccg agattgggcc actgcacgcc agcctgggtg acagagtgag 105660
actccgtctc aaaaaaaaaa aaaagaaaac agaacaaaac aaaaaatgag gaagatgttt 105720
atttagtgaa atagaaagat atccaagata tgttgctaat aaaaacaaaa aaaaacacat 105780
ggcacaaata aggtgcatgt tactatctgg gtcttttaaa aaggggtgtg gaagaatgtg 105840
tgtgtgtgtt ttcttcaata tgtgtagaat atctctggaa ggaaatttaa aaactggcga 105900
cctcagctgt ctccagagag ggaaaattgg tgggttgcag gcagaggtga aagattttcc 105960
cactatgact ttgaaaatat cttttcaagt ttaaagcacc aactgcatta tatatcaaga 106020
gtaaataaaa tacaatttta aaatgaaatg gcatttgtaa aacacttgat agatgttcag 106080
ttaacagcca tcaggattta ttagtaattg tggtttttgc cattaaaagt aattttaaaa 106140
gtaatggttt tgccattagt tttaatgaca aaaaccacaa ttagttttgc accaaccaaa 106200
taccttacgc ttctggtgag ggctcagaag agagatcata agtaaagaac actagaaagg 106260
atgtttaata ttatttccct ttttaaaact ggtggttgtc aaagcagtct gaaggaaaag 106320
tctccagaac tggtgatcat gttcacatag cagtagtttg gagtcaacag aaatcatctt 106380
caccatcaac ctagctccga aagaggccga gccaatctag ttcccttggc caaccacctt 106440
cctcccaacc tatacgttgg ggtgatccac tacaaagctc tcaagagtat acacaatgct 106500
actacagtca ttgtgagtga gatctgaagg gagcataggc taggactcat gagattatag 106560
ttcagagttt ctactgtaat tcctttcagc aaagtgaaga gcatgtcact ggggttacaa 106620
gtgttactgg ctacttagct agtgctagat tacccaaaaa agtaaccggg tctcatgggc 106680
agaggtggga aagtctgtta agcacatagt gaaggatgaa tctgggctga ttggaaaaca 106740
tttgtttgaa tgaatgtagc atttatttcc aagaataagc atgtccagac tgattgagca 106800
agagttatat ttagttcagt cgttctgatg tccattttga gaaagctaaa ccagcatgcc 106860
agaaggggtc aaagatcaca ttttgcatga aagaaaagcc aatggaaatg ggactgttag 106920
acttaaactt ttgggtattt tacactagga cttagaaagg actttattcc tttttatatt 106980
ttctgtctct agcattgcaa gtagcaacag agtgaatttt tggtatttgg aatactctct 107040
cctcattttt cctttgggaa aatcattagc ctatttgatt tgactgctgt tgaatatgtt 107100

ccatgacatc cataagtcta ccttcaggca ttgcttgtgc ttgtttatta catatgatta 107160
attgcttaca taagaacaat gatgatccct gcagggggag gagggatgtg aagaacaact 107220
tacttaggca ttccaggggc taacaaggga tggtttgata cagctcattc ttgttttgaa 107280
tcttcatgcc tcttgagaaa gaaaggagca gaatatgctt tgcatggcgc tatgtcctcc 107340
caggtcctat gttttctttt ttgtatttta tcagttctct acgtaaggat tttctcagtt 107400
taatataatt tccattttta aaattcaaat acctacatat gaggtgggaa aggaaattaa 107460
tcagatgcat tcctataaac tgcagactta aattaagacc ttgagcaaac tggcattttg 107520
atgacttgag gattcagtgg gtgggacagt tgtacatctt gaactctggg tacactgccg 107580
aaagcgaggc ctgagaaggc tgtgttgagg aaggagtgtc agtgactgag cctgttggaa 107640
agggaggctg agagagatga ggacacatac gtaagggaga acacattggc tggccaggtg 107700
tttttttttc tgctgtggat tttgattaga attctgggtc tcattaagtg acccatgttg 107760
aatagagatt ggatagggct ggcttggaaa gccctcagtg aagagatgaa ctcgacagca 107820
tgataaggaa gtagatgcaa attcaggaca ggaaatggag aagggcaggg tgagaaaagg 107880
gaacaaattt acaatttgtg ccattagcca tttattagaa tttggtataa gaatttgtgg 107940
aattattgtt tccttctgga tttatggtac aaaaacctac tgacaagtgt cactaaaagt 108000
gatgtcttta ttcaattatc aaccaaaagt tttacactaa attcttttga gtctttgaat 108060
gtttatgtta tttccaataa tctttccagt tcttttaaaa ggctgatttt taggaaggct 108120
agcacttttt gatcacagaa tagtttcccc aatgagtatg atagcttcaa tatctttcaa 108180
attagattct tttggaaatt gaactgtgtc aaatcaaata aaacaagtag ggtttttttgc 108240
tgttattgtt gaaggtatta tatttgcaag tttgtacaat ttagcagata taggaaaggt 108300
ctaatttttt tctttagcta gaatgttcag ttcaaattat acagatataa gcatgagacc 108360
taaatgaact ctcttctgat gagtaatgaa tggaaggtag attactcatt tcttcatgtt 108420
caaaaaacaa attatgtctc tgaacacagt tgtttcaatg tttttaactt atgttctcaa 108480
gctcttccca tttctttatc agtgatttgg gaatgtgaag gcagtcttgt tgtagtttcc 108540
aaagatttag gcatttttga gactctaaga atagttcatt aagccaccaa ttagtccctt 108600
aattttattt aagctgagca agcaaggccc acactaaaag tcagaagaat gcggcatcta 108660
cccagagagg ctgagggtgg gatagaaaga aggaggcact ccaagtttgg agacacagat 108720
tctattccag ttcctcaagc tgcatgacct tcagcaagtc tcctgtgttg ttcaggaccc 108780
cagtttcttt ataagtgcaa tgaagagttt ggactcaaca acttataaag aaacattcat 108840
tctaattgtc tattgtgtga aaagaatgtg agttgttcta gaaaatatga atctgctccc 108900
caattgtcct ccaagctctg gttctaaatt tcagttatta tagttacttc tttcgaggta 108960

aatcattgag gaagtcagtt aatgcagaga tgatgtctgg aaggagttta caagttcatt 109020
gttcaaatag tatttgttgg gggccaacta caactacaca tcagggattg tgctcacatt 109080
ggactagtgt ctttggaaaa cagagtcctt gtgtgccctt tttgaattta caggccaact 109140
tggggaagca gagagacaaa taggaaatcc aacagcatga taagtagtat aatgagaatg 109200
tttagggaga tacgagcatt tataggagta gcactcaaca ggaaatcaag gaaggcttcc 109260
tggtggctat gatgtctcaa gggagacttg aagcatgagt aggagtatgt cagatgaaaa 109320
gggatgggag gaagatttca ggtagggtaa tttgcatacg tgaatattta gacccagaga 109380
aagtccagag ggactgagga acttaaaaga aatccagtat gggaggagca tagtacctgt 109440
tggggagggt ggggactctt gataaatgag gatagtaatt caagttcttt ggctcttgag 109500
ttacttttgg acaagttgag cttcaagtag ctagtacata ggaggagctt aataaatatt 109560
tattgaatga atgaacatgt aagtaaaaat gtctaatagg tacctggcaa agtggacttc 109620
ctagtcagga aagagaggtt ggttggagat ttctattttg aaattttcaa tgtatatatg 109680
aaagtaaaaa cacaaggagt ggttgtgatc atccaggaga aggtatgcag aggagggaag 109740
agggtctagg ggctgaatcc tggggtaccc acacctgaga ggagggagag gaagaggggc 109800
tctcagaaga gcctgagcag gaagggccat tgtaatagga ctaaacacta agcccctgaa 109860
gtcagctctc tgggggcaga ggtttttgtt ctgtttattt tgggatgcct gctgttgacc 109920
agattatgct cccctaccca cccaaattca catattgaag ccctaacact caatgtgact 109980
gtatctggag atagggtctt tgggaggtaa ttatggttaa atgatgtttt aaggctggga 110040
ccctactctg ataggactgt ggccttataa aaagagctct ctctttttct gtcctcctcc 110100
ttctacctcc tccaggaccc accccaaccc cacactgaga aaataaatcg ctgttgttta 110160
aatcacccag tctatggtat tttgttacag tagcctgagc tgaccaaaac agttctccgc 110220
acttagaaat ggtcctggta aatacagcta atcaatattt ttttgagtga attgaatgaa 110280
tgagtcattc actggcatgc ttataaatgc attactccct ccacagtgtg attgtcattg 110340
agcaggttgg gagaagggag gagctctcgc aaacatggaa gtcctgggtg cttagggaga 110400
atgggaaggt gccgtggtca caaaatcgga agaaagagct gaaaggctga aaggctgaaa 110460
atgctaaact gctcaaatct cctctgtctt tttttaaaaa aaatcatttg gctaggctgg 110520
ctctgaaatt ggggatggtt cttggttgat tatgtggtgt gtgtgtgtgt gtgtgtgtgt 110580
gtgtgtaaag gcccttctta acattagata ggactatctg aatctaagtg aatttaggca 110640
caatctagat attgctcttc atagagaaat aatactctaa gctggcaatg aaattcctca 110700
agtaaacaac agataaaggg aaggagaggg acacagtgta acaataattg gaataattgg 110760
aattcttgta tcactcatgg cacttacagt gaaagtactt tctacattgt cttctattac 110820
atgcaacata ggtacattat ataaaaagat cctaaaatat tgttaaaaga aattttccca 110880

gaaaatgtaa gataaaagtt actaaattct attaagcttg caagttaaat ccaaatcatt 110940

cattgactca taatcaagaa cagtcatgct gcctgacaac caaaaaattc aaactgtctt 111000

catcaagtcc tgggtgggaa gccaggaaat gggatctgag atcacaggct ttgtgagagt 111060

cagggtcttt ggcgtgattc ttggtcacaa atcatgcatt tttttccttg gagagatagt 111120

gggaaagaaa aggctgttta aaaacatgtt aaagaatact gattccattt gacttcttgt 111180

taatgtgtac ccttgcagac ttgggtaaaa cttgatcatg ttaaaagcca ttgtacttaa 111240

agattcatga ctagagtgtt aggacaattc tgtggccttg gggtcccat tgcagcaaga 111300

agagtctgac tgagaagagg atagctccgt cctttgatgc ctagtaacct gcatgcccag 111360

cactttgctg gctgggccat tggtaggaaa tgcggtacat gcacttcaat ctgtaagaga 111420

tcatggtcca tagagatcat agatttattt tgccacctgg cccagatggt gatagaagtc 111480

aggacaatgg ttgcctagag gggttgggag tttattagaa tgggacacaa agggaaattt 111540

ctggggtgat ggaaatactc tatactttca ttgggatgat tgttacatgg acatatccat 111600

ctgtcaaatt catcaaatgg tatgctttag atacatgtat ttcattgtat ataaattttg 111660

cctcaagaaa caaaaactca aggtcaggtg aaaaaagtcc aggagctaat caaaaaccct 111720

taaaataagt gaccaaacta tttgtgaaaa tgagcagaaa cttaaaggga aattctttgt 111780

tatttttcca cattagagga aaattgatgg tatcagataa atgctcaaaa ataagcttcc 111840

aaacagagca tttataatca gtacaataat aagtttgaac acagacaaac cttcatgtct 111900

cagagtgaag gaaccatttc tagtagtata agctattacg gagatagttc cacatttatt 111960

ttctttcatg tttcttttgg aggaataatg tttttgtaaa tgtaaaatgc ttttaatgga 112020

gctagagaag aagctatgtt tggtcatagt attatctgct tgcgaactaa aaattcagag 112080

ctctcttgtg aatcatgctg ccaagaattg cagacttaag cctcaagatt gtacgaattt 112140

aaattttgac tttatggcct cagttggcat gaagatacaa agataatctt ctagcaaaaa 112200

cagttttgaa agaccgggcg tgatggctca tgcctataat cccagcactt tgggaggttg 112260

aggcgggtgg atcacctgtg gtcaggagtt caggaccagc ctggccaaca tggtgaaatc 112320

ctgtctcaac taaacataca aaaaaattag ctgggtgtgg tggcggacac ctgtaatccc 112380

agctacttag ggaggctgag gcaggagaat cgcttgaacc caggaggcag aggttgcagt 112440

gagccgagat cacaccagtg tactccagcc tgggtgacaa aagtgaaact ctgtctcaaa 112500

aaaaaaaaaa aatggtagaa tgtcacatgg aaagttaact tttagagata gtaatccaga 112560

cacctataat attacattgt ccttttaagt atctatatca ataaattaac caacatttgt 112620

tgacttaaca tggccaggga cagtgctggg ttctcataat tcccaagtaa ttctagccag 112680

accttgtcct caaggagctt atgagcagca tgaggagata aaaaacagat aaataactct 112740

aattcaggaa gaatttgata aatgcatgat aactactact agaattcaga ggagaggaaa 112800
ttttctttga atagagacta aaggaaggaa taatgaatgt ggtagcattt gggctggacc 112860
tattacaaga ggcatgcttt cccctgacaa gccaagaaag tggggattta aggtagaaga 112920
aaagaaaagc cttgagttct tggagtatta aaattttgct tggctgaagc ataggttaca 112980
gtcggaggag atggactagg aaggatcgtt atgggcagag aggaagccct gaactcatgg 113040
gggaagctat gatttgggtt aaagacagag ctgggtcatg tcaagatgga tcctgaagca 113100
gtaaaaaaaa atccaagaaa gtaaacaggt tgcagggttt aggatgaagt ccgggaggaa 113160
agggcagagt ggtccttagg aggccgttag gacaggtaag gtaatgggtc tcaaagggag 113220
tggccgaaat gcaatggaaa aagagagatt gtaaagctag aaggcttagg aattgcctct 113280
tgattaggtg tggaaggcaa gggaaaatca gccctcgaag aagacagtga gattttaatc 113340
tgggtggctg gagagacagt gatgctgggc acagacacgg ggaagttgag aggaacacca 113400
tgtttgagaa tggtgactca tatttgaaca agcctgcaat gcccagcaga ccgctggaaa 113460
agtggggctg gagacacatt caacggagga gccagatcaa tctttaccct tcttcacctg 113520
agagagccag taagtcacgg ctggaacgtg tgtgtccagc aggagagggt agggagggaa 113580
gccaagagag ctgggagccc gagtgaagtt tttgccaaag gcagaagagg aaagtcggcg 113640
tagcacagta tactttccca cccatgctca ccaagcccag ggacaaggct caccaagatg 113700
agtttggaag agaatgctgg agagaaagtg gttaagaaaa ctgcctttac tgaacttctt 113760
gggctaactt tgattgtaag tctctgaaca atcaaagcct gtgaggagac agctaacctt 113820
cttattcttc ctatgtcaat agtgaacaat tgcagatccc ctttcctttc cttctccttt 113880
cccctgttcc tctctcctcc ctccctgaat actcttgctt ttttctggga ctggtctaga 113940
gcatgggtgg ccattgttga cctacaggag gcaccactgt caccaacaaa gggtaacagt 114000
ctttcttttc aatatttatt tatatccagt atttattttc aatactgact atggagagag 114060
ctctcctgtg ctcaaacact gcaatactgg gggtctttca aagcacaaaa acatatattt 114120
gcatgatggc atcattaaca tttttatggc tttctatttc ttttttgtac tggtctcaag 114180
agccactcat aaatctctca gtaactgcat agtgtcccag ggccagagac cggccactcc 114240
tggcattgtg attagagtca tttaatatcc aaggtggtga ctaatgtctg gcaacaaagc 114300
ctccattggg tgtcatgtgt cctgggaccc tgagcgtggg cactctagga gcacctcagt 114360
attgcgtgtt agtactatgg ccgagagaat agttgagaaa gtggtcaaga ggtggatcca 114420
tgtgaacgcc actgggaaat gagagacctc gttcccaatc acggtcagtg caactcgaaa 114480
gcctaaaatc agtttaaaac aaaggtatct acctttatct tatgttcata tcctaggctt 114540
ttaataatac gtatttttca catgtttaca gaaagcagtc aactgagcta ttcatggaaa 114600
ggtttgtggg tttggttaac gaagtggagg agtattacat ttcagctgga aacacatccc 114660

tagaatgcca aaacatttat tccaaagtct ggtttcctgg tgcaatcgga ggcatggcaa 114720
tgcctctgtt cagagactgg gggctagggc cagtaaggca tttgatccac atgtatccca 114780
gaaggctttt attgttaaat tatattcttt cggaaaaacc acccatgtcc tattttgtaa 114840
acttgatatc catacacttt tgactggcat tctattttag ccgtaagact atgattcaca 114900
gcaagcctgt ttttcctctt gcttggggtg gcagcagaaa gcatagggta ctttccagcc 114960
tccaagggta ggggcaaagg ggctggggtt tctcctcccc agtacagctt tctctggctg 115020
tgccacactg ctccctgtga gcagacagca agtctcccct cactccccac tgccattcat 115080
ccagcgctgt gcagtagccc agctgcgtgt ctgccgggag ggctgccaa gtgccctgcc 115140
tactggctgc ttcccgaatc cctgccattc cacgcacaaa cacatccaca cactctctct 115200
gcctagttca cacactgagc cactcgcaca tgcgagcaca ttccttcctt ccttctcact 115260
ctctcggccc ttgacttcta caagcccatg gaacatttct ggaaagacgt tcttgatcca 115320
gcagggtagg cttgttttga tttctctctc tgtagcttta gcattttgag aaagcaactt 115380
acctttctgg ctagtgtctg tatcctagca gggagatgag gattgctgtt ctccatgggg 115440
gtatgtgtgt gtctcctttt tctttcagga cttgtaggat tctttgtgcc atttgcatat 115500
aatttggcag gttcacattt tttaagagcc ctatgaagtg ctttttgcat gtgttttaaa 115560
aaggcatttg aaaattgaaa gtgtgattta tggaaattaa atcatctgta aaaaattgct 115620
ttggaaagta atgattgctg gccataaagg gaaatatctg cgatgcacct aatgtgtttt 115680
taacccttta tttgctgaca atctatagtc attaatgcta aactcgattt tggcttcagc 115740
tacatttgca tattgtccaa caatggtcta tttttgtaag aattagataa aatgtatact 115800
tgatataaaa tagtcaaaaa tgtaactctt agtaacagta agcttggcat ttagatagac 115860
catgaacact tcgtcagata ctctgttggg tgtttgggat agcaattaaa acaaagtatt 115920
gatagttgta tcagagtcta ttaggctgca gcaaaggaag tttattcaaa agtataaact 115980
atccaagatt atagacgcat gatatacttc acctattttt tgtctcctta atatgtatat 116040
atatatatat atatatatat atacacatat atgtgtgtgt gtatgtgcgt gtgcatgttt 116100
aacttttaat tcagttaaaa actttttct atttgttttt catctggata tttgattctg 116160
catatcctag cccaagtgaa ccgagaagat cgagttgtag gactaaagga tagacatgca 116220
gaaatgcatt ttaaaaatct gttagctgga ccagaccgac aatgtaacat aattgccaaa 116280
gctttggttc gtgacctgag gttatgtttg gtatgaaaag gtcacatttt atattcagtt 116340
ttctgaagtt ttggttgcat aaccaacctg tggaaggcat gaacacccat gtgcgcccta 116400
accaaaggtt tttctgaatc atccttcaca tgagaattcc taatgggacc aagtacagta 116460
ctgtggtcca acataaacac acaagtcagg ctgagagaat ctcagaaggt tgtggaaggg 116520

tctatctact ttgggagcat tttgcagagg aagaaactga ggtcctggca ggttgcattc 116580
tcctgatggc aaaatgcagc tcttcctata tgtataccct gaatctccgc ccccttcccc 116640
tcagatgccc cctgtcagtt cccccagctg ctaaatatag ctgtctgtgg ctggctgcgt 116700
atgcaaccgc acaccccatt ctatctgccc tatctcggtt acagtgtagt cctccccagg 116760
gtcatcctat gtacacacta cgtatttcta gccaacgagg agggggaatc aaacagaaag 116820
agagacaaac agagatatat cggagtctgg cacggggcac ataaggcagc acattagaga 116880
aagccggccc ctggatccgt ctttcgcgtt tattttaagc ccagtcttcc ctgggccacc 116940
tttagcagat cctcgtgcgc ccccgccccc tggccgtgaa actcagcctc tatccagcag 117000
cgacgacaag taaagtaaag ttcagggaag ctgctctttg ggatcgctcc aaatcgagtt 117060
gtgcctggag tgatgtttaa gccaatgtca gggcaaggca acagtccctg gccgtcctcc 117120
agcacctttg taatgcatat gagctcggga gaccagtact taaagttgga ggcccgggag 117180
cccaggagct ggcggagggc gttcgtcctg ggactgcact tgctcccgtc gggtcgcccg 117240
gcttcaccgg acccgcaggc tcccggggca gggccggggc cagagctcgc gtgtcggcgg 117300
gacatgcgct gcgtcgcctc taacctcggg ctgtgctctt tttccaggtg gcccgccggt 117360
ttctgagcct tctgccctgc ggggacacgg tctgcaccct gcccgcggcc acggaccatg 117420
accatgaccc tccacaccaa agcatctggg atggccctac tgcatcagat ccaagggaac 117480
gagctggagc ccctgaaccg tccgcagctc aagatccccc tggagcggcc cctgggcgag 117540
gtgtacctgg acagcagcaa gcccgccgtg tacaactacc ccgagggcgc cgcctacgag 117600
ttcaacgccg cggccgccgc caacgcgcag gtctacggtc agaccggcct cccctacggc 117660
cccgggtctg aggctgcggc gttcggctcc aacggcctgg ggggtttccc cccactcaac 117720
agcgtgtctc cgagcccgct gatgctactg cacccgccgc cgcagctgtc gcctttcctg 117780
cagccccacg gccagcaggt gccctactac ctggagaacg agcccagcgg ctacacggtg 117840
cgcgaggccg gcccgccggc attctacagg tacccgcgcc cgcgccgccc gtcggggtgg 117900
ccgccgcgcc cggcaggagg gagggaggga gggagggaga agggagagcc tagggagctg 117960
cgggagccgc gggacgcgcg acccgagggt gcgcgcaggg agcccggggc gcgcggccca 118020
gcccgggggt tctgcgtgca gcccgcgctg cgttcagagt caagttctct cgccgggcag 118080
ctgaaaaaaa cgtactctcc acccacttac cgtccgtgcg agaggcagac ccgaaagccc 118140
gggcttccta acaaaacaca cgttggaaaa ccagacaaag cagcagttat ttgtggggga 118200
aaacacctcc aggcaaataa acacggggcg ctttgagtca cttgggaagg tctcgctctt 118260
ggcatttaaa gttgggggtg tttggagtta gcagagctca gcagagtttt atttatcctt 118320
ttaatgtttt tgtttaatgt gctccccaaa tttcctttca tctagactat ttgattggaa 118380
atatgtcagc tatgatgatg actttctggg aagcgattcc tgtcacccgc tttcccctcc 118440

tccccacccc acgtcctggg gctttagaga gcgattggga gttgaatggg tctgatttcg 118500

gagttagctg gctgagtccg cgctggagcg gattgctggc atgtgacttc tgacagccgg 118560

aaatttgtag gtgtcccgcg agtttaaaac aagccatatg gaagcacaag tgcttaaaaa 118620

taatctcctg ccagcccagt gacaagcctg tcccacccgg ggagaatgcc ccggagtggc 118680

gtgcgggtca gccagggtct gcgcctcgca gccactgtgg aaggagcgcg gccggtccag 118740

gacacaggag accactttgt gacttcaatg gcgaaggttg tgtgtcctca ttttaatttt 118800

tttccctaca agaattgttc tttctccctc tcctctccct cccattttct cttgcccagt 118860

ttctcctttt gttttttgtt ttttgttttc ctgatgggcc tgcagaggga ttaggtgggc 118920

gcttctggtg aacaccttcc taggtggcca caggacaggt gtaccccgga ctgggtttgg 118980

aagcttcagg gcgccacatg gctgggtcct gaattaggca tttcccaact gtacactggt 119040

atccggactg gtgtccctat atctttctgc cttgtaagcc gtggaccagt tttgttcag 119100

tattctgttt ccagggatat ttatagcaga aggaagggga ctaaagtgca gtttggcccc 119160

agaggatact gaagggcaga ttctgggggt attcagtgtg catcttcagc cgccttggag 119220

aaatttagag catcccacag ccacgcagat ccaagctgtc tttactcaaa agacaaacaa 119280

tgaacaaaac ttttaaaggt tggcatattt caaattaatt ttacttgttt taatttaggg 119340

ttaaaacaga gaaaaaggat ttcttctgcc caccttttt tttttaaatg gaagaacaaa 119400

gtacagcgat taagtctaat tccacacaac atttaaaact gcttgatgtg aaggaaggca 119460

ctggtatgat gtgaattcca taaccttatg atggactcca gaaaccattt tcttccctat 119520

ttaattttca gttcttttat tgcaaattaa tgctgctgaa tttcaatggg cactaatgag 119580

actgctcctt ggtagattat ttactgcctt gctaataatt acaaagtgaa cctggtcaaa 119640

tacagagggg atcgcatctt attcaaaatt gttcatcatc ccagtgataa gtggtatcag 119700

tgtaatatgc cctatcttac actttctgca ttacatgata ttcaaacact cttagaataa 119760

taaaaaaaga gacaaggaac ttaaaaatta aaaaaaaaac ttgcacaaat gggactctgt 119820

gtggaaattc agttttagaa tgatttttcc tgtgttttat ttcccggatt atctttcctc 119880

ttttgttaga attctgcctg ttattatcca gcaaggaaaa gaagcatcta tgcaagttct 119940

tcatatggac agatattatt tagtattttt cccctctcag tttttctgct taaatgactc 120000

tgggtataaa ggaaaggatt gattgggctc ttttaggaaa ctttaagttt cttaagtagt 120060

tctcaaaagt tttggggctg aaagcagtgt tttcaaactg cttgtcatga cccagagggt 120120

catgaactca gtttagtgag tctagaatat tttttaaaag gactaaaatg gaaaggaata 120180

taatagaaaa tatcagagtg catggtattt cgtaaggata agttttgttt cctgaaaatc 120240

tgttttaatt atatgtgctt ctgtgtgctg attgtgatgt aaaatgtatt tcttactgtg 120300

gattgaattc aaagaaaaaa ttagaaagct aatggcctaa aatattatat gttcagtaga 120360
aaacaaaaaa ttcaggcaag tggctggttg tttttaccta tacaaatcaa aaggctattt 120420
tgattgtctt cattttcccc ttataaatta ggttggtgtc tttagtcatt taggctaagt 120480
tttactatct gattcttaac ttttctattg tagaatggtg ctgtcatgtg gactgtcctc 120540
ccgagtgtcc cactggatgt tcagagaatt tatgtgaagg tcacgtcatt tagcattgag 120600
atgctgtggt taccttcttc catttcttcc ataatatgca gccacatcta tgtgtgaaga 120660
aatgtaatag ataaaatttc tctggacgca taataatgtg agaaagattg tcacatgtcc 120720
cagcaaattg ttattaatat aaatttgtta cttggcaagc tgagattttg caagatgtta 120780
ctcaaaattt cacaatgaag gaaacaggga gtcatcttat cctgggttcc tttttagat 120840
ttcaaacaac ttaggaactt tgaataaaac taaagatgaa gcttaactat atcaactatc 120900
ctttttaaag ttctaattag gaatttaatg ctgcatgctt atttcagttt tattactcag 120960
tattcttaaa agttagacgt ctctcacttc tccaaaaaac ttggcaaatg tataaatctt 121020
ttgcatcaaa atcaatgccc tgctaatttg tatcctggcc atctgcatat tttggacaac 121080
taatttttcc actggtgatc atttgaaact ctttctcaac tttgaataga gactgatttc 121140
caaagtgaga tttaagtgac taagtttcaa gtttccgata catttttcct tttacttaga 121200
taacatttca gccccccttcc tttctgatct tactttttta ttaatttaaa ttgttactga 121260
ttacgtgaca ctttgtgctg gtctaagaat agtccagagt cacatattcc ctggtgaatg 121320
agcatatttt cggatgaaaa cggaatcaca tcttcaatcc ccatttcatt ttcacctcct 121380
ccatgtggct tgtacctgtt tggaagaaag ctcctgaagg ataattgcca cttattctaa 121440
tctttctcac actcatttaa tttggatccc tggctaaagt tgttatttac ttttgtgatt 121500
atacttagtc tatgacattc ataatttggg aaaattctca ggtttgagaa ttttggcggc 121560
ttgggatttc ttttagtttc ttatagtttt aaggatatgt aagacaggtg taagaaactg 121620
ccaaggggag gaaccataga tatcaggaaa aactagaaaa gatgccagac ttaccattaa 121680
tgaatgatga gacaatagta actttgttaa gtgagattgt atatgtgaaa gtggtataga 121740
aactaaacaa acattaggtg tttttattat tttactcaca tgttaatatt tgttttggtg 121800
ctttcatagg ctaaaaagct gggaaataac agatttaagt ggtcaggaat tttgttataa 121860
atatagaatg atgattatat gaaatctttt cctgtgaaag tcaaatttaa gtaaaatctt 121920
tatcaccatc tgcaacattt gtctgcagcc tggcttacca ggttatcata aagaacattt 121980
attttacaga tacattaaag aaagtcaaaa ccctgattat gtgtaaacaa ttttacataa 122040
ggaaatatat gaattttaat tatatttttc taaaatccgt actcagcatg aaattaatac 122100
atcttaaccc ctccctgtga cttcattatt atttttaatg taactttaga agaacccagt 122160
agagagagca gcgtgctaag tgtgtttctt tcttttccag acaactttga atggagagga 122220

gcaaattagt cttttggttt aattctgtct cagtttgctt atctaaagaa aggaaaacag 122280
agtggctaca cttgtttaga accatatgca tactccagag aaagatgctc tattaatcca 122340
aaaaatacag ccacttgaaa ccagccaaag cgaaagtgta agggacttca tggaaaggag 122400
gcagttcacc aaagttattg aggggtttta tattttaaac tccgccagtg aattgacgtg 122460
taatgtcact tacaaaaaaa aaaaaagtat gtctgagctg ttcgctactt cgtctctaaa 122520
atatactcat actgatctct gaaatcccag aatttaagtg ggctggaggt tacgggaagc 122580
acctttataa tatccttaat ctcatgaggg aagaaaccat aattgctgaa ttctctgcct 122640
tggataatat caggagggac tctgaagaaa gttttgcagt aatcaacaat gttttaaatt 122700
atgtgtatat ttttagatca cctcaaaaaa tataggaagc acagaatgac aactattctg 122760
gtctcaactg acacaatttt atgtagttta ataaagtaat aatttcaaga aacgtgggca 122820
aataagaaag agtatgactt tcttacaacc cgcttgtaag tgatgtggtg gtggtaatga 122880
tccatgattt tgatgatgac gatgatgatg aaaatgaagt ttttgtctca gtttgggtag 122940
gtggtatttc tggatgcctc ctatggaccc tggagatgtt catcctatac agaaatccaa 123000
tcctttaaat ctacttggct cattgtttta gaattctaat tccatagtct gaaaatttta 123060
ataatgatat taccaataat attagaaact tattaagtac ctataattgc tatacaaaaa 123120
atttaaaaga acccaaaatt ccaagcaaga ctgaaaattt tttgtctctc ctctgaacta 123180
tttagaggga caaattagtt tgttcttata atatctactt taaataaatg tgccatcttt 123240
aataagatag tagacttctt tgtttggtaa tgttctattt tttggagatc ctatgagtta 123300
cacttgggaa aattataaaa gttcacttaa agttaataaa atccattaag taatgttcag 123360
aactagacat ttccaaatga gcccttgaaa agctcaggtg ggttcttttt gagagttccc 123420
caaatgttgt caaccccagg aggaatggaa gacctctgca gttttgttat tcagattctc 123480
atctccttct cagaagccgt agaactggcc gggccctaag gtccacgctc cttggttcca 123540
gttctgtctt ccatccttcg gtcccgggct cattctgcct gttcctaaac ggtggcaagt 123600
taggggcccc agcagccaac ttgtgcttac ctggcactac ttcctgggca gttttcttgg 123660
ctccttgact tgttgggcgg cttgggattt cttttatggc cctgaaagca aaagacaatg 123720
ttctctttta gtttcctgca attaaatgat gttagaaata gtcatcttca cattggcgta 123780
cttcctcttt cttctgtagg tcttttagaa ttttgagtcc attctcatat tttcttgttt 123840
catttgcttt attttctaat acatagaagt ttaaactccc tttaaagagt ttttggcctc 123900
ttttacccta ttaagctttc tttttctttt tctgttttag ttgttccatc tgtgtattct 123960
cagatatttt tctttcacct tttctggttt atttctttat tgacctgtct catctgttat 124020
tttaatgaaa tttggaacag ggctaaacag agttcctacc tcagccagta taagaatata 124080

ccgtaataac tcagagtggt attaactaga ttaaaagttt caaaaagtga tgtttttctt 124140
gtctctgagg atagaaactt caacaaaata aagaagaaat tttcaattag tagaatttct 124200
ttgaaagttt gttcattcat tcatttggct accttattcc aaattgagtc attcattgag 124260
ggcttagact atataaagtg tggttttgtt ttcccagcag ttcatgcaac agcattgcac 124320
ctagcagctg ggaagtctta tagcatgaat aggtgagatt ctaataccag aatctcctgc 124380
atgtgtaaac taacagtgta gtcttgactg ttgtctccca gtaaacttgg tttcaggagt 124440
tttagatcca tgtgaacgtg tacaaggcat ttttgctaac tgtaacttcc cacttaatca 124500
acaaaaacaa aaacactcat ttctgaacat tcagtgcatt catgattaat cttaattaca 124560
ccacaaaggt atttttcaat ggtgattttg cgggagtggg gtaacagttt cgaaagcaac 124620
attgtcagaa acatagttga ttttaaaggt tctttctggt gactttgact tctgcttttt 124680
tagaagacct tacacagagt tgtatttatt tctcctggaa tatttcaagc aattcagagt 124740
gaaagggtat acattccaat ttgcgtatga gataaaattt agttacattg agaagctatt 124800
ttctttagtt acagggaaaa aattgtaggg cttttggaag cctctttgat ttctaatagg 124860
aggaatccct gagcactggt ccaaacagaa atcatctctt cttcattgct gtatttccct 124920
caagctctta gcaaagtgca tggcacgtga aagcccggag aagctgttgg ttgaaagaat 124980
ggatggtggt gggcaggaag catcagggac atggtttgct tcagtctatt ggctgggaga 125040
aaggccattt aggaagggat ccttagatgc cactggaaga atgtgggaag tttgtgaatc 125100
tctctttctc aggaacaaaa gtagaaaaag gactccacac agcattccaa gtacagtcgg 125160
ccctcattat tcatggattc tgtatttgca aattcgctga cttactgacg tttatttgta 125220
accttcgagt caacactcac ggtgctttct cagtcctttg cagacgtgtg gaatggcaaa 125280
aaaatttgag ttatatgacg tatatgttcc cagctgaggc tgagcaaggc tcacttctcc 125340
ttgcagccct cagactataa acaagtgtcc ctcttgctat ctacttcgtg ttatgatttt 125400
tgcattttca taatccctgt tgatgatttt gctgtttaaa atggccccta agcatggtcc 125460
tgaagtactg tctagggatt ctaagacaag gctctgacgt gtcttaagag aaaatacgtg 125520
tttgataagc tttattcagg catgagttac aatgctgttg gccatgagtt caatgatggt 125580
gaatcaacag gatatattaa atacagtgtt tttgaacaga aaaacatata aaacaaggtt 125640
atgtattaat gagttggcaa aaatgctgtg accaaaggct cccaggaacc taccctattt 125700
tcccctcaat gcaatggttc agtatttgct aattcagtgt ttgaggtgac tttatagaac 125760
atgagtacca tgaataatga gaatcgattc tgtataatag agtgatgaaa gcacaggtct 125820
gggagccagc agctatattt ctattctggc gtgactcctg tgtagttgtc atcactggca 125880
aattgcttaa ctgtgtgcct cagtttccta atctgtaaaa gctacatcgt ttggatgatg 125940
tgaggattaa acaaattcat agatgtctag ggcttataac attcctggca cataacaagt 126000

cattatttt tattactact tcggaaggga attgagtact ataccctgaa gaaggtgagt 126060
atgggaattc tctacgggtc tggaatgtcc ctatatttgt ttattttgcc ttcaagtgac 126120
taactttaat accctattgt gattagaagt taaacttctg caaccaaaag gaagcaggaa 126180
gctagtattt cttgaagtgc ttattacatg ccaggtactg tgctacaaaa acaaaacaaa 126240
acaactgtaa aaaaaacttc aaatttggct gcgtgcagct gctcatgcct gtcatcccag 126300
cactttgagg aactgaaggg aggattgctt gagtccagga gttccagacc agcctgggca 126360
acacagtgag accctgtctc tacaaaaaaa caaaaacaaa aacaaaggca ctccaaatca 126420
gtaaaaatta atcaatcaat aaaaagagtg aggggcatta agtattgtgg actgaagcaa 126480
tcccagagag ggaattaatt gaagctgagg taagcagctt atggagaagc tatgatgtac 126540
agagggcaag gaaggaattt ttctgtaatt tggaaaaatg ggaactgtga gaaagaagga 126600
gttggaagct catacttagg gagcatctac aaggacgtct ttttcacgtt ggttggaata 126660
tccaaatcaa ggattatttc agaatcaccc agatgattaa aaaactactg agatccaggt 126720
tgtatttcag cagttctgac aattgctctg ggtcgaagct tgaatcagta gttaagaaaa 126780
acaacaaaca aaacaaattt tggggctttt ctcactgatt tacagttaaa gctcatttac 126840
tcttcctatg actttagatg gaggatattt ccaagtcttc aggatggaga catggaggga 126900
agtgagacta gtgatgtgcc tcaaggtttt gctgttgttc taaccatgag gagcactatt 126960
caaacccagg tctgctagat ttccaagtct tcatttcctt gggcctcttg gatttcagaa 127020
gcagagggta aaaggagtgc tggggagaaa gatcacagta gctttcaatt ctactcctca 127080
gctttccaaa ataagtttca agactggccg ttgcatttga tatggaataa atacaaagaa 127140
ggtagattga agggtatgaa gatgcagatt tttgatacca gatatgaaga taacattagg 127200
aagcaatcta aaacatggac acaaacacac acctgtgcca gttagcctgt ataattcgat 127260
ttttgttaag tgtttagata actgaaggta atttaagccc tcatatcttc ccttcatagg 127320
gttctttttc cctctggttc atcagagagt tgccaccaat tcaggctgtt agtggtacac 127380
ataacctcta gcattgttga tacagctata aaatcccaaa tatcagtaca attgttgatt 127440
gcataaaatt tccagttgca tggttggaaa gtcctgtaag tttgaatcct taaaccagtc 127500
ttaaatgtgg aggaggactc aattaaagct ctcctcgtgt cctccctctg acgtatttgc 127560
aaaatccttt ccacaaatag aatactgttt ttaatgcttc cccagtccaa ttttgcgttg 127620
tagaagacga atttatggat gagggaagtg gcattcaggc actccagctt ggtatagaag 127680
cccatggtgt ctggtcctca gtcctcaagc ccgctcattt cctcatgtga actcagaata 127740
agcagctgaa agcaagtctt caaaatctca gagatatgta taaatgcaag tgtttgggtg 127800
agaagtgaac atgggtctcc tctagtgccc acactacttg actaacaggt tttgggctcc 127860

acacaatgag ggattatcaa cccctgtccc agggctctct gggtcttggt tctttgtttt 127920
tgatgctcag caattgtgat cagtgaaacc aatgttgctt ttctatcaag agtccaaccc 127980
ttttctaaga agggttgtgt ttgatattag ggaatagcta gcaaagttat caagtaactt 128040
gtagaaacat tcttttgcaa gagttcttat actgaatgac tgtagttgac agcagtgcag 128100
tactggtcat tttctaggac atcttaaaaa cactgatgag aagtttcctc tcagatgtct 128160
gtcatgtcat tcttgccttt ctctacacag ggtcagtttt ctcttattgc tgttaggagt 128220
tcctcattgg tttttcagct tttgggcttt caaactctaa ttaatcataa gctactagag 128280
tgtactacct aaagtgtgta tatacacata tatacacaca cacacacaca tatatatacc 128340
tggtatacat atatatatat aatatacata tatcatatat actccccaac ctgatctggt 128400
tcttcctctg cataaaagac ctcaggccag tcagagaaaa catgtatgtt ccatggtgtg 128460
gcaatcaagc ccttgtattt ggttccaatc agtctcctaa ctattactcc aagaagctct 128520
tgttgaaaga gccatgttta aatggcatgt tcctactttc ttcttcatag tgatcttcat 128580
ctgtaccatg tacccttctt tcttcttgtt ccatctctgt taggctgatt ctacccagaa 128640
gtcaaggttc agctcaaatg ctatccctat caggtgaatt ttccacctgg catttgttcg 128700
gtgtgcattg tgtgcataca gcaccttttc ccggtacctt tactgtaatc accagataat 128760
tcctttcatt ttagttgtaa atagagttgt cttccccctc tatggaatag attttattaa 128820
tgtatagagc agcagtcccc agcctctgga ccatggactc gtactggttt ggggcctgtt 128880
aggaactggg ccgcacagca ggaggtgagc agtgggcatg caagtgatgc ttcatctgta 128940
tttacagctg ctccccatcg cttgcattat gcctgagctc cgcctcctgt cagatcagcg 129000
gtagcattag attttcatag gaatgcaaac cctactgtga actgtgtatg tgagggatct 129060
gggttcttct tatgagaatc taattcctga tgatctgtca ttgtgtccca tcacccccag 129120
atgggactgt ctagttgcag gaaaacaagt tcagggctct cactgaatct acattatggt 129180
gagttgcata attatttcat tatatgttac agtataatac taatagaaat aaagtgcgca 129240
ataaatgtga tgcactggaa tcatcccaaa accatcccca gttccatctg tggaaaaatt 129300
gtcttccatg aaaccggtca tggaactggt gccaaaaatg ttggggacca ctcttataag 129360
gcatattaga gtaatttcat agatttccta attcatttat catattcatt cactcagcaa 129420
gcattactgg atgttgatca tgtactggct ttggtggtag gtgcagagat tgggaacatt 129480
gtcatcaagg agtttatggt tgagtgaggg agatgacaag tggatagaca atgaaaaaac 129540
agtagaataa gaactgtgat agaaaagaga cagccaggag cattgaggag aggcacttaa 129600
ccagatggag gatcttggtc cattgatatg gaggtcaaaa tggtttaata gagcaagtga 129660
ccctttcaac tgaatttttt aagaatgagg atttagccag acaaagaagg gcaggtgagg 129720
ttgtgaagag gaactgagtg gtactcttca gagctccagc ccagttcctt ggacagaata 129780

aatgcttact aacttataga gctgaatatt gaattaataa aataagggta aactgttaag 129840

aatcagagaa ataacttaaa gaacactgat agctagtgtt ttttgaacac catgtaccca 129900

ggtgccttgc cgaaaacctt aatgatcatc ttgtttaaac cttacatttc tcataagagg 129960

ctggtactat tgttattctc attttatggg acgtagaaac taagacttgg agaggggaag 130020

tgacttgccc aaggtcatac aaccagtact ggagaattag ggattctaga tctagaattt 130080

ggactctgga gcttaaggtt ttaacccacg acattatgca gagaaattga caggattttt 130140

ctgttgctga tcaatttact tggcagttag tttgttactt ccttgtcttt attttagttg 130200

tgacaatgct ttcatcttag actgtgtccc gaggctgctg cttttatttt tatgggaaat 130260

ggctattttt atgatccttg ctaaaagcat gtttaaacaa ttttccatta agtaggggga 130320

tgtttttcct tctaatatca gaagccaata aatgaaattc tacaaagact tgctggtagc 130380

aaccttagga atttctttgc atgtgaaacc catctgagaa cttaaaatct gggtaaaatt 130440

gtagtgtaat ttggtgcaat cgtctctttg cacaaataac atcataaaat catagtattg 130500

tcatctagga ggggccttag acatgatgga atcctacctt ttatattttc caggtgaaga 130560

aatcaaagtc tagaaaggtg aaggaacttc ccccaaagtt tcccagctgg tagagacaga 130620

accagggcta ggtcctctat tctgactcct gaccactacc tcacacctaa tagatggagg 130680

catgcccagt tcctgttcac cgagggcatc agaccatgcc atactcattg ctactgttcc 130740

agcatttata gtagaagctc aagcaagcag gatgacagaa tacctaattc tggtcactac 130800

aacattataa tgatggctaa agtgaatgcc ccagccatgc ttgtctagac aggccatctg 130860

tttaattggt atatggttca cgtgagaatt tttaacctct gtttgtcgag tcggtgttag 130920

ttctctagtg atgaattatt tcctatactt ccatttagat tatttactct taatttaata 130980

accatacatt gtttactttg gtattgaaga ttcccttgtt tttcttcttt ttttctgttt 131040

ccagggctta aaggttagga gtgaccttgc cagacttccc tggagactta cactgtctcc 131100

tttcagattt ctgaagcagt tgggtgctat ttttagtcca ctatcaccaa tgtgaaaatg 131160

gaacttgcat tatttcatta tagatatttc actttagtat tgacagaatt aaaaaaataa 131220

tttgatctgt gcttgatcta gcagccaggt tacaatagac atttttagtt acctggtcca 131280

catgttgaaa aacatgtgtc ttctctgaga ctaatgacta agcccgatgt tggttatata 131340

ctgtttacta ttaaattttc cccttgtagt ttaatattgt tccaggaaat gaaatgaaag 131400

tttaataaga atggcaattg atggacccat atgtcggaag tataactaat gtccccgtta 131460

catgtgttaa agaaaggcat ggctggtggg ttgtaactgt actacaccaa gatgatttga 131520

cacaacttat tctacagaga tatatattta tcaggataga atttataact aaacaaaact 131580

atagcatttt ttcactttga ttttttttaa atgagtcaaa gaactgctag aattgtcagt 131640

taaaaaattt taaaaggaga tatgaaaaaa tcttacaatt cacaatgctg taaagagata 131700
atgtagggat taatatgttc ttgatatcaa tattttatga cttttataca tgtagaagca 131760
aaacaatttg aggtaggtga agttagtatg gacttcttga gattgtcctt cacatttctt 131820
ttcctttcgg tgaaaaattg aaggccaaaa tgtattttct tctggttttg aaaatactgt 131880
caagatcctt gcaacaaaat gagttcctct aaggagctga aaacaaagct cactcccctc 131940
gtgatactct gagaggcttt gctcagcatc ctgcattctg gtgattcctt ggagacagat 132000
gatgctaaac acaggaagat taggtcaatg gtaacttttt ctaagtcaat atttcttctc 132060
cttgggagat gatcatttta aatcttcccg aagtccaggc taaacctttc taattgaatc 132120
tccatgaagg agagctccag caggtggaga ggaagtgaga aagagaaatg aaagctgcac 132180
gcctcatgac gctgtgccag ggagttctta aaggtgaggg agtttctttt tggtaaccta 132240
agctatgtga atcagaaggt tcattagctt gtttcttttt ctttttgta aactcctaca 132300
taattttagt aaacaggaac agtaacctaa tgtgatatcc cactggccca agacttagtg 132360
catcttcaaa gttgcttaat tatgtccgaa acagactttt gtctcttgat gagaaaagca 132420
tggttaaacg tgtgatgatt tcctattgtc ctgagctcag atctgtaatt gtggccagat 132480
tcatgcatct ctgctgcctt ctcttagaag aatcatatgt aggcttgtca gataaaacag 132540
gatgcccagg taaactggaa tttcagttaa ataacaaata acattttagc atgtcccatg 132600
caatattata ctaaaatatt atttgttgtt tatctgaaat tcaaatttaa ttgaatgtcc 132660
tgtatttttg ttggttacat ctggcagccc tagccatgct gcctttctgc ttaatgggct 132720
taattttttg aaggctggag gttttctgtt atggtgcccg tttccacctg cttttctacc 132780
aggaaaggag gcatgctgat gtagaatttg catccttatt tttgtcatta ttattgatta 132840
taacagatga cataggttta gattaaacct acaatgacat tgctgtcatt cagataattg 132900
taattattgc taattgtaaa gaaggataat ttttttgaa atgactatta tttgtttttt 132960
gtttttgttt ttgtttttct tttttctaa ttatacttta aattctaggg tacatgtgca 133020
caatgtgcag gtttgttaca tatgtataca tgtgccatgt tggtgtgctg cacctattaa 133080
ctcatccttt acattaggta tatctcctaa tgctatccct ccccccctacc cccaccccac 133140
gacaggtccc ggagtgtgat gttccccacc ctgtgtccaa ctgttctcat tgttcaattc 133200
ccacctatga gtgagaacat gcggtgtttg gttttttgtc cttgggatag tttgctgaga 133260
atgatggttt ccagcttcat ccatgtccct acaaagaaca tgaactcatc ctttttttatg 133320
gctgcatagt attccatggt gtatatgtgc cacattttct taatccagtc tatcattgat 133380
ggatgtttgg gttggttcca agtctttgtt attgtgtata gtgccacaat aaacatacat 133440
gtgcatgtgt ctttatagca gcatgattta taatcctttg ggtatatacc cagtaatggg 133500
atggctgggt caaatggtat ttctagttct agatccctga ggaatcgcca cactgacttc 133560

cacaatggtt gaactagttt acagtcccac caacagtgta aaagtgttcc tgtttctcca 133620
catcctctcc agcacctgtt gtttcctgac tttttaatga ttgccattct aactggtgtg 133680
agatgatatc tcattgtggt tttgatttgc atttctctga tggccagtga tgatgagcat 133740
tttttcatgt gtctgttggc tgcataaatg tcttcttttc agaagtgtct gttcatatcc 133800
ttcgcccact tgttgatggg gttgttgttt tttttcttgt aaatttgttt gagttctttg 133860
tagattctgg atattagccc tttatcagat gagtagattg caaaaatttt ctcccatttt 133920
gtaggttgcc tgttcactct gacggtagtt tcttttgctg tgcagaagct ctttcgttta 133980
attagatccc atttgtcaat tttggctttt gttgccattg cttttggtgc tttggacatg 134040
aagtccttgc ccatacctat gtcctgaatg gtattgcctg ggttttcttc tagggttttt 134100
atggttttag gtctaacatt taagaagaag gatacttaaa gtataaggga aaatgttaca 134160
atgtatgaag ggaacatgaa gaaatagaat ctggtaaaaa agagttcttg cttttgggag 134220
gccaaggcct cctggctaac atgatgaaac ctcatctcta ctaaaaatac aaaaaattag 134280
ccgggcgtgg tggcacacgc ctgcagtccc agctgcttgg gaggctgagg caggagaacc 134340
acttgaaccc aggaggtgta ggttgcagtg agccaagctt gcaccactgc actccaggct 134400
gggcaacaga gcgagactcc atctcaaaaa aaaaaagaaa aaaaagagtt cttgctttca 134460
aaactatgga ttaggtaact tttgtgaatg agtaagatca tgagtattat aaaaatagca 134520
cctttctttt ttgtcttggg gaaattatct tattttttaa ttggatttca gaaaagagta 134580
tttcagagaa ataaatctct gaaatgcttt ttgaagtgtg aaagatttag aagacaaaag 134640
caaacctcct gtctagataa acattaaaga gatctgccct cccctcctct acctattcag 134700
gttgcaacac tttgggggtg gctgccttgg tagagcttga tcgtgactct ggtggcttgg 134760
gagatggcat gctgcacaag ggattcatgg ttacagcggg cttgtgggac tggggctctc 134820
caatacgtgg ttgggtttgt aaagaaatca gagctatggt gtgaacaaaa ggatatgcat 134880
gggagacagt gagacaagga aatgctccag aaattattgg aatataggtc agataactaa 134940
ctgtacttgt gccattttct gggggaaaat tctctgaagg ctttttggga aaagaatgga 135000
agtgagaatt ctcaggtcct caaaatattt ccttttactc agtcctaacc tgaggccgtt 135060
aaagaattcc cagagtcacg atggaaggca tgtttgggag taagagccag agtgagggtt 135120
agaaatgtgt tgttggccag gtatggtgga tcatgcctgt aatcccagca ctttgggagg 135180
ccaaggcagg tggaccacct gaggtcagga gtttgagacc agcctggcca aaatggagaa 135240
acctcgtctc taccaaaaat acaaaaatta gccaagtgtg gtgacacgtg cctgtaatcg 135300
agctcttcgg gaggctgaga caggagaatc acttggaccc aggaggtgga ggttgcagtg 135360
agccaagatc atgccactgc actccagcct gggtggcaga gcaagactcc atctcaaaaa 135420

aaaaaaaaaa aaaaaagaaa gaaatgtgtt ttccagggtt ctgggtactt aggaatttgg 135480

ttgcttttgc aggtggaagt ggaggtgact aggtaacagc tgagtgattt tgccccagtt 135540

ggacatgagc caggttgagc agaaagccct gggatgcggg gaggggggtg gcggggaagg 135600

aattgaaagt tggttgtgtg gtttggcttt ggcttcatgg catgctcaca ccttgcttcg 135660

catagcatgc ttagactaca gcaggagcat caggaagtgg atttctgagc tcaatacaaa 135720

aagttataaa taccacctat aagggcaata aagatatata gttgattttc ttctttgcaa 135780

ggccaaatct tataggaaca taagagcgaa tgagttacag cctgggaatt tgagccttat 135840

attcagagat tttaggttgc ttctgattcc gctgtctaga caaaaccatg agaggatagt 135900

gtctagaaat gagaggaagc tcttccaatg cagaggctag aatgtgtcag cctgtgctgc 135960

gaggcctggg atagatgttt ctgaaaagta aaagggcagc tttcctactg gatacttgat 136020

cctcaggctc tagaaaactc tgctttatta actttgttga cttcctaggc accacatggg 136080

atccttgttc ttcctccttg taagcagtaa ttgaaatcag tttggcagcc tggtttacag 136140

tgaccatggt ggcttgtctc ccgtgctctt acctcactct gttgatgttg taaaacctcc 136200

agctaacttc atggggtggc tgacccacgt tgctcattta ttcattcaac acatattcat 136260

tgaccatcta ctctatgcca ggtattgtta tcagcactgg gaatagatca gtgaactatt 136320

gatctatttg tctaatggga caaattgaca aattgggaaa gattccatta cacaggtgac 136380

atttaagcaa agtcttgaat aagggaggga atagtaccat gagatatcct ggtgaaaagc 136440

aatttaggct gagggcacag cagggaagag gccctgatgt gggaacatcc ctggtgtctt 136500

gaggtacaga ggccagcatg gctggcacgg agtaagaagt tggaggtgcc gggcatggtg 136560

actcacacct gtaatcccag cactttgggt ggctgaggca gatgggtcac ctgagcccag 136620

gagcttgaga ccagcctggg caacatggtg agaccccatc tctacaaaaa aatacaaaga 136680

aaattagcca gatgtggtag catgcatctg tagtcccaat tgcttgggag gctgagatgg 136740

gaggatcaaa ttacttggga ggctgagatg ggaggatcac ttgagtccag gaggtggagg 136800

ttgcagtgag ctgagatcat gtcagggtga cagagcgaga ccctgtctca aaaaaaaaaa 136860

aaaagaaaaa gaaaaaagaa aaaaaaagaa gttggaggtg agtaaggaga ggaacgtggg 136920

ggacagagtc ctcaggactc tggcttttac tctgagtgag tcgaaaatcc aattaaaggt 136980

ttgaaagaga ggaatgacct gatctgacat tttattgtga acgttttcaa atctttacag 137040

aagtggaaga gcataacgat ccttcatgta cacatcgccc agcttcaact atgatgtttc 137100

atttgtaaat atttccgtct acacttccaa aggatgatga ctatttttaa aagtccaact 137160

ataataccat tatattttaa aagttaaaac actatgtctt taaatatcaa gagtttgtat 137220

tgattcgcac tttgaaggtc gagctgatga aatttcctga ggggttggat gtgacatgag 137280

agaggagtca agtattgcat ggtaattaaa aacctttgca gcatagtcca tttaccgaaa 137340

gactatatgt atgcacttca aagcaggttt taaagattaa catcaagcat ctggcttcat 137400
gagttttaac ttcttttcat aaatgttata caatgtcatc atctctccag ctagagaaaa 137460
tgctattatt cttattttca aatgaggaaa atgacgcaga attatttaca tattatgtaa 137520
cttggtccca agtcccttag atactggttt agaaaatcct agtaaactgg aagtgactta 137580
tccaaaatta aatttatttt tgctctattg tcttttgttg cctatgggaa ctttgtgcag 137640
gtaactaggc acatgtcagg actgatttac tgacctctca aggtatcttt aattattttg 137700
ggggatatca cggaatgagt tctacacaat tcatttgaat cgaattgaac ttaagaaaat 137760
tcaaatgatg cattggctgc ctcctattta ttacatgctg ctcataggca taacagcata 137820
gtctaacaag tataaaacct gtgtaactgt agctttcagt gcagtgtgat gagggctgag 137880
aagatagtgg tacaaagaag agaggtagca gagtgaagct gagtcaatat gatgaagatt 137940
tctctagact tgaaagggct agaaaaggtt attcttggca ggaaaaaaac atgagccaag 138000
gcataaggat aagcacaggc atggcagatt tgggaatgtc atgtaatttg ttgctgggct 138060
gcaaagtaca tggaagggga gtgaaggaac agaaggagat gaatctggag ggagaggtta 138120
aagtgttcca gagagcaata tgtaggtgtt actctaagtc aaagaggtcg taatagcatg 138180
tccagactcc aaaactctaa acaagtcata gaattgctgc cttggtaggg catatcacac 138240
acatcaaccc aatcctctgt caccatgaca tccatataac tgcaactcta tacatttccc 138300
agcctatgtt cccagagtct ccagatgaca ttgtctgcaa actgcactgc agaaggctct 138360
gctatgtctt cttaaaagta agcaagactg ttttcctttg ttacatgagc agcaaaagga 138420
tagggtgctc tttgacctca cttactgtag ggtggatagg aaagtcaagg aagagtaacc 138480
cagaagattt agttttaact ttcgcatcaa agaggtccct tagcatctgc tcagagatgt 138540
cacaatttct ggtgtgtgat tatgtttaag aattcggcct tgccactgtt gaagttgttc 138600
tgtggaaaaa gaacctctct taattttaca tgatgcccaa cttctctttt attccagaat 138660
cactcatatg ctgttggact ctttccagcc atgtgtgcta acctaggcaa tgtcataata 138720
gatgaattat gtttactttg tctttgatat ctcagctctt ttatcttcta ttcaagttcc 138780
cacctccatc attactgata gtgttcgttg aacaaagaat atgtcagata tacagaagtg 138840
tttctcccct tttctctgtc tctcttttcc ttcctttcct ttagtttcct ttctctgtct 138900
gttttctgat gcctcatttt agaaaagtga ttttttttgt gggaaaatca ttttagcatt 138960
agaaacgcaa tggctatcac tgacagcttc ctctgatgaa acggccattt gtcatcatta 139020
cacggtcatg ggagtgctaa gaagacttaa atgcagggct accacccctt cccaattcat 139080
cttttatcca ttttatttct ctaaggaaag ggtttgaaaa atgggctttg ccctcttgga 139140
tgcagtgaag aaattctagc tggctacaga tgttattgtt ggtcggaggc aagggataaa 139200

atcatggtca caccattgta gcgccagatg gggaatgtag caaacatagt tgtaatttct 139260
cattttacag atgaagaaac tgaggtgcag aggggttcgg tgacttgttt aaggcatgta 139320
attgttattg gcagctttct gttcagaact taagagtatg agtcagtcta gatcttttca 139380
tcacaatact ctgctcctct tactttttcc tgaaatttgt cacattgaca gcaatgtgat 139440
ccctaatgac acacagattc ccaaataatt tttgtagtaa aaatttccat ttgcaattct 139500
ggacatgtgt gtgtgtggaa ttttatgtga tgacatattg gtcctatctt ttgaatagga 139560
tcataaatga aatgacttat ggatcacatt caaaagcagg ccaggggcca atgtgtaagc 139620
aggtgggttt tcatatttgg agttctgtac ttttgtgtta gtcagtgggt ctaggactct 139680
tgtagtgtat ttcccaaggg ccaaagtctt ctgccttgag gtgtcagctt tccaaggcag 139740
aggctggatg ctttctcttc cttctgggct cctttctctt aggcttcccc cttctcttct 139800
cctccatttg tatctgtcct ttttctcggt actttccctg gctggtctca gctagatgct 139860
cactcaatgc tgttgaataa atgaatgaat ttcgtagtaa ttctgcaggt aaatcaagtt 139920
attgtctccc aatacggtgc tatgctttct gaggaaatta gactggaagt caggcttttt 139980
aaaaaagaag atgtggtgtc aaattgcagc tctctctctc tctcgactta cctttttct 140040
atcatccata tgccttcttt cttgtatctt tgggttccca gacctcacca ttcattagca 140100
cttggaatgg attggtaaga ataaagaaag gagaggtggt gaaactcagc ttgggtcatc 140160
tggttacaca ttagtaactg acaaaagata aaaagataca gactaaatgg gtttttaggg 140220
aactttttcc agtctattct tgtttcccat tagtgtgaaa aatcaacact tgcttgtatt 140280
ttggggtgaa gacatttttc ttaagtgagt gggaaagcct cttgacattt taccgagagc 140340
cttaaatttt gaatggtgaa tgctaatgtt ctttgtgcat aaagaatttc agaacttgta 140400
tatatgagca ttaatgatgc atcattttct atttgtgagt taaactaggt attatctgta 140460
atcatatttt taggaaacat tcaaactttc atcaagtcat tctcttatat gactctcagc 140520
tccattaact ctgttttcat ggaactcaac agagttctta acgtttgcat tataaattaa 140580
attagcattt cccctcaaag aagtattgct gtccttacaa taaataattg tagacaattt 140640
cttttctttt cttttttttt tttttgagac aggttctctc tctgtcaccc atgctggagt 140700
gcagtggcac agtcacagct cactgcagcc ttgacctcct gggctcaagc aatcttccca 140760
cctcaacctc ctgagtagct agaattatag gtgcacacca gacctggcta atgtttaaat 140820
tttttgtaga gttggggtct tgctatgttg cccaggctgg tctctaactc ttgggctgaa 140880
gcattcctcc caccgcagcc ttccagagca gtgagattac aggtgtgagc taccatgccc 140940
agctaattgc aggtgatttc taatgggatt tagtatttct gggtttaagg atgagatctg 141000
aggtaatgac tttgtttcca gatgtgaaat aatttgctct tgggttgtga gccctttggg 141060
tgggctccca aggatcctgc tctcttccag gagcccaggc tctggggtca gactgcctgg 141120

gtccttgact ccctgttttc tgattgtaca actttggtga gtggcctaat tcctctgtgc 141180

cttggctacc ttggttacta tttctaaaac aactggtgtt gtagtagtac tgcttagagt 141240

actttcaagg gttaaatgaa ttaatccatg taaaacgctt aaaatagtgc ctgccacaac 141300

catcaattta gtgtgaaaat ctgctcacct gcttggccag cccctttcac tttattaaac 141360

caagggtcgt gctgggtttt ccagaagtct aagttgcggt ctaatctttg tgcagaagct 141420

gaaatagcag ccataacgtt ctccctagat gatttcgtgg agcttctttg aactgtatct 141480

atctccagtc atttttgtgg aagaaatttt cttctgtact ttttagggat gagaattacc 141540

tgccttggtt tattaactaa aagacaccat gattacaaat aaaattaaat aaatattgta 141600

tcactaaata gataatatga gatagatgta ttaagttttc agataaacag tataaaagag 141660

ctagagtaat ttgtaaaaag ttgggaggac ctattttgtc atgcaggaaa caatttttaa 141720

cttgcctacc ccagaacata gctaccacat ggttagggtt tgcccaaacc tggcccagga 141780

gtcatttacc ttgagctttc ctaaaaagga ggatcaggat tttcctctcc agactctatc 141840

attttaggta gagtccttct tgtcaattct ttttaagaac atacatttac ttttgtggaa 141900

aataaataga tacaaaataa atacatacaa aattgcatag caattagaaa tacccaggag 141960

gtatgttatg gtcacagaca caaactgcct ccaacttctg tccatccata gtgatattta 142020

aagcagagag aggtacacag gtaaccacat ttagatggac tgggatgttg ccacacatac 142080

aagcattgat aactggcttc tcattacctg aatacattct tctgtcagag caacagactc 142140

agctatgctt ctggcaaaat tgttcttaat tctctattga ttaatttatt cggtaagtat 142200

ttattgggta ttttctgtct gaaaagtgcg attccaggtg ctttatgtgt ctctgtgtgt 142260

gggtgttata taaatactta taatactgta tccatactct tgaaaagctt agttgggaag 142320

gcaaggcatg caataaggaa cacagaattt tagtcattcc acaaccatct gttgaatggc 142380

tgctattgtt agtatcgtgg tggaaactga gaagcaaaga tgactataat aggatctctt 142440

ttctggagat gcacagtgga cacgtagtta tatgatgatg ataaggactc cagaatagtt 142500

ctatacatga tgctctgggg ccacatgcag attctgatga gaaacaatta actctttttg 142560

gctgctacct gagaaggggt aattgtcact caggaggttt ttgccttttg accaacatag 142620

aaaggagtgt gagtgaaggc tagaggtgta ctaacttggt cagggcaggg tgacacataa 142680

aattaaccat cacagggaag ggtagggctg gagaggcaga ctgtggccag gttacaatgc 142740

gctgaggcta aggagactgt gtttatcctg taggccagtg ggtcttactc tgaagtcttt 142800

tgggtgggac attcatggac ttcaagagac ctgtgaatgc cctaagatta taagtaaaat 142860

ctgtgagtct gtaactaaag ctaaagctat ttttctgggg cccaccatct aaagaagatt 142920

ctgaagcctt agggtagccg tggaggagac atgaaggtcc attttgcatg gtagaaccct 142980

gcctggctct tgctgcagtg tgggaggaca ggtttgcaat gtggaggtgt ggcaggcatg 143040
gatttgggag gattggcaga ggactcacca tgtccataca ctcactgaga tggcaaatat 143100
ttattaatca tccaactgtg tatcagacac taagaataag ctgggaggcc atggcaagtg 143160
aggtcaccac agtccctgcc acagtggagg ttatggtata caggtaaggc agggaagagc 143220
actgcaaagg gtttgcccat tgcatcagtc atttatttat gcacatgttg attcaacaat 143280
tatttctatg ccaagctgtc ttcaaggtgc tggaggaaat gaagcgtaca tttcactggg 143340
gaagacagac aataagtaaa cacattaaaa tctggcttgg cttgatgttg gggaggggtg 143400
agtgccatag agaaaacaaa ccatttatgc agccaacaaa catatgaaaa aaatctcatc 143460
atcactggcc attagagaaa tgcaaatcaa aaccacaatg atataccatc tcacgccagt 143520
tagaatggtg atcattaaaa agtcaggaaa caacagatgc tggagaggat gtggagaaat 143580
aggaacactt ttacactgtt ggtgggagtg taaattagtt cagccattgt ggaagacagt 143640
gtgatgatcc ctcaaggatc tagaaccaga aataccattt ggcccagcaa tcccattact 143700
ggctatatac ctaaaggatt ataaatcatt ctactagaaa gacacatgca cacgtatgtt 143760
tattgcagca ttgttcacaa tagcaaagac ttggaaccaa cccaaatgcc catcaatgat 143820
agactggata aagaaaatgt ggcacatata caccatggaa tactatgcag acataaaaaa 143880
ggatgaagta atgtcctttg cagggacatg ggtgaagctg gaaaccatca ttctcagcaa 143940
actaacacag gaacagaaaa ccacacactg catgttctca ctggtaagtg gaaattgaac 144000
aatgagaaca catggacaca gggacgggaa cattacacac ctggggtcta tcaggggggtt 144060
gggggctaag ggagtgatag cattaggaga aataccaaat gtagatgacg ggctgatggg 144120
tgcagcaaac caccatggca cgtgtatacc tatgtaacaa acttgcacat tctgcacatg 144180
tatcccagaa cttaaagtat aattaaaaaa aaaagaaaag aaaacaaacc agtgtaagag 144240
gatggaaagt aataggctcg tttagaatgg tgtgagaaag ccaggcaggg agaaggcgct 144300
gagacaggga ggtcctggat gtgtttgtgg aagagctgtg gcagcacctg gaacttgggg 144360
agcaagggaa ggagtgtggg caggcaaggg tgagggtgca gggggtcatg ctgggccttc 144420
caggtcacgg aaggacttga gctttactct tgttgtggtg agaagctgct gagggcttgg 144480
agttagggga gtgaaaagat ctctactata atagggagag ttcgggatct gtaacttaac 144540
cccaggagcc agcaaagctc cctggaggaa atgcagttta agctgagaat gggaggataa 144600
acaggtgttt ttcagagaag aggaagggtg ctctaggcac agagaacaac atgctggaat 144660
gcttctacta gatcataggg gcaaaatggg agtgcaggag taggagaggg ctttctggga 144720
aagatactta ttttaatttt gcatgcattg agtttttgag gtttctttgg tttgttcatg 144780
tggaggtgca gagtgggtat ttagcacata ggtctgaagt ccaggggagg ggtgtgggac 144840
agcagttgga tgtggcagag attccacaaa gagcaaatat catctgagaa tggcagaggg 144900

ctgagggcag agccctgagg aacactggtg tttaggagcc tgctggagaa agaaaatact 144960
gcaaagggaa cggaagtgga gtggttgcca gacatagaag ctagtgtcta actagatgtc 145020
atgagatgtg gggaaggtgt tacgtatcta agaatgcaaa gttgaacccc tgtgaactgt 145080
aatacttaag ataagtcgta taaattgtct ggaactagag cttgattttc caggagagat 145140
gaaatgtgtg taggtgacag gaaacaatga atatgtgggc gagtgtagtg tgagcaattt 145200
ctcagaggtg aatttgacag cattttgctt aggaagctac aaagagacca atgctagttg 145260
gtgcaaggaa ttcaagaatt tggacttaag tctatataat gatgattttt ttttttaac 145320
ttgagtttcc cggtttatca ctcccagaat ataggcagaa gtttgagatt tttatgtgta 145380
ttttctggaa aagatagttt cagtgttttt tacattctca aacaggttta tgatccaaag 145440
aaaaggcagt ggtcacagat acatgaaacg acaaggtatt caaaggagaa cgttgtactt 145500
tatgacagtt ctttgggcag tggcttgcag gatgagtttg aggaatgatt ggaggcagga 145560
gagtaattct agtaattcaa atgtggagta ttgttgatct ctcagacaca aatggaaaaa 145620
caaggaattc aaagaaagat aggcagagtg ttttgaagaa ataattgatg aaatttggta 145680
atgagttaga tgtaggagat atatttagca aatatttatt aaggactgta ttaatctgtt 145740
atcatgctgc taataaagac ataccaagac tgggtaaatt ataaagaaaa agagatttaa 145800
tggactcaca gtgccacgtg gttggggagg cctcacaatc atggcataaa gcaaaggagg 145860
aacaaagtca cgtcttacat ggcaatagag tgtgtgcaag ggaactgcca tttataaaac 145920
catcagattt catgagaaat attcactatc atgagaacag cacagacaaa agcctgccac 145980
catgatttaa ttacctccca ctgagttccc ccaggacaca tggaattatg gaagctacaa 146040
ttcaagataa gatttaggtg gggatacagc caaaccatat caaggaccta ctgtatatgg 146100
ttaaaattgg gagcaaatga gacatgattc ttgccttctt ggagtttact gtttactagg 146160
ggaacataca cttgtcaata atcacccaaa tataggattg gaaattgtgg taagtgccat 146220
gaaaaacaag tatagggaat tttgagtgta catagctttg gggacttgat ttgatgaggg 146280
agccttatga agttattgca ctagaactga attaaaccac atttctagga agtggacatc 146340
tatttgttgg ttctttaaat ttagctttac agaaatattt cctttaaaaa ccaaggcttc 146400
ttaaattttt aaaactgctt ggctaatcag gggaataatg cttttggata gctggtatcg 146460
ttatttatgg ttggaaaaac aacagtattt gattacattg agctttaaac ttttcctttg 146520
attaatgaaa attttattgg cccatagttt ttattatgct ctgtttttac ttggtccaag 146580
agattctatt ctctggaccc aatatgaata ccttcagaca tccctctttt tttttttttt 146640
tttcacccag gctggagtgc actggcacga tctaggctca ctgcaacctc tgcctcctgt 146700
gttcaagcaa ttctctgcct cagcctcccg agtagctggg attacaggca cctgccacca 146760

cacctggctt atttttgtat ttttaccaga gatggggttt caccatctcg gccaggctgg 146820
tcttgaactc ctgacctcat gattcaccca ccttggtctc ctaaagtgct gggattacag 146880
gcatgagcca ccacacccag cccagacatc cctcttaatt atgttgaata tgtaatatcg 146940
gtgatttcat ttgaaaatat ttagtagtcg aactagatca aggcagttaa gcttcctatt 147000
tccatagatg cagtggtatt gtgtcttttt tatatgatct ctcatgcttc tggacatcct 147060
tttttctgct attcttcatt ccttagctac acttggtgct tcgtggttgt aatgcattgt 147120
catagatgcg ttcatttctc attcgatctt cagctctatt tctttccaga gaatctctac 147180
aggcatctgt taggttgaag gacatctaat gtcttaatgt gtagcttggt aaaccagtca 147240
actttctatc tgagtcttaa gagaaagtgt ccaagatgag aaacggtaca ggtttggtga 147300
caactcagtg agaaaaagaa gaattttaca aggaaggagg tatcttagta attttgctaa 147360
agaagtaggt aaaccttcac ttataataaa gggatagggc tcggttaggg tttgtgaagt 147420
ctccccttag gaaagcaaac cctgaaatat tttgaatctt ttaaagaagg aaaataagag 147480
tcttttaaat aaatttttaa aatttatttt atatattttt tatagacagg ctctcactct 147540
gtctcccagg ctggaatgca gtggtgcaat catagctcac tgcagccttg aatgcctggg 147600
ctcaagcggt ccttctgtcc cagcctcctg agtagctggg actgcaggca tgagccaatg 147660
tgcccagcaa gagacattca ttttggtact gtgatggtac agaaaaacaa agggcctttg 147720
aggccgaagg agcagaagaa ggatggactt agacatggta taggcacttt ctactaaaga 147780
gctgtgaagc taaaaatgcc aggtctatga caggtgcagt gggccaaggc caggtagaga 147840
gcagcaggaa gagaggaggt ggggacctgt acctaggccc atctgctggg actgatctag 147900
ccataggtac tcagagaagc ccagattggt gcctgaccca cccttatggc ccagacatgg 147960
acacctccca gtctgttcct tcctgctgcc catggatggg ctgtgttagt ctgtattctg 148020
aggacacagc tctctgtcta gaggaagtta tgttatcttg atctgatgga tactcaacgt 148080
gaacattatt tcaacgtgcc acagggtctt ggagcccaga ggaagaccgc tcttgccttt 148140
tagtttatat tctttgtttt tttttaaata acattttgac agtctttatg gagtaagtct 148200
gggccaaaat gataattgac aatgttattt acatggattt ctaagttggc taaaaaagtt 148260
cctttatggt tagtgaatat agcccatgta gtttccccgt cttctttaga tgccttctat 148320
ttctatgccc aaagtctgca gttgattttc agtaagctgg gggtcatctt agagataaaa 148380
tgtagatgaa tggcattttg ctgacagcat acatctttgc tatttctgag gaaaatgggc 148440
tctcgctatt aaatcttttg tcaatattta taaaaatagt atttacatat tctatctata 148500
ttgtggaaac tatacattta ttgattcagt catttgatat caatgttgtt gagtccctat 148560
tccaagtgag gcactatgct ctaagcacat ggcattttaa agatgaataa gacaccaaga 148620
actttgcaga tagtaatgga aatgagaatt aatcaattga agattaatat agtaagtagc 148680

agaagagaaa taaaaaaatc ttctagagag ttcagaacag ggatgttgat tcaagtttat 148740
ggggattagg agtggctggt aagggaggca ttcaggcaaa agacataaaa atgcagtatt 148800
cccctcgcac tcattaggat ggctactata ttagaaaaag aagagagtaa gtgttggaga 148860
ggatatagag caaatagaaa ccttgtgcct tgttcatgag aatgtaaaat ggtgcagcca 148920
ctgtggaaaa cactggtgat tcctcaaaaa atcaaaatag aattatcata tgatccagta 148980
attctacttc tgggtatata tctaaaagaa ttaaaaatct gggtcttgaa gaaatatttg 149040
tatactcata gttatagcaa cattattcat aatagccaaa aagtagaagc aatccagatg 149100
tctatagatg gatgaatggg taaacaaagt ctgtgtagta tatacagaca atggcatatt 149160
agtcacatca tggaccttca ggacattatc ctaagtgaaa tatgctagac acaaaaagca 149220
aaagtagggt ttcacttaat gaggtatcta gaattgccac attcacagag aacaaaagta 149280
gattggtggc tgctagggga taggggaagg agaaaatggg gaattattgt tgaatgggta 149340
tggagtttca gttttgtgaa atgaaaatgt tctgaagact ggttgcacga tgatgtgagt 149400
atatctaaca tgattgaatt gatgaacact taagcgtggt tacgatggta aattttgtgt 149460
tatatatatc ttaccacaat ttaaaaaata tagcatttta ttatgtaggc gtgggtggga 149520
agatacttga cacattggaa cttctggcca tgcgtatact gttcactcac ttattccttc 149580
attcattcaa caaacatgta ttgaatgctt gctatgtgct gggcactgag ctagatataa 149640
caattaataa ggcttataag acattgaatc tatcaatttc atgcttgcta aatatctact 149700
cccacctcca aaggcactaa gcttctacag ttagatattc atagctgctt cctactgact 149760
tgaatcatgc ataggatatt agtaaacaag caataaaaag atttgaggtt gatgggggtg 149820
ggttcaacag catggtggtg aaatggaaag agatgggtaa cagaatatga actagaattg 149880
aaaactgtga gccagtgctc tctaatgaac attaaaaaat aaagaattcc tatttgaggc 149940
tgccaacctc agaactaagt tatttagaat ggacgaaatt ggcaaagtca gacgtactca 150000
acccaaggag ccaatatttt gtgaatatta tggcaaatgt agtttgagaa ccactaccac 150060
aaaattgtga accataataa tgactgagaa ggcagggaga ggttatacaa tttgggctaa 150120
aaggaaagac agggcttgtg aaggggagcg ccagtgaaag tcagtgtggt tcgggtattt 150180
gggtggggac tggaagcagg aagcttgagc ttcctttgcc aagagaccct gctggaaggg 150240
ctatcatcaa ttgactttag ctcatcttag gattttcatt ttttaaaaaa tgttcacagg 150300
aaccttcact ccatctatac tttcaatgtc tgcctacctt tctttcttat acaactttga 150360
acactctctc cattcattta aatatattat ggagtgccaa ctacatgcca ggtactgtgc 150420
tgggctctta ttccaccttt atttgattgc acatgcctgc caagtcctgg gccaatataa 150480
catctactcc tatgtctggt ctggcgagag atgcaaactc atcttcctct actttcctta 150540

cctccttcct tccagtcttc ttcaagttgt cttcattgag gcaatttctt ttacctgtgt 150600
ttttaatccc aactcctcta gtttccttct tggctttatt cttttatctt cctctttgtg 150660
ctttcaaaca ttccctttct cctggcccat gcccttcagt ctacacgagg ccttctcaag 150720
tctcttcatt ctaaaaaatt cattttcttg ggtcttatat tcttcagctg ccaccctatc 150780
tgtatctttt cctattctcc tccaagttct caaaggaatg ccttccctca ttttcatctc 150840
cttacattcc atctgctgaa ttttggcttg tgcctgtacc tgtctaagga aactccttgc 150900
taagagtctg ctttgtcagg tctgaattca cttaaccagt ctttgctttg ttggacttct 150960
ctgccccatt tgccattctt gatcatcctc tccataaacc tttctactta aagcatttta 151020
cttccttatt ttcttggttt tcctagaatc tccttactgt tcattttcag cttcctttct 151080
gtgttcctct tctcttccta catttttttt tagctttcta ctttcttaaa gcattttact 151140
tccttatttt cttggttttc ctagaatttt cttactgttc attttcagtt tcctttctgt 151200
gttcctctga ttgtctctct ttctacattt ttttttctg tgttcctctg attttcacgc 151260
agtctggagt tgtcatgatc aatcatagcc tactgcagcc tcgacatcct aggctcaagt 151320
gattctccca cctcagcctt acaagtagct aggactacag tcacacatca ccattctcag 151380
ctaatttttt taagaagcat ttttatagag atggagtctt gctatattgt gcaggctggg 151440
ctcaaactac agggcttaaa caattctcct gctttggcct cccaaagtgc tgggattcca 151500
ggcatgaacc accatgctca gtctctacat gttcctaaag aggagttttg aatattgaag 151560
aacagtattt tcaaattaca ttattcaagt tataaaaact gatatccagg gttatgtggc 151620
aatgacgtaa aaatttgaat tgttattttt ttgacacatg ttctgtgttg tccatcagtt 151680
catctgagtt ccaaatgtcc cagctgtttt atgctttgtc tctgtttccc agagaccctg 151740
agtgtggtct agagttggga tgagcattgg tctctaatgg ttctgaaata attgtatatt 151800
cctgcaaaaa cattaagtct attagaaacc agctaatttc attttgtcat ttttataggt 151860
aacatattct ggtgcaggta gtatgttttt aaaacaagtt tgcaataaac aatttcccct 151920
caaggttaat ataataggca acaccttttg ctgcaacaga cggcaagagg taatgaaaga 151980
ttagcttaca ttatgattca ttatttcaaa atgtcaggat aaagtggatc tgctgcatct 152040
cccagagagt gcatgttttg cttttctaat gttaatggat ttactgtttt tttcccccca 152100
ggccaaattc agataatcga cgccagggtg gcagagaaag attggccagt accaatgaca 152160
agggaagtat ggctatggaa tctgccaagg agactcgcta ctgtgcagtg tgcaatgact 152220
atgcttcagg ctaccattat ggagtctggt cctgtgaggg ctgcaaggcc ttcttcaaga 152280
gaagtattca aggtaatagt gtgttgaaaa cgacttctat ttttgatcct atgagcagat 152340
cctaagagcc aaagcgactg aggaaggaag acatagaatc agccatttgt acaaaacatg 152400
aatccctagt aggtccacta gtatctttgg tagaaacatg gagaagagac aggatctcag 152460

gagaaggagt tgacacatgg cagggcagct gaggctgagt aattccgctt ccttcctttg 152520
gcaagactca atcagtcttg agcaactcta cagaagaatt ccactagctg gatctctgag 152580
gaaaaaagaa atgttgtctg tgccctgact ggggaatgcc agatggacat tcatgtttgg 152640
taggcaactt tgcctatatg atctggtata tgctgttaat tgtccatgca taattatctc 152700
tctactcagg ccttgtccag gcaaatattc tgttttgttc tagtttagct tgttctcccc 152760
tttctctctt ccatctcttt cttgtctcaa tggatgacag gatattttgc tatgagctga 152820
ctcagtggtt ggtgtcttgt aatggggaga tatcatcttt atcaaacagt tattaagtat 152880
ctacctgtag catttcattt tcccgcctgc ctccattgtt ttcttgtcta tagtttgcca 152940
attatagcta atatacggag agctatactt tatttctact ccagaaatgt ctctattatt 153000
gcattataat aggataccct ggggaaacac taatcatttt tactacctaa aatacctatg 153060
ctgaatatcc tttatctgat aggaacagag atctgacagc agcttaggct aaccaaattc 153120
atttttatc ttaagtgtgg ggcattttc tctcttctta ttctttacct tttcagctta 153180
agtgaaggtt agtataaaca ctaagaatat ttctgatgga gttttcatgt gattccttct 153240
acaaaaaccc agatttaagt aacttgttga aaaccagagt ccgctaagtt aataaacact 153300
gattgaagaa gtgattctca tggactttct gtgatagctc tttcctgccc tgatatgaga 153360
tgaaagctgg gggatggtat atagtattta tttttccttc cgttgccagt gggacttttt 153420
tttttttttt aaaagctgtt catatcttaa tcgagtagca tgtgaggtca acatggtcta 153480
ttttaaaagc atttcttcg acacattgct tttaacatct tttagaactc tgctgtgaga 153540
cacatggact tttttgttgg tatttttata caattaatga tattctcaat agtaatcttt 153600
gtgtgtgtat atatatagaa ataaattcta aatgtaagtt aatatattta ttattttct 153660
aaacatatat aaatatatat atgcacacag gctatttaat tttattagat gatgctattt 153720
taattcagaa aaaaatgaca tttatatttt gatttaggtt agtataagcc cttagaggtg 153780
ttttgacaac tctcttaatt tgtggtttta ctgtttattt gattttatat aatctaaaat 153840
accattgttt ttaccaagca tttaatttgg cagtgaaaga gcgtctgaca gaggtatggt 153900
tagtagatag gtctaactgc acaactggat ggattgagct gagactgttt cctcatcagt 153960
aaaaatgatt tgaagcagtg gttggcaaag tttttctgta aagggccaga taataatatt 154020
ttaggcttta caagggccat gcagtctctg ttgcagctac cgaactggat tatagcctgt 154080
aaggtgacct gtaaacacat ggaagtgatt atgtgctaat aaaactttat ttatcagaat 154140
aggtaacaga tcagccctgg cccgtggccg atccctgatt taatgtttat ttatctgatc 154200
taaatacctt tatttatgga agggaatagg ggattttaa atctaaagtt ttgattattc 154260
acattttact gagaacttac tctatacctg attagatgtt ccgagagaaa taaaaaaaaa 154320

gtgtaagaca taatccataa taccacaaaa tttaaaatgt atttaggaaa tttatttgag 154380
gaagtaaatg tacttgttct catgatacaa tcagaaagta agtcagtatt gataaagtgt 154440
tacctgtatg agaaagataa ggaaaacaat agagagatgt aagaaatgaa aataccagtt 154500
ataaattaaa attattaaga ttgaaagtgg aaatgatctt cctccgagaa acaatggcaa 154560
tattctcaca aattttttac atcatttttg ttcagcattt aagataaaat tatataaatt 154620
cccataacat ttagtattgt ctctaagcat taagaacaga aaaaacagaa ggaaaatata 154680
tttctaaaaa tcaacgaata cagtgtgaga tgtttcattg gtatggcatt atctcaagtt 154740
caaacatttt gaaaaatgtc tgcttactct ttgatagtta aaaacaagta tctcagctgg 154800
cgtggtggct caggcctgta accccagcag tttgggaggc tgaggcgagt ggatcacaag 154860
gtcaggagat cgagaccatc ctggccaaca tggtgaaacc ccatctctac taaaaatatg 154920
aaaattagct gagcgtggtg gtgcacacct gtagtcccag ctacttggga ggctgaggca 154980
ggataattgc ttgaacctgg gaggcagagg ttgcagtgag ctgagatcat gccactgccg 155040
tccagcctgg tgacagagtg agactccatc tcaaaaaaca aacaaaacaa caccaccacc 155100
actaacaaaa acctcttatc gccgtcttgt atacgcagac cagctagtag aattttactg 155160
aaacagtagc ctataaaaat gcaattccac ttggtttcag aaacttcttg tgtatcatag 155220
tgtgaagtca cttatcttag gcttttaaaa tgggataaat attgagtcca aagttctgga 155280
agaagcctag aaagaaggca gagttattaa cttttagata tagggaggaa ccttaaaatt 155340
attcagttct tcattcattc acttattcat tgactagctt tactaacaaa gccctatgca 155400
agaccctgga aatgcaatga tagaaaaacc tggtccctac cctcacagaa cttgtgaggt 155460
aaagggggat acagactgat aaaccagcaa ttagatgatg gtgtcaagat agaggtgaag 155520
gcagtgtctt ataggatcca aactccactc agtcctggtg gtggttgagt ctggctatca 155580
gaggtttcct gattaaatct ggagggtgag tcaagggagc atggtgaaga aggagggaat 155640
gcatgtttag ccatgtgaat gagtccatga gtgaagacca ggaggaaagg cagagcgcgg 155700
ggaattctat gcgtaatatt taacaaaatt aatgtactgt taaacaaaga catttctggg 155760
ccatggattt aatcctagac tgtgtaaaaa ccaagtaatt gatttccttt atactttaaa 155820
agcatttcca tgtatttgat ttgtttgtgt gtataaaagg gaaataccac aacaagttta 155880
agggtttcta gttctgcttt ctcatcatag tcttgataac ttggaactaa aaagtttttg 155940
ctgaaattgt ctgtgactct ttataaatca cactgcccct caaacacatt taaggatggt 156000
gaagggtctg acacgtaggt gggaagttct gaagatgccg cagctctccc tgttttcctt 156060
gttacttaag aagagagcta gaaatgagtg tacatcagat tattctcatg ttctaagtgt 156120
tttggttgaa gaggtaaagt gtttggcttg aaagcataca aattttcatc cactacttag 156180
tgtacaaact tgattactta agagattgag taatggcctc cagtgaaacg cattcttttt 156240

aaaaagcaaa gtgaaggatg ctatttaagt cagaaggggc aaaattggat attttatgag 156300
tttattaatc attgcaggca tagaagtagt gttccttaag atgtgtttta gacagagtcc 156360
ctgggatgag ttatataagc agatctggtt gtagcttcag cagccagata ctacctttga 156420
gtattacttc aaggaaaaag gactccactg agctcactgc ttctctttca ttattatttc 156480
agaaggttgt gtggcgtaga gggggctcag gcctacctat acaccactag ctatgttgcc 156540
attttatatt atttctataa ggtgccaaca gaagctgctc atcagatcag acagacatag 156600
cccaggcaag tattgattta cagatgatct ttggccagga agacatggta tcagggtaga 156660
gtctggttat gggtcaatgc agtggggacc ttaggtccta caggtataac tgagagcctg 156720
atccaccagg ccttagaaag cttcagggtg agacagtcca gcaccctgga tagctccttt 156780
aacagctgtg gccggtaagc aggcacttat ttgctaaaga actcaagccc atttagctgg 156840
cttcatctgc tttgtagagc tctgttaaaa agagttccta tttctccaca tcctctccag 156900
cacctgttgt ttcctgactt tttaatgatt gccattctaa ctggtgtgag atgatatctc 156960
atagtggttt tgatttgcat ttctctgatg gccagtgatg atgagcattt cttcatgtgt 157020
tttttggctg cataaatgtc ttcttttgag aagtgtctgt tcatgtcctt cgcccacttt 157080
ttgatggggt tgtttgtttt tttcttgtaa atttgtttga gttcattgta gattctggat 157140
attagccctt tgtcagatga gtaggttgcg aaaattttct cccatgttgt aggttgcctg 157200
ttcactctga tggtagtttc ttttgctgtg cagaagctct ttagtttaat tagatcccat 157260
ttgtcaattt tggcttttgt tgccattgct tttggtgttt tggacatgaa gtccttgccc 157320
acgcctatgt cctgaatggt aattcctagg ttttcttcta gggtttttat ggttttagtt 157380
ggtgggactg taaactagtt caaccattgt ggaagtcagt gtggcgattc ctcagggatc 157440
tagaactaga aataccattt gacccagcta tcccattact gggtatatac ccaaaggact 157500
ataaatcatg ctgctataaa gacacatgca catgtatgtt tattgcggca ctattcacaa 157560
tagcaaagac ttggaaccaa cccaaatgtc caacaatgat agactggatt aagaaaatgt 157620
ggcacatata caccatggaa tactatgcag ccataaaaaa tgatgagttc atgtcctttg 157680
tagggacatg gatgaaattg gaaaccatca ttctcagtaa actatcgcaa gaacaaaaaa 157740
ccaaacaccg catattctca ctcataggtg ggaattgaac aatgagatca cttggacaca 157800
ggaaggggaa tatcacactc tggggactgt ggtggggtcg ggggaggggg gagggatagc 157860
attgggagat atacctaatg ctagatgacg agttagtggg tgcagcgcac cagcatggca 157920
catgtataca tatgtaacta acctgcacaa tgtgcacatg tacactaaaa cttagagtat 157980
aataaaaaaa aaaaatttaa aaaaaaaaag agatcttagt tcttttgggc ttggggactc 158040
actcttgttc acttaaagtg gattggttct tacatttatt ttcatagttg tgtctggtca 158100

ccttctggct tgatgtatgc ccctatgtct ggaaaaggtt agagaatggg gtagaagtgg 158160
aggctgcccg cccatcatgt aactgtttta tcttttcaga gatacaattg ggatcctaac 158220
tctttggtct ccgtatttcc acacttgctc ttatacgatt cagtttccat gcaacagcag 158280
gagagatatt ttagatatat taatcaggtc ctatggtctt gagtttacag ctctcagtgt 158340
aaatcttact ctcaagagaa aaattttcct tctgcagctg aaaacattcg acatattctc 158400
tctcctattc cttctccccc attcactgta ctccaggcac accagctttt atttttattt 158460
attttttgct attcccccca acacgccaac atgttccttc ctcaggatct cagcacatgt 158520
ggttcccttt ttccctttgc ttgaaatacc cagatcttta cttggctggt ttcttgtcat 158580
tcagattctg cccaaagtca ctttctcaga agtagtgtta cccttccacg agtaatacta 158640
ctcttacttc tctttacagc tctcaaattc ttatgaagtt ttcctattta ttcatttgtt 158700
tgattattgt ctgtctgcat caggtaccgt gcaaacttgg atgtaaactc agtgatagct 158760
gatttctgtt cagcattgtt tgttgctata gctccagtgc ttttagtcat gttgggcacg 158820
tccctactaa atagtagaat atggcgaagg gtcagagtca tgatagatag ccggatgtgg 158880
tggctcatgc ctataatccc agcactttgg taggctgaga tgggcagatc acgaggtcag 158940
gagttcaaga ccagcctgac caacatggtg aaaccctgtc tctactaaaa atacaaaaat 159000
tagccagttg tgatggcatg tgcctgtaat cccagctact caggagactg aggcaggata 159060
attgcttgaa ctcgggaggt ggaggttgca gtgagctgag atcgcgacac tgcactccag 159120
cctgggcgac agagtgagac ttcatctcaa aaaaaaaaaa ataataatga tagataaaaa 159180
ctgctagagg ctctctgaga aggaagaaat gtcactgggt taggttggta agagaagtgt 159240
taatgcaagt ggtacatttg atataagcat gcaaataaat ggtagtttgg aggaaaattc 159300
cagaaatgtg aaacagtatt gagaaattga gtaagccagc tttgagagaa cacacggctt 159360
attgtcctct ccttctgttt tgctcctcag ctctctaaag tgggcattct ctagggtcct 159420
gtcttcaatc tcagcttctt tttcttcttc atccttctct ttccacctag tttcctaggg 159480
aattatatat atatatatat atatatatat aattttgtat atatatacaa aattatatat 159540
atatataatt ttatatatat ataattttat atatatataa aaaattatat atatataatt 159600
ttatatatat atacaaaatt atatatatat gtctggtaca ggctgaattg tgtctcccag 159660
aaagatatgt tgaagtccta atgctcagtg cctcagaatg tgaccttatt tggaaataat 159720
gtcattccag gtgtaattag ttaaggtgaa gttatactgt ggtgtaaccc acagtatact 159780
tccagtatat acttcagtat atgggccctt aatctaatgt gactgatatc tttagaataa 159840
gaagaaaatt tggacacaaa cacagagaag agaatgtcat atgataacag agagagagag 159900
agagaagaga gagagggagg agagagaggg aggagagaga gggaggagag agagagagag 159960
aaggagaaag aaagagagga gacagaagag aaacatctac aaagcaagga acaccaaaga 160020

ttgctggtgc ctaggaaact aatataataa cataatattt ggggcctaaa gtgttacagc 160080
tgcaccaata tctcccaaat cttgctccta gctcctgccc cagcctgatt ttctcagtga 160140
ctcccctgtg tttccctctg ccaccagccc ctccttctgc ctgctgtgtg tgacagttcc 160200
cttgccactc cctccggcat ccgtacttca aactggggag ttagttattt ccattgtttc 160260
ttattgcagc ctctggctct gacgtttcca gagctgttgc atagctctat tgagtctatc 160320
tccttaaatg cattttcatc cacttaccat tgctgagtta gctgcattac ctttcaccag 160380
gatgcttgca ataatttatt gtttccattt gccttctcct tcatctaagc cgtttgctta 160440
tgtctttttt cttgttgcga gacattctgc acattgccac tctattagtg gtcataaagc 160500
aaatcactga tcatgtcaat acgcagtaca agatccttta atgacctcca tagcccatgg 160560
aatggtcctt aaacaagagt tcaaggactg ccacaatctt gttctagcct atctttctag 160620
tcattttaaa gtcaccattt ttacttgtga aataggcact ccagacacag tgaattcctt 160680
gttcttctac aaatatgatg attccatttc tttgctggga atttctttcc agatttacct 160740
gtaaaaattc ttcgaaccct aatcaaaagt gactgttgtt aagcccctga agataatgca 160800
gaaattctct ctctcctggg acctcgatat tagtttattc cattgtattg catatatttg 160860
attgcccatg ttctgtccca tactgactgt aaagtcctta aaggtgaggg cccaatattc 160920
tcagagtcac tcaataaata aataaaagaa taaatggaaa ttaggatcag tttgtgggct 160980
ttagcaacac aaaaacatta tactttttca acatgggaga ggtatgatga aggagttttt 161040
tttttttttt gagacagagt ctcactctgt cactgaggct gcagtgcagt ggcattgtgt 161100
cagctcacaa caacctccgt ctcctgggtt caagcaattc tcctgcctca gcctcccgag 161160
tagctaggat tacaggcgtc caccaccatg cctggctagt ttttatattt ttagtagaga 161220
cggggtttca ccatgttggc caggctggtc ttgaactcct gacctcaggt gatctacccg 161280
ccttggcctc ccaaagtgct gggattacag gcgtgagccg ccgcacccgg ctgatgaaag 161340
agtctttaat gcagattaat ctggcagagg tatataggag ggaccagaga agggaaagaa 161400
tcaaagcgtg aagaccaatt tggctgatat tcaactagct tagatgtact aaaaatctgt 161460
actttttggt atttgtgaaa tggaaagaag gggaatagaa taaaggatat tataatgaaa 161520
ggatatacat tgcttgaagg taattaaata tgggttatcc aggagataaa agagttaaag 161580
aggttcagac atagactgaa tgaactgaga aatgaatgac ttggtcacca agaggaaggc 161640
cagtcatcag gggtaggat aagttcaatt ctagacatgc tgcatttgag atgatagctg 161700
gatgtccaga tggaattatc cagcagccac agaaacagaa tcagctctct gcggatattc 161760
caggggtggg gatttgaatt aatttcctca attaatttta aagaaacttg atgaaaagaa 161820
tggtctgaat acttcttgaa ggttgcacat tattaataat ggagaaataa ctctaaaacc 161880

ttcctcttga ttttcataat aatataagca ttccctgaat cttaccaaac cttgtaagaa 161940

acactcttat tataaaaagt gtatgtgcaa agcccttcta aacaggaaaa tgataaatta 162000

gtcctacagg gccaaatgca gctctctggg agcttacaat tcagaaagaa catcctgcta 162060

ccagcacatt aagctgtaca aatagtaaac tgcagaaaca aatataagca tttttatgat 162120

gtccaaacaa gaaccaagca ggtgtttttt tttttttttt tgcagattat ttatactgtg 162180

gcagttcata gcctcctttt cggacccaga gcttgcataa tccttccctt atttctactt 162240

acgtgtttta ctctccatca tgtgttaaca tacatactgt gcaacagaaa tgactatgga 162300

ggctgagggc agcagagttt tagtgtgtac acatatgagc tgtcatgtaa ttttcaagtg 162360

aaagcctttg caatgaaact ttttaaaaga aagtcatggc cgggtgcggt ggctcatgcc 162420

tataatccag cactttggga ggctgaggca ggcagatcat gaggtcaaga gattgagacc 162480

atcctggcca acatgatgaa accccgtctc tactaaaaat acaaaaatta gctaggcatg 162540

gtggtgtgca cctgtagtcc cagctactca ggaggtggag gcaggagaat ggcttgaact 162600

cgagaggtgg aggttgtagt gagccgagat tgcaccactg cactccagcc tggcgacaga 162660

gtgagactcg tctcaaaaaa aaaaaaaaaa aaaaaaaaaa aaagagaaag aaagaaagaa 162720

ggaaaaagaa agaaagagaa agaaaggcgg gcattaactt caggtattgg taaatttgct 162780

aggtgtttgg ctactgtttc tcatcagaga aatagaaaga cacaccatga aagtcaaggc 162840

ctgaaaacct cattccatgt aagaatgaca tccccagtgt taagtgcttg ttagtggtta 162900

atgcgatcct gtgaaactta gatgtgtttg tgcacacatg cacgcatata tttgaagaac 162960

tcagaagagt taaatcacag cctttcaacc tgtgaaatga cagtagttct tctttttttcc 163020

tctccctttg gctaagtcat ctttatcttg gagataatta agacaaaaat gcctctgaca 163080

aataaaatca gtatagaacc cctatttctt ggcagctttt gtggacacag ctgaagcttt 163140

cagaggtctt gaaaaaccat ggcaacaaat gcctttgaag ggtaagcaaa ggttccaaat 163200

gtttttaatc gctggtgttt tttctgctac cacttcaagc atttttcttc atttttttgtt 163260

catctgatca aaattaaatt tccaatttcc ctactaagtg gttctgtcct ggtcatgcca 163320

ttgactattt ccattaaagt agtagagttt gtgcccacat atggttgtta agctcatcaa 163380

caattccatt agaaagcttt gtttatcagt ggcaataatt tcccataaaa attatagata 163440

ggttttaatg ggcacatttt caaaaggcat caactcgtcc tcaaaattat gtgctgacac 163500

tgttcttaca accatggttc gtggcctaat tccaccaaat ttctctcttt ttcatagaga 163560

actgttgtca gtagctttat atccttttaa agagaatggt ctgtatctct gatactcatt 163620

cagagaaatg agtattttag acgtaggttg ctaattttaa gctatatact acactatgtc 163680

agcactatat aggttatctt gtaacctgct tgcctggcta tttggctgaa aaataacaac 163740

atgtaaggaa aatctatttc ataagtctaa catttacttt gtaaaacttg ttctggctac 163800

tctgttaatt ttccacttac gtgtgggtta gagagcagat ttgatttttt tttaagcgaa 163860
agatatggct tacctgagaa aagaacatag tggggaaagc actcctatta ttttcctcat 163920
atttccattt tcctttagcg gaaataaaaa gacatttcag tttttcagtt gctaagaaat 163980
gaaggaacca aagacaaaac aacttaatta ttaaattaca atttatttct gtaataagca 164040
ctcgttctct ctgttttcct ggggaaagag tatgtggact ttcaatttta tccaaataag 164100
catcatcttt ctctgattag tgtggcagtt tcaaaatcat gtattaggaa gtacagagtg 164160
aatgagtaga gaatttctaa attagcaccc aaggttgggt ggctagatta tgtttataaa 164220
tatgaacttt tgtattaagt gcaatgattt aaaagaatgc ctgcatcact ttagggcatt 164280
tcattaagtg ctgtgcacaa tatttttcct tatacatcat aaaagataaa ttatagttca 164340
taaaatagta taaattccta attattttgt gcttttgaca cctcagagtt actaataagg 164400
gatttcgttt taaaatgata tttatttatt tatttagaga cgcagtcttt ctctgttgcc 164460
caggttggag tgcagtggtg cgatcttggc tcattgcaac ctctgcctcc caggttcaag 164520
cgattctcct gcctcagcct ccagagtagc tgggaccaca ggcatgggcc accacaccca 164580
actaattttt gtatttttgg tggagacggg gtttcactgt gttgggcaca ctagtctcta 164640
actcctgacc tcaagtggtc ctcctgcctt ggcctcccaa agtgctggga ttacaggcgt 164700
gagccactgt gcctggctcg ttttaaaata atttaaaagt atattttgcc accactatta 164760
accagttaag ccatgatggt atattataat caccatggag atggtttttc tctttatttt 164820
attttgtttg ttttctgatt gctagcatgc tgattactct tcctattcta caagtgcctg 164880
caggccagcc ccatttttgc tctcttcact tcatttttca tttctccctg tttcactctt 164940
catagcacat ttgtactctg tccacaccta gagacctccc tgttgaagtt cttcacattc 165000
tcttccctag agagggttaa cttgttgagc tcagagactt aaacttaaaa taaaatgtaa 165060
cagatatgta ttgaatgact attttatttt agctctagga gaaatgcaaa gatatatcat 165120
ccatagaccc tgaaatccag ctaagggttc tgcttataca caaggtgaaa ggttgtgata 165180
atgcacatta aacaatagaa gagttaaatt cagcactata agtgatgcct caaagcagca 165240
taatgggaaa aaggggtatt tcagaaaaaa atggtatgag ttcagaggca agagaaaaca 165300
gaatgctcta gattaaactc aaagacttta tggaggaggt ggtgtttcaa tagatacctt 165360
tccacagaac catgaagaga gttctactta attgtaagtg gcctgtgatt tgtcgttagt 165420
cattgcatgc tcaattctgc atgtgtgata actgtgtatt tttatggcaa aatcgcaatt 165480
acttttgcac caacctaata gaatagtgtg ggtttaaaaa aactatttgt aatatattcg 165540
aacatatttc ttatatatat gtttcaccat cattagcagt aaatctattt tgcatccaga 165600
gattgaataa tattttgttt ctaggatgtg agtgatgtac tattttaact tacttttgaa 165660

gtttgacatg ggagtgatat taataaaatc taaagatgct cctaaagagg agcaatagat 165720
aatgtaggta tagataatgt agctattatg gaggtaagtg tatttgatac tttttattat 165780
gatatgaagg gaaacaacta gtcccaagct acaatttatt aaggtaggct tagcttatac 165840
gccataattt ttatgtttgc aactttcttg attttcttcc tctggcttta cttctatggg 165900
atatttatga aaggatgttt ttaaaaatgg ctagagactt ttgcttctaa gagttgacat 165960
aaacttttcc acaacatgaa caattagtag atgatacact tagatacact cttgaatttt 166020
taaaattgtg gcagtaaaac tgatctcaga gaaggaagaa aatgctttgg ccagctttta 166080
tcaattatat acaacgtgtt tggaagattc agttcctctc gccttctttc tccctatata 166140
atactttttc ttaaggttgg tactattgat gcacttggtg taaacacatg agataatagc 166200
aaaatatgaa tctaggtccc agaaatgggc ttctttcact ctgccacctg ttctgactgc 166260
atttctcctg actttctgta aatcccgggg aggagctgga agagcaaggt gtgtgctagc 166320
ataactttgg ggatgaagta cccttctttt tttctgattt tgttcccaca tctccttact 166380
ggaccaagta agggcatcag tgtcaaactt cctgattgaa gatacatttt tgccctacca 166440
tgaagggctc tgggtaacat ttattgctta taaaatgctt ttttggtggc tactttgttt 166500
ctgaatataa ggcaagataa aaagtttgat tcaagagttt cttgtaaaca ctttgtgtgt 166560
gcgtgtgtat tcatatatgg ctgcacacgt acataagtcc atacataagt acagtccacc 166620
ctccatgttt gtgggttcaa catccatgga ttcaaccgac tgcaaatcaa aactatttgg 166680
aaaaaaatgg atggtagtgt ctgtgctgaa cacatacaga gattttcctt gtcattattc 166740
cctaagcaat ataacaactg tttacagaga atgtacattg tattaggtat tataaataat 166800
ctagagatgt tttaagctat atgggatgat gtgcgtaggt tatatgcaaa tattatgtca 166860
ttttatataa gggacttaaa catctataga ttttggtgtc tgaggagtct tcgaacaaat 166920
cccccacaga tcctgagggg ccactgtata tatatatctt cataaacaca cccacacaca 166980
caaacacacc cacccaccca cacacacaca cacaccaatg tgtatatact ttttttttctt 167040
ttttttaatt cgagaccaag tctcgctctg tcgcccaagc tggagtgcag tggcgcaaac 167100
ttgcctcatt gcaacctctg cctcccgggt tcaagtgatt ctcctgcttc agcctcccta 167160
gtagctggga ttacaggcgc ctgccaccat gcctggctaa tttttgtagt tttagtagag 167220
gcagggtttc accatgttgg ccatgctagt ctcgaactcc tgacctcaag tgatttgcct 167280
gcctcggtct cccaaagtgc tgggattaca gatgtgagct accacaccca gttgcaatgg 167340
gtatagactt ttgaaatgtg tactacaaaa tattaacagt gactatctct gaatgatagg 167400
attatggata aattttgttc tttttgcata tctgtatttt tatttttcca cagtaaacat 167460
cttttacttc tgtaataaaa acttcattaa aaaatccatg ctgcaattga ttaattatat 167520
actaactttt ttttactctt ccctcagtac agaaagatta ttctgtattt accaaatctt 167580

tacactttat ttgcaaatca ttttgactgt ctgtttgcaa gaggtcaaat ttgcactcaa 167640
gttcagcttt cgttcattat ccatgagccc aagagaaatt gtgtcaggtg gacatcaggt 167700
agctgaggcc aatacaatat tattcctttg ggttgttgag aaatcatagc agaggaaagc 167760
ctgtgttgat aatagagtac caccaggaat ctaggtcttt acagctgctg gcctgaccag 167820
caagggtaaa atatttcagt cacgcagcca gtgattatta accctagctc tatattccag 167880
agaggaagag ctagcctttt tgggttgtga tctgtcactg attgcagttg cacatatact 167940
agccatgagg tctacacaca tgtgaagagc atgctacaca catagactag atacactgac 168000
tcatcagcct ggaaacagtg cctgaccctc actttgattg tttgaatgtg tcctgaccca 168060
ttaccctgcc tttggatccg ttcctgtttt tttttttttt tttaaagcat tttatctgtg 168120
aattaaaata gtactttcca ttgcttcatt tttttttccc tgaggagttt agctttgttt 168180
aattagaaac aagacaacaa tcattactgg tctggtgcaa tcttgtttag gactcctctt 168240
ttgcttagaa ctatacctgg attagggatc actgtgattt agagtgtgca gtatgaggtt 168300
atttaaatac ttaatagtat atttgaaatg aaaagagtag aaattaaaga ctgaatcatt 168360
taccaagaaa taacagagga gaccaattta cttctgtaag atttagtaga ggctgttccc 168420
ttctttataa ttggctttgt ttcagttacc agttaagtgg ggtgaactgt tattaaaagg 168480
ctatgggtaa ggttacagta gcattcttct aagaaaaact acaggagtag tcactgtgca 168540
gctatctgct ttgtacctgc caatatggca gtagatatgg aagggtcaga aaaacaggac 168600
ttcatgttca ataacttttc ctttttcctt tctttccctt attttttca tgtcttgtat 168660
gcaagatagc atgcctattc tttaccttca atgtattttt gaatttttaa tccagatcat 168720
attttatcac aatttgtaac atgtagctaa taaaaatggt tattacatga gaacttgctt 168780
tacaaattat agatcaaatt tttatacaaa ttattatgtc accatagaaa aatagttacg 168840
atttatctgt ttagtcttag aaaaattatt tcacatatta gttaaggctt ttagttttga 168900
gtgagcaaaa tccaattcaa actagctttg ggtagaggtg ggggtaaaag agaggaatgg 168960
atttcaatgg aaggtttact tgcgtgtctt ggctaactgc aaacatggca gggtgcaacc 169020
agaccttagt gatcacagac ccagggactc agaccctgtg aggaccttag ccttaccgcc 169080
actcttctcc atgtgtcagc ttcgcgttct ctgtctgcaa gtatgcattt tccctaaggt 169140
gggaagcaga gttggcaaca gctgccaggg ttgacttcct gtggcattct tgcagttagc 169200
aaatattaga gaaggactct gcttggccca gctttgggac acacctctgg accaactagc 169260
tgttctcagg aggcagtaac atgtacagag gtgatccctg ggctcgcccc tgtgaaatca 169320
ttgtgagcca atcactacct ccctccctga tgctgatgtc tactatgttt gatgtctctg 169380
aacttccatt tcctcaattg tgaatggcac cctggttcct accttgctgg atagatgtag 169440

tgattagagt tcatgcttaa cctacaggtg ctctgtaaag aatcattggc atcattacct 169500
cttgttggct ctatcccatt ccctatagat atctttctgc tctgagacta agcagagaaa 169560
ataatttaac atttaattat tgtgcttcta aatccacttt tacctcttct ttctttactt 169620
ttctctttat tatttttaat tatttcaaaa ataaaattgt atatatttaa ggtgtacaag 169680
atgttttgat atacatatac ataatgaaat gattactgca gtcaagctaa ttaacatcaa 169740
tctcctcacc tagttatcat ttttgggggg catggagtga gagcacctga aatctctctt 169800
agcaaatttc cagtgtataa tacagtattg ttaaacatag tcatcatgtt gtacattaga 169860
tttctagact tgttcatgct acataactgc aactttgtac cctttgacca atacctctct 169920
atttctccca ctattccatg cctgaaattg tgggatatag gtatatagtg tgtgtgatat 169980
atatagaggg agtgatatat gtatatataa ttgtgtgata tatatgtgtg tatatacaaa 170040
cacacagaca tacacacatg tacatatgta tatacatgca cacatacaca atgggatatt 170100
attcagcttt caaaaaggac atcctgccat ttgcaacaac atggatgaac ctggatgaca 170160
ttatgttaag tgaaacaagc tggacacaaa aagaaacatg caccttactt cttagacaca 170220
aatttctttc tcatagccag cacctgaaaa tagggccaag tattttgcat gatttacaat 170280
tgactgtata aatgtagcat agccagttat atgatgtgtt accatttctg ctataaaatc 170340
aaaagaagag atcagaacat ttaaaaactt ccctgtggat ttctacctct tacctaagct 170400
ttgttcttgt ttttttaaac ctcatgaaca cgaagcttta gatagcggag gtcatgtatc 170460
cttgggctca aatatacagt ttcttagcaa tctttgattg ctacattgcc ctttttttgtg 170520
tttttaactg gccttccctg acttaaaata tactttaaaa aataacaaag gtggtattta 170580
gtttgcacag tatggaatta aaggtaaaca actttaaatt aaatttggtt ggatgtataa 170640
attattaaaa tatcctttct ctcatggctg ggtaagtctg cattttccat tcagcctgct 170700
atgtctcttt ccatgagaac actcccgaag aatggggccc tgcttccaaa caagccaagc 170760
attttgtagt aaattttaaa aagctaaatt tcataataac ctttataatt gctacctgaa 170820
aatggagttt ggaatggcac caaattatta atcagtagaa ggagaataga ttgcaaaaag 170880
gtaattatat caaattgaat gcttcttgaa atatattctg ttaaataaaa tacagttcct 170940
cttccctgtt ctcatttgta agcaatgagg acaattcagg tagcatgaat caggaatcag 171000
agtacttttc tgttccgcag attggcttaa tcattttggg ctcacttcaa actgtagatg 171060
agcattgtcc tttaacctgc cgggcagcag aggtgaccac gttcactgca aagcccagaa 171120
gtgagttgtt catctgggtt agggtctcag gaagaggcat gttatgactt ccccacattc 171180
ctggacagtt tagactgtaa ttcttggttg tttaaaaagg tgtttgaatg tattttgttc 171240
agaggcttga gtaaatggct cttattctta cactttttt tttgcctgct ttttccagca 171300
ttagaatagt ttcagccctg acaattagcc ttatgctgac tggagtggat tttttgcgta 171360

ggaccaagtg ttttgcatga tttacaatgg actgcataaa tgtagcctaa caagttatat 171420

gatgtgaaga aaattactta agggtaaaaa tggattgttt actgagaaaa tgatgttatt 171480

ataaactggt ttgagggtat tgtggtttag atgctgtcct tacaaatttg agctctgttg 171540

gtttggagat ttaatatcta ataataatag ttaatgtgta atgaatactt gccctgctcc 171600

aggctctatg ttacgtgtcc tgaatgcatc atctcattta atcctccata aaatttgaag 171660

cagtcagcat tgtaattaat ctcacttttc agatgaggaa accaaggtat gtaagaaaca 171720

aaaccagtgt tgaatctgac tagtacgact gcaaagcctg actcttaacc attgtgttct 171780

gttgccttct ccaagggaaa ggagtgcttc cagaaaggcc attggaagtg aggagtgtca 171840

cctgataata gctggctcct catcccacta ggttaacagg caacagatgg gggccacaga 171900

gttggctgga gttagtcctg gttgccaagg gtaaatgggg ctgttaccat gcaatggata 171960

gaaactagga tgctcctgtg tgtctcctta tatcctacca ggtgtagcat gaaggttaag 172020

gacaattccg tcctcttatt tggagaggag accaaggccc tgtaggacta aaggtttggg 172080

tgagctgaaa gccaagggca attggcccag cttggggagg agagttacaa atacatttgt 172140

gactatcaga ccagaaacag actgcggtgt tcacctgcgg tttgggtacc tgctgcttgt 172200

tctttgctgg ggtatctgaa gactgaaaac acagctcaca cttagccttt ttcctattct 172260

gtgtgaaaga actgtagtag ttttctcttg cttaacaagg ttctagtggt gaagtttatt 172320

gcttagtctg tgagttgtag ggaaataacc acaggatggg atggtagcag aacgagaaaa 172380

gaaaagaaga ttgtagatgc caggctggtg tcggcttgaa atttttaaag tatacagttc 172440

ttctttatgg attattaaaa aaaaacagca aatgaataca caagattaaa tgttaagcag 172500

tacaacattg tacatgatga aaggttaaag tctttctctg gtctcattca aaaatctaca 172560

gtctcttgta gtatagtttg aagtcaggta gcgtggtgcc tccagctttg ttcttttggc 172620

ttaggattga cttggcaatg cgggctcttt tttggttcca tatgaacttt aaaattctgt 172680

gatgaaagtc attggtagct tgatggggat ggcattgaat ctataaatta ccttgggcag 172740

tatggccatt ttcacgatat tgattcttcc tatccatgag cgtggaatgt tcttccattt 172800

gtctgtgtcc tcttttattt cattgagcag tggttttgtg gttctccttg aagaggtcct 172860

tcacatccct tgtaaggtgg attcctaggt attttattct cattgaagca attgtgaatg 172920

ggacttcact catgatttgg ctctctgttt gtctgttatt ggtgtataga atgcttatga 172980

tttttgcaca ttgattttgt atcctgagac tttgctgaag ttgcttatca gcttaaggag 173040

attttgggct gagacgatgg agttttctag atatacaatc atgtcatctg caaacaggga 173100

caatttgact tcctcttttg ctaattgaat actctttatt tctttctcct gcctaattgc 173160

cctgtccaga acttccaaca ctatgttgaa taggatggta ctggtaccaa aacagagata 173220

tagaccaatg gaacagaaca gagccctcag aaataatacc acacatctac aaccatctga 173280

tctttgacaa acgtgacaaa aacaagaaat ggggaaagga ttccctattt aataaatagt 173340

gctgggaaaa ctggctagcc atatgtagaa agctgaaact ggatcacttc cttacacctt 173400

atacaaaaat taattcaaga tggattaaag acttaaatgt tagaactaaa accataaaaa 173460

ccctagaaga aaacctaggc aataccattc aggacatagg catgggcaag gacttcatgt 173520

ctaaaacacc aaaagcaatg gcaacaaaag ccaaaattga caaatgggat ctaattaaac 173580

taaagagctt ctgttctttg ctggggtatc tgaagactga aaacacagca aaagaaacta 173640

ccatcagact gaacaggcaa cctacagaat gggagaaaat ttttgcaatc tactcatctg 173700

acaaagggct aatatccaga atctacaaag aactcaaaca aatttacaag aaaaaaggaa 173760

ccccatcaac aagtgggtga aggatatgaa cagacacttc tcaaaagaag acatttatgc 173820

agccaacaga cacatgaaaa aatgctcatc atcattggcc atcagagaaa tgcaaatcaa 173880

aaccacaatg agataccatc tcacaccagt tagaatggtg atcattagaa agtcaggaaa 173940

cgacaggtgc tggagaggat gtggagaaat aggaacactt ttacactgtt ggtgggactg 174000

taaactggtt caaccattgt ggaagacagt gtggcgattc ctcagggatc tacaactaga 174060

aataccattt gacccagcca tcccattact gggtatatac ccaaaggatt ataaatcatg 174120

ctgctataaa gacacatgca cacgtatgtt tattgcggca ctattcacaa tagcaaagac 174180

ttggaaccaa cctaaatatc caacaacaat aggctagatt aagaaaatgt ggcacatata 174240

caccatggaa tactatgcag ccataaaaaa ggatgagttc atatactttg tagggacatg 174300

gatgaagctg gaaaccatca ttctcagcaa actattgcaa ggacaaaaaa ccaaacactg 174360

catgttctca ctcataggtg ggaattgaac aataagaaca cttggacaca gggtggggaa 174420

cattacacac tggggcctgt tgtggggtgg ggggaggggg gagggatagc attaggagat 174480

ataactaatg taaatgatga gttaatgggt gcagcacacc aacatggcac atgtatacat 174540

atgtaacaaa cctgcacatt gtgcacatgt accctagaac ttaaagtata ataaaaaata 174600

tttaaaaaat ctacagtccc tttctcaata ggtaaacact attaacaatt tcttctgaaa 174660

attacatatg tacacacaca cacaccccac acatagatat tttgttaaca tacattacct 174720

tatgctctac acattattct gtgcctagct tttctcatcg aataatgtgt attggagatc 174780

tttccatata agtacatatt ggtctcactc attcattttt aatggctgta tattagtcca 174840

tagtatggag taataatacc ttttattggc acaaagtttt gcagttgaca aagggtttgc 174900

atatattgtt tcattgggtt gtatagcagt catcaacgga catatcaaga atcccagaag 174960

ggtaacctgg gcttcaccca ggatcgcatg gagcttggga ctcaaacagc taacattccc 175020

taaacaggcc catattcctg caatttagtg cagctgctac aacatttagg taaactcttt 175080

gaaagcagta aaaagatcac caatctggga catcaagaag ccagacgaca aggagacata 175140

gacctcaggt gagtgagaaa agagaataat tgaagtttag aactgaatgg gcactaacga 175200
acaacagata catctctttc ctttcaccga tgaggacctg ggtgtggcct gtggatgtta 175260
agtagcatgt ttaggccaca cagctggatt gttttctttc ctgaggctag ctctggagag 175320
gggaggaaac ggagatgcta gatttttccg ggctctcttt tctcatcagc tagaatggga 175380
tgtggatgga atgcagcttg agaagccaaa caccttgag aaatgagaac tctgctttct 175440
gatgtggggc gcatcctatc tgagataact ctcctgccag cgcagtatga ccattgcgac 175500
ttgagctgtg tgggtgccca ggtggtgtag accttaaaac cactcctgct tttgtttact 175560
gaatgtctaa tattcacaca gttctttgtc tgagtgcagg gccaaagcag caaataacga 175620
agaagctgtg cccaaccata agaactatga aatgctccag atcatctacg ctagtttgag 175680
ttaaatttac catgcaaata aatatagaac atttcttctt atgcatagat tgtatttgtt 175740
ctattaaaca accatcctgt aaaggtttta ttcattcatt tatttttatt tgttaggaga 175800
aattgttatt taagctaaga aagtagacta gtagttattt cttttttttt gcttattata 175860
ctttacgttt ggggatacat gtgcaggtta gttacgtagg tatacacgtg ccatggtggt 175920
ttgctgcacc catcaacctg tcatctaaat taggtatttc ttctaatgct atcccttccc 175980
tagcccccca ccccctgaca ggccccagtg tgtgatgttc ccctccctgt gtccatgtat 176040
tctcattgtt cagctcccac ttatgagcga gaacatgcag tgtttggttt tctgttcctg 176100
tgttagtttg ctgagaatga tggtttccag cttcatccat gtccctataa aggacatgat 176160
tgcatccttt tttatggctg catagtattc catggtgtat atgtgccaca ttttctttat 176220
ccagtctatc attgatgggc atttgggttg gtttcaagtc tttgctattg tgaatagtgc 176280
tgcagtaaac atacgtgtgc gtgtgttttt agaatagaat gatttataat cctttgggta 176340
tatacccagt aatgggatgg ctgggtcaaa tactagaagg ctacagtaac caaaacagca 176400
tggtactggt accaaaacag atatctagac cactggaaca gatcagaggc ctcagaaata 176460
atgctacaca tgtacaaccc tctgatcttt gataaacctg acaaaacaag caatggggaa 176520
aggattccct atttaataaa tggtgttggg aaaactgact agccatatgc agaaaactga 176580
aactggaccc cttccttaca ccttataaga aaattaactc aagatggatt aaagacttaa 176640
atgtaagacc taaaaccata aaaaccctag aagaaaacct aggcaataca attcaggaca 176700
taggcatggg caaagagttc atgactaaaa taccaaaagc aatggcaaca aaagctataa 176760
ttgacaaatg ggatctaatt aaactaaaga acaactgcac agcaaaagaa actatcatca 176820
gagtgaacag gcaacctaca gaatgggaga aaatttttgc aatctatcca tctaacaaag 176880
ggctaatatc cagaatgtag aaggaacttc aacaaattta cacacacaca cacacacaca 176940
cacacacaaa caaccccatc aaaaagtggg tgaaggatat gaacagacag ttctcaaaag 177000

aagacattta cactgccaac aacataaata tgctgccaac aagcatatga aaaaaagctc 177060
atcatcactg gtcaagagaa atgcaaatca aaaccacaat gacatactat ctcacaccag 177120
ttagaatggc aatcattaag aagtcaggaa acaacagatg ctagagagga tgtggggaaa 177180
taggaacgtt tttacactgt tggtgggagt gtaaattagt tcaaccattg tggaagacag 177240
tgtggcgatt cctcaaggac ctacaattag tagttatttc taggtctgac gggctgtctt 177300
ttagtttgtt ttcttattct gaggtggact taaaccaatt ttaaaaacag ggatgactga 177360
attcgtgtgt taatcttgta ctacaggaac cctttgacat tgattcagaa gcacccaagt 177420
gtttgttcat taatcctttt ttatttttgc atattattta tttttcaact tttattttaa 177480
aaatcagggg tacatatgca ggtttgttac aaaggtatat tgagtgatgc tgaggtctgg 177540
catatggatg aatctgtcac tcaggtaatg agcatagtac ccaatgatag ttttttgtac 177600
ccaatgatag ttttttgttt tctttgtttt tttttgtttg tttgtttttt gagatggagt 177660
ctcgctctgt cacccaggct agagtgcaat ggcttgatct cggctcaatg caacctccgc 177720
ctcccagatt ccagcaattc tcctgcctca gcctcccaag tagctgggac tacaggcgta 177780
caccaccatg cccggctaat ttttgtattt ttagtagaga tggggtttta ccatattggc 177840
caggctggtt ttgaactcct gacctcaggt gatctgccca cctcggcctc ccaaagtggt 177900
gggattacag gcgtcagcca ctgtgcctgg ccccaataga tagtttttca gtccttgccc 177960
ctgttcctcc ttcccacttc tagttatcac cagtgtctat tgtttctgtc tttatgttca 178020
tgtgtaccaa atatttagct cccacttata agtgagaaca catggtattt ggttttctgt 178080
ttctatgtta gtttgcttag atagacctcc agctgcatcc acgttgctgc aaatgacacg 178140
atttcattct tttttatggc tgtgtagtat tccgtggtgt agatatacca tatattcttt 178200
atctaggata actgatgggc aattgggttg attccatgtc tttgctattt tgagtagtgt 178260
tctgatgacc atatgggtgc atgtgtcttt tcaatagaac aatttatttt cattactctt 178320
atcaggatac ctgggaggaa cttctcctac gaagttaatt tggggggact cctgaagatg 178380
agtgaaaccc cttattaagc acttagaggg tcgaaagtgt acaagggaac gttcaggtgt 178440
gacagcttga gagacatgca tattccaccc ccacaccaga gcttagtgaa ccgtcttttc 178500
tattttctcc caaagcacca aaatggccca gaaattggaa gatggaaaat ggacttattt 178560
atgagtctgg gaaggcaggg cagtaagcac agtcctgttc agggttctga gttttaccct 178620
cttgctctga ttgcgagatc attttcctct tccttgcttc ttcagcttag gacaagaaca 178680
gtttggaaat tgtttctact actttggaca ccatagttta gatttcaaat gagtacaagt 178740
gggaggaaag cttggataat tctctggaaa taatcgaaca gagtgaagga gaggggtcta 178800
caggtgagct gaatgctgca tggcattgaa gtaatcacac cagtcgtcac aattctctcc 178860
tctttgatgg ttatctgttg tcccagaagg aacaatttca gaaagtcctt ttctggactg 178920

tagaatagca cttgcttatt tgatgagccc tgagaagcat tactgaaagc ggttcattgt 178980

ccctgaggta ttacaatgag atggtggtca ctgatttcat tatgttttcc tttattgcag 179040

ctgttggttt gatcctttgc caggtgctta aaacaattgt ggttttgcag atggtaagtt 179100

agaggttgga caaaaaaagg gatcatgtca ctgccctggc caaaatttca acagactggg 179160

gtctagtgag ggcaaataga tagaggcttt cctcttcact ttgtgttatt tagaaaaaga 179220

aactttccag gacaaatttc tttcctagaa ttcctttttt aaaaattttt tttctttgaa 179280

aatttactta gatgcaaata atatattttt cttcctttta aataataaaa gtaagatgtc 179340

tcttggaggt ggtggttgtc actgacaaga ttaactagaa ctgactagct gtaaaaatat 179400

aatttgggat gcattattaa ggcatgccat ttttatttgc atgccattgt gtacagatgt 179460

ggttgtgaaa tagttcaaat catggcacat tgaatgtcct cactggattt ttaggaatgt 179520

gttcactgag acagccaaat cctatttcat tttctttggc tcattgcatt ggctgtaaat 179580

tggagatatt cactttaata tgtgagtcaa aatttatttc caaacataat actgcagttg 179640

ttctgtcaca gaatataaat ttcttattta tttccttaat acgttgcttt ctactttttc 179700

ttttttctt tattttattc tggagtatgt ggaaaggttt tccagaaaga tttgcatatg 179760

ccataatcta ctgatgaata cttttttgg gttactcttt catattttgg gagatataac 179820

tatggaagtg ttaggaatca tgggttctgg aaatagtttt attactgctt ctgaaatgcc 179880

ctcccaatga taccatatag taattccatc agggaataat atttttatta tagtttaaaa 179940

tataacttaa tatttaggtg ctcttgttca gtcatcgtca agttcttttt atttcaccaa 180000

ccctaccatg gcactcctga aagacttgtg aatgcgacag acctggattt aatcatggct 180060

ttgccatctg ctagccaaga gaacttgaac aagtgagtca acttcttgga gtctcatttt 180120

ctgcttctgt aacatgggaa ctagggtaat ctaactcatt gcttgtgatg attagatgag 180180

gcaaaatgct gagttcacct agcccagcac ctggtccatg ggaagcatgt gggttctgct 180240

gctacccagt ccttggccca gtgcatggtg cacagaaggg aatctgaaca ggccaacttt 180300

attcctattc ttgacccacc ccatgtagat gcttcctaca tcttcagctt cttcttcttc 180360

ttcttttttt ttttaaggca gggtctcact ctgtccccca ggctgaagtg cagtggcaca 180420

accacagctc agggcaacct cgacctccta ggatcaagtg atcctcccac ctcagcctcc 180480

tgagtaactg ggatgacagg accacactac cacacttggc taatttaaaa acttttgtag 180540

agctggggtc ttgctatgtt gcccaggctg gtctcaaact cctggattca agtgatgccc 180600

tcacctcagc ctcccaaagt gcctggaaaa caggcctcca cacccagcct tcaacttcat 180660

tttaaaaaat tgtggtaaac tatacaattc atccatgaaa gcagaaacca cttgtacccc 180720

aaaagctatt gaaatttaaa aatatatata ttaaaaataa aaataaaatt gtgataagat 180780

atacataata tgaaatttac tactttaatc atttttaagt gtacggttca gtggcattga 180840
gtacattcac atttttgtgc aaccggaact tttcatcctc ccaaagtgaa gctctgtact 180900
tattttttat tttattttat tttttgagat ggagtttcac tctagtcgcc caggctggag 180960
tgcagtggtg caatctcggc tcactgcaac ctctgcttcc tgggttcaag agattctcct 181020
gcctcagcct cccaagtagc tgggattaca ggtgcccacc accacaccca gctaagtttt 181080
tgtattttta gtagagacgg ggttttgcta tgttgggcag gctggtctct aactcctgat 181140
ctcaggtgat ctgcccgcct cagcctccca aaatgctggg attacaggca tgagccactg 181200
tgcccggcca gctctgtgct cattaaacaa tgactccaag ttccccttcc ccacatctcc 181260
tgctgacctc tcttctactt tctgtctctg tgagtttaac tattctaggt acgtcatgta 181320
agtgcatcta tgtgatattt gtccttttgt gtctggctta tttcacttag cataatgtct 181380
tcatgattca ttcatgttgt agcatgtgtc agagtttcct tcctttttaa ggctgaataa 181440
tactccactg tatggataga ccacacttta tttatccatt tgtctgttga tggacatttg 181500
gatgatttcc atcttgtggg tatactgagt aattttgcta tgaacatggg tataaaaata 181560
tctatttgag tttctgcttt caattatttt gggtctacac ctcaaagcgg aattgttgga 181620
agtggaattg ctttccactt gttggaagtg gaacatggtc tcccacttca ttttgacaca 181680
acttccaagc ttcagaactg tatttacaac agcgtgctgg gaggtcgctt tgagtttatg 181740
acggaaatct catatcaaca ggttttaaat tgttccatac gacttcttgc cacccctgtc 181800
ccaattcaga tctttttttt gattaatggt atgaacaatt ttcaaaatat cctggcacaa 181860
tctattagaa tatttgactt tctcctcctc ctccctttag ccctaactat ttggcaaagc 181920
tgttgaaaac ttcttcacat cttctctctg cctcatctac tttgctactc aaagatgatt 181980
agtagatgtt ttggtatgat tttttttcct tggcataact gtgaatactt ttaatccttc 182040
atgttttatg acaatcatac attcccactt ttgtgtaaaa acattttgaa ttcttctttc 182100
tataacaaat tctttgccag tcttcctttt ctttttgctt ttagtgagtt ccaatgatat 182160
cattgtttga acctgttttc taattctatg ctgctattcc catactccct ctttgctttg 182220
gttacagtag ataatgtggg tggtcctacc aagacaaagc atcactcagg agtcaagggc 182280
tggggtagat acagacacta ggtactgcag aaagtcaata tctttctcag agtgtaaggc 182340
aggacaagac ttctgttccc ttgtaccgtt gtggctgggg agttggactg tgcatttcat 182400
ccttgtcata taagtcaaag tattgcccaa ataacttaat tttggtgtgg tcttagtaag 182460
tatttgtctc gtagatatgt taaatagaag acagtaacat tgggttggct gtgttaattc 182520
ctcacttttt ctttcctata catgagcttc ctaagaagcg gaacacttgg tggtcagaca 182580
gttgcagaga ttccttgcat gattgcaatc tggaaagtag ataccattct tgaatgaaga 182640
gcttgccctt tggagaagct gggctttcca tatatggagg ttgttagaat gcataagagt 182700

tcagcttctg gaatgaaagg agaccttggt tcaaaaccca tctgccactt gcttgctgtg 182760
tggcttaaat caggctttta aacttcaatt ataattttgc atctgtataa taggattaat 182820
ataattcctt cctcataaag ttcctagggg gttaaatgaa acaatcaatg tataacacac 182880
ttcctggcat gtgatgttca gtacctagaa gactcggttt ccttggtaga gagaatattt 182940
ggctagacaa gcttatggaa ttaccccag ataggaagtg agcacaagtg tgaaatgaac 183000
aagccagagc aggaacggct cttggaagcg tctactcagg cctgggcagt tgctggttta 183060
tataacagtg ctttgaaaat tcacgtgcag attcttacta ttttcccaaa tgttacagct 183120
caaaactatg ttgtctgtac cttaacacct aaaggataat atagtctttc actgataaaa 183180
ctaaaatgtc ataggttttc ctttggccaa atatgtatag aaacttgtga tttcacatca 183240
gatttaaagc tgtatttaac actctatgaa aacatactga tgcttagaag tagaaaggaa 183300
gtcagatttt gacatcttac ttgtcaactt aaattattta tagttcctgg atgcttcaaa 183360
atgtgataaa ccatagttaa ttttatgtaa atattcgatg agtgccttta ataaggagac 183420
tgtaaaggta gccaagcttt atatatgtta gctacattta tgggtcaatc gggtataaaa 183480
aataggactt cgaaaataaa atattatttt gtcggactcc tccaatgagg ctttttcgca 183540
ggattagcta aaattggctc ttatttgatg tgtgagtgct taaacattgg agaattcatt 183600
tttcttttag aattcatttt tatttctgag ccttaaaata tgaacagtta gctaaatgtt 183660
tgtatatgtt gataaggaat gctaagtgtt tattccttaa tgggacgaca ccttttcccg 183720
gtttacataa cttgcctttt aatctaacct tatgaaagtc tccttgactt taattttttt 183780
tcagagtact gtatatctct ttagggaatg catttattta aaaaattata aagcaagaat 183840
agatgtgata tattttgaag ttttctagtc acaaattaaa tccctagatg tgttgtagtt 183900
tgtggagcac tttgaaatgt gccaattcaa gatggaaata gcaggaaaga accattcaag 183960
tacgatttct gactccataa agttaggaag ttatgataaa ggaaaaataa ctacaccaca 184020
tacttatggc acagaattgc attattggga caaattgatc ttcaaatttg taggctatga 184080
tggaagcaaa tatttgtagt atcttaatat tcaactgtta agccaggaga cgagtactct 184140
gaacttcagc ttctcataaa atcagtcagt tacatgacag tttatgtagt ttatatgtaa 184200
gaaacccttt gatcaagata tgccttttct tcagccttgt taatacttca tttataagga 184260
tttttatttc taggaaaata ataccataga cctattttat ttaaagctaa agtgtttcct 184320
ggtgatggtg gtgaatgggg agatgattca aggaaactgc taatcttgta gagtttagta 184380
aaatcttgga atagaaattt taaaaagtta aaacacacta tgaaaacaaa tcattattag 184440
taaaatgaac catattaaaa tgtctccata accaacgtat tatagcaggg gaaaatggca 184500
ttttaattca gaaaaacatt tctatataaa acaagctttg gaataatttg aatatgttga 184560

tttttctttg gggcaatcat gaaatacagt catattagga aagaggcaag gcctcaaaac 184620
ggaaagagta gtgaggataa ttcatgagca atgcgtggct tcatggatcc cttcctggcc 184680
ctcttcatct atgaacatct gctttatgtt catgtctggc ctcacccagt gctgaagagc 184740
agactgcccc tgcttagaac caagccttgt tcttgtggat ttgagttttg gggtcctgag 184800
gtagaatggc catcatattg ttcagggtcc tcaccttccc actcattact tcctttatag 184860
aaccccagtc atccccctca ggaggcctgc gctccacaaa tgaagttggg ggtgagggga 184920
gctttagcat ctcagtattg ttcagatatt ggatacttgt tgagctccac tatgtgtgtg 184980
agaatgcgct gggctcagga tcaatagcca taaatgagac agatatggcc catcccttgc 185040
catgcctaaa ctgatctggg cattgagaca agcagttaaa acccaacata gtaagtccta 185100
tgatgagaca caaactaagc atggctggaa gggcagataa gatttcacag aggaactgac 185160
atcttgactg gcacacttag aagatgagta ggagtcagtt gggccaagag gagaaaaagt 185220
gtgtttccat gaagaagaag agcatgtgcc aagtcctggg ggtgagaagc atggcatgtg 185280
tcaacagaaa gacatgatca gagcttggat gggagttaga gcggggagag aaggcaagga 185340
aaaacataag agctttgcaa actgtgaagg cattgcattt agatgtcatt tttagagctt 185400
ggaggagcct caggacattg aggcaggata gtggcctgat ggcgtatgat tttaggaaaa 185460
atcactgtgg ccaccctgtg gggaaggatt ggcgggggaa ggctggaggc aaagggtcct 185520
gcggtgggct gtggtaatga tctgggctgg agaaggtatg gtggtgccca gcgcaaaatt 185580
ggtttcgctt tgaaaaataa ttagaaaata tttgttttga tctgtgtgcc tgtggtggga 185640
ggtggggagg gagggtcaag tgaggaagga gattgaatct agaataatgc ccatgtttca 185700
ggcttcacgt gcagacattt cttggttcag ctgggaagtg aataggaaca gactggcgag 185760
gaaagaagac agtcgtcagt tctgtctggg gcacatccat cttgacgagt gtgagagagt 185820
caagtcttga tggacagggg caggtggaca ttcaggaaac tcaggaaaga gatttgatct 185880
gaagtgacca ccagaaaata ctgacggaaa agaggtgaca aaaggagaga cctgcaatat 185940
tatattggct ttctctcagg caactgcttg gcctgtcttt atattccttt tgaatctctg 186000
catatgtacg ggccattatt tattttcaca actaacaaat gcagactttc tgtgtaatga 186060
caacaaagcc aaaccagctg ctaccaaagg agggaaaatc agaagagaag gaaaaagaac 186120
aagggaagct tggggaaaag ctgaatgtgg gtccttctgt tgctgcaggg gctggggtgg 186180
gcccggtgat tcctactgag aggcgttttc tctccccgct tcctgtcttt ctggttccat 186240
ctcattcacc tcctgtcccc tccacttcct gccagtcaaa ccttagattc ctccagaggc 186300
tttttatttt tatcttttga tgggcaagaa aatagtgcgg attatttttc caaaccttca 186360
cctgaacatc acatcgtggc tttggcccta tgggcttggt tcatccgggc ctgcacagaa 186420
ggacttttcg ggccagtctg gtcacataca tcgagtcctg tcttttcagt taaaaaaaaa 186480

cacacacaca cacactgcta tgtttcacta agacaactgg tgtgagttgt tttttagaaa 186540
atcaactcta cttcagtaag attttctcaa gcattatctt gagaagacca gataataaaa 186600
tttaaaaaga atttcttctt ttttttttat tatactttaa gttttagggt acatgtgcac 186660
attgtgcagg ttagttacat atgtatacat gtgccatgct ggtgagctgc acccactaac 186720
tcgtcatcta gcattaggta tatctcccga tgctatctta atgcaactta aatcaattgc 186780
tttaataaca catattgacc aagttacact cattaaggaa aaaaaactac tttgttgttt 186840
ttcttcttct gacgtgagct gaagacttag aaatagttgt taatagtgtt tggttaataa 186900
gaatttgttt taattgcata tatcaaatat gtatttatta aacttttctt tttgtcagtt 186960
tatcaattct agctttgtca caaaaggttt gccgtatgaa catgattctg ttgtacatct 187020
atttccattt ttgttaagag acgaattcaa ttgtaaaaat ctagtacctt ttattcatta 187080
aacatgttag ttcaggaatt tcacttggtt ctacaaagat acatatctac agtggatggc 187140
cagtgcaaac atgagactca gccaactggt ctctgaccca attcagttct cctgtcttct 187200
tctggcttac aaagtaactg gctctgggga gaaagtgagt caaagtaata tttggtttga 187260
atggttattg actattttct tctgaaactt aatgtatact aattaatttt ttattttatt 187320
cttttttttt tagagatagg gtctcgctct gttgtccatg ctggagtgca gtggtgctat 187380
cacagctcac tgcagccttg acctcctggg ctcaagcgat tctcctgtct cagcctcccg 187440
agtagctagg attacaggca tgtgccatca tgccaggcta atatttaatt tttttttttt 187500
gtagagacaa gatattgtta tgttgcccag gctggtctaa aactcctggt ctcaaactat 187560
cctcccacct cagcttcctg aagtactgga attataggca tgagctgcca cacctggcca 187620
tatactcatt ttttgttaaa agctgaaata tatcagcata tactgcataa ataccacagg 187680
agactaaaca ctgaaagttt ctttagggta tcagaagaat acacttttttg cttgcagtta 187740
gcatctgcac agataagttt tgtttctggt tctattactt cttcagtttg accctattaa 187800
taaggacaat tctaaaaata ataactgtgt ctggatatct gaatccgtgt gtggtctttt 187860
actgaagtta caggtttata actctgctga ctagtttgct ggtttctgtt atgcaataga 187920
agagtgcaaa tgttaatttg atctgaagcc cgtgtataca gtgactttta taatgtatat 187980
ttaaagatgg aaagccaagt tttatgaggc cagcatttct tgtcaagtcc tcactccacc 188040
ctcttctaat tgggcctgac ccttaagttg aataaagaac aaagagctct gtagttaaaa 188100
catttcactg catgttgcat cttgccctta gtaaatggaa aaaaatagag acttaagcag 188160
agaatctgaa ctagggtgtg aaatatatat tcagttttgg ggtgggagaa tgagaaccat 188220
gttttacaat agtatacata acttccttag tctaaattca ggacatttcc ccaagatatg 188280
taagaattta gacttatgca gacagacttt ataaaaacat gccaatattt attagtttgt 188340

gaattttaat attctgctcc ctataaacca gatttatttt gagggataaa gggatggagg 188400
tgacttctaa atcttagagc agaaacttcc tgtgggcagc tggacatatg taccaggagc 188460
tcagagaaga gggttgtgtt gggagcgtac attctgagtg atctgcattt ggtgatgatg 188520
gaagccatgg acatgcacta gattgtcttg tgggagaata tggagtagta agagaagaag 188580
aagacctagg attgagccct gagcacctct ggcttaatgt tggatggagg aaattgaatc 188640
tgtaaacaat accgggaggc tgcagcctga gaggcagaag gaactggggt gtttgggatt 188700
atggaagcca agggaaaaag cctgtctcac agcgggaagg gaggtatcaa cattgtaagc 188760
tgcttcagat aggttatgta ggatgtggac tgaaaaatac ctgtaaaatt tggcaacgcg 188820
attcattggt aatcctaggg aagttgcttt tttggggtaa ctaaggtgga aaacagattg 188880
gtgtgggttg agcaatgcaa gagaggtgaa gaaaaggaga tttcatgtgc agacgtttct 188940
gagttcagct gggaacaagg gataagatag aagatggaag ttagagagca tgtggggcaa 189000
gggagactct tttatgggac agtctcgcat gtgatcaaag ccaatgagta aggagcattt 189060
gagggagaga gagtcaatca acaagagaga aaagaaagag ggttcatcat aaaataacgg 189120
taacaacaac gaacatcttt tgagtgtttt ctatatccgg ggaactatgg taaactccta 189180
acctgcattc tcacattcaa ttcttagaat cgttggatgt ggtgggttcc atgatttcct 189240
ctagattagc gaggaggaaa gagagatcta gaaatgtcag gtagcttgct cagagttctc 189300
caggtagtca gtcatggact aatttgtgaa ctgaaggact gaacttcgtc accacccagc 189360
ccaccaagcc ggcttgactt taggtattct gtgctgcatg tgagtaccga cttaaattat 189420
attttaagaa gggctacttt gaaactctct ctctgaaaac tctatttcta aaagctctac 189480
cctcacaaca attttggcaa gcagtcttgg taaaaccaaa ccaaaccaaa ccaaaaccaa 189540
aaaaccttat ctgctgagaa aatataacca cataaaatat ggtgctacaa aatatagact 189600
gtgtgaactg aaggtgactt gcccaaagga ctcctgaagc aattggctgc tgtagaaatt 189660
aagtccacgg gaggtttttt gttctgtttt tttttttttt tttttgagat ggattctcac 189720
tctgttgcct aggctggagt gcagtggcgc aatctcggct cactgcaacc tccgcctccc 189780
gggttcaagc gattctcctg cctcagcttc ctgagtagct gggattacag gtgtacacca 189840
ccatacccag gttttttttg tatttttagt agagacgggg tttcaccatg ttggtcaggc 189900
tggtcttgaa ctcctgacgt cgtgatccac ctgcctcggc ctcccaaagt tctgggatta 189960
caggcatgag ccaccgtgcc cggcccatga gaggttttgt ttgcacttca agaaggacag 190020
aaaaaggcag gcaggctggg gagcaacata gtaaggctga ggaagtgata ggaaaacagc 190080
ctccaaaagg tttccctgta gattctgact ggctaagttt cctgaaataa tattaattct 190140
gtcctcttgc ttttaatagg acataacgac tatatgtgtc cagccaccaa ccagtgcacc 190200
attgataaaa acaggaggaa gagctgccag gcctgccggc tccgtaaatg ctacgaagtg 190260

ggaatgatga aaggtggtag gtacatctct cccaggggcc cttggggatg gccctggcca 190320
ccgcccagtg ctggctctac ccattggaat aacaccatgg gaattttgtg ttttttttctt 190380
ttaattgttt tttttctatt cttatttttc tttgcaacaa aagtattttc ataatccatt 190440
ttattttaaa aaggtggaag tgtctggaac tggaaattct aacatggcat tttgtgtttt 190500
ggattttcaa tgtaaataat tatattttaa atcaaaggtg tgtgggaggc ggtgatggaa 190560
ggaaacgaag agtgcttagt aaattattct agaaatattt ttcagttact gtttatgttg 190620
caaatgctag aaaatgatat ctgaggataa actttcccta aattgagact tgtaaatgtg 190680
aaagctgagt agctaattta tagccttcca gtctgttatc atcccttaag gaatgtgaat 190740
ttcaatcaaa aggcagtttt ctcctttaga acctgagtga accagccact ttcttaacct 190800
cagtgtctag catggtgctg gcatagttga ttactgagca ctacactaac aagtgtcaga 190860
gcatgcatgg tttgtgactg tgggtttgtg tttttgtggt ctttgtggct gtgtgtgtgt 190920
gtggttttct gtcttctcat gtatccgatc ttccagtttt gtcatacagg aatctggaaa 190980
ctgaatccca gttctgggaa tattaggagc cccataaatg tggttgcctg attgacctca 191040
ttgtattctt gggagtctca tcttgaggaa cattgcttct agtcttggat agccttcagt 191100
gtagtggaag agaacaagta gaaaatgtgt aattaaaata aagtcatgag gctgaggtgg 191160
gcagatcatc tgaggtcagg agttcgagac cagcttggcc aacaaggtga aaccctgtct 191220
ctattgaaaa tacaaaaatt agctgggtat ggtggcgagt gccagtaggc ccagctactt 191280
gggaggtgga ggcaggagaa tcacttgaac ctggcaggtg gaggggggcag tgaactgaga 191340
tggcgccact gcactccagc ctgggtgata gagtgagact ctgtctcaat aaataaataa 191400
ataaataaat aaataaataa ataaataaat aaataaagta acggattcat ttagtgtttc 191460
agaaggatac tgaagggagg gaagggctga tgatggtgct acctgctgat tgtaataggg 191520
aaaagcccgt ttcttttctg aaggaggtga agcttggggt gatttaagga gaacaaaatt 191580
tcaatgaaaa ggaatagtga ttttgcaaat agtgggcagc taacctttaa atcattctta 191640
ctgggacgca ggaggaagga gaggaaagac caagaatgga gattatgatg aagacaagct 191700
tgattgttaa caagcttaca gtgacaggtt tcatagtccc ggggtcataa atacgccaga 191760
caggttgagg tttggaatgc agcatttgga ataaattctc ctggtgaaga gatgcaatgt 191820
tgaatttacg cattctgtga gctgtgacta tgactataca tcttgaacat ctgaaaaagc 191880
agctttaatt caaagggtaa ttaattccaa gaatttaaca gctacattca gaaaatgaca 191940
cttgagtcat atggattaaa tatttaagga attcatcttt ctttgtactg ttggagaatt 192000
agtcaggctt aattaaactt acaaaatgcg tcttaaggta acttggtaag tgacaggaat 192060
ataacagctg cataagaaaa gctttatttg aaacagtgga gtcgaggttt aatattcttc 192120

tttggcgatt atatgtaggt taagcacagg ctcttccata cattctcctt gaaagctgaa 192180

ataatcaagt catgaatatg tccaaaacaa tttttaaaat gtgaggggta cccatgcatt 192240

gaccccattt cataaaaccg attctgtttt tttttttgta attaatatcc agatacggtc 192300

tggctgcatg aatataaatt acgcattctc atttttaatc taacaaaaat tcatatatgc 192360

aaagacataa taaaagtctc cccactgttt tctcctagga atctagataa tttgactgta 192420

cacaacagac tgtggatggc tccatacaca cttgcacatg tatattgatg actgtaaaat 192480

atatatctgt attagttttc tagggctgcc ataatgaaat accacaggct gggtggctta 192540

aacaacggaa attaattttc tcccaattct ggaagctgga catgcataat caaagtgctg 192600

gaatatttgg tttctagtga gccctccctt tttttttttt gagagatgga gtcttgctct 192660

gttggccagg atggagtgca gtggcactat ctcagctcac tgaaaccttc acctcccagg 192720

ttcaggcgat tctcctgctt caacttccca agtagctggg actacaggtg tgtgccacca 192780

tacccagcta atttttgtgt tttttttttt tgtagagacg gagtttcact ctatgttggc 192840

caggctggtc ttgaactctt gacctcaggt gatccgccca cctcggcttc ctaaagtgct 192900

gggattatag gcgtgagcca ccatgcccgg cctgtgaggc ctctcttcta ggcttgtggc 192960

tagccgtctt ctgcctgtgt cctcacatgg cctttcctct gcgtccgagc actcttggta 193020

tctctttctc ttctcacaag ccctgttgga ttaggagtcc acccttatga cctcatttaa 193080

ccttagttac cgccaaaaag gccctatttc caaatatagt cataccaggg gctagggctt 193140

caacatacaa gttttgtggg gacacagttc ataacaggct gccctcccaa aataccatag 193200

actgggtggc ttaagtaacg gaagtcaatt gcctcatagc tctggaggct gaagtctgag 193260

actaaggtgt tggcaggttt gatttttccc gaggcctctc tccttggctt gaagatggct 193320

gccttctctc tgtgtcccca catggccttt tctcggggca tccacagctt cttcttcttc 193380

ttacgaagac accagtcata tcggattagg gccccacaca caggacctca ttgaatctta 193440

atcacctcac ttaaggtaca atttccaaat acagtcacat tctgaggccc agggggtaag 193500

atttcaacat atgagtttta agggtacaca attcagtatg tggcaatatc ccataggggt 193560

tactcagtga ttgttcagta tttctttaca tgtaaggaaa acatgtatct ttctggcagt 193620

ttttatatgt acataagttt taaatgaagc tttaaattca taaaattata gcccgtgaga 193680

acatcttatt ttaatgatat gtataaagta cttgccataa atcaatataa tttattgtat 193740

tttctacaac atggaatacc acacatggcc acaggatacc ctctataaca actccttgaa 193800

aaatcccatt gaataaagcc ttaatttaca actggaagta caatgataac taactataaa 193860

atgtgcagtc acatggcaaa ggccagctgt gactgaataa atctttacaa catgttttca 193920

accattaggg ttcacaacaa tccactttct taaccaggct ggttaccaac taggatattg 193980

aaactctacc tatgaaataa aaagtttagt ttcagttaaa agagaaccac agacctactc 194040

cctattttca tagcaaggct gaaaaaattc actgaagatc tattgtaaag atccaaaaga 194100

gtgcatgatt tgtctccatg aactaacaat tttaatctgt tataatactt actgcctctg 194160

aaaatcccag catcattatt agatttggtt ctgaatcatg gtagttctat aagatgcagg 194220

atttgaattg cactttcggg aatgtgcttt tctatcctag tacatgtgct ggtttctctg 194280

cattgctttt tcctcttcat ttgtctcctt cctcttgaac tttaggttgt agattacatg 194340

tctcttcttc aggaaggcct acacagttac ctgacagttc ctgaggctcg aagttcaaaa 194400

tcaaggtgct ggaaaattca tgttctagct ttaatggcaa acagctcttc tcttagctct 194460

tatcaccatt attattagtc attcaatgtc tgtctttaca tatggtccat aaactccatg 194520

atggcgcggg ggtggggggt ctctgtgtct tgtttaccga tcttgcatga aacaagtcac 194580

aaggtaggtg ttcaagaagt atttttttgc actaattaat gaattgattc aattcattta 194640

aatagttatt tgtgtagggc ttcttatatg caaacccttg ggaataaagc aaatatgaat 194700

aagattgtag tcccttccct caatgtcctc acagtataaa gagaacattt gcatgggaag 194760

cggcaaataa aaatctcaac ggcaagtgga gactgggttg caagatggct caaatattga 194820

atgatcagaa tgggagtcta gagctttaaa caaccactcc caggtttagt ccaactcaga 194880

gtattactca gacacctgac atccaagaca tatattatta ctcaagaatg tggccttttt 194940

gctgagtttg gtagaaagag gaactcttcc aaagataaat gtgttcaaat gctggctcct 195000

cctcaaactt gctgaacaac cttgttaaag tcacttttat gagcttcact aatttcaaat 195060

atgaaatgga gataaataaa ttttattgtc caagcacggt ggcttacgcc tgtaatccca 195120

gcactttggg aggctgaggt gggcggatca cctgaggtca ggagttcaag accagcctgg 195180

ccaacaaggt gaaacctcgt ctctacaaaa ttagccaggt gtgatggcag aagcctgtaa 195240

tcccagctac tcaggaggag gaggcaggag aatcacttga acccagtagg cacaggttgc 195300

agtgagccga gattgcacca ctgcattcca gcctgggcaa cagagtgaga ctctgtctca 195360

aaaataaata tataaataaa caaataaata aattttactt aatgagtttg tcatgaagat 195420

gacaaatcgt aacatgggaa aatataaatc taaagtgcca cacaagtgtt agctattaca 195480

tatctttttt gtcttaaaaa taattactat cttagttaaa aatttcatca tatacgagca 195540

ctgctgggtt tttttttca gctgaaagaa agagaatggc aatttcatca cttctacaag 195600

atataaattt ctaatttctt tgtggagaat aaaaacttgg cttaaacacc taagcttttt 195660

tttcttttct gatctcatat gctgaatata gctgaatgac aagtgagatg tgttatttat 195720

cattttatga tgccagagat ttacacgaat ttgtgaaagg ttttttctta ccatagattc 195780

agaggcgtgt ttgaggcaag gttttcattg tgcagtgagg aaattggcac atagaaatca 195840

attgatgtgc aggactcttg gactgactat tctggttatg tttccttata acacattaag 195900

aagaaatttg agataaagta gtaattgtca actagtcttt atttatttta ataacaaatg 195960
ttgcagtata atttttagaa atattccaaa ataacaaata aatccaaaaa ttaacgggat 196020
aattttaaag tgagaaaata acatactact aatatagaca gggaaatata catgtgtcca 196080
gaatgattca gaagcccaat gaaacactgg aaaataaaat attttcctgt tgcttgggtt 196140
taatatttat cactatgagt tatcgtaatt tgctttattg atggataaac tcccaacctg 196200
gggactgcga ttggaatcct ctttaatcct acattggcta tcctttatca aaagtaaaca 196260
accggctggc catggtggct cacacctgta atcccagcat tttgggaggc cgaggcgggt 196320
ggatcacctg agatcaggag tttgagacca gcctgaccaa catggtataa cctcgtctct 196380
actaaggata caaaaattag ctgggtgtag ttgtgcccac ctgtaatccc agctatttgg 196440
gagcctgagg caggagaatt gcttgaactc aggagacgga ggttgcagta ggccaaaatc 196500
acaccactgc actccagcct gggtgacgga atgagactcc atctcaaaaa aaaaaaaaaa 196560
agtatacaac caattattaa tgttcatgat ggtagaatca aattaataaa ttaaaaatgt 196620
agatgtgaat tacttttttt ggattgccga ataattgtgg aagatttgta gctgcatgtg 196680
aatccaagtt tcctcaaaag gtaggttggg gtgctcctat ttaggattga atttaagaaa 196740
aatgttaaag aaaagctagc tgctctttac caaagagaga ctggttgttt taagcagtaa 196800
tctaatctat atatttatga tattgaaact attattttat taattcatat aatagatatt 196860
tattaaaaac tccccatatt ataatttttt atttgcaaac aaatatactg aatcaagtaa 196920
gtgaatcaca tatggacctt aaagactaat gaatccaatg tttgacagta ttttatatca 196980
atcaagctct ggtttccaca agcagaagca gatcgtcatc tatgaaacag ataaaagttg 197040
atttggcata ccctctcttt attcattatg atgttcatga gaaaccttct tagaacccta 197100
gggctccaag gaacacagtt tgcaagccac tgatctaatc taactccttc atcctctagg 197160
tatgaaaatc aaggccctcc attacctatt agggatgaag gcaggtcagc ctctgtgact 197220
gtcagttcag ctggaggaag catgagtgta ggcagcacaa caatccctat aaggaccatg 197280
ttcttaagct ggtttttttt ttcctagtag tttttacagt ttcaggtcta acatttaagt 197340
ctttaatcca ggttgagttg atttttgtat acggcatgag ataagtgtcc aatttcattg 197400
ttcggtgtgt agatatccag tttctctaga aacatttatc aaagaaactg tcctttctcc 197460
attgtgtgct tttagcagct ttgtcaaaaa ttaattggct ctaaatgaat gaatttattt 197520
ctgggttctc tattctgttc cattggtcaa tacatgctgt tttaattact atagctttat 197580
agtatatttt gagtcaggta gtgttatgcc tctagctttg ttcttctagc tccagattgt 197640
tttggctatt cagggttttt ttgtggttcc atacaaattt ttggattgtt tttccacttt 197700
tgtaaaaaat gtcattgcaa ttttgatagg cattgcattg aatctgtaga ttgatttgtg 197760
tcatatggat atttttaaca atattaattc ttccagtcta tgtatggtat atctttccat 197820

ttatttgtgt cttcttcagt ttcttttatt gatgtctata gcttttagca tacaattctt 197880

tcacttcctt ggttaaattt attcccaaat attttgtttt atttttatag ctattataaa 197940

tgaaatttct tcttgatttc tttttttgga tagtttgctg ttagtgtata gaaacactac 198000

tgatttctgt atgttgattt tctcttctga aactttactg aatttgttta ttagttctaa 198060

tagtttttttg tgtggagtct atacttacat gatctatgtg taagatcatg tcatcagcaa 198120

acacgggcaa tttaacttct tctttttcaa tttgcatgcc tttatttttt ttcttgcata 198180

attgctctgg caggaactct cagtactatg ttgaatagaa gtggtgagag tgggcatcct 198240

tgtcttgtta ttgatcttag gggaagactt ttcaaatttt caccattgag tatgatgtta 198300

gctgtaggct tgtcatatat ggcctttatt gtgttgaggt acactctttc tatatttaat 198360

ttgttgagag tttttgatat gaaaggattt tgaattttgt caaatgcatt ttttcctcta 198420

ttgagatgat tgtatggttt ttgtccttca ttctgttaat gtgtgtacca tagttataga 198480

tttgcatatg ttgaaccatc gttgcatccc tgggcaaatc ccactcgatc atggtgaatg 198540

attctttttc atgtcatgac aatcaatttt gctagtattt tgttgtggat ttttgcatct 198600

atattcatca gggatattgg catgacattt aattttcttg taataccctt gtctggcttt 198660

agtatcacag taatgctggc cttgtaaaat gagtttggaa gtattccctc ctcttcaatt 198720

tttgggaaga gtttgaggat tggtattagt tctttaaata tttggtagag ttcagcaata 198780

aagcccatta ggtcctgggc ttttcttcca tagaggactt tttattgctg attcagtctt 198840

ctttctagct attggtctgg tcagactttc tatttcttca tgattcagac ttgctaggtt 198900

tatgtgtcta agactttatc catttattcc aagttatcca agttgttggt gtgtaattca 198960

tcatagtact cttataattt tttgcatttc tgtgctatca gttgtaatgt ctcctctttt 199020

atttctgatt cagtttattt gtgtcttctc acttttttttc ttagtctagt taaaggttaa 199080

tcaattttgt ttatcttttt aaaaacaaat tcgtagtttc actgatcttt tgttttgtgt 199140

ttttagtctc aattttattt atttttcctc cgatctttat tattttcttc cttctactaa 199200

cttggggctt agtttatttt tatttttcta gttatttgtg gtataacatt agattgtttg 199260

agatcttctt ctttgatgaa gacatttatt gctataaact tccctcttga aaatactctt 199320

gctgcatccc acaaattttg gtatgttgtg tgtccatttt catttgtttc aagatatttt 199380

tcaacttttc tttttatttc ttctttgacc cactggttgg ttctgagtat gttgttttat 199440

tttcaagtat ttgtgaattt tccaaaatta ttcttgttga tttatagttt catattattt 199500

atggttggaa aaaatacttg atatggtgtc aatgttctta aatttattaa gacttatttt 199560

gtgtcctaac atatgaacta tactgttttt tttctttttt tttttttgag acagagtctc 199620

actgtgttgc ccaggctgga gtgcactggc tcaatcttgg ttgactgcaa cctccgcctc 199680

ctgggttcaa gctattctca tacctcagcc tcccgagcag ctgggaatac aggagcacac 199740
cgccatgcct ggctaacttt ttgtattttt tagtagagat ggggtttcac catttgggtc 199800
aggctgctct caaactcctg acttcaaatg atctgcccac cttagcctcc caaagtgctg 199860
ggattacagg agtgagccac cgtgccaggc catgatctat actgttttat ctaacattca 199920
gtggataatg ttccatgtgt atttgagaag aatgtgtgtt ctgttgctgt tggatgaaat 199980
attctgtatg tatctgttaa gtccatctgt gctaaagtat agtttaagtt tgaactttca 200040
ttattgattt tctgtccgaa taatctgtcc attgctaaaa gtggggtact gagttcccca 200100
atattattgt attacagtct atcttccctt tcatatcaat taatatttgt ttcattcatt 200160
taggtactcc aatgttgggt gcatatgtat gttcacctgt tatatcctct taatgaattg 200220
accctcttgt cattatttaa tggccttctt tgtcttattt tatagtttgt gacttaaagc 200280
ctattttatt tgatataaat atagctgtct ctgctttctt ttggcttcta ttttcataga 200340
atgccttttt tggtcccttc gccttcattc tctgtgtttt cttaaccatg cattgaatct 200400
cttctagaca tcatataaca gggtcttgtt tttttaaatc catttactca ctgtatctct 200460
ttcttcctat cttgctgtct tcctttgtga ttaggcgatt ttctatagtg gtatcctttg 200520
aattcttact ttttgccttt tgtatatttg ctacaggatt ttgctttgtg attaccataa 200580
ggcttacata aaagatctta tagttatacc aggctatgtt caactgttta taacataact 200640
ttgattaatt ttttaatact caatttattt gattgcttat ttagagataa ttttgattcc 200700
tatcagaaga aaactgaggt atctttatgt accttttcag gaatgaccac gtctcacctt 200760
accttgaatg ttacaagaaa tgctggcagt gactcaaggc caggcaaaaa tttcagaaag 200820
ggtttatctt gtttgtacaa ttttcttttg caaaaataaa tgattcttga tatgatgtaa 200880
ggactttttc cgtagataga acctataaaa tgcagaactt tggggcagta attatatgaa 200940
tgaattgcta ctttaaaatt ctgtaagtcc ccaggaattt ccatatgctt ttttttgttt 201000
gtttcagtgg ttgtgatcag gctaatgggt agtttttaga gctgaaaaga acttaaagat 201060
catctagtgc accttttgt ttttcagttg gattgttctt gtccaaatca ctaagctaag 201120
ttaactgctc agacaggaaa ccaagtctcc tgacccatgg tttaatgctc ctttcattgt 201180
tatgcgtctg cctggctgac ctgttagtaa cataaagtgt catgagagat agacatatgc 201240
atgagtaaga tattaatatc ccataaacca aaactgtgac ttaggaaatg ttacggtggg 201300
cacaggatat tactcctctt caacaaaaac atcaatcata tgttggctta gttgcacagc 201360
agaagtacca aaataaattt atatagacgg tgtcacagat actttaaaaa gacctactta 201420
agatttaatt acctaatatt atttaatatt taatataatt taaaaggggc atttttatct 201480
ttcagccaag gttttaattg tttttagata attctatttt tgaaagtctg attttgtttt 201540
aaaatgccca ttctggtata taaaaacatc attttgtcaa ataggagtcc cagctaaggg 201600

ccaagcgtgc aattaaactt gactctttac ctactaccta ggaaccttgg gcagatcaca 201660
tactttttct tcactcagtt tttacttctg aaaaatgcca gctgaagtat gtttcaaatc 201720
ttaaatttta tgttttatga tctaagttca gattaacaat ggaatggtta gaatccaaag 201780
ctaaaggtag tatatacaag tgtatattgg gggttgggag caactgccgc agctatcata 201840
tttacatttc aagctcaatt tagaacaact gcgggcaact gtaaaaaccg gtaggaatgg 201900
gcagagttat tgtcttcact agtcattgtg gaaagtgaat ctcttctgaa tggagagtca 201960
caaaaatgag tttgatgctt tcatgatagt gaaatgagaa gatggccata tattgctata 202020
aatcaccagc aacataaaag aaaaaattca aaaatcaagt ttgttgaggc aggactctga 202080
aaagataact aagctttttt tttttttttt ttctgttaga gatgtaataa tcatgactaa 202140
gactggaatc ccaagagctg gtgagacttt ctagtttctg ccttgtggaa agagaaagga 202200
agaggccaaa aaagataatg aattttggaa agttcctgat taaatatcgc ataggctagt 202260
ctggttatgt ctgcttgaaa acttatccag taggtataaa ccaacacact tgaggccagt 202320
ctttttggaa attacagaaa tatatgtcag ccttaagaga catcctatga tgaagagcag 202380
agggagttgt ttataaagaa atgttctcat tggctgggca tgatggctca cgcctgtaat 202440
cccagcactt tgggaggccg agctgggtgg atcacttgag atcagaagtt ccagagcagc 202500
ctggctaaca tggtgaaacc ccgtctctac taaaaatata aaaaaattag ccgggcatgg 202560
tggcgggcgc ctgtagtcgc agctactcgg gatactgagg caggagaatg gcgtgaacct 202620
gggaggcgga gcttgcagtg agccaagatc gtgccactgc actccagact gggcgacaga 202680
gcaagactct gtctcaaaaa aaaattagcc aggcatggcg gggagcgcct gtaatcccag 202740
ctactcagga ggctgaggca gggaaattgc ttgaacccag gaggtggagg ttacagtgag 202800
ttaagatggc accattgcac tccagcctgg gtaacagagc tagactccat ctcaaaaaaa 202860
gaaaagaaat gttctggtca tgcctggaga taatacatgg tttagtttat gcttgattca 202920
gcaaataaaa attggtgagg aacatacaga attatattta ggtgattata ttttttatat 202980
tttatatata aaatttatat tttttatatt ttaggtgatt atatttttaa acgcttgctt 203040
tacaagacta atcattatta cttttttaag ttttgtgata tattttattt aactaatata 203100
ccccaaatag tatttcaata cagaatcaac atttaaaaat tattgataca ttatgtatgt 203160
ttttttcctt cactaagact tccaaatcct ccaagtgttt cacacttaca tcttattttg 203220
gaccagccag attttagtgc tcatttgacc atgtgatctg tgcttaagga ggctgctgga 203280
gctgaggctg ggctgcagta ccaacataga agtctttatc tgtcaaacca ggagtttgag 203340
atccctgagg gatatgggaa ccataaagga tcttaagcag agaagtgact ttcttaattt 203400
tgaacctatc ctggtgttct catagattag aggagagaga tttaggagtg agggatcaag 203460

caagaaggtg gctccacatt gttttccata gtagttgtac tagtttacat tcccaccagc 203520
agtatagaaa tgttcgcttt tcactacatc cacaccaacg tctattattt tttgattttt 203580
tgaaaatggc cattcttgtg ggagtaaggt ggtattgcat tgtggttttg agttgcattt 203640
ccctgatctt tggtgatgtt gagtattttt tcatatgttt gttggccact ttgtatcttc 203700
ttttgagaac tgtctattca tgtccttagc ccactttttg atgggattgt ttgttttttt 203760
tcttgctaat ttgtttgagt tccttgtaga ttctggatct taatcctttg ttagatgtat 203820
agattatgtc tccttctctg tgggttgttt gtttactctg ctgctgctcc ttttgtgcaa 203880
aagctcttta gtttaactaa gtcccactta tttatctttg tttttgttgc atttgctttt 203940
gggtttttgg tcataaaatc cttgcctaag cccatgtcta gaagggtttt tctgacgtta 204000
acttctagaa ttttatggtt ccaggtctta gatttaagtc cttgatccat cttgagttga 204060
tttttgtgta aagtgagaga tgaagatcca gttgcattct cctacatgtg gtttggcaat 204120
tatcccagta ccatttgttg aatagggtgt cctttcccca ctttatgttt ttgttggctt 204180
tggcaaagag cagttggctg taagtatttg ggtttatttc tcggttctct actctgttcc 204240
tttggtctac atgcctattt ttataccagt accatgctgt ttcggtgact atggccttat 204300
agtatagttt gaaatcaggt aatgtgatgc ctccagattt gttctttttg cttagtctta 204360
ctttggctat gtgggctctt ttttggttcc atattaattt taggattttt tttctagttc 204420
tgtgaagaat gatggtggta ttttgatggg aattgcattg aagttgtaga ttgcttttgg 204480
cagtatgatc attttcacaa tattgattct actcatccat gagcatgaga tgtgtttcca 204540
tttgtttgtg tcatctatga tttatttctt tgagaggtgt tttgtagttt tctttgtaga 204600
ggtccttcac cttcttggtt agtttttgtt ttttttggtt tttttttttt tttgcaacta 204660
ttgtgaaaag gagtgagttc ttgatttgat tctcaccttg gttgctgctg ttggtgtata 204720
gcagagctac tgatttgtgt acattaattt tgtatcctga aactctgctg aattcattaa 204780
tcagttctag gagctttttg ggggagtctt tagggttttc tagttataaa atcatatcat 204840
cagcaaacag cgacagtttg aattcctctt tactaatttg gatgtccttt atttctttct 204900
cttgtctgct ccggctagga cttctggtac tgtgttgaat agtgagagtg ggcatctttg 204960
ttttgttcca gttctcagag gaaatgcttt caacttttcc ccattcagta ttatgttggc 205020
tgtgggttta tcatagatgc cttttattac agtgaggtac cttgtatacc gattttgctg 205080
aaggttttaa tcataaaggg atgctggatt ttgtcaaatg ctttttctgt atctattgcg 205140
attattgcga tggtcatgcg atttttgttt ttaattttgt tcaggtggtg tgtcacattt 205200
attgacttgc atatgttaaa ccatccctgc atccctggta tgaaacccac ttgatcattg 205260
tggattatct ttttgatatg ctgtttgatt tggttagcta gtatttcgtt aaggatattt 205320
ttttgagatg gagttttgct cttgttgccc aggctggagt gtagtggcac tgcaacttcc 205380

actttctggt ttccagcgat tctcctgcct cagctccctg agtcgctagg attacaggca 205440
cctgccacca cacccagcta atttttgtat ttttagtaga gatagggttt cgctatgttg 205500
gccaggcggg tcttgaactc ctgacctcgt gatccacctg ccttggcctc tcaaaggact 205560
ctcaaagtgt tgggattaca ggcgtgagcc acagcgcccg gccagatttt tgcatctatg 205620
ttcattagga atattggcct gtaggtttct ttttttgttt tgttctttct tggttttggt 205680
acaagggtga tactggcttc atcgaatgat ttagggagga ttccctcttt cttcatcttg 205740
tggaatagtg tcaataggat tggtaccaat tcttctttga gtgtctggta gaattcagct 205800
gtgaattcat ctggtcctgg actttttgtg ttgttggtaa tttttaaatt accatttcaa 205860
tcttgctgct tgttattggc ctgttcagga tttctaattc ttcctgactt aagctaggag 205920
gtttgtatct ttccaggaat ttacctatct cttctaggtt ttctagttta tgtgtgtaaa 205980
ggtgttcata gtagccttga atgatctttt gtatttctgt ggggttggtt gtaatatctc 206040
ctgtttgttt ctaattgagc ttatttggac cttctctctt cttttcttgg ttaatcttgc 206100
taatggtcta tcaatttcat ttatcttttc aaataaccag cttctttttg tttcatttat 206160
cttttgtatt ttttttcaa tttcctttag ctctgctctg atcttggtat tttctttctt 206220
ctgctggctt tgggtttcgt ttgtttttat ttctgtagtt ccttgatttg tgaccttaga 206280
ttgtctattt gtgctctttc agagtttttg agtaggcatt taaggctgtg aactttcctc 206340
ttcgcattgc ttttgctgta tcccagaggt ttggatggtt gtgtcactat tattgttcaa 206400
ttcgaagaat tttaaaattt tcatcttgat ttcattgttg acccaatgat cattcaggag 206460
caggttattt cacttccatg tatttgcatg ttttggaagg ttccttttgg agtcgatttc 206520
cagttttatt ccactgtgga ctgagagagt agttgacata gttttaattt tcttagtttt 206580
tttgacactt gtttgtggca tatcatatgg tctatcctag agaaagttgc atacgctgat 206640
gaatagaatg tatattctgt ggttgttggg tagaatgtta tgtaaagatc tgttaagtcc 206700
atttgttcca gggtacaatt taaatccatt gtttctttgt tgactttctg tcttgatgac 206760
ctgtctagta ttgtcagtgg agtattgaag tcccccacta ttattgtatt gctgtctgtc 206820
tcattactta ggtgtagtag taattgttct atacatttgg gagttccagt gttaggtgca 206880
tatatattta ggattatggt attttccttt tggacaaggc cttttatcat tatataatgc 206940
tcttctttgt ctttttaac tgctgttgtt ttaaagtttg ttttgtctga tataagaata 207000
gctactccta cttgcttttg gtgtccattt gcatggaatg tctttttcca cacccttacc 207060
ttaagtttat gtgagtcctt atgtgttagg tgagtttctt gaaggcagca gatacttggt 207120
tggtgattgc ttatccattc tgcaattctg tatcttttaa gtggagcatt taggctattt 207180
aaattcaatg ttagtattga gatgtgaggt actattctgt tcatcatgcc atttgttgcc 207240

tgtatacctt ggattttttt tagtagtatt tttgttttat acctccaatg agatttacac 207300
attaaggagg ttctgttttg atgtgtttcc agcatttgtt tcaagatttg gagctccttt 207360
tagcagttcg tgtagtcatg tagtgctggc ttggtagtgg cgaattctct tagcgttgtt 207420
tttatctgaa aaagactgta tctgtccttc atttaagaag cttagttttg ctggatacaa 207480
aattcttggc tgctaattgt tttctttaaa gaagctgaag atagggcccc aatcccttct 207540
agtttatagg gtttctgctg agaaatctgt taacttgatg ggttttcctt tataggttac 207600
ctggtgcttt tgcctcacag ctcttaagat tatttttctt atcttaactt tagataacct 207660
tatgacaatg tgcctaggca atgatctttt tgtgatgaat ttttcaggtg ttatttgagc 207720
ttcttgtatt tggatgtcta ggtctctaat aaggctaagg aagttttcct caattatccc 207780
cccagatatg ttttccaaac ttttagattt ctcttctttc tcaggaacac caattattct 207840
taggtttggt tgtttaattc tgtcccaaac ttcttggagg ttttgttcat tttttttttt 207900
ttttcttttc tttttttttt ttaattgatc attcttgggt gtttctcgca gagggggatt 207960
tggcaggatc acaggacaat agtggaggga aggtcagcag ataaacaagt gcacaaaggt 208020
ctctggtttt cctaggcaga ggaccctgcg gccttctgca gtttttgtgt ccctgggtac 208080
ttgagattag ggagtggtga tgactcttaa cgagcatgct gccttcaagc atctgtttaa 208140
caaagcacat cttgcaccgc ccttaatcca ttcaaccctg agtggataca ccacatgttt 208200
cagagagcac agggttgggg gtaaggtcac agatcaacag gatcccaagg cagaagaatt 208260
tttcttagta cagaacaaaa tgaaaagtct cccacgtcta cctctttcta cacagacacg 208320
gcaaccatcc gatttctcaa tcttttcccc acctttcccc cctttctatt ccacaaaact 208380
gccattgtca tcatggcccg ttctcaatga gctgttgggc acacctccca gacggggtgg 208440
tggccgggca gagcggctcc tcacttccca gtaggggcgg ccgggcagag gcgcccctca 208500
cctcccggac ggggcggctg gccgggcggg gggctgaacc cccacctccc tcccggacgg 208560
ggcggctggc cgggtggggg gctgaccccc ccacctccct cccggacggg gcggctggcc 208620
gggcgggggg ctgacccccc cgcctccctc ccggacgggg cggctggccg ggcagagggg 208680
ctcctcactt cccagtaggg gcagctgggc agaggcgccc ctcacctccc ggactgggca 208740
gctggccagg cgggggggctg aacccccacc tccctcccgg acggggcggc tggccgggcg 208800
gggggctgac ccccccacct ccctcccgga cggggcggct ggccaggcgg ggggctgacc 208860
cccccacctc cttcccggac ggggcggctg gccgggcaga ggggctcctc acttcccagt 208920
aggggcggct gggcagaggc gcccctcacc tcccggactg ggcagctggc caggcggggg 208980
gctgaccccc ccacctccct cccggacggg gcggctggcc gggcgggggg ctgacccccc 209040
cacctccctc ccggacgggg cggctggccg ggcggggagc tgaccccccc acctccctcc 209100
cggacggggt ggctgccggg cggagacgct cctcacttcc cagacggggt ggctgccggg 209160

ctgaggggct cctcacttct cagacagggc ggttgccagg cagagggtct cctcacttct 209220

cagacggggc ggcccggcag agacgctcct cacatcccag acggggcggc agggcagagg 209280

cgctccccac atctcagacg atgggcggca gggcagagac gctcctcact tcctagatgg 209340

gatggcggcc gggaagaggc gctcctcact tcctagatgg gatggcggct gggcagagac 209400

actcctcact ttccagactg ggcagccagg cagaggggct cctcacatcc cagacgatgg 209460

cggccaggca gagacgctcc tcacttccca gacggggtgg ccccgggcag aggctgcaat 209520

ctcggcactt tgggaggcca aggcaggctg ctgggaggtg gaggttgtag cgagctgaga 209580

tcacgccact gcactccagc ctgggcacca ttgagcactg agtgcggttt tgttcatttt 209640

ttaaattctt ttttctttgt ctttgttgga ttgagttaat ttgaaaacct tgtctttgag 209700

ctctgaagtt ctttcttatg cttgttttat tctattgctg agactttcaa gaacattttg 209760

catttctcta agtgtgtcct tcatttcctg aagttgtgat tgttttttat ttatactaac 209820

tatttcactg aagatttctc ccctcatttc ttgtatcatt tttttgactt ccttaaattg 209880

gacttcacct ttctctggtg cctccttaat tagcttaaca atcgaccttc tgaattcttt 209940

ttcaggtgac tcagggattt cttcttggct tggatccatt gctggtgagc tagtgtgatt 210000

ttttgagggg tattaaagaa ccttgttttg tcgtattact gggattgttt ttctggttcc 210060

ttctcatttg gtaggctatg tctgagggaa gtactagggc tcaaggctgc tgttcagatt 210120

cttttgtccc acaggttgtt ttcttgatgt agtactctcc cccttttctt agagatgtgg 210180

cttcctggga accgacctgt agtgactgtt atttctcttc tggatctagc cattcagcag 210240

ggctaccagg ctccaggcta gtactggggg ctgtctgctc agagtcctgt gctatgggct 210300

gttttcaggt ctcacagcgt tggattccag cacctgctct gatagaggtg gcaggggagt 210360

gaaatggact ctgcaggggt ccttagcttt tgttgtttaa tgcactattt ttgtgctggt 210420

tggcctcctg ccaggaggtg gcactttcaa gacagcgtca gctgtggtag tatagggagg 210480

atcaggcagt ggccagggcc ttagaactcc caagagtata tgacctttgc cttcagctac 210540

caggatggat agggaatgac catcaggtgg ggcaaggcta ggactgtctg agctcagact 210600

ctcctcgggt gagtcttgct gaggctgtgc tgtggggcag gggggtgagg ttcccaggtc 210660

aatggagtta tgttcccaga ggattatggc tgcctctgct gcgtcatgca ggctgtcagg 210720

aatgtggggg aaagccggca gttacaggcc tcacccagct cccttgcaac ccccaaaacc 210780

ggtctcactc ctgtgcccta ccaacagcat caagtttgtt tgcaggcagc ggatgagcaa 210840

ggctaagaac ttgccgcagg ctaccagcct cccagcaaag aatgtaagta gggctttcat 210900

gcctcccttc ctgttgaatc tgtacaccaa attcactccc tcccccaagt tctggccagg 210960

tgacttcatg tttggttgga attgttacaa agttcagctg gaggtttcct tctcgctgtg 211020

gtcttttccc agttcctctg gccaccctcc ccaaggaccc ctgtgagaca aggtagaaat 211080

ggcttattag gggacccaga gagcccacag ggcttttccc cttgctttct ctacccttta 211140

tttcactcag ctgtctaatt aaatcagctc caggtaagtt catatccttc tcccatgatc 211200

tggagctgca gattccccag tgagggtgtg tgttcggggg tgggctatcc ccctttccta 211260

ctttcacagc ttgggcactc acagtatttg gggtgtctcc tgggtcctgt aggagcaaac 211320

tgcttccttc acagggtctg tggattctct tggctttcct ggtatattcc tgtagtagtt 211380

ctggagcaga agtttatggt gtgagtttcc acacactgct ctgttcatcc aagtgagagc 211440

tgcaatctag tcctgcctcc tttccaccat tttttggcct tgacgtatta catttatttt 211500

atgtttgaag ttttggcagt tttattttga aaattttaca taacaaatgc caagccaaat 211560

ccaaacttct tggcctaaat catttacaga taacctttac tgagcattta ctacatgaag 211620

gtgttgttga cacaggggat ttaagatgtg atcactgacc acaacatcca ggcaacctaa 211680

aagtgaaaag aggtcattca caaaaaatag gaccaaactt cctccattcc tcaagggacc 211740

taggactgga gttggcctaa aaattcctgc tgtatcacct cacccaccag ttttgatctt 211800

gctctcaggg atcaaggaag ctcaaggttt ctggcccttt tggctcatgt tcttggaggt 211860

atattctatt gtcaagttaa agtcgtaccc atcctagatc tgtttctctc tctttgtggt 211920

tccagtctca tagaaaacat aactatgtga aaacaccaac taccccataa agctattctg 211980

agactcaaag acatgaactc tattttgata atcacaaatt gatgtataga tgtcagatat 212040

cattcatgta ttatgtagca ttgtggttta agtcttagct gttggaatca gactacttgc 212100

attcaaattt tagctctgtt gataggtgtg taaccttgaa caagatacgt aacctcttca 212160

gacctttgtc tctttgtttg tcaaataacg acaataataa tactaactta gtaagtatgt 212220

ataaggaggc atgtaaagct tctattgtat ttggcttatg gtgagccctt catataaatt 212280

ggttacaatg ggtaatgaaa agtacgttca atttttccca caaggattta atctaatctc 212340

taaataaaat gccagtttta gtatactgtc atcattataa ggaaactata attcttaagt 212400

aattaattac tttggttccc tttggataat tagtgataca taaaatgcag taccttgccc 212460

tgaggaacct acaagttagc ctccctctaa tcctggcatt caaagtcctc caccttccga 212520

attacacctg ttttttcagg cattgtctcc cactttccta gcaagccttt ggctccagga 212580

aactgttctg ttcattgacg acagaatgta ttttgtcccg tatgcctccc ttcaatctga 212640

agttcatttt tagtaatttt ttttattagt agtgactttc cccatttact aatgcctttc 212700

tctttagctc atttagaatt ccctggaagt catcatgttt tccttctgaa ttgctatcat 212760

attctgtctc tgtgtctaat cagaaagtta ggtgagctgc tgtctcagct cctgtgttaa 212820

tccaggtact gagctctttg taccctgcca gcttgagggc aacttggatg cgctggtgtg 212880

tagtgatttg caactgtgag agagccgttt ccatcaacaa gtgacagctt ttggttctaa 212940

acactgcttg acctcattct ggtttgaaca tatactttgg cctctgccct ctacctcacc 213000

tccagttctg atcttgggca tgccacagaa ccccatctac actaggcttt cattttggac 213060

ttctaagtcc tttacttgac cacttatttt ctttaattgc tagacttaat catccatgca 213120

tatgacagcc actcagctct gaccccaata cttcattgag gctcatgcca gtctgacctc 213180

tcacagggaa gagcacccct gccggtgagc ccctggccct tggattatgc tgtcccacca 213240

gtgcccagct ggctgcttgg cactgcatgt gatgtatctt tgtttattga tcgcttaatt 213300

gaatgggata taggattata ttccatgttc cagatcaagt tgagccaagc tgacaactta 213360

gtattaaaaa aatatattta tctcctatct gtccaattaa aattttaatt tctagaggta 213420

gagacagtca cattgctatt taattcctag cacagcagta tttttgtcaa tatttgttat 213480

tagtggtgag tttaaattat aaaggaaaaa agagaagtgg tgatttatgt cagcgcttgg 213540

ctaaactatt cccttgatat aagtctatta ttcaggtcac ataagcatga ctgaagtaat 213600

atcacaacca taatatcata attatctctc ttgctgtctc tgtcactttc tcacttttgt 213660

ttgtgcatag tgcatagacc tgtagaaaat ttattagtaa tctgaagcat aaaaccaaat 213720

attacaacta tcataacaaa taaataaaac tgtgcactcc tttagggagt agtaaagcca 213780

tttctaaaat taagtcaaag taagattccc ttattattga gatattttag tggtatacac 213840

tatcataagt aagatgaata attttgttag cagctgacac tgctatatag atatttatct 213900

tgggaagttc tcagtgtaac atctttactg tgtttttctc tttagattat acttttatgt 213960

ttattatttt gtttcgtggg ggattcaaaa atatgcattt tatgccatac ttggggattc 214020

cctataaatt attagttgta atatgacagt tccatcatga attttccagg tattctttta 214080

tgcaagcaga taatagctca ttttggtttt taacacattc atgcatattc tttctctctc 214140

aataaatatg gttttaattt attaaatgaa agatttaaaa atgtgctaag cattttaata 214200

ataatcgatt ttggattaac ttgttatgtt tactctaggg ctgtagttca ccatttattc 214260

agttaagtcc ttccccaaat tcaatattga acatgtggaa ttgattcgtt tcacgtggat 214320

gtatcattta ctcccaagat gttggttttt ggcatttagt actggtaatg ggccaggaaa 214380

gtgctcatct atatttgttg ttattcactg attgcatctt gcccttgcac ccactgagac 214440

gatgggaaag tagcaacaat actaggtgat ttctttgatt taaacccaat taaaagaatt 214500

agagagttgt ctgatacaag gccaaagaac taagaacaga aacaaaagca aaacaacaaa 214560

cagcagcaca aactccagtg agataaattt ttaaaacatt gggaatattt aaaaaataaa 214620

aacactccaa tgaaccaccc aggttttatt aaagagtaga gaactcaaac agcagaaggc 214680

agagctggtg gaggaaactc agcaactgct cagaaatgaa acaacctagg aaggaggtgc 214740

gagtgacctg aaactccttt taaaaacaga aaggacaaaa agaggtatgg gctgacaaaa 214800

```
ggaaagtggt agattactga tgtattgcat tatgcttaga atgtctcaga atgcgagagg     214860
cagtaaacac accagaagag gatacaatat ccggaccatg tgcaactgca aataaatgtt     214920
tgggttaaaa cttggttgat tctaaattac atgaagagct gatgataatt gaggcagaac     214980
tgatagacct aacaatagaa aaaaatcgat ttaaattcag agaagaggtc atttaagaaa     215040
gtataatgaa gccacttaat aaaaggaaat attcctctaa ctaaagtttg agatttaggg     215100
cataatcctc aaagacaggc taatataatc tattttttga ttaaaaaaag gaacttttgg     215160
tttaaagtaa gatgttcagc tgctcttaga gtttatttct ccatatttgg gcatataaga     215220
atttaaggaa aaatatagaa atataagaaa gatttcatga gaatcacaag catagtttat     215280
aggcaagata gccttttctg tttgaaagcg aaaaatacta tttcaacatg taaaaaccta     215340
agtcagtttt cactggcatg tcccacaatc atgcctgaaa taatggttga agacagatgt     215400
gagacatttc aagggcaata gattaataca tgaaacccac ttatgcctag cgttccatta     215460
ttggaacgct aagcatgtgg gagttattta tatcctattg ctcaaggtca tctccaaggt     215520
ctgagttttc actcatgcaa aaattcaaaa aattgcaacc tcggcgtaaa tgggttaaca     215580
aaaagttaat gctggacagt aaaataaact actaaattag acacaccata ttttttaaat     215640
tataagagat taagaactat gagatattta aaaagccacc cacagaagta gtaggacagg     215700
tagagaagga taaattctaa caatcaactg tatctccacc ccaccttgta aatgacaaat     215760
tagtttactt agtcaattca ggaaattaaa cgtatacatt ttgtgttaaa acagaagact     215820
ttttttaaaa aagtgtgtta gttaaggtta attttgacct agttaagaaa aagcagttta     215880
gagtcttgaa atggaaatga gaaatacata attaccctta aaaataagtt gatagtgaat     215940
tttatctagg attcttaaga catttttaat atttaatgaa aatgaactac acaattatta     216000
aaaaatagtt gcctgggttt agaaaaatga tcccttaata agacatatac aacagcctga     216060
atttttattt ccaaatacta tagcaacaaa ataccggcaa gaaaaatgca cagaaacatt     216120
ggatagtctc ttcttactaa tcttttacag atatttatat attctgaatt ctaactttta     216180
atttatttta tgtattataa atgtcatcta gtatgcagct tgactttgct ttatggtatc     216240
atctgtttca cagaaaattt taagttgtgg aatttatcgt atttttcttt tatagtttgt     216300
gctttaagac ctgtctaata aatctttcct tccctggacg ctataaagtt atttgtctat     216360
attctgttaa aaggaataaa actttgcttt ttttcacatt ttgttactta tttcacctga     216420
aagagaattt tgtttattat gtgaggtagg aatataactt aatttttttc cattgaataa     216480
ccaaattgtc ccagagcaat taagcaatac attctttccc tgttgatctg tgaagctacc     216540
tccattaggc atgaagttgt cctgttgaag agaatctgat tctgagctct cttcactatt     216600
tctttatttg tctatgtcta cactaaaagc aatttaattt ctgtgtcttt ataattactt     216660
tgactttata atccttgaaa gggactttgt tcttcttcaa aattgttttg gctatttatg     216720
```

gctacttttg ctttcaaatg agtttttaaa tcaaacttta atgagattgc attaaactta 216780

taaattaatc tgaggaggat tgacatcttc atgatattta gtcttcctac ccattaacat 216840

tatataactc tccatttatt ttaggtcttt ttgaatgact tctaataaag tttttcaatt 216900

ttctccaaaa tatcttacat attaaaattt actcatagct gtctcatctt actgatattt 216960

taaagacata tctttttaaa aattatatta ttaaattgtt tcaggtatgt cttttttttt 217020

gaagttttac aaatttggga ttatactgcc tcttcattca acattttccc atgctgttgg 217080

ggatccttca agtcaaaatt attagaggag ctgcattaat attccagcat ggacatacta 217140

taatttattt aattatttcc cgaattgtat gtttttgttt ctaacttcac agttttcaac 217200

atgactggga acagcttttc ttgctaaatc cttgcacgcc acagtaatta tctcactggg 217260

ccaaaagata ggtacttttt catgactttc gccatatatt gtcaaaatgg aaccaatggt 217320

gatttgttca gaaatcatgg tacatcatat aaaaagatta ctctgcaagc atcacaaact 217380

gtgatatata gaaatgtgtt tattgacatg aaatagatca tgaagaaagt gattacaaat 217440

ggtatttaag cattttaagt tatataggct tatactgagc aataacaaag agggtgaata 217500

aatgaatgaa tatgtgattc tggaagggta tgtaacaaaa ggttaacaat ggttagccct 217560

gggtaagggg attatgtatg acttttattt ccttcttttt ttcttccttt ccccttatgt 217620

ttttaacaat gtacatgaat cgtttatgaa taaaataaaa aactttgata ttcttatata 217680

ggtttagtag gataacattt ctccttcatt tctttcattt ttagaaatct ttcttcacgg 217740

aaattctttt ttttaaatgt ttatttctgt ctttgaggag tgagattgac tggctatctt 217800

ttaatacctg tgtgtattat cattattgta tcttgctgat aaatttcaca acagttaatt 217860

attttattcc ccatttcata cttagctcta ttttaggcaa gttcacaaat atcattgaat 217920

gagttcattc aatgtcatca tgatgatatt gaaatgtcaa tagcagatga cttgtttctg 217980

gggtgttaat atgcctgttg gttacatttg tgttttttgg tgatggaagg cagccactga 218040

ggcaaacagc aagaatacag ctaagcctct aactgcatac tcttctcact cgttgcttaa 218100

aactgaagcg tggataattg gtgtgcattc cctaaaaagt tatgtcatga tgcagtagag 218160

aaataagtta actttcctat ccctgtatca aagtttcttt tgaaggtaac tgagacagcg 218220

ccaaataagg agcagagttt ctgtgaatca tgaaatcttt ctaaacaatt gagaaaaaaa 218280

aaactgttct gaaacaaaaa cttgtgccct atccttttat ttgaatgtgt tttcttaagg 218340

ccacaactgg cagagatttg atgattttat gtttaaagca attttttttt tttttcatag 218400

caaggagtcc cactgccatc aggttttgtt tttggagaga ttctgtaact gacatagggt 218460

aacttactct gatggcttgc tccacattca ctacaagtac atactgttct gaaaaatctc 218520

aaagttgaaa tatttttatt tgggcaactt ctgcaatcaa ggtaattcac tctatctttg 218580

gagaataaaa tggaaaaggg caacccaatc tctgtgacct taaacttcca ctgaaactta 218640
gagacggtta gcttagctgg tgagagcgtg ctgctaataa caccaaggtc gtggtttcta 218700
tcccattaca gggcagtcaa gctcaaggga aaaaccctgt tctttggcac agcttccatg 218760
gcctgctagc cgtttacagc gaggcaaaag agtagagatg ttttagtgaa ctaggccatg 218820
acttggggag tctccacctt gaatctggtt gtgggaccaa ggaagagaat ctgctgaggc 218880
tgggtagaga gactctagag caccttaggc cacaggacag cagaggatga gaataacaga 218940
actggcttca gaaagtaggt gtcagtccaa ggcactgtat tcaatacatg tagcaaagaa 219000
tgagcttctg acaccaggaa agtcttcaga tggcagtgac tgcataaacc ggtgtcagga 219060
tgtctgacta taaaggatgg gacaccttcc atcaggtgag agctctggag gtggagtctg 219120
ttcctggaag aagcatgggt aaatgtcatc aaaggttccc tcagccacca ctgggttcag 219180
ggctgaggtg cacctaggaa ggcttgggca gaagcatgac ccctggaact tggccaactg 219240
agccatcaga gagtggtgca ggtgaggagt ggtagatgtg tagtgaacca ctcagctgtc 219300
tgcccaggcc ttgggtgtgg aagtagagtc ttctgtgtct ttgggcagac tgaattataa 219360
gcttcaccag atcgccaagc taggaatgtt gacatacctg gcttggggtc agggagggac 219420
tgaactgtag gtgggggcc ccaacaaatc taatcataag aatctatgag cgtggagaca 219480
gaaggaccaa agaagggatg ctgtaaccaa ttatcattat tgaaaaacta ggcagagatt 219540
acattcaatt ggcagtacca cccttttat ctctgacata gttccagaag gatatactat 219600
gtatctagtg tgacactgtc tgatagaatt gtctgaaatg atggaagtgt tctgtggcta 219660
cccattttga tagccactag ccacaggtgg ctattgagca cttgatatgt tgttggtgtg 219720
actgaggaac taaattttta attcaattta aattaaattt aatttaaatt taagtagcaa 219780
catgtggctg gtggttactg gattgaaccg aacagatcta gatactagag taagtggtag 219840
cagcaaatcc tatattggaa caatgtaaat taacaattat aaatatatga gcaggacaaa 219900
gcccaaatgg gcctgtactc ttagttttgc tatatattca accataacta gcactcattt 219960
tttaaactta taaaatgcaa actcatttgg ttgtgatgat gaatgctcag gtaattgttg 220020
agtttcaaca cagtgacagc caggaactca ttttggagac ttggattgtg ccacagtttg 220080
tccctctata aaatgatgat gaaaatttta tcagtatctg tctcaagctg tagttaatgg 220140
gccaatttct aaatattcct gtcaaattgt atagccattg ttttcaaagt agttttgaaa 220200
gtaggccatt ggtaaagcaa tctattaaag tgcaggaaga aagtaataga atctcttact 220260
ttttaaagtc tcattatgaa aattatattt cagtttgtat gccttcgatg tacataatat 220320
attaatacac aaatatatta atgcagcggt ccccaaactt tttctcacca gggactggtt 220380
tcctggacga cagtatttcc atggattgca gcggggatgg tttcaggatg aaactgaccc 220440
acctcagatc atcaggcatt attaggttct cataaggagc acacagcctt gagcaggtgc 220500

agttcacata tagggtttgc gcacctatga gaatctaatg ctgccgctga tctgacagga 220560
aatggggctc aggcagtaat gctcgctcac tgctcacctc ctgctgtgtg gcccagttcc 220620
taacaggcca tgaactggta ccagtctgtg gcctgggggc tggggagtcc tgcattaatg 220680
taattaaata tgtacatttc ttgagagtgt ttgttcaaat atttttcct aatagagacg 220740
tataacttag aaagtatgga accctctggt cagtagcata ttttatcaaa aaggctatcc 220800
gtcgtgctaa ccaatgtgat taaatgttca ttttagtctc agatattttg ataacataat 220860
aatagggaag ttatggttat tgcatggctt ttctttactg tattttcaat aactatatta 220920
atgggttcag ttgttactag ttttataatt ctgttttatt ctcaatgagt cttcctgttt 220980
ctccccacgt ctttcctatg gttcatttcc ctgcatcttt acagctccct ggaacattct 221040
ctcaaatcaa tcagaggctt caaaactggg cagtctcatt ttgtaacctg ctctgactcg 221100
cctctgggag gcctccctcc ccactcacag ctgccttcct gcatgatgta aaatgaatac 221160
agtgccagaa agactgatcc ttggaacctc ttccttactt agtttcttta tagttctgat 221220
tttagaaatg accattttac ttccagtgcc cttccccaca ccagttagct gagactctat 221280
gcaggaagtt cccagtgatc agaactgttc ccatagctcc caggtgattc tgacatgcac 221340
ccggcattga gcaccatgga catggctgct gtttgttatc ctcgagctct gcaaagcagt 221400
gtcggatatt gcttgattgt tgcacattaa tctactgcac ggtccggtag gtagccacta 221460
gccacatgtg gccatttaga tgtaaatgaa tttaaattaa atagtattat aagttcaaca 221520
cctcagttgc actctctgta ttcaagtgct cagtagctac aagtggttgg tggctaccat 221580
tttgggcagt gctactatac aacatttcta tcaccacaga gagtgctgat gaataatagt 221640
actaacaact ggaaacattg aaatggagag gtgaaggaga gaaggcagaa tataagagaa 221700
acttcaaagc attcagcgtg tctgataggc gctgtatctt acattatgtt tggagcatag 221760
tttttgaatt tcttaatttt caaattcggt tgaaacagat tttggtcaat tgaagtgaag 221820
gactatgcct gctttaattt gcgtagctta cctttgtttc acgtgtctcc cttattacag 221880
tgtttgtagc agcatctaac tcaatcccta acttcttttt ggtgcaggga ccatttaaaa 221940
acaatttgat catagaggtt atctcctttg caatccatat tgttttatgg aacaggtgaa 222000
cagctggtga caatagcctc aaggtttcta atcccaccaa aggaaaatat ggtgtcagta 222060
attaccttgt actatgatat ttgtgaaatc caagagaggg ctagatctca ggctctaata 222120
atacatccgt ataaacatct cagtagggca ccacggaaga gtcttaggat atcaggaaca 222180
ataagagcgg gagaatatcg accttaatat tctttacact tattaaaaag tgaaaagaca 222240
gccataaagc agaatgagtt cctgtccttt gcagggacat ggatgaagcc agaagccatc 222300
attctcagca aactaacaca ggaacagaaa accaaacacc tcatgttcgc actcataagt 222360

gggagttgaa caatgagaac acatggacac agggagtgga gcatcacaca acggggcctg 222420
tcgggagttg ggggactagg ggagggagag cattaggaca aatacctaat gcatgtgggg 222480
cttaaaacct agatgacggg ttgataggtg tagcaaacca ccatggcaca tgtataccta 222540
tgtaacaaac ctgcacgttc tgcacatata tcccagaact aaaagtaaaa taaatgaaaa 222600
agaaataaaa aataataaaa taaaataaaa taaatgaaaa gtagccattt taccagttga 222660
gtatttactg acttttggtg aaaatcactt tctaagatta ctagaaacct tatgaccctc 222720
ggcctctact cccaccacgt gcggattttc acctccacct actttgtcac gtcacatgcc 222780
cttctgctct ttaggacact tgtattgggt tcctccccca cacgtaaaca gaacacacag 222840
gtctgtgaat ggacctcatt atgctcattt gtctttgcca agaactattg aaaatgatca 222900
caaatgacta tgtaattata attaagattt tgtacatgca tacactttat ttttaacaag 222960
tttgtcctgt taaatgtcac cccattttga atagtaacat tcactggtgc aaggaagaat 223020
tattaaaatg acaatttaac tgatatcctt gacaggtaag ccagttattg tatttcataa 223080
ttgtgattat tacaatacct atgaagatta attaggacaa tttattctat aggacttggt 223140
tatcaaaatg atcttttttc ccattttagt ccatacatct tcagttttaa tttccttctt 223200
ccaaagaact ctgagtcttg cccagtgtga tacttcagat tttgtaaaaa tataatgaag 223260
aagaataaca tgtatcagat ctttttttta atgtactggg ctaaaaatta taagcaccat 223320
caaattacta gaaaattctt ttaacactct gcttcttagt taaaaaatga ttcctttttg 223380
tgataatctt ataattttca aagtatcatt ttagaaattt taaaaaatta ttcttgtttt 223440
cattcagtcc atttcatcag aacttagatt ttttcttccc tctacagtat gtggtcttga 223500
tgtgctttct ccttgcaaaa taaaatcttt agtttcagga tctctgtgtt ctattccttc 223560
aatttaaaat cataggaaaa ataactatca tttccttatg tctgtctcca attcccccat 223620
cattttctgt gagccaattt tcttcttttt cttctgtcta ctgtaaggaa ctttttactc 223680
tgctctattc tctgcaattc agggctcaca atttctttga cttctcagat tatcctttgt 223740
acttgtgtct actttttcat taaaaaaaaa aaggcttcgg tcttattttg actttatcat 223800
ctttatattg ccatatttaa aacccatctg cttattgatt gaaccaaact tttcatctta 223860
cttcatcata ctgaatgtat aacttctgaa tgacttaaaa atcatcacat aatgtgagag 223920
aattttctat catattcagg tgactgcatt gtaagcaatg aagttgaaga aaaatagctt 223980
ataatggata taataccaag aaataataag aatgagaatc tgcgtatatc atatactggg 224040
ttaacaagct cacaaggcaa atcaccattt tattagatat ttaacaaatc atactagtac 224100
tggaggaagt atagcagaaa aaaggagtat ggacattggg attagctgcc ttgctaatct 224160
tttgtcagtc cctagtgaca tgcttttggg ttgagtcatt gttacctccc tgagctgttt 224220
gtttataaaa tgggggtaaa tatctcagat gtttcagtgt tgtgacatat aaagcgtcct 224280

ccatagtaca taataccgta agcactccat aaggtaattc ttgtcctttt aaaaagtata 224340
cacttttgag ttcaggacac gtacactgta acctagttgt aaccatttta ataaaagaag 224400
gttttgatca tttttgataa atgtactaca tgaatagtta acttgccttt cacttcctat 224460
accaacggaa attttggtaa caaatttaaa gaaagcatta ggttgaatat cccatctgat 224520
taaatgtcaa ctaaatattg tttctggctt ttaaaatatt tctaattcag ttataaaaca 224580
ggtctattac tcaggaggct gaggcaggaa aattgcttga accagggagt cggaggttgc 224640
agtgagccga gatcatgcca ttgcactcca gcctggcgac agagtgagat tccgtctcaa 224700
aaaaaaaaaa ggtgaaatga aaaacaaaaa agaggggtga aatttctctg cattctccct 224760
ttctgctgtt gggaaagccc tttcattacc aagaatgcat ggaattctgc ttattaattt 224820
ctccatctat gtgtctctgt gaaaaatact aattatttac aattttgtaa gctatgaata 224880
aggtctatgt tttctctgaa attatagcaa caggagcatt tccccctttat ttgggccgca 224940
atcttttcat tctgattctt accataactt ctgacttctg actttatcat gtcttctgat 225000
ttctactaaa catgaacaga atgaatgact tctagaaatg gatacaggtg taatacatat 225060
aaggccaggg aaccacttaa ttatctttac aaagtactag gacttcagaa aaatcaaatg 225120
atgaaatgga gctagcttta gtatgttcta cttatcattg gactattaat catgtctgtc 225180
tgatgagtta cctaggactt caactgtttt tatagattta aagaaaatat tataaatgta 225240
tcttacctgg atttaagact ggacagctga ttttggattt ttttctgcta ccctgttgtt 225300
gatatcactg acatcagcct ttgcattttg actggaggct cctttctctc tttctctctc 225360
tctctctctc tctctatagg aaaccagtag ttatgtcagg ttgctaagta ttttcccact 225420
gaaggacaaa tattctggag cagtatatat ttagcatttg acatttgata aacaagcttg 225480
tttgagatat tttcaataca atttcagctt tggcaattgt aagcaaagga tccagttggt 225540
aattggtgaa atgaacagat gtggacacgc tgcctgcatc gagatgcatt aatttttctg 225600
aaaagtggat aagatcttgt gagaatgaat aatggaattc aaatcagatg gggtgagata 225660
tttgatattc aggaacaaat cacagtataa tagatctgct actttgtttg gttttaatgg 225720
ggagttgcta atgctgaaat atagtcagag aaaaggaacg tggaatttct atagaggaga 225780
gcctggctct ggggtaatac tactgtcctt ggtggaatgt tgtcttttct gaagacattg 225840
gttcaattca atacatgaaa cattcatcga ttattattta gaaaagctaa tctactgaca 225900
agacacagag ttaagtagtg aaaatacaga gacctagaca cagttcttat taagaaattt 225960
tttaatttag gtggcatatt tcagagagaa attctctgct tggggcaata ggtacctcct 226020
ttggaataag catggtgaga aagaattgct tctggcagat tagtgagaag agtgagatat 226080
tttctttcga tgacctctct attaaaattt gagtggtaaa actttgtgat gattcaggct 226140

cttcataatt cttaatttta tcatcctcta ataccaggac ttgcccagat caatatttta 226200
ggaaacagtc atgcttgcta aacccaggga tttctattaa ccactagata agacataagt 226260
ttgtcatgca acaagtattt attgagtcca taatttgccc agcactgggc tttggtactc 226320
tgggccttga tactctaggt tgtgtcagag atgtctaata tatttaaggt gacagagttc 226380
acaaatgaga ctttatgaaa atgaatatcc ctttttaagt ctcgagaaat atgatgtgca 226440
cccgcttgag gctttattaa atctccagcg agcatgaact tgtttgtgac ccaattgtag 226500
aattgttgta tgatgactat cccactggca ggcacttctt gtccaagaga atgtaattgg 226560
atgttggtga ctcaagcaat cctgggaaga ctcctccatg accaaattaa agacaacagg 226620
ggcttggttt gtactcagct ctacaccaat cagcatgaga caaagaagag atccatggca 226680
aagtggggag actactgttt attttacaag ctagagaagg aaaactgcag ttcctgagtt 226740
gcaaaactgc taagaaatgg gagaacacag aactttacag gctgaacctt ggtgtcttag 226800
ttattcctct tggctacaga atgccaacca gtaggagatt catcactgag atattacagg 226860
gaaatgaagc caggcaagaa aaatatgtat tccctttctt ctcagcccat aaattttgtt 226920
attaataaat cagactctta tagaacagct tgccacggct gtctctagaa atgtttttat 226980
tagcagaatt tttattaaaa taaaatacac agtagtttta agagtgaaca ttatagtttt 227040
aggtcataag ggtgtcatg gagaacacat gtggatgcta cttgccagtt acttgatcta 227100
ggccttgact cttggttttc tgttgctcgg ataagttagc atgatttgac atgcagttaa 227160
aggtgtggta acctgtgatt gatttcccat tcttgatgct ccctgcctgg acttctcgga 227220
aaatttcaaa acaattcatt cttctatgag ggctgcctcc ttggttgctt cggctaaaca 227280
gtgttgagca acaagttggg aaaggaacgt tgcagtaact tttaaaaatt aagttagaaa 227340
caaatgtcat caaagtaaat gataatttgc caatatgatt gagcaaaaag atgaagatgc 227400
acgctcccta cgtatttgct ttgcagaaga attaatttga aaaataataa catattttaa 227460
aatgaattgt aaatataaac acatgctaat tgcagagggg aatccctgtg attattcagc 227520
agtttgtgtt tgtcaagtgc ttttcagtca gtccattcca gctcagcagg gcagaggctt 227580
gggctgttgt aaacttgtgg gcagataccc agtgatgggg cagacatgag caggtagggc 227640
tgacagcatg tgaattgagt tttcttcagt catgctgttg cagctgctcc cttccctcat 227700
tgctgagttt gccacagcag gtaggaacct aactctggag cctgggatga aggagaaccc 227760
acattgggct tgaggaacaa gatctgccac cctggtaggc cctggttaaa tctcatgcaa 227820
gtgtagcaat gagagagggt atgattgagt tctaatttag gaggaaaggg gataatgtgc 227880
cctttgcacc cccaggagaa atcattgctc atctgtgcct aagtagactt atcaagggca 227940
gttggttcac aatggtgtat caccccaaag gactatagtg ttatgagaac ttgccagtgt 228000
atttgaattt gggtgctggc agatgacatc atgaggtatt atggttaccc ataaatatgc 228060

tagtttattg agaaggtggt agacatgcta gtggatgaga gggaaggaca gaagcagtta 228120

gtaaacagca tctgcaacaa ttcagttaac tggtggttgt cacagtagca tggtggaaaa 228180

gttggcaatt aataacttct aagaaaactg aactaatgaa ccaatcctgc gtgtgctatg 228240

tgtataacct ccttctcact attaacagat ttgttccaaa cttatataag acaatgaaaa 228300

taaagcttgg caatataggg aagggatgga gggataaagc tgtaaatcac gtcacaggca 228360

aattaagata taccactgga tcaagggatt taatgcagaa agactgatcc taacttattt 228420

cttttattta gcaataagat ttgttactta cattgattat ttaaaatgag ttgcattatt 228480

agaaaggact attttgaaga caactataat aaaatgtcag taactgatag tagccaagat 228540

attttaaata tatcaaagtt gtgtcattaa tattaatgtg tcccttaata tgaagtcctg 228600

cccaggctta tttatgtatt caacagacac atacctgttg aagtgtaaca gatattctgg 228660

acacaagcaa tggtaaacaa gacagatgca gtccctgctt tcatgcaatt tgcaattgaa 228720

tggtctttga cattttattg tgattgtttt agttatttaa ttggagaagt tttaatttta 228780

aatttgtgtt atattcagtg ttaaggaaca aaaatgtaat gtgcatttct gagactcagt 228840

aacacttctg gtttttcctt tttcatttaa agaaaaattt agtgcccaag ataagctaga 228900

atttttggaa tcaagtaatt gatgacctgg gagccaattt tattacaata gtgtttttag 228960

tggtcttaga acttttcaga ggtggtagct ctgaaaataa cactgtaata aattcacaca 229020

tacatctatc atccaataaa tgttaattga ggcccactac agcattgtgt tagattctga 229080

ggttacaaat tgttgacccc atgtcgaaca tgttgacccc atcaatgtaa tcagtttgac 229140

attacacagt catttaaaca ttaaagtgtc agcggatatt ttagttgtaa ttttgataag 229200

tgctctgaag gagaaaacag tgggtgttgt gaaaagcagt atttggttga ttgattatta 229260

tagaggatct gagaaaactt acttgaggaa ggaacatttg gctgaactca aagagatgag 229320

taggagttaa gtaagcaagg aagaaaagaa gacacatgaa ggaggaagaa tgttctagaa 229380

acacacacac acaggtatat gtatatgtac atgtatatgt atatgcatat gtcttcctta 229440

gaaacatata aacagctgca acatgattga acttatttac ccaattacca aagcttattt 229500

tgcaccatga aggtggagat aaaggctttt taagcagcaa ggatagaatc tttgtagttt 229560

ttatttatag gctggttctt ctgacaactt taatttttca tctttaccaa cttcatggtc 229620

ttcagtacat acaatgcaat cattattata aaattatatt ttgactcaaa ctctaaggta 229680

ggatgattca gctgtgcgcc atcaacatag cagcatgaat ggtagagact agtcattcca 229740

aacagtgaag gggcaacgta aaactaattt taatattata tgaaagtact tcttgccctt 229800

gactgctttt tttttttttt gaagaaagca aactttaaaa atttatttta gatttacaga 229860

attattgcaa ggatagtaag agagttctca tatatgcctc acccagtttc ctctattatc 229920

gacatcttac attatatggt acatctatca taactaatga accaatattg tttcattatt 229980
agtaactaaa tctatacttt attcagattt tctaagtttt cctctaatgt tctttttctg 230040
tcccaggacc ccatcaggat atggtatgta tagttgtcat gtcttcccag gctcgccatg 230100
gttgtgacag tttctgagac tttcattgtt tttgataccc caggtagttt aggcattttg 230160
tagaatgcct cccagtctga atttgtctga tgttttcctc atggtttgac tggctttaat 230220
gtgttttggg gaggaagacc acagaggtta agtgtgattg tcatcacatc gtatcaaggg 230280
tacatgccat caatatgact tatcactgtt gatattaacc ttgatcatct ggcttgagat 230340
agtatttgtc aggtttctgt attatacagt tactcttctc cctgtccata cagtactttt 230400
tggaagaagc cattttgtgc agctcatttt ttttttaatt ttaattttaa gttctggggt 230460
acatgtgcag catgtgcatg tttgttacat agttaaacgt gtgccatggt ggtttgctgc 230520
acccgtcagc tcatcaccta ggcattaaag ccaacatgca ttagttgttt ttcctaatgc 230580
tctccctccc ccaaccccaa tctgacaggt cccagtgtgt gttgttcccc tccctgtgtc 230640
catgtgttct cattgttcag ctcccacttc taagtgagaa catgtggtgt ttgggtctct 230700
gttcctgcat tagtttgctg aggataatgg cttccagctc catccatgtc tctgcaaagg 230760
atttgatatc cttccttttt atggctgcat agtattccat ggggtttatg taccacattt 230820
tctttatcca gtctatcact gataggcatt tggtttgatt ccatgccttt actattgtga 230880
atagtgctgc agtgaacata tgcatacatg tatctttgta atagagtgat ttatatttca 230940
ctgggtatat acccagtaat gggattgcca ggttgaatgg tatttctagt tctagatctt 231000
tgaggaattg ctacaccatc ttcctcaatg gttgaactaa ttaatttaca ttccgaccaa 231060
cagtgtaaaa gtgttcctat ttctttgcaa cctcgccagc atctgttgtt tcttggcttt 231120
ttaatgatca ccattctgac tggtgtgaga tggtatctca ttgtggtttt gatttgcatt 231180
tctctaatga tcagtgatgt tgagcttttt tcatatgttc tttggcctca tgaatgtctt 231240
cttttgaaaa gtgtctgttc atgtcctctt ccaacttttt aatagggttg tttgtctttt 231300
cttgcaaatt tgtctaagtt ccttgtagat tctggatatt aaacctttgt cagatgtata 231360
gattgcaaaa aatttccccc agtctgtagg ttgcctgttt tctctgatga ttgtttcttt 231420
tgctgtacag aagatcttta gtttaattag atcccatttg tcaattttgg cttttttttg 231480
caattgcttt tggcagtttt gtcatgaaat ctttgcctgt gcctatatat ttattgcata 231540
gattttcttc taggggactt caaactatgc tacaaaactt caataaccaa aacagcatgg 231600
tactggtaca aatacagaca catagaccaa tggaacagaa tagagaactc agaaataaga 231660
ccacacatct acaacgattt gaccttcgag aaacatgacc aaaacaagca atggggaaag 231720
gattgcctat ttaataaatg gtgctgggag aactggctag ccacatacag aaaattgaaa 231780
ctggacccct tccttacacc ttatacaaaa attaattcaa gatggattaa agacttaagt 231840

ggagctcata tttaaggagt gagaagatat gctctacctc tttaagggtg gagtagctct 231900
ataaattatt tggaagtgtc tattctcctc cattaattta tttagtcaac aattagtatc 231960
agccatctag aacccatgaa tatttatgct ttgggtacag tccaatacta ttttattttg 232020
tagctcatct tgttccagct ttggccattt ggagattttt cagttggctc ctgtatctct 232080
ttggcttctt acatatcatt gtagggtttt ttaaaagcct tttcttactt tctgtcacta 232140
caagatagtt cagacttatc ttctgtattt tttgccccag ttctatgatc agccacttct 232200
ccaaggagca ataatttcct tcaatgaaaa ccaagatatg ggctgttggt gtacttgttg 232260
ttattgtgtg ttgttacttc tagatcctct aagctgatag tgcaaagaga tatatgtgtg 232320
tgtaccaacc tatatatcta cacacatata aaaatatttc tatttgtaac catctgtatc 232380
tatcttaggc taaacctgag tacctactga tgtctccaat tctaacctgc aacagcatgg 232440
aacattctag ccttctcctc ttacttatct gtcacttcct ataccaatag tgagaaacct 232500
ggctcctacc atctgctatt tatttactta attatttaat tccactatac ttctatggaa 232560
gtttcagaat tgttaatctg tactcatgta cgaaacaact ttatcaacta gagtatagtg 232620
tttatataca gttcctttgc ctttattcta acagattcca cttactcatt ttccgagtca 232680
cttaggttag cgccttattt tcctaagtcc attagtgagt ttgcttcatg tatttgtcat 232740
acatttaaat tcttttgtaa tattgtgcat tccatcccag tttcccctga catcctaaat 232800
taacttttta agtttggata cattgtggtc tattctttgt tctgtaaagc tttatggatt 232860
ttgacaagta tttaatgtat tgtatcacca ttatagtaat atagttccta tggaatagaa 232920
tagtttctat cgctgtagca tagaatagtt tctctactct ataaaatatc ctgtgtttct 232980
ctaattcaac ccctccttcc caccttgaac tcctgacaac ccctggtctg tttaatatct 233040
ttcttttgtc tcttctagaa tatcatataa ttgaaatcat acaatatgta gctttttcag 233100
actggctact ttcacttagc aatattcatg taagtttcat ctatatattt tcatggtctg 233160
atagctcatt tctttttaat cactgaataa tacttttatt tatccactca ctggtgaaga 233220
atctcttgat tgcttctaaa ttcatggcaa ttatgaatga aactgctcta aacatttttg 233280
tgcaggtttt tgtgtgcatg tgtattttca aattagttgg gtaaatatct aggaattcaa 233340
tttctgcatc attgtggtaa taatgtgttt agcttcataa gaaattgcca acctatcttc 233400
caaaatagct gtaccatttt gcattcccac cagcaatgaa tgagagttct tgatgctcga 233460
catccttgtc agcatttgat ttttgtcagt gttttggatt ttaactattg taatagatgt 233520
gtagtagtgt ttgattgttt taatttgcaa tttctttttc ttttttgaga tggagtctcg 233580
ctctgtcgcc caggctggag tgcagtggca cgatcttcgc tcattgcaac ctctgcctcc 233640
tgggttcaag caattctctg cctcaagctc ccaagtacct gggattacag gcgcctgcca 233700

ccatacccgg ctaattgttg tatttttagt agagatgggg tttcaccata ttggccaggc 233760
tggtcttgaa ctcctgacct tgtgatccac ccgccctggc ctcccaaagt gctgggatta 233820
cagacataag ccacggcgtc cggcctgcaa tttcttaatg acaaaaaata ttgaggatat 233880
tttcacatac tttttttgcc aactgtattt tttttaatta atttttattt ttattttatt 233940
ttgtaacttt tattttagat ttggggtaca tatgtacatt tgttaataca ggcaaatttg 234000
tgtcacaggg gtttggtgta cagatcatct cgtcacccag gtactaagca tagttcttga 234060
tagttctttt tcctgatcct ctccccactc ccactctgtt ccctcacgta ggccccagtg 234120
tctcttgttc ccctctttat gcccattggt tctcattatt tatctctcac ttaaaagtga 234180
gaacatgcag tatttggttt tccactcctg cattagtttg ctaaggataa tgtcctccag 234240
ctccatcctt gttcctgtac aggacatgct ctcgtgtttt ttttctttct ttttatttta 234300
atggctgaat agtattccac ggtgtctatg tactacattg ttttttttta aaccctgcat 234360
accattgatg ggcatttagg ttgattccat gtttttgcta ttgtgaatgg tgttgcaatg 234420
aacctacatg tgcatgtgtc tttatggtag aacaatttat attccactgg gcatataccc 234480
aggaatggga ttgctgggtt gaatggtaat tctcctttta ggtctttgag ggatttccac 234540
actgctttcc acaatgggtg aactaattta cactcccacc agcagtgtat aagtcttccc 234600
ttttctccat aacctcccca gcatctggtt ttttttgttt gtttgtttgt ttttttagta 234660
tttaataata gccattctga ctggtgtgag atgatatctc atcatggctt taatttacat 234720
ttctctaatg attagtgata tgtagcattt tttcatttgt tgccaactgt atgtattttt 234780
caatgaggtg tacatcagat cttttgccca ttttaaaagt ggggttttgg gctgggcgca 234840
gtggctcacg cctgtaatcc cagcactttg ggaagctgag gcaggcagat cacctgaggt 234900
caggagttcg agaccattct ggccaacatg gtgaaacccct gtctctacta aaaatacaaa 234960
aattagtcgg acatggtgtc gggcacttgt aatcccagct acttgggagg ctgaggcagg 235020
agaatcactg gaacccagga ggtggaggtt gcagtcagct gagactgaac cattgcactc 235080
cagtctgggc aacaagaatg aaactccatc tcaaaataca tacatacata catacgtaca 235140
tacataaaat tgggtttttg ttttcttttt gttgagtttg aggagttttt ttgtatattt 235200
tgattacaag tcttttatca gccatgtgtt tcacaaataa tttctcccag tttgtggctt 235260
atcttttcac tctcttaatt gtttttttca aagtagaaat ttaaatttta atgaagccca 235320
atttattaat ttttttcttc catattgtgc tattggtgtt gtatataaaa acttactacc 235380
aaattcaata tcatatagaa ttttttctgt tttcttcaag aagtagtttt ataattttgc 235440
attatatgtt tagatcaatg attcacctta agttttgttt aaggtgtaag gtttgtgtat 235500
aagtttttct ttttccacat caatgtccag ttgcttcagc aacattttct ttttatatat 235560
atcttaaggg taatcagcgc aatattttct gaaaagatga cctttttctc atttaattgg 235620

ctcttctttg tcaaagatca gttgacctta tttgtgtgga tctatttctg gacttcttac 235680

tctgtttcac tagtccatct gtttatactt taaccagtac catactgcct ttattactgt 235740

agcctttatg gtaagtcttg aaatgaaata gtgcaagtgc tccaccttct tcagaatttt 235800

gttcttcttc agtattaaag gctattctag gtcttttgcc cttctttaaa catgttggaa 235860

tcaattgtca atatctacaa aatagattat tgggatttgg atttagatta ctctgaatct 235920

gttaattaag ttgggaagaa ttgacatttt atcaatattg aataatatga acatgtaata 235980

atattgaact ttcaatctct attatctctt catcatttta agatcttctt tcatttttca 236040

ttgttttata gatttttaca tataggcctt gtacatactt tgttaattta tatcttagta 236100

ttgaatgtac tattataaat ggtattttct aaactttgaa ttcctgttat tcatgatgat 236160

atgtaggaaa gaaattgact tttgtatatt gacattagat cctttaaccg tggcatcatt 236220

acttattagt tccaggggag attttgttgt tgttgttgat tcattggaat tttctgcata 236280

gataatcatg ccatctgtga ataaagatgt tttatttctt ccttcccaat ctatatatct 236340

tttatttctt tttttgcctt attgcacttg ctggtatttc tagcataatg tataatagga 236400

ggaatgagat aagatatctt agaattatcc tcatcttcag gggaaagtgg ttagtttttt 236460

gtcattaaga ataatgttag ctattgtttt tttaaatttc ctatatgaaa ttgaggaaat 236520

ttctgtctat tctgaatttg ctgagttttt aatcataaat agctgttgaa ttttgtcaaa 236580

tagttttcct gtgtcaatta atatgatcat atgacttttc ccgttttcac tgttaatgtg 236640

gcagattata ttgatttatt ttcaaatgtt gaatttgcca tcagacatgg aataaatccc 236700

atttgttcat gatgtataat ttattttatg catcgtttgt tctgtcttgc taacattttg 236760

ttgagatttt gtgccagtgc tcaggagaga tattggtctc tagttttact ttcttataat 236820

atctttatct gatttgggta ttaggataat tctagactca gaatgagtta ggatgtgttt 236880

tctctgcctg tttactaaca cagattgtag agaattggca caatttcttt cttgaagatt 236940

tgttagaagt aatcttgcca ccacctgagc cagatgattt ctttagaagg taattagtta 237000

ttgaatcaat atatttaata tatatagaga tatttaggct atttatttct ccatgtgtga 237060

gttttggtag tttgtgtatt tcaaggaatt ggtccatttc atccaaatta tcaaattcgt 237120

gagcatagag ttgttcataa tattccttta ttatcctttt aatctccaag agaccagtcg 237180

tggtgacttc tctttcattt atgatattgg taatttatgt tttctgtctc tctttttttt 237240

ttgccagagt ctaactctgc cacccaggct ggagtgcaat ggtgtgatct ctgctcactg 237300

caacctctgc ctcttgggtt caagtgattc tcatgtgtca gcctcccgag tagctggtat 237360

tacaggcatg ctccaataca cttggctaat tttttttttt gtatttttag tagagatgaa 237420

gttttaccat gctggccagg ttggtcttga actcctggcc tcaagtgctc tgcctgcctc 237480

ggcctcccaa agtgctagga ttacaggcgt gagccaccgt gcccggcttc tttttcttaa 237540
ttagcctgaa tagaagttta tcaattttat tgctctttta aaataaccag tttttgtttc 237600
actgagtttc tttatcatgt ttctgttttc aattttattg gcatctgctc taatttcaga 237660
tgctccttga cttatgatgg ggttgtgtcc cagtacatcc actgtaattt gaaaatatca 237720
taagtctttt gacttatgta atgcatctaa cctaccaaac attatcgctt agcctaacct 237780
cccttaaatg tgctcagaac acatacatta gcctacagtt gagcaaaatg atctggcaac 237840
aaaacacact atagagtatt gatggtttac cccgatgatc acatagctga ctgagagctg 237900
cggcttgctg ctgctgccca gcattaagtg agagtattgt tccatatatt gctagcacag 237960
aagatctaaa ttgaaaattc aaaatacagt ttctactgaa tgcatgcata ttacttttgc 238020
accattgtga agtcaaaaaa aataataaat caaaccatct taagttggga actgtctata 238080
ttattctttc tcttctgctt gctttaagct tatctagttt ttttcttctc tagtttcctt 238140
aggtggtggc ttaggttggt tattgatatt atatattttt cttatctaat atatttactt 238200
aatgctatga atttttctct aagcactgct tttcctgcat cccacaaatt ttgatgttct 238260
atttttatct tcatttagct caaaatagtt taccatttat tttgaggctt tttctttgac 238320
tcatacgttc tttaaaagtg tgttgttcaa tctctaaata tttcgatatt ttccagctat 238380
ctttctgttg atttctaatt tatttccaat ttggtgtgag agcctacttt gtacactttc 238440
tgttctttta aatttgttaa gggtgttttg tgacccagaa tgtggtctat cttggtgcgt 238500
attccatcag aacttgagaa gaatgtgtat taagttgtgg tttgatggag tattctataa 238560
atattaatta gaccatgttg attcatcata ccgtttaggt caactatatc tgtattaatt 238620
ttctgcctgc ttgcactagc aattactgac agcggaatgg tgaggtttct aagtataata 238680
atggtttggg cttgtctatt tccccttta gtttcaatca ttttgcttca tgtgttttga 238740
ttcacttttg ttaggtatac acatatatac acatatttat gattgttgta tcatcttgaa 238800
caactgaccc ctttatcatc atctttatcc ttggtacttt tccttctttg gtagtctgct 238860
ttgcatgaaa ttaatatagc cactccagct ttatttttgt gaatgttagc atggtgtatc 238920
tttctccatt cctttacttt taacatatca gagttattac atttaaagtg ggcattatta 238980
ctaggataaa taccaaataa agtattttgc atgctgggct taaaacctag atgacaggtt 239040
aataggtgca gcaaaccacc atggcacatg tatacttatg gaacaaaact gcacattctg 239100
cacatgtatc ccagaattta aagtaaaata aaaaataaat aaataaaaat taaaaagtaa 239160
aaataaatta gctgggtgtg gaggcagcca gctactcagg gggctgaggc aggagaatca 239220
aaataaataa atcaataagt ggggcttttt gtagacaaca tatagtttgg tctttcttaa 239280
aattcaatct gacaatttcc atcttttaac tggtatattt aaacaattta tatttaaagc 239340
aagtgttgat atatttgaat aaaaataaac ctgtgtttat taactatttc atatttgttg 239400

ccaaacgctt ctttttggtc tttcgtagtt ttatttgagc aatgtatgtc ataccatttt 239460
atcttttctc ttagagtatt aactatactt cttttttttt tttttgagac agagttttcc 239520
tcttgttgcc caggcgggag tgcaatggtg cgatcttggc ttaccgcaac ctctgcctcc 239580
tgggttcaag cgattctcct gcctcagtct cccgaatagc tggaattaca ggcatgtgcc 239640
accatgcccg gctaattttg tatttttagt agagacggag tttctccatg ttggtcaggc 239700
tggggtctta aactcctgac ctcaggtgat ccaccggcct ctgcctccca aagtgctggg 239760
tttacaggag tgagccaccg cacccggctt aactatactt ctttaaagaa tttttgtagt 239820
ggtgccgcta aagttcacag tatacatttt taagtattct aaatacacct tcaaataaca 239880
ctattccttt ttacatgaaa tatagggata ttataacata gtattctcaa ttcctcctta 239940
ctgtcccttg tgacatagct gtcatttatt tcatttcact tacctatata ctataatcac 240000
ctatacattg ctgctattat gattttaaac aggcagttat tgtttacgtc aattaagaat 240060
ttagaaagaa tttagaatcc tgtgtaaaat aaatttcctt ttattttacc ttaattcatg 240120
cattctctga ttatcttcca tgctttatgt agatccaagt ttctgactta tatcaccttc 240180
ctcttgcttg aagaacatct tttaacatat tctgcagggc aagtcagctg gtgatgaatt 240240
ctctgaattt ttgtctgatt ttttttaat atttccttca cttttgaagg ataatttccc 240300
tgcatctaga attctaaatt ggtcactttt ttcaacattt tatatatttt acttcacttt 240360
ctttattaat gtacggtttc tgaagagaaa tctgctgtat ttcatcctgt tctctatggt 240420
taggtgcttc cctgccctgg ctctggcttt ttcaagattt tctccctgtc tttggttttt 240480
ctacagtttg aatacaatat gcctaggtgt tgtttgtttt gttttttgt aaggagtggc 240540
atgtatttat cttcttgata ctccctgagc ttcctggatc tgtggtttgg tgtctgtcat 240600
taattttgaa aagttctcag ccattactac ctcaaatatt tcttcttcgc ctttcttttt 240660
ttttctttct ggtattccaa ttatgcatat gcttgttata ccttttgctt tttcattctt 240720
ttgattcttt acatttcagt tggggacgtt tctgttgact tatctttcag ctcactgatt 240780
atttccttgg ccgtgttgaa tcaattgatt agtcctcaaa gacatttttc atttctgtta 240840
caccattttt acatttctag catttgcttt tgattctttc ttaaaatttc tatctttctg 240900
cttatattac ccatctttta ttgtatgttg tcttcttttt ccattagagc cctcaaactc 240960
ttttttttta ttatacttta agttttaggg tacatgtgca cattgtgcag gttagttaca 241020
tatgtataca tgtgccatgc ttgtgcgctg cacccactaa ctcgtcatct agcattaggt 241080
atatctccca atgctatccc tccccccctcc ccccacccca caacactccc cagagtgtga 241140
tattcccctt cctgtgtcca tgtgatctca ttgttcagtt cccacctatg agtgagaata 241200
tgcggtgttt ggttttttgt tcttgcgata gtttactgag aatgatgatt tccaatttca 241260

tccatgtccc tacaaaggac atgaactcat catttttttat ggctgcatag tattccgtgg 241320
tgtatatgtg ccacattttc ttaatccagt ctatcattgt tggacatttg ggttggttcc 241380
aagtctttgc tattgtgaat aatgccgcaa taaacatacg tgtgcatgtg tctttatagc 241440
agcatgattt atagtcattt gggcatatac ccagtaatgg gatagctggg tcaaatggta 241500
tttctagttc tagatccctg aggaatcgcc acactgactt ccacaatggt tgaactagtt 241560
tacagtccca ccaacagtgt aaaagtcttc ctatttctcc acatcctctc cagcacctgt 241620
tgtttcctga ctttttaatg attgccattc taactggtgt gagatggtat ctcattgtgg 241680
ttttgatttg catttctctg atggctagtg atgatgagca ttttttcatg tgtctgttgg 241740
ctgcataaat gtcttctttt gagaagtgtc tgttcatgtc ctttgcccac tttttgatgg 241800
ggttgtttgt ttttttcttg taaatttgtt tgagttcatt gtagattctg gatattagcc 241860
ctttgtcaga tgagtaggtt gcgaaaattt tctcccattt tgtaggttgc ctgttcactc 241920
tgatggtagt ttcttttgct gtgcagaagc tctttagttt aattagatcc catttgtcaa 241980
ttttggcttt tgttgccatt gcttttggtg ttttggacat gaagtccttg cccatgccta 242040
tgtcctgaat ggtaatgcct aggttttctt ctagggtttt tatggtttta ggtctaacgt 242100
ttaaatcttt aatccatctt gaattgattt ttgtataagg tgtaaggaag ggatccagtt 242160
tcagctttct acatatggct agccagtttt cccagcacca tttattaaat agggaatcct 242220
ttccctgttg cttgtttttc tcaggtttgt caaagatcag atagttgtag gtatgcggcg 242280
ttatttctga gggctctgtt ctgttccatt gatctatatc tctgttttgg taccagtacc 242340
atgctgtttt ggttactgta gccttgtagt atagtttgaa gtcaggtagt gtgatgcctc 242400
cagctttgtt cttttggctt aggattgact tggcgatgtg ggctcttttt tggttccata 242460
tgaactttaa agtagttttt tccaattctg tgaagaaaga tattggtagc ttgatgggga 242520
tggcattgaa tctgtaaatt accttgggca gtatggccat tttcacgata ttgattcttc 242580
ctacccatga gcatggaatg ttcttccgtt tgtttgtatc ctcttttatt tcattgagca 242640
gtggtttgta gttctccttg aagaggtcct tcacatccct tgtaagttgt attcctaggt 242700
attttattct ctttgaagca attgtgaatg ggagttcact catgatttgg ctctctgttt 242760
gtctgttgtt ggtgtataag aatgcttgtg atttttgtac attgattttg tatcctgaga 242820
ctttgctgaa gttgcttatc agctgaagga gattttgggc tgagacaatg ggttttcta 242880
gatatacaat catgtcgtct gcaaacaggg acaatttgac ttcctctttt cctaattgaa 242940
taccctttat ttccttctcc tgcctaattg ccctggccag aacttccaac actatgttga 243000
ataggagcgg tgagagaggg catccctgtc ttgtgccagt tttcaaaggg aatgcttcca 243060
gtttttgccc attcagtatg atattggctg tgggtttgtc atagatagct cttattattt 243120
tgacatacgt cccatcaata cctaatttat tgggagtttt tagcatgaag agttgttgaa 243180

ttttgtcaaa ggctttttct gcatctattg agataatcat gtggtttttg tctttgtaga 243240
gccctcaaat tcctaatcac aattgtttat ctctttcctg acattccaca tccaatccat 243300
cagcatgtct tattggcttt actttcaaaa taaattaaac tcagccactt ctcagcattt 243360
ttaaaatcac cctaatacaa accccctgct acctcatcaa ctgcaatggc ttcctaactt 243420
attttgtaac ttttgatctt tgagttcttc caagagccaa gagttcttcc aaagctataa 243480
accctatcat tggcactcct ctgctctatg gaaagcagtg gcttctcatc tctttcagag 243540
tataatgcaa agctctcacc ttagctggca tggccctgtg ggattggcct cccttgtctt 243600
actttttcct tgttcaggct gctgtgacct ctctggtctt tggctcttac tagaaacctt 243660
tgaacccgtt tccttcccag tgtcagtatt tgtgtgttgt tcgttcaccc ttctcacttc 243720
attctggttt ctacagcaca gagaagtagt agtccctgat ctaaacaccc tctccacctg 243780
ctttccactt tgtttttcc cctagcactt accattatca gatatcatat atttatttgt 243840
ttattgtcta acttcctcac aaaaatatga cgttgtgagg atagggattt ggctttttgc 243900
cccagagcag tgcctgactc tcaataactt tgttgtatta agtgaatgaa taaaataaaa 243960
ttaaaattga ttcaaagtgt atgagcatgt gtacatttta cataagtgat acatgacatt 244020
cttccattcc ttgggggctc ttttaaccat tctcaaccag ttgtagtacc tttaaaaaat 244080
catgatgaag atgattttga gagctaattt tggtaggaga cagcagtttt agcctgtccg 244140
ctccatgtgc agaataatag cctattttat tacatctgat attcaagcac tagaatctat 244200
caataggtaa ataatttcca aaaataaaag catcagtggg aaaacaggga aattattttt 244260
aaaaaagaat ttctagacca ggatgtgtgc aatttgtaat ctctaataag aaacgttata 244320
gctgataatt ccagcaatta cagaatcatg atcatatcca taatggatca gtagaggctc 244380
tggcttatat aatagcttgc cctctcagga ttctagagcc ccatatgtaa acacagaaca 244440
catttatatt gattgacagt gcatgtaggt ttctacagat tgtgccagtc agtgttttct 244500
aaggcagtta tgccatggta attaaaatta tgggctctga aatcagcctg cctgggttca 244560
aaccccagct tcatgtgtca gcttccttgg ctgtaataag gaatactaat agcacctgcc 244620
ttgtggcttt gaaaattaca tgatgtaact ttgtcaagag cttagatgag cacttgggat 244680
atggcgagtg ctcaataaat gttagtttac catcatcatc atcattatta ttattactga 244740
ttgcaagcaa gacaaactgg ttctcacttc agcagaaaaa gaaattactg aagtaatttt 244800
ggttagatca cagatttaac gtgaaggatg aataaccaga cttggaaaac aggtggaaac 244860
caagaggcag tcagcatggc atagcagcca gcacttcacc agtgtggtgc cttgggggca 244920
gcctggcagg agccactgcc ttcactcctg gactgcagat ctaccacagg acagcagact 244980
gaattgtcct atgtccagac ttcacagtca cagggagcag gctgcatgca ggtggggccc 245040

aggtcaccta ccttcaccct agagtctgga gccacagaaa acagtaattg tccttgtagc 245100
ttttgtgatg gaaagcaagt cctgacaccc accaactcac atactaggga attcaccaaa 245160
tgtaggacgg cagctaagat gctgggaaac caagaattaa caaatgagta ttacaccaat 245220
tagttaattc attacagatt tcaatatttt ccaaaaaaca tcctacaaag aacagctcac 245280
tttaatatac tccaacaaat gatgaaatct ctccttggtc atcagctttt ctaaatttct 245340
gtaactttag gtgattctga atttcttagt gattctgaac ttctagaaga ttctgaaaca 245400
gaacagtctt actttgggat gtattcaaat cctagagatc agtcacatca atctgtgccc 245460
tttttcatta tgtaacatga aacaggagga cctttccaac ttgccttggg aacctgctcc 245520
tacaacaggg tatattatca catttaataa agaaagagta atatttgcat tgtgaccctt 245580
catctgaggt catcgggact gtgctgtcct cccaagctcc actgtaaagg gatagacaac 245640
aaccttgtct cccagtcatt gtaatctgta tcagagagca tcattagatg aatgaagatg 245700
gaaatgttct cacggtcagc aggtggggaa ggcaagactt gctgattgga agcaatgaac 245760
catagttcat tctaaggctt ggggagagca actttgagga aatgaggccc ctaaacttgc 245820
catgtaggag aagttggggg gtggtaaatt gggcaccctt tatctactaa acgtaatctt 245880
tacttcccca ccctctaatt tttctcagtt gggcttgaaa ttgtttttgc ttgttacact 245940
ttatgggaaa agaaagggaa atcctaataa ggtccatgcc agcaaaactc tagaagaaga 246000
aactaagaat ttcaaattga cagtttgtta atgagaaaag acaaggttaa aggattttgt 246060
taaacagatg accaaaataa ttattggaaa cttctgcttc tggccaaggt ggaataataa 246120
gggctacatt taccttccca ccagaaaata taataacaaa caaaaactgg acaaaacact 246180
ggacatgaca ctgttgtctt taaaccttgg acatcaatca gtgcaggatg ttattttgta 246240
agaaagggaa aaaaattggg gtgagccttc tgattgtttc cagactggag agaagtttca 246300
ggccacagca agtggggagg aacctaggtg taagtatatg tataaggtag cacatcatag 246360
tggccaaatg aatggcacag gaaagaaaaa ctacaagaac agaggaggta gggccacaaa 246420
actagagtgg ggtccaagat tcactatgga taaaatgggg tgttaatttg gttttcaagg 246480
ccaatacaag aggaggcata gaaaaagaac aaggtggtgt tgggaagtag tataaaaagg 246540
ggcacaggct tatccttgtt ttataaaaaa caaaggtttt tggaagggat ttgtagagat 246600
aagtttgaaa agtagtttga gactatgtgt ggaaattcat gaatcttaga gtttgatttt 246660
attcaggaga tcagtagctt ttaaaatgta aaataaaacc agtaaactct atctaaaata 246720
aattcttccc ctaatcccaa tataaaaaat acatataaaa gcgtagctgc tctagacaaa 246780
atggcgggta tgagatgacc agaaacctca acagcactac ctctggtagc cccatagaac 246840
tgccaagagt tttcagagta tagttaattc ctgctgacgg ctaaggcagt cattgagtga 246900
ttggaaagaa gacggaagat agacaagatc agttaattgg gttggaggga agactgaaga 246960

taggcaaggt gacaaattag gaactgttgc aatgatctag gcaagaagga acctatgaac 247020

taggagggag gcttaaagac agcaaggagg gaactatagg cagggacatg tgaggaaatg 247080

cttagtagtt aaggtgagtt tgtaggtatg aaaggagaga aggaaagaac attaagtagc 247140

attaagtgaa ccagataatt tatagctcct tgacctgaaa catttagagg ctgtgataac 247200

actaataata ataatctata attacataaa ttacgtagtg taatgatata tcatgatata 247260

atcatcttac caatgcttta cagtcaatga cattgtggta cagagacttt ggaggcagat 247320

agacctcgat tgtaacctct gtgctgcccg tggtatgacc ttgagcatgg tagttaatct 247380

gaacttcttt cttttctgtt aaattgggga caatgacagt acttaacctt atagtacttg 247440

aagggagagt gttagaattg atactgggga gtctctccac agaaagttgt attagttatt 247500

gtctttattt cattcttaat gaattagcac tcccgttttc tttaacatgt tgaatttaaa 247560

acctcttagt gtatttcttt gtcttgcctt ttaatacccc atgaattatt gagaaacaaa 247620

agaaagtgat tatgcaaatg tttgaaatat ctaataatac atgaacgtag tcatgggaaa 247680

ctggagaaat aactttactg atattatact agttttttt ctggaagcat agcatattaa 247740

gaaaactcat ttcaatgaaa gaaaatttaa aaattagagt gcattagaag cataatccaa 247800

tgaattctat tcctaatgaa tactcaggca gtatgttaac tttttctgag atacaatagc 247860

caagccaaag aatttaaaga atgaaaaaaa cagatgatta aacaaactgt gaagtaatta 247920

gaggtagtct ttgaaaatgc ctcattaggc atttgactca ttaggacaga tccttttatt 247980

ttagggccag gacataatta tttaagcagt tgatgtgtct ttagctcctt tcaccttgcc 248040

acaagttgtc gcctgtactg cctttccacc tagcttccaa gtccaggccg actttgaaga 248100

gattccttag ggctcacctc ccctggagag tgccctctgt accatctctc tccctttcct 248160

cctcattcct attgcctgag tttattgttt taataaattc ccataagtta acacctccta 248220

gggtggatta gaatcattca aatccacatt gattgttact aagtagaaat tttatactga 248280

gcctttctaa atccttacaa caacgtgacg aggatattat agttgcttta acccaaggag 248340

atgaaattca aactggtggc attgcacact tacagtgggc ttgcaggatg aaagagagct 248400

tggtatctcg atcccttata caaaacaggt gggtcttgtt agaaaaattc ttcaataatt 248460

gttaaggtta aaattttgaa aagtggttca aagaaatttg ctttgatgca aatattgtct 248520

agtcagttac ataacttgag ctataatgac agtgtacact agctatccag ggcacgacat 248580

ctctattgtg ctgttgaata tactgctcaa ccattctgga aaatggcatg ttcatgtatg 248640

aatgacaatg tatcttgtgt gtatggcatt ccaagccaac atgttggtcc ctgcaagtaa 248700

acttcttgac tgtcaagagg ctgcctgttt aatatttgca tgatctaaac taattgtttc 248760

ctttgttttt ttcctctgct ttatgagctg gtagtttgac cttttggctt ttcccctgag 248820

agttaacaaa agttagacag ttggggggtg aacttaagta aaatccatat cactctatgt 248880
tgctgctcta ctaacaattc aagaaaatgt cttggtagaa agcaaggaat gagttttaaa 248940
ttttctcttt gaatttcaat tatccatgcc actcctgtgg tccaccattc tatataatta 249000
agaataagct gtatgttcca tagtgacaca ggctgttatt tctgatttgc agttttcact 249060
taacctaggg taaaattgaa atagaagtcg catccttttt tttttacctt cttccactca 249120
attctagtta gatattttg aacttctata aaatgtctat gcaatattat caccaataat 249180
gtgcaaaatt actactccca tgtagaagag gatccattct tcattgaacc ataccettgg 249240
gatcctatca catgcagttg gccatataat attttatatt attgtttttt tgttttgttt 249300
gttaagtttg tacctacata atggattaaa ttcaaaatct ctgtttaact gaaacaaaat 249360
ctgtttaact gaagcaaaaa aacaaaatct ctgttttttg tttgtacaaa acaaaaaaca 249420
ataatatttt tgttttgttt gtatacttca tagggacttt ggaatttgga aaatatcgtt 249480
catttagggt atatatatta gtccattttc atactgctat aaagaaatac ttgagactgg 249540
gtaatttata aagaaaaata ggcttaatga actcacagtt ccacatggct ggggaggcct 249600
cacagtcatg gcagaaggtg aaggggaagc aaaggcatgt cttatatggc agcaggcaag 249660
agagtatatg caagggaact gccctgtatg aaaccatcag atctcatgag acttatttac 249720
tatcatgaga acagcacagg aaaaacctgc ccccatgatt cagttacctc ccactgagtc 249780
cctcccacat gtggggatta tgggaactac aattcaagat gagatttgga tggggacaca 249840
gccaaaccat atcagtacat taaagaaaat atttgttagc gctaagtgaa tctactttca 249900
cctacttgat tatttgatga ttttataaat agtcatctct ttcctcatct ttccagtcgt 249960
attttgttgt tccaaagcca aaacctgtgt gtcttgtatt caacaacctg aatcatacag 250020
ttgtgagacg taaatgggtg tggtggttcc acttacttct ttacatagta ctacaactaa 250080
gccatctaag tagtctgctc ctaaattcct ccaagaaggg aaatatcatg atttgattta 250140
aatgtgacat cctttcttag agaagttttc tttgtctcct tcttccccag tcacttcctc 250200
ccatccactg tgctttcggc agttcattcg ttcattaatt cattcaacga atattgaata 250260
acttttgatc aatgtcttcc tccctccaag ctgaaaactt gatggagggt aacatgatct 250320
gtctttgttt accactgcat cttccgtgaa tatacattgt aagtattgaa cggattttta 250380
ctaaatgaac aaatactttg ataaaatatt tttgattact gccaatatca gtttctgaat 250440
tgattctaaa attcttctgc tggaaagtag attagcgtta gtatgactgc tgaagccttt 250500
taaagtgggt ggtaatacta cgtttcgttt tgtttcatta acctaagtgc tgttctttgg 250560
aatcttttta aagtaagata aattacattc atgaaagaag catattattt ttaaagtact 250620
ttattttgga aaggtaaaat gcttgtgtag ttataatttg gttactcttg atttcacctt 250680
aggaaaaaca atatcacctt ctaaccattt cttttttagt caaatctctt gcttctattt 250740

ctctctgtag atccgctatt aaagactgta atcactgctg catctttcct gtaaggcttg 250800
atcgcattgt taatttcttt ctaaacttgt agagtaggtg tataaatcgt atttgggtaa 250860
tacactgact aatactgaag aaccaggcat tttcttaccc cagtcctcac acgaagtagg 250920
gaaatacaag tcagaactta tcttctcaaa actctgacct cagaatttcc tgagaaatct 250980
gaacctaaaa agattctttg cttttgacat tttttctctg gtgtccaccg caaggctctt 251040
gctcttacat tttttttttt tttctaatgt ttcaaataga agatggtaga gtcatatagt 251100
acaagctcag catcggaggg gctactggag gtcaactagt gcaaatgctt tctgaataat 251160
ggaatccttt gggaataccc ccttgaaagt cttcatctag cctcttcttg gaaaccctca 251220
gtgatacccc tcatcgtctc ctatgatggc agcttctatc tttgtcgtca gctctgacta 251280
ttacaaactg cttccttaca ttgagctgta actaactaaa aagtttccac accctgcttc 251340
tattttagcc tttgggctca taaagaacaa gttgaacccg tcttatgcag aacaacccat 251400
ctacgggaga tgctcaggtt tcctccatgt tttcctttct ctaggctaac ccttcgttgt 251460
tcctcactta ctcactatat aacctgattt catgtctcct tacccttctt gttactctaa 251520
cttgagtagg ctagtttcag gatccagcaa taagtgtgat acatcaggta tgctctgatg 251580
agttgagagt agagtggaca tagcatctcc cttaattcag atattgtgat ctaattggca 251640
aatctggcaa taaaagttga aatgcctgat ccaggacaat ggctggtcag gtgccattgt 251700
tctcatcttt tacttttagg tgtccctcaa tttgttaagt tagtacctac gtaatgtcct 251760
gaaacttgtt aagtttgtac ctacataatg gattaaattc aaaatctctg tttaactgaa 251820
aacaaaagca aacttctttt cagagccaga atctggaatc atacgtaaca gagaatgata 251880
ttgtacaagt tgcttcatct ctttaagtaa cggtttctca aacttacaag attattgtga 251940
gaactaaatt agttctaaag tgcttccatg taaagtgtat gtaaatgctt aaaatatata 252000
gtaagtgctg aatgcatatt agaaataata ataatcttta ttataatttt tactatttca 252060
tgagaagtac ttattttaat actgagcaaa taggaagagt tcatggcttc tctatgtttt 252120
tgaagtgtca tttaatatat tatatatata aaacatatat atatttcagt cacacatttg 252180
tccaaatacc ttgacaaatt aaaacaaaat aagacaaaat tctcacgcta gttatttgtt 252240
ataaagtaat agaacaagtg atatgttata aagagcattt atttctcatg tctttgatat 252300
taaaaatagt tgtattaact ttttatcaaa acgattgctt ccttcatata aatctaagaa 252360
ttatgctgtc tgataaatat tggagagatt aacttctttg aaaatataga agctttttgt 252420
ctttttaaaa tagttgtttt tttgcagatg ttaatacatt tcagcagtac agtatggcct 252480
ttttcaggtt aaggtgctga gcccaaacct caaagaatca ctgcaaaaag attggatccc 252540
ccctcttcac cccatttcgt aatttagtta gtgagaacca caactggcta aacctttgtg 252600

gggggccggg cactgtggct catgcctata atcccagcac tttgggaggc cgaggcaggc 252660
agatcacaag gtcaggaaat cgagaccatc ctggctaaca cggcgaaacc ccgtctctac 252720
taaaaataca aaaaattagc cgggcatggt ggcaggtgcc tgtagtccca gctactcagg 252780
aggctgaggc aggagaatgg cgtgaacctg ggaggcgggg cttgcagtgg gccgagatcc 252840
cgccactgca ctccagcctg ggtgacacag cgagactcca tcttaaaaaa aaaacaaaaa 252900
aaaacaaaaa caaaaaaaaa accacctttg gggggaaatt atcaaataaa acaaactctt 252960
tttagaattt tacaactttt atgttaggaa aaaacaaata catttgtgaa aagcttaaaa 253020
tccagtaaat gacttgaggg acttggggca atcctagggt gatgaggagc aggttagtaa 253080
cagtgaagga cttagcaccc caggggccca gaggctgtaa tataccttat gagcaagtca 253140
ttcttattta gtcttgccca ttaagaagtc tacttggact aaatgctttt aaaaatgccc 253200
ttttaattta ctattaaaag aatattccta gcagaagtag tcttggatgc taaattctat 253260
tttaagaata actaaaatta gaattctgtt ctttttataa cacctgttac acacacaccc 253320
ctacctagtg tgtcggaatc agtttgtatg ggctcaccaa agcctactgt tcaattttca 253380
ggagttttgt aagccatttg atgtcagaca agtggcctga agtttgttat ggtggtggta 253440
tttacaccat gaaaattggc atgttatggt ggtagtattt acaccatgaa aactgctaca 253500
aatagaaatc tttttcttct tctcttggag agccacttgt tgaacactta ccagctcacc 253560
tgtgcttgaa agtatttctt caaataaaat gaaagctggt tagctttgaa aattttttgt 253620
ataaaagttt acacgggaaa aaaataaact aatttttttt tccacctgtg ttttcaggga 253680
tacgaaaaga ccgaagagga gggagaatgt tgaaacacaa gcgccagaga gatgatgggg 253740
agggcagggg tgaagtgggg tctgctggag acatgagagc tgccaacctt tggccaagcc 253800
cgctcatgat caaacgctct aagaagaaca gcctggcctt gtccctgacg gccgaccaga 253860
tggtcagtgc cttgttggat gctgagcccc cgatactcta ttccgagtat gatcctacca 253920
gacccttcag tgaagcttcg atgatgggct tactgaccaa cctggcagac agggagctgg 253980
ttcacatgat caactgggcg aagagggtgc caggtaagaa tgcgaagcgc agcttttaag 254040
agtcaatagc ttttcaagaa cttgttgtga tgtcatggga gaaatagtgg gggaaaaaga 254100
agcaataaca tgttatgtaa ttggtttcaa ggttacagga gatgtgttca ttttcagtat 254160
caatacactg taattttcca ggagattagg aaataatatt tttaaatcag aatctagaag 254220
actgaaattc ttaaattgac ataatttatt tttaacccat ctcatttacc aaaaagattt 254280
agggtggaca ctacatggta aaactattta atagtgtatg ttcacagtag cagaaacttt 254340
taacactaaa tgaactacaa aagtttgtaa tattaatgac ctttgttgaa aacatctcaa 254400
ttattaatca aacgatttta tcttaaaaag atttttaaga ttcggtgtgg tggctcgtgc 254460
ctgtaatcct agcacttttt ggggctgagg tgggaggatt gcttgagccc aggagcttga 254520

ggccatccgg ggcaacgtgg cgaaaccctg tctctacaac aaatttttaa aaattagctg 254580
gatgcagtgg cacacacctg tggtcccagt tatgggggag gccgaggtga gaggatggct 254640
tgagtccagg aggtcaaagc tacagtgaac catgtttgtg tggagtgcca ctgcactcca 254700
gcccaggtga cagagcaaga ccgtgtcata aaaaataaac cacacaaaaa aagagaaaga 254760
tctttatgga ttaaaaagat aataataagt gtatttactg aatgccaatt atttatccaa 254820
cctggtgtat gcttagtgtt ttaggagaaa gagaaaggca atggaaaaat aaattaaggt 254880
atcatccctg aaagaaactt ttagaaagac acagtggctg aagtgatacc ttgttccttc 254940
agttgattct ctcagaactg gtgttctggt aaaattggac tgttactcct gttattcagg 255000
gagaagaact caagtttgta tggcaacaag actagaaaat gactttctcc ctgccccagt 255060
gtattcgttc aggagctaat gtagataaac cgaggcaaga agaagctaaa ttttttttct 255120
gggcttatag gttaaatgag tgatagattt agttggaggt tttctcattt ggtttctttt 255180
aatagatgaa attaattgtt tcctatgaag catgaaatgt tttatatgaa actaaaaaaa 255240
tgtggagttt gtacttgcat ttcaagggtc actgctctgt tataggccaa gtgaacttat 255300
gtctggcctt agagaatctt acatgtattt gcatctatca gtatataaac atgtgggccg 255360
tagaataagg agccagcagt accagaaccc agccttgtta gaggccacca ttttggtggt 255420
tgagtggtta ttagtttaca tggaagcatg gagaataata ggcaaatgta ggttttcagt 255480
gtcagtcgaa ctggcaaaca aaaatttctg gtcatcttca gaatgaaaag ttttcttgag 255540
tacctacata ttttagcatt ttcatatgaa gcagatacat tataagttaa ttgttggaat 255600
ctaattgtaa tatggctgta agttttttct ttatatttgt tattgccttg ttcttatatt 255660
atagggaaag agaaacaaac aaacaagcaa agaaactaat ggtcatatat ttgagagcca 255720
actcttggtt gctgttcagt tttattttcc tgtaaacata tattttccct tatgaaatct 255780
tgggaatatt agctctggag cactgctgaa ctcaagtaca gacattttca tgtgtatcag 255840
tagattccca tcatgacatt tttataataa tgttgaagag cattttaaac aactggaatt 255900
aagctcaaat acattaccag tggttgaaga attcacatca ataatcttct gaatttagga 255960
ataaaatgga gaagtcaagg aaaggaaaat attatacaca ggctagcaat agttaaaata 256020
caattattaa agccagagct agacaaaatt atggcaatga gatgtgtaac aaaaccactc 256080
tgtaacttca tcatgttcgt ttaaaagacc tgaatgattc caaaatcctt agaccaataa 256140
actatgtctt cttacttgat aattaaaaaa aagaattata aaggcaaaaa tgaaattata 256200
tgtatgtgtg tttgtgtgtg ttccatggga atacagctgt tgtaaatcaa aggcctactt 256260
gcctgaccaa gcagaataaa aatagtcatt gattttaaag agactaaaag tgagggaaga 256320
aaaaagtctt ctgcaaaagc tctacagatg gttgtcaaac ttttcagaaa aatagacaaa 256380

gcaacatttt gaaaaggatt tatatctttt atatattcaa ataccatctg ttatttaaaa 256440
aacatagccc acactgaata tctgatatag acaatataac gtttatgaga taatagtttg 256500
agaatgacaa taataatagt aagttttaaa agaggaaata agccgggcac agtggctcat 256560
gcctgtaatc ccagcacttt gggaggccga ggcgggtgga tcacctgagg tcaggagttc 256620
aagaccagcc tggccaacat gacaaaaccc tgtctctaca aaacagtaca aaaattagcc 256680
aggcttggtg gcacacatcc atagtcccag gtacttggga tgttgaggtg ggagaattgc 256740
ttgaacctgg gaggcagaga ctgcagtgag ccgagacccc acaactgcac tccggcctgg 256800
gcaacaatga gactctgtct ctaaataaat aaataaagaa ggaaatagta aatgtcatta 256860
gtaagaagaa tagcaaaatt tcagttctag aaatacccag aattagctat atgatacaaa 256920
aaccctcatg aaacatttgg ataatcctca gaatacctat caatataaca aaaactatga 256980
attgacttca ttttgaatga tgaattttta acaaaaatag tcaatacttt gggcttagta 257040
gctctagaag tgcttctttc ttctttcttt taattaacag aatattgtca gaattttcaa 257100
atgttgatga ttataagttg aattttcctt ttcattaacc cctggggctt attttttgac 257160
ttgatatgtt tctctactca gaattaagat gtgaagagct ttgggataga atgcatcata 257220
caagtgtgag agtcaagttc caatccataa agtacttctg gtagacctgg ataaagatga 257280
ccttaagaaa tgattttttt tctctttgca gtttaaaaac aacagtagca acaagaatga 257340
taaaacctat gaagccagct ctgacaaaag atgttttttt tataacacat actatgtatc 257400
tactttttga tatttatcta tcgagaagac ttttctcctt attgtttgct ctgaaatttg 257460
tttatattta ataggaattt tatagcctta tccctgggta gaaattcagt ttttttaaaa 257520
gtagataatt aaatcttaat ttactattat tttacacatg gtgaaactag tcaataaatg 257580
taatatcatt gagctgaagg ttaaaaagga aaaaatatga ctgcaagagt ggttgtattt 257640
ttgatttcag catcacactc aattgcatca ttgagtggtt cattcttagt ttcagtgtta 257700
atgaaataca tctgagtttt tttccccaga agcttgaatt gtgtcaccaa agtgtcattt 257760
tgatttattg aaagttaagc cctttccaaa attcaccata attttacatg tctcgaaagc 257820
aattttatac ttcaagtctg tgctatagtt ctatatattt tatgaagatt tggatagata 257880
tctagccctg agttttttat tgcctgttaa atacttataa cccaaagtgt agcagcctca 257940
tgaactgctg ctgggcacag atctgggaga gtgcactcag agtgttggcc caggaagagg 258000
aaaagaggaa aaggggcagt tcagtggcag cttggcttcc tgtgaaacat gtcaccttaa 258060
ccacttcctc attcttttag tggcaaacta gacgaccttc tccttcccct ttcctgccga 258120
gtccccctct aatcaacact ctagaaggcc cctcttcctc ctcagtcctg actccctgtc 258180
atttacttca ggttgcctcc ttgctcagtt ttggccccag gggtaggcaa tctcctcttt 258240
ctggacatgt ctcccaccag ccccaagttc agatttcttg gaagtttctg tgttgtctcg 258300

aggatgcatg gttgtgtgag ttattccagg gctcaaggcc tcttctgtgt ctgtattccc 258360
caggaatcca ggaattcact tctctgcctc tcatcctcat cctcatcatt aaaatgagca 258420
gatttgagct caggaagtgc ttgaataaat gaataaatga acaaatgaaa ggtgattttt 258480
gaaagtctta attttaagaa gtctctgagc cctgttacag ctcaattttc ccataaacta 258540
gaactgctct ctaaggctgt gacatttctc ctttttcctc tcctcaaaat ctacctgctg 258600
ttctgatatc tccctgacag ccaccatagt gagattctat ttccatttcc aatctctctt 258660
ttgtaaggga cagagaaaca gctagagaaa tatggagcca tgcgctctga ggactttagc 258720
aggcttcaac tctatctgca agtgagtttc acttagcgaa tgaaattgga aacttaaagt 258780
gggtagggat gagggttccc ggagaaggtg taatacctca gtctgggaat tgggagcatc 258840
tacaaggaac acactcaatt ctgggaggtt cctgtagatt tcagagattt agcagggctt 258900
cccagcactc tgcttcccaa cctgtcattg gaccagtaaa ccctgctctc aaactgttgt 258960
ggtgtccagc atgctttggc aaggtaatga aagataacat gacatggaca tatgggtgac 259020
atcctggaga gatgacagaa gcctctgttt aaggacaaga tttgccattt agattttgca 259080
cccactgtat aataagagcc ttaggattgg gctggaatcg ccctagcagg catgatggca 259140
gcccctctgg actggcaata tgcagctttt ttgcaagtgt tccatgtcca agtcacccca 259200
cccagctttc tactgctccc gtggaagctc tggtgaaacg caaggaaagc agctgctgtt 259260
gactggattt ttctttcaca tgaaactttg aagcctcata gatgcttatt ggcctggatg 259320
ctattaaacc tttaaaaatc cctttctcat cttgagagta tttgagaaac atgtctgggg 259380
cattttgccc accctcctcc aggttctgtg tcagtgagtg atatggtttg cctgtgtccc 259440
cacccaaatt tcatcttgaa ttcccatctg ttgtaggaga gacccatggc aggtagttga 259500
atcatgggag caggtctttc ccatgttcct gtgatagtga gtaagtctca agagatctga 259560
tggtgttaaa aaggggagtt tttctgcaca agctcttctt ctcttgtctg ccaccatgtg 259620
agatgtgcct tccaccttct gccatgattg tgaggcctcc ccagccacaa tggaactgta 259680
agtccattaa gcctctttct tttgtaaatt gcctactctg ggatgtgtct ttatgagcag 259740
tgtgaaaaca gactaataca gtgagtctgg gtcagtgtgt gtttatgttg aacgtagtgg 259800
acttgtggtg tccccagggc accctgtggg gaatgttggc ctgtggcttt gctacttcca 259860
gggggatttg gcatggagaa tgtgtgtttt aagtaataga tagattatga ttgaagtgtg 259920
ttatgggctg agttgtctct cctcaaaaag acatgttaaa gtcctaaccc ccagtatctc 259980
agaatctgac cttctttgga aatagtgcct ttatataggt aatcaacttc aagtgaagtc 260040
attagcacaa gccctaatcc aataaggact ggcattctaa tgaaagggga aatttagaca 260100
tagaaacaga catgcacaga agaagatgat atgaagagac acaggaagag gacggtcatg 260160

tgactggagt gctgggtctg caagctgaga aatgccaagg tttactggct aacatcggaa 260220
actagaaggg gcaagagtgg actctccccc tcagagagag tatggccttg atttcagact 260280
tctagcctcc agaactgtga ggcgtacatt tctgttgttt ttgaagccac ctagtttttg 260340
atactttgta tgggagccct aggaaattaa tacaaagtgt attaaaaata gaattttctt 260400
ctattgtatt tttgaggcaa aagaaataag gagctattga tttaaggagg aaggtttggc 260460
tcatctagta tagacttggc taatcattta ctcttgtata tttctcttct gcctgcccaa 260520
gtagttcact gaacaggctt agaagttgat ttataattgt aagagtttac acatcagaag 260580
ttaatttata attgttaaca gtttcatgca tctacctcat aaataccttg taaaagattt 260640
tacccttcag aataatgttt tcctctggta ctttagtagt ttatcctttt tttgcatctt 260700
tgatgcattt gaattcatcc tggtatgctg tgtgagctgg ggctccaatg tcgttttcct 260760
acagatggct ccacagttgt ttcggtttca ttcgaacagc ctcctacaga agtatttttc 260820
agacttcttt tatacttgac ttccaagttt actactggtt aagagctttt aaagtagggg 260880
gaaagatatg acccattggc ctactcgttt gattattttt tccaattctg aaatcaaaaa 260940
ttaaaaaatt tattcaaagt tatagtttca cttaatttat attaactgca tagatagtgt 261000
gattcatctg tcctgcttgg acatatttag taacttttaa ttaatcttag agataaaaga 261060
tagaaatcta tgagacttga gacattcaaa taagaccagt actgataaga ggtaatcagg 261120
tgaagtgcca tttggttgta ataggacatc aaaattgttc cccaagagtg aaggatgttc 261180
cattctcact tttcccatct gtcctcccaa attctttcac tcttcctgat gctttgaacc 261240
aacttgaaaa gttagtccac tcagaacctc agctgttaaa cttttagcct ctgcaaacaa 261300
actagccgtt ttcctctttg tccctttacc atcaagctca gttgcttctc ctactcctct 261360
cccttctgcc aggggctcca cccttgttcc tcattcaaca gtggaatcat ctgtgtgcct 261420
gtctcctctt tactgtgtac cattgggtcc tcttcttcac catctctaaa tttatgcttc 261480
ttccctgttc cactgtcacc agcttggtct acatcctact ttgatcattt ggatttaggt 261540
cccaccctaa tcgagtatga cttcatctta acttgattgc atctgcaaag acctgatttc 261600
caaatacgac cacaagtaaa ggttcaggtg gacatgaata ttaggagaga cagcacttca 261660
attaacagtt tggcattagg actttattat ctagcttagt aattgtttta acagataaat 261720
aatagtatcc cctaaagttt aaaatgcaga accagattat gcctcagggt gcctaacctg 261780
agaggaagag tctttcttca acagactctg ggaaagacct ttgtaaaaag gctggtgacc 261840
attctatttg atttctactt tccaatatta actgcttaag tcagacaatc tccttggccc 261900
acgctttccc ctgccattct ccccttcaca taatgtcttc attttatttt tcccttgttt 261960
tattccctga actcccttct ctctattcag attccctgcc catttttcct cctaatgaga 262020
cgcctgactt cctacctcct ttttgaaact tcaaactgct tagcaaatac aattttgaat 262080

tgagtttgct gtcacagaac aaaaactagt ccttggtcca gagtatcttg caggactgaa 262140
tcaactgtgg tctaagacac aataataact cttaattatt ttcttgtttc cttcatggta 262200
tatttgttgc tggatgatga gtttatatta tggttgaatt tcctttttga aatgaaaaga 262260
tgaatagtct ttattggctg ggtatctatt tttatccaga agactttgca aatgagaaat 262320
atttctacaa tctgagttta atttgcttaa gcaatgaact tgttctttaa aagtcgtata 262380
tgtgcaaaat aaatttaaat taaataaatc atattttaat gtagtcaggg tagctttcat 262440
ttaaataaaa cctatgacaa atatccacgt gaagttatca ctcctttaac aaaacaagtg 262500
ttctcctttt ttattgaaat actagttttc acaaattgag attccttaat gtgatgaccg 262560
cttggactga ctatagtata gctgcgttct tagaatggcc ctgaaaccac aaggcctctc 262620
caagatgcat gtggctgact ttctgaggtg actgcattga atctttctgg cttagcttat 262680
tctgcatcca gatccctgga agctatttat ccccaaacag tggtatattt agacccattg 262740
tgtgctaacc tctctgtgcc ctaagtacca acatagccaa gccaaagttc gaactttgct 262800
gccttacaat ggaggttaca tcgtagtgat gaaagaggtt aattttcttt aggagttgaa 262860
gaccagagaa ctgcaaaaag gggagaaaat cagtgtggcc tctaatgaga gctaagtgag 262920
tgaggcaata gaaaacagtg aggaccggcc agtgcggtgg ctcatgcctg taatcccaat 262980
actttggaag gctgaggtgg gcggatcacg aggtcaagag atcaagatca tcctggccaa 263040
catggtgaaa ccctgtctct actaaaaata caaaaattag ctgggtgtgg tggtgggtac 263100
ctgtagtccc agctactcgg gaggctgagc ggggagaatc gcttgaaccc gggaggcaga 263160
ggttgcagtg agtcgagatt gcgccactgc actccagccg gagtgagact ccgtctaaaa 263220
aaaaaaaaga atacagtgag gaccttggag ggaatcttac agtggagcag aattaaaaga 263280
gaagaattca cctatttcct atataactcc ctctgtaacc gataactttc tagagaaagc 263340
tcactgaata tatttgaaat ttgtttcata ccacttttat gttattgcct tctttcacag 263400
gaaccagaga gctctaacct gtagatttgc tttgttgtct ttactttaga ctacagccta 263460
aactgactgt tgatttttgg cacaattatt acatctctcc tttcaccccg cctctagccc 263520
tttcttaatt acccccatta ggcatttttc ttgctagcgt gaactaaatc ccctgtctca 263580
agcacgcgcc tgtacattga aatagaatgt taattcattg acgaaattaa ctgttcttga 263640
ttgggaaccc ctagttccag ggaatcttaa ggtttcaatt ctgttgcctt ggttactaaa 263700
ctgttccatg cagtttgctc aactttgaat ggatacttct cttaggaaca ttctcccttg 263760
taagtaaatt gcttgacgtg ttaaaatcta agttctgttt tacaattctt gagacaaatt 263820
caccatgatt tagttttaaa aacaagtggc cttgtccttt tagtctagga tttatcactt 263880
agctgtatct agcaaagccc ctcacacaat agtaggaaaa agcagaagtg tgattgctaa 263940

atgaagggga aatgtaataa aatgatggaa gtgccctttt ttagggggaa ttaaagtttc 264000
tctttgtagt gttacatgta ttcattatgt gactttcata aatacaaacc ccaataaaac 264060
ggtgggcagt tccgtctctg gagatgttaa gtgtctcttc atagaataac atggcacatc 264120
ataacatttg attcctttta cctggtcaag atgtttgctt atggcatttg aaatgcatat 264180
tttcaaacta ggtagatgaa tggtttaaat ataaaaattc attcaaggcc ctgagctata 264240
gaagaatatc ttccaaaaca tgcattcaag ttctatctgt cagtgttttt ctttttgcat 264300
caataaggca gcaatggaat acaatcagat ttattttttc tttattgctg tctacaattc 264360
agagatataa cctgaaaaat atgttgtctc ttcccaagta gatcttgtaa ttctctgaac 264420
tgtgatccag accaatgccg cctttacatt gggtctgtgg aagtgggagg gtccactgaa 264480
cctggttatg agaggtttca aattgaaaag atttacaagt atgtaaataa aatcaaatag 264540
tcaattagta tctaacatat acatgtcaaa acacatagcc ccaatctcat aaaatcaaag 264600
agatctacct aaatagattt gtttgcctaa caaatagcaa gaccactttg acagaatctt 264660
tagcatgaca gtgattgtca aaatgggtaa atggttatta ttgcccagtt aagtaatttc 264720
tgatttgctt tgccttgatt actcttaata aactaaactt aacataccat agaagcaata 264780
ttttttaaaa agagggtata ttctagagct agatagggtg ttgtgcaaca atgtgaatgt 264840
acttactacc attaaactgc atacataaaa agggttgaaa tggtaaattt tatgttatat 264900
acattttacc aaaataaaga aaagttataa tactttccgt agcatatata aaaaagaaac 264960
agacatttca tagcaaaaat aggataaaaa tgatattaaa ttcatagggg atttctctta 265020
tctaaaactt tagatttgat tcttagataa gaatataaac acattgcaag aagataaatc 265080
aataagccaa tgaagtaagg tggaataaat aaatagaaat aggtgatact tgcggctctg 265140
acagcatctg ataataatag tacaaacagt ttcatttcat ttaacagtta tttgatgaac 265200
tcctgtgtgc taggaactgt tatttaaaaa aagtaacttt tattttaggt tcaggggtac 265260
atgtgcaagt ttgttatata ggtaaactca tggctcgggg cttggtacac agattatttt 265320
gtcacccagg tactaagcat agtacccaac agtatttttt tttccgaacc tctcctcctc 265380
tcaccctccc tcacgtaggc ccagtgtctg ttgctcccct ctttttgtcc atgtgttctc 265440
attatttagc tcctacttat aagtgagaat atgcattatc aggttttctg cttctgtgtc 265500
agtttgctaa ggataatggt ctccaattcc atccatgttc ctgaaaagga catgatctct 265560
ttctttgttt ttatggctgc agtgtattcc atggtgtata tgtaccacat tttcttcatc 265620
cagtctacca ttggtggaca tttaggttga ttccatgtct ttactattgt gaatagtgct 265680
gtaatgaaca tacgcgttca tgtgtcttta tggtagaacg atatataatc ctttgagtat 265740
atacccagta gtgggattcc ttggttgaat ggtagttctg tttttagttc ttttagttct 265800
gtgtgtgaaa acacaccaga gaacaaaatt gaccagatct ctgccttcac agaacgttca 265860

ttctactgta ccagttattt catgaggaat atgaatactg ttattactct ctgcccattt 265920

cacacatgaa ggagcagaaa tggagcagag tttactaatt tatctgttgc agatagtagc 265980

taattgtgtg ccaggtgctg ttccaagcat tttacccctg ttaactcagc tgagtgcttt 266040

tattttcccc attgtatgcc caacttatgg atgacagcac tgaggtagag aaaggttaat 266100

aacatagaat agtttagtat tagggaaaca atttgaattc taggacactt ttactgaaac 266160

ttagcatagg ctaaagaagt gatcgtgcat gtttaaagag ttgtatcatt ttatcattat 266220

ttgactttct tttccaaaaa aaacaaccct tctcctttct ctctcttcca tgtgacaaat 266280

acatgtctgt atacatacat atatatgtaa atataaatat atataaaaat atatataaat 266340

ataaataaat atatatatat atatataaat atatatatat atataaaatt tttttttttg 266400

agatggagtc tcactctgtt gcccaggctg gagtgcagtg gcatgatctc ggctcactgt 266460

aagctccgcc tcctgggttc atgccattct tctgcctcag cctcccgagt agctgggact 266520

acaggtgccc gccaccacgc ccggctaatt ttttgtattt tttagtagag atgggctttc 266580

actgtgttag ccaggatggt ctcgatctcc tgacctcgtg atccgcccac ctcggcctcc 266640

caaagtgctg ggattacagg tgtgaaccac cacgtgcgac ccacatgtct gtatattaca 266700

gccgttgaag gacacaactt ttgccattgg gcgacccagt cccctagtaa gtgttcaaaa 266760

atattcgcta ttatcattgt tggtgatttc gaggatttgg atataattaa tttgagccta 266820

atttttttaag actattacat aactatacaa aaaaatcatg agagagcctg gaattttgca 266880

aaactaggat agaaaccagt ttctaaaaag cattttaaac attcagcttg tgtatttctt 266940

ttcacttgta ttttgtgatc tgcctccatg ctttcatgcc tttctcttca aacaatctta 267000

ttaccactaa ccaaaaacaa ttaaaatttg attgttttgt ttttacatgt tgttacagtt 267060

aagaaaaaaa aattctaggt attgacccct gccagttttc ggagttaata ctaactagtg 267120

atttggggct acatttaagt gctttaagct atcctacaag cttaaagtag ttatacatgc 267180

aaatacttaa atgaattagg tacaagaata cagaggtaag aaatagaatc tattgtagaa 267240

taaatcctat ttcagatgtg actgttttag gatttgaatc aaccacatat tacagaaaac 267300

cccaagataa agccttggca taaccaagac aaagtttatt tttttctctc acataaaatt 267360

agtccagact tattttacca gtggtatggc aactccacag tcaaagggac ccaagctcat 267420

ctgaccttcg attcccttat cctgggacag gcttacattc tcaaggtcga ctcctggcct 267480

aatgggggcta ctggagcttc agccatcaca ctctgtgctt cctgttatat ctactggtca 267540

gaacttcaga tacatctgtc taaaaggaac gctgaaaata cagtctttaa gctgtgcaat 267600

tgtacctgga ataaatgagt tcttttaata aaaagagttg aatggatatt tggtgggcaa 267660

atagcagtcc atgctacaat aataaacttc acaattttag aattagatat cttgtaatga 267720

atattctgac gtggtgtcaa cttataccat agatagatta taactttgaa aaggaattag 267780
aacacaataa taataaattc acactaaggc tcttttgggg aaaaaaaatc tctaccattt 267840
attgagtgtt tattatcttc acaacatccc tatgagttag ggattatttt tgtggacatt 267900
ttacagataa agaaacagag gcattgagat gataactagc ttgactaagg agtggcggag 267960
ctctgggcag tgtggttcct gtgtgcccat aaggccattg cacaatgctg cttcatcatg 268020
taattcagaa agtattgcag gatgtggcag ttggtcatca tgaatgttca ttgttatctt 268080
gtggctttgt gaaaaattct ggtcattcaa acgatttgac tgttgagatt ctgtgcacat 268140
gagattgtac tgtgtacatg tttctaaaaa tgtgtgttat agatcaaaat gcacacactc 268200
atttacctct aagaacagtt cttatattga agggaaatta tctgtacgtg agagaacaac 268260
gtggttttga gtacaagtgg tgaacagaca ttgaatagtt gttttcagaa agcatcactc 268320
ccctgtacct cagaacccag cctcagtgct ggttgcgagc caacagcttt gatgtcctga 268380
agtttttgca tcacttctct tttgcccctc tttgggattc aaagatgata agtttgaagt 268440
ggagttctac cgcttccatt ggaagagaaa aagtttccgt gtgtgtgtgt gtgtgtgtgc 268500
gtgtgtgtgt ttacggagag gagttctatg catctgcaga gggtgctgcc atcaacaagg 268560
agaacagagt gggatgaagg gtttgggaag ccaggatggg catctctgac gagctccaga 268620
actctccacc taagtgtgca agtgtaaaca ctccctgatg tgtgaactgg ccatctcaag 268680
actaagtatt tagcaggaaa tgccccctat tcaacccttg ccttctcagg tgtagaggtt 268740
gggttgtctc ttccatcttt gtttgaatta tgtcattaat caaccttagt gaaagatcac 268800
ttagtcattt gtgacagcat aagttcttaa ttgttaggaa tcactggtgc ggcacacatc 268860
tttttctatc catgaacaac gtcaaatgct tattttctca tgagctttta ttttttcctt 268920
ttaaaaaaag tttcttagga taaacacagc ctttttcctt gtctctccct tgccctctca 268980
tctttttctc aatctttata ttcctatatg tcactgaaga gtccccgtgc caacgctgtg 269040
cagtgggagg ctcctacctc caccagcttt tgaggaggtt gtagtcctgc aaccttagag 269100
gttccacagc caagctgggg gtctttctgg agcatgggtg gtgaatctga gatctatgca 269160
cccaggaagc ctgacacatt attgtggtgt ctcaattctt ttttttttaa ttagaaaaat 269220
tgtatcaaat tgcatttggt gagagcaaaa ataaactgaa gttggttgag ctttggaaga 269280
ctacaagcca ctgtaatatt taagatttct tgacctccag aactaacatt tgtcctgtca 269340
gagaaaataa ttactcctgt tgagaataca tgcattaaag taagatgttc actactctat 269400
atgatcacca aacattaaat aatatgtttt acacatgtat gtactatgtg ttcaagtgtt 269460
tataaatcca tggagccaat agaatgtaag ttctatgagg gcagaaattt taatccattt 269520
tgttcctaga acagttcctg acacatactg gtgttagggg taggtcttca gattttatgg 269580
atcaaatgga atctccacac ttaaaaaaac ttagagaaaa actgccttaa tgtgcccata 269640

gtctggttgg aaatggcagc tccaggttca ttgattgctt tgttcattcg gcacttttca 269700

ctggacatgc tatgagccca gcagtgttct gggtttttgg gatacaccaa gccctgcctc 269760

cccggtgctt aacattctag tgggggagac agaaagaaag caaacatggt ggacaactta 269820

attatctatt tttgaaagtt gacaggaact gtggacaaaa gaaaacaaga gctggagaca 269880

gcagtgccag ggcagggtga gggtcaagtt agagtaagcc tcattgagag atcttttgag 269940

caacacctga aggaggaggt gagaaagtta gccatgtgga gggagcagca ttccaggcaa 270000

gtggcccacc aagtgcagag tcctgacagc aagagcagtt ctggaatatt ctagaaacag 270060

gaagaagacc aatgtgacta gagcagagtg aggcaggagt gagagaaaat gtgatcagga 270120

aagtaaggtc ctgtggccac taagattttg gcttttattc tgaaggaaat gaggactcat 270180

tgcaggactt tgagagcatg atctgacttg tcacaagtgt tctctttgtc tactgggttg 270240

agaagacatc caaagggggc caaagattga ggcagggaga gcagctagga caggctctag 270300

caatctaggt gataaatgag gaccgatggt ggctctgaca agggtggtaa tggagacatg 270360

gtgaggagtg accacatttc aaatatatct tgaggtagag ctgacaggat tttcccgatg 270420

ggctgggcgt agtatgtgag agaaagaatt cagttatgga tgattatttt gtcctgaaca 270480

atgataaaag ttgagtcaac aactgatatg gggaagtcta caggtggaac aagtttttga 270540

ggaggaaatc agttcagtgt tggctatgtt gaatttgaga tgtttttaga ccatctgagt 270600

ggagaaatct ggagtttagg agagaggctg gacttgatat gatgttatgg gtcctcagca 270660

tagagatgat gaatgagatt agcaaagtag tgagtgtaaa aaggaaaggg aaggagacca 270720

aagattgagc cttgggacag tcagaaagaa gagagggaac ccgcaaagga gattgaaatg 270780

gaatccatct cactgtggca tcgtgaaaga caaatgaaga tggtataaga tgaaaaagtg 270840

atcagctgta tcaaatgctg ctattgggtc aagtagggtg agaattgata attttccatc 270900

ttgtcaagag cagccgtgat agagggaggg ggtggagata tacttggaat gagttcatga 270960

aaaaatggga gggaagggat tagaagcagc aagtataagc agctctttca aaggagggag 271020

atggggaatg gctactggga tgccatcaca tagataggaa ggtggaaccc tgtgcactgg 271080

aggagggagt gtcctcacag aggaggacag gcaggaaggt agagtgggct gctttgggca 271140

catgtggttc tgtggatgtt ctcttttgac agcttcagtt cttcagggaa ttgggagcaa 271200

gttcatcagc tgagagtgaa catggggcag acagtgtgag aggtcaaagc ggcaagagta 271260

gttgtgccac agtcttttag gagagaggga aagtaaatgg aatggggaag gaaagtaaat 271320

agcatggctg ctgagctgcg ttacaagccc acccggtgtt gggttgtgag gtgtagttgt 271380

gggctcttct tcagtcggat tgtcagcatg ggcggctggc agagagttga atctgacagg 271440

gcagcagttc tggaaaatga gtatgatgag tcaagaaagg gaccaggaag tggaaactgt 271500

gtgtgaggca gtgatgctga tgaatgactg tggaaaacaa tggaggtgag gagggagtgg 271560
atgttgagtg gctaggggac agtgaaaagg gagtaggatc cactagcttg ctgcttgtct 271620
cttataatta ggatcaagag cttctgccgt cttctaggca ttctgctgct tcaagggagt 271680
ctctgagatt gggttctgca tggatgtatt gaaacatgca cctaggtgtg tttggagtcc 271740
tgccagagac ctgctttctc tttattctta tcctgaggcc ctttaggagg ctcagatgga 271800
ataattttta tggtttctta aaacccaagt gggttaccta agccatcata gttctgcccc 271860
ctgttccctg agaggaccac gggagcaccc tgaaatgggt tttccactct gccatccccc 271920
acttgcctgc ttgggtgttg acctcagcca tctacacata tcccggatgt agtgttgggc 271980
ccctggttga tgtcattcct tttcaaatgg gatcttttac tacagcaatc cagcaacaaa 272040
ctaaatagcg atagagtttt ctttccctta caaaccacaa ccacaaccaa gggaaaaacc 272100
aaaccaaacc aaccaaacaa acaaaacaaa agtatgtggc ttcttaccac agccccctat 272160
ctcgcccaag ccttgtggct ctttgatctc tggtgttctg aaattgcgta atcatgtgct 272220
tgctcttttt cattcattgt gctgggcaca cacttaatgg accctttcaa gttggttttc 272280
ttttatgggg taagggtagg caggactatt ttttaagatt tcttccactt tgtcttccaa 272340
ttgttctaat gagtttttta aaattgtgcc tgtcctgttt tttaaattcca agagatattc 272400
tttgtcctct aaatgtttct tataaatata tataaaatga ataatataag catatagttt 272460
tgcatagatt tgagttcgct gttacagtgt ccttttattt ctctgaggat attcattata 272520
ggttttttaa aaaacacttt ttctggtccc caaattatct cttctctcta gacttcctcc 272580
attcatgcct tccttccccc ctccctccct tcctttgctc ttgctcttga ttttggaggc 272640
ttgtcataaa tatttgacca tccattcatt ttttaaccat aacgaactaa aaatgttaat 272700
tggagccctg tgtgttcctt gagcagaatg tttggattaa tgagcttccg tgcaatgttt 272760
tctctggaat ggattaattt ctccagatct ataatttttc aatctctttc caggggggtaa 272820
aagcctggtt gtctgtttca tgaaagtaaa aatgtgggta agagtgatcc cagcattcag 272880
aatgcagact tagtagctgc ttgttttcag tcctgggact tcacccaccc tcaccacagt 272940
tggtatccca aagtgtgaaa ctccttagat tatctttctc aagaaaatga aactcagata 273000
ttcgtgtcct ttggccattt ttgattagct tatttgtttt cttccttttg aattgtttga 273060
attccttatg tattttgaat attaacccct tattagattt atggtttgca aatattttcc 273120
cccactctgt gggttatctt ttcactttgt caattgttta ctttgctgtg cggaaagctt 273180
tttagtttca tgctatcaaa ctaattttgc ttttgttgcc tgtgcattca aggttgtatc 273240
caaagaactc attgcccaga tcaatgtcat ggagcatttc tcctatgttt tcttctggta 273300
tttatatagt ttcagtgctt ttctttaaat tttaatccat tttgaatgga tttttaatag 273360
ggcataagtg tcacttccat ttttttatat gtggatatcc agttttccca acaccattta 273420

ttaaagaggc tgtcctttcc ctattgtgta ttcttggcac ttttgttgta aataagttga 273480
tcttacttgt gcgggtttat ttctgggcct tctaatctgt tccattaatt catgtgtctg 273540
tttttatgcc agtatcacac tgctttgatt acaatagctt tataatatat cttgaaatca 273600
gagagtttga tgcctctagc tttgttcttt ttgctcaaga ttgttttggt taattggggt 273660
cttttgtggt tccatacaga tttaaagatt attttttcta tttctgtgaa aaatggcatt 273720
ggaaatttga taggaagtgc attgaatctg cagatcactt tggatagcat agattttttt 273780
taacaataat aatttttcca ctccatgaag aggtatactt ttttcattta tttttgtttt 273840
cttctatttc ttttatctgt gttctgtagt ttttagtatt caggtgtttc acctccttgg 273900
ttgaatttac cccaagtatt ttgctgttgt tgctattgta aatggaattg ttttcttaat 273960
ttccttttttg gataattctt tattattata tagaaaagct atggattttt gcatgtttat 274020
tttgtatgct tcaactttac tgcatttgtt tatcaggttt taacagtttt ttgataaaag 274080
ttttggtgtt ttctgtatat atgatgatgt catcagcaaa cagagacaat ttcatttccc 274140
tattttcttt cttttttttt ttttgagatg gagtctcact ctgtcaccca ggttggagtg 274200
cagtggtgcg atctccactc actgcaagct ctgcctcctg ggttcacacc attctcctac 274260
ctcagcctcc cgtgtagctg ggctacagg tgcccgccac cacgcccagc taattttttt 274320
gtatttttag tacagacgga gtttcactgt gttagccagg atggtctcga tctcctgacc 274380
tcgtgatcca cccgcctcag ccttccaaag tgctgggatt acaggcatga gccaccgcgc 274440
ccggcccccta ttttcttaat cacgcgatat atacaggctt gttgaaaaac atgaaaatga 274500
taaagaagtt tatgtattaa gaggcaaaag ctatgatgtt caactcctct aaaaccactt 274560
cttagtaggt gaacattgtt aaacagtttt gtgaatctgc ttccagattt tttctaggca 274620
tgttcataca catatacaca cataatttat ttttacccag ttgcgatcat gtatcatctg 274680
atccaaaact cgctattttt cagttaatat gcagtgactg tctttccaaa ccatagtata 274740
tatatatatc tctaagaatt tttcaaatct gctatagttt ttaaacatat ttattgttac 274800
atacatatgt gtataagtta cttatttgct tatttctttg aatatctttg tctagctttg 274860
gaatcacata aataatgagt acaaatggta aagaaatttc atgtggaagg atatatagaa 274920
aaagtacaag tctctcttct catccactct atctcattac ctaaaaggtt atccttagag 274980
ctttatataa tacatccctg aagtttagat cattttattg cctcccaatt gttgagagta 275040
aggaatttag tacatatacc tttcctttca ctttcctacc ctctacctac ttaatgagtt 275100
cgattatagt ttttcagttc acccactgtt aaataatata cctatattta taatatacct 275160
atataatata cctataataa acctatattt ttgacttatc aactttggac atatctattg 275220
gtgacttgtg atgagagatg aaataatttg tacatttacc cttcttttcc attcttttcc 275280

cctacccatt ctcccctttt tggctaaatg attgctttta ggttgataag gtttataata 275340
gctactgttc tgtggttata aaatttgttt catattttat gtgaaatttg attatacaac 275400
tagcattcaa aacattatta tgtaaatatt agttactgta atcacacagt gacgctcaag 275460
gaggtattta ctaagcatac agatcattcc ttctggatcc aactcagcag ttgctttgct 275520
gtgccttacc tcatctttcg tggattcctt ttcttttgat tgcaatatat ccttgagtaa 275580
tttttcagaa tcgatatgta ctgtctgaac tctcttatac ccaaaaaatt tgctttcaca 275640
tttgctaata gtttgacaga gtatcaaatt ctgctttcaa attctttccc cttcatggct 275700
ttgaagatac tgccccacta tatattctaa aatccattgc tgctaatgag aaattctggt 275760
gtcactctga ctcttatttc tttcatatca cttggcattt tcttccgaga aaatattact 275820
tatcttcaga atgatgaacc ttctctatca tgtctctagg tgttagggct tctttcattt 275880
aatcctgtag gtcctcaata ggctttttaa ttaaagtctc attttattcg ggctctgtga 275940
aatttttttgt tatgttttgg cttctttttc tcttttgttg tctctctttt ctccttgaat 276000
gtctttctga tagatatagc attcccatta tctatcatgt ctttcagatt atcctacatt 276060
tttctctttg ttttcttttt ctaccatgtt tagaattctc ttacacgtaa gacatcactt 276120
actagatctt cagtcatatc ctttttatta ttcagtctgt gcatttttaa ttttcaattt 276180
tggcaaccac atttttaatt tctaagaatc tattttgcta atagtacttt tgtcctgaaa 276240
ctttttatat cctgcaatat ttagaaagag cacaccctta tccacagctg attaggagta 276300
cactctgaat gcagggcacg atgggcgaac attttgttac tggcctcctc aagcctcaat 276360
gcccagaagt tattgcctcc atcagaaacc cttcttttgt tctatccctc acttccaaat 276420
gaagttccag cttaattttt gctgtcactg tagtgggaca agcaaatccc tatttcacgt 276480
gatcattgct caaacctcta ttgacaatcc tgctttgcat tcccttattt agagtgggat 276540
ggaaaataaa ggctatgaag ggggaagaat ctgatcccac cattctcctt tttcattgca 276600
gttttctcct tttttatgga tgcaataagc tgttgcaatc tctcaggatg gaaaagaaca 276660
ttttaaaact actctactgc cagaatgatc tctttttttt aattatactg taagttctgg 276720
gatacatgtg cagaacatgc aggtttgtta cataggtata tacgtgccat ggtggtttgc 276780
tgcacccacc aacccatcat ttacattagg tatttctcct aaatgctatc cttcgcctag 276840
cccctcaccc cttgacaggt cccagtgtgt gatgtttccc tccctgtgtc catgtgttct 276900
cattattcaa ctccccctta tgagtgagaa catgtggtgt ttggttttct cttcctgagt 276960
tagtttgctg agaatgatgg tttccaactt catccatgtc cctgcaaagg acatgagctc 277020
atccttttt atagctgcat agtattccat tgtgtatatg tgccacattt tctttatcca 277080
gtctgtcatt gatgggcatt tgggttggtt ccaagtcttt gctattgtga atagtgccac 277140
aataaacata cgtgttcatg tgtctttata gtagaatgat ttataatcct ttgggtatat 277200

acccagtaat gggattgctg ggtcaaatgg tatttctggt tctagatcct tgaagaatca 277260
ccacgctgtc ttccacaatg gttgactaat ttacactccc accaacagtg taaaggcatt 277320
cctatttctc cacatcctct ccagcatctg ttgtttcctg actttttaat gattgccatt 277380
ctaactggca taagatggta tgtcgttctg gttttgattt gcatttctct aatgatcagg 277440
gatgatcagc tttttttcat atgtttgttg gcctcacaaa tgtcttcttt tgagaagtgt 277500
ctgttcatat ccttcgccca ctttttgatg gggttgtttt tttttcttgt aaatttgttt 277560
aagtaccaaa aacagataca tagactgatg gaacagaaca gaggcctcag aaataacacc 277620
acacacctac aaccatctga tctttgacaa accggacaaa aacaagaaat ggggaaagga 277680
ttccctattt aataaatggt gttgggaaaa ctgactagcc atatgcagaa aactgaaact 277740
ggaccccttc cttacacctt acacaaaaat taactcaaga tggattaaag acttaaatgt 277800
aagacctaaa accataaaaa ccctagaaga aaacctaggc agtacaattc aggacacagg 277860
catgggcaaa tacttcatga ctaaaatacc aaaaagcaat ggcaacaaaa gtcaaaattg 277920
acaaatggga tctaattaaa ctaaagagct tttgcacagc aaaagaaacc atcatcaggg 277980
tgaacaggca acctacagaa tgggagaaaa tttttgcaat ctatccgtct gacaaatggc 278040
taatatccag aatgatctct attttatttt ttatgacata ttaatcatgt ttgttctctt 278100
gtgtcctctc tttctttctc tcgattttcc agtggtccat ggtggtctat ttgtagggtt 278160
catatttaca aatgggagtc aaggtgactc cactacagtt atgtgaaaaa gtttccctgc 278220
catgcctctc cctacactgg gcgagctgat ctggttccgg gtcagtggat ggagcatttc 278280
ctctcgcagg cacaccttca ggctggtggg agcaacttgg gccatggact ggaaccatgt 278340
caatggagaa gacttcctct gtgccaggtc ctggtcagag ctgccgtctt gatttatttg 278400
tatccctctt tccgtctgtg gtaaagatct ggaggttctt aagctctctc tggcctcaaa 278460
acccacagca ggaaatttcc tcactcagat cacccacatt tttagcaagc agagcttcag 278520
ttttgagtgg agggcaggag tattgggcag tagggaagga cacagttctc tacgccgttc 278580
tgaatgcagt gcattaataa actctcttca cagttgctcc agggtgcttt gcattggtga 278640
acttctggct tccaggaaga acctggttac ctctgatgtg ttgggttgtt gttccatcta 278700
ctctaaattc tgtgccaatc catcccatct gtttcctacc attggtttat ttttaaaaaa 278760
tattttatat ttatgtagat ttatgaactt gattattata ccatcattag agtttgggtc 278820
cagagtcaaa cttctataaa cctgtaatca aaaattggcc tttattttca tgtagaagga 278880
agtagcagac caaaatggag ttctatattc catttttcta aatgagccat tgagatgtag 278940
agcaagcaag tgccctttttg agggaatgtg tgtgtgtgtg tgcacacatg caagggcatt 279000
ttcccatgtc tcccatcctt taattattcc agtagacagt gggccagcag caggtggtgg 279060

cataacttct ccatttgcag tgctgtggct gtggtgggat ctgaacagga ggcaaccttg 279120
gcaacagtag gaatagaatg tggagtcagc aggaagcgct tttccctccc acttacctgt 279180
cttcttgtag cagacagcag tgcgggcagc ttcggctgac ttgcttagcc tgtttccatt 279240
gtgagcataa acaccttaga cgtttttca tttcaggaca ctggagcttg atgaaattaa 279300
actacactca ttgtctcagg caaatctcct aatcctattc tgtgcttttg ttgttgttgt 279360
tgttgttgtt agtggtgtgt ttctctctcc acttcgcttt ccttttaaaa cacaacaaat 279420
tttttttttt aattttaaat gaacttattc attgcctccc tatgtgtccc ctcctttcta 279480
gtgatattgc cgctgttcat tcccagctgt tcttgattct tgtacaagac tctttgctgg 279540
gcttctgcct tctagacgct tcaccttccc gttatttctt ccctctgccc ccggaaaggc 279600
ccttaccatg gctctgatga tgatgacagc tgaccctttt ccaaggtgga atccacgttt 279660
gattctctga tcttgtactg gcttagcaca gtgctcggca ttcagtaaat aaggttaagg 279720
aaggccagtg atatagagta gtagaaaaca gcagttaatg aaggcttgct ttctgtcaca 279780
ggttcctctc ctccaggact gcagcatctc tgtctttctg tcgtagcata caagacagca 279840
tctctgtctt tctattgcaa cttccaggtg agaagccctc ccacaagcat atacaaattt 279900
tatggaaaca gacaaaaatt tgtagggaag caaatccctt caaggttgag tctttctggt 279960
cttctagaag gtaagcttgg tgatggggca gtcacagccc tagtgcttat ggaacttggc 280020
cctttgggga cagatctcag ttaactaaag aaatgctatg gcacagagta ccttgaatct 280080
cctctaattt atgctgtaca gggaattatt cacactttat tgttaataac acattacatg 280140
ggggaacacc agacagctga ccaggactcc tgtgcttgaa agcttctgct cttcagcagg 280200
gcagccagcc tcttctcggt ttagcacaga gcttttattc tcctcgctct ctctaaactc 280260
ctgttcaaga cagtttccaa ttctgttgaa ttcactcatt tacctcatct gtgcctttcc 280320
ccacctcatt ctctcccctt cagagtgccc atttctgtgt ttcaaatccc acaaggccaa 280380
gcttagatgc tattatgtct atgacattct ccacctctat cccaataaaa acctttgatt 280440
tcatcttgct gtttgtatct ttactctgtt atatttcacc cataccagag ttgtttgtgg 280500
acatgcctga tttccttcag gctgtaggca tatttgattc atttttgtac tccatgagtg 280560
cctaacatag catcttatac accatagata attaatagat gcctcttggc tgcatttgta 280620
ttaaattttt accatgtact tgtcctagct agcagagact tgggggggaaa taatgatgac 280680
tgatttttac ttatttattt attttttatt cccacaggtt tttgaggaac aggtgatgtt 280740
tggttacatg aataagttct ttagtggtga tttctgagat tttggtgcat ccatcacacg 280800
agcggtgtac actgtatcta gtttgtaatt tttttatccc tcactccttt ccctcccttt 280860
ctcccgagtc cccaaagtcc attgtatcat tcttatgcct ttacataccc atagcttagc 280920
tcccacttat gagtgagaat atatgatgta tggtttttca ttcccgagtt acttcacttt 280980

gaatagtggt ctccaattcc atccagaacc ctcaaaacca tgcaaaaccg tggaaacaaa 281040

atagcctgct cctgaatgat cattaagtca acaatgaaat caagagagaa atttaaaaat 281100

tctttgagct gaatgataat agcgatacaa cttaccaaaa cctctgggat acagcaaaag 281160

cagtgtcaag aggaaagttc atagcattaa atacctacat caaaaagtct gaaagagcac 281220

aaataggcaa tctaaggtca cacctcagag aactagggaa acaagaacaa atcaaaccca 281280

aacccagcag aaggaaagaa ataacgaaga tcagagcaga actaaatgaa attgaaacaa 281340

acaacaacaa taaaaaagat aaatgaaaca aaaggctgtt tttttgaaaa gataaataaa 281400

atggatataa cactagtgag attaaccagg aagagagagg atccaaataa gctcaattag 281460

aaacgaaaca ggagctatta taatcaatac cacagaaata caaaagatat tggaagctac 281520

tatgaacatc tttacacaca taaactagaa aacttaaagg agatggataa attcctggaa 281580

atatacaacc ctcctaggtt aagccagaaa gaattagaaa ctctaaacag accaatagca 281640

agcagcgaga ttgaaatgat aataaaaaaa attgccaaca aaaaaagtcc aggaccacac 281700

agattcacag ctaaattgta tcagacattc aaagaaaaat tgataccaat cctactgaaa 281760

ctattccaca agacagagaa agagggaatc ctccctaaat cattttatga agccagtatc 281820

accctaatac caaaaccagg aaaggacata acaaaaaaga aaactacagc ccaatatccc 281880

tgataaatat agatgcaaaa atccttaaca aaatactagc taagtgaatc caacagcata 281940

tcagaaagat aatcaactat gatcaggtgg gtttcatacc agggatgcag ggatggttta 282000

acattcacaa gtcaataaat gtgatacacc acatgaatag aattaaaaac aaaaatcaca 282060

tgagcatctc aatagatgca gaaaaagcat ttgacaaaac ctagcatcca attatgacaa 282120

tttttaaata tggggaatgg tcttcatgga aaagtagatg ttaatggcac ctgtattagg 282180

gttctctaga gggacagaac taataggata gatgtatata taaaggggag tttattaaga 282240

agtattgact cacacgatca caaggttggg ttccgcaata ggccgtctgc aagctgaaga 282300

gcaaggaagc cagtccaagt cccaaaacct caaaagcagg gaagccaaaa gtgcagcctt 282360

cagtctgtgg ttgaaggtat aagagtccca aagctgaaga acttcacgtc agaaattcga 282420

gggcaggaag catccagcat gggagaaaga tgtaggccag aagactaaac cagtctagtc 282480

tttccatgtt cttctgcctg cttttattct ggccatgctg gcagctgatt agatggtgcc 282540

cacccagatt gagggcgggt ctgcctttcc cagctcactg actcaaatgt taatctcctt 282600

tggcaacatc ctcacagata cacccaggag caatactttg catccttcaa tccaatcaag 282660

ttgacattac atattaatca tcacagcaac caaaagatta tctcatttaa tccttacaat 282720

agctctgtgt agtgggtata tattttcctt ttgctgaaga ggaagtaggc ttagaggggt 282780

tgagtaattt gcccagaata cctaggtagt agaagatagt agagccatta tattctgtct 282840

gcattcaaga gtgtgctgct ttatttgttc ctgaattgca cacaagctaa agaatacaac 282900
tggatgttca gtctactcct attttgataa cttggctaac tttactgcag agtctgcaga 282960
ggtgaccaat tttactctgg gagataccag ggaatccgcc atgtaattcc tcaggctgag 283020
tgttttggaa acacaaagct gtcactgcta ttaagtgatg ttttttcctg agagtctact 283080
atgctcccag taccagcctc cgcaggccct ctgtctaccc cttttctgtc ctgatgtgtt 283140
ctagcaggca ctgagaaagt cattgtagaa gttaccatga ctttccaaat gcttccagaa 283200
gcagaacaca atttcacagg agggccaata agtatgaatg ccaccttgat agaaagtgaa 283260
gttttgcgtc ctgttgaaca ctcagtaaat gcttcctgaa tagatgcagg attggcaaaa 283320
catagctccc cagctttcat aattcagact cagaggccct tcgtgctcct gttattcttg 283380
ttacttcatg tcaggtacct aaaagggttg tttttcagtt ttcgtcattg atttaaagca 283440
gaggttagta aactttttct gtaaaggcca ggtagttagt attttagcct ttgtgagtat 283500
ttggtctcac aaaagcagcc atagatgata tgtaaatgaa tgtgcatggc tgtgttctga 283560
taaaacttta tttaaaaatg aggggtcatc tgggctgtgg tttttcaacc cctgatttaa 283620
aacaaaaaca aaatgtgcta tgcctgttag atgctactac atttgattct gtgttttcca 283680
tatttttcta aaatgtactg aacaggaaat gaacaaactg atgacacaat gatctaattt 283740
aatcattgag ataagagtgg cgccaactct tattgagtat ttgttatgtg tcaggcactt 283800
ggctaagcac tttacatgtt ttctatgatt tataaagcaa atattgttat tatcttcatt 283860
ttttgctgag ggaaggaata aagtcacagc cagcttacag aggctgccgg aggtcacaca 283920
tcaccagtac actgaaacac gtggtctgat tacaggatcc atgatctcct gtacagatca 283980
tagtgatatt gacctgagag gaaagagcct aggacttaca atcagaaatc tgggttcaca 284040
tttcaactcc gttccatgcc aaattacatt aaaactgggc ctcagctttc ccacctgtga 284100
aagaggaata atactaggac ttgccctccc aaccttagag attgctgtga ggataaaagc 284160
ctatatgaat gtgctttata aactataaac accatacaaa tgtttgttat aagtctgcca 284220
tttgaataca tcttagtctt tttttcctat gtgaaataaa acattttaag acaagcaatg 284280
aaattcttga aactataaca ggtttttaag ggtgttgatc cccaaagccg tcttcactat 284340
tttgctccct ctctgactcg ctctgtctct gcctagcagc gctagtcttt tctacgtgtt 284400
tgatctgttg ttcctgtgtt taggcccatc cctactacac agcggaagtc agctattttt 284460
tctctttcct ttgctctgct gagcaagatt aaactctttt cacctggact tttcctagat 284520
tactcaggct gggtggaaat ggggtagctg tgaggctggg atgttactgt acaattcaaa 284580
tttatgatct gaacttaact agatcttgct gaagattgac ctggctagca cagttttgag 284640
gcacctatct ttcagtccct tctgtcctct tggcctggcc actggccaca tgttagtagc 284700
ttacttctaa ggatggaatg ccatgcatct cctacaaggt tgtctctatg cttcacacaa 284760

agtaaaagaa tggaggcaac tttgaatgct gctcattttc actcaggcaa tccctttaca 284820
atattgtgct tagtaaactc cccccactct ttttttccct tagcttgttt tttttttcta 284880
ataaaagtct atatcaattt gatgtattag ttagggttct ctggagaaac agaaccagta 284940
ggatcaattg attggtcaat caatcaatca agacaaaaag agattcatct ggaaggagtt 285000
gattcatgcc attgcggtca ttggctagtc tgaaatctgt aaggcaggct ggctgactgg 285060
aagctcaggc gggatttctc tgctgcagtc tcaaggcaga attccgtcct cttggggaaa 285120
cctcagtctt tgctcttaaa gctttcaatt gattggatga ggcctacaca cattatggag 285180
aataatctgt tttacttaaa gtctactgat tgtaaacatt aattatattt aaaaagtact 285240
ttcacagcaa catttagact agtgtttgac caacaactgg gcaccacggt caagccaagt 285300
tggcatatag aattagccag caaacttggt attcacaatc tgtcattata gtcttatcta 285360
taaacctaag gtttagagaa acgctggtca ttctgaacaa ttccctgtga atgcagagat 285420
caaggagcct ccccttaact gggtaggcag tgtgaaatac ctgcagtgta cttgacctta 285480
ctgttcaccc actggcagct gttcctacag cttggcttca gatatataat attacattgg 285540
ctaacttcaa agagcattgt tttccaagga tagagatggt gatggtgtat attactttta 285600
ccagccccag acctgtaaga ggttctcatc tcttttgttt aagattttgt tgtttttttt 285660
taatgctcaa taaaagaagc tttattcttt cattaaaaaa caaatctcag aagaacaggg 285720
aagaagaggg tagcctatga aagtgtgatt gttatttgat accatcgtga cttagcttgt 285780
tttcacctaa gactctgaga ttctattctg tttatgtgag atttgggctg agttcaacta 285840
ctatccatat taactgaatc ataaaacaat taatccatca tctggttaca ttttggtggg 285900
gagggagcat ttaacacaac actaagccac atatttcact attttttttt tttgcagcaa 285960
attgacttaa ctgcttgctt tcacaccatg cctataaaac ctttttggtt ttagaggttc 286020
tatgtgtctg gaaagtacag tgaagtatct catcagaata gtattaatat ttcttatgac 286080
atttcatatg tcctgatatg ggttaatgga gaaatatcat aagagtagta attctcttgc 286140
tgttattatt tgtcctattt agacaaacag aacatagaat taataagaat tcaaaaactt 286200
taatcactga tagagattaa atgatgttta tattagtcat catcacctaa tatcttaaat 286260
atttaacctt ttatgcagtg tttgcctctc tacttgggta tgcccttaac agtaaaaagt 286320
gttgataatg ctctcccttt tggcactcac agaattgcta tacatatata tgtataaaat 286380
atatgtaaat ataaaaatac atagttaaaa aaataaatct accactaaat atagtaaggg 286440
agttttaaac tgaggttttt gaagtcttta agaatttatt tagagacttc ttttgttgga 286500
agtatggtgg ctagatgcca tgaaaaaccc actgaaaaca agtgtaagtg ctaggtaata 286560
tgttgaaaca tatcttttga aatgtattac tgagctggca agaaagtatg ccattgtaga 286620

gagtgaaaat aagtgaaggc agaatcctgg gaggtaagcc agcctgaaga tattgatgac 286680
actggatggc cttaagtttc cattttgact ggcatgtaat ccagccaaag attcccacag 286740
aaatccgagg ttccaaatgg taaatccgtg gtgacattgg ggtgaccgag aaatgaactc 286800
tgtgcagaaa ggaatggtaa caacacttgt ctcgtacaac tttggctctg ggtaggggaa 286860
aggaaaacag cttcctagag aaatgttaac cacaagctag ccctcatgag agatttcagc 286920
taggatggat gctatctgtg tagtgcaaaa aaaaaaaaaa aaaatccata gccagatttt 286980
gtgctttaat gtggacctag gtcaatagtg actgcaaagc cagaagcagt ggctcacccc 287040
tgtaatccca gcactttggg aggccgaagg aggtggatcg cttgaggtca ggagttcgag 287100
accagcctgg ccaacatggt gaaaccctat atctactaaa aatacaaaaa tgagcctaga 287160
gtggtggtgg gtgcctgtat tcccagctac ttgggaggct gaggcacaag aattgcttga 287220
acccaggagg cggaggttgc agtgagccaa aatcgcacca ctgcattcta gcctgggcaa 287280
cagagtgaga caccatctca aaaataataa taataaaaaa aagtgactgc aggggactgg 287340
gaagaggaaa cacaaatctt tactggggaa tggaatttca agtgtatgca ccaaccctta 287400
gaaatcacaa aacatctgac ccttctgaaa ataaccacca tgagtgagac agcaggaaaa 287460
aaaaaaagaa aaaaaaaaaa aaaacaagga aagagaggga caatagaatc agactcatac 287520
ctacaaaact taaaaattaa ataaaatgaa tcagactcag aggcttcaga gtttgattta 287580
aatacattat acaaaagttc tgtgtgaaat atgtttaaga tgtagaaggg tgcttgaaat 287640
gcacaaagtc aacagctgaa aaattgacca agcagatatg aaaataaaat ccaatagagg 287700
gcatctggaa ataaaaaagt ataataatta tagtgaaaaa agttattaga tgggttaatt 287760
agtagaatgg atatagcaga agaaagattt agtcaactga aaaatatatc caaagaatag 287820
gtccagaatt aagcttaggg agataaagag atggtcaata cgaacattag gagacatgga 287880
gaacagtgta cgaaggtgta ataaattcta actggagtta aagaggagat agtaaagagg 287940
aagaaaaaac acaaaactca aaaaatgtag tggctgagaa tcttccagaa gtattgaaag 288000
acaccaatcc acagattcag aaacctacaa tttgcaagaa aaagtaaaaa gaatttcaca 288060
ccaagatcca tcttcatgga ctggcagagc ataagagatg aagagcttat cttgaaaatg 288120
cagccagaga gaaaaggtct atcgcagtta aaggaagagg caagcagatg actgagttct 288180
caatgccaac tgaaaatgag aagccagtgg cacaccttca atatgttggg agaatataat 288240
tttcaactta aaatagtgta cattgtaaaa ttacatttca aaaaaagagg gtagtataat 288300
gacattatca aataaaaacc atcccttata acaaatatca ctaaagaaac tgttggagga 288360
tgtagtttag gtttctagaa aggatgtctg atatgcaaga aagaatggta agtcaaatat 288420
tgagaaatat gtgaggaaat agaaacaaga ataactaatg aatcaatgat actgtctaat 288480
ttgaaggtta aatgattagg tagaactaaa atatgaaaca ataatagtat gtaagtcggg 288540

aggaggagga tgaagcttga agtgctctat gcatcttgca tctctggtgt ctttcttgtg 288600
tccaaacttt ttcttctttc aaggacacca gttagactag attagggtcc acctaatgac 288660
ctcattttaa cttatttgcc tctttaaagg ttctatctcc aaatacggtc ccattttgag 288720
gcactagggg ttagggtttc aacgtataaa ttttgggggg acacattcca gccgatagca 288780
attataaata caacttttat agtaaaagcc catcttctag aacaggtaga gaacaaggtt 288840
acttattttt tttaagaaca aaattggttt aaattatcct aaaatgattt taggagaaaa 288900
tggacattaa atatacaaca tttcctcctt tattatttta ttgatgactt gatatgtttt 288960
ttaaaactca gtaaaataaa tgattagtct cataatcata agagagttat gcccttattt 289020
taatgccata aaactgtgac ttctgaagaa atacaatcat ataaaatcat attttaattt 289080
acttttgaga atgtacagta actcaggaag gcagagtgtg gaacctgctt taatgaatag 289140
ataagaatga ctagcaatta aaaattaatt atctataatt cttctgaagt tcttgaaaat 289200
aatctcttct aacctgggta aatattcctc cttttcagaa atgtttgcaa tgttgctttt 289260
ttttaaatta atctcttttt cttagataat ggaacataaa tcttaggcac atttgctttg 289320
taaataccaa attgcacaca gattaaaatt taatgagctt ttactgctgt aatctcaaca 289380
agcagagtct ttgctgttgg tttttcaata agaaatactt tattttgtta tacagtggtc 289440
attaagaacc ttaaagataa tcaggacaga ttttccaaat tgcataatgc tattgcctag 289500
ctatttgcat gtattttttt taaatgacaa gacatttcgt taaatattta cttgcctgac 289560
gttgaaagat ttagacaaac aatgtacttc tagttgtcac tttttcaaaa agattcttag 289620
ggaaaagtta gccgttttgt tggttggtaa gtgatgggtt ttcttggaac gtgttgcttg 289680
ctaattttac cagagaagaa aataaattca aatggtaagc tacattccaa tttgtattct 289740
tcttctaaag tcctccctgt ttgcattctc ggattttgac actcctacac accagttaaa 289800
gcacttgtta gagtgtccaa aaaattgttg caattaattt cccagtctct tctctctttg 289860
atgtgtcact ataatgcttt gagtaatttt cagtgtctgg catctgttca aggacaattt 289920
ctggatttta ggagggggag aagcaataaa ggaaacaaca tatgtttttt cctaaatagg 289980
aggcattttt tgtttgtttg tttgaacaaa aatcaaaaac tttcttttct atataggtga 290040
caaaaagata cacgactcat tggttgttaa ctaccattgc attcttttct ctttggagta 290100
gcccttaagg accagattag accagccatc tgcatcatgt agcatgtctg ggttaccttt 290160
cccaaaagaa gttcagatag atgaactttg caaaccagag ccatctgaag tttgtttttt 290220
ctttttgaga cagagtctca ttctgttgcc caggctggag tgctgtggtg caatctcagc 290280
tcaatgcaac ctccgcctcc cgggttcaag cgattctcct gcctcaacct cccaagcagc 290340
tgggactaca ggcacgtgcc accacgtcca gctaattttt gtatttttag tagagacagg 290400

gtttcagcat attggccagg ctggtctcaa actcctgacc ttgtggtcca cccgttttgg 290460
cctctcaaag tgctgggatt ataggcataa gccaccacgc ccagtccatc tgaagtttta 290520
aaatggctgc aggtatctta gtaggagact aattttattt tccaatgatt agaaaaaaat 290580
gaccagacca agaactctag ggttatgttt catctcatat cctgtgcagg atgttctgag 290640
aagtttctat ttgtatctgt ggagtggttc tagtcttgtt catcttgcat tttaaggttt 290700
ggaggtattt tatcttgcct tgcttcttta ccaccttgct ttaatttccc cctatattgc 290760
catttctaaa gtaatgactc attttcaagg gtgggtctga aaggaaagaa ataataaaac 290820
cagagatctg gagaagtttc tcgttcatgg tcttattcac tttttaataa gagcatctag 290880
aacagtgagt ggtgccccag cagatatctg gaaatttttg tggaattgaa ttgaatttgt 290940
tcttagagta aatgacaata aagaaaatag tattatggaa actttcgacc caagggaaat 291000
atcaaggcta aagttgctat attatttttc ttctgatgga tgctgagttt ttctccttaa 291060
aaaacaaatc cacatttaaa cataaggcat tgtttgatgg cctatcacat tcagtagaat 291120
ggatttgaga aatacgtgta ggatgcctat taaaaactct gttttgaaat taaacctaag 291180
actataagat tgtctgttta aaaaaattat tgctgctgta atttgagcaa atctgaacta 291240
tattgaactt tagaaattca aatgtatgca atcttgtaat ataacttact ccatcggtag 291300
acatagttct ttgtcccacc tggcttccag cagtttgtct gtctgtcatt aggctaaact 291360
cttcatgttc ctaaacatgt tttcctcaaa aggggacata tttcttgggt cagatgtaaa 291420
catcaagcta aggagttctg ctgtgcgtct agacaaataa ggttcatttt catcctctac 291480
atagtgttaa acttgaattt aagatcacac agattccttt acacgttacc aatgtaatga 291540
tatagacaat ctcactttat tcattcactc tgtttaattc attcaataaa aacgtataca 291600
ttggctgcct actgtgtgcc agttggtgaa gatataaaga tgattaaggc aagggtctgt 291660
gctgacatgc ctaatgggca ggctgacaca cctaaacgcc agggaaaaag agggatgaga 291720
cctgggcctc tgaagccctg gccacctggc tcgaagctca ctttaatgtg cttctgggtg 291780
tattccatga ctttttaaag gcgcttatgt ttcctttctg gtaaagcatg tttaacatgc 291840
aaaatgctta tttcattgag tagcttttaa aaagtgttca tagaatgtct agaacagtgc 291900
ttgtctaata gaagtataat tcaggtcacg taggtaatat taaattttct agtagccaca 291960
ttaaaaagta aaaagcaaca ggcaaagtta atattttttt ttttcctgag atggagtctt 292020
gctttgtcac ccaggctgga atgcagtggt gtgatctcgg ctcactgcaa gctccgcctc 292080
ccgggttcaa gccagtctcc tgcctcagcc tcctgagtag ctgggactat aggcgcccgc 292140
caccacacct ggctaatttt ttgtattttt agtagagacg gggtttcacc gtgttatcca 292200
ggatggtcgt taattttaat aatatattca ttaattttaa taatatattc atttagtcct 292260
atataaaaaa attagcattt caacatgtaa tcaatagaat atattattag tcaactattt 292320

tgcattcttt gttttgtacc atctttgaat ttcaatttgt attttatact tacagtgcat 292380

ctcaatttta atgctaaatg tcatcggaga tattcgatct ctgtttagct ttaatatatt 292440

tttgggttga aaaagtaaat tcacataacc aagttcttcc aaatatactt gaaagtgttt 292500

ctgataactg agttatcaac ttaaaaatgt aagttaatga caataaatga aagaaaaatt 292560

ctgttcctta gctgcactga ccacatttta gtgttcaata gtcgcaggtg gccagtggct 292620

atcatattgg atagtacagc tttagaatga cagttcagtg caacaagtgc tataatagac 292680

tcatgaactg gaagctccgg gaagttaaga atgggatcaa tgaagtgggt cttgaaaaac 292740

caataaaaat tcaccacgta gagaggagga gggcgactac tccaattcaa tcttttgaaa 292800

gcatgtgaag gagcaacagt agacttcttt aattatttaa tagtaattta ttgaacatga 292860

attatgggag gtgcatggtg tgggtgctgg tgagacatag ttcctgagct caagtagctt 292920

ggtgtctaaa tattcatggg cccatttttc agaaaggatg gatatatgtg tgatcctggg 292980

tgtgccaaat gctgtggctt cctgaagctt agatttccag cttgtcacct tcaaggttac 293040

cttgtgaata ggactttttt gagctgtaag taaatttact ttgcctattt atttccaatg 293100

gaaaaaaagc tttttttaaaa aataaatctc atcttattgc ctatgattgg ccaagacaac 293160

atggcccata cagaaggttt ttgatggctt ctgaggtctg ttatcatttg cttattggca 293220

tttcagctgt caaccagggt tctgtcaaat tccattcctg cctttagctc ttttacttga 293280

atacctgggg caatggcaga agtggttgct atttgtcacc tttccaggat gttagtcgtg 293340

tccttgagac aaatagaaaa tttaaagtca gatgacttga ttcctctgcc agttaagacc 293400

cttagagagt cctcagaatg ttgcttattt ttaaatttca agtccctggt aaggatcaag 293460

taaactcccc aatttgcaga tttctatcca gttgactatg gattttgcct gttgctttgt 293520

ttccaccaac tctccctgaa gatgaggcgc acagacagac aactcacagg caagaacagc 293580

ctggtccatc ttgaaagatt ctcaagacta ttctccacaa gataattgtc tacttttaaa 293640

aatattcagt aaagggaatt tttgctgtta tccttggttg tgtttttagt atctcatcat 293700

ccttctagtt gtaggaggct tttcctaaca tctaacccat atgtctgttg tctcatcagg 293760

tgtttctatt aaggctactt ccccatcaat cttaattttt tttttaatct tctgagatgt 293820

ataggttaag ttgaaatcag agactttcat aaaatggtaa gatggccatt taaacgcaac 293880

tatgaggaaa taatgtaagc aggattccat tggagaacca aacactagca acaactaact 293940

tggtaatcaa tgttgggact tgaagttagg ctaaaatcaa tagtaatggc actcgtatgt 294000

acaaatgcag aactttttac acacaatgat ttttccctct gttattatac actagctgtg 294060

tctgagtaga cagtccagct ccactacctg cagtccaccc tgggctgtgt aatctgagca 294120

ctgatgggct gttatttgta cgtattactt ctatgacact gttttttcat ctgtgttaga 294180

aatgtctttc tttcttgaat aagctgcttc aatttcttat agacagattc tgcttttatg 294240
acccttgttt ctacaaagta atctctactc atgctgaaat ctcagacaat tttaacaaat 294300
atttagagta cttaattttt ctttgaattc taaatattat ccttctttag ttcaccatag 294360
ttggatattt tgagataact gtggaggaaa cagcactgat cctggggcca tgaaacctga 294420
tttcaagatc tagtcccttc tttaattttg cacaactaat ttaatcacat tcatccttaa 294480
tttcatcatc attaaaatag cagagaataa ttcccgtttc atggagttga tatgcaatga 294540
aatagcatat caatgtatgt atgaatgtcc cccaacatag agtactacac aaatgaaacg 294600
tgtcactcag cataacgtta tgtgctcctt cctgcaactg gattgcactc cagtgggatt 294660
tcatgctggt gagatggctg tgctgctgga cttcatggga caccaatctt tgaaaataag 294720
cctgatctgg ctgggcgtgg tggcttatgc ctgtaatccc agcactgtgg gaggctgaga 294780
cgggcggatc acctgaagtc aggagtttga gaccagcctg gccaacatgg tgaaatccta 294840
tctctactaa aaatacaaaa aaattagctg ggcatggtgg catgtgcctg taatcccagc 294900
tacttgggag gctgaggcag gagaatcact tgaacccagg agatgaagac tgtagtgagc 294960
cgagattgcg ccactgcact ctagctgggg caacagagtg agactctgtc tcaaaaaaaa 295020
aaaaaaaaaa aaaaaaaaca caaacaaaaa aaaaaaagaa aaagaaaata aacctgatct 295080
atagatgagg ccagtggatc ttgtgaagag ttgaaaggtc aggtatcagt cttataccat 295140
gtcggatggg gcaatcgtca taactttta aaaaattatt tatttatttt gagacagtgt 295200
ctcacgctgt cccccaggct ggagtgcagt ggcatgtttt cggctcactg caacctccgc 295260
ctcctgcgtt caagcgattc tcatgcctcg gcctccctag taactggatt acaggtgtgt 295320
gccaccatcc ctggctaatt ttttgtattt ttagtataga cagggtttca ccacgttggc 295380
ccaggctggt ctcgaactcc tgacctcagg tgatccacct gcctcagcct cccaaagtgc 295440
tgggattaca ggtgtgagcc accgtgcctg ccccaattgt tacagctttt taagtggatt 295500
atatgcctgc ttcaggcatt agacataata agttaatctg ttgataagtt tcttcatttc 295560
ttaattttca gttgtgtttt gatgtgtgtg gtctgggtga gcggctcagt gctgctgctg 295620
tggagaaggc gtgatatgga agggatgtga ctgtccttct tcatcttcat aacggggcca 295680
ggactggggt gaggtgtgtg agatgaatga agcactggtt ttgatacaaa atttaagaaa 295740
aatcaaattc aagaggtaat attttaatac gatatttgta aaaaatcaaa attagtgcaa 295800
aaatttgtga tgagcaaaat gttagataaa ggcattgttg cttttctttt tctgtgtatt 295860
gtaacacacc atcacttagt ggagaaacag ctctcacttt cctcctcccc agatacaacc 295920
atttatgttg aattaaactt ctagttctcc tatacctgtg agcatccccc aggccccttg 295980
gtatggagaa agcagtttat ttgtcttagt aagattataa aagtggcaat aaaatgtgaa 296040
tgtaatgaac aaaatgcaca ttttgtggac caacttgttg ttttagatct atttttgaaa 296100

actcattagg tatgtgcatg aggcaaaata attcaagtgc agttggatat taaatgaaaa 296160

agtgtcaggt cccctttct gccatattct cttctgtgtc ttcattagtg gaaaccattt 296220

tgaccagttc ttataattct aatgatttgc tgatggctct aattcttggt ttatcaactt 296280

caagcagaat gtcttgacat cttggtatga aagttaaaga atatgaatca tttgttctct 296340

cccttctcct ttcctctttc tctgcctctc agtttttgtt gtatgctcac ggagtatgtg 296400

tgtgagcatg tgtgtgtgtg tgtgtgcacg cgagcacgtg tgtgtgtgtg cgcgcccatg 296460

ggtccatttt tgcatggcag gctttgtttt agaaagattg gttacaacag ctgccccacc 296520

aatctcctct acagggaagg attcctactc tgagaccttg gtcttttcca ggcagatcac 296580

ttacatttcc tcacagatta tttctcatcc tgcagcctgg gtccaaatgg gctttgctgt 296640

gaacagtgac tctcaggctt ctggtctctg ctgtgcttga gtgtcgttga gatgctccat 296700

ctcctcctac tgcacccacc cagcaatcat ctctctttca ggaattcacc aaagtctttt 296760

ttaaaattat tcttttgttt tgagacagag tcttgctctg ttgcccaggc tggagtacag 296820

tggcgcaatc tccgctcgct gcaacctctg cctcctgggt tcaagtgatt ctcctgcctc 296880

agcctcctga ctggcaggga ctacaggcat gtgtcaccac acccagctaa tttttgtatt 296940

tttagtagag atggtgtttc accatattgg ccaggctggt ctcaaactcc tgacctcagg 297000

tgatctgccg ggtccattt tacgtggcag actttatttt agaaagatag gttacgacag 297060

ttgctccacc catcccccct gcagggaagg agtcctactc tgagaccttg gtcttttcca 297120

ggcagatcac ttacctttcc tcacagatta tttctcatcc tgcagcctgg gtccaaagtg 297180

ctgggattac aagcgtaagc caccacatcc ggctcaccaa agtctttggg tggttgatgt 297240

catatgcctc cagttgatag cacttttaag aatttttcct ttttgtatgc tgttattatt 297300

tttagaaggc ttttggatta ttattgttaa agagtatgcc atgtctatct tttaactttt 297360

aagcatctat tgctttgtta cttgtacttt atatatatgt atatccttcc cccaataaga 297420

caaagagtgc aaataatcac ctcccttgcc tgttgtttgg gtttaattca ggagtcagtg 297480

gaattaaaag cctaaactca gtatagtttt aaagcagcag tccccaaccc ttttggcacc 297540

agggactggc tttgtggaag acagtttttc catggaccag tgtgggggct ggaaggtgat 297600

tccaggatga ttcaggtaca ttacattaat tgtgcacttt atttctatta ttattacatt 297660

gcaatacata aggaagtaat tatacaactt accataacat agaatcaatg ggagccttga 297720

gcttcttttc ctgcaactag acggtcccat ctgggggtga tgggagacag tgacagatta 297780

tcaggcatta gattatcata aggagtgcac aacctagatc ccttgtgtgc acagttcaca 297840

gtaggattcg tgctcctatg agaatctaat gctgccactg atctgacagg aggtggagct 297900

caggcagtaa tgcgagcaat gggagtggct gtaaatacag atgaagcttc acttgcacgt 297960

tcactgctca cctcctgttg ggcggcccag ttcctaacca gggttgggga cccctgcttt 298020
aaagaatatg tttgtggatt catagaggga aacaacacac actggggcct ttcggagggt 298080
ggagggtggg aggaggcaga tgatcagaaa aaatattaat aactaatgtg tactaggctt 298140
aatatctggg tgttgaaata atctatacaa caaacccccca tgacacaagt ttacctatgt 298200
gagaaacctg cacgtgtact ccagagctta aaataaatgt taaaaaattc cctccaaaaa 298260
ggatatattt gcatcggagt tatatttgta tatgagtata tatttgtata tataaatata 298320
cttgtatatg aaaaaaatat cttttttctt tcattttatt ttccaaacat taaagtcggg 298380
cacattggtt aggaatttca ccaatacttt ttacaaaact gaggacttgg atgataaagg 298440
cactttttaa aagattacac actggcatgg ggaataatac ctttataaag atgatctatg 298500
tgttcctgaa tatcccgatg catctcctag ctggatcttc cgctccacaa aaattcataa 298560
gatgagattc ttgaattaga atgcatgcct taatgtatta agtctatgta gagagagtta 298620
tttagtccat gtaaattaag tgggaatatt tttatttgat tactgtttta tctggatctt 298680
gcccacttaa ggccttcaaa aatgaaattt aaggcctgtt aagcagaccc aacatcaaca 298740
gcatattctc tctctcttgt aagtattgtc tagttgataa aaaatttcaa aaacatgtct 298800
taatcaaaaa caagatgatc cagcacaggt ttaataaatg ttttgtgaat atggcacatt 298860
cgtgtctcat tactacagtt ttcctatgct gtctttctca ataattcccc ccaaaattgt 298920
agtgtttaca ttatggcatt tatagttacc tctgaaccta aaaaattaac ttcaaaagta 298980
tttaaaaaaa tcattatatt taaaaccatt tcatgtttaa atggtttgta cagggcaatg 299040
aaaggaaatg tagtaataaa cacagaatca gatttggtca ctaatatttt tctgcagttg 299100
aaatatatgt agactggctt agggtctaaa tagcattgaa tcctgttacc ttccatctat 299160
aatcaattaa tgtattatga cattgttgtc ctcagatcac tgggatctca tggtaagtaa 299220
gtaaagggat tattctgtgc attttcccaa tatctatatt agttttaata taccttttatg 299280
ttaatgtatg acactgacac ttagtaattg gattaacttc ctatcagaat ttgtttttca 299340
ctcatacttt gtatacatgt cttaagggta gggtagatgt acattttttt ctgtgttagc 299400
ctatctgttt ctgtgacact acatgctttc tgtcctccaa tttgtgtttc tttccgtgta 299460
caaatatgca tcatctacca tctacatcta caaaacatgt aggcttccaa tgttttcatg 299520
taaatacatt tcatgagtcc tcagtagagt gttagatgag tggttaatta gtaatgttaa 299580
taaatgtaaa ttacaataaa aacatttata atgttaataa atataagttt attgaactta 299640
aataatttaa acatcttctg aaatgtagta gaaattatga ttagccataa ctagtacagt 299700
attttttgct tgtttaattt ttttggtgat attccatttg tggaatgagt gtgtgtgtgt 299760
gtgtgcagta cagacagaaa ggagagaaac atatctgcat gttatgttag aaagagtagt 299820
gttagctacc ctaccaaatt tacatactgg ctgaacacag tatgtaaaaa attatttttt 299880

tagtccattg ctggtgttcc ttgttgttgg gtgactctat tccacatggt gatgcaggga 299940
accaatctcc ttccttctgt ggctgtgtaa tcccttgcaa ccttgaagtc ctctgcatct 300000
aactttagga tgaggaaagt gaaggtggag gatatagccc tcttgactgc cttgacctgg 300060
acctggaaca tctcttttgc tcccattcca tggggaagaa ctagtgacat ggtggcccat 300120
agctgcagag gggaggctga gaaatgcagc ctctggataa gaagcctact cccagtggtt 300180
actgtacatt gtgggaggga agcttgaatt ctgatggaca gtgagaacca ttctccacac 300240
aataacgaaa tgacgtcttc tcaaccttgg caatgtagca tcctgtgact taagtatgta 300300
aataattatc agtacaggtc agtgaaaaat taggtcacct ttcccttcca cttttaattc 300360
tatagtttaa tttgcctatc tgctacatat tatatatatg ggaatagata agattatcta 300420
taaacatgta taaatttgtt tcctacacta aaaagaaaaa tgacaaagaa gaattaagtt 300480
agttgcccaa atccacacaa taccaccagg agaaagtcta ggaaccatac cttctaaatc 300540
caggtctaca cttctttagt gcactaaatt ttttcctaat atagctctga ggagactggt 300600
gtttaagagg agaatgttaa gccaaaagcc catttcactt ggctctgtac agacagtaag 300660
ttaatttcca ggatgtataa ctcctgattt tctgtgatga cagagaaaat actgacctga 300720
tttgggtaac tctgaggttt ggagatcttg aatagcctca tgttcctgaa cttctttacc 300780
atacatgaat tacttcttag gaagaaacat ttattctgta gaatttggtt tcgcatttta 300840
tttttatttt cttagacact ggtagttgga aattcaggaa gaatttgact acaaaaaaca 300900
tttattaaaa agagtttcta atctcacctg ctaagtgcta caaggaaaaa aaaaaataca 300960
ttttgagccc tcaaggggct ccaaatacgg ttgaaggaaa attagaccca gaatagtata 301020
tgattaggaa caaaacgatg gcagagaaat gagagtttag agaaaggaga gataatcctg 301080
agttacctta ggaagagaaa atttcacaga gaaaacggcc cccacactga tttggggaag 301140
atgattcaga cttgtctcaa tggcatgagg agcaggggcc agatgggaaa ggacatcgtg 301200
gtggaaatga cacgatgagc agaagcttag aagtacttct gcttcacgtc tgtgattctg 301260
cctctcccct tgtttctgca gtgctaagtt gagtgtagtt ccccacacaa accctgagct 301320
ttcttcccct ccaggctgct ccatcagtca gacaggagtg ccttctcagg ggatcctctt 301380
cctcatctta tcgggccact catctcgtcg cagacattcc catactcctg gttgacttgc 301440
tcttccctct ttttaactga catgagcagg catcacctct tccaggaaac ttccctgaca 301500
ccaggctgag tctctctggt gtcggggaag gcttctgtat tactgcatct gccattttga 301560
tttaaggttt tctgctcatt ccaactcaac agtgagctct ttgagggcag aaattgtgtc 301620
ttattcattt ttcttgttat gccctaaaaa tgtattgtga taatatataa gtaacgtaca 301680
atttaccatt ttcacctctt gaagtgtata gctcagtggc gttaagtgca ttcacactgt 301740

tgtgcaacca ttatcaccat tcatctccag aactttctca tcatcccagg caaactctgt 301800

atccattaca caatagctct ccatttcctc ctgccctcag cctcaggaaa cctcccatct 301860

actttctctt tctacgcgtt taactactca tatagatgga atcatccaac atttttcctt 301920

ttgtgcctgg cttatttcac ttgacataat gttttcaagg ttcttttatg ttttcttatg 301980

cgtttttata tctctctatc tttgatagca cccaatacat agaagacaat gaatgttttt 302040

gtgagtgaaa acataagacc agaaagaaat aggatgtcat attctaggga cactcaagac 302100

actttcttga ggacagaggc actgtagctt ggaatttgga agatgaggtt ggcaaggtgg 302160

gtggggaaag gatggaaaag tgatacgttt cttgaagcat gtggatttgc ttccataagc 302220

agtggtggac cagtgggcac ccttatctat taaaagaatg atttttttg tgactcgtgt 302280

agagcactgt cttagttacc taccccagtg gtagataaga gaagcggatt aaaaagagat 302340

ctgaacctga ggaaatggag actaccaagt tttttgtaaa tatgtctgtt ataacatata 302400

ttatctagta cttgcgatgc ctgtgccaaa gcatgctttt tggtttagtt aagcctactt 302460

agctcgctaa tttcagtaat tttggcttga attgcaaaaa gttgatgggg gagatggggg 302520

agtctctgac atccttcccc cactcacagg tttgaaaaat aaaattatga agagcaaaag 302580

gatctttttc tgtggtaaat tgtacttcat gacaataaac gagttgtggt tttagtggtt 302640

aatttataaa ttaggccatc tggttcttct gtaatctaaa gatttttata tatatgtaaa 302700

atacaaagtt gtcctgggca actgccaatc tataaaggag aaaacatctt gaactctgtg 302760

gagataataa aaccatattc agctgccaaa gtgctgctga catactgtcc aatgagacac 302820

ccaacgcctt tgcattacag ccagcaaggt gtggagctag atgtattgat ctatttaata 302880

atattagctt ctcttctctg ggtcggactt ggcccagagc aagtgaacag gacctatcta 302940

tctccttctc aaaagtcaca gctttcttac caaattgtac aactcaagcc acaaagaaaa 303000

caagtcaagg aaaggaccag aggagcacga gggcacatgc cttgttccca gaccctggga 303060

ccagctccag ctctggctcc ttgcagatcc cgctcttctc aagttctctg gctttgatcc 303120

tagttttctg ccctactatt cctgacatgt acagcactta gcaattcata acttgcttta 303180

ttaaccaata tctcaacaga acttttaaac aacctgggag gaagcagagt gggtgttact 303240

gtcctcattg cacagataat gaaactgagg ttcagaaagg ttgagtgtct tgcccaaggc 303300

cacacggctt cttagtggag aagccaggat ctgcaccaat gtcttccatt ggacacactg 303360

ccacttaata tggtacttag tttttctttc tagatgaagc agtatgagga attctaccat 303420

ggatatctct ctctattttt ttaaactgca aaataatgat aatgataata atgaatactt 303480

ctagaataca gtctctatgc caggcactat atgctttata tgtactaact tgcttaattc 303540

tcctaacaac cctagaaggt agttactaat attatcacta tttttaaatg gggaaactga 303600

gttacagaga tggtaaataa gttgcccaag gtcacagagg cagccagatt agtgacaaaa 303660

gtttgagagt actcagtttg aattttacat gttccgtgga tatggtttat gccaaatacc 303720
ttctcctttc tagacctcag tttcctcatg tatcaaacaa gtggactgca ctatacaact 303780
ctgaggtccc ttctagcctg aagattagag tcttttatgc ccacatgata tggaaaacat 303840
taatgctggc tctttaagac tggaaccttg tcatttaaaa aaataagctc tactaaattt 303900
gcagtaaact gtccaaatac gatctctggt ctctgatttg tatctctcac agagcatgat 303960
acaatgctag acacatatat actcattaaa aacttgctga attaaattgt aaaacattcc 304020
tccagataga ccgtaagggt gagctgatgc cctaatatgc tcctcagctg ctgacttagg 304080
acccgtgggt gacaaaccct gtccttgttg ttccatctgt gacagtgggc agtgtccaaa 304140
atgagtctct tcatcaaaag tgagtccagg aaactggatc agagtaagta aggagtcttc 304200
cttgcttctt tttctctaaa atacaataca gtgcattcta gcaaactacc agcatgccta 304260
taaatctcat tttaaaaata tcaggaaata gatgctcatt gataaagagg gtaagaatga 304320
gcaagggaga gagtcaagcc tgtacatttg tgcttagggt attttcattt gagaaattat 304380
cattggaaag atcaatttcc aaggcaggcg ttttgtcttg acttaataaa ctaatcttta 304440
tcaataataa ttaaataatg tcattttccc agatacaaag gggaaaccat aatgcctttc 304500
atatgccttc tcatatcttc agtgtttctc aaacccagct gtggggtctt tttagggaag 304560
atacaaggag ggtgagacca tgtggagcac acactcagca gccactgtga agtcatgggt 304620
cccttgctac ctttggaagc tgacaatctg catgaaaagc agcccctagg aacatgggta 304680
gtggcccggg atccaaaact cctgaatcca taaaagggct tttgacttct tgttccagga 304740
caagtgacat ctgacatgct tgcctgcttg tttgcctttc ttccttctcc tatccgtctt 304800
tccatccatc tgtccatcag tctatctgtc catccagtca tccatccatc cttccatcca 304860
ttcgtctatt catccatccg tccttccatc catccatcca agatttattg agcacttaca 304920
tgacaggaag tatgagacac aaaagaaaat tctacacaat tctcctaggg acatgaacta 304980
atggttagca aaaatatatg ggaagtcctg cttcaagtaa aattatgact taagatatac 305040
atcagatatt tgaaaaagat atttttaagt tcaacattga aattctaact acattgtgtc 305100
cttaatttct attttctagt tagacttttt tgaagataca aatcatttga attatcatta 305160
ttattgttac ttgattttat ttattccaca tgaatggtaa gaattttaaa ggaaggtggg 305220
tgccctaaaa atacatttat gccaactgga ttgaatcacc ttgaatcaca atgagtacaa 305280
caactgctaa tgagctaaca cttaatgagt aatgaccagg caccagacac tgttctagca 305340
ctttatactt actgatttct ttactcttca tgataaccct atgaggaaag tgcttttttg 305400
ttaagctcca ttttcagctg aagaagctga ggcctggaga agttacatag ctttctcatg 305460
gtgtctccac tatgaagtag ctgagctgag atctcaccca aggccctgct gggttcaggc 305520

cacactctta agtactcaca gctgcatcac actgaatatc atctccctgc actgccattt 305580
catttgagca gggctttgcc tgtcaagaac attcatatct tttgataaac agtgcatttt 305640
ttccctgata gtctgtatgg gacagcctcc ttgtttgcat ctgaaggaca gggacttcag 305700
gacctcctgc tgtggtcacc aaggaatcac attcaaactt ctcagcaagg tgttatggtc 305760
cttctcagtt tgcctacgat ccattgaggc aaaggtttcc ttctctcaat acaccctctg 305820
ctttagccag gggacctcc cactgtcctc tctatgtgtt ctatttgttc ctgactgtta 305880
gtacccgctt gtgtggtttc catgatcctt cttctgcctg gaatgaggct cctcttgagt 305940
cctgagagct tcatttctgt ctgtcttttg ggctgtgcca aagcccctct gccttcgtgg 306000
actccccact gattcattac caattttcct ctcctctggg ctcttcttag attgcaaatg 306060
gtgcctctgg attgggtacc aagacttgca gtgccttatt ttgataccac taatatgaga 306120
ccatgttata tgcctcttga gatatcattc cctttctagc aatagacagt gataagcatg 306180
cgtatagagg gtaaacgtag gactttgagg ttcatccatc tcaatagaca aaagtaaaag 306240
tatgctaatt tcaatccttc acacagtaaa ttgaatgtaa taggtcttca gtaactattt 306300
gaggaatgaa attgttattg atatattgat ttaactgagc aaatccataa gtgccacgct 306360
tggagtaaag tacagggaat ctcatttttt gggtaatttc aaacaagtag gagaccatcc 306420
actatctatt aaggacaaag gagatttgag tgacaaacat gttccagacc atccaatggt 306480
gttattctac tacttcatcc cagctagtaa aatggggcaa taagaaccaa aaagcaggta 306540
ctgtccctat gtaagtagac ttcctagtcc tggccaaaac aaaaacaaaa aaaaaacaaa 306600
gcaacacaaa acaaaaacaa tgtgtgaaga gcatcttacc gttcattgct ttggatgatt 306660
tgtgcaaatc tagttaatgg gggtggatct gatcagtctt tgtaggaagt cagtattggc 306720
atatgtctgg cttctgaatt ttctaaaaat ataattattt atttttaaat ccattcaatc 306780
aacactgaat ttactaatca actactaaat aagtaaaaga tcctatagta gatccttcaa 306840
gaaatggaaa ttgagacatt aggtcactat aaaccccaac ccttgtaatc tttttgtggc 306900
aaccttagga aacttggaat gctttttaca ctctgataaa ggtttaattg taatcatagc 306960
cccagtgttg caaaatgagt cagaagatac aaagtatctc ataggataat attatagctg 307020
taggtacttc aaatggacaa gtataacaca ttatgactgc taatatggat gcattgggag 307080
gaaaataagc cctaagaagc aatatgcaga aaagctatga ttgctggaga cagatagatc 307140
caagaaccta ggtaattttt cagcaaactg tggttccact ttcgcctctt tcttgacatt 307200
gactgtgttt ttaacaggga aaaagttatc tgaaattttt gatagagttc aaactttaaa 307260
tcgtgttcac tttgctaaaa ctccactgga tggagtggca gaagagaaaa aacacagaaa 307320
taaaagaaaa gcagtaatgg agaatggaaa atctttaaag atgctaccat cctgaaaaga 307380
ttcgaaagca agcccaggaa agccacgggg agggaaagag cgcttagagg tgtccccaaa 307440

gctttcagaa gtgatgaggg aaaccccagc tcttcttgtt acttcttggc attttgttcc 307500
tctatacgtt agcttttggt taatctttca tgttacttat tatctctgtt ttaaaatttc 307560
acattaactc attggtttca tatcccactt aacaaattag atattttcct tgggcttatt 307620
aagaaaatta tttgctctca tatgttatgg ctttagattt tctgtgtagc taggactgtt 307680
cttaaatttt tatctttacc tttgttattt ctctgaagca tccccattgg ggtggcagtg 307740
gggggtatta aatggttatg taaaatttac atgtaattgt gcaatttaca catagcctca 307800
aatataatga ggcctagtct agctgcccat accaagagga tattatagtt gccaataaaa 307860
tcagtattat ttcttttttt tttctgttct tttttagctt cttgcaaaca ttcactcaga 307920
atccttttttg aatatcagtt ttgtgaaaag caccgtgctg ggttctttgg gaaaagccaa 307980
gatgagggca aatgcatcct ccttcctagt gagtctagca cacagatgac tcaggaagtc 308040
atgcactgga ggaggagtat cttctagaac tggggaaagg ttaaggaagg gatcagaaac 308100
ctttttcact caaccaatta cattttcctg aaagcatgtt attaatcgta catgatcttg 308160
cagaccccag atagagacta tatcactata tcaattgttt tcttgaacaa ctggagttaa 308220
ttctcatatg tttggttacc cttcaagtat ttatgtttag gatctgtttt cttcctctta 308280
acttacattc caaagtttag gtatccttcc cctccatttg ctgtccaaaa cagcatcctt 308340
tcatgtagct acatgccatt ggaatgattt ggtttttgcc atggattcct agtcctaaat 308400
ttggactttt attggtatct aagatgtcat ggacagaacc cacagatgtt gtacaaaaag 308460
ggctgagtgg acagtccaac agagttacat catctgttcc aaacaccctt gtaatggttg 308520
tatcagcgtc aatgtctgag ttgctgtcct ggttctcctg gccagtcctc agctggccat 308580
gcctgttcag gcaccagaga aagcttcata cctcaggcaa atctttacaa gggatttgaa 308640
aggcaagaaa catgtatttt ggaggtttta cctgtttatt tcatgtatag cttcatatta 308700
acagattttg ctatcactta aaagtaaata aaatgatttt gatttctcaa aatcatctta 308760
taatttatta atctatttta aggcttgcat ttacatttaa tcttcaaatc ttgatgcatt 308820
ttcacagagt ttctttagtt agttaaggat attaatgact tcctttaata gttgatgaag 308880
tgagtcatca tcgacagtga gtcactaagg ccaactcttg tacctgctct taaatagcag 308940
ttgttttact agatttgtac tgaaaccttt ttaaaaatct gtttgtctta tatataacaa 309000
gcatttccag tagttaataa tactttctta gctcttcaat cattttatga gagcattaca 309060
agaaaagcag gagatgggga aagagatatc ttgtttgcca tgtaggatgc atgaaattct 309120
tacttagtct gttgtgtttt ggattcgaga gaaaatatgt aatgaatcaa aatgtttcct 309180
acagtcctct gaaaagatgt aatgagacag tcttccagca gcctttgatc atctgggaag 309240
tcattgactt tgatccttat cctatcaggg gctcttaccc tggggtccat aaatacccag 309300

gcatttttaa gaagtggatt tcagtataac ttgtttcatt ttgtgatttt taaaaatatt 309360
ttgttttatg cacttaaaac cttaatctga gaagtcatct gtagactata ccagatttcc 309420
aaatggaata aaaatattta agaactctct tatgtgtgtg tttcaaccta taaattttat 309480
atgtgtagct catggttagg tggtaagtag agaatttgct atctgtgtaa tatttggtac 309540
tttttataat gaaaaaggaa gtaatacaat gataggtacc atttatttgt tgaccaccca 309600
tattttccag ggaatgtgat agatactttt tatatattct ttctagtttt caccataaca 309660
tgctgcaggg atagtgttac cattctcatt ttgcagatga agaaattgaa acccagaaca 309720
attcgttcat tcattcaaaa aatatccata gagcatacac taagagaagg cactctgcta 309780
ggcaccagaa attgaataat tgaccccaaa tcacctaact cataagtggc aaagctggac 309840
ctaacaaagc ccattatccc ttctatcaca cattccatcc tcataatatc atattctaca 309900
tagaaaaact taacagtcct gttgagacac ttggaactct aagaaggtag tcaacagata 309960
cttttctctt tatataaata tgatatctaa ttagcttttc ctggaaagga gtgattctgc 310020
ctttttttaa ttctagcatt ccctgagcaa attagagccc aggtaatgat ttcctgagat 310080
aagtcaattc tcttctagga caatctctag tcttgtcatc atgagatgga ataacaacgg 310140
tctatgtcat ttcagtgacg gcacctgcat gtccttgtat ctaagaaaag acacctcctt 310200
ctctgccttg ctaaatattt gtaaactttg ctttaattgg tatatgtgcc actgttttgt 310260
atgtgggagg ggtttatggt tttgttttgt tttttggtct tttttagtaa ccccaatcca 310320
aactattctc atttgtaacc taaaatactt agaacaagag gctttgcaat cttgaagacc 310380
gaatacattt ttactactaa atgtaaggca gatttgagcc acccattttg tagtgcttga 310440
ttatggcaac ccgaggaaac taatacggtt actctttatc tttattagga aatactgctg 310500
tagttcctgt tgtaatcctt agggagccaa cattgttgat gagacctagt accaagccag 310560
aggccatgtt gaactagcaa gatatgggat agtttttact ttcaaagtaa ttagcatcta 310620
gagtctagag agtttctctt acagggtggc ctggtgtctt agtctatttt ctgttcctat 310680
aatagaatac ctgagactgg gtaatgtaca aattataaag atttgtttga ctcacaatcc 310740
tagaggctgg gaggttcaag atcagacagc cacatctggc catcctttaa tgagggcctc 310800
atgctgtgtc ataacacagc agagaattgg aaggagaaat gggcacatgc aaagagaaag 310860
aaaggggacg aaaggggcta actcactttg taactatgag actaatccat tccctggaga 310920
actaatttat tctcatgaga aagacatgaa tacatcttaa catcctaatc acttcttaaa 310980
ggcctcaact ctgtacactg ctacattgta acaggcaatt aaatttcagc atgagttttg 311040
gtggggacaa actacatccc aactgtagta cctagctacc cactttattt ttagagtggc 311100
tatttaatga cctggcaaga tctactgtgc ttgtaaaaga atccaccatg ggttaaagaa 311160
aaatagtttc tggcttccag tctccttttg gcctctctct agaatggcat atacaagttc 311220

agtggttact gacttcaaag gtggggtgga agacactgaa atgaattttt ctcctttctt 311280
tggttggttg ctctctctct ttgttgctga aatttgtgta ggataatgga atgaatatgg 311340
gagataggga actatagaaa gactggaggg aagggagaag tcttttctga attaaccccc 311400
catgtgcttg tgagaccctt ggaccccaaa gggctcctga ctggctgggc ccactccttc 311460
tgtatatgga gccatagggc ttttctctgg cttcccagtt gaagcattgt catatgcttt 311520
gatgctcagc atttctggca attttacctt gtaaaactcc cgtttcgaca ttctgaactt 311580
aagtagaaaa tgggaatata agcctcttgt tttttgctct taatccattc cagaactata 311640
agtccctggg ttcttggcaa caacaggtgt tcctctagat tttaaatcaa gtgtgtgtgt 311700
gtgtgtgtgt gtgtgtgtgt gtgtgtggaa tatctcacta aaacgacagt gttgtcatca 311760
attttggctt catggagggt cttgagctgg gtggagatgg gaggaagcta tccttgtcca 311820
tgcggtttgg agacttgctc taaaaaagag accgtctact tccctcgtag tttttgtttt 311880
gaagacattt gtagtgttta ccaggagcat acagttatct ggaaaatgca gactgatgac 311940
aatagtattt gaaatgctcg tagatctgct tctaggttga tggaaagaat gtatgttcca 312000
aggtacaagt gcttgttgac ttttcggatg cagccagtct gtgctctttg tcaggtaact 312060
cggaatcact ccacagtagc tgtccttcct gtccatttga caagcactta aaagggcagt 312120
cttaagtact ttacttcata gtttattaat tttttccctt ctttagtttg caatctactc 312180
tcagaaactg aaaaataatt tcttttacct ttttcccctt tctcatttaa gctgaaattg 312240
atttctttct gagcgtgtaa ggaaaaaaat aaaaagaagc tgtggttaat acaattgcct 312300
ttgctgaaca aaaataaaag actagtttac cttcaagctg tgctgtatca gagtaaggat 312360
ctgttctgtc actcccagga gattttgata agagagagta caaaatttca taatatctac 312420
atagcataat tgtagcaaat ataagaagta ttataattgt ttatttgggg atgagggtgg 312480
ggatgggttt ttttgtgtgt gtttggaaaa ttgggaagct agaagatact cttaaatcca 312540
tcagcttctt cagaactctt ctcaaatgct tattgtcatg acaaccatct cttcataaac 312600
caaggctctg agtgtaatac agatggggct ggtaacaagg taggattgcc atctgctttg 312660
ttttctctaa gggacattat ttttttgtga tccccaagtc tcatcctcaa atctgaaagt 312720
cttgacttat ttttctttct ggagtcagat agagctgtga gattaattct tttctcctga 312780
caaactactt cctcacctgt catcaaacaa cttacttgtt aaatggaggg aaaaaagggt 312840
aattatgtgt gtgtgtgtgt gtgtgtgtgt gtgtgtgaaa acaaatatac atattttaat 312900
gatttattta tctgtctact ttgcaaagtc tcctatgagg tacctagtaa ttcaataata 312960
cagtcaagtg tgagatccag aaacaagtag gagtgaggca atcattttcc catgaaacaa 313020
gtaaaattat tactgctttt gaacatttta acacctgtgc ttcctgatgg tggggctgca 313080

tgaagtttgt aagtagtgtc atcattctct tattaggtaa caaataggag cataattatt 313140
taggagagga aaaccttttc ttgttaccaa atttggtgag aaatttgcca aagaaatgaa 313200
tgcttacagg agggatacag cttaatatct tcatactccc ctttgtcaaa gatacaagtg 313260
acattttctc atatcaactt cctacaaaaa gtgaggccat acatttttaa aaagaatata 313320
cccagtattt taaaagtttg tatatatcgt gatcagaatt aagatatatc cagcttttga 313380
aaaaatatac atatttagcc attaaaaggt atgtggtaaa ctaaactgat gaataaaggt 313440
tatgggaaat atttcataaa atttatattt aaagtaaaga acaatttctc tctttgtatt 313500
atagttattt tacataagct acaatctata attctaaacc ttaatttctt gaagggcttt 313560
ataagcctca tttttctttc taaatagaga agttcataca ctgcttttat gtcctatatg 313620
ttgatgtgta aaaataatca aatactagtg ggtgagtaga atgtaactac aatatggtac 313680
acacacagac acacaggatg actaaactgt gcacatgagc tgatgtccag agcagggaaa 313740
agctgctaaa gaatgtttga agtagagtca gaatgcagtt ttgtggttaa gaccaataat 313800
ttttcaaagg agcattataa ggagtgtggt tacttgggtt ggagttgaaa accaagcaac 313860
agttgggcag acttgaaacc agttttgtct ttaggtttct tttcttaatg attggtctac 313920
aatgagttag ccactcatcg tatttaaggt cttggtaaag gagagacttt ctttgccatg 313980
tagtttatac ctcatgtatg cattacagta gagagctcca ggaaatactg caggccttct 314040
actggttttt atcctctatt tagataaaaa tgaggaagaa gtagaattat cataaacaat 314100
cctgttggct tttagttgag atatactaca gaaacatcac ctttgaatct gtatatgtaa 314160
aaaaaaaaaa tctcagagta agatataagt tctgttgcat tgttctgaag gaatgggtaa 314220
tgtcagaaat gtcttcatta cattacataa agtaatacac agtgctgtta tcagattgca 314280
tagtcaaagt tggcaagttt gcatatttac atagatatca tgaccttcat cattcatact 314340
tctttcaaga aatggtcatc ttaatatggg gtatttattt gaggcataca ccatttattt 314400
taagaagcag tacctttgtt tattgtaaaa cttctggact aaattcttca attttttctc 314460
aaatgaattc agtttttttgt tttttttctt accactggtt tttactgcat agcgtttgcc 314520
tgaagaacac cactttgttt cccaaggcaa gtagtcacta caaggcgagt tttgttctgt 314580
ctatcccaag gcaaatagac agcagcaaac atagtgtgga gggctgctgg gttcagtaga 314640
aaaccatcaa ctatttctaa ttgggggtgt atgaagacag ctgcattctg gacatgctaa 314700
acatcttgaa atgcttggag tgtaataaaa ccccaaacgt gctttctttt ctcatttctt 314760
tgcacatgta ttttaggcaa aatataatcc acatgctgtt gttccctctc cttttaaatc 314820
atgaaaaatg ttgtttttat agggctattt gaggtccagg atgccttttt atccttttta 314880
ttaggttatt tttatatata aatgaaaaga aaaacataaa cagaaaatac aacatgacat 314940
catgaatgag ggcaagcaca aactgcccaa tttaagtttg aagttataaa cctaaaatat 315000

tttaaaacaa aacatttctc ttggtgtttt gcaaaagtaa tgctttcttc tattgggcag 315060
aaattgcagc ctgttgggat gaaggagaga atagaactaa tatttattga gtagctacca 315120
tggctttctg ggacatctgc taacccctta atttttacag taaccctaag tggtaggcca 315180
gtccatctgc attttacaga caggaaatgc tttcagagat attaattaga tggctgtaga 315240
ccacagaaga attgaaagtc aggtcttctt gacttttcag ctcatgactt cattacttct 315300
gccctcctca ttctgttggg agatgggtgg gagtgtgccc tccagataat cagaaagtcc 315360
catgttggga tgtgatttgg aaatagctag tcctcaggcc tgtggacgaa ggctattgta 315420
gcctcaagaa agaaagagtc agcttgatat tggcaaggtg gttatttagt gtggcagctg 315480
cataagagca ttggataggt ggtatgatat gggatggggg tcagggtgag agctgctcac 315540
acctccttat tatggcaggg caaaaccaga atagtcacag tgatggtgat gtagggtagg 315600
ttcaagagaa agctcatgtt cttctagcat tgagactcac aatatttcaa aacaagtgat 315660
tatttattcc attactattt attttggtga tggatgttgc atttatttta ttttctaaca 315720
gtagtacata tttggtaaga gttgtttgtg acattcttta tttaaatgga ttaaacccat 315780
tttcttaatt tttttaagtg tttgtcctgt tatcgaagat atcttaaagt gttacagaat 315840
gggaatcata gctttaatga gtctgcatat aagagagtcc aaacattttt tttttggaga 315900
gaaggaaggt catttcaact gtactatagg aacgataggg cccatgatcc atggtatggc 315960
aataagttac tattttttgg agaataaatc gcaaaatatg ggacgtgaag actgcttggt 316020
atttacacag gagtctagtt cttctgatac tatatattct ataagctttc tcccagaaat 316080
ccaaagtttt atttagcttg tagccatcat tgtgccacaa tcttccttcc atgatttatt 316140
tttttcccat actgtactct tgcactgagt ctttggcatt gaaagggaaa gagatgaatt 316200
attcaactct tcttctattg agtgaaaagc caatacagaa aaaaaaatgc aacaggcaat 316260
ttcatgactg acattttgga gaacattggg gtcatcatat gactgagaaa atgattatct 316320
cttttgaagg tcaggaaacc aatatttttt tttccagtga atctactgag ggaaccaacc 316380
acctagcttt ttgtaaatag cactcctcag ggaaatgttg ctttttttctt ttgcagtgtt 316440
tctgctttga atcttcatag actgtatccc atggaagcag gaatataatt actgtagtgc 316500
ttactttttt tcatgtctct actgaagagc caccctgatt tatcttttac tccaaggtag 316560
tgtggaactt tacaaggatt tagattatat tagtcatttt gcttggagat ctctgcacac 316620
ttttcaacat catttctaag agccttgcca cactcagtaa aagagggtga ggcttcaaga 316680
atctctgtgt accaacgcaa attaggcaat ttgtaagaag tccctagtag agcagagtgg 316740
agagagggct caggatctcg tgaggcctgg ggcagggagg tggacatgct atgagctcct 316800
ttcacagtca acacaaagtt aacacatctt ggaggaaaaa ccgttactgt ataatggaag 316860

gcagaaggca gggatgtctg tgcttggtga tgaagctata agtagtgagc tgatttatga 316920
tggaggggtg aattgtgcca tgaaagagac tcccctcaat tttattagcc agattactgc 316980
ccatatgcgg ggtgaggagt gagcatttta aggcatttct ctctgttttc aatatatcat 317040
ctctggattc actattactg ggacttgtga atatagacta gggattaaaa cctgagtttg 317100
cttttaaaaa actttatttt attaaatgag atttctgagc aactgatttc acttagtctt 317160
atcaaactat tcttactact ttttcgatat gcattctgtc cctcctgtgt gaaggccaca 317220
ggcccatggc cccatgtca ttgagatgga ggtcctcaag tgatggcggg atgaatcacc 317280
tctcacttgc cacctgcgag aatgtctatc actgatacct ctccttatgt cccgtagcag 317340
ctgctaagct cccacgttat tattggtgac actttctagt gttcaggaga gaacaggatt 317400
tactttttga atgcctctaa attattatag caggttcatt tggaattgca tatcagaaca 317460
ttcttcctgt ttatcaggaa aataaaccaa ggtcttgaaa cttgatgtct atccatagtt 317520
ggcccaatat aattttgcat gtggtttatt acattatttt tctccattgg ctatgaactg 317580
aaggcatctc atggatattt attccacgct gaaaaggtag ttagatgctg tgtggtaaag 317640
tcgagaccag tatattggaa gaaaagagac actccatcta ataatctttc atagtcttca 317700
cattcattga gaaggttact tgataacctt tcctgcacac tctctcccaa ccctgaccca 317760
ggaacttgca gagcacactg tgcaacagat ctaataaccc ttgtctgcag gcaatatgca 317820
cagctgggaa agataattaa atagactctt tttgagaggt ctacaacatt tcaacggaag 317880
gatcgagcaa tccggctggc ctccgactga aaactattac caacagacat acttcagaca 317940
gattccatga tcaccatcct tatgtggcac aagtgcttat ggggagcttc tttggcttcc 318000
aaaaggccac taaaactgtc agacctaaat aatactggaa ctgtaaagtg ggaattaaaa 318060
attataattc catttaacat gtaacgttct catcagaaag gggcctttgg ttttccatat 318120
tgatcggtta catggtcagc tgcaattgta actcatgaga cacacccaga tatcatcctg 318180
cgtgattctt tttgaggttt atttagggtg ttagataatc cgaggtaggt ttcatcatgg 318240
gtggcaatat tacatatagc aaatgtcatg agaagagaga caaagcctcc cttttgaatg 318300
ttaacattta tgcaataagt ttaatgtacc gtgtaagttt acattactat tatgagactt 318360
aacctaaaaa attggggtga aatcttaaag aattatgaaa ttaagaaatg aaaccagagg 318420
gctttcgttt taagtactga tcacttaaac caaaaccagt ttggcttatc ctatgagatc 318480
gtgggtattg agaaagagga acatttgctg ctgcatccgg agatgtttcc ttgagagcaa 318540
gactagttcc ttttgaccca gtacatccta caggtaaatc actgcatgct gtagagatac 318600
ctgttccctc ccttccccca cccccctgggg atctgaaacc aaatcattgc tttttcatct 318660
tttacttatc cttataaaca aagccaccca ctttctcatt tttcctgatg ccaagtcctt 318720
ctttacatga gtggaatcca ctcttttcaa tggaaagaca cctgctctga gacaggctac 318780

actgtcatga gccttggcat ggctgagtga tgtagtggaa ggtgcaataa atttaaactt 318840

taaaaatgca aaatttgatg gaatcatgca tcttgtgcct acagtgagta tatggtctag 318900

caggatttac agatacatgc atgattaatt tgaatacagt atagcaaaat gatctgttaa 318960

aaaacacatg aaaagatgtg caaccttatt agtcattagg aaaatgcaca taaaaccacc 319020

gttcctgtgt gatacctctg tacgtctatt agaatgtata aaatttaaaa agactgaaca 319080

tagcaagcac tggtgaggtt gtagaccaac tggtgctttc atgttttgct ggtgagaatg 319140

taaacattac aactactttg aaaacagttt gacagtttct taaaaagcta aaacatccac 319200

ctggcatgct atatacagat atcctactct tacgtattta accaagagaa ataaaagcat 319260

atatccattc aaaaacttgt aaataaattg ctcctagcag ctttatttgt aatagccaaa 319320

aactagaaac aacccaaatg tccaatgaaa ggatacatcg tatttattta taggacatat 319380

ccatgcaatg gaataccact taggaataga aagaatcaac tgttcatcat acatacaacc 319440

acatggctaa gtcttaaaaa taattatgct tagttaagaa gtcagacaaa aaggtaagag 319500

actgttagga gctgaattag cgggttaaca gtggcaatga aaaaggagaa aattatagct 319560

acctcaagat agaattgatg agctttgatt atgaaatgat ggtaagagag agggccatag 319620

actctgaggt ctgtctctag ctttggtttc cctatgtgga tcattatcta tttggatata 319680

agaattatct gaaagatgat acttaagcat gtaagaaatg ataagctacc tgtttaatga 319740

tgagcttttg ttacctccaa gaaccccaag aagaaacatc ctgtaagcag accattcata 319800

cttgattgtt aaaggaaaac aaaattccat tttgtctttt tcatcaacac aagtatagct 319860

ggctaataaa agtgtaatat tcatgagaga aaaagaaaag aacacgcaca cacatacact 319920

caacaggatt cctagagtca ttcccatgga taagagggaa ggaaaggttg agggcaaaga 319980

gagaagggaa ggaaacagac tatgatggga tatcttaggg caaaagaata gggggctgat 320040

ttggaaggca gcaaacttca tggatacttg atttatttaa tgtccctgtc actctggcgt 320100

atcttaatgt gtgtggtgtg tgatttattt agtcacttga tccaaattct ctaattaagt 320160

ggagctcaga agatatagtg tccattgtgg tcaagggcac aggttttggc ttcaacagac 320220

aaataagctc tctgagcatc tgtcttctca tctgtacaat gggaatatca gttcctacct 320280

cttcaggttg ttgtattcat aaataaattg tgtacaagaa acattaggat tgcttcttac 320340

ccgtagtaag ggctcaacaa accttctcct ccttctcctc ctccttctcc ttcttcttct 320400

tcctcctcct cctcttcttc ttccttttgc ttttctcctt ccttcttcct tcctttcttc 320460

ctacctcctt ctcattattg ttattaggta accacaatat tatcagtaat gattggagaa 320520

agctttaatc tcctagttca ccattagaaa acaagaacac attttggtgg ttattacccg 320580

aagtaatcat aatgtcacct ttttttccat ctgactcatt atcccaagtg atttatttat 320640

atatggagtt ttctgagtct ttcttttaca tattacaaaa aaagagtgtg atttagggac 320700
gaagcaaaga aataaaaatt tagtgacttt cattctgcct gtgccccaat tcctattggg 320760
cataaggcaa gtaatttaaa tttcttagca ccttagcatc ttctactcaa acagaaatga 320820
ggaacagtca caggttacta ttatagctgt ctaagtagaa ggcacacaag ttttcacact 320880
gagtataaca ctttatagaa agctaagtgt gttgctcaag ttggtacatt tctgtagatg 320940
tgacactatg gcactaagaa acttaatgcc acattgaaat tcattgagat agctagactt 321000
taaaaataat tacttgactt cactataaag tatgttcgta ttgcatttac tccatctagt 321060
agaaaataga ccttgtcagt tcaaatccct gttgcattaa tttcaccagt aatgagtctt 321120
tttcatttga gtcagcaggg tttttcttgc ttgttttcag gctttgtgga tttgaccctc 321180
catgatcagg tccaccttct agaatgtgcc tggctagaga tcctgatgat tggtctcgtc 321240
tggcgctcca tggagcaccc agggaagcta ctgtttgctc ctaacttgct cttggacagg 321300
taagtgacct ggctgtagct taggagtagc atgttcttta cgatcatagt tcattcatga 321360
aactatttta ttcatctctc ggtgaagctt cagagaactt tattaggtat gtttacttaa 321420
caaaagagtg cattgggggt gatgaagcct agtcaaattc acagaaagct aaggataact 321480
ttctgctaga cattacctca gaagaattct attatttcta atacacacac acacacacac 321540
acacacacac actcacactc tctctctctc tctctctgtc attatgaatg gtaattttct 321600
aactccatct tcaacttgta tcatataaaa attataataa cctctcttta attaaaatct 321660
gttgttgctt cttgtacatc cataccacaa tagcctattc attttcttct ccaattttcc 321720
catccgtaaa atgaagaaat ttgaccagag ttctgaaggt cacattcagg tcgacaaatt 321780
cattttcatg ttcaaatatg ttaccttctt taacatacca ttctggggtt gccttggaat 321840
gtgggtccca ttgttttttt tttttcagtc attgcttaga gtcatagaat ttagatatta 321900
ctcaatagca gctgccactg atagagtctc caccctgcac cagctgtgat gctaaacact 321960
ttacatatat tatctcattt aatcatcacc ggactcctag gaggcaggaa tgtcatcatc 322020
catgttttac cagaaaggaa actaaatctc agagacatcc tgctacttgc aaaaagagga 322080
aagctcacta aatggtggag ccagagttca aattcaagat ctttctggct ccggtatgct 322140
ctgttacctc ctgtgctggg cacatggtct tcccactctc atgttcagtg atgcctcctt 322200
tggtctgctg ccatagcatt ctgttttcca ggtaaatctt gtctttgtgg gctacataac 322260
attttggatg agaaagaacc attttgttgt ttcttgcaat cctattttgc ctccgtgcca 322320
agcagtctaa ggctgccagg ctgcccacag tgcatctctg catggtactt tacttcagag 322380
catttcacta tctttctaca cctgccaagt gcctggagca gagttgcagc aaaatttatt 322440
taagtactgg agaattgtac aaggtgttgg ttattgtttt gtcattttgt gttaacacag 322500
cttttgaaaa acagtggtga tacagtttaa gaagcatatt ttgctgtctg tggaatatat 322560

tttgatatca cagtttcaca aaattatcaa gaatggccac tagtcttgtt ccttagaact 322620
gtactcacaa tgtattctgt cagccttata gaggatgtct ttaccgtgtt ctttcctttc 322680
cattccttgt ttcctttcca ttttttccta tctcatttct cctctctttt cctttgcttt 322740
cctctttctt tctttctcct gtcctcttaa ttttttttgtc ttatagcagt gtggttttgt 322800
aaccaagtga tatcaagttt gcaaatgaaa atcttagacc acacgtaaca tttccctgcc 322860
tactgcctgg agtgtctacc atgtttaggt ttttggacat tcataactca tttgctcctt 322920
gatttccatc ctcatctgta tctatcttct tttttaaaat ttaattcaat ttttataatg 322980
ttgacacaat tatgcagatt catagggtac acagtgatgt tttgatacat ataatgtgtg 323040
gtgatcttat ctatcttctt gagtacacac ttgcttgagg acagtcatca aataattgca 323100
ttttgaatat gtgaaggttt ttgacattac tgcacccaaa aaactcatat tgccagtgag 323160
gtgatatggc ctaggatttt atcagctaca gccttctctt tcctttctgt acactccagt 323220
ggtggctaat ttctttcct ctctcacaga agtatgaatg actaaaagtt ctcatctcta 323280
ttcattccta ctttctaaaa cttcagatcg gaaatttgaa ttacctctag accaggattt 323340
gtcagtctct ttactattga cattttgggt cagatcattc tttcattctt tcttggttga 323400
ggggttgata ttgtttggtg tgtcctcatc caaatctcaa attatagctc ccatagttcc 323460
catgtgtcat gggagggacc cggtgggagg taactgaaac ataagcaaag gtctttccct 323520
tgctcctctc atgacagtga atttctcatg atatctaatg gttttataaa ggggagttcc 323580
cctgcacacg ctctctctct tccccgttgc catgaaagat gtgactttgc tccttcttcc 323640
ccatgattgt gaggcttccc cagtcacaag gaactgtgag tccattaaac ctttttcttt 323700
gtaaattacc cagtctcagg taggtcttta ttagcagctt gagaacagac taatacaagg 323760
ggctgtcttg tgcattttag gatgtttaac agcatccctg gactccacca gctagctgcc 323820
agtagcaacc tccacttcct ccagttacga taactaacaa tgtctccgga catttctacc 323880
tatcttctgt tgtgtcaggg ggtgggggag gtaaaattgc ctctggttga gaatcaccac 323940
tctatgcttt cccaactcaa tgttctataa gctcctcaga cacactgtac tctaaactgc 324000
acactcactt tatcttctgc aacaagtcta ttccttttct tttctgtctt ggtgacatca 324060
acactcacct agacactaaa gctagaagct ttaagttacc catggattct accttctccc 324120
tcaccaaatt atccagttaa ctatcaagtc atctatgaac tgcttctgaa tccagacctc 324180
ccctctatcc ccattcctct gcatggcaca ggtcctggtt gtctctgctg cagattcctt 324240
gcttctgcac cactgactca ctttcttggt cctgctactc tttctttcag tccatctccc 324300
atactgctgc tggagaaggc tttctaaggc acagctgtga tcatgatgct tcctgactca 324360
cctttcagtg gctctctcaa ttcttaacat ggagaaaccc catctctact aaaaatacaa 324420

agttagccgg gcatggtggc tcatgcctgt aatcccagct acccgagagg ctgaggcagg 324480
agaatcgctt gaacatggga ggcagacgtt gcagtgagcc aaaattgtgc cattgcactc 324540
cagcctggat gaaattccgt ctcaaaaaaa gaaaagaaaa tactgtgtag acttcttatc 324600
ttaataatca tgaatcacta ttacaagtgt ctcaaacgca aatttctgaa gagtcacagg 324660
tgacccagtg agcccttgtc caggctaaaa acacctggtg gctgaattct gaacttcaca 324720
cattcgaatt tctcttccat tgcttctgag agaccctcca ccattacggt tccttatcag 324780
agtcatatct acctataagg ctcatttcag gtgccgtctt tttataaaat cttccttcaa 324840
actttctcct cttcagcctt tatatataca aaaggctttt ctttcttttc cttttctaag 324900
tttccattgt agttggtttg cttttctctt tattattctc attccaccat attagttatg 324960
attggatgtc ttcttacctt ccctactagc tcataaactt cttgtggtca aaaaccagat 325020
cttgttcgtc ttgtgtgatc tccagtttac gaagtcttcc atgataccca acaaatattt 325080
gttaattgca ctggccaagg tcacccaggt ggcttgtggc caaatcagaa gaatctacat 325140
cttctgatag tcatggcagc tgttattccc atccaactca cttcctgtgc attgctactc 325200
atcaattccc cacttattct tttaaaaaca ttgcataaat acatatctat gtgttttgga 325260
aagattttct taatctataa gcgcatttgg cgtgtgctta tatgtctgct tatatgccaa 325320
atttgaaatt ccaaatttcg ccatttggaa tttcaaatgg ggaaaagcaa agactcttat 325380
ctttcccata atcaaacccc ttatgaagta aaatccttaa ggtctctcta caggttaaaa 325440
ataattaggt gatatgattt ggctgtgttc ccacccaaat ttcaccttta attgtaataa 325500
tccccaggtg tcaagggtgg ggccaggtgg agataattga atcatgtggg ttgttttccc 325560
catactgtta gtgtggtagt gaataagttt catgagatct gatgatttta taaatgggag 325620
tccccctgca caagctctct tgcctgctgc catgtaagat gtgactttgc tcctccttgc 325680
cttccgccat gattatgagg cctcctcagc catgtggaac tctgagtcaa ttaaacctct 325740
ttcttttata tattacccag tctcaagtat gtctttatta gcagcatgag aacaaactaa 325800
tacattaggt atatgttaat gatgaggaat gtgtataaat acatatacat ttatatttaa 325860
atattaatat ttacaaataa catgatatta aaaatatggt ctataatttt ttaaattatc 325920
tacctttcta atcattaaca tgcactaaat attattaaat ttcaatatta tttaattacc 325980
atcttggtgt ttgaggataa ctgcttttga tggcattgat agatgcatta acagtgaaac 326040
attgtgacca acagcccatc tgagatttta tacttgtaga agatggcttc tgagtgacag 326100
ctgagaagac tgtttatgtc tttgccctac aggttcctaa cccctttaat aaaatagagc 326160
tctctgatgc agatgacaca tgggccttct ttttgctctt gtcccatcac attgtacata 326220
gtaaacattt ttagcaacaa atagtagata cttataactt taaaagctaa gtggttgaca 326280
gctgagaggc agatgatggt aatttcatca tttttctcat atctcaggca ttgtgacact 326340

acctctgcaa ggtcaactgt ctccataggc tgttttcatt tgcgtagaaa tagggggaa 326400
agatagcttg aagtcatcag gagccctgca acccatggat taccaatgtt gctaactgga 326460
gcctggattt catcttcaat tgattgtaat gatgtcttgt tctctcaaat ctttgcaaat 326520
ctaggcattt ataagactat cctgtaggtc ccttacaaga ccatgaggat gctagaactt 326580
accctagtct tttcttgtaa ttgcttaaat gttagtattg gcaaatgggg tttggtgatt 326640
ataagagtaa aaaaacctgc tgccatccca catttgtgag cagaggatac atttcctatc 326700
ttgtgtccat ttaaaaagaa tgattgcttg attggcttcc tgatgacaac ccatgatata 326760
ctgattctgt tagtttataa ctttcctagt agtcatatgc ttataggcca attttatcct 326820
tgccatgctt agcctagtca caatttccag ttcttctctg tatcctgcag cagtgagttc 326880
caaacactta atgctgtcat ctcttcctgt gaaaaccaag aaataaaggt tattataagg 326940
tataaataaa gaacccagtg atttctcttg ggtgtgtgt gtccatgtgt gtgtatattt 327000
tacaagagga aagttaaaga tactaagaat gcctgtgaaa gtaatcagga aatggagaaa 327060
acttgttttc cattctagca cctaatcctt tggtgtgttt tggcactaga aaacacaaaa 327120
tatgtcctct gtgtctacta ggaatgcctc tcttcattta gtcctgcttg agtgctcatg 327180
atggaaaaat atagactgaa aacaggagca aaagtgttca tcctactcat tccttgtggg 327240
gtctctttgc tttcaaagag atgttagaga tgttagtaaa tggtgttaga agaaacaatg 327300
taaattgcct gtttagtaag atgatccagt ttctaaggaa ctgttttact ggcgccttcc 327360
atgctaacaa attctgagaa atatttgcgg atttctcagt gaagccaaag cactctcttt 327420
tgactcctat ttactctcag agaaaaaaac tattcatcca tttgaaaagc agggaccaat 327480
tcaacaagca gaatattccc tctactaagc ctttggccag agagttgttg ctctagttct 327540
ttccatcact cctgcatcag gaggctctgc ctgcaggaaa tagtattgga caacagaaag 327600
ttcttttact tgtaggcata aaataggtat aaattctcct ccttaagaga aaaacaggtt 327660
gctgcaccag aaagaacatt tgcctcttct actaaatagc agactgtttt ccaatgataa 327720
actcatttta aatagaaaaa aaaaagtcat tctctacaaa caagaacatt ttcattttag 327780
gttgtttatt gtaaatattt cagaaaaaaa atatgcaaaa aaaaaaatgc ccaaaaatca 327840
atgcttccag taacatattt attagctatc tttttcccca tgtaaaactt caggtaaatt 327900
ctttgcagat tttttacttg gaagaatgat aaaaaaaaaa aaaaaagttc tgaaatgagt 327960
tgaattcatc cacgtgtgtt tttgattcac atgaagagta ggacctccct ttatattccc 328020
tcttcaaatt ctcccctctg aaaaatgggt ggtacactca aaggaggacc gcacacattg 328080
tagtagctgg ggtgttgggg gtggaaggag caggttttgg gggtgggagg agcggtgagt 328140
gatcctttca gcaaagtcag tcctgggcag gagacggctt caggaatact gtcagcttta 328200

ctggattcca ccatcgcttt ccaggactgt ttaggccctg ggcccttgaa gggtttgcgt 328260
gcctcttgtc tccattcata cctcaagaac tttgttcatg ttaatttttt ttcactctat 328320
catatggaat tgagtaaaaa aagaaaaaaa aaaggaagcc aacacttact taactgcttt 328380
aatgtatagg actctgttct gggttgtctt ctaaagtaca tttcattcaa tgtgtgagaa 328440
tacagatagg aaagagtttt gctagtttct attttgactg tggatccatt gattctttct 328500
ggttctcagc ccttaattgc tcagctgcag ataccacttg actgactctt aaaagtcttt 328560
ggtttattgc ccaattctgg gaatatgaaa ctgtaagctg tttaggagaa aaactgagac 328620
caaaagaaag gagaagtgag ccaaactgcc attatgattg ccacttctta ttgaagataa 328680
atcaaaatat ccatttgact agaaatcaat tgaattatgc actttaaatg ggccaattca 328740
atgatacctg aactatatct caaaaaaggt gttaaacaca ggcacacacg tacacacaca 328800
tattggaatt ccaggaaaag tcactttgat caacaagatt atcagtcgtc aaagtgcttg 328860
gaagggttct attggaataa agctaaaaaa aatcaaagta aaatttctcc ttaaaaacaa 328920
aagcaattaa gcagcactgt attataaaat atgctaagct gcaagtcaaa attcttaaga 328980
gacttaagcc tgtaagcagt aaggatccac tcaatgtaaa gttactccag agggaaaggc 329040
gtgagcccaa ccaaagtatt aatgctgtgt aggaggttaa tttgactctt cttaacattt 329100
ttacacacca cattatgttt tattcaccat ttattattaa ctgaatttat taaagtataa 329160
tcaaaataat atttttgaga gtttaaaaat attctcataa tatttaagtt acatatacta 329220
tgcagggacc gtcaagtaat tccactctga catctgtatt tattaccgcc acctgtccat 329280
gatggtatta gaaagatcct catttgtttt aatggatgcc tctaaaatcc agttttaaga 329340
gggggttggc cactttcaag tttgtgttag ttaggattca gtttaaccca gctcaacagg 329400
tatttactaa gtaccagcat atccataggg cgctgtatga tactggagga cacacagaaa 329460
agagatgtgt aagaggcact cctgccctca aggagtttac tattgagtgg tagtgtaata 329520
tgaataaaca aatgaatctg ctcaaaagca gggcattcta agcaggccct caccagcctc 329580
tcatcattcg catctagtgt atcttcctcc atcactttct aaacgctatg gattctgagt 329640
gaaaaccaat cgctggttta tgcatgtatg catatatgaa ttcattcttg acattgccat 329700
gcagagtatt tagtataact agacttatta tccatttatc ctgcctagaa gcatactgta 329760
atatatataa ctctaaaagc aagatcattc cttatgttgg ttcttttgag ctggcagggg 329820
gaaagcagag tatgttggca ggggtctatc aaggtagatg agcccagttt gtacatcttt 329880
gaaatattac caatgccaat gtgttagtac tgcttggatc tcttctatga caacagttct 329940
tatttttttcc tttttttttgt ttgtttgttt tttgagacag gatcttgctc tgtcacctag 330000
acaggagcgc agtggtgtga tctcggctca cttcaatttc cgcctcctgg gttcaagcaa 330060
ttcttgtgcc tcagcctaca gagtagttgg gattacacgc atgcgccatc atgcctggct 330120

aatttttgca tttttagtag agatggggtt ttgccacgtt ggccaggctg gtctggaact 330180
cctggcctca agtgatctac ccgccttggc ctcccaaatt gctgggatta caggtgtgag 330240
ccaccacacc tggcccatca gttcttaatt tgatgaatgg atagaggttt ggcttcaaat 330300
aaatggagtt agtctgtcaa tatctgtttc ttttaaaatt tgcaattgtt tttattttta 330360
gtgtcatgct atgtgccctt aatgatctag aattaagttc taatctctac cctggtataa 330420
ttgatttctc cctgtggact cctaatataa gaagaaagat gaagtttttc ttttaatgct 330480
tttagagatc ttcattaagt catttaatat tcttatttgc tatttcccag tgtgatggtt 330540
aagactgagt gtcaacttga ttggattgaa ggatacaaag tattaatcct gggtgtgtct 330600
gtgagggtgt taccaaagga gattaatatt tgagtcagtg ggctgtggaa ggcacaccca 330660
cccttaatct ggtgggcacg atctagtcag ctgccagcga atataaggca ggcagaaaaa 330720
cgtgaaaggg gaaactggcc tagcctccca gcctacatct ttctaccatg ctgaatgctt 330780
cctgcccttg agcattggac tccaaaatat tcagttttgg gactcggact ggctctcctt 330840
gctgctcagc ttgcagacag tctattgtgg gaccttccga tcatgtaagt taatgcttaa 330900
taaactcccc tttatatata tcctattagt tctgtccctc tagagaaccc tgactaatac 330960
acccagtgtg ttcttttatc aaatagaaag gagatataga aaacacactg tccatgtctt 331020
tgattgttac accacagcag agaatcaatt tcttaacctg cttacttatt taccattctc 331080
caatggtgcc aattctgaga tccatcatga ctttatcatt agaaaagggg atgtaacaaa 331140
catgatgaga tgttacggga atactgctta ctgcatgata tggtttggct gtgtcctcac 331200
ccaaatctca tcctgaattt tagcctccca tacttcccac gtgttgtggg agggacccgg 331260
tgggaggtaa ttgactcatg gggggtggttt ctcccatact cttctcctgg tagtgaataa 331320
gtctcacaag atctgatggt tttataaggg gttttccctt tcacttggct ctcattctct 331380
cttgcctgct gccatgcaag acatcccttt gctcctcctt catcttctgc catgattgtg 331440
aggcctcccc agccatgtgg aactgtaagt cctttaaacc tcttttcttt ataaattgcc 331500
cagtctcatg tatgtcttta tcagcagcat gaaaacggac taatacactg catgctcagt 331560
atactcaggc actctggaaa ctaggtttct acttcggacc cctgagagaa aagggttatg 331620
tatctccaag tgtggtcatg acaagcatca ctactgtgtg cagttgggcc tgtggattga 331680
gccaccttag cctctgagga aagcagaacc tgagagcatg cttaattcag agtgtgtgtg 331740
acttggagtg tggaaaggaa gatttccaga tttgcggtat tggcgactga aaggatggca 331800
atgctattga tggagattag gaagacagga gagaggtaga tgttgagcaa ctccgaggag 331860
tttttcctca tacatgttgg gtttaaagtg cagggaagat atcctgaagg ttattccagg 331920
cagaaatttg gagctcagaa gagacatcag agctgaagat aaaaaatttg agagccatct 331980

ccgtagctaa aagtgtggaa cgaatgtgat gactagacaa gataatagag aaaaggtatc 332040
caaaggcctg aggtcaagac ctcttaaaag gctgactttt cagggtttta ggtagaaaaa 332100
gagtcccagc ctggggttaa ggcagttttt tgagaagtag aggtacaaag ttccttgttc 332160
caaaggaagg gtgtctttca aaagaaaaag gatgggccgg gtgcagtggc ttacgcatgt 332220
aatctcagca ctttgggagg ccgaggctgg cagatcacct gaggttggga gttcgagacc 332280
aacctgacca acatggagaa accctgtctc tactaaaaat aaaaattaaa aattagccgg 332340
gcttggtggt gcatgcctgt aatcccagct actcaggagg ctgaggcagg agaatcactt 332400
gaacctggga ggcagaggtt gcagtgagcc aagttctcac cattgcactc cagcctgggc 332460
aacaagagtg aaactctgtc tcaaaaaaaa aaaaaagaga gaaaaagaaa aaggatggtg 332520
aattgggcca aataccagag agatggacag gagtaagaag aaaaagacta aagatttgtt 332580
catcaggagc tcattgtttc taaagggagc atttttagta aagaggaagg aggaataaaa 332640
tgcagtataa tatgttaaat ctaagtataa tttataaaag agaaaatggg ggtggaatat 332700
tacaaaaaag gaactataaa ccagctcttt gaggtatttg tcagtgagga aaacagtggg 332760
gctctagttc ttgatagcaa cagcatcaaa gggaactttt taaactaaaa tattatgaat 332820
ttttcaaact gaaataatga gagaataata caaagaaatc tcatctgccc ataatccaaa 332880
ttcagtagtt attaaaattt tgccccattg cttaatccat taatctcatt tcattttgtc 332940
ctttttcttt gtttattttt ggctaaaaca ttttaaagac aaatcctaaa tatcatacat 333000
cagtaggcat ttacaaaaaa atttcttgat aatcacaatg ccattctcat accaaataaa 333060
cttataataa ttctgtgata tcatctaaaa aacagttctt ttctacatca aaaatgtctg 333120
tttatagttg atttattcaa tttagagtcc aaacaagttc catatattag acattaggtc 333180
attaatgtct gtctatttga atttataaca atttccactt aatttatttt ctccagcttc 333240
tgtggagact aggtaagttg ttttgtagct gtctggatat atctgattgc ttccttgcag 333300
tgtcatttaa cttgttcctc tatcctcagt gtttctgcac atggaagtta gcctcatagc 333360
aggggttggc atactttagc ctgcttacca aatacgactc ccttacattt ttgtaaataa 333420
aatgttactg gaacactgtc acatatgttg gggtcataga aagagtttcc gtgtccccaa 333480
gttataattg cattcactta tattttcttc tagtaatttt taccctaca tgttaggggt 333540
caatttggaa tttctcttca tatacagtat gcaataaaga atggctttta tatttttcca 333600
aatgtatatt cagttgtctc aacaccattt attaaaaagt cagtatttct caagtgattt 333660
gagatctacc cttaattaca cagtaaattt ctctgtagtc ttgtcttttt ctaatatgtt 333720
aattctgtac caagatttgt tcatctgtta atggatcaat atcacatagc tttgtttata 333780
gaaggtgcac attgttttaa ttctggccat gctatcccac caaatattgc cctttgattt 333840
ttcagagttt tactgtggat tcttccttgt ttctttttct atgtgacctt tagaattaac 333900

ttgtttaatt ctataagtaa atttattgat aattttattg aaattatgta aaatataaat 333960
tggcttagga agtactgata tctccatgac attaagtctt catatctaag accaaggggt 334020
gccttttcat ttgttaaata ttctttgtgt ctttcagaag tgttttcaag tatccttatg 334080
tgtattttgc acaaattttt atatttgtgc ctattggatg ccacgtgcag ggttaaaaaa 334140
tattatacct attttatata gatatatatg tatataaaaa tatatattat atatagttat 334200
aatacataaa tatattttaa tatattatat aatatataca tgtattgtat ttatatatgt 334260
atttatatgt gtatctataa atatacatat gtatgtatct ataaatatac atatatgtgt 334320
acatatacac atatatacac attatatgta tgtttataca catataaata catatataaa 334380
tatactagaa agtccatggg gtttcctagt atacaatcat attgtctgca aatagagatt 334440
gttatacctt ttcctttcta ttccaaagtg tctaaaagat tttttcttag ctagtggcat 334500
tggatgacac ctataatgtc ttctaaaaat agtagcagtc ataggcacca tttccttatt 334560
cttgaatatt cattcatgtt acaaagttta taggaatttc tgaattatta agtactttta 334620
ataggaatga aggttattgt cattattgca tcaaaattca taagaaagtt tggtgtcaaa 334680
tttgtgcttt gtgtgtagtc ttcatcacgc tttttttttt ttttttaacc agtgttaaac 334740
atccattaca aaaatagttt ggaagttttt cttttttttt ttcctatgtc ctggaaggtg 334800
taagtagaat tggaattatt tgatctttaa gatgtaaaat attggtagaa ttctaccttt 334860
gaaagcacct ggacatggtg acttcttaat aggtgagctc tctggaaact ttatttcttc 334920
tgtagtaatt ggtctcctta gactttctat tccttctgga gtcagatatt gtatatcaaa 334980
tcttctgtta aagttatcca tttcttcttg gtattccaat ttttcacatt gagttggaca 335040
aatgatttct gatgatgttt aaaaaatttc tctggtttga tggttacttt gaccgtataa 335100
tttattttgt gtatttatgc ttcttttaga aacttaggtc agctagtggt tcctctattt 335160
tgttgatttt tttcctcaaa gaactagctt aagtttattt tattagttct actagttttc 335220
tgctttctaa ctcattaagt tatgccttta gctttattaa atccttcatt ctgctatcag 335280
ttaatgtttt agttcatttt ctagcttttt gagttgatac ttaatttatt ttcatttcat 335340
gatataggta actgatgctc agaattttct caaagcatca gttttcgctg ggccccatag 335400
attctaatat atcttacttt cattattatt atttcctaaa aattcagcaa tttcatgttt 335460
tattttgtct ctcattcaag agttgtttaa tagacagttt aatttccagg ttggatgggc 335520
attttgtttt gtttttgtt ttactgttta taaatacttt tggccttggg atcagagaat 335580
actgcttgta ttacaattat tactgaatat ttttgtggtc taatataatt tcagctttca 335640
aaaatgttcc atgtatactt gaaatgaagg tttatttacc cttataaaga tagaaggtaa 335700
aatattagct atgcccatgt ctatttatct tcttataatt tttgcttaat gaaaactaat 335760

gatccattat ctgctgcata agtatttatg tttgctttct ctttattatg ccttatggct 335820
tttgtgttaa aaagctattg atttatttaa tattttggaa ggattgaatt ttaccctatc 335880
taatatcaag atcatgactc ttgattcctt tttgttttca cttgtttaat atatctttct 335940
tctatcttta ttttaaacac aagaagagct ttgctttcag tttctatttt acatagtcta 336000
caatcggatt ttacttatta gtcaatctga gaatactttt aatagatgag ttacacaaat 336060
acatatttat tgataccaac acagtttaat tcaagatctg ttctattatt ttatatttat 336120
tatatattaa attgtcttct ttatttacta tttattgatt tattgaattt attttgcttt 336180
caggtatttt gtatttttgt tctggttgtt agatttatgc atacttagtc aactcttcct 336240
ttataaagca ttcattttct actatgagca gtagtaaaat tagctagtaa cttaattcct 336300
gagcactctc cagtcctagt cactagtaat aacgcagtgt taaactttgt aatattaatt 336360
atacttaagc ctctattact tggtttgtca aaatcaaatg atatcaattt acttccagca 336420
catacctatg aggtaattgt aagctaataa taacttccat ggctattctt ttctcttttt 336480
ttttttttga ggtggagttt cgctcttgtt gcccaggttg gagtgcaatg gcacgatctc 336540
ggctcactgc aacttctgcc tcccgggttc cagcgattct cctgcctcag cctcccaagt 336600
acctgggatt acaggcatca gccaccccac ctggctaatt ttgtattttt agtagagaca 336660
gggtttcccc atgttggcca ggctggtcac gaattcctga cctcaagtga tccaccccat 336720
cagtctccca cagtgctggg attacaggtg taagccaccg cacccagcct ctcatcttat 336780
ttttattact tatatttttc tacattatta gaagttataa tacttatatg cacatttctt 336840
tagccccaac cccatttttt gatattagct ctattattaa gatatttaat gtccaccttc 336900
agattatatg ctgatgattc tctaatcgtt tttggttgtc tggagctttt ttactgttta 336960
ttttcaggaa gagctcatga taactctttt ttctttggtt taaaaatgtt tataattatt 337020
tttctctgga ctttatgctg agaaatttgg ctgcatacaa aatctctggc ttacattttt 337080
tctcaagtat ttttaagtga ttgcttcatt attttgtagt ataaaatgtt gctcacaaga 337140
agctggatgc cagtctgatt ttctttccct cattagtgat ttggactttt tgtctgaatg 337200
ttgaattttt aaatataaac ataaattgca atacttttat taagatatgt ctctgcattg 337260
aacattttag gtccgttttc ctaggtacat agaatgtctt ttgaatatgt agactcaaat 337320
cagacttctt gattctcttg atttatagtt ttaaatattt gtttgggggc gggtgcggtg 337380
gctcatgcct gtaatcccag cactttggga ggccgaggcg ggtagatcat aaggttagca 337440
gattgagacc atcctggata acatggtgaa accccgtctc tactaaaaat acaaaaaata 337500
tagccaggtg tggtggcagg cacctgtagt cccagctact cgggaggctg aggcaggaga 337560
atggcctgaa tccgggaggc agagcttgca gtgagccaag atcacgccat tgcactccag 337620
ccttggcgac agagtgagac tttgtctcaa aaaaaaataa atagggggcg gttccaagat 337680

ggcagaatag gaacagctcc agtctgcagc tcccagcatg agtgatgcag aagacaaatg 337740
atttttgcat ttccaaatga ggtaccaggt tcatctcact ggggactatt ggacagtggg 337800
tgcaggacag tgggtgcagc gcaccaagca tgagccgaag cagagcgagg catcgcctca 337860
cctgggaagt gcaaggggtc agggaattcc ctttcctagc caaggaaagg ggtgacagac 337920
ggcacctgga aaatcgggtc actcccaccc taatactggg cttttccgat ggtcttagca 337980
aacggcacac caggagatta tatcctgtgc ctggctcaga gggtcctaca cccacagagc 338040
ctcgctcatt gctagcacag cagtctgaga tcaaactgca aggtgcaagc gaggctaggg 338100
gacgggcgcc tgccattgct gaggcttgag taggtaaaca aagtggctgg gaagctggaa 338160
ctgggtggag cccaccgcag ctcaaggagg cctgcctgtc tctgtagact ccacctctgg 338220
gggcagggca tagccaaaca gaaggcagca gaaacctctg cagactgaaa tgtccctgtc 338280
tgacagcttt gaagacagta gtggttctcc cagcatgcag cttgagatct gagaacgggc 338340
agactgcctc ctcaagtggg tccctgaccc ccgagtagcc taactgggca caccccagta 338400
ggggcagact gacacctcac atggccgggt actcctctga gacaaaacct ccagaggaac 338460
gatcaggcag caacatttgc tgttcaccaa tatccgctgt tctgcagcct ccgctgctga 338520
tacccaggca aacagggtct ggagtggacc tccagcaaac tctgacagac ctgcagctga 338580
gggtcctgac tgttagaagg aaaactaaca aacagaaagg acatccacac caaaatccca 338640
tctgtacgtc accatcatca aagaccaaag gtagataaat ccacaaagat ggggaaaaaa 338700
cagagtagaa aaacagaaag ttctaaaaat cagagcacct ctcctcctcc aaaggaacgc 338760
agctcctcac cagcaacgga acaaagctgg atggagaatg actttgacta attgagaaaa 338820
gaaggcttca gacgatgaaa attttctgaa ctaaaggagg aagttcgaac ccacggcaaa 338880
gaagttaaaa accttgaaaa aagattagac gaatggctac ctagaataac caatgcacag 338940
aagtccttaa aggacctgat ggagctgaca accaaggcac aagaactatg tgacgaatgc 339000
acaagcttca gtagctgatt caatcaactg gaagaaaggt tatcagtgat ggaagatcaa 339060
atgaatgaaa tgaagtgaga agagaagttt agagaaagaa gaataaaaag aaatgaacaa 339120
agcctccaag aaatatggga ctatgtgaaa agaccaaatc tacgtctgat tggtgtacct 339180
gaaagtgatg gggagaatgg aaccaagttg gaaaacactc tgcaggatat tatccaggag 339240
aacttcccca atctagcaag gcaggccaac attcacattc aggaaataca gagaacacca 339300
caaagatact ccttgagaag agcaactcca agacacataa ttgacagatt caccaaagtt 339360
gaaatgaagg aaaaaatgtt aagggcagcc agagagaaag attgggttac ccacaaaggg 339420
aagcccatca gactaacagc ggatctcttg gcagaaactc tacaagccag aagagagtgg 339480
gggccgatat tacacattct taaagaaaag aattttcaac ccagaatctc atatccagcc 339540

aaactaagct tcataagtga aggagaaata aaatacttta cagacaagca aatgctgaga 339600
gattttgtca ccaccaggcc tgccctacaa gagctcctga aggaagcact aaacacggaa 339660
aggaacaact agtaccagcc actgcaaaaa catgcaaatt ggaaagacca tcgaggctag 339720
gaagaaactg caactaacaa gcaaaataag cagctaacat cataatgaca ggatcaaatt 339780
cacacataac aatattaacc ttaaatgtaa atgggctaaa tgcttcaatt aaaagacaca 339840
gactggcaaa ttggataaag agtcaagacc catcagcgtg ctgtattcag gaaacccatc 339900
tcacgtgcag agacacacat aggctcaaaa taaagggatg gaggaaaatc taccaagcaa 339960
atggaaaaca aaaaaaggca ggggttgcaa tcctagtctc tgttaaaaca gactttaaac 340020
caacaaagat caaaagagac aaagaaggcc attacataat agtaaaggga tcaattcaac 340080
aagaagagct aactatccta aatatatatg cacccaatac aggagcaccc agattcataa 340140
agcaagtcct tagtgaccta caaagagact tagactccca cacaataata atgggagact 340200
ttaacatccc actgtcaaca ttagacagat caacgagaca gaaaattaac aaggatatcc 340260
aggaactgaa ctcagctctg caccaagcag acctaataga catctacaga actctccacc 340320
ccaaatcaac agattataca ttcttctcag caccacgccg gacttaatcc aaaattgacc 340380
acatagttgg aagtaaaaca ctcctcagca aatgtaaaag aacagaaatt ataacaaact 340440
atctctcaga ccacagtgca atcaaactag aactcaggat taagaaactc actcaaaacc 340500
gctcaactac atggaaactg aacaacctgc tcctgaatga ctactgggta cataacgaaa 340560
tgaaggcaga aataaagatg ttctttgaaa ccaatgagaa caaagacaca acataccaga 340620
atctctggga cacattcaaa gcagtgtgta gagggaactt tatagcacta aatgcccgca 340680
agagaaagca ggaaagatct aaaattgaca ccctaacatc acaattaaaa gaactagaga 340740
agcaagagca aacacgttca aaagctagca gaagacaaga aataactaag atcagagcag 340800
aactgaagga gatagagaca caaaaaaccc ttcaaaaaat caatgaatct aggagctggt 340860
tttttgaaaa gatcaataaa actgatagac tgctagcatg actaataaag aagaaaagag 340920
agaagaatca aatagatgca ataaaaaatg ataaagggga tatcaccact gatcccacag 340980
aaatacaaac taccatcaga gaatactata aacacctcta cacaaataaa ctagaacatc 341040
tagaagaaat ggataaattc ctcgacacat acaccctccc aagactaaac caggaagaag 341100
ctgaatctct gaatagacca ataataggct ctgaagttga ggcaataatt aatagcttac 341160
caatcaaaaa aaagtccagg accagacgga ttcacagctg aattctacca gaggtacaag 341220
gaggagctgg taccattcct tcggaaacta ttccaatcaa tagaaaaaga gggaatcctc 341280
cctaactcat tttatgaggc cagcatcatc ctgataccaa agcctggcag agacacaaca 341340
aaaaaagaga attttagacc aatatccctg atgaacatcg atgcaaaaat cctgagtaaa 341400
atactggcaa accgaatcca gcagcacatc aaaaagctta tccaccatgg tcaagtgggc 341460

ttcatccctg ggatgcaagg ctggttcaac atatgcaagt caataaacat aatccagcat 341520

ataaacagaa ccaatgacaa aaaccacatg attatctcaa tagatgcaga aaaggccttt 341580

gacaaaattc aacaaccctt catgctaaaa actctcaata aattaggtat tgatgggacc 341640

tatctcaaaa taataagagc tatctatgac aaacccacag ccaatatcat actgaatgga 341700

caaaacctgg gagcattccc tttgaaaact ggcacaagac agggatgccc tctctcacca 341760

ctcctattca acatagtgtt ggaagttctg gttagggcaa tcaggcagga gaaggaaata 341820

aagggtattc aattaggaaa agaggaagtc aaattgtccc tgtttgcaga tgacatgatt 341880

gcatatctag aaaactccat cgtctcagcg caaaatctcc ttaagctgat aagcagcttc 341940

agcaaagtct caggatacaa aatcaatgtg caaaaatcac aggcattctt atacaccaat 342000

aacagacaaa cagagagcca aatcatgagt gaacttccat tcacaattgc ttcaaaggaa 342060

taaaatacct aagaatccaa cttacaaggg atgtgaagga cctcttcaag gagaactaca 342120

aaccactgct caacaaaata aaagaagata caaacaaatg caagaacatt ccatgcttat 342180

gggtaggaag aatcaatatc atgaaaatgg ccatactgcc caaggtaatt tatagattca 342240

atgctatccc catcaagcta ccaatgactt tcttcacaga attggaaaaa actactttaa 342300

agttcatatg gaaccaaaaa agagcccgca ttgccaagtc aatcataagc caaaagaaca 342360

aagctggagg catcatgcta cctgacttca aactatacta caaggccaca gtaacgaaaa 342420

cagcatggta ctggtaccaa aacagagata tagaccaatg gaacagaaca gagccctcag 342480

aaataatgcc atgtatctac aactgtctga tctttgacaa acctgagaaa aacaagaaat 342540

ggggaaagga ttccctattt aataaatggt gctgggaaaa ctggctagcc atatgtagaa 342600

agctgaaact ggatcccttc cttacacctt atacaaaaat taattcaaga tggattaaag 342660

acttaaatgt tagatcaaaa accataaaaa ccctagaaga aaacctaggc aataccattc 342720

aggacatagg catgggcaag gacttcatgt ctaaaacacc aaaagcaatg gcaacaaaag 342780

ccaaaattga caaatgggat ctaattaaac taaagagctt ctgcacagca aaagagaaac 342840

taccatcaga gtgaacaggc aacctacaga atgggagaaa atctttgcaa tctactcatt 342900

tgacaaaggg ctaatatcca gaatctacaa agaactcaaa caaatttaca agaaaaaaac 342960

aaacaacacc atcaacaaat gggcgaagga tatgaacaga cacttctcaa aagaagacat 343020

ttatgcagcc aacagacaca tgaaaaaatg ctcatcatca ctggccatca gagaaatgca 343080

aatcaaaacc acaatgagat accatctcac accagttaga atggtgatta ttaagaagtc 343140

agggaaaaac aggtgctgga gaggatgtgg agaaatagga agacttttac actgttggtg 343200

ggactgtaaa ctagttcaac cattgtggaa ttcagtgtgg cgattcctca gggatctaga 343260

actagaaata ccatttgacc cagccatccc attactgggt atatacccaa aggattataa 343320

atcatgctgc tataaagaca catgcacacg tatgtttatt gcagcactat tcacaatagc 343380
aaagacttgg aaccaaccca aatgtccaac aatgatctgg attaagaaaa tgtggcacat 343440
atataccatg gaatactatg cagccataaa aaaggatgag ttcatgtcct ttgtaaggac 343500
atggatgaaa ctggaaacca tcattgtgag cgaactatca caaggacaaa aaaccaaaca 343560
ccacatgttc tcactcatag gtgggagttg aacaatgaga acacgtggac acaggaaggg 343620
gaacatcaca caccggggcc ttttgtgtgg ttggggaggg gggagggatg cattaggaga 343680
tacacctaat gtaaatgacg agttaatggg tgcagcacac caacatggca catgtatgca 343740
tatgtaacaa acctgcccat tgtgcacatg taccctaaaa cttaaagtac aataaaaata 343800
aataaataaa taaataaata aatactagtt tggctgcatt gctttgattt tcttctttag 343860
gggcctgttc atacatacaa aggataccat ttacctgtct tctatatgac tttctcttaa 343920
atcctttcat tcttttgttt taatttttat cttcacatcc tttatttctg tcactgtgct 343980
gtccatagag tttgtctgct gtgtgtcctt ataattaagt ctatgttctg aatgtttttc 344040
ctttttttaga aattcagttc taaagtgttt aatttctcgt atttttttttt ctgttgcctc 344100
accatatcat ttctgagttt tctgtttctg aaatgtgcaa ctaactcttt ccaagcattg 344160
accagattct tcagtctttt taattcattc tgaaataact gggctacagt ttcatctgct 344220
ttgtggacag aagtgccaca aagagccgaa ttgtcagtgc agacccacat gaatcataga 344280
tcttaacgag gtttttacta acgactagca aaggatacaa gctaaaaatg ggtacaagca 344340
aacacagcat cattcatcac tgtaaagact ctgaactatc acatggaact tcaaaaggat 344400
tcttcttctt tgctgcaatg tgttttgatt tttggagtga tagatgtttg ctaactacgc 344460
acgtgacaaa aatttgctta gaggaagcca tgttagtttt gatgctactc aactttgtat 344520
tttgttagtc atgagataga aagcctgtga gattacatgc ccttctttta gcagctgcat 344580
cccataaaat taaaaattcc agtttttttt aaaccatgaa caatttagtc agacttaata 344640
tatcctattt aaaacattct tagataagaa cttcctttgt tattttgcta atatacaatc 344700
ccaccaaatg tctacaagaa gggctagtta atcaatatac caaagatcaa atctatttat 344760
atgaaccaca aatacaaaac ataattatac atatataatt ttatataatt taatttataa 344820
ttatatacat tttatagcag caatcaaaac tatcatgtat tttgggatga atttaataaa 344880
gaaatagcag gagctctgta gaaacaagta tgaacttcta ttggaagaca gtaatatcat 344940
aaaacattaa aaagaaagct ataccatgtt tatggttagg aagattcact attgaaaata 345000
catcaattct tgacaaatcg gtctataaat tcagtgcgtg tccaatcaat atatttagct 345060
catttaaaat gtatatggaa aaggctaagt accaaagata atcaaatgct cttggaagat 345120
gaaaattaaa gttgagagat ggggtaaaaa actataattc agttctaaag tcataaagct 345180
ataataatta atacagtatg gcattggcat agaaataagc acattgacta aataaataga 345240

aaatacagtt cagaaaccaa actctacatt tatgaaaaac tgacctatat cagaacgagc 345300
attgagacta caggagaaca gaggaactag taagttaatg gtcctgacac aattgatatc 345360
tacatagaaa aaaaaactta atccttcttt actctatgta aaaacaataa ttccagatga 345420
atgtaggact taatggagag ccaaaactaa aacttttata ggaaaacata gaaaaatacc 345480
tatctcaggg tcgggaatta ggtcttcaac atggcaccaa aagtactaac cataaaagaa 345540
acattaataa agtcaacctt tttaaaatca acaactttaa ttaaaaaacc agggccaggc 345600
acggtggctc acgcctgtaa tcccaggagg ccaaggcagg cagatcacga ggtcaggaga 345660
tcgagaccag cctggccaac atggcaaaac ctcgtctcta ctaaaaatat aaaaattagc 345720
tgggcgtggt ggcgggtgcc tgtagtccca gctacttggg aggctgaggc aggagaatgg 345780
tgtgaacctg ggaggcggag cttgcagtga gctgaaatcg tgccactgca ctccagcctg 345840
ggcaacagtg cgagactcca tctcaaaaaa caaaaacaaa aacaaaatca aaacaaaaaa 345900
caaaacacag gttgctggaa aaatggttac ttatttttat tatgctttat aaaatataca 345960
gttatattat caaacttctt ttgtaagaat caaatattat gctgagaata tggcaagaaa 346020
aaagaaaaca acctccacca ctcataatgg cattcccttt acccaccatc aactaagtca 346080
ggaacctgcg tcactttcaa gttttctgaa atgtggactg aaaatacttg tggagggccc 346140
tcacagatgc atttagaaat ggtatgttct ggaaaatgga actgaaatgt tttagcgaga 346200
gtcatgggcc caaaccttga aatagaggtt ggtggagttt tctatagtat gtaattacaa 346260
cacaataaga ctcaaaagca tttcaataaa gtgtggatgg aagacaagat actgcttcaa 346320
gggcttcatt agctttctgc agactggagc tctccaaggc atggggagga accctgttta 346380
cctttggttc agaatcagtt ttaaccatca ttgagggcta ggtcagagtg cagcattaag 346440
tccttgggaa tacactgggt ggggaaagaa aagagagcaa tgtttttcta aaagcccaga 346500
aatgggctta ctgtgtttac cagttgtttc caggataatg tattgcagtg ccaattgctg 346560
atgagatggt aggattatac ttcagtccct gctttacatt tatttcttaa agaagcttct 346620
ggtaaattag agcaatagca tcggcttagt ttagtgttgt tctgttggac taaggatatc 346680
agttctatcc gtatggtcgg gcctaaagcc tgggaaatat ttaatgaagg gagagagggg 346740
gagagagtga gcatgcaaaa gagagagaga aaaacaaata acaaaacaaa aaccaagaca 346800
tttcccttta tagtaagaat gatgaggaaa acatgtttag ccatacaaga tatcaagata 346860
atctcttatt tctttcttga aaatgcaagt acaaatgcct gcaaagataa aatatcctct 346920
ggatggagtg gaaaggttca ccaggcctct gaaatcacgt gaatgatgtt gcgctttgct 346980
gttaatgaag ctcggtgcat ttttcatttc agtttctact aagcatttat gagctattac 347040
ccttcccttc cctaaactgc gttgtttttt aaaaagcctt agaggcattc cttctagaaa 347100

ataaaggtaa gtgttaagtg gtgataattt ggtaataggt gtcatgcttg tggttcataa 347160
tgtgttaccc tacacatttt ctcaattatc cctagaacag ctttttgagg atatgaagtt 347220
agaccttaca aagcacatct ttctgctgga aaaatgaggt ttatagtggt taagtttagt 347280
tgcttatgat cacaaggcta gagagtggcc ggaatcaaga ctcttcaccc tgatttcagt 347340
gctttttcac tccaccatta atatttattg ttgataaata atatcaacac tttcctaggt 347400
gtattagggt catctagagg gacaagacta ataggataga tgtatatatg aaaaggagtt 347460
tattaaggag tattgactca caccatcaca aggtgacgtc ccacaatagg ccatctgcaa 347520
gctgaggggc aaggaagcca gtctgagtcc caaaacctca aaaataggga agctgacagt 347580
gcagccttca gtctgtggcc aaaggcccaa gagcccctgg caaaccacta gtgtaggtcc 347640
aagagtccaa aagctaaagg attggagtcc aatgtttgag ggcaggaagc atccgtcatg 347700
ggagaaagat ggaagccaga agacagccag tctagtcctt ccacgttcct ctgcctgctt 347760
ttatcctagc cacgctggca tgatgatgac atggtgcccg cccagattga ggatgggtct 347820
ccatcttcca gttcactgac acaaatgtta atcttctttg gtaataccct cacagacaca 347880
cccaaggaca gcactttgca tccttcaata caatcaagtt ggcactcaat aataaccatc 347940
acaagtccac accttgtcaa cttgatccca catacatctc cttaaatcat acatgatctc 348000
caaatacaga caataatgtc ataattacac tgaacataat acaactatcg ttcatacaac 348060
cagaaatgca ccaattccca aaccaaatgt tattacataa agttaacaac acttaaatgc 348120
tgatatgaag tcaataaata cttttttttt ttaaaagatg gagtcttgct ttgttgccca 348180
ggctggaatg cagtggtgcg atattggctc actgcaacct ccgcctcctg ggttcaagca 348240
attctctgcc tcagcctccc gagtagctgg gattacaggc acccaccgcc acacctggct 348300
aatttttgta tttttagtaa agacggggtt ttgccatcct ggccaggctg gtcttgaact 348360
cctgacctcg tgatccaccc acctcggcct cccaaagtgg tggatcccaa agtgatccac 348420
ccaccttgac ctcccaggtt gtaagccact aaatcttatg tcacatgata caggaaaaag 348480
aaaggaagta aaatgaagat attgtcttag tacaagtgta tacaggcaga aagatgttct 348540
taacaaaata aggaggaaat actcatgaca attacagtaa cctggttgct gcaactcatc 348600
acatggtcgt agctgttatt gatgactacc ttcttctaca acccattctg ttttcccttt 348660
gcctctagca agtacctcag caggtcatgg ttctttacct ggtggagtgt cccaaacctt 348720
cattcctgaa gggtctgggc catttgtagt cctgcctgga tcgagttgtt gtcatttttt 348780
attgacctta atcacagggc ttggtaatac taagagacac cctaaggaat ttcctgtatt 348840
ccacacatgt tcttccttac cttcattatg gagtagtaga ctgagttcat cttgataggc 348900
taagtcagtc accccagcca acactaactc ctttcttagc ctgttgactt agaggtagga 348960
ggagcccgag attgcaatct taatttccag tttaatgaaa tcattattgt gtctcctggt 349020

ggcaacattc atcgccctgg aactgagact tctaggccag cagaacgtaa tgctgtggaa 349080

caagaagcac aaattttact agcgggtctc taggggtgat ggtgagtggt gccacttcca 349140

tttccacccc ttgattcctg gacccatgaa tcctggctat gggagaaaca gtaccaaata 349200

ctggacactg atccggagca tacacagcct tctggaaaac tttgccccag ccctgcaaag 349260

tattgtcgcc tagttggcat tgtaattgtg acttcaaaag gccattccac cgttctatca 349320

atccggctgc ttcaggatga tggggaacag ggtaagacca gtgaattcca tgagcatgag 349380

cccactgctg cacttcttta gccataaagt gagtgccttg ttcagaggca atgctgtgtg 349440

taatgccatg acagtgggta aagcattcca taagtccatg gatggtagtc ttggcagaag 349500

cattgcctgc aggataggca aacccatatc tagagtaagt gcccattcca gtgaagacaa 349560

accactgttc tttctgtgat ggaagaggtt cagtataatc aacctgccac caagtagctg 349620

gctgatcacc ccgaggaatg gtgccatatc gcggcctcag tgtttgtctc tgctgctggc 349680

aaattgggca ctcagttgtg gctgtagcca ggtcagcctt ggtacgtgga agtccatgtt 349740

gctgagcccg tgcgtaacct ccatccctgc caccacagcc actttgttca tgggcctatt 349800

gggcgatgac aatggtggct ggggaaagag gctgagtggt atccacagaa cgagtcatcc 349860

aatccacttg attattaaaa tcctcctctg ctgaggtcat cttttggtga gcactcacat 349920

gagatacaaa tatcttcaca gtttttgacc actcagagag gtccatccat ccacatacct 349980

cttccccaaa tttcattgtc atcagttttc caatcatgct tctttcaagt ccctgaccat 350040

ccagccaaac cattggctac ctcccatgaa tcagtatata atcacacatc ttgccatttc 350100

tccttctaag caaagtgcac agccaggggc actgctcaaa gttctgtgca ttgggaagat 350160

ttcccttcac tgctgccctt cagggatgtc ctagaaaggg gctgtagtgc tgcagctgtc 350220

cacttttggg tggtgactgc atcatgcaga gccatctgta aaccaggccc ttgtcttctc 350280

ttcctctgtc agcttatcat agggaactcc ccatgaggcc atcagtgcag gctgggggag 350340

agaaggcagg gtggcaggag tggggaccat gggcatttga gctacttcct catgtaaagt 350400

acttgttccc tcaggacttg cttgagcctg atcatgtata taccacttcc atttgatgat 350460

ggaatgctgc tgtgcacaac ccactttatg gctagatggg tcaaaaagca tctagttcat 350520

gataggcaat ttaggtagct tggtgatttg atgacctgta gtcaaacata cagtttctac 350580

caaagcacag taacaggcca agagttgtct ctcacaagga gagtagttat ctgcaggaga 350640

tgcagggcct tgctccaaat tcctagaggc ctcccctgtg attgacctat aggggcctgc 350700

caagggctcc aaacaacatc cctatctgcc attgacactt caagcaccat tggatctgcc 350760

aggtcatatg gcccaagtgg cagagcagct tgccaagcat tctggacctg ttgcagagcc 350820

ttctcttctg gatcccactc aaaactggaa gcctttcagg tcacttgata aatgggctgg 350880

agtaacacac ccaaatgagg aatgtgttgc ctccaaaatc caaataggcc tgctaggcat 350940
tgtgcctctt tcttagttgt aggaggggcc aaaggcagca acttatcctt catcttagaa 351000
ggaatatctt gacagacccc acaccactgg acccctagaa gttttactga ggtagaagtt 351060
ccctaaattt tagtcaattt tatttcccaa cctctggcac acaagtgtct caccaataaa 351120
tccagtatgt ttgctacttc ttgctcattg gatccaatca gcataatgtc atgaatataa 351180
tggaccagtg tgatatcttg tggaagagaa aaactatcaa gatctctcca acaagattat 351240
gacacaaagc tggagaatcg atataccccct gaggtaggac agtaaaggta tattgctggc 351300
cttgccagct gaaggcaaat tgcttctgct gggccttatg gacaggaatg gagaaaaagc 351360
catttgccaa atcaatggtt gcataccagg taccaggaga tgtgttaatt tgctcaagta 351420
atgaacccat atctggtcca gcagctgcaa ttggagtcac cgcttagtta agcttacaat 351480
aatccactgt cattttacaa gatccatctg tcttctgcac aggccaaaca ggaaagttga 351540
acggggatgt ggtgggaatc gccacccctg catctttgaa gtccttgatg gtggcactaa 351600
cttcctccag ggatgcaatg ttgtgtttga tttactattt ttctaggtag aggcagctgt 351660
aatggcttcc atttggcctt tcccaccata atagccctca ccctaccagt cagggagcca 351720
atgtgggggt tctgccagct gctaaatatg tctatgccaa ttatgcattc tggcactagg 351780
gaaatgacca caggatgagt ctgggaccca ccagccccac tgtaagttgg acctgagcta 351840
aaattccatt aattaactga tctccaaaag cccctacttt aactggagga ccacgtgatg 351900
ttttgggtcc cctggaatca acgtcagctc agagccagtg tccagttgtc cctgaaatgt 351960
ctgattattt ccctttcccc agtgcacagt taccctggta aaaggccaga ggtctcctgg 352020
gggaaggatg ggagaaagag tcactgcata aattgtcagc aatgtagtgg ggtccttcct 352080
caaggggacc cagcctcccc ttcattcaag gggttctggg tctataaact ggttcaagtc 352140
tgcaaattga ttgaggggcc gtgattcttt gtataatttt taatttaaat tagtcttttg 352200
tctacttgac ctggaagttt tctgcttata taaattaagc aagaatgcag taggcctctt 352260
gtcaatttca ctttttagga acactgagat taagtagcca atgccagagc tctacacaag 352320
tcagactatt ctgattgttg ccttgcctcc gctgaccttt acggtaattg cacccacctt 352380
gccttgatgc ttgagtgcca ccacttggcc catgccacct caggatccaa ttattaccat 352440
tgtatttaaa ttttgtagtt gaatgactgt ggttcccact ataatatcta gcatgcagag 352500
aagagcaatc acagagctct tcaaggatgc aggtgctgcc ctcacaaatc tatttcacaa 352560
tgtgctggtc aaaggtatat cttctgtact ctcccagctg ggatgagtag gactaaagtg 352620
acgaattcac tccaccatcc cagtctccat aagcctttgg atcccttcct ctacattaaa 352680
ccaagggaga tcaggcattt ccagctcact cacagtgggc caccttttga tctatatttc 352740
agctaaccaa gcaaataaac tattagaacc ttttttaact ctccaagctg caacattaaa 352800

tgcagaattc ctgcttagtg ggcccaaatc aatcaattca gcctgatcca actttatgtt 352860
ccttctacca ttagcccaca cccttaatat ccattcccat gcctgttctc cagatttctg 352920
cttatataaa ttagaaaact caagcagttc ttttggagtg tagcgcacct ccttgtgggt 352980
cacattctga acctcatctc taaggggcct gccgggactt tagtctagtt ctataactat 353040
aagcaaacag gggtattgag ggtgggtcct taggagaatc aacatcgtct ttcctggcaa 353100
ctgcctcaag ggaggtcatc gctgatgcct taggcagtgc agggttaaac tcctcagaca 353160
aaggtgaaaa gcctgatggc agcgcaggtg gaggagggga tgttgcctcc cctctgttgc 353220
ctgtttcctc tggtaaaaaa gattcatcag aatttagaag ctcagtgccc ccagccttat 353280
cagggtcctc ccgcatggcc ccattccaag ttgcagggta ccattctttt tcaatcaatg 353340
ctgtcacttt aacagtaaac acctggcaag gctgtgcacg tacctttcgt tgcaggtcag 353400
ccactcacat gataagagct tgtgtctgat tttccgcaat ttcagctctt tctctacagg 353460
agataagact cgaacccagg gcaatcttag aagatttgag gctcagaatg tggttctgga 353520
gctgggagat agaatccctg agttcatcat ttttttgttt tcatcacttt gattagtgaa 353580
cttaggagca accaactagc ttcattatat tccttggttc tccacatatg gttaaaggta 353640
ttaggtataa agtcactaaa ctccttgcct ctcatgagca gtgaatcagg agtatcagat 353700
gcatttattt tgcataactc tctacacagt tcacagcaag gactatcagt gttctccaca 353760
ctattagaag tagagtccct agcattttgg cgtctaatca tatttagcag ccaactccag 353820
aaaccccaaa accatctaaa gaaatccatc cgtaaaattc tgttcctcta gaaccactcc 353880
tggtaccaaa atctgtatta gtcaggattc gctagaggga caggactcac aggtgagatg 353940
tatatataaa aggcagttta ttaaggagaa ttgactcaca caatcacaag gtgaagtccc 354000
acaatagtct gcaagctgag gagcaaggaa gccagtccaa gtcccaaaac ctcaaaacta 354060
gggaagccga cagtgcggcc ttcagtctct ggctgacagc ctgagggccc ctggcaaacc 354120
actggtgtag gtctaagagt tcaaaagctg aagaacttgt agtccaatgt tcgaggccag 354180
gaagcatcca gcacaggaga aagatggaag ccggaagact tagccagtct agtccttcca 354240
tgttcctctg cctgtttttg tcctagccat gctggcagct gattagatgg tgcccaccca 354300
gattgaggat gggtctctat cttccagtcc actgactcaa atgttaatct cctttgacaa 354360
caccctcaca gactcactca ggaacaatac tttgcatcct tcaattcaat cacattgaca 354420
ctcagtatta accatcaccc taggctttag ggatataggg aaaaacatga ctcattgctg 354480
ttatctgaga gtacataatc tattggaaga aaggaaaaca gttacatgta aggcttatat 354540
cagtataaat tagataactg ccaagtgaga gaggtagagg tagcaagtgc tgtctgtggg 354600
tgcatgttaa ctcagtctta atcttggaag aagtggcagt gtggaatggt acatggagaa 354660

gcagaaggga caattactgt gagcagatga atggctcact acaggggcat gggaagaaga 354720
tgcaagagca gtggaatctt taattgacga acctggtacc aagctgccaa gtcttagtct 354780
caggactggc accgtgcact tggccattga aaagactttg gaacctgtgg agtgaagagt 354840
tgtaggaatt ttcaaagcct cagtgtagga acaatgtgag aatcaaccag ggcatggtac 354900
tccacactgt gttcagatcc actggtgtta ggggagtccc agagaggcct cttagagggt 354960
tgaggaggat gggatcaggc atagcagaga ctagggacac gaggctctgc ggccagactg 355020
caggtctgct accaactaca gtgtgatttt gcctagtcac ataatctctg tgggcctcag 355080
tttttgttt tttgtttttt tctattacaa cagtaccttt ctcgtggagt tgtagtgatt 355140
agatatctag taccaagact atgcttgtta gcactgagta agaactcaat gaaggttagc 355200
tttcattggt actatattac tattgttagt gttggtggtg gagctgaatc gcctaaccta 355260
tgaggttggt gctgaaatga agaaaagaat aacagtgttt taagaagttt ggtcactaga 355320
agtggagctt agtaattaaa gaaaaagagt gaacactttt accttcttgt ggaagttagt 355380
aagtctacaa aatgttaatt ctgcatttga atgatcattt gggagactct tattgtccta 355440
tttgcactga aaaagtcact gaatcattat tttagaactg gaataacacc tgagatctag 355500
gccagcactt tgcaagttgt gctctatggg acttttcatg gaagtggctg aggagttgcc 355560
ttgaaggaag gcagagggag tgggtcttgg gacacccttc cagttataaa acaggacgtt 355620
gattctgttt tgtagttgca gcatcacata taatttcatt tattaagagg atcccacttc 355680
taaaaataag ttgaaaacca gtgatttaat ccagcctctc tgtttgcata tgaggaaatg 355740
gaattccgga aaggttaggt gatttgtcca aggttgcaga ctagactatt tattaataga 355800
gtagggtcag gaacaaaagc ctgctccttg ctggtccagc gcctgttact agttacataa 355860
tgaatgctac ctattgctgc acagtgccaa atcattgcac ttttcagatt ttactctaat 355920
caaaagaaaa aaattaaagt gcacttccaa atcagtactt atatgcaaga gcttcaagaa 355980
acaaactagt atttaacttg gtggttacat attgactgta ttttcattga gtgaggttag 356040
aagagattga gaagcgtgaa atgaagttac aaagtagaaa ctatatggtg aactcaaggc 356100
aaagattgtc catagtaaaa gaagacaaaa taagaatgaa agagacaaaa gaattgccaa 356160
tgagttgtaa tcttaaaaga aagatatatt taataaaata ggattgattg ttttgaatgt 356220
gggctgagaa gtcctgccat cttccattga ctctgctcac aggccttgtt tgtgaactgg 356280
cttccatgga tagcattact ctcctgacag ctgcagctcc aaattcagca tgaaaaggct 356340
ggcaattcta agaggaaggg agggtttagg ccacttttaa ttctactttg tcattgcaag 356400
tttcttgcat tgtttggcat ttgttggtct tccttactgg atttcaaaac tagaacactt 356460
tttcctagcc ttggaaccaa ccacgaaaat aaccacctct taccctcatg aagaacactt 356520
taagtttttc tcttttaaaa atgaagtctg gaatatctcc aaacatcttg tcactcacgc 356580

cttcatttaa atgtcaccct ctcaaccaca aggaaactaa aattgccccc tacttgctac 356640
tccctgtttt attttctagt agaacatact acagttggag atcatctcat ccatgtcttt 356700
tattcttgtc tcttatttgc ctctgcttga ctgtaagctc atggaaagta agggaccttc 356760
accaactagc aaagtgctta ggacctagta agaacctggt caattctaac tgagtgaatg 356820
actgaattcc tgtgagaagt caacatgaaa attcctaggt cattagttta gttattggaa 356880
ctccaacact gtattggaaa agtgtcatgg aaaggatagt tagtggatta aggtttttta 356940
taagaggaag agggaaagaa aggcattgtc tttacttcag gcatttcaat ggccatgatg 357000
gagcctaaat attgtatgtt gcattttgtt ttggttttgt gtgcacaaga gctttgccag 357060
tggaatgaac gggttaagaa atgtgagttt tcttttcgtc ctgctataga gacttaagga 357120
attgccctgt gtgagttcct tgagggcaga gaaaaataca aatccatgaa tacctgaaag 357180
ccctgatttg gcccctaagc aatctatgca tgtaacaaca ttgcatttgt accctgtaca 357240
tttataaaca ttttaagaaa agtataagag aaacacaaat tttggttact cctctctgaa 357300
aactgctctg atgtcgcttg gccgggggaa ttaagacctc ttcctgtggc ctgtggccac 357360
tatgtgctcc cggctctcca ggctcatcca gggccactgt gcctggggtc acagggttcc 357420
aggatgtcaa ccttctttca gcttctctga ctcctcaagc attttttttt tttttttttt 357480
ttttttttgg tttccaagac ccaaacgcat gtctctcttc ttggcacact cttcccaact 357540
tttttcctgg ctaattgcta gttatttagt ttgagattaa atgtcactcc ctcggcctgg 357600
cgcggtggct cacgcctgta atcccagcac tttcggacgc cgaggcaggt ggaccacgag 357660
gtcaagagat cgagaccatc ctggccaaca tggtgaaacc ccgtctctat taaaaataca 357720
aaaaaattta gccaggcgtg gtggcatgtg cctgaagtcc cagttactcg ggaggctgag 357780
gcaggggaat tgcttgagga ggatattgca gtgagctgga tcgcacctct gcactccagc 357840
ctggagacaa agtgtgacac catctcaaaa aaaaaaaaaa aaaaaaaagt cactcactca 357900
cagagggctt ctcagatact cccctgcccc ctcatttaag ttgcaccgct tgttgtattc 357960
tcttataagc cccattcttt ttcttcttgg cattgatcaa attaacagct ttattttatt 358020
ttagcattgt gagtgtatat gtgtgtttaa tgtctgcttc ctgactaaac tgtaccctgc 358080
aggaaggcaa aactgtgtct gtgttgctca ttgttaaacc ttcagcactg aactcagtgc 358140
ctcgaacata ggagattcca actcaatatt tactgcgtga aggaatgaat gaatctttat 358200
gtccctcgtg cctaacataa agtctgccat atacaatgga ctaaaaaata gtattcagct 358260
aaaactgagt tacggagaag atgaagtatt aattgtattt tttacagaga aacaatggtc 358320
agtgtatcaa aaatagagac cctgctctca gatatataac atagaactct cttcattcat 358380
ttgccttcat tttagttaac agaatctgtt catctaagta gagcagagaa aaacttcata 358440

attgctgtgt ctgtgttatt ccaaaacatt taaacaaaag gtgaataact gaggtattct 358500
tccctgtggt acatacttga tgtgggcatt ttaaaagatt gatcaaatct cctttcagct 358560
ggatatttga gtaggcacaa ccattacaga attttcctct agggacttac tttagctctt 358620
aaactatgta aactgaacaa gcaaactcaa gtggatcatt atctatagaa gtatagaatt 358680
ccatctccct ttggcaaacc attcaaaccc agaagtgtgt tttacacatt ctgacagaag 358740
catctgtaat aaccaactct aacctccttt cttaccactt tagcacccaa aagtataaga 358800
aaagaggaca tgttaaggct tgttctattt attagaaaat atataaagag ttcttggctt 358860
aagaactctt ctggctatca gctccctgat gtgaaaaaag taaatagcaa ggggtagcat 358920
ggagtcttac tcccgtgtga cagacagctt aagaaagaca attggacatc atgtgactac 358980
atgattcaag ctaaagtcca gacacatctt ttccataggc caattgaaca ttttctctgt 359040
aatttccaca ataaccattt gcaccagcat gaatggagag ggtctgagtt cttctgggtg 359100
agtaaagggt gtgtcagtta tctgtcctct gtcaccagga tttagaggca ggctcatagt 359160
gactcttgta aagttgaggt tcgcctgtgg aggctgcaaa aaagagggag gaagagagag 359220
tgaggttcct cttggctctg tgccagggat gtgattagag gaccctggga gggccttcat 359280
cctacgaggg gttggaagtg gaaatggatg tgtgtggatg acctggtaac gtgtcacagc 359340
cccttccacc ccatagtagt cagggattta atgcctcaac aaggcaggtc tctgaaggag 359400
actgactttt ctctctctct ctgaatgata ctgcctgaga gaaatactca cttcctgctt 359460
tgttttcaac aagtatggac ttccttacac aaaaagaatc ttttttgctt ttgtcccccc 359520
atttcactgg aaatctatcc actgggcacc actgttggtc ggcttccttt catagattcc 359580
tttatgtttc aaattttaaa aagacaataa tagcaacaaa gtgaaatatg gatttaagct 359640
aggaaaagca gagaactgaa actttttttcc tgcaatcata cttcccagct tcttcagcaa 359700
gatctgcttg gctgggaaca tgccttctga gaactttaca tttctaaact gctgctaaat 359760
tgctccatgc atttattcct caaactctca gggaagatag cactggcttt cagtctcata 359820
ccaaccattc ttaaggtaat ggaccaaaag tcatttccac ttgaaatata atccttctaa 359880
atgaatcatg caacatggtt tttacctcca ttttttcaga ataactctaa gatgatgatc 359940
ctctggccat attttaaaaa tttctcttat ttgttttaat tttcttccat tttttcttta 360000
attttgtatc cagacactgc cttctctaag aggaatgaca tttttgcggg ttatcatgat 360060
atatttttag cccatttgcc ttgacccata gcttacaaca gttaaagaac actaacatat 360120
aaacatagag ttttgcccaa ggccattagc tttgcatgtt aacaagtggg cagaaggaag 360180
gctgggcatg agagagacat gctgtccttc tggtgagatc atcaatccca ttcttgtaac 360240
cttcagatca ttgcatttgt tcagaatcgt ctgcagagga aaacatctca aagttgggag 360300
gttgttgaag agtcagaaac aaatgggtcc tgggtaattt ttcaaaacag gctttttgtc 360360

aattgtcagt cagctgttta atattgtcaa ttgtcactca gctgtttaat attgtgcttt 360420
gttataagga taggcactgt ggtctcagcg ttcttaagct tttacatttc cgttatttgg 360480
ttttacttct gcattagtag agtagatgta gagtagatgc cacctctagg aactctacca 360540
gcagcagcat cttagactag aagctccctc tctaggacta taatctctgt agcctccact 360600
tctcccaccc cttatttctt ctgagcctac ttggtctttg atacttacta taatactttc 360660
tattctctta tcataaacag ttttctgatt tcattttcct ctggtttcat actaagcccc 360720
ataatcagtc actgcaagat taccttctag aatttctgtg ccctcaagtt ttctccatat 360780
ctagagagtc agtcatctgc cttgagccac atccactgtc tactttcttc acttccatcc 360840
ccaggctgcc cagcactgat gaaaacaaaa caaaaacaaa caagcaaaca aaaacaaaaa 360900
caaaaaactg actaggatct caatctggtt tgcaaatcca tgcttctctt tcttgacttc 360960
tttagacatc aacagaattg acgcctcctc ttgacctggg acacccctcc tccttggtgc 361020
tttcttctac ctccccctgc ttctctgtat gtcttttgca ggctcatctt tctcttaggc 361080
tgtcttcccc catctctcct ctccctctgt gcactcaggc agtctccctt gtcaaccccc 361140
aaaactgctt ctccagaccc cagtgtttct ctgagctcca ggtccacaat tctctaaggt 361200
ccatgtggat gtccctcacc atttcaaact tctcttccac agggagtccc tggttttcac 361260
taccatagca tattaaaata ccatgcattt cccttcatac cactaattac aactttgttt 361320
ctttttttta aattttatgt tttaaaaatt atcttctgcc cacatatatt gtaagctcct 361380
tttgagaaag acacattgct gtcctggtca ttactatatt cttatcaact agcaggggcc 361440
gtggtcgggg cttgtgttag tccattttgt gctgctataa aggaatacct gatgccaggt 361500
aatttataaa gaaaataggt tttatttggc tgagggtttt gcaaactgta caagaagcat 361560
ggcgccagca tctacttctg gtgaggcctg aagcagcttt tactcatggt ggaaggcaaa 361620
gagagagcag gcgtttcaca cagcaaagga gagagcaaga gagatgccag gctccttaaa 361680
cgacctgctc tcccataaac tgaaagagca agaactcact cattacggta ggatggcacc 361740
aaaacagttg tgagggatcc accccgaaca cctcccacca tgccccacct ccaacactgg 361800
gatcaaattt caatgtgaga tttggaggga acacacatct accctttatc agtgctcaat 361860
aagctcgctg aatgtagaac agtgatataa ggcaggggtg ggccaagtat aggcgacagg 361920
acaaatttgg cctgctactg cttttgtaaa taaagtttta tttatttaca aaataaataa 361980
ataaatacag tcataatcat tggttacatt ttgttcaaag ctgcttttgc attaaaacat 362040
cagagttgag tagctgccac agagactgta atggccccac aaagcctaaa atagttaccc 362100
cgtggtcttt tacagaaaaa gtctgatata aaggacagtc tcacagcaca gaggaaaagc 362160
atataaccca aaggaggaaa gagtagagca tagtggactg cagagaagtt tccctggacc 362220

caatctgtgc cttgaaagat gagaaggaca cctccaaagg caggatgggg gtgaggtggg 362280
gtgggcggtg gtgatccaag cagacagcac gggcaagtgt tctggaaaac ttgcctttgt 362340
tcacaagtat cctgcagtat aaattgcaag gtgggcagta agaaatggac ttgaagagat 362400
tgaacaaggg ccagaaaccc aaggacattg ctaaggagtt tgtgccttag cccatccagg 362460
gccctagcag ccttgactga tgcttctgag gtcctggagg ctccaggctc aggctacctt 362520
cccatattct ctgaaaataa tatcacattt gtttagtaaa aaaattgttt actaaaaaat 362580
taacctctca gaatttctgc attcacattt atttcttctc atatcctcga gttagaaaaa 362640
ggtatttctc tcctgtgatg atattgattc tgcactcatc tattttcatt actgtctcct 362700
tccctccctc cctcagtccc tcccttagtc tatctcactc taccatcttt tcctttggac 362760
cttctgacat tcaacatctt ggtcgctcct atccagccac acctctgacc attcaaccat 362820
ttgtcctctt gattggttcc accttgtcct atcacgctgg atatgctgtt gctcccaaat 362880
tgcctccctc tgccagtttc ttgccccttc ctccttctgt ctctccctct ctctttcaat 362940
ctttctctcc ctcagtctct ctttctcttt ccctctctct caatctttct cttcctcagt 363000
ctctctccca ttctctttct ctttctctca atctctctct tcctctctct cttatcctct 363060
ctttctctca atccctctct ccctctttct ctctctcaat ctgtttctct gttaatctct 363120
ctttctctct ccttctttct gtctctgtct ctgtctgcct ctctctctct ctctctcttc 363180
cctagtgagc tcacttgctt tcccagtttc agtgatttct attcagatat ctccaaaatt 363240
tgtatttccg gatattttat gctttcatgg aggccgtgta aattggtata acctttttgg 363300
aaggcgtttt ggcaatactt agtaaaaggt taaatgttta caccccttga ttctgctatt 363360
gtatgtgtaa ggatttttc ctgcatactt ttgtaagtgt agaaaatata tgtataggaa 363420
taattgcaca ttgtttgttg tagctgaaaa ttacaaataa tataaatgcc tataatcaga 363480
gcagttaaat acatatgaac acagtgaaat accaggcact catttaaaag atgaggtata 363540
ttttaacaag cctatttgaa aagataccca atcttagcaa gtgaaaaaaa aactcatcaa 363600
attgatatat ttagcgctgt ccctctctca agtttcagac ccacatttc aattttctgt 363660
tgtcatcttg acttagtctc gtgaaatttt aaactaatca tttttaaggg aagatatttt 363720
cttcccttaa agatgaaatt cagacaaacc tctgaatttc cctctttctg ctaatttctt 363780
gctttctttt atcatctttc catccatcct aacatacagt tccagaggag tctttaaatc 363840
cttccttatt gcctaacacc gaggatcatt tgctgaaatc ttgttgattt ttccctcaga 363900
tcaacaaagg attttcagaa tagccctgat ttctctttat caccacggtg ctgtctcacg 363960
tcttctctcc ccctcctcct acctgccgtc tcttccagac tcgtctctct cccccaatct 364020
cctttgcgca ctgatgccaa attagtcaca acaaaagtgt ttacgtgatc gtgtcaccca 364080
ctggtaagct cttaatggtc ttcccttaca cattatctcc catttcatct caggagcctt 364140

ttattaacca ctcttcacag tctgactcag ccctctttgc cagtttctct ctttacaaat 364200
agcaaagtct atgagctaga ttactcaata ttccctgaat aggtcttctc ttctcccacc 364260
tcttggcctt tccagtacca ttcaatcttc ctggaaattc ctgaaatttc tttcctcctc 364320
actcagtcaa tgttttgaga cccacatcaa atgctacata ctatttgttc ctttactgat 364380
catgccagtt ggaaatgatt cgaatctgta aagcagggtg ctatggaatg acatttctta 364440
tagccctttg gtcatttggg agcaatgtct aacataatat cctccaggtc catccatgtt 364500
gttgcaagcg acaggatttc cttcttgtta aagggtgagt agcattccat tgtacatata 364560
ttccatattt tattttttt tgctcatctg ttgatggtca cccaggttga ttccatatct 364620
tggctattgt gaataatgct acagtgaact tgggagtgca gatatctctt caacatattg 364680
atttcatctc ctttggatat atacccagta gtgggattat tggatcaaat ggtaattata 364740
tttctaattt tgggcagggt cctctgtagt agctccataa tggttgtagt aatttacata 364800
tccaaccaag cacgccaggg ttcacttttc ctcatgtcct cgccgacaag cctgatattc 364860
ttatttgcct cttagcgctt cagcctttcc ctcgtgactt aacggtgact cccttgagac 364920
tacttgaaat aataagtttg gatggcaagg aaataccctt ctgctgtcac cctttgccat 364980
aagactgagt tactttgtaa acaaagaaga tttacttggt cttccatgcc caagaccctt 365040
ttacttttca tcgcttaata tttcatggcc aaaaactgtt ggttcttcca atcttgtaag 365100
gccatttatc tttgatcttg ctccattccc ttccattttt gcttgagaat cattcagttc 365160
ttgcctgatc tcacgtcttt ctcgcaacac cttactaatg actgccagag tgtccactac 365220
acactatcgg tctcatttga ccacttcccc aggcatgtag cctacctgcc aagttctttc 365280
acagcagata ttttgcagtg ttgtaagagg gctccctagc ttgctatgtt ttcttactac 365340
ctggcattag gaagtaaaca tttttcatgg cagtattcac ttctagtacc aatttctttt 365400
ttaatctaca tgggctaact gctgtatcaa acagccatca aatcccaatg ttaattgcaa 365460
cttacagtac gttgtgatgt ttctgtctgt atactcattc agacacacag agtttaagta 365520
atttgttcaa gatcacaaag ttagtaaatg gtggcatcaa gatttgaacc caggcagctg 365580
gactcaagag tctaaacaac tttcatttta aaattttcat ctttcaagat atatcactta 365640
caatttccat cttcatcact ttcttccata cttcagagct gcatatgtaa acctcttgat 365700
catataaata tttatggagt ctcagcattt tgagcacatg ctgagagatt ttgtagcatt 365760
gaagataaag aagctctttc aatacatcta atcttgccac tattgatttt gaacagcatc 365820
ttaatttgtt accttttttaa aaccatattg tacttttctt caggacagct aaattaaatg 365880
tcttatgttg atcccctggg ttttctcagt ttccatttat cagtttcctt ttatataaag 365940
agatgctaat gggtaacctt acattttgta aggcatattt ttgaagatat acaagtgaag 366000

gtttgatctg tacaccctcg ttagagctct ttattttaca tgtggaggaa gagatgcctt 366060
agggcttctg aaaaacaggc ttttattttt atagaggaga aacttccaga acatgccatg 366120
agcaaatgct ctgtcaagtc tggttctttc ccccattact taagtgcaaa tggaccacat 366180
ttgaagaaga taagtagcag aacaaggctc cccacaaggg gattttgagg tgctctgaaa 366240
accagactga cataatccat ggaggagttc atcactgcag gctgtcacgt ctcacagtgg 366300
gagtcatagg ctggtctgct tgataaacta ctcctgatct atgactggag ctgtgtgctt 366360
cctttaaaaa taaatcttgt ttttccatgc cccacgtgct tctcagcctc aggagcacct 366420
catggattta gactaatctt gtcttttaca gtcatgttac ttccagattc ctaggaatca 366480
ccccatttct gatgccgttg atccatccat acccactgaa attgagctaa atgctctagt 366540
ctgatttgcc ccttgaagtt agtatagaat attttggcta gactttgaaa atgaagccct 366600
gaccacaatg cataatgacc tcagtagaca ctgaagagtt cctttgcatc aggcaagtga 366660
caagtatcta tctcatcttc aaagtccaca tagatatgtg ccagattgta ggttgacaaa 366720
tcactttttc actagctcac gtggcaagcc ttttcacctt gtaataatgc tagtgagaga 366780
cccataggta ccacataatt ggccagtcat tgagattcaa gcaacatttg gataatttag 366840
caaaactatc atcagaatca tcaaagtaca taatttttat ttattatgca aatagtggat 366900
tcactaaagt gcttagacca attaagttgc tcccattgaa aaacagggtg attttattct 366960
taagaatata tcaggataaa cttcttgtag cctctaaaca gtactgctta gaaaagtgca 367020
gcttcactgc attgcatatc cccccagaag ccctcaaagt tcagatgtac accacaaaaa 367080
catgaagcac atgtgtttgc atggaaggta ggctctgggg tcttcctatc ataatatgtc 367140
tatgattaat agtcaacata tgccccctcc acattcccta aaggaacata cacatgcata 367200
cacacacaca cacacacaca cacacacaca cacgtgagaa ctgaattgag agtttggggt 367260
atacaccatc cctgagggca cagtctacag ctcctaatgt gtgctgtaga gcaagcatta 367320
tattgttgac aggtaataat tcctggttag gaataacagg ataccagtga gaagaggctg 367380
agaaaaatac actcttgctg gaagagtcaa gaacgagctc ttcgtgggaa actatgggag 367440
acgattgaaa aaggacaaga agaaaggaga attgttaagt ctccactttc tcagtttctg 367500
aactgaactt ttgctcacag caggtgaaca caggctccct cccacactga gacttcctcc 367560
cctgccggtg tgcagtctgt taagtaaaca gagacgttat actgagaaag gaacatttct 367620
ctattttggc tatgagttgt tatttttaaa gattcatcct ccaaacattt tcccctatcc 367680
ttgtttattt tttcactaga aagaagatga actgcttaaa aaaaaaattc aactccatga 367740
caagaaaaac ccacttctag tatgttctcc ccactcaatg ccaatggagc ctttggtgtg 367800
agcactctgc cagccacaca ttgggcagca tacctcttga aattcctgct gtgcttctat 367860
agagcttcca tgcatccaga ttttcaaagt caaagcaaaa tgcgcatggc agtttttttc 367920

tgactccttc aagttaaaag gccaacctct cccttgacat atccatgtat ttatcagtat 367980
atctataggt aatacttaaa ggtgatcctt actgtgtgcc acactccatt ctaagcactt 368040
tacataaaaa acctcatttc actttatttg ggaataggat gacaatttct tcaatattga 368100
gatgggttga atctggggag actgatgtta gatgcaaaaa gctttctttc tagaactttt 368160
cctgtgtctt gcacctggct ttctccctgt cttgttccca ttctagatct ctttactgac 368220
agtgcccatg gctttcttct tggtactcta gtgttgagca taagaactag tatgtaatag 368280
gctctcagta aatcttgttg aatgaattaa agtagagcaa gaattaaaga cagagaagaa 368340
gatcttcttg ttggagttct ttcttttggt tgataagcaa tgtgatttga cgtaacaatc 368400
ttgagtccta atcccctcat ttggaaaatg gaaataataa tgctaatctt agaggtcact 368460
tctaagaagt atatgaatat atcagaagta atcaacattt ttaagtcaaa gaaaagaacg 368520
tctgtgaaaa agaaaactaa tgagctagtt gaaaaaatat taattggatc tttttctcat 368580
accttacgcc aaaataaatc cccaaaaatg gttgcacaac attgtgaata tactgaaatc 368640
cattgaatca cactctttaa atgggcaaat gttatgttat gtgattatct ttcaataaaa 368700
ttgttataaa aatgaataaa ccccagaaga tcaaaaattt aaacataaaa gcatggaagt 368760
atactagaag gaaatataag ataattttat atgttatgtt gtatatattt atatgtaaaa 368820
ttttttttatt tatttttctt tgcttttaaa agcaagacat aatgttctag aggaataaat 368880
gaaacgttcg ataaatttaa ttctctatta aatgttttgc agaagaaaat accaggaaca 368940
acatcagaag aaaaaacaaa aaaccataag aaaaacaatt aaaaatacat atgacaaatg 369000
gcttattact ataatattta aagaactctt aaaaatctat gtgaaaatga caaataacag 369060
aaaaatacgt gcaaaggaca agagcaggca gtttagagaa agcctatacc aatatattct 369120
cttatcaaca gtctattgta caagctattt gaaagcaatg tttgaactat caaaatttaa 369180
aatacataag ccctttttatc tagcaattct acttccaaga atttattcta tagctatctg 369240
tacacatatc caaagacata aacataagga tagtcattga atgaaatgtt tttaacaaca 369300
aaatacttga aagcaaagaa atgtcaccca agaaagaagt gattgaagaa agtttagcat 369360
tcagttcagt gaagacatat tgagctgtca aaaaaagttg atggagaact ccaaggtcta 369420
ttaactaaaa acaaaacaag gtgctaaaca atatgtttag tgttcatatt ttaattgttt 369480
atatatttca caaatatata tttgtgcatg cagaaaatat tttaacttct ttctaacccc 369540
gtcaaacaca tcatgccaaa attcaacttg tgataaagaa aaaaataaat gaattccttt 369600
cgtgtgtagt atctcattta aattcttcaa caacccaata aaaaagatac agacacatgt 369660
ttttaagtac tacatgttat gcacctttgg tttgcttatt taatagttgc aactactaat 369720
gttaaattct tgatgccaag gttaaaagcc tgaacttcta aaaatatcaa gatctgacat 369780

ttttctgttt tcacagatta atctgccatc tcctgacctc caacacactg ctctatgttc 369840
tttcatgctg ctcctcctcc catgtttagg accctcttcc aaggaggcat tcacatctct 369900
gcttgatgaa accctacatt atcttaaagc gtccactcga atgccaccta cttatatacc 369960
atctcctgca ataccaatgg gctcagccca accatagccc atagttattg tagttgttct 370020
tgtacttcaa aagcactaca aaacacaacc atcaggactt gttacattat ttgaaggcta 370080
tgagcatctt cagccgaggc cctgttttta ttcccagaac taccacattg tttagaatat 370140
agtagcagat caatatacgt gtattagata aatcgtttac ccagatcttg atcattttca 370200
attacccata ggttgaagaa ctccatattt aacatggcag acttgaggac tgaactacct 370260
acctcttcta agaagttgaa atgagaatgt tttattgatg ggaaattatt tttttgtttt 370320
gccttctaga attcaaatga atgttcatat tccatgaaga caatggctga tagtttttttg 370380
ttaaagattt agaaccagtg gatttttatg aatgtgaacc ctttcatgtc ttgtggaaga 370440
ttttctgttt tttaatcttt ttatttattt atttattttt gctatgtttt cataggaacc 370500
agggaaaatg tgtagagggc atggtggaga tcttcgacat gctgctggct acatcatctc 370560
ggttccgcat gatgaatctg cagggagagg agtttgtgtg cctcaaatct attattttgc 370620
ttaattctgg tgagttgata acacaagata actcaatgct ggatgaaatg tttatttgta 370680
gttttcaacc agatacgatc tacccactcc aaaggcataa tgtcataaat agaaagaaac 370740
tactgacaca cgttttaaaa taacctacca acattgcaga ttccttataa aggtagaacc 370800
atgctagcca aatagacaca tgaaaaattg taatttggca ttgaatcaaa tggcctttga 370860
gctaaaattt ttgtatgctt tcacagatag gatgttttta ttcaaatggt acatgtatat 370920
agacatatgt tagttgatag ttatattatg tctgaaaata agtagaccaa gtaattctgt 370980
taagaaattg tgaccaattc caggctccag atagtaaaga aagagggtta tttgagacag 371040
accatgtttc tggtcaaaac tgactagcta aaaatatagt tggcttagag atagaaaaac 371100
ctgtttctaa aacagaagaa tgtggaatgc aataaattgt ccagctgaaa gaacattttc 371160
catttgctct atgaagtctg attctactgc cccttcgtat ttatttgttt gagaaagctt 371220
agctaagagc aacatctgtt ttttgttttt gtttttgttt ttgttttgtt tttgttttca 371280
atgtagtgag gctggctgtt gtataaagag ttactctatg tcaccaagat ggaaactatg 371340
gttcagcctg aattagtgcc ctgactcctc ctagcctctt ggtatttgga tctaagctct 371400
agctctacag cctctgggac ctaaagctca cattgggtat cagcggtaca gcagctcccc 371460
ttaagctcca cctttccccc tggctctaac cccactttgt gcctcatccc tcattctcac 371520
atagaaatca tagctatttc gaatctctgg gcctgactta gtttcttatg ccattagaca 371580
tagtctcaaa ttcctcaata gctgaattgc agcttgtatt gatcacatac atagagaatc 371640
cgcacttcct tctctttcca cgtgtttttg tctctttctg atgagtgctg atcccttccc 371700

gcttccccta acaatttttt acttttcttc cccaccactg ctaggaacct gtgttgctta 371760
atggaatcag cccttccttc tcttttcttg tgtttttgtc tctttctgat gaatgccaat 371820
cccttcccac ctcccatgac aattttccac ttctcttccc caccaccact agtaacctgt 371880
gttgcctaat ggttatcaca gtaatcattc tcacttatca actaataaat ggaaatctgt 371940
actattacac taggcaagga ctgaattaca gaatgaagtc ccatgataaa tgatgtgatc 372000
cacactaagg aagtgattac cactccattc aggtttctgt ttccactcac gtgcattgtg 372060
cttttcattt cagtcgtttg tcctgtacat tgtagggtcc agatcccaca atggctcttt 372120
attggatgag agttctggga gcagtgccac tcagctacat ggtgccaggt cctgaacctg 372180
tgccttcttc ggtggagggc tggcacgtgc tgacagcttt catgtgggca atctgggaac 372240
ttcagagaag gcaggcctat taagtgttaa gactccccac cccgaacttt tactgagaaa 372300
aagtacccca gacaggaagt aaaattagcc agagttgtat gatccacaca gtggatgctc 372360
tgatctcagt aataaaaaat atttcctcca gatccatata gacttttcct gcattattgt 372420
tttgtttctg ttcctatggc agagtgagtt tttaaaacta ttatgcaaga aatatcagga 372480
tttttgccac caaaaagttg gattcataga cccagggttt ttacaaccca gggggaaaaa 372540
actttcagcc ctcacaagaa agtattttat taaagactgg cacccaaacc tcaagactat 372600
atttctcact gcaggatttg gcccctgtct gcctccttct cagactagtt aaactttcat 372660
actccatcct tgttccttcg cttctttctt aggatcttct gtgcacttct ttcttaggat 372720
cttcttggca cttctttgct cttgaagggc agaaacgcta ttgtagaaat tcaaggcact 372780
gtatgctgac atagtttata gttgtctttt tcatgagata acacagcggc tggcagctcc 372840
tcttttctat caaggacagt actgctggct caggagagca gtaagcaaac agaagctgtc 372900
tctcaaccct gtacaaagcg aaaccactct tttccctaca aaagtgagct gtgccccaga 372960
aaagcggatc tgctgctgag cagggctcct catgtctcct attgttcctg aaaaacagca 373020
tcaaagaggg caagactgac acacaatgtt gtagcattag gagccttttt cagaataaaa 373080
aagcaatcag tcatatgaag catgtggtca taccacaatc aacaattttt ctccacaact 373140
ggagatatgg atttttctaa aagtccaact gattcatggc cctgatacgg ggcagctcta 373200
cctctcatga gaccaatgac caagtgactc tgactccagg aatctctgac agaagccaag 373260
ctggaaacgg ctcaggaaac ctgagctaga agatgccttt ctgacacctg ggaaacaaaa 373320
cctcagtgta tggaggaacc caaggttttg tgttgagccc cagtcgccaa ggtctgagga 373380
ggctgggttt atattggccc cgattttgtg gccgaggcac aaggggaatt gaaaggttgt 373440
tgtgaggacc tgctcggaat gtctttctgg aagtttggag agggtctctc aaatgccaaa 373500
atttccatgg gaagccattt tcacagctgc ttgggagtgg gagatattcg tcatcatttc 373560

tgccccattt agaataattt attactttgc ttgcaaaaac ccggaatcat ccataggaca 373620
cacgttgttt gtttttccac tccacaccaa aggacagagg cattcctggt taatccaaga 373680
tgccaacttc aagacattct gagaactagc tggacagagc tgaagtgtag ggcaaatcag 373740
aacagaagtg atgggcccag ggcaaaagcc cgtgaagcag cagctgcgga ggaaggatgg 373800
gagctggagc tgcaaagggg gccatggtgg agactctggg cctgcccaca gtatcccctc 373860
tgcccaatgg gaattcagtt ttcctgcata tcattcaatt atttctcatt tttctaggcc 373920
tagctcacct tattcgggaa gttttgcgac taccccagtc cataatgaat ttcctctcta 373980
gactatttca attcttacag tcactaccac acagtctagg atttggttag ttggttgatt 374040
tcattcattt attcaataat tcaatgttta ttgagttcct actatggact ttgtgccagg 374100
tatacaaaac tgaatgttac tgttgtgtgg ggtttttttc tttgcatttt taaagttaac 374160
ctggattttt ctctcactct ttatgcatgt atttcctaat ctccctataa actatttgta 374220
tttttttgga ttcctcacag tctatttagc ctgtggactg tcaacagatg ttacatcatt 374280
cagtcattta atagatgctt atttgctaga catggccagg aggacctgga aagtcctcat 374340
ttttcccatc tgttcttacc attctgaaat gtatcatgct cttcagtgct ccacagatct 374400
agatgatatc aaattattaa atggaaaaag caagacccat ctccttgggt tattgtggat 374460
aagggggaaa ttgtgtagga agtgtctatt gcagttcatg gaccattgta ggcccttagt 374520
caacaacagc tatcatcatc actgatgaga atctctggct taaacggagt aggttcatgc 374580
ctttttttcag tgtaatgaag tgttttagtt taaaggcctt tagcaaggct tacaataaaa 374640
aaaagtgggg actccagggt aacaccaaca atagagacga ccatggctgt tatcctgaga 374700
ctattaatta tgccacgtaa ttagatttag agaaaactta gagcaacatt tatcttgtgg 374760
ataataattg attgtgattg ggacttaccg ttgagagacc ctgatgaaaa ctctatggtg 374820
aattgctgtg gagtcacatg ggttcatgca tcattcatca gtgggccctg gttttagcct 374880
cataacccct gagaatccac ctctatcaga aaaccaccct ttcagtgaaa gttcctcaag 374940
gctcagaaaa attcatatgt caaagcttta tatgtaaaaa cctatactct ctaataaatg 375000
tttggtttgg ccttcacatt caatttaata attatttcta cctcttttct aaaccgctct 375060
taaccacagt tctatatatt taattctgaa gagcctcatg cacctccctc cctggtaact 375120
ttatctactt ctctccctgg agaaccagcc agaccatgat tccagccctc tgtcctctct 375180
tcaaccgtag gtcctctttt tctctcctga ctcctcaaac cttctccagt tacccggatg 375240
ccttctgtgt gctgctggct tctgcatcac tgttcacagg gccacttgtt cccctggaaa 375300
tgtcttatgc tcttgaaatg ctatagcctt gttcacctgt tcatccaaaa ctgatcctca 375360
gacaacattt ctctttcaat tggtggaata ataaagagga atatgggtca aaattcatag 375420
acatttaaat acccaaggtg caaagttaag agaaaacaag ataggagaaa gaattatggc 375480

aacatctcta aaatgtgtac tttagtgttt agcaaaaaag ggactatgga atgtcaaaca 375540
ggaacatata tgttaaacat aaacatgaag cagtgccagc tgctgtctgc catatagtca 375600
tgctttgtta atgtggcaca tcttgccatg atggtgccct catgaaacga aggatgctga 375660
agcagatgcc attctgtttg ctttgatcag aagccactct ctggtcaagg aagtatttgt 375720
ttcatgctta attataacaa gttctcatca caaagattat tttctttctc cagatctgga 375780
ttgtttgcac tcactttata ttattagaca attactcaga ttgtttatgt ttttatgttt 375840
agaaataaga cagtgattca gagagtccct gtatttagat gaaagaacat tttttaaaca 375900
tattgactgt aaacactgaa aatcattaat tctcaacagt tgaaggtttc tgacaggaac 375960
ggcccttgga aattgtaaat gggcacattg tcaggtatgc ccattgttgt cacattggag 376020
aagattggat tctgttctct ctggagtcct ttgtaatgcc aagcttcctc tccagggagc 376080
tggcttattg tcattatttt gagataaatg gcacttcata gtattaagtg gttcattgtc 376140
tcttctctgg ctgactgtat tctgtaggca gccactcaac tttggagcct ttctctgcta 376200
ccaacttttg gtgttgcctt aagcaagcct catggctcct gttagtttcc tgacaacatg 376260
gtatagcatg gactttgtca tcagagagga ctgaattcag gagaagcaac tctgccactt 376320
ccttggcgga agggggatga ctgatgtcct ttgataagct gcttccctga gcctaataaa 376380
aaaatagaaa taatgatact taaactcatg gaattgttgt gacaattgaa agaatgacca 376440
gttagtaaag catcttgcac catgactagc acacagtaga tgcacatgag tgttactaag 376500
ctttagttat gccctccaaa tacaaagcct tagttttgtc actatgaaag aaattactct 376560
gttcttctta gcaggtaata aataaatccc tgcctggaaa ttctgcagga gaatttcacc 376620
cctgcgcttt caggttaaaa gattgcattc ccatgatgaa gatgttctgt gaatagaatc 376680
atctcaagtc ccaaatggtg tggatttgct cctgcatctt gttgcagaga ttctcttgga 376740
gcttctgaga actggggaaa ccaacaaaat gcctgtagcc tggggaaaat gtagggagtt 376800
ttagcatgaa tttgtgggag gtgagaaaag gcagcaggtg tcgaaagacg ggaaatcctt 376860
gcactgttct agatgttagg agccattttc cattcacatt cagcacctca ggaggagaag 376920
ctgcctatca ctttgtgtcc tcaagagaac caacaaagga gaatgccatc tcccagatac 376980
tcaatttgat taccatttta tcctaccttc tctagggtcc agacattatg taaattggca 377040
ttaagtttga ataatgtatg gacccagttt aaaaggaaaa aatgctttgg gaggctgagg 377100
caggtgcatt gcctgaggtc aggagttcca gaacagcctg gccaacacag tgaaacccca 377160
tctctactaa aaatacaaaa agttagctgg gcacggtggc gggcacctgt aatcccagct 377220
actcaggagg ctaatgcagg agaatcactt gaacctggga ggcagaggtt gcagtgagcc 377280
aagatcgtgc cattgcactc cagcctgggt gatgagagca aaactccatc tcaaaaaatt 377340

tttaaaacaa aaggaaaaaa tgctcccctg attcccgcag tgctgactta aaatgttctt 377400
agtatgccaa tgttgcttaa atatgaatga ctgtagcttc tacttaaatt ggcaaccgca 377460
ccaaatataa actgcaaatg tttatagctc ttatggaagt ataaaagcaa aacaaattct 377520
taaattaaat acaaactaag agggaaactg ataataacaa tgttttgtgt aattaagttg 377580
ctgtttgcaa catgcctggg gcagactcct ggcctctttg catctgacat gagaggctac 377640
caaggatgtg gccacaactg ggctctccca gcacttgctt gcgctcagaa ctgtgccaaa 377700
acctttgctc gcccagtgtt ctgcaacgtc catcatttgg atttggcagg aacacatcat 377760
ttatgcattt tgtttgcttc tgtatttagt tgagatactt aaaatataac tgctagattc 377820
taaggcattc ataagcatct gtgaaaaatg tgttacttag gaaacagttc agggttccat 377880
ggattatcta gattggtgag tcttaagact gaccacacaa ttcatctggg ggtgtggagg 377940
aggggagctt tgagaaaaat ctctgtatga gcacccacac ccagagattc ctatgcaatt 378000
atcccagagt ggtgccttaa tatcaacatt tgtttaaaac catcccccaa gactaaaatt 378060
tgtgaccggg atttagaacc attgacttaa ttgatgcaga acactctgat attaatgtct 378120
tcatgtcttt tggaatgtca ttaagacaaa agcataaaat tttaaaattc ttaaagacaa 378180
tttaaagacc tatgagaatc catctttaat tctcaggagc gtgtggaacc tagtttgaaa 378240
actactggca tcgacaattg aatttccact aaaataaaat agctctctag tatattacaa 378300
aactacccat tctgcaaact gcaggggagc tactgataat gcttggaact gtgccaggca 378360
ctgcctgcat aaaaatgagt aaggtccact tcctccatgg actgggttgg gtaggaggca 378420
aagataatta accaattatt ttaatattat gagttcaggg ttgtaatgaa agtaagtatt 378480
gagtgctgca gagacccagc aaaggagtcc ttagctgaga actgatcctt tgctgagtga 378540
cgaggacatt attgaaccgc caggcttgat ggcatgttgc atggtaacag accagtacac 378600
caaacagcag gagctgtagc agagaaagag tttaataatt gtgtggcagc caaataagga 378660
gacagaagga aaccttaaat ccacctcttg aaaaagtctg ggactaggct ttttaagaga 378720
attctggcaa gaaggaggct gaggagctgg gggtaactga ttggttagga cataggagag 378780
gagacaataa ggatatagaa actccattct tgcactgggt cagttcctcg gagggggtcc 378840
tcactctggc tggcagcagt gaacccattg gaatgcagga tctgaaaaat atctcaaaat 378900
ggcaaacttg aggggttttt tttagcatca aagatgttat ctatagaagt taggacattg 378960
tgacaggggc tacgtgactt tgaagtgtta agtggctgtc agaaagtgag ctattgcagc 379020
ggtccccaac ctttttggta ccagggacca gcttcatgaa agaaaatttt tccacagatg 379080
gggtggtgta gggtggggtg ggggatagct tcgggatgaa actgatccac ctcagatcat 379140
caggcattag ttagattctc atgaggggca cacagcccag atccctcaca tgggcagttc 379200
acagtagggt tcacagccct atgaggttct aatgccactg ctgatctgac aggtggcaga 379260

gctcaggcag taatgcttcc ttgcccacca ctcacctctt gctgtgtggc tcagttcctg 379320

acagattacg gactggtacc agtttgcaac ccaggcagct ggggacctct gggctgtagg 379380

gtaggctggt taatgcttag ctgtgtttct attcaaagct tatgcttttg tttaaaacct 379440

agtaatttgg ttttgttaat tttatgaaga tgatttcaag gatgagtagg attagctgga 379500

gtcagaagga cacaagggaa tttctggcaa caacattaaa ctaccagtga atgtctcatc 379560

aagtcactgg acctctaatt ctaagcagta ctgtcaacct tataggttat gatgtataag 379620

atagtgttgt gatgtataag atagtgcaaa aacctaagtt aatatcacca tttctgtagt 379680

actctctatt tcaaaggaaa aaatcttcag cagtctaata tcctaccaaa attttgaagt 379740

caagacctcc ctacactaat caaagaacta gaggtgcaat caggccatat atttggaaag 379800

gccataggtg ccctccctct gtccttgttc ccagcatact aaactatagt ctaaaagact 379860

ttaaaacttg aactgaagaa aaccttaagg aaacaacaac aaaattccat ttatttcctg 379920

agcccaaaag tagaataatt gtttttttaat gaatttttct ttccctgtaa gacatactat 379980

gaattgttca atgggtattc aaagcatatg taatagaata tgtatgcttt cctgaaaaat 380040

tgaaatggaa catcaaactg ttctgtaaag ttttgagtct agaccaaact cacagcactt 380100

agccagaata tgtacatctg catctgccac tcatggcccc atcgctccct ccaactgctt 380160

ctcttgggct gaaggatgga aaagcaaact ccctgcgttg agttccatct tgccctgcat 380220

ggcctccccc atggggtcat tgagctcccc accagcttcc acctcttcca gcactttccg 380280

ccagagggcc cgtggctgct tttgcagact gaaattgatt gtggttgatt cagctccatt 380340

tcctctgagt ttttacttta gatgtgcttt ttaggttgca tttttcttct ggcatataag 380400

aaagacagca atattccatg gaacagagtg atgtctgtgg ctttgtcaac cgttgtttgt 380460

tagcttgatc accatttatt gagtgtttac tactttccag gcattgtgct aagtggtaga 380520

aatacaaagt tgcctttact tgattcatct gtaaaataga gagagtagta gttcctacag 380580

tgtggtttat gtgaggcata gggactgctc cctgaaacct agggacaact ccctagcagc 380640

gacccagtgc tcatatcaat agtggtaaca attaggaagg cagctcctac ctcaaagacc 380700

tcatgatctg atggtaaaga cagatataac aagaaaatta ttctttttaa atataaaatt 380760

gtgatataaa aatagttttc actaaattgg atttttatga aatttacaga aacaaaataa 380820

taaattaagg aaatataaaa ccacctgaca ttagccaacc atgttgatgt tctgattatg 380880

ttactgcaaa ggagtgtagg gttaagaacg agggactttg attccaagat caaatccctt 380940

tctgctattt cttagctgtc tgactttggg caagttgctt aaaccctttg tgcttctgtg 381000

tgcttgccca tgagatggag attataatag cgtctacctc atagggctat ttttaataca 381060

agttagctct tagaacaata cctacttaaa ctaacaattt gtattaggta ctgttttatt 381120

ctccatccgt actgtgtatg catatattca tatttatatt tacatattct aattaggatt 381180
atatacatgt actttcttta aaaataagat tttaaataac tatataatca gtcatgatga 381240
tacaacatga tttattcaac aaatctgatg ctgttaaaca cgtaagtgat ttctctttca 381300
ggattataaa taacatttgt tttgcatttt tttcatacat cttttgctca cttttcattg 381360
ttactttgta tatgttgcta gaaattgatt tgctggtgaa aagggtataa acatttttaa 381420
ggctcttgct acatattgcc tgctcttcat acatattgcc tactgcccca ggaaactatc 381480
aattacactt ttaccaagag tgtatgaaag cattcacatg aatgtaacct tatcatgcaa 381540
tgcgggccat gagacagagg agagttatgg agggtagacg gcttccccgc tgctcagcca 381600
ctgccctctc ctctaaaaga gccacagtca ggaatttcta catagatcct attataaaca 381660
tctccttttg aaacaaatta tcttctctca gagcattttc ttcattttag tatctatata 381720
ggtccttgtt atgttcattt taagttttgt gacctgcact gacacaatgc tgatgaactg 381780
cttattccag gcagtctgag ttctcaaagc atttgcattt tatgtcttat cctctgtttt 381840
gggctctttg ccactgattt ttttttaagt gatattttaa gaaagaggag ggttgatttg 381900
ttgtgagtgt atctctcttt aaaaactttt ctagggaagg acaacctctt ccaaagactt 381960
tgtttgcttt gtgtgattgg catgtccttc cctggtcagt aatgagcttg aaggaagcta 382020
ctggacacgg gactgaacat ggggttttgc tatcactcta gctgtgcctt tctctagtga 382080
tcaaggagat gttggttcag aaagtgccct gtctaatccc actaaggccc gaaggaccaa 382140
gtgctgtcta ctctccagat tctctgcaac agaaagcaga tgcttaggct tcatggtcca 382200
ggttggaaca ggtagttagc tctgaggaca gttatgcatg aatgtcagga ggggactggc 382260
aggggaacat ttatttattc tttaaaattg gaatcaacag ttatggatga aacccagaat 382320
gcaaaattca ctttcatctt gacacgcaac acatctgtcc tttctccttg aatagcaagt 382380
attaatcatt gagttagaca atgtaacctt cacattcagt gaaagccaaa cactcagcac 382440
cttctagaaa aatcttagtg ccatgctttc ttagcatatt gcaagtcctc tgaggatgat 382500
gtgttgttca gctttgaagc tgatcttttg tacttgctta tgtagctcag gctagtgata 382560
acatgccaga ggcatttcca actaagaaaa ttattcacag aagctttaat tacaaatttg 382620
ttagccccac ttcctgctaa aatggccagc cttgaagagt ttcggaaatg tgagcagcct 382680
taaggatgat agaaatatta tgaaatagta ataagtaata cccatacagc aaactccttt 382740
ttctttggag tatccgttca tcagccagat ttccctctca acagtccagg tgttgacgaa 382800
gatcatgaaa taataggaat gaaagggact tcgattttgt atttttaata gcattttcct 382860
ccaccaaatt acgaaagtaa tgcatgacca aggcagagaa cttggaaaat acagaaaagc 382920
caccaagcaa aataatctcc caagaaatca taatttcacc acacaaaagt aaccattgtc 382980
aacattttgg tgaaaatcct taattaaaga gactcttaaa agatgccagt tcgctgccac 383040

ttcttccttt ctccacacca tcctgcaccc tgttttgaaa tgcagaattg tttctaaatc 383100
attccaggaa aattattagt tttctcaaat atctctggag gcgagtctgt tggcagtcat 383160
ttctagtttc tcacaaacct tgaagttagg aaattatttg tgtagctttt gagcactggg 383220
ccagaagtga gggatgtttg ggttctagtt ccagctctac cacttattta ccaactggcc 383280
gacctcaggc aagtccaggc tagcggcccc gcctcaagcc cctccagatg cagcatctgc 383340
aaccccactt ccttcacttc ctttcgcctc agtttcccaa caatttccta gtaattgtac 383400
tgaatcattg tttttcattt tttacattaa aatttattta ttttatttta tttttaaaga 383460
ctttattttt tagaacagat ttagatttgc agcaaaactg agtggcaagt acagaaagtt 383520
cccatatatt ccctgccccc gcacatacac agtctccctc actatcagca tccccaacca 383580
gagaggtaca ttggttacag ctgatgaatc tacatcgaca cgtcattatc acccaaagtg 383640
catagtttgc attggcgttg ggcattttag ggcttttgac acatgtatgc actattgtaa 383700
tatacagaat aatttcactg ccctaaaaat cctctgtgct cctcctattc attcctccct 383760
ccaccccatt cctggcaact actgatcttt ttactgtctc cacagttttg ccttctccag 383820
aatgtcatat acttggaatc ataaggtttg tagccttttc agattggctt ctttcactca 383880
gtgatatgca tttaaggttc ttccatgtct ttttatggct tgatagctca tttcttttta 383940
tcattgaata ctactactcc attgtctgga ttatacacac tttatttata cattcaccta 384000
ctgaaggatg tgttggttgc atccaagttt tggcaattat gaatgaagtt gctgtaaaca 384060
cccatgtgca ggtaattgtg ttgatatgca tttttaatgc ttttggataa acactcagga 384120
gcacaattgc tggattgtat agtatgttta gttttgaagg aaactgccaa accatcttcc 384180
caagtggctg taccattttc catttccacc aacaatgagt gagagttcct gttgctccac 384240
atcctcatca gcatttggtg ttgtcaatgt tctggatttt ggccattcta acaggtgtat 384300
tgtggtatct cattttgttt taattcgcat tttttggata atatcacatg gagcatcttt 384360
taataagctt atttgccatc tgtgtgtctt ctgtggtgag gtgtctgtta aactctttgg 384420
tccatttttt taatcaggtt gtttgcgttc ttatcgttga gttttaaaag ctctttgtat 384480
attttggata acagtcattt agcaggtatg tcttttgcaa atattttctc ccagtctgtg 384540
gcttgtcttc tcattctctg gagatcagtg gtttttacac tttcttctgc tgaagccaag 384600
tattgtggaa gaagccatgg ggtcaccaga ggataggtgc acagggtagt gagggaaagt 384660
tggagtgaat gggcttggtg cccccacccc gcaacatttc tcttgcttta atcagagaag 384720
tttgacttca tccagattgc atatgaggac tttacatgag gttcaccttt ttatttgttt 384780
taaaaggggt ttttactctc aaaatgtttt aaagaccact gaactagatg atcatcttaa 384840
ggccctttta agatttatga ttctggaatt ctgtggctct atttttccaa tgtaatataa 384900

ccttcctctc gttaaggaaa aatgagataa acctgttata aacactcatg tgacattttt 384960
tcaatacagt tttcatccac gtagtgctca cttagaatga tagaaacaat acacctaaag 385020
aatatgcacc attcacttgc ccactcatct ctcaaagaga ggaactttca acactagtta 385080
tctttttaaa aataatcaga gagaattctc agccacttca attctagccc aggaaaaaga 385140
gggaaaatac atttgaaggt ttctggctca tgtgatacat ttgcaaacca gaaccaagat 385200
gatatgactc tcatttatta gtgactattg caaagctttg caggctatta agcaggctgt 385260
tcaatttagg gtgatatgga catgtgcata ttggccaatt atatatgctc agatcgtatg 385320
tcattttttt tctgcatttt agtctaagtg tcagataggt gtttcatgat gttttataaa 385380
cacttctgtt cacattaaca ttgttcattg atttggcaag gaatgaaggg gtgtcatttt 385440
catgatttta tttgtatatt atgtattcca cagaggattt gaaatgcctt accaaatttt 385500
atgtaatgta aaggggtaaa ccaatatttg acacatcatt ataaattgag gataatctga 385560
agaaaataat aaatccagga gacagattat cacactgata tggtttggct ctgtgtcccc 385620
acccaaattt catcttgaat tttactcaca taatttccat gtgttgtggg agggacccag 385680
tgggagatca tttgaatcat aggagtggtt tcctccatgc tgttctcatt gtagtgaata 385740
agtctcatga gatctgatgg ttttatcagg ggtttccact tttatatctt cctcattttt 385800
tctcttgcca ccaccatgta agaagtgcct ttcgcctccc accatgattc tgaattctga 385860
accctcccca gccatgtgga actgtaaatc caattaaacc tctttttctt cccagtcttg 385920
ggtatgtctt tatcagcagc acgaaaatgg actaatacag taaattggta caaggagtgg 385980
ggtgttgctg aaaagatagc cgaaaatgtg gaagcaatct tggaactgga tatcagggag 386040
aggttggagc agtttggagg gctcaaaaaa gacaggaaaa tgtgggaaag tttgaaactt 386100
cctagagact tgttgaatgg ccttgacaag aatactgata gtgatatgaa caataaagtc 386160
caggctgagg tggtctcaga tggggatgaa gaaactgttg ggaagtggag caaaggtgac 386220
tcttgttata tcttagcaaa gagactggtg gcattttacc cctgctgtag agatttgtgg 386280
aatttgaact tgagagaaat gatttggggt acctggtaaa agaaatttct aagcagcaaa 386340
acattcaaaa ggtgacttgg gtgttgttaa aaccattctg ttttaaaaga gaaacagcat 386400
aaaagttcag aaaatttgca gcctgatgat gcagtaggaa agaaaaaccc atttttttga 386460
ggagaaattc aagctggctg cagaaatttg cataagtaac aaggagccaa atgttaatcc 386520
ccaagacaat ggggaaaatg tctccagagc atgtcatagg tcttcatggc agcccctccc 386580
atcacagacc cggaagccta ggaggaaaaa aacagttttg tgggccagtc ccagggtccc 386640
catgctgtgt gcagcctagg aacttggtgc cctgcatctc agctgctcca gctattgcta 386700
aaaggggctg aggtaccacg gtttcagagg ttgcaagccc caaaccttgg cagctttcat 386760
gtggtgttga gcctgtgtgt acacagaagt taagaattga ggtttgggaa cctccactta 386820

tatttcagaa gatacgtgga aatgcctgga tacccaggca aacatttgct gcagaggtgg 386880

ggccctcatg gagggcctct gctagggcaa tgaggaaggg aaatgtgggg ttggaacccc 386940

cacacagagt ccccactggg gcactgccta gtggagctgt aagaggagga ccactgtcct 387000

ccagaccgca gaatagtaga tccactgaca gcttgcacca tgtgcctgga aaagccacag 387060

acactcaacg ccagcctgtg aaagcagtca gggttggagg tggtggtggc tataccctat 387120

aaagccacag gggcagagct gcccaagact atgggaacct acctcttgca tcagcatgac 387180

ctggatgtga gacattcagt caaaggagat attttgaagc tttagaattt gactgccctg 387240

gtggatttta gacttgtgtg ggccctgtaa ccccttttgtt ttggccaatt tctcccattt 387300

ggagctgctg tatttaccca atgcctaaac ccgcattgta tctaagaagt aactagcttg 387360

attttgattt tacaggctca taggcaaaag ggacttgcct tgtctcagat gagactttgg 387420

actgtggact tttggttaat tctgaaatga gttaagactt tgggggactg ttgggaaggc 387480

atgattgctt ttgaaatgtg aggacatgag atttggagag gccaggggtg gaatgttatg 387540

gtttggctct gtgtccccac ccaaatctca tcttgaatta tactcccata attccaaagt 387600

gttgtgagag ggacctggtg ggaaacaatt tgaatcatga ggccagtttc ccctatactg 387660

ttcttgtggt agtgagtaag tctcacgaga tctcatggtt ttatcagggt ttccgctttt 387720

gcatcttcct cattttctct tgctgctgcc atgtaagaag tgcctttcac aagcataagg 387780

aagaagtata cttgctggag cagcaggctg tccttcttct gacgatatgg actgcaagtg 387840

cctatgttta ctttgaccaa gacggccttt cttttcacag aaagccaggg ttcacccctt 387900

gaatttcata agacctctct ttgcagtggg ttagatactc ttttatattt ttatactctg 387960

tcaggaaaac attttttttt ccatttaaaa atcagtcttt tggttgcttc caagatggct 388020

gaataggaac agctctggtc tgtagctccc agcgagatcg aagcagaagg caggtgattt 388080

ctgcatttcc aactgaggca cctggttcat ctcactggga ctggttggac agtgggtgca 388140

gcccacggag ggtgagccga actggggcag ggtgtcacct cacccacaaa gtgcaagggg 388200

tcaagggatt tccctttcct agccaaggga agctgtgaca gactgtagct ggagaaacag 388260

tacactcctg acaaaatact gcacttttcc ccacagtctt gacaactgga agaccaggag 388320

ataccctccc ttgcctggct cagtgggtca cacgcccatg gagacttgct cactgctagc 388380

gcagcagtct gagatcaacc tgcaatgctg ctgcttgatg cggggagggg cgtctgccat 388440

tgatgaagct tgagtagctc acagcgtaaa caaagcagca gggaagcttg aactgggcag 388500

agcccacctc agctcagcaa ggcctactgc ctctctagat tccacctctg ggggcagcac 388560

atagcagaac aaaaggcagc agacagcttc cgcagactta aacatccctg tctgacacct 388620

ctgaagaggg cagtggttct ctcagcacag tgttcaagct ccaagaacca agagaccgcc 388680

acctcaagca ggtccctgac ccccatgtag cctgactggg agacacctcc cagtaggggc 388740
cgacagacac ttcaaacagg caggtgccct ctgggacgaa gcttccagag gaaggatcag 388800
gcagcaatat ttgttgttct gcagcctccg ctggtgatac ccaggcaaac agggtctggg 388860
gtggacctcc agcaaactcc aacaggcctg cagctgaggg gcctgacggt tagaaggaaa 388920
actaacaaac agaaaggaat agcatcaaca tcaacaaaaa ggacatccac accaaaaccc 388980
catctgtagg tcatcaacat caaagaccaa aggtagataa aaccacaaag atggggagaa 389040
accagagcag aaaagtggaa aattccaaaa accagagcgc ctcttctgct ccaaaggatt 389100
gcagctcctc accagcaagg gaacaaaaca ggatggagaa tgagtttgat gagttgacag 389160
aagtaggctt cagaaggtcg gtaataacaa acttctccaa gctaaaggaa catgttctaa 389220
cccatcgcaa ggaggctaaa aaccttgaaa aaaggttaga tgaatggcta actagaataa 389280
acagtgtaga gaagaactta aatgacctga tggagctgaa aaccacagca tgagaacttc 389340
gtgatgtatg cacaagcttc gatagctgat ttgatcaagt ggaagaaagg atatcagtga 389400
ttgaagatca aattaatgaa ataaagtgag aagacaagat gagagaaaag aagagtgaaa 389460
agaaacgaac aaagcctcca agaaatatgg gactatgtga aacaaacaaa tatatgtttc 389520
attggtgtac tgggaagtga tggggagaat ggaaccaagt tagaaaacac tcttcaggat 389580
attatccagg acaacttccc caacctagca aggcaggcca acattcaaat tcaggaaata 389640
cagagaacac tacaaagata ctcctcaaga agagcaaccc caagacacat aattttcaga 389700
tttaaaaagt atgaaatgaa ggaaaaaatg ttaagggcag ccagagagaa aagtcgggtt 389760
acccacaaag ggaagcccat cagactaatg ggatctctca gcagaaaccc tacaagccag 389820
aagagagtgg gggccaatat tcaacattct taaaaattct taaaaaaaga agaattttca 389880
acccagaatc tcatatccag ccaaactaag cttcataagt gaaggagaaa taaaatcctt 389940
tacagacaag caaatgctga gagattttgt caccaccagg cctgccttag aagagctcct 390000
gaaggaagca ctaaacatgg aaaggaacaa acagtaccag ccactgcaaa aacattccaa 390060
attgtaaaga ccatcgacgc tatgaagaaa ctgcatcaat taacgggcaa aataaccagc 390120
taacatcaga atgacaggat caaattcaaa cataacaata ttaaccttaa atgtaaatgg 390180
gctaaatgct ccaattaaaa gacacagact ggcaaattgg ataaagagtc aagactgtgc 390240
tgtattcagg agacccatct cacgtgcaaa aatgcacata ggctcaaaat aaagggtcga 390300
aggaaaatct actgagcaaa tggaaagaaa aaaaaaagca ggggttgcaa tcccagtctc 390360
tgataaaaca gaatttaaac caacaaagat caaaagagac aaagacagcc acaacaagaa 390420
gagctaacta ccctaaatat atatgcaccc agtacaggag cacccagatt cataaagcaa 390480
gtccttagag acctacgaag agacatagac tcccacacaa taataatggg agactgggag 390540
acaccccaca gtcaatatta aacagatcaa caagacagaa ggttaacaag gatatccagg 390600

acttgaactc agctctagac caagtggacc caatagacat ctacagaact ctccacccct 390660
aatcaacaga atatacattc ttctcagcac cacattgcac ttattttaaa attgaccaca 390720
taattggaag taaaacactc ctcagcaaat gtaaaagaac agaagtcaca acaaactgtc 390780
tctcagacca cagtgcaatc aaattagaac tcaggattaa gaaactgact caaatccaca 390840
cagctacgtg gaaactgaac aacctgctcc tgaatgacta ctgggtaaat aacaaaatga 390900
aggcagaaat aaagatgttc tttgaaacca atgagaacaa ggacacaatg taccagaatt 390960
tctgggacac atttaaagca gtgtgtagag ggaaatttat agcactaaat gcccacgaga 391020
gaaagcagga aagatctaaa atcgacaccc taacatcaca attgaaagaa ctagagaagc 391080
aagagcaaac aaattcaaaa gcaagcagaa ggcaagaaat aactgagatc agagcagaac 391140
tgaaggagac agagacacaa aaagccgttc aaaaaatcaa tgaatccagg agctggtttt 391200
ttgaaaagat caacaaaatt gatagaccac tagcaagact aataaagaag aaaagagaga 391260
agaatcgaat agatgcaata aaaaatgata aagggatatc accaccgatc ccacagaaat 391320
acaaactacc atcagagaat actataaact cctctacgca aataaactgg aaaatctaga 391380
agaaatggat aaattcctgg acacatacac cctcccaaga ctcaaccagg aagaagttga 391440
atctctgaag acaccaataa caggtactga accaaggttt ggaccaacca aaaatgtcca 391500
ggaccagatg gattcacagt tgaattctac cagaggtaca aagaggagct ggtaccattc 391560
cttcagaaac tattccaatc aatagaaaaa gagggactcc tccctaactc attttatgag 391620
gccagcatca tcctggtacc aaaacctggc agagacacaa caaaaaaaga gaattttagg 391680
ctaatatccc tgatgaacat cgatgtgaaa atcctcaata aaatactggc aaaccaaatc 391740
cagcagcaca tcaaaaagcc tatccaccaa aaccaagtca gcttcattcc tgggatgcaa 391800
gactggttca acatacgcaa ataaataaat gtaatccatc acataaacag aaccaatgac 391860
aaaaaccaca tgattatctc aatagatgca gaaaaggcct ttgacaaaat tcaacagcct 391920
ttcttgctaa aaactctaaa taaattaggt attgatggaa cgtacctcaa aataataaga 391980
gctatttatg acaaacccac agccaatatc atactgaatg ggcaaaacct ggaagcattc 392040
cttttgaaaa ccagcacaag ataagaatgc cctctctcac cactcctatt caacatagtg 392100
ttggaagttc tggctagggc aatcaggcaa gagaaagaaa taaagggtat tcagttagga 392160
aaagaggaag tcaaattgtc tctgtttgca gatgacacga ttgtatattt agaaaacccc 392220
attgtctcag cccaaaatct ccttaagctg ataagcaact tcagcaaagt ctcaggatac 392280
aaaatcaatg tgcaaaaatc acaggcattc ctgtacacca ataatagaca aacagagagc 392340
caaatcatga gtgaactccc attcagaatt actacaaaga gaataaaata cctaggaatc 392400
caacttacaa gggatgtgaa ggacctcttc aaggagaact acaaaccact gctcaacgaa 392460

ataaaagagg acacaaacaa atggaagaac attccatgct cgtggatagg aataatcaat 392520

atcacgaaaa tggtcatact gcccaaggta atttatagat tcagtgctat ccccatcaag 392580

ctaccattga ctttcttcac agacttggaa aaaaactact ttaaagctca catggaacca 392640

aaaaagagcc tgcatagcca agacaatcct aagcaaaaag aacaaagctg gaggcatcac 392700

gctacctgac ttcaaaccat actacaaggc tacagtaact aaaacagcat ggtactggta 392760

ccaaaataga tatatagact aatggaacag aacagaggcc tcagaaatga caccacacat 392820

ctgcaaccat ctgatctttg acaaacctga caaaaacaag aaatggggaa aggattccct 392880

atttaataaa tggtgctagg aaaactggct agccatatgt agaaagctga aactggatcc 392940

cttccttaca ctgtatacaa aaattcgctc aagaaggatt aaagacttaa atgtaagacc 393000

taacacctag aagaaaacct acacaatacc attcaggaca taggcatggg caaagacttt 393060

atgactaaaa caccaaaagc aatggcatca aaagccaaaa tagacaaatt ggatctaatt 393120

aaactaaaga gcttctgcac agcaaaggaa actatcatca gagtgaacag gcaacctaca 393180

gagtgggaga aaatttttgc aatccaccca tctgacaaag ggctaatatc tttgtagaat 393240

ctgcaaagaa cttaaacaaa tttacaagaa aaaaacaaac ccatcaaaaa agttggcaaa 393300

ggatatgaac acctttacac tgttggtggg agtgtaaatt agttcaacca ttgtggaaga 393360

cagtgtggtg attcctcaag gatctagaac tagaaatacc atttgaccca gtgatctcat 393420

tagtgggtat atacccaaag gattataaat catgctacta taaagacaca tgcacacata 393480

tgtttattgc agcactattc acaataccaa agacttggaa ccaacccaaa tgtccatcaa 393540

cgatagactg gataaagaaa atgtggcaca tatacaccat ggaatactat gcagccataa 393600

aaaaggatga gttcctgtcc tttgcaggga cgtggatgaa gctggaaacc atcattctaa 393660

gcaaactatc atgaggacat aaaaccaaac actgcatatt ctcactcata ggtgggagtt 393720

gaacaatgag aacacatgga cacagcacaa ggaacatcac acaccggggc ctgtcagggg 393780

ttgggggct gggggaggga tagcattagg agaaatacct aatgtaaatg atgagttgat 393840

gggtgcagca aaccaacatg gcacatgtat acctatgtaa caaacctgca cgttgtgcac 393900

ctgtacccta gaacttaaag tataataaaa aaaaaagaaa gaaaagaaaa gaaaaaaaga 393960

agtggctttt acctcccacc acgattctaa ggcctctcag ccatgtggaa ctgtaagtcc 394020

agttaaacct ctttttcttc ccagtctcga gtatgtcttt atcagcagcg tgaaaatgaa 394080

ctcatacaca cacacagaag tatatcttta cagttcccag gatggactgc aagtttggct 394140

ctaaatttcc tttgcctggc caaaacaaaa agaacacatt ttattataag gttcacaggc 394200

ttttaattga aaaaaaaaaa aaaacagtta ttcaacatga aaactctttt tcagacaaag 394260

ttctacaaag tctatttctc acaagtagga ctttggcaag gattgtgcaa ctgacaattc 394320

aaagtcattt attccgtggt gagaaaccgg agtacagatc tttaccagtt aggggatgat 394380

ccatgtgttt tgataatcac cttggtggtg atcagactaa aggtctccta tgaaaaccat 394440
taacttcaca ctatagctga taggatgcac tgaagccctt accgctgact gcatggatga 394500
gagctaggcc agagcattgg caggcagagt ttataacatg gggcaggaag cattgaaatt 394560
ctattccacc tcttggtgta tcaggtcagg agtcaaatct cctatcttcc tcttctggat 394620
cgctttcctc ctgaagtgtc tggcatagat gtctaggtat agcttggcat ggtggtgtgt 394680
gcctatagac ccagctactt gagaggctga ggtgggagga ttgcttgagc gcaggagttt 394740
gaggctgcag tgagccatga ccacaccact gtactccacc ctgggtgaca gagcaagacc 394800
ctgtctctct ttctaaaaaa acaaaacaaa acaaaacaaa acaaaaagaa aaaaagatgt 394860
ccaagtgtgt tctggttact gctgctgact gctaaattta acagctagaa aaaaaaagtt 394920
tggactctat ggatcataaa tttggataag acacagaggg aatggcttgt ctcatgttca 394980
caatgtctaa agtcttaaat ggggagagtc gtcaaccagg ggtaatttga gagttatggg 395040
ctggaaccac cttgaggcat ctttattcat aggctagcaa ttgatgttgg ctgttggctg 395100
ggacgtcagc tggagctgtc aactgggaga cctgtctgat atcccagcat aggacactag 395160
acctctcaca aagtggcgag ggctccagaa gcaagtgtcc cagctgacaa aaaaaaaaag 395220
tttcatagcc ttctacgaca tgcacttaga agtggtaatg tgtcacttcc agccctttct 395280
gttgattaca agtgagtcat gagtccactc agtttcaagg gagagggccc agatcccata 395340
tctccatggg aagagtgcca agaacacctt gtagaaaagc ttgtggatag gagatattgg 395400
tgtggtcatc cttggaaaac acaatcttcc acagtgtgca agagaggctt cctgctctgc 395460
attgtggctt aattattgtt gaaataccag gcaagaaaag gtaggatcca gggaaacaaa 395520
tgtatccagg atgttttggt ctgccttttc taggaaaaaa ggaaaccctc aaagcctctg 395580
agactaggtc accccagtgg gactagaagt tccacgtgga accttctgca aactcctctg 395640
caatagatgg actgtgcatt ttccaaatgt gtctttacat catctccttt ctgaccttgg 395700
gaagatagta actgactgtg gagtaggaaa tccagaaccc tgattgtttt aaaatatatt 395760
acagatacaa gtaatgaaaa gaaggctcct aaggaaaata tagatccgta atggctttgt 395820
cccaaaagct attaggctat aatattttat tacaaaggct atatttatta caaaatgtat 395880
ttaaataaat acttaatatt tatttaaata aataatattt attttattat taataattaa 395940
tatttaaata aatataatat ttcttacaaa aattaaaaac tgggacttta tatttaaagg 396000
tgagacatta aattcttttt aaaaagtgca catgtaagat ttttttctca agtcaactga 396060
accagttcat cagtttcact atgaatgtgt tgatgcacca agtatttact ttctagaaca 396120
tctagtagac gtcactaaaa aattatacca agttgaaaaa tgtcactgaa tcagaggcat 396180
gtggttggtt atgtagtcat ctgtgccagc tgggaatata gcaataccaa atgaacagca 396240

ctgccatgtt cccttgctgg gttccagcaa aaacactcat cttcactgtc gtctctcttt 396300
gagacctctt agagaggact ttgatgagtg tatggtctta tacacactag tctaacaata 396360
cacatttgct caaggattgg gttacgagca ggctcttcac ctttcaggtg aacaattaca 396420
catcaggaga aggatggaag tttcctatct atgaacaaat atttcccaaa gcaataatct 396480
ctttattcaa ctcacgtaat gagaagtcag ttcctctgtg ccataaagca atctctttga 396540
agtcttcagt aatgttgagg gttttaatgt tgagggcttt atcaaactaa acattcaagt 396600
gtcttaggct ttccccttag ttctttgagc aaattggttg ctatgaagta tgttagtgtt 396660
ccgaatgaat ggatagcatg atgggggatg tctaggtggt tccagaattt agattgtggt 396720
ttccataaca aagtgtggca gtattggcat tagggaagag aaatgacttc taaataactt 396780
ctcaaagttt actaaagcct taagaatgta ggaaatggcc acaaaaggag gtatacaaaa 396840
cattcctctg tctttattcc tctttataca aacgtattca ctttcccatc agcaagtcat 396900
aaaatacttc tcatcttgca ttacagtagg acacctacta taacaatagg ctacaaaatg 396960
tataatggct caaacaccta gcagtttatt tcttatttac ttaacaatgc tgggtccacg 397020
aacatgttga tatcataacc ctcttttatg tggtcactca gggactcagg ctgttgaggg 397080
ctctgtcatc atgaatgggt ggcttccaag gtcatcctaa gagttatctc cattctaacc 397140
agtccagcca gaaaggcaga acaggcttgg aggagcaaag atatccatgg atagatcatc 397200
ctcttaaagt cattgtagca ttgtgatagg gctcttttca caaaacaagg atacaatcca 397260
tgttttcaag attttacatt ttagtagcca caatacaaag aagctaagat agtcttgtga 397320
tttgtcatca tggctgttaa tggagagtgt tttaaaagca ctgatatcag ggctccaccc 397380
tcagcttcca atcatcaggt ttggatggaa cctgacagct gcatcttgta aaactccacg 397440
ggagattctg agagacagcc aagtttgaga ttcacaaacc ttaagagaaa aaatctcctt 397500
gaattgccat aacaaactac cagtctaata aaacatacat attcctttga ctactcttaa 397560
atgaaacaaa taggtaataa tttacctgta catataatta aaaatgtaat gtattttata 397620
accatcatat aaaggagaaa ttatattaaa ctatattaaa ataaaatgat atatatttca 397680
atgcagatgc ccaagcttga ctaaagtagg aaacataata aagtaataaa atgcttatat 397740
gtacttataa tgaatgtttg gctatgagaa gatgatcaga ggctttttgt ccaagaagta 397800
caggcatatg aaaaagtaga gcaagagatg gacaccagac tgagaaaact aaaaactaac 397860
caacctattt gtgtatgata aaaggagaga aaaaaaattg attagtaaaa atatgctacc 397920
gtgtggtctg aatgtttgtc ccttccaaaa ctcatgttga aatttaatcc cccatgtggc 397980
agtattgaga agtgggacct ttaagaggtg attggttcaa gaagattctg cgctcatgag 398040
tggttgaaac cattcacgga ataacgggtt aatggatcaa tgagttatcc caggagtggg 398100
actggtagct ttataagaga aggaagagag acctgcgcta gcacactcag cctccttgcc 398160

atgtgagcca tctcgggacc ccgcagagtc cccagcagaa agaaggccct cacccaatgt 398220
gtccccttga cctaggattt ctcaacctcc ataactgtag gaaatgaatt ccttttcttt 398280
gtaaattatc cagtttcagg tattctgtta taaacaacag aaaatgggct aaaacacacg 398340
ccaagaaaat tgaaagactg tggaagtgaa agaagaataa aaaatgaagt aattttgcaa 398400
atgagtctgg ctttattata agtgtattgt caaagtgatt ccctttgtta taagatgaaa 398460
aagagccaaa atggatagaa acatttatcc tatcttaata tcccactaca ttcaaggcaa 398520
atattcagtc agtctctaga acttaacaag gccttggaga tgatatcctt tggatgagca 398580
atggagaaga caagatggat tggaaaaaga aacaaacaaa caaaaaaaca tgtaagaggc 398640
tatcaggaaa actctggaga cgatgtcaat gtaaaacaga tacaaacctc aaaataattt 398700
taatatgtac attgcacaaa aatggacttc ttatcatgtt acaaatttat tttaaaacat 398760
ataggaggca acaacaatat ttcattgatg tgtactattt tataattgtt ggctctaatt 398820
ttatttagta gagtcttaca tctcctctct acatatttac atccggttgt attgtttttg 398880
tagtacctgt tcaactgcac tgaaatcatt tcaattaaat gcatggttga aaagctgaca 398940
taatacattc aattgccaaa ttgcataaag tgaataagct atttaataat ttggagaaaa 399000
tatattccat ttaaacttcc ttctctataa ccagttcata cactggacct tttgggcaca 399060
gccatgtgaa agcttttcaa aacttttcac tacttttttt tgagacggag tctcactctg 399120
tcacccaggc tagagtgcag tggtgcgatc tcggctcact gcaacctcca cctcccaggt 399180
ccaagtgatt ctcctgcctc agcctcccga gtagctggga ttacaggcac ctgacaccac 399240
acctgactaa tttttgtaat tttagtagag acaggatttc accatgttgg ccaggatggt 399300
cttgaactcc tcatctcaag tgttccgcct gccttggcct cccaaagtgg tgggttacag 399360
gcatgagtca ttgcacccag ccactataga tttttatttg tagtagattt agttcagtag 399420
ggcatttgag aggcatagga actggaacaa atccttcatt gtttgggact gccttgcaca 399480
ttgtaggaca tccagtacag tcccttccac gaagtggcag cagcagcctc gggctgtcca 399540
aacaccctgt tagggacagt gccactcccg gaaccacaga agaaccacaa tcataggaga 399600
agcatacaac aaacatggtg ttcatcggac ggtcagcttc agtggcactt ttgagcaagc 399660
acactatttg gttgtagaat gagtccctgg cagggcaccc gaaaggctga tgtgtgactc 399720
atccctagta cctctgagaa tgctgaaatc tcctccttct ccaagctgag tggcagcttt 399780
ctggtccaga ctacttaaat tgtgacttga ggaattttct tcactttttc ctttgagtca 399840
ttctgttttt gatcaagatg cgatagaatg aaggattctt tcagggtctg ctgtcacatc 399900
cttcctttgg gaacagatag aaaatctggc ctggcttgaa gagtttgtcc tcaaacatct 399960
ggtaagccat aggcatcagc atctcttact tctccagaga catttagatg tgtcccccat 400020

ctccatggag gcccatggca aggggacccc tagttgtact agagcttcct tctgggcaca 400080
gccacactgc acaggcactg cagtgtacag cacagctcct ccagcttccg gaagactcca 400140
ttatctagaa aagtgctgag aaatggagaa gggggttcct ctggggtgga gatggaaaga 400200
ggaaaaggaa gggagaatgt ccaagcaaaa tgagagaagg tgagggacag cagaattgag 400260
agtgagggag agggagagag caagagactg ggttgagatg tcttgtacat gggggtgtcc 400320
tcgtggcttc actccccttt cgtttgtacc actccgtttc cccagactct tcctaaatat 400380
cccccttattg gctacaagat ccagattgcc tagaagctca gatttgctga cagcacctca 400440
agactggtta atgacatata gatttttttct tgtttccatc tttgggattt ggagcaatct 400500
ccagagttgc aaaagcctac cttgttgcaa taatattttt gagggtccac actgtctgct 400560
cacttgctct atgctgctgg gcacagtggt gtggccatcc agggtgggag agaccacctg 400620
ggatatggaa attaccagca gccctgtttt gctcttgcag tggttgccaa agcacacgaa 400680
gcattctggg aatctgaatt tctgttgttt ctcccaaatg gcccctgaag ggcaacaggc 400740
agcacacatg gaccacattt tgccagaaaa gtccagaaag aagatggctg ctgttgtcaa 400800
ctattggcat ttcaacagga cctattttta tttctcagta tatttgcaca actaggaagc 400860
atctgctgac aaggatgaga tgataagctg tattgatgcc aaaacaggac tagaaaaaca 400920
ggcttgttct gcaaatggcc tgcatcctga gagtccgtgt taagaacatt ctctctctgc 400980
aggcaatatc tgctcttaaa acattaacac ttgaaaggaa gtaaggcttg gaatccttag 401040
catagttatg cacacactag gttctcagta agtgcatgct ggatgaagga acccacacgc 401100
accttaagga ggatggaaca ttggcagccc aagaaaaaag tcactcccca gaggccgagt 401160
gccatgtctt tgacctccat tggcaaggtc atggtgaaat gctgtctctt ggggtcgctg 401220
aggtcccagc acggtgtgcc ttcacaccgt gcaaacaata agaagagaag agaggcacac 401280
atgtcacctt tccaggcagg gacatccaag ctgtttgagt ctccattctg tgatttcctt 401340
gaacaagaaa gggattattg gtgaatgagg ccagcatcct gggtgggaac aaaaaaggaa 401400
attggtagaa agtacatcat ccaagttggt ctaactcagc cctgtggctt gggcgggttc 401460
ctaggatatc cagccggagg cagaggctct aggggatgtg aggctgcact gggtatgatg 401520
acacagagga ctgtgagagg cagagaaaag gggagaagag ctgaggccct cctagcccta 401580
acctcccagc tcacaccctc tgctatctgc cacctccagg ccaacaccac ggccatggga 401640
ggtttgccca tttttctgat agacacattc tttctctacc aacagcccac atggaacata 401700
agtgaagtaa ttcagcagtt tccttcttaa tctgctgtct ctctgctgct tactcattct 401760
gtgctgtttg ctttagatca aggtgcccca gggtaccaaa agctacatta tcctattcaa 401820
ctgcatggaa accaagatcc cgaccattct ggtttgttgt tattgttacg ttagttttaa 401880
accttagcca ttttccattc tgggcagtta caactctgtt tactatgagt tcctctgtgt 401940

agatgtttct cccatacccg acctacaaac tgctacgtga gcagtaactt gtcaccatgt 402000
tatccgcata aagtcaggga ttgataaact tgggagcctt tagctcttga aggtgctgat 402060
gaggaagggg agaggggagg agaaggaatt tgtttgtgag atggtcagtg ggctgaatag 402120
tggtatgacc aaaaagtgtt actataaata cacataatct ggaaaacaaa aatactgtca 402180
tctcctccct gcaaggcgct gtgattaggc aggatggagc ttattgctgc agtgttagaa 402240
aatgcatcct tgtagtccca aaaaagcctg ttccatcctg cccatctctt gttctcaata 402300
cttcttactt cacggtcact cattttcttc ctgtccttgg gttgaggtct caacttcttg 402360
taatagaatt ttttcagtta taagcgacag aaatccaact cacactatct taagcaaaaa 402420
ggggattcac tgatacgtgt gttaggaagg atatatgaaa gctcaaaggt caagtgaaaa 402480
aaatgtaaga acgaagacct tggtggggtg tggtggctca cgcctatcat cccagcattt 402540
tgggaggccg aggcaggtgg gtcacaaggt caggagttca agaccagcct gcccaagatg 402600
gcgaaacccc atctctacta aaaacacaaa aattagccgg gtgtggtggc aggctcgtgt 402660
attcccagct acttgggagg ctgaggcaga gaactgttta aacctgggag gcagaggttg 402720
cagtgagttg agatgtgcca ctgcactcca gcctgggcgg cagagtgaga ctctgtctca 402780
aaaaaaaaac aaaaaaaaaa aactaagacc ttgtgactac tacctactac ctggatctct 402840
ctctctctct ctctctcttt ctctctctct ctctcacccc cccacactat cacctctcct 402900
tgttgtcagt agtttccttt ctgcagacag gagttccatg tgctgggaag agggccattc 402960
agagcctcgt attctcatcc tctcagttta gcaatgcgag aagaaaaaac ttttgtctca 403020
caacctcata tacaatctca gaaataggct cttattttct ggcttgaggc tgtccagagg 403080
caccaggcac tgtgatggtc tcactttctg tcctggatct atctgtgggg cccagacagc 403140
aggatctatt tcagaaaata ggcgagagag gaaaaactta tgtggccgta aaaaacagcg 403200
gttttcaagt ggcctatccc catccacatc ataaactttt ctgctgaggc ttaaaagtgc 403260
ttagacttca aacttgatct aaaacagaaa tcctacctgg aaaaatcacc ttccagtcac 403320
ttagccaatc gctcatcact ctctgtctct gaatctcacc acgagtttca cacgtctccc 403380
gagagagcgt ctcctttatc attaccacat gtggagtcag cagattgggt ttcatctaag 403440
ctctgcctac ctactatccg agttccctag caggttagtg actttctctg cctttttgtt 403500
tcttgatgtg aaaaggaaca acagtggctg ctcacagggt tgtcagttac atggtcaatt 403560
acattacatg gacaattcat gatggtgtca gacctgtctg agaaaaggtg gcccatgtta 403620
ccatgacagg ccccaaggct aacagcccct ttctgggtca tggctcatac aaaggagccc 403680
tccttggttt gctggtgctc tgagacttgc aaatgcatta ggaattttac actgtaaccc 403740
ggttttaaat gggtccagag catccccatt gctagactac tgtgctgagg aagggcactg 403800

gctcattgtt acatcccatg aacactctgg gtctcctaga cctcatcctc tttgagcttc 403860
tctctctcac tctctctctg cgcattcagg agtgtacaca tttctgtcca gcaccctgaa 403920
gtctctggaa gagaaggacc atatccaccg agtcctggac aagatcacag acactttgat 403980
ccacctgatg gccaaggcag gcctgaccct gcagcagcag caccagcggc tggcccagct 404040
cctcctcatc ctctcccaca tcaggcacat gaggtgaggc atctgtgggc ttcctacagg 404100
agagacataa agaaaacatg cccccaaacc tatgtgacag ctggccggga aggactggtg 404160
cctgcatatg gagagtgcac ttgtgacagt tcctggcata gaataagcat aaatgctata 404220
ggaggacaga agagagaggt tttaaatctg cgagggtcac agggcaagtg tcagagaagg 404280
catagaggaa gcgatactta cgcttggttt aaagcatgtg ctttggggca gtggtttaga 404340
gattgggtgc agtgtgcaaa taggaggagg gggccaatca agagaagtca gtgaatgtac 404400
acatcccctg aagacactgg gtacatctgt tatggtccct gtgtacactc ggatattcta 404460
aatgagagac cctggggcca gatgcctcac ggagagtcaa tgttgactcc ttcatacctg 404520
ttgaacccct tcttgtcacc cccattgcca ttaccctagt ctaagccacc atcattttct 404580
gttggacttc tgtgccagtc actggatgtg tatccctgcc tgtaatctag atcctttcta 404640
gatcattctc catgatgtgt ccagagtgat cattctagaa ccaaatctga tgtcatcctc 404700
ctgcttaaaa cccttaagtt ctcattgctt ttagataaaa tataaaccct ttcactttgt 404760
ttttattact ccttatgatt cagcccttac ttagcttaca aggacttcct catgtctgag 404820
ctctagccat aatagctgac tgttggatga gtcatcgggg ggaggttctt cttcaggctg 404880
cagattggcg gtcagccctc ctgacctcag ctgggctcac tcacacatct gggaggcagc 404940
tgcctgaggc cagggtgact ctgctctatg cctcatgtct cctatcctcc ttgtacccac 405000
atgttagctg ggacatgttc tcatcgcaat ggcagaggca tgagaaagtg atcctgtttg 405060
ggcaagtgca ttttactctt ttctttcact catgtccact gacatttcat tggccaagca 405120
agcatgtggg gtgtccagag tcaaagaggg acatgttgcc ggggtcatga ctatttctga 405180
gcaataatct aacctatcat agctcagatc tcactccttg caggaagcat ctcctaatcc 405240
ttaagaccta gttggaactt acactctgtt cttccctatg gttactgccc attttactat 405300
ttttctgccc atttagactg acaactctgt gagccatcat tgcaatatgg cttgcttacc 405360
caatatcacc tagcatgatg tagagcacat agtaaatact caacaaatgt tttctgtaag 405420
agctaaggaa ggacatcagc agacaaaata gagtctttct atgccaaaag gtgaaagtgg 405480
tctcatttcc attccattgt cctgtaattt ggctagccaa aagcttagtg gataccattc 405540
ccataagtag acctcatctg cagggaagag atggggagac tgctgggtta tttattgcag 405600
actccagcag gaccacaaca atgattcaaa gtgatgacac tagaaggaca ggtgccaatg 405660
ggtgatgaag ccacaatgac gatgtcaagt ggtatcacta aaaggacagg tactcatggg 405720

tgatgaagcc tacatgacct tgggcttaca ccaggaccct aggaatgaaa gagattcctt 405780
ctcttagtgg ctatgaggga aggcctatga tgtcttaagg aggttggatt cgatcatttt 405840
taagggacct tcaaatcctt agaccgtgat tcaaatggta agaagtttgc tccattaaac 405900
tcccaggcac caccctttcc ggagacaacc tttaaaaatt attagctcct gggaaaatag 405960
atgtatctca ttttctttgt atccctcaca gtgccatatg tgaagactta catatggtat 406020
atgcccccaa cattactact gagtgagtga acaaagaaat gagggaatga atggatgaac 406080
aaatggctca tggcaggctt ttccctgccc tgtgaattcg gcttctactt ggtaagaagg 406140
tgattgcaat ctctgttctc agggttcccc aaggattcct gtgtaacaga tgaaggaatc 406200
acgtctgtca ggtagagtag gtgactgcat tcagagtata catttccagt ctcccctgtc 406260
tttttctttt gatgagtatt atagagaggg aagccatgag tggattagat gccaaaatcc 406320
ctggctgaga gaataacctt accctggagg aaaacatatt agctttgact ctgagctggg 406380
aatttcggtg atgttgtaga ttcaatgcat tgcagttggg tgtttttatt tgttgaaagg 406440
aattgctgaa ttttcaaatc cattaactgc ttgtcagcat tagcaagcct aattagttaa 406500
tactaagtaa atttgcacta aatatacaac cctggctgat tttactggcc acgtctggca 406560
gagggcagca gcagggagaa agctctgtag agtttctgtt ggaatcgtgt gaaaagctgg 406620
agaggttgct tctctttctc tctctctctc tttctctctc tctctctgac acacacacac 406680
acatacacac acacacaatt gtaataataa taatattttg gttcctcact aagccaatct 406740
aaatcgcaga agtctatttt gttaagtaag cttggcccca gccatatgtt gctactcaga 406800
gaatttaata tcagatttca tctgactgta aacgtgaatc atcaggttgc acaaggaaca 406860
cagtggcaga tccagggggc atttaacttt tatagcattt taaatgaaaa aaaaaaaaag 406920
taacacttaa acaagtaatt tagatcatgc tgtaggccct gaatagcttt gatgttgtgt 406980
tttcatggca agtctccaac ttgagctgaa ttttccccta ctaaaaatgc aaatttttct 407040
aagaccttct cttggtcctg ctactcatga tacatatttc ttttaaaaga aatttcaaac 407100
tagataatag ttgttctcct ccccaccccc gccaccagta gtgtggtggg gcagcagagt 407160
tgtggctagt ggaggagagc agaggaggag agtagggaaa ggagaatgcc atttgcctac 407220
atttccctct gcccatttcc cgctgcccat ttcccccctt gttttcctga acgtgaactg 407280
agctctgggc actgttttag gcctagcagg ggacaggata aagcctgctt ctctaggaat 407340
tcgcactgag ggtgtgagtg tgtgcacgtg tgtgtttgga ggcgggagaa taaacacaaa 407400
taaataaaaa ggagaatttc aggcagtgat aagagtgctg agaaaaacag aacggtgtga 407460
aagaggaagg ctgagcctgc agaggcttga ggctgctgcc actgggtagc ggtaggcctt 407520
tccgaggagg cggcatttga agaccggagg aaggttcatc ccagcaagta ggaacagcaa 407580

gtgtaggtcc cctaagtctt gggggagctt agttccttta agggcagcac aaaaatcagt 407640
gtggctccgg agagcacatt aggggagaga ggcaggaaga gcttggagac atggatggaa 407700
gctggaccag ttgggccttg ttgaacatgg aaaggcattt agatcgtatt ctgagttaaa 407760
tgggaagtga cgtgagagat ttaacaatgg agcgtcttga actgctttac tcatttaaaa 407820
tacccactcc tgcttggctg aatatctcat gttgtctttt tagaagcttt ggcgatccta 407880
tttgaatgca tttaggtcct attggagggg aataggatct catttgaggc cacggaggtc 407940
catggaagtc acctgcatag caaataccct gaaagtggct gcagggagag tgtgagggtg 408000
ggaccgccct ggtaggaggt ggaaaatgaa aaacacacgg ccatgagttc cagattaggg 408060
cttctgaaag ccctcagctt tcccagctcc catcctaaag tgggtcttta aacaggaaga 408120
aagaaagatt gctaagtgtc tttggagttc ctcttccttc cccttctagg gatttcagca 408180
ctcctggggc tcgggttggc tctaaagtag tcctttctgt gtcttcccac ctacagtaac 408240
aaaggcatgg agcatctgta cagcatgaag tgcaagaacg tggtgcccct ctatgacctg 408300
ctgctggaga tgctggacgc ccaccgccta catgcgccca ctagccgtgg aggggcatcc 408360
gtggaggaga cggaccaaag ccacttggcc actgcgggct ctacttcatc gcattccttg 408420
caaaagtatt acatcacggg ggaggcagag ggtttccctg ccacggtctg agagctccct 408480
ggctcccaca cggttcagat aatccctgct gcattttacc ctcatcatgc accactttag 408540
ccaaattctg tctcctgcat acactccggc atgcatccaa caccaatggc tttctagatg 408600
agtggccatt catttgcttg ctcagttctt agtggcacat cttctgtctt ctgttgggaa 408660
cagccaaagg gattccaagg ctaaatcttt gtaacagctc tctttccccc ttgctatgtt 408720
actaagcgtg aggattcccg tagctcttca cagctgaact cagtctatgg gttggggctc 408780
agataactct gtgcatttaa gctacttgta gagacccagg cctggagagt agacattttg 408840
cctctgataa gcacttttta aatggctcta agaataagcc acagcaaaga atttaaagtg 408900
gctcctttaa ttggtgactt ggagaaagct aggtcaaggg tttattatag caccctcttg 408960
tattcctatg gcaatgcatc cttttatgaa agtggtacac cttaaagctt ttatatgact 409020
gtagcagagt atctggtgat tgtcaattca ttccccctat aggaatacaa ggggcacaca 409080
gggaaggcag atcccctagt tggcaagact attttaactt gatacactgc agattcagat 409140
gtgctgaaag ctctgcctct ggctttccgg tcatgggttc cagttaattc atgcctccca 409200
tggacctatg gagagcagca agttgatctt agttaagtct ccctatatga gggataagtt 409260
cctgattttt gtttttattt ttgtgttaca aaagaaagcc ctccctccct gaacttgcag 409320
taaggtcagc ttcaggacct gttccagtgg gcactgtact tggatcttcc cggcgtgtgt 409380
gtgccttaca caggggtgaa ctgttcactg tggtgatgca tgatgagggt aaatggtagt 409440
tgaaaggagc aggggccctg gtgttgcatt tagccctggg gcatggagct gaacagtact 409500

tgtgcaggat tgttgtggct actagagaac aagagggaaa gtagggcaga aactggatac 409560
agttctgagg cacagccaga cttgctcagg gtggccctgc cacaggctgc agctacctag 409620
gaacattcct tgcagacccc gcattgccct ttgggggtgc cctgggatcc ctggggtagt 409680
ccagctcttc ttcatttccc agcgtggccc tggttggaag aagcagctgt cacagctgct 409740
gtagacagct gtgttcctac aattggccca gcaccctggg gcacgggaga agggtgggga 409800
ccgttgctgt cactactcag gctgactggg gcctggtcag attacgtatg cccttggtgg 409860
tttagagata atccaaaatc agggtttggt ttggggaaga aaatcctccc ccttcctccc 409920
ccgccccgtt ccctaccgcc tccactcctg ccagctcatt tccttcaatt tcctttgacc 409980
tataggctaa aaaagaaagg ctcattccag ccacagggca gccttccctg ggcctttgct 410040
tctctagcac aattatgggt tacttccttt ttcttaacaa aaaagaatgt ttgatttcct 410100
ctgggtgacc ttattgtctg taattgaaac cctattgaga ggtgatgtct gtgttagcca 410160
atgacccagg tgagctgctc gggcttctct tggtatgtct tgtttggaaa agtggatttc 410220
attcatttct gattgtccag ttaagtgatc accaaaggac tgagaatctg ggagggcaaa 410280
aaaaaaaaaa aagtttttat gtgcacttaa atttggggac aattttatgt atctgtgtta 410340
aggatatgtt taagaacata attcttttgt tgctgtttgt ttaagaagca ccttagtttg 410400
tttaagaagc accttatata gtataatata tatttttttg aaattacatt gcttgtttat 410460
cagacaattg aatgtagtaa ttctgttctg gatttaattt gactgggtta acatgcaaaa 410520
accaaggaaa aatatttagt tttttttttt ttttttgtat acttttcaag ctaccttgtc 410580
atgtatacag tcatttatgc ctaaagcctg gtgattattc atttaaatga agatcacatt 410640
tcatatcaac ttttgtatcc acagtagaca aaatagcact aatccagatg cctattgttg 410700
gatactgaat gacagacaat cttatgtagc aaagattatg cctgaaaagg aaaattattc 410760
agggcagcta attttgcttt taccaaaata tcagtagtaa tatttttgga cagtagctaa 410820
tgggtcagtg ggttcttttt aatgtttata cttagatttt cttttaaaaa aattaaaata 410880
aaacaaaaaa aaatttctag gactagacga tgtaatacca gctaaagcca aacaattata 410940
cagtggaagg ttttacatta ttcatccaat gtgtttctat tcatgttaag atactactac 411000
atttgaagtg ggcagagaac atcagatgat tgaaatgttc gcccaggggt ctccagcaac 411060
tttggaaatc tctttgtatt tttacttgaa gtgccactaa tggacagcag atattttctg 411120
gctgatgttg gtattgggtg taggaacatg atttaaaaaa aaactcttgc ctctgctttc 411180
ccccactctg aggcaagtta aaatgtaaaa gatgtgattt atctgggggg ctcaggtatg 411240
gtggggaagt ggattcagga atctggggaa tggcaaatat attaagaaga gtattgaaag 411300
tatttggagg aaaatggtta attctgggtg tgcaccaggg ttcagtagag tccacttctg 411360

ccctggagac cacaaatcaa ctagctccat ttacagccat ttctaaaatg gcagcttcag 411420
ttctagagaa gaaagaacaa catcagcagt aaagtccatg gaatagctag tggtctgtgt 411480
ttcttttcgc cattgcctag cttgccgtaa tgattctata atgccatcat gcagcaatta 411540
tgagaggcta ggtcatccaa agagaagacc ctatcaatgt aggttgcaaa atctaacccc 411600
taaggaagtg cagtctttga tttgatttcc ctagtaacct tgcagatatg tttaaccaag 411660
ccatagccca tgccttttga gggctgaaca aataagggac ttactgataa tttacttttg 411720
atcacattaa ggtgttctca ccttgaaatc ttatacactg aaatggccat tgatttaggc 411780
cactggctta gagtactcct tcccctgcat gacactgatt acaaatactt tcctattcat 411840
actttccaat tatgagatgg actgtgggta ctgggagtga tcactaacac catagtaatg 411900
tctaatattc acaggcagat ctgcttgggg aagctagtta tgtgaaaggc aaatagagtc 411960
atacagtagc tcaaaaggca accataattc tctttggtgc aggtcttggg agcgtgatct 412020
agattacact gcaccattcc caagttaatc ccctgaaaac ttactctcaa ctggagcaaa 412080
tgaactttgg tcccaaatat ccatcttttc agtagcgtta attatgctct gtttccaact 412140
gcatttcctt tccaattgaa ttaaagtgtg gcctcgtttt tagtcattta aaattgtttt 412200
ctaagtaatt gctgcctcta ttatggcact tcaattttgc actgtctttt gagattcaag 412260
aaaaatttct attctttttt ttgcatccaa ttgtgcctga acttttaaaa tatgtaaatg 412320
ctgccatgtt ccaaacccat cgtcagtgtg tgtgtttaga gctgtgcacc ctagaaacaa 412380
catattgtcc catgagcagg tgcctgagac acagacccct ttgcattcac agagaggtca 412440
ttggttatag agacttgaat taataagtga cattatgcca gtttctgttc tctcacaggt 412500
gataaacaat gctttttgtg cactacatac tcttcagtgt agagctcttg ttttatggga 412560
aaaggctcaa atgccaaatt gtgtttgatg gattaatatg ccctttgcc gatgcatact 412620
attactgatg tgactcggtt ttgtcgcagc tttgctttgt ttaatgaaac acacttgtaa 412680
acctcttttg cactttgaaa aagaatccag cgggatgctc gagcacctgt aaacaatttt 412740
ctcaacctat ttgatgttca aataaagaat taaactaaa 412779

<210> 42
<211> 595
<212> PRT
<213> Homo sapiens

<400> 42
Met Thr Met Thr Leu His Thr Lys Ala Ser Gly Met Ala Leu Leu His
1               5                   10                  15
Gln Ile Gln Gly Asn Glu Leu Glu Pro Leu Asn Arg Pro Gln Leu Lys
            20                  25                  30
Ile Pro Leu Glu Arg Pro Leu Gly Glu Val Tyr Leu Asp Ser Ser Lys
        35                  40                  45
Pro Ala Val Tyr Asn Tyr Pro Glu Gly Ala Ala Tyr Glu Phe Asn Ala

```
        50                  55                  60
Ala Ala Ala Ala Asn Ala Gln Val Tyr Gly Gln Thr Gly Leu Pro Tyr
65                  70                  75                  80
Gly Pro Gly Ser Glu Ala Ala Ala Phe Gly Ser Asn Gly Leu Gly Gly
                85                  90                  95
Phe Pro Pro Leu Asn Ser Val Ser Pro Ser Pro Leu Met Leu Leu His
            100                 105                 110
Pro Pro Pro Gln Leu Ser Pro Phe Leu Gln Pro His Gly Gln Gln Val
        115                 120                 125
Pro Tyr Tyr Leu Glu Asn Glu Pro Ser Gly Tyr Thr Val Arg Glu Ala
    130                 135                 140
Gly Pro Pro Ala Phe Tyr Arg Pro Asn Ser Asp Asn Arg Arg Gln Gly
145                 150                 155                 160
Gly Arg Glu Arg Leu Ala Ser Thr Asn Asp Lys Gly Ser Met Ala Met
                165                 170                 175
Glu Ser Ala Lys Glu Thr Arg Tyr Cys Ala Val Cys Asn Asp Tyr Ala
            180                 185                 190
Ser Gly Tyr His Tyr Gly Val Trp Ser Cys Glu Gly Cys Lys Ala Phe
        195                 200                 205
Phe Lys Arg Ser Ile Gln Gly His Asn Asp Tyr Met Cys Pro Ala Thr
    210                 215                 220
Asn Gln Cys Thr Ile Asp Lys Asn Arg Arg Lys Ser Cys Gln Ala Cys
225                 230                 235                 240
Arg Leu Arg Lys Cys Tyr Glu Val Gly Met Met Lys Gly Gly Ile Arg
                245                 250                 255
Lys Asp Arg Arg Gly Gly Arg Met Leu Lys His Lys Arg Gln Arg Asp
            260                 265                 270
Asp Gly Glu Gly Arg Gly Glu Val Gly Ser Ala Gly Asp Met Arg Ala
        275                 280                 285
Ala Asn Leu Trp Pro Ser Pro Leu Met Ile Lys Arg Ser Lys Lys Asn
    290                 295                 300
Ser Leu Ala Leu Ser Leu Thr Ala Asp Gln Met Val Ser Ala Leu Leu
305                 310                 315                 320
Asp Ala Glu Pro Pro Ile Leu Tyr Ser Glu Tyr Asp Pro Thr Arg Pro
                325                 330                 335
Phe Ser Glu Ala Ser Met Met Gly Leu Leu Thr Asn Leu Ala Asp Arg
            340                 345                 350
Glu Leu Val His Met Ile Asn Trp Ala Lys Arg Val Pro Gly Phe Val
        355                 360                 365
Asp Leu Thr Leu His Asp Gln Val His Leu Leu Glu Cys Ala Trp Leu
    370                 375                 380
Glu Ile Leu Met Ile Gly Leu Val Trp Arg Ser Met Glu His Pro Gly
385                 390                 395                 400
Lys Leu Leu Phe Ala Pro Asn Leu Leu Leu Asp Arg Asn Gln Gly Lys
                405                 410                 415
Cys Val Glu Gly Met Val Glu Ile Phe Asp Met Leu Leu Ala Thr Ser
            420                 425                 430
Ser Arg Phe Arg Met Met Asn Leu Gln Gly Glu Glu Phe Val Cys Leu
        435                 440                 445
Lys Ser Ile Ile Leu Leu Asn Ser Gly Val Tyr Thr Phe Leu Ser Ser
    450                 455                 460
Thr Leu Lys Ser Leu Glu Glu Lys Asp His Ile His Arg Val Leu Asp
465                 470                 475                 480
Lys Ile Thr Asp Thr Leu Ile His Leu Met Ala Lys Ala Gly Leu Thr
                485                 490                 495
Leu Gln Gln Gln His Gln Arg Leu Ala Gln Leu Leu Leu Ile Leu Ser
            500                 505                 510
His Ile Arg His Met Ser Asn Lys Gly Met Glu His Leu Tyr Ser Met
        515                 520                 525
Lys Cys Lys Asn Val Val Pro Leu Tyr Asp Leu Leu Leu Glu Met Leu
    530                 535                 540
Asp Ala His Arg Leu His Ala Pro Thr Ser Arg Gly Gly Ala Ser Val
545                 550                 555                 560
```

```
Glu Glu Thr Asp Gln Ser His Leu Ala Thr Ala Gly Ser Thr Ser Ser
                565                 570                 575
His Ser Leu Gln Lys Tyr Tyr Ile Thr Gly Glu Ala Glu Gly Phe Pro
            580                 585                 590
Ala Thr Val
        595

<210> 43
<211> 20065
<212> DNA
<213> Homo sapiens

<220>
<221> source
<222> 1..20065
<223> /mol_type="unassigned DNA"
      /organism="Homo sapiens"

<400> 43
ggcgcaacgc tgagcagctg gcgcgtcccg cgcggcccca gttctgcgca gcttcccgag      60

gctccgcacc agccgcgctt ctgtccgcct gcaggtaggg agcgttgttc ctccgcgggt     120

gcccacggcc cagtatctct ggctagctcg ctgggcactt taggacggag ggtctctaca     180

ccctttcttt gggatggaga gaggagaagg gaaagggaac gcgatggtct agggggcagt     240

agagccaatt acctgttggg gttaataaga acaggcaatg catctggcct tcctccaggc     300

gcgattcagt tttgctctaa aaataattta tacctctaaa aataaataag ataggtagta     360

taggataggt agtcattctt atgcgactgt gtgttcagaa tatagctctg atgctaggct     420

ggaggtctgg acacgggtcc aagtccaccg ccagctgctt gctagtaaca tgacttgtgt     480

aagttatccc agctgcagca tctaagtaag tctcttcctg cgctaagcag gtccaggatc     540

cctgaacgga atttatttgc tctgtccatt ctgagaaccc aaaggagtcc taaaagagga     600

atggaggagc ctaagaataa aaatagtata ataaaacatt tcttagacac attgaccttg     660

gcctatgtca aagttcagtc tgggtttgtc ttataacaca aggagtaaaa gtaccattgt     720

tctacctctt tttttaatac ttgaaaaaaa tttactgtgg atgcttttct atgaattaaa     780

taaccttcta aaaaatgttt tcattgctgc attcgattag attgggtaac taaatgaaat     840

taattcctca ctgttgggta taaaggttat ttacagtggt tctgtcttag ccattcactg     900

aactcattgc atatatatct ctggaatatt gctgattgtt tccttcaagt aaacttagaa     960

gtgtaactac ttagtcaaag agcctgaata ttttaaaggc cttttgaaga aaactgaaaa    1020

tgctttccag aaaggatgta tcagttgaca atgacagtcg tcaacagtat ttaaggagaa    1080

ctatgatact ctgaagaaaa acttagcctt tctcagtaaa agtaggtagg cagaggccac    1140

atgacagcag ttagagtgtg gtcttcaagg aagtcacaga aatactgtgg ggaattgaaa    1200

ccccatgtgg aaaatgtaca agagtgtctc agtgtgactg agaaggaggt tgggcatggg    1260

gtttcatgga gtttaataaa gtttggtcac ttagtagagg tttaataaat caactgtctt    1320
```

aatctttgat cctacttaag aatttttttt ttgtttttgt agagatgggg ctcttgttat 1380

gttgcccagg ctgttctcga actcctagcc tcaggcgatc ctccctcctc aggctccaga 1440

agtcctggga ttactggcgg gagccaccat gcaggcctct tgctcctact tttgagaaag 1500

gaagtttaac cggttttttt tgtctttttt tttttttttt tgagacagag tctcactctg 1560

ttgcccatgc tggagtgcag tggtgcaatc tcagctcact gcctcccggg ttcaagtgat 1620

tctcctgcct cagcctcccg agtagctggg actacaggca cctgccacca cgcccagcta 1680

atttttgtat ttttagtaga aatggggttt caccatattg gccaggctga tctcgaactc 1740

ctgacctcag gtgatccgcc tgcctcggcc tcccaaagtg ctgggattac aggcatgagc 1800

cactgctcct ggctgcttaa ctttttctct atctcatcct cctacccatc ctacccttgg 1860

aagatagaga agtagtatta gttccatagt gttatactgg gcttccccca gggacaaacc 1920

cacttcccca acctgaatga gccatcactt cttccccagt ttacatttca ttgctcttta 1980

aatgtctcca ttcggatatg ggaattcaca tatggtcata attcttacct gaagaagatg 2040

tcagtcttct tctcttagac caactgccct gatatgaggt ttagaggtta aagaacatgt 2100

gtgtatttac atgatctttg tattctgcct tttcgtccct cactaatgac agctgcaccc 2160

caaggaaatg gagctgtgga agagagggtt tgataagaaa ttaagtaaat attggatcta 2220

atccatcacc ctccaggaag cctttattac tcctaaaaat ttcaaccaaa ttcattaaag 2280

gacaagaact ccaccagagt aggccataaa cattggcaaa attagttgta atccatgact 2340

agatttaatg tccctttgtt ttattcccat atggttataa tgctttgctt ggcattaggg 2400

gtattttaag ttttcttctg cctagtaagt gaatttgtgt ttataataca ataatcataa 2460

aatatcacat taatatttta taactgtaca gttataaaat attttataag taatatttat 2520

attttataag taatatttta taactgtaca gttaactctg gcccaaggaa aagatagtct 2580

gatagatgct gcagccccat tttagcaaat gtgacctcac aggcctgaat gccatcgcta 2640

ttccacatct acaggataga cggaaaggaa agaaataaaa aaataggtac ctaacactgg 2700

caagaggatg atgactcatg ttatttcact taaccttttt atcttttaac atgaaggact 2760

catacaggtt gataagaaac cagtgacata aacagaccaa aaaatgatca gatctttcaa 2820

attagcaaaa aaataatatt ttttaaacaa tgggtgaaaa tacagtgtaa cagtaccaat 2880

tatcaacatg tgttgagaac cagaaaaatg ttctttttct ttgatcagca acactatttg 2940

ggaaaatcta tcctcagggc ctagcctggg gccctggcac acagtaggca ctcaacgaat 3000

atttgctgaa cacacaaata cttatgatat tttaaaaaat tggcaacaat ctgataccta 3060

acaatagagg gattaaatat tatggaactg ttaaataaga tgcttatgaa taccatgcag 3120

taagatgggc aatatttatg ccataagctt taatgaaaca aatgggtatt aaatgtatga 3180

taaggttata aattactttt taaaagatta cagggaaaaa aattgaaaga tatacactga 3240

aatgtttttt gctcacagtg gtgacaaggt ttctcagcac tggcactgtt gacgttttag 3300
gctgtatgtc tttgctgtgg gaggctggcc tgtgcactgc agggtgtttg gcagcactct 3360
tggcctctgc ccctagatag caatagcagt cctccctcaa ccagcccaat tttgacaacc 3420
aaaaatgttt ccaggcatca ccagatgctc cctgggtgag agtgatgaaa tagtagggga 3480
ttttcccctt cttttcttat tttctgtaat tccattatat tactttaata ataaagaaaa 3540
aaacataaaa aataaacgaa tgttattatt ctacgtcagt ttggatgttt ggactccatt 3600
ttggggttct ttccattata tcacttggtc tgctaaacat tctacggttt ggtaaggtga 3660
agtgattcat gaaattttgg ttttattttt ttcctgatac taaaaataaa acattctttc 3720
acttggaaat ttggacacag aacaccaaaa aaaatccata atctcatctc tctttttctg 3780
tcttttcctt cctttttttcc ctttaaaaac aataaagagt gaaacctacc tgttctccct 3840
ctaatttaat tcctaaatat aatcactgtc aatatcttgg acatttcctg tgtctaaaca 3900
cacacacaca cttttttttt tcagcaaaag tggatttctg ctacatgtag tgttctgcaa 3960
cttactttct atgtgtttac aaaatcagta catgtacata tgctgaattc agtccttaat 4020
ggtattatat tttgtgaata taccaaaatt tgtttaacca cttagacaat ctaggatatt 4080
ctcagtttgc tgttatgagc aatgctcttc ctttacatat acagacatat atatatatat 4140
gtgtgtgtgt gtgtttttgt tttagtagga tagatttcta ggagagggtg aaaggtctta 4200
tgacatccgc atttacgatt gtaataggaa gtatcaaagt gccccctaaa gaaaaaaatc 4260
ctcccattag tgggtaagaa agcctatttg ttcatatctt cacaaacact aaatattaga 4320
aatatttaca attgtggtca agctcataag tgaaaatggt atttcatatc ttatattttt 4380
tattgtgaga ttgaacatct ttcatatgtt tacatgtcac ctgtatttct tattctctga 4440
actatatgtt atgacctttc actttttttc ctcatgggtt atgtgtagtt tgtatagttg 4500
tcttattgat tgttaggagc tatttatata ttaggaacat taatctcctg tcttatatat 4560
acgtggcatc gattagttga tcatttgtga gttcatgtct gtatacaaag attggagagg 4620
cactaagagg gaaaacttac ctctttctta tcaaagtttg taaatatatg tataacagaa 4680
gagggagaaa atattaataa atgcacagat tggctgaaat agagtataaa tcttttactc 4740
ccctacttca acataaactg caaaaggaga gtgacttttc tttcactctg acttccgtat 4800
tcctcatgct taaaatagtg cctagcacag aagaggtgct caatcagtgt ttgctaaacg 4860
aaataattag tcacatttca agcaggatga ctaaatgaag aatagaatct aggcagatac 4920
tctggaagag tggctgtgag tcattcatat cttagtatga attagtcaaa tccaactctc 4980
tccccttccc actccccact gttagtagaa gaatctgttt attgagagaa tagatttata 5040
atttagaata agtgagaggg gcagaagagg agattttgaa ggatggcacc tgaaggagga 5100

```
ctagcatggc tgagacagtg aagtggaagc cttgaatagc taaagggtaa gatgaaagta     5160
tttagctgta gggggaaaaa gcattgacag gttggaaaag taaaagtcag attctccttg     5220
ctctgaaatt ttgtacaggg caggttctac taggtatgtt acaatgcaga aaaaacatga     5280
aataattgag aggaatttgg tgcaatatta tcttcttggc ttcttttgag tgggcagatt     5340
tttttcacgg cctgtaacta taataaattt gaaacttctc atcttttagt aacttttttc     5400
acttaagttt atgtggctgt gggcaatgga atgaagatat tgaacttcca attccctgtt     5460
gggtttccac aattacaagt caatcatgac tggttattag aagactattt cagttagaac     5520
caccaagtcc catattgtca tattgtatgt ttaattatta agtgaagcag tcttcttttc     5580
gtgttttcca taattagggc attccagaaa gatgaggata tttgctgtct ttatattcat     5640
gacctactgg catttgctga acggtaagac accaaatcct tccattaggt tctatatttt     5700
aaatatttta accatgagtt taaaactaaa atgatcattt aaaatgcatg caattttctt     5760
atagagagaa cattctattc tttcttctac tttacacaat ggcaaagtct tctttctact     5820
ttacgcaatg ataaagttac ctgtgtcatt ttgtaaaaat atagagaata tagacaaatt     5880
gaaagacaca aaataatcta ttacccattt cccagggtta actactgaaa atatctgggg     5940
aaatggcctg tatgtataca tttatttgtt tgctttcaac aaggccaaga tcctttgatc     6000
tttcagtctt ggttgctctg tgacatgcct ttcctgatga ggatacttta aggaagaatt     6060
gtaagataca tggaaaatgt caggctaaca cagtactggc atcaccctgt gctctttcct     6120
gaactccata ccaatgtact tcttgccaga aaactgatca aaagtttagg gaagtaaaaa     6180
gagatgactg ttagaatcta ccattccctc tatgtaggaa gcaaataggt gtcctgtcaa     6240
aggacattct ggggatgtct acatgaaacc aactctccct ggttgtaagg actccatctc     6300
catataatat ttatacagta atatatgttt ataaattgtg ggggcaactt gtttagctaa     6360
ttttattatt ctgctattgg gacactgtgt ctcagcatga gatatagtgt cccaaaacat     6420
atttcaagcc cattggataa aatatgtgtt tagcaagttc ttaaatataa tgataacata     6480
accgaccaga taaagtgatt tataaacgct gtgccaattt tgtaaatgtt tcgaggaatt     6540
ttcccttttc tgaagattgt ccttctttct ttttagcatt tactgtcacg gttcccaagg     6600
acctatatgt ggtagagtat ggtagcaata tgacaattga atgcaaattc ccagtagaaa     6660
aacaattaga cctggctgca ctaattgtct attgggaaat ggaggataag aacattattc     6720
aatttgtgca tggagaggaa gacctgaagg ttcagcatag tagctacaga cagagggccc     6780
ggctgttgaa ggaccagctc tccctgggaa atgctgcact tcagatcaca gatgtgaaat     6840
tgcaggatgc aggggtgtac cgctgcatga tcagctatgg tggtgccgac tacaagcgaa     6900
ttactgtgaa agtcaatggt aagaattatt atagatgaga ggcctgatct ttattgaaaa     6960
catattccaa gtgttgaaga cttttcattc ttgtaagtcc atacttattt tcaaacagaa     7020
```

cagcatagtc tgttcattca ttcattcaat tcatgaattc attcacataa ttatccaatt 7080
tcttgagcac ctatttgata gtcactggaa atccagagac aaacaacaca gagccatgtt 7140
ctacagtatg tacagttttc caaaaagaat ttctagtctt tactttttta ttacaaatgg 7200
aatacgtata cttgcaaata attcagatac tgtggaagag atcaaatgaa ttgcaaaagt 7260
gtccctcctc ccttcaccac tatctcccat ggcatgcaga gagagtaacc attatttgtg 7320
tgtccctcca gaaatttttt tattcaacta ctattttttt attttattag gtccgtcagt 7380
tttccttttt tgagcctctc tatatcaaat gcaaataaat atattcagaa caaaccccac 7440
tgtaaggttc acattaaaaa agacttgaag tcaccctatg aagacaaaaa ataatcacat 7500
taagtgtgaa agaacctatt cttccagtac aggataagcc atacttactg ggcatatatt 7560
catcttgaaa atctatactg atgttgtctt ggggaattga aaaggaacta ggagtgttag 7620
ttcctcggta ttgacccaca gttatgttat caggtcactt gagttcaaag ttttgtgttg 7680
gcactagcta agtaaaggaa aacacctctg ctttcattgt tgagtttcac agaattgaga 7740
gctgaaagga tcccaggcag gagcagctaa tccaaactcc cacaaagaac aaaaatcccc 7800
cagaggatct tctgttctta tatttcctgc aatggcgtcc ctgtcatatc ccacaatggc 7860
ctccctgcca tttggatatc ccttccatat cctgttgaaa ttactcccta atagtaagct 7920
gaaatctgcc cctctagttg tagtcttggg attatttcat ttacatgatg accttttaat 7980
atttgactag aattaaatca tctccccttg gtctttccat tcctgggcta actaccatca 8040
atctgagggc taacaataca agtagaaaaa gtatacattt gtcactgatc actgatcaat 8100
tattaatcaa tgatcactga taactataaa ctcaaaaaca aaatcatgtg gggattaaga 8160
gaaatgtatc agttttatgt tgtatttctg gtccctgata ctggctcagg taatgccact 8220
attgtcaaga agataccact tgtaaagtag atttaatttt cattatattt taccatatgc 8280
ttctccattc atgacatctc ttgagatgtt gtggtttata ctttcagttt ttctccagtc 8340
catccgcaaa tatcaggcat ctactgtgtt ccaagatatt aaagaaatca tcatgactta 8400
gcctcatcaa cagcattgct agatctggga tggaaaggaa gagtataatc ctggcagtca 8460
ggaagaaggc agcataaagt ataagtttct gcttccaaaa aaggtctctc atcagcctgt 8520
agggagtgtg tagggaaggg acagctgtcc ttgtagtagg gaagggtttt attcaggtcg 8580
tctgggctcc ataatatccc ttgtgtatct gcagtctcct ttgccatgga tcaacacaat 8640
aggaaatctt ccggcactga tggtttttcc aagggggagt tcttcctgga gcaaagcaaa 8700
tgaccaacca ggtttgagga cctgatttgt ttgacaattc cattttgtat tgtaaattac 8760
ttaattggca ttctactccc aatccatctt gtcatttgca tacagtggtt ttgggattga 8820
gttcagctat accaaaagtc tgaaccttct gcacttagaa caaggcaacc accaagcttc 8880

acttgcactg aggccgtgtc tccaatggaa atgaggcagc tggcttgcag gagcttccca 8940
actcagggaa gtagaactcc tgagtcacct ccatatgcaa atgatttcac agtaatgctg 9000
ttgaacttca cttcccatca cagcaaatgt gtggtaacat agcttcccca caggagttta 9060
ctcaccatgg tattttaaag gtgaaacatt tcaaaactga aatttgaaag aatttagttt 9120
tggattcact caattatcac tatcacttcg ggtgttattg cacctttctt gtttgtgagt 9180
ttaaatgcca gactctcagg ccactaactt tcaattaaaa gtgtttttct ttaatcgctg 9240
aacctaacag cagggaaaac gaaatgttca ttcagacttt cagaaccttc aatgagatta 9300
ggcagctgaa agatcaaagt gttgcatagt tgtcccgata aagctatttg gatcatatgg 9360
accaaatcga ctgctgtcat tccccaccaa ccccatctct ccccaaaatt cccagccctg 9420
tttaagtgtt ctctgtagca tttatctcta tctagtatat tgtgtagcat atcatatcat 9480
acttttctgt tttgtttatt gtctctctcc tcctagaata taaactccac aagcacaaag 9540
atttgggcct gttttataat attgttgcat ccccagggcc tgatatacag cagagtggtg 9600
gtacgaaaag agcacacaaa aaaatatttg ttgagtcaat gaatgaatga tttcctcaaa 9660
taggattagc ctaaaatttt ggaaacatga acagatttgg atatgtgaaa atttatttcc 9720
agactgttca tcaggaactg ttagcagctt ctaaagggta cactggagca gcagtagtaa 9780
aaggaggaag aggagcagct ctgctactgc tactatcgag tactactaca attagcactt 9840
gcttattctg tgtgttaggc cctgtactga acactctgtc taaattagtt catttcctcc 9900
tggaaatgac tctagggggt aagtgcttca tcatgtaaga tgagtatttt tcacattttg 9960
ttgtgtctga aatctgagtg tgtctttcaa tgatggaatc tttgattcca tgataagtgg 10020
tattattccc attttaagga tgaggaaact gaggtccaaa gaaattaagt aatttgccca 10080
aattcaccca gcctagaaaa tgataaagct agttctaaac ccaagcagat tagctctgaa 10140
gtctgggccc ttaataacca ctttttattg cctatatttg tacctctggt gtacgtatca 10200
agttatatgt tgacttcaaa actatcatga ccttttcttg gttttgattg tccaacatta 10260
gtatagtgtt ctgggtctgc aaaaattttg attactcatc tcatctgtaa aacattttga 10320
actcgtgtgt ttgtgcatgc acatttgtgt gtaattataa aaattttact ttctgttaat 10380
atataagttg tatcataaga aactgccgtt tttgaagagc aaaaaaaggt tgaatgttac 10440
cagttacatc tggttcaacc taatagacat ttgtacaaaa acagacattt taagaggttg 10500
aaataaaaat ttaataaaca atattttcag tttttactaa ttgtgatgct tcactatcat 10560
tagctaatat gtcaaggcat aatatacctt agggtgaact ttatcattaa caaaggtgga 10620
tggtgtcaat aatcttgagg tttgtgtttt tttatataac actgcgaggt ctaattaagt 10680
acttactgtt taccacctca tacagtggcc gataaaaagt gtcacttctg ctgtttcctc 10740
tgggttgtgc ttgaattatt agtattatct tcagtcctca gtttctttgt gggaaacttt 10800

ttaattagtt gtttaatttt gtaagatggt tagtttagtc aaaattagat aagagaattt 10860
gaaaatccgt agctacccca aagcaaccta cacataagaa ctattatttt tgtgttttga 10920
aatcataatt ttattgattt ccagtgtttc cactggtagt ggtttcattg atataggagt 10980
atcaaaacat cactcattat ttatttcagt ttcatttgat cctagccgtt ttgtattaac 11040
tctctgtgaa gaaattacct cacaaatcta ttgctgtcct tggtaaagga atggagaatt 11100
aaggctctag atcattagtg gttacactat agtattagaa gtaaaaaaaa gattatacca 11160
acaaaataag aacatgttaa tgtacttgta atgaataaac atgaataaag ctcttatgct 11220
atataggtgc actaaacaat ctactagaat tgtcagcaaa ctacgtatct taatcctgaa 11280
agggtcccaa accaatgatc taaaattgaa tcaaactttc ttccttgagc ataattactt 11340
aaatgattta ttaaaatagc cagcatttaa aagcttaaaa tgtaaatatc ataatgtggt 11400
atcctagata gcatcccaga acagaaaaag gatattaggg aaaaactgga ggaatggaat 11460
aaattatgca gtttagttat taataatgta ctaacgtcct tagttatgac gattgtacca 11520
tggtaatgta agatactaac aatagaggaa accgggtaag gagtatacag taactctata 11580
ctatctttgc aactttttg taaatttaaa acttctaaaa taaagaacaa atttaaacat 11640
taaaaagtat caccaggaac atatatcact gtttacagat gaaatactat gtattttcat 11700
atctaatttc tgatcattga cttcaaatca gaaaagtgaa tgacacctca aaatcaggtt 11760
ttctgtttac tgaagtctaa gaaaagaaag cataccagct ggagagattc atgtttataa 11820
agacagattt ataacaacaa aaataaaata tccaagaata aatttaagaa gaagcacttt 11880
actgagaaac atatgaaaac ctgaacaaat ggagagggat attttgtatt tgaatagaaa 11940
gacttctggt ttaaagataa ttctctttaa attattttt gtagaaattt aaggggtaca 12000
agagcagtgt tgtcacatgg atatattaca tagtggtgaa gtctggggtt ttagtgtaaa 12060
ttaatcttta cattttgttt gagcccaata aatgtaccaa catgattttt atagaaagat 12120
agtcattcct attaatccaa acttgtccca actttgaatt gaattgaggc agagctagca 12180
ggtgttcccc acggctgagg catctgaaca ttaagcatat ccctctgaga accagcctgc 12240
attgatactc tttctaatgt ggacagcatc aagctatgta cgtagttctg tgctcagcaa 12300
aagccctgac ttctttttgt ttatgtccta gccccataca acaaaatcaa ccaaagaatt 12360
ttggttgtgg atccagtcac ctctgaacat gaactgacat gtcaggctga gggctacccc 12420
aaggccgaag tcatctggac aagcagtgac catcaagtcc tgagtggtaa gaccaccacc 12480
accaattcca agagagagga gaagcttttc aatgtgacca gcacactgag aatcaacaca 12540
acaactaatg agattttcta ctgcactttt aggagattag atcctgagga aaaccataca 12600
gctgaattgg tcatcccagg taatattctg aatgtgtcca ttaaaatatg tctaacactg 12660

tcccctagca cctagcatga tgtctgccta tcatagtcat tcagtgattg ttgaataaat 12720
gaatgaatga ataacactat gtttacaaaa tatatcctaa ttcctcacct ccattcatcc 12780
aaaccatatt gttacttaat aaacattcag cagatattta tggaatatac cttttgttcc 12840
atgcattgta gtactcattg gatacacata gaataataag actcagttca cactcttcag 12900
gaaacagata aaaaactaag aaacaaacaa aaaacaggca atccaacacc atgtgggaaa 12960
tgctttcata gccgggaaac ctggggaata cctgagagga atactcaatt caggccttgt 13020
ttcaggaatc caaatcctgg cacatcagag ctgcttccct ctttccaggg tggcaggaaa 13080
taaatggaac atatttttct atcttatgcc aaacatgagg gacccttttct ccccggtgcc 13140
tctcccaagg tagtctacaa tatttcaact ctagcagtct gcttagtgca tagaacatga 13200
ggctgtgtgt ccctgggcaa attactagac ttctgtgtgc ttcactttcc ctgtaggatt 13260
ataatctact gagcaagctt attgtaaggg tcagattagc aacagtgtat gaaaatgatt 13320
tgagaccatt gcctgcacaa attcaactat tttttttat ctcactactc tacagaagta 13380
ggtagggtgg gagacagagt ctgatgagag gctcagaatg tgaaagaaag tgaggcgagt 13440
gagcatgata tttaatataa acacaaagat attctgagaa gagctgctca ctgccccctc 13500
ccccaataca tgttgatagg aaaatgccac gtacttcagc aaaaacaact gaaaaattag 13560
atagaaaagt caatcaatag gaaaagataa tccaggacgg tgttgtgaac agaaagaggg 13620
ggaaaaaact ttagaaaatg atggggatgc tcttactggg gtacgagtcc tcaggtattg 13680
aactggcttt cagtaaaagc tagattagtg ggttcctgcc atttacaagc tgttttatga 13740
caacttactt gttgggtggc ctacagtaac tcacctaact gcactgagtc tgtttcctca 13800
tctgtaaatt ggggattttt ttttaaatac ctggcatgcc taactcataa agttgttctg 13860
aaactgaaat aaaacatacg tgaacaggca ttgtaaactg taagttacgg aaaaagctgg 13920
ctgttgttgt gtctttaaag tttcacctgg gtagtcaaag atggatcatg ggtctcagtg 13980
gagagctgag ccaggcagga gctgactaag ggtgagaggt gggagttagc agcctctgaa 14040
catctgtgta ccatgggacc ccctttcctc ctgcatggta ccccagacaa ggagcctagt 14100
aagagatact aatggcttgt tgtccagaga tgttcaaact gcagagaaag ataagacaac 14160
aagcattggc ctccaatcat gatgacagat aggaggaggt gggagctcct tagcagtgct 14220
ggttggcctt ccatgttcta ctgtgggcca tctctgccat gtactgtagg ctactagctt 14280
ctatattaaa gaatgcaaga ggggccagga gcggaggctc atgcctgtaa tctcagcact 14340
ttgggaggcc aaggtgggca gatcacttga ggtcaggagt ttgtgaccag cctggccaac 14400
atggtgaaac tctgccttta ctaaaaatat aaaaattagc tgggtgtggt ggtgtgcacc 14460
tgtaatccca gctactcggg agactgaggc acaagaattg cttgaacctg ggaggcggaa 14520
gttgcagtga gcccagattg cgccactgca ctccaccctg ggcaacagag aaagactctg 14580

cctcaaaaaa aaaaaaaaaa agcaagagga agtgaaataa tcaaggccgc catttaatag 14640
tgagcagcca ctccatgtgg tactgtgcaa gcacattata aatattagcc tcacaagaaa 14700
tgtattagca tttgtatttt gtacactggt taagtatctt gcccaagacc tcaaaactgg 14760
ttaagggcag cagaatttag ccccagcacc accttttcaa agcctgggct tctcacactt 14820
ctccatgctg ttcccatttt aacacaggta tctcgccatt ccagccactc aaactttggc 14880
atttaagaaa attatcctaa agctaaacta aacttcaagg atgaccattc tcctgacccc 14940
ttcccatcaa aattttatct ttagtcagtt tgttttcgtt ttgttttgtt tttcagaact 15000
acctctggca catcctccaa atgaaaggac tcacttggta attctgggag ccatcttatt 15060
atgccttggt gtagcactga cattcatctt ccgtttaaga aaaggtagta tttccttaat 15120
tgcagtggtc tccactgggg gtgaggaagg ggtgagaatt ggatcatggc tgcaaggaaa 15180
cccgacttaa cctctgcaag gtggtgcaaa ggcattccac tgttcaacag caattatatt 15240
gaagctgagt gggatcactg ggtgaagatg aagcgtaagg ggtgaggggc aggagaatgg 15300
gtatggatgg aggtagaaga tgcagtgtca tacagttttt ttctatcatg aaaataacca 15360
cagacttaca gaagagaaag agctaaaatg cccgtcattt tcagttgcat tttagtcttg 15420
cattagttgc aaccagctgg tttctgggta ccctaagtaa taaaaatagt tcctctgtag 15480
aactgtagta tgtttaccat agagtatttt gcaaaatttt tggtagagga tgttacataa 15540
tttgcatgtg ttcatttctc catttacctg tgggaacaat taaaatccag gaaaatgagt 15600
atattcaaat aatttcctcc catttaagat gagtcagagt aaataattcc tccaatactt 15660
agagaagtat accaagagat ccagtgatgg tatagagttg tctgatgtta aatagggaag 15720
tagaatatgg aaggggattc caatagtcgt tgaaaaattc cccataaccc cttacatggg 15780
ggaaagtagt gttaactgag agagtagaga taagctgttt ccaaaaatta tattcttaac 15840
aggactgaga tagccagaat ataaggatca agtttcaatg acagtaagat cctgagatgg 15900
agttgatttg cacaaagaaa taattgttgc cagcatgcat tttgaatatt tctctggaaa 15960
aaaagattag ttggcagtag aaatggatag aaatcaatag atattaaaat acctcagaat 16020
ttggttcatc tctgggaaaa gatgaaaaat aaaagtgtat actcctcaag aacatctagg 16080
atcaaaagca tgtgccctac actattgaat taattaacct cataagttgg gacctgtgga 16140
ataaggatgt ccaccagact tcctagggat tacaaatgtt tcacagaact tgaaatttaa 16200
acttgggtca ctgtatggga tgtagagctg tgctatatgg aaataaaaat gatttctttt 16260
tctcaaggga gaatgatgga tgtgaaaaaa tgtggcatcc aagatacaaa ctcaaagaag 16320
caaagtggta agaatatcag aaggaattgg gaagtaaaag tcaaaggaaa caaaaagcta 16380
aagcaataac aaagagaaat ccatcagtca taatctcctc tccttttaaa gaatgctggt 16440

tcccctttgc ctcacagcta acacaagaac tcctccaccg tctgaggagg tttaggagca 16500
gggaagggga aggagtcagc ttcatttgct aatcttctgt tgccctgcac cctagcagct 16560
ccttgcagca ggggacaagg atgacttagg tggatggata attaattgat tctaaaatat 16620
tgtgtgtcag tattgtaata ctatgttaat tgcaccatgc acggtatctc atttaatccc 16680
ccaccccttg ccattaccaa agagagagag agagagagag agagaaatac tagaatttat 16740
cctcatttta cagtagagaa aacagagggt caagaagata atgtaaagtg cccaagaaca 16800
cacagctgat cacaaaaatc aagcttgggg gccattagcc taaccacaga cccttactct 16860
taacccatct gcttcaatcc attttgctac aaatgtttac atttataagc agggcagaaa 16920
aacctcatcc aggttattga actaagaaga aagttatatt aaggtttcta atttttttaa 16980
tgtagttaga aaccaaactt aacaatgagc ccaagtttaa agcagtctaa ttaacctgga 17040
caagctcagg caagtttcat tctgtggccc atagcatcat ctgtgttgta aagctaagta 17100
gcaaatgttg tttgggtcat gctgggggac aagccatccc aatttgctca ggactgaggg 17160
gttttccagg atatcatgta aggataattg ggtacaaata taacctgctg ctttctctca 17220
tttcaaattt atcatttatc atatcagcaa ctatgagtta tgttttttat tagatttctt 17280
gttacttttt ccccagacca cttcccatga aattaatata ctattatcac tctccagata 17340
cacatttgga ggagacgtaa tccagcattg gaacttctga tcttcaagca gggattctca 17400
acctgtggtt taggggttca tcggggctga gcgtgacaag aggaaggaat gggcccgtgg 17460
gatgcaggca atgtgggact taaaaggccc aagcactgaa aatggaacct ggcgaaagca 17520
gaggaggaga atgaagaaag atggagtcaa acagggagcc tggagggaga ccttgatact 17580
ttcaaatgcc tgagggctc atcgacgcct gtgacaggga gaaaggatac ttctgaacaa 17640
ggagcctcca agcaaatcat ccattgctca tcctaggaag acgggttgag aatccctaat 17700
ttgagggtca gttcctgcag aagtgccctt tgcctccact caatgcctca atttgttttc 17760
tgcatgactg agagtctcag tgttggaacg ggacagtatt tatgtatgag tttttcctat 17820
ttattttgag tctgtgaggt cttcttgtca tgtgagtgtg gttgtgaatg atttcttttg 17880
aagatatatt gtagtagatg ttacaatttt gtcgccaaac taaacttgct gcttaatgat 17940
ttgctcacat ctagtaaaac atggagtatt tgtaaggtgc ttggtctcct ctataactac 18000
aagtatacat tggaagcata aagatcaaac cgttggttgc ataggatgtc acctttattt 18060
aacccattaa tactctggtt gacctaatct tattctcaga cctcaagtgt ctgtgcagta 18120
tctgttccat ttaaatatca gctttacaat tatgtggtag cctacacaca taatctcatt 18180
tcatcgctgt aaccaccctg ttgtgataac cactattatt ttacccatcg tacagctgag 18240
gaagcaaaca gattaagtaa cttgcccaaa ccagtaaata gcagacctca gactgccacc 18300
cactgtcctt ttataataca atttacagct atattttact ttaagcaatt cttttattca 18360

aaaaccattt attaagtgcc cttgcaatat caatcgctgt gccaggcatt gaatctacag 18420
atgtgagcaa gacaaagtac ctgtcctcaa ggagctcata gtataatgag gagattaaca 18480
agaaaatgta ttattacaat ttagtccagt gtcatagcat aaggatgatg cgaggggaaa 18540
acccgagcag tgttgccaag aggaggaaat aggccaatgt ggtctgggac ggttggatat 18600
acttaaacat cttaataatc agagtaattt tcatttacaa agagaggtcg gtacttaaaa 18660
taaccctgaa aaataacact ggaattcctt ttctagcatt atatttattc ctgatttgcc 18720
tttgccatat aatctaatgc ttgtttatat agtgtctggt attgtttaac agttctgtct 18780
tttctattta aatgccacta aattttaaat tcatacccttt ccatgattca aaattcaaaa 18840
gatcccatgg gagatggttg gaaaatctcc acttcatcct ccaagccatt caagtttcct 18900
ttccagaagc aactgctact gcctttcatt catatgttct tctaaagata gtctacattt 18960
ggaaatgtat gttaaaagca cgtatttttta aaattttttt cctaaatagt aacacattgt 19020
atgtctgctg tgtactttgc tatttttatt tattttagtg tttcttatat agcagatgga 19080
atgaatttga agttcccagg gctgaggatc catgccttct ttgtttctaa gttatctttc 19140
ccatagcttt tcattatctt tcatatgatc cagtatatgt taaatatgtc ctacatatac 19200
atttagacaa ccaccatttg ttaagtattt gctctaggac agagtttgga tttgtttatg 19260
tttgctcaaa aggagaccca tgggctctcc agggtgcact gagtcaatct agtcctaaaa 19320
agcaatctta ttattaactc tgtatgacag aatcatgtct ggaacttttg ttttctgctt 19380
tctgtcaagt ataaacttca ctttgatgct gtacttgcaa aatcacattt tctttctgga 19440
aattccggca gtgtaccttg actgctagct accctgtgcc agaaaagcct cattcgttgt 19500
gcttgaaccc ttgaatgcca ccagctgtca tcactacaca gccctcctaa gaggcttcct 19560
ggaggtttcg agattcagat gccctgggag atcccagagt ttcctttccc tcttggccat 19620
attctggtgt caatgacaag gagtaccttg gctttgccac atgtcaaggc tgaagaaaca 19680
gtgtctccaa cagagctcct tgtgttatct gtttgtacat gtgcatttgt acagtaattg 19740
gtgtgacagt gttctttgtg tgaattacag gcaagaattg tggctgagca aggcacatag 19800
tctactcagt ctattcctaa gtcctaactc ctccttgtgg tgttggattt gtaaggcact 19860
ttatcccttt tgtctcatgt ttcatcgtaa atggcatagg cagagatgat acctaattct 19920
gcatttgatt gtcacttttt gtacctgcat taatttaata aaatattctt atttattttg 19980
ttacttggta caccagcatg tccattttct tgtttatttt gtgtttaata aaatgttcag 20040
tttaacatcc cagtggagaa agtta 20065

<210> 44
<211> 3691
<212> DNA

<213> Homo sapiens

<220>
<221> source
<222> 1..3691
<223> /mol_type="unassigned DNA"
/organism="Homo sapiens"

<400> 44
ggcgcaacgc tgagcagctg gcgcgtcccg cgcggcccca gttctgcgca gcttcccgag 60

gctccgcacc agccgcgctt ctgtccgcct gcagggcatt ccagaaagat gaggatattt 120

gctgtcttta tattcatgac ctactggcat ttgctgaacg catttactgt cacggttccc 180

aaggacctat atgtggtaga gtatggtagc aatatgacaa ttgaatgcaa attcccagta 240

gaaaaacaat tagacctggc tgcactaatt gtctattggg aaatggagga taagaacatt 300

attcaatttg tgcatggaga ggaagacctg aaggttcagc atagtagcta cagacagagg 360

gcccggctgt tgaaggacca gctctccctg ggaaatgctg cacttcagat cacagatgtg 420

aaattgcagg atgcaggggt gtaccgctgc atgatcagct atggtggtgc cgactacaag 480

cgaattactg tgaaagtcaa tgccccatac aacaaaatca accaaagaat tttggttgtg 540

gatccagtca cctctgaaca tgaactgaca tgtcaggctg agggctaccc caaggccgaa 600

gtcatctgga caagcagtga ccatcaagtc ctgagtggta agaccaccac caccaattcc 660

aagagagagg agaagctttt caatgtgacc agcacactga gaatcaacac aacaactaat 720

gagattttct actgcacttt taggagatta gatcctgagg aaaaccatac agctgaattg 780

gtcatcccag aactacctct ggcacatcct ccaaatgaaa ggactcactt ggtaattctg 840

ggagccatct tattatgcct tggtgtagca ctgacattca tcttccgttt aagaaaaggg 900

agaatgatgg atgtgaaaaa atgtggcatc caagatacaa actcaaagaa gcaaagtgat 960

acacatttgg aggagacgta atccagcatt ggaacttctg atcttcaagc agggattctc 1020

aacctgtggt ttaggggttc atcggggctg agcgtgacaa gaggaaggaa tgggcccgtg 1080

ggatgcaggc aatgtgggac ttaaaaggcc caagcactga aaatggaacc tggcgaaagc 1140

agaggaggag aatgaagaaa gatggagtca aacagggagc ctggagggag accttgatac 1200

tttcaaatgc ctgaggggct catcgacgcc tgtgacaggg agaaaggata cttctgaaca 1260

aggagcctcc aagcaaatca tccattgctc atcctaggaa gacgggttga gaatccctaa 1320

tttgagggtc agttcctgca gaagtgccct ttgcctccac tcaatgcctc aatttgtttt 1380

ctgcatgact gagagtctca gtgttggaac gggacagtat ttatgtatga gtttttccta 1440

tttattttga gtctgtgagg tcttcttgtc atgtgagtgt ggttgtgaat gatttctttt 1500

gaagatatat tgtagtagat gttacaattt tgtcgccaaa ctaaacttgc tgcttaatga 1560

tttgctcaca tctagtaaaa catggagtat ttgtaaggtg cttggtctcc tctataacta 1620

caagtataca ttggaagcat aaagatcaaa ccgttggttg cataggatgt cacctttatt 1680
taacccatta atactctggt tgacctaatc ttattctcag acctcaagtg tctgtgcagt 1740
atctgttcca tttaaatatc agctttacaa ttatgtggta gcctacacac ataatctcat 1800
ttcatcgctg taaccaccct gttgtgataa ccactattat tttacccatc gtacagctga 1860
ggaagcaaac agattaagta acttgcccaa accagtaaat agcagacctc agactgccac 1920
ccactgtcct tttataatac aatttacagc tatattttac tttaagcaat tcttttattc 1980
aaaaaccatt tattaagtgc ccttgcaata tcaatcgctg tgccaggcat tgaatctaca 2040
gatgtgagca agacaaagta cctgtcctca aggagctcat agtataatga ggagattaac 2100
aagaaaatgt attattacaa tttagtccag tgtcatagca taaggatgat gcgaggggaa 2160
aacccgagca gtgttgccaa gaggaggaaa taggccaatg tggtctggga cggttggata 2220
tacttaaaca tcttaataat cagagtaatt ttcatttaca aagagaggtc ggtacttaaa 2280
ataaccctga aaaataacac tggaattcct tttctagcat tatatttatt cctgatttgc 2340
ctttgccata taatctaatg cttgtttata tagtgtctgg tattgtttaa cagttctgtc 2400
ttttctattt aaatgccact aaattttaaa ttcatacctt tccatgattc aaaattcaaa 2460
agatcccatg ggagatggtt ggaaaatctc cacttcatcc tccaagccat tcaagtttcc 2520
tttccagaag caactgctac tgcctttcat tcatatgttc ttctaaagat agtctacatt 2580
tggaaatgta tgttaaaagc acgtattttt aaaatttttt tcctaaatag taacacattg 2640
tatgtctgct gtgtactttg ctatttttat ttattttagt gtttcttata tagcagatgg 2700
aatgaatttg aagttcccag ggctgaggat ccatgccttc tttgtttcta agttatcttt 2760
cccatagctt ttcattatct ttcatatgat ccagtatatg ttaaatatgt cctacatata 2820
catttagaca accaccattt gttaagtatt tgctctagga cagagtttgg atttgtttat 2880
gtttgctcaa aaggagaccc atgggctctc cagggtgcac tgagtcaatc tagtcctaaa 2940
aagcaatctt attattaact ctgtatgaca gaatcatgtc tggaactttt gttttctgct 3000
ttctgtcaag tataaacttc actttgatgc tgtacttgca aaatcacatt ttctttctgg 3060
aaattccggc agtgtacctt gactgctagc taccctgtgc cagaaaagcc tcattcgttg 3120
tgcttgaacc cttgaatgcc accagctgtc atcactacac agccctccta agaggcttcc 3180
tggaggtttc gagattcaga tgccctggga gatcccagag tttcctttcc ctcttggcca 3240
tattctggtg tcaatgacaa ggagtacctt ggctttgcca catgtcaagg ctgaagaaac 3300
agtgtctcca acagagctcc ttgtgttatc tgtttgtaca tgtgcatttg tacagtaatt 3360
ggtgtgacag tgttctttgt gtgaattaca ggcaagaatt gtggctgagc aaggcacata 3420
gtctactcag tctattccta agtcctaact cctccttgtg gtgttggatt tgtaaggcac 3480
tttatccctt ttgtctcatg tttcatcgta aatggcatag gcagagatga tacctaattc 3540

tgcatttgat tgtcactttt tgtacctgca ttaatttaat aaaatattct tatttatttt 3600

gttacttggt acaccagcat gtccattttc ttgtttattt tgtgtttaat aaaatgttca 3660

gtttaacatc ccagtggaga aagttaaaaa a 3691

<210> 45
<211> 290
<212> PRT
<213> Homo sapiens

<400> 45
Met Arg Ile Phe Ala Val Phe Ile Phe Met Thr Tyr Trp His Leu Leu
1 5 10 15
Asn Ala Phe Thr Val Thr Val Pro Lys Asp Leu Tyr Val Val Glu Tyr
20 25 30
Gly Ser Asn Met Thr Ile Glu Cys Lys Phe Pro Val Glu Lys Gln Leu
35 40 45
Asp Leu Ala Ala Leu Ile Val Tyr Trp Glu Met Glu Asp Lys Asn Ile
50 55 60
Ile Gln Phe Val His Gly Glu Glu Asp Leu Lys Val Gln His Ser Ser
65 70 75 80
Tyr Arg Gln Arg Ala Arg Leu Leu Lys Asp Gln Leu Ser Leu Gly Asn
85 90 95
Ala Ala Leu Gln Ile Thr Asp Val Lys Leu Gln Asp Ala Gly Val Tyr
100 105 110
Arg Cys Met Ile Ser Tyr Gly Gly Ala Asp Tyr Lys Arg Ile Thr Val
115 120 125
Lys Val Asn Ala Pro Tyr Asn Lys Ile Asn Gln Arg Ile Leu Val Val
130 135 140
Asp Pro Val Thr Ser Glu His Glu Leu Thr Cys Gln Ala Glu Gly Tyr
145 150 155 160
Pro Lys Ala Glu Val Ile Trp Thr Ser Ser Asp His Gln Val Leu Ser
165 170 175
Gly Lys Thr Thr Thr Thr Asn Ser Lys Arg Glu Glu Lys Leu Phe Asn
180 185 190
Val Thr Ser Thr Leu Arg Ile Asn Thr Thr Thr Asn Glu Ile Phe Tyr
195 200 205
Cys Thr Phe Arg Arg Leu Asp Pro Glu Glu Asn His Thr Ala Glu Leu
210 215 220
Val Ile Pro Glu Leu Pro Leu Ala His Pro Pro Asn Glu Arg Thr His
225 230 235 240
Leu Val Ile Leu Gly Ala Ile Leu Leu Cys Leu Gly Val Ala Leu Thr
245 250 255
Phe Ile Phe Arg Leu Arg Lys Gly Arg Met Met Asp Val Lys Lys Cys
260 265 270
Gly Ile Gln Asp Thr Asn Ser Lys Lys Gln Ser Asp Thr His Leu Glu
275 280 285
Glu Thr
290

<210> 46
<211> 3349
<212> DNA
<213> Homo sapiens

<220>
<221> source
<222> 1..3349
<223> /mol_type="unassigned DNA"

/organism="Homo sapiens"

<400> 46
ggcgcaacgc tgagcagctg gcgcgtcccg cgcggcccca gttctgcgca gcttcccgag 60

gctccgcacc agccgcgctt ctgtccgcct gcagggcatt ccagaaagat gaggatattt 120

gctgtcttta tattcatgac ctactggcat ttgctgaacg ccccatacaa caaaatcaac 180

caaagaattt tggttgtgga tccagtcacc tctgaacatg aactgacatg tcaggctgag 240

ggctacccca aggccgaagt catctggaca agcagtgacc atcaagtcct gagtggtaag 300

accaccacca ccaattccaa gagagaggag aagcttttca atgtgaccag cacactgaga 360

atcaacacaa caactaatga gattttctac tgcactttta ggagattaga tcctgaggaa 420

aaccatacag ctgaattggt catcccagaa ctacctctgg cacatcctcc aaatgaaagg 480

actcacttgg taattctggg agccatctta ttatgccttg gtgtagcact gacattcatc 540

ttccgtttaa gaaaagggag aatgatggat gtgaaaaaat gtggcatcca agatacaaac 600

tcaaagaagc aaagtgatac acatttggag gagacgtaat ccagcattgg aacttctgat 660

cttcaagcag ggattctcaa cctgtggttt aggggttcat cggggctgag cgtgacaaga 720

ggaaggaatg ggcccgtggg atgcaggcaa tgtgggactt aaaaggccca agcactgaaa 780

atggaacctg gcgaaagcag aggaggagaa tgaagaaaga tggagtcaaa cagggagcct 840

ggagggagac cttgatactt tcaaatgcct gaggggctca tcgacgcctg tgacagggag 900

aaaggatact tctgaacaag gagcctccaa gcaaatcatc cattgctcat cctaggaaga 960

cgggttgaga atccctaatt tgagggtcag ttcctgcaga agtgcccttt gcctccactc 1020

aatgcctcaa tttgttttct gcatgactga gagtctcagt gttggaacgg gacagtattt 1080

atgtatgagt ttttcctatt tattttgagt ctgtgaggtc ttcttgtcat gtgagtgtgg 1140

ttgtgaatga tttcttttga agatatattg tagtagatgt tacaattttg tcgccaaact 1200

aaacttgctg cttaatgatt tgctcacatc tagtaaaaca tggagtattt gtaaggtgct 1260

tggtctcctc tataactaca agtatacatt ggaagcataa agatcaaacc gttggttgca 1320

taggatgtca cctttattta acccattaat actctggttg acctaatctt attctcagac 1380

ctcaagtgtc tgtgcagtat ctgttccatt taaatatcag ctttacaatt atgtggtagc 1440

ctacacacat aatctcattt catcgctgta accaccctgt tgtgataacc actattattt 1500

tacccatcgt acagctgagg aagcaaacag attaagtaac ttgcccaaac cagtaaatag 1560

cagacctcag actgccaccc actgtccttt tataatacaa tttacagcta tattttactt 1620

taagcaattc ttttattcaa aaaccattta ttaagtgccc ttgcaatatc aatcgctgtg 1680

ccaggcattg aatctacaga tgtgagcaag acaaagtacc tgtcctcaag gagctcatag 1740

tataatgagg agattaacaa gaaaatgtat tattacaatt tagtccagtg tcatagcata 1800

aggatgatgc gaggggaaaa cccgagcagt gttgccaaga ggaggaaata ggccaatgtg 1860

gtctgggacg gttggatata cttaaacatc ttaataatca gagtaatttt catttacaaa 1920

gagaggtcgg tacttaaaat aaccctgaaa aataacactg gaattccttt tctagcatta 1980

tatttattcc tgatttgcct ttgccatata atctaatgct tgtttatata gtgtctggta 2040

ttgtttaaca gttctgtctt ttctatttaa atgccactaa attttaaatt catacctttc 2100

catgattcaa aattcaaaag atcccatggg agatggttgg aaaatctcca cttcatcctc 2160

caagccattc aagtttcctt tccagaagca actgctactg cctttcattc atatgttctt 2220

ctaaagatag tctacatttg gaaatgtatg ttaaaagcac gtatttttaa aatttttttc 2280

ctaaatagta acacattgta tgtctgctgt gtactttgct atttttattt attttagtgt 2340

ttcttatata gcagatggaa tgaatttgaa gttcccaggg ctgaggatcc atgccttctt 2400

tgtttctaag ttatctttcc catagctttt cattatcttt catatgatcc agtatatgtt 2460

aaatatgtcc tacatataca tttagacaac caccatttgt taagtatttg ctctaggaca 2520

gagtttggat ttgtttatgt ttgctcaaaa ggagacccat gggctctcca gggtgcactg 2580

agtcaatcta gtcctaaaaa gcaatcttat tattaactct gtatgacaga atcatgtctg 2640

gaacttttgt tttctgcttt ctgtcaagta taaacttcac tttgatgctg tacttgcaaa 2700

atcacatttt ctttctggaa attccggcag tgtaccttga ctgctagcta ccctgtgcca 2760

gaaaagcctc attcgttgtg cttgaaccct tgaatgccac cagctgtcat cactacacag 2820

ccctcctaag aggcttcctg gaggtttcga gattcagatg ccctgggaga tcccagagtt 2880

tcctttccct cttggccata ttctggtgtc aatgacaagg agtaccttgg ctttgccaca 2940

tgtcaaggct gaagaaacag tgtctccaac agagctcctt gtgttatctg tttgtacatg 3000

tgcatttgta cagtaattgg tgtgacagtg ttctttgtgt gaattacagg caagaattgt 3060

ggctgagcaa ggcacatagt ctactcagtc tattcctaag tcctaactcc tccttgtggt 3120

gttggatttg taaggcactt tatccctttt gtctcatgtt tcatcgtaaa tggcataggc 3180

agagatgata cctaattctg catttgattg tcactttttg tacctgcatt aatttaataa 3240

aatattctta tttattttgt tacttggtac accagcatgt ccatttctt gtttattttg 3300

tgtttaataa aatgttcagt ttaacatccc agtggagaaa gttaaaaaa 3349

<210> 47
<211> 176
<212> PRT
<213> Homo sapiens

<400> 47
Met Arg Ile Phe Ala Val Phe Ile Phe Met Thr Tyr Trp His Leu Leu
1 5 10 15
Asn Ala Pro Tyr Asn Lys Ile Asn Gln Arg Ile Leu Val Val Asp Pro

```
            20                  25                  30
Val Thr Ser Glu His Glu Leu Thr Cys Gln Ala Glu Gly Tyr Pro Lys
        35                  40                  45
Ala Glu Val Ile Trp Thr Ser Ser Asp His Gln Val Leu Ser Gly Lys
    50                  55                  60
Thr Thr Thr Thr Asn Ser Lys Arg Glu Glu Lys Leu Phe Asn Val Thr
65                  70                  75                  80
Ser Thr Leu Arg Ile Asn Thr Thr Thr Asn Glu Ile Phe Tyr Cys Thr
                85                  90                  95
Phe Arg Arg Leu Asp Pro Glu Glu Asn His Thr Ala Glu Leu Val Ile
            100                 105                 110
Pro Glu Leu Pro Leu Ala His Pro Pro Asn Glu Arg Thr His Leu Val
        115                 120                 125
Ile Leu Gly Ala Ile Leu Leu Cys Leu Gly Val Ala Leu Thr Phe Ile
    130                 135                 140
Phe Arg Leu Arg Lys Gly Arg Met Met Asp Val Lys Lys Cys Gly Ile
145                 150                 155                 160
Gln Asp Thr Asn Ser Lys Lys Gln Ser Asp Thr His Leu Glu Glu Thr
                165                 170                 175
```

<210> 48
<211> 3518
<212> DNA
<213> Homo sapiens

<220>
<221> source
<222> 1..3518
<223> /mol_type="unassigned DNA"
/organism="Homo sapiens"

<400> 48

```
ggcgcaacgc tgagcagctg gcgcgtcccg cgcggcccca gttctgcgca gcttcccgag      60
gctccgcacc agccgcgctt ctgtccgcct gcagggcatt ccagaaagat gaggatattt     120
gctgtcttta tattcatgac ctactggcat ttgctgaacg catttactgt cacggttccc     180
aaggacctat atgtggtaga gtatggtagc aatatgacaa ttgaatgcaa attcccagta     240
gaaaaacaat tagacctggc tgcactaatt gtctattggg aaatggagga taagaacatt     300
attcaatttg tgcatggaga ggaagacctg aaggttcagc atagtagcta cagacagagg     360
gcccggctgt tgaaggacca gctctccctg ggaaatgctg cacttcagat cacagatgtg     420
aaattgcagg atgcaggggt gtaccgctgc atgatcagct atggtggtgc cgactacaag     480
cgaattactg tgaaagtcaa tgccccatac aacaaaatca accaaagaat tttggttgtg     540
gatccagtca cctctgaaca tgaactgaca tgtcaggctg agggctaccc caaggccgaa     600
gtcatctgga caagcagtga ccatcaagtc ctgagtggag attagatcct gaggaaaacc     660
atacagctga attggtcatc ccagaactac ctctggcaca tcctccaaat gaaaggactc     720
acttgggaga atgatggatg tgaaaaaatg tggcatccaa gatacaaact caaagaagca     780
aagtgataca catttggagg agacgtaatc cagcattgga acttctgatc ttcaagcagg     840
gattctcaac ctgtggttta ggggttcatc ggggctgagc gtgacaagag gaaggaatgg     900
```

gcccgtggga tgcaggcaat gtgggactta aaaggcccaa gcactgaaaa tggaacctgg 960
cgaaagcaga ggaggagaat gaagaaagat ggagtcaaac agggagcctg gagggagacc 1020
ttgatacttt caaatgcctg aggggctcat cgacgcctgt gacagggaga aaggatactt 1080
ctgaacaagg agcctccaag caaatcatcc attgctcatc ctaggaagac gggttgagaa 1140
tccctaattt gagggtcagt tcctgcagaa gtgccctttg cctccactca atgcctcaat 1200
ttgttttctg catgactgag agtctcagtg ttggaacggg acagtattta tgtatgagtt 1260
tttcctattt attttgagtc tgtgaggtct tcttgtcatg tgagtgtggt tgtgaatgat 1320
ttcttttgaa gatatattgt agtagatgtt acaattttgt cgccaaacta aacttgctgc 1380
ttaatgattt gctcacatct agtaaaacat ggagtatttg taaggtgctt ggtctcctct 1440
ataactacaa gtatacattg gaagcataaa gatcaaaccg ttggttgcat aggatgtcac 1500
ctttatttaa cccattaata ctctggttga cctaatctta ttctcagacc tcaagtgtct 1560
gtgcagtatc tgttccattt aaatatcagc tttacaatta tgtggtagcc tacacacata 1620
atctcatttc atcgctgtaa ccaccctgtt gtgataacca ctattatttt acccatcgta 1680
cagctgagga agcaaacaga ttaagtaact tgcccaaacc agtaaatagc agacctcaga 1740
ctgccaccca ctgtcctttt ataatacaat ttacagctat attttacttt aagcaattct 1800
tttattcaaa aaccatttat taagtgccct tgcaatatca atcgctgtgc caggcattga 1860
atctacagat gtgagcaaga caaagtacct gtcctcaagg agctcatagt ataatgagga 1920
gattaacaag aaaatgtatt attacaattt agtccagtgt catagcataa ggatgatgcg 1980
aggggaaaac ccgagcagtg ttgccaagag gaggaaatag gccaatgtgg tctgggacgg 2040
ttggatatac ttaaacatct taataatcag agtaattttc atttacaaag agaggtcggt 2100
acttaaaata accctgaaaa ataacactgg aattcctttt ctagcattat atttattcct 2160
gatttgcctt tgccatataa tctaatgctt gtttatatag tgtctggtat tgtttaacag 2220
ttctgtcttt tctatttaaa tgccactaaa ttttaaattc ataccttttcc atgattcaaa 2280
attcaaaaga tcccatggga gatggttgga aaatctccac ttcatcctcc aagccattca 2340
agtttccttt ccagaagcaa ctgctactgc ctttcattca tatgttcttc taaagatagt 2400
ctacatttgg aaatgtatgt taaaagcacg tatttttaaa atttttttcc taaatagtaa 2460
cacattgtat gtctgctgtg tactttgcta tttttattta ttttagtgtt tcttatatag 2520
cagatggaat gaatttgaag ttcccagggc tgaggatcca tgccttcttt gtttctaagt 2580
tatctttccc atagcttttc attatctttc atatgatcca gtatatgtta aatatgtcct 2640
acatatacat ttagacaacc accatttgtt aagtatttgc tctaggacag agtttggatt 2700
tgtttatgtt tgctcaaaag gagacccatg ggctctccag ggtgcactga gtcaatctag 2760

tcctaaaaag caatcttatt attaactctg tatgacagaa tcatgtctgg aacttttgtt 2820

ttctgctttc tgtcaagtat aaacttcact ttgatgctgt acttgcaaaa tcacattttc 2880

tttctggaaa ttccggcagt gtaccttgac tgctagctac cctgtgccag aaaagcctca 2940

ttcgttgtgc ttgaaccctt gaatgccacc agctgtcatc actacacagc cctcctaaga 3000

ggcttcctgg aggtttcgag attcagatgc cctgggagat cccagagttt cctttccctc 3060

ttggccatat tctggtgtca atgacaagga gtaccttggc tttgccacat gtcaaggctg 3120

aagaaacagt gtctccaaca gagctccttg tgttatctgt ttgtacatgt gcatttgtac 3180

agtaattggt gtgacagtgt tctttgtgtg aattacaggc aagaattgtg gctgagcaag 3240

gcacatagtc tactcagtct attcctaagt cctaactcct ccttgtggtg ttggatttgt 3300

aaggcacttt atcccttttg tctcatgttt catcgtaaat ggcataggca gagatgatac 3360

ctaattctgc atttgattgt cactttttgt acctgcatta atttaataaa atattcttat 3420

ttattttgtt acttggtaca ccagcatgtc cattttcttg tttattttgt gtttaataaa 3480

atgttcagtt taacatccca gtggagaaag ttaaaaaa 3518

<210> 49
<211> 4972
<212> DNA
<213> Homo sapiens

<220>
<221> source
<222> 1..4972
<223> /mol_type="unassigned DNA"
/organism="Homo sapiens"

<400> 49
cacattgttc tgatcatctg aagatcagct attagaagag aaagatcagt taagtccttt 60

ggacctgatc agcttgatac aagaactact gatttcaact tctttggctt aattctctcg 120

gaaacgatga aatatacaag ttatatcttg gcttttcagc tctgcatcgt tttgggttct 180

cttggctgtt actgccagga cccatatgta aaagaagcag aaaaccttaa gaaatatttt 240

gtaagtatga ctttttaata gtacttgttt gtggttgaaa atgactgaat atcgacttgc 300

tgtagcatct ctgataggct gtcatctctt gtaggcagtc attttgagat ttggtgttat 360

tttgttaatt attgactaga tgagttcctt gactaaataa tctagatatt gttttaacct 420

tctgctcagt ttgtatagag acttaaaagg gatttatgaa ttttccaaaa gatgggcata 480

atatgggtat gaagcataat gatgttaata attttgtggt gggaactcat tcagttgtga 540

tagtcaagga gtatgcagat tgaaaaaaat gattggttat tagtttttga cttctcagac 600

tctaaggtca agattagcat taaaaaggta ataggaaatg tttacaaatt aaagtcaaaa 660

aggtccttaa agctttggct taaaaaaata actgataggt gattttctcc aaaaagtgat 720

ttcaacattc tgcttctcta tctatattac ttgtgaagta ttccggaact tcgttgctca 780
ctgggatttt ggaagaatta tgattctggc taaggaatgt ttaaaaattt taagtgaatt 840
ttttgagttt cttttaaaat tttattgatg gttaatgaaa agtttttaca ttttaaatat 900
ttcattattt gtttaaaact tagctgttat aattatagct gtcataataa tattcagaca 960
ttcacaattg attttattct tacaacacaa aatcaaatca cacacacaca cacacacaca 1020
cacacactcg cacatgtttg gaactatctt ttaaagctcg tataataata ccctacagga 1080
aggcacagta gatgtaatag aaacctgtac cattgggggg cagtatttta tagtggggtg 1140
gctttgctgt tttttgtttt tgtatttttt agcctagctt gaaaatactt tctttagctt 1200
actatagttt ttgggacctt tggagtatca gctttgttga gctcatttgt gacattgcaa 1260
tttaatggtt atattgggaa ataaaaaagc taaaagaaca taatagtctt tgtctatatc 1320
tcacataagc cttttgggaa tacttattgt tagaactaag cagaagagtt gaaaaggaaa 1380
tcagtgaata ttgtcacatc tgagttcaat gaaacttgaa atatattttt aaggcaattt 1440
atgggctaat tgtaaaccaa tttttctttt tttttttttt agaatgcagg tcattcagat 1500
gtagcggata atggaactct tttcttaggc attttgaaga attggaaaga ggtaagctga 1560
atattcccat ttggctaatt ttcctgttgc ttgctttctg atggataaat tcacatcatc 1620
ctctgtttgt gctctttcct tccaaggaga gtgacagaaa aataatgcag agccaaattg 1680
tctcctttta cttcaaactt tttaaaaact ttaaagatga ccagagcatc caaaagagtg 1740
tggagaccat caaggaagac atgaatgtca agtttttcaa tagcaacaaa aagaaacgag 1800
atgacttcga aaagctgact aattattcgg tgaggctatt taaattcttt ctttggtttc 1860
attgccgagg gtcttgcaaa gcatttattc tccagaaagt agacattagc tatttaacag 1920
ttgctaaagc tatgaactca actcatggct gaaactctac cttactattt ccattcgtgt 1980
ttgggtgact ttgcaaagcc agtaagagaa tcgctgaagt atgtaatgta gagaaatgct 2040
ggcattgtaa ctattgcgta aagacaggtg agttgacaaa tccagtgaag aggaagtagg 2100
tgaggaagaa gcgggggagta ctgagaagca gttctctcat tgtcccttgc tcatatgatg 2160
gaaattctct tactttgaat gagaggctgt ctgtcttaat ggaaagagca gtgggaggag 2220
ctgagaagat gtgtgttctc ctcccaactc agccaccaag gaactgtgat gaatcacatg 2280
gctggctggg gctcagtttc ctcatcttaa aaggaaactg ttaggctcac tgtataagtt 2340
tgatgacctt ctttgctcca aaactctaca atgcaaagaa tagaaaatga gaatgagata 2400
gaagaaagct acagtctttg aataggtacc agggacaccc cactgcaagt ctctagccaa 2460
cctatcagat tgtactgccc aattagaagc aagaatggtt gctgtttgtt tgtttttagg 2520
gaaaaataga tagaatttat accttatgaa aagattgttc tatcaactct ctatcaactt 2580
tcagaatatc tcagctggag aactccttag actcctaagt cttacctcat gaacttgtat 2640

ctttaagtta tggcttctat aaacagaaag ataacgttga ggcataaaga caaatcatgt 2700
ttttcaaaat gttttctaga agacaaaggc ctctagattc ctttggggtt gactttgata 2760
taaatgggct caaatgagag ggaccagggt cttcaagcta gcatttgtgt tcttaggata 2820
tgtgctcagc tttcactatt gctgggcctg cctctcactc ctctcatgta agcccccaga 2880
aacagaaagg agagacatgg caacaggtct cctttggtta taaactagac actcagcact 2940
tgtttctaat ccagtggtgc ccctggctta ctgttcagtc ctggataagt ctcttagttt 3000
cttggtgatg atttgaacat tggaaagtaa aatctgtcac ttgcaaacac acagcttgtc 3060
gaaaattttt tctactctgc aggaactggg ccttaaaaaa atgaaaaaaa atctgtggtt 3120
tcttccttct ggaagctaca aacctcctgt ttcttgatgg gcaatcttga gtgagctcta 3180
ttaattatta ttctctttgg ctcagttgct aagctatttt atgcatgtta tgccctttga 3240
caattagtct ttagctgtaa tcccccagcc atcctcagaa atgtggtgag tagccatagt 3300
gttcccaaga ttagaaaaaa tgtaatggca gagccaagag gaaggtaaat ggtccacatt 3360
ttatgaagca tcatctaaat ggccctattg gttagagtga ggagatgcaa gtagttcaat 3420
ttgcttgcct agaaggcagg gtactggaaa agttgttgca attcttaatt ttaaacttta 3480
tatatcagta agccatatat aaatatgatt gggggtgttt attttaaaat ctattatgga 3540
aattgagaga ctgacctaat ctgggagaaa ttaaaaatta cagttttcac tcgttttgga 3600
tttggtgttt tctagggtac ctaacctaga tcagtggttc tcaaacttag gtggatgtca 3660
gaatcacctg gggagcttag tgaatgcaca gggcacagtc cttccacttc atgcacctgg 3720
atctctgagg tctttgacag gtttccggat taatctgcta tgcacaacag tgagaatcat 3780
tgacctatag ttactcattt gatgcataca ggaaagactg aagtataaag tgatataatt 3840
ggtagattga tgatagagag gtcatagaaa cagtctcatc ctcctttaga tgagaaaata 3900
gaagttcaga gaggttaagt agctggctca aggtcagaat tattgcatgc atgagattca 3960
aacccacctt tttatgctga ctccacaacc aggagtcttt tcactatata atttcaagaa 4020
ttctatagaa gtagatttaa agatatgtga tggactccac cacattatag cacaactaga 4080
aatgtaattg taatttttag cttcaactgc tgaagaagta aatattgtat attaaggtaa 4140
tacggtccat tttttaaagg aatactttta ttttcactga ccatcatgac attagcagaa 4200
tatcctgatg gcttatatgc ctgaaattaa ttttgctctt ttctttcccg ataggtaact 4260
gacttgaatg tccaacgcaa agcaatacat gaactcatcc aagtgatggc tgaactgtcg 4320
ccagcagcta aaacagggaa gcgaaaaagg agtcagatgc tgtttcgagg tcgaagagca 4380
tcccagtaat ggttgtcctg cctgcaatat ttgaatttta aatctaaatc tatttattaa 4440
tatttaacat tatttatatg gggaatatat ttttagactc atcaatcaaa taagtattta 4500

taatagcaac ttttgtgtaa tgaaaatgaa tatctattaa tatatgtatt atttataatt 4560

cctatatcct gtgactgtct cacttaatcc tttgttttct gactaattag gcaaggctat 4620

gtgattacaa ggctttatct caggggccaa ctaggcagcc aacctaagca agatcccatg 4680

ggttgtgtgt ttatttcact tgatgataca atgaacactt ataagtgaag tgatactatc 4740

cagttactgc cggtttgaaa atatgcctgc aatctgagcc agtgctttaa tggcatgtca 4800

gacagaactt gaatgtgtca ggtgaccctg atgaaaacat agcatctcag gagatttcat 4860

gcctggtgct tccaaatatt gttgacaact gtgactgtac ccaaatggaa agtaactcat 4920

ttgttaaaat tatcaatatc taatatatat gaataaagtg taagttcaca ac 4972

<210> 50
<211> 1240
<212> DNA
<213> Homo sapiens

<220>
<221> source
<222> 1..1240
<223> /mol_type="unassigned DNA"
/organism="Homo sapiens"

<400> 50
cacattgttc tgatcatctg aagatcagct attagaagag aaagatcagt taagtccttt 60

ggacctgatc agcttgatac aagaactact gatttcaact tctttggctt aattctctcg 120

gaaacgatga aatatacaag ttatatcttg gcttttcagc tctgcatcgt tttgggttct 180

cttggctgtt actgccagga cccatatgta aaagaagcag aaaaccttaa gaaatatttt 240

aatgcaggtc attcagatgt agcggataat ggaactcttt tcttaggcat tttgaagaat 300

tggaaagagg agagtgacag aaaaataatg cagagccaaa ttgtctcctt ttacttcaaa 360

ctttttaaaa actttaaaga tgaccagagc atccaaaaga gtgtggagac catcaaggaa 420

gacatgaatg tcaagttttt caatagcaac aaaaagaaac gagatgactt cgaaaagctg 480

actaattatt cggtaactga cttgaatgtc caacgcaaag caatacatga actcatccaa 540

gtgatggctg aactgtcgcc agcagctaaa acagggaagc gaaaaaggag tcagatgctg 600

tttcgaggtc gaagagcatc ccagtaatgg ttgtcctgcc tgcaatattt gaattttaaa 660

tctaaatcta tttattaata tttaacatta tttatatggg gaatatattt ttagactcat 720

caatcaaata agtatttata atagcaactt ttgtgtaatg aaaatgaata tctattaata 780

tatgtattat ttataattcc tatatcctgt gactgtctca cttaatcctt tgttttctga 840

ctaattaggc aaggctatgt gattacaagg ctttatctca ggggccaact aggcagccaa 900

cctaagcaag atcccatggg ttgtgtgttt atttcacttg atgatacaat gaacacttat 960

aagtgaagtg atactatcca gttactgccg gtttgaaaat atgcctgcaa tctgagccag 1020

```
tgctttaatg gcatgtcaga cagaacttga atgtgtcagg tgaccctgat gaaaacatag    1080

catctcagga gatttcatgc ctggtgcttc caaatattgt tgacaactgt gactgtaccc    1140

aaatggaaag taactcattt gttaaaatta tcaatatcta atatatatga ataaagtgta    1200

agttcacaac aaaaaaaaaa aaaaaaaaaa aaaaaaaaaa                          1240
```

```
<210> 51
<211> 166
<212> PRT
<213> Homo sapiens


<400> 51
Met Lys Tyr Thr Ser Tyr Ile Leu Ala Phe Gln Leu Cys Ile Val Leu
1               5                   10                  15
Gly Ser Leu Gly Cys Tyr Cys Gln Asp Pro Tyr Val Lys Glu Ala Glu
            20                  25                  30
Asn Leu Lys Lys Tyr Phe Asn Ala Gly His Ser Asp Val Ala Asp Asn
        35                  40                  45
Gly Thr Leu Phe Leu Gly Ile Leu Lys Asn Trp Lys Glu Glu Ser Asp
    50                  55                  60
Arg Lys Ile Met Gln Ser Gln Ile Val Ser Phe Tyr Phe Lys Leu Phe
65                  70                  75                  80
Lys Asn Phe Lys Asp Asp Gln Ser Ile Gln Lys Ser Val Glu Thr Ile
                85                  90                  95
Lys Glu Asp Met Asn Val Lys Phe Phe Asn Ser Asn Lys Lys Lys Arg
            100                 105                 110
Asp Asp Phe Glu Lys Leu Thr Asn Tyr Ser Val Thr Asp Leu Asn Val
        115                 120                 125
Gln Arg Lys Ala Ile His Glu Leu Ile Gln Val Met Ala Glu Leu Ser
    130                 135                 140
Pro Ala Ala Lys Thr Gly Lys Arg Lys Arg Ser Gln Met Leu Phe Arg
145                 150                 155                 160
Gly Arg Arg Ala Ser Gln
                165
```

## Description

The present invention relates to means and methods for determining whether a patient is in need of a PD-L1 inhibitor cotherapy. A patient is determined to be in need of the PD-L1 inhibitor cotherapy if a low or absent ER expression level and an expression level of programmed death ligand 1 (PD-L1) that is increased in comparison to a control is measured in vitro in a sample from the patient. The patient is undergoing therapy comprising a modulator of the HER2/neu (ErbB2) signaling pathway (like Trastuzumab) and a chemotherapeutic agent (like dodetaxel) or such a therapy is contemplated for the patient. Also provided herein are means and methods for treating a cancer in a cancer patient for whom therapy comprising a modulator of the HER2/neu (ErbB2) signaling pathway (like Trastuzumab) and a chemotherapeutic agent (like dodetaxel) is contemplated, wherein the patient is to receive PD-L1 inhibitor cotherapy.

The HER family of receptor tyrosine kinases are important mediators of cell growth, differentiation and survival. The receptor family includes four distinct members including epidermal growth factor receptor (EGFR, ErbB1, or HER1), HER2 (ErbB2 or p185$^{neu}$), HER3 (ErbB3) and HER4 (ErbB4 or tyro2).

EGFR, encoded by the *erb*B1 gene, has been causally implicated in human malignancy. In particular, increased expression of EGFR has been observed in breast, bladder, lung, head, neck and stomach cancer as well as glioblastomas. Increased EGFR receptor expression is often associated with increased production of the EGFR ligand, transforming growth factor alpha (TGF-$\alpha$), by the same tumor cells resulting in receptor activation by an autocrine stimulatory pathway. Baselga and Mendelsohn Pharmac. Ther. 64:127-154 (1994). Monoclonal antibodies directed against the EGFR or its ligands, TGF-$\alpha$ and EGF, have been evaluated as therapeutic agents in the treatment of such malignancies. See, *e.g.,* Baselga and Mendelsohn., *supra;* Masui et al. Cancer Research 44:1002-1007 (1984); and Wu et al. J. Clin. Invest. 95:1897-1905 (1995). The second member of the HER family, p185$^{neu}$, was originally identified as the product of the transforming gene from neuroblastomas of chemically treated rats. The activated form of the *neu* proto-oncogene results from a point mutation (valine to glutamic acid) in the transmembrane region of the encoded protein. Amplification of the human homolog of *neu* is observed in breast and ovarian cancers and correlates with a poor prognosis (Slamon et al., Science, 235:177-182 (1987); Slamon et al., Science, 244:707-712 (1989); and US Pat No. 4,968,603). To date, no point mutation analogous to that in the *neu* proto-oncogene has been reported for human tumors. Overexpression of HER2 (frequently but not uniformly due to gene amplification) has also been observed in other carcinomas including carcinomas of the stomach, endometrium, salivary gland, lung, kidney, colon, thyroid, pancreas and bladder. See, among others, King et al., Science, 229:974 (1985); Yokota et al., Lancet: 1:765-767 (1986); Fukushige et al., Mol Cell Biol., 6:955-958 (1986); Guerin et al., Oncogene Res., 3:21-31 (1988); Cohen et al., Oncogene, 4:81-88 (1989); Yonemura et al., Cancer Res., 51:1034 (1991); Borst et al., Gynecol. Oncol., 38:364 (1990); Weiner et al., Cancer Res., 50:421-425 (1990); Kern et al., Cancer Res., 50:5184 (1990); Park et al., Cancer Res., 49:6605 (1989); Zhau et al., Mol. Carcinog., 3:254-257 (1990); Aasland et al. Br. J. Cancer 57:358-363 (1988); Williams et al. Pathobiology 59:46-52 (1991); and McCann et al., Cancer, 65:88-92 (1990). HER2 may be overexpressed in prostate cancer (Gu et al. Cancer Lett. 99:185-9 (1996); Ross et al. Hum. Pathol. 28:827-33 (1997); Ross et al. Cancer 79:2162-70 (1997); and Sadasivan et al. J. Urol. 150:126-31 (1993)).

Antibodies directed against the rat p185$^{neu}$ and human HER2 protein products have been described. Drebin and colleagues have raised antibodies against the rat *neu* gene product, p185$^{neu}$ See, for example, Drebin et al., Cell 41:695-706 (1985); Myers et al., Meth. Enzym. 198:277-290 (1991); and WOP94/22478. Drebin et al. Oncogene 2:273-277 (1988) report that mixtures of antibodies reactive with two distinct regions of p185$^{neu}$ result in synergistic antitumor effects on *neu*-transformed NIH-3T3 cells implanted into nude mice. See also U.S. Patent 5,824,311 issued October 20, 1998.

Hudziak et al., Mol. Cell. Biol. 9(3):1165-1172 (1989) describe the generation of a panel of HER2 antibodies which were characterized using the human breast tumor cell line SK-BR-3. Relative cell proliferation of the SK-BR-3 cells following exposure to the antibodies was determined by crystal violet staining of the monolayers after 72 hours. Using this assay, maximum inhibition was obtained with the antibody called 4D5 which inhibited cellular proliferation by 56%. Other antibodies in the panel reduced cellular proliferation to a lesser extent in this assay. The antibody 4D5 was further found to sensitize HER2-overexpressing breast tumor cell lines to the cytotoxic effects of TNF-$\alpha$. See also U.S. Patent No. 5,677,171 issued October 14, 1997. The HER2 antibodies discussed in Hudziak *et al.* are further characterized in Fendly et al. Cancer Research 50:1550-1558 (1990); Kotts et al. In Vitro 26(3):59A (1990); Sarup et al. Growth Regulation 1:72-82 (1991); Shepard et al. J. Clin. Immunol. 11(3):117-127 (1991); Kumar et al. Mol. Cell. Biol. 11(2):979-986 (1991); Lewis et al. Cancer Immunol. Immunother. 37:255-263 (1993); Pietras et al. Oncogene 9:1829-1838 (1994); Vitetta et al. Cancer Research 54:5301-5309 (1994); Sliwkowski et al. J. Biol. Chem. 269(20):14661-14665 (1994); Scott et al. J. Biol. Chem. 266:14300-5 (1991); D'souza et al. Proc. Natl. Acad. Sci. 91:7202-7206 (1994); Lewis et al. Cancer Research 56:1457-1465 (1996); and Schaefer et al. Oncogene 15:1385-1394 (1997).

A recombinant humanized version of the murine HER2 antibody 4D5 (huMAb4D5-8, rhuMAb HER2, Trastuzumab or Herceptin™ ; U.S. Patent No. 5,821,337) is clinically active in patients with HER2-overexpressing metastatic breast cancers that have received extensive prior anti-cancer therapy (Baselga et al., J. Clin. Oncol. 14:737-744 (1996)). Trastuzumab received marketing approval from the Food and Drug Administration September 25, 1998 for the treatment of patients with metastatic breast cancer whose tumors overexpress the HER2 protein.

Humanized anti-ErbB2 antibodies include huMAb4D5-1, huMAb4D5-2, huMAb4D5-3, huMAb4D5-4, huMAb4D5-5, huMAb4D5-6, huMAb4D5-7 and huMAb4D5-8 (HERCEPTIN®) as described in Table 3 of US Patent 5,821,337 expressly incorporated herein by reference; humanized 520C9 (WO 93/21319) and humanized 2C4 antibodies as described in WO 01/000245 expressly incorporated herein by reference.

Pertuzumab (see e.g. WO 01/000245) is the first of a new class of agents known as HER dimerization inhibitors (HDIs). Pertuzumab binds to HER2 at its dimerization domain, thereby inhibiting its ability to form active dimer receptor complexes and thus blocking the downstream signal cascade that ultimately results in cell growth and division (see Franklin, M.C., Cancer Cell 5 (2004) 317-328). Pertuzumab is a fully humanized recombinant monoclonal antibody directed against the extracellular domain of HER2. Binding of Pertuzumab to the HER2 on human epithelial cells prevents HER2 from forming complexes with other members of the HER family (including EGFR, HER3, HER4) and probably also HER2 homodimerization. By blocking complex formation, Pertuzumab prevents the growth stimulatory effects and cell survival signals activated by ligands of HER1, HER3 and HER4 (e.g. EGF, TGFalpha, amphiregulin, and the heregulins). Another name for Pertuzumab is 2C4. Pertuzumab is a fully humanized recombinant monoclonal antibody based on the human IgG1(K) framework sequences. The structure of Pertuzumab consists of two heavy chains (449 residues) and two light chains (214 residues). Compared to Trastuzumab (Herceptin®), Pertuzumab has 12 amino acid differences in the light chain and 29 amino acid differences in the IgG1 heavy chain.

Other HER2 antibodies with various properties have been described in Tagliabue et al. Int. J. Cancer 47:933-937 (1991); McKenzie et al. Oncogene 4:543-548 (1989); Maier et al. Cancer Res. 51:5361-5369 (1991); Bacus et al. Molecular Carcinogenesis 3:350-362 (1990); Stancovski et al. PNAS (USA) 88:8691-8695 (1991); Bacus et al. Cancer Research 52:2580-2589 (1992); Xu et al. Int. J. Cancer 53:401-408 (1993); WO094/00136; Kasprzyk et al. Cancer Research 52:2771-2776 (1992);Hancock et al. Cancer Res. 51:4575-4580 (1991); Shawver et al. Cancer Res. 54:1367-1373 (1994); Arteaga et al. Cancer Res. 54:3758-3765 (1994); Harwerth et al. J. Biol. Chem. 267:15160-15167 (1992); U.S. Patent No. 5,783,186; and Klapper et al. Oncogene 14:2099-2109 (1997).

Homology screening has resulted in the identification of two other HER receptor family members; HER3 (US Pat. Nos. 5,183,884 and 5,480,968 as well as Kraus et al. PNAS (USA) 86:9193-9197 (1989)) and HER4 (EP Pat. Appln. No 599,274; Plowman et al., Proc. Natl. Acad. Sci. USA, 90:1746-1750 (1993); and Plowman et al., Nature, 366:473-475 (1993)). Both of these receptors display increased expression on at least some breast cancer cell lines.

The HER receptors are generally found in various combinations in cells and heterodimerization is thought to increase the diversity of cellular responses to a variety of HER ligands (Earp et al. Breast Cancer Research and Treatment 35: 115-132 (1995)). EGFR is bound by six different ligands; epidermal growth factor (EGF), transforming growth factor alpha (TGF-a), amphiregulin, heparin binding epidermal growth factor (HB-EGF), betacellulin and epiregulin (Groenen et al. Growth Factors 11:235-257 (1994)). A family of heregulin proteins resulting from alternative splicing of a single gene are ligands for HER3 and HER4. The heregulin family includes alpha, beta and gamma heregulins (Holmes et al., Science, 256:1205-1210 (1992); U.S. Patent No. 5,641,869; and Schaefer et al. Oncogene 15:1385-1394 (1997)); neu differentiation factors (NDFs), glial growth factors (GGFs); acetylcholine receptor inducing activity (ARIA); and sensory and motor neuron derived factor (SMDF). For a review, see Groenen et al. Growth Factors 11:235-257 (1994); Lemke, G. Molec. & Cell. Neurosci. 7:247-262 (1996) and Lee et al. Pharm. Rev. 47:51-85 (1995). Recently three additional HER ligands were identified; neuregulin-2 (NRG-2) which is reported to bind either HER3 or HER4 (Chang et al. Nature 387 509-512 (1997); and Carraway et al Nature 387:512-516 (1997)); neuregulin-3 which binds HER4 (Zhang et al. PNAS (USA) 94(18):9562-7 (1997)); and neuregulin-4 which binds HER4 (Harari et al. Oncogene 18:2681-89 (1999)) HB-EGF, betacellulin and epiregulin also bind to HER4.

While EGF and TGF$\alpha$ do not bind HER2, EGF stimulates EGFR and HER2 to form a heterodimer, which activates EGFR and results in transphosphorylation of HER2 in the heterodimer. Dimerization and/or transphosphorylation appears to activate the HER2 tyrosine kinase. See Earp *et al., supra.* Likewise, when HER3 is co-expressed with HER2, an active signaling complex is formed and antibodies directed against HER2 are capable of disrupting this complex (Sliwkowski et al., J. Biol. Chem., 269(20):14661-14665 (1994)). Additionally, the affinity of HER3 for heregulin (HRG) is increased to a higher affinity state when co-expressed with HER2. See also, Levi et al., Journal of Neuroscience 15: 1329-1340 (1995); Morrissey et al., Proc. Natl. Acad. Sci. USA 92: 1431-1435 (1995); and Lewis et al., Cancer Res., 56:1457-1465 (1996) with respect to the HER2-HER3 protein complex. HER4, like HER3, forms an active signaling complex with HER2 (Carraway and Cantley, Cell 78:5-8 (1994)).

Also antibody variant compositions are described in the art. US Patent No. 6,339,142 describes a HER2 antibody composition comprising a mixture of anti-HER2 antibody and one or more acidic variants thereof, wherein the amount of the acidic variant(s) is less than about 25%. Trastuzumab is the exemplified HER2 antibody. Reid et al. Poster presented at Well Characterized Biotech Pharmaceuticals conference (January, 2003) "Effects of Cell Culture Process Changes on Humanized Antibody Characteristics" describes an unnamed, humanized IgG1 antibody composition with N-terminal heterogeneities due to combinations of VHS signal peptide, N-terminal glutamine, and pyroglutamic acid on the heavy chain thereof. Harris et al. "The Ideal Chromatographic Antibody Characterization Method" talk presented at the IBC Antibody Production Conference (February, 2002) reports a VHS extension on the heavy chain of E25, a humanized anti-IgE antibody. Rouse et al. Poster presented at WCBP "Glycoprotein Characterization by High Resolution Mass Spectrometry and Its Application to Biopharmaceutical Development" (January 6-9, 2004) describes a monoclonal antibody composition with N-terminal heterogeneity resulting from AHS or HS signal peptide residues on the light chain thereof. In a presentation at IBC Meeting (September, 2000) "Strategic Use of Comparability Studies and Assays for Well Characterized Biologicals," Jill Porter discussed a late-eluting form of ZENAPAX™ with three extra amino acid residues on the heavy chain thereof. US2006/0018899 describes a composition comprising a main species pertuzumab antibody and an amino-terminal leader extension variant, as well as other variant forms of the pertuzumab antibody.

Patent publications related to HER antibodies include: US 5,677,171, US 5,720,937, US 5,720,954, US 5,725,856, US 5,770,195, US 5,772,997, US 6,165,464, US 6,387,371, US 6,399,063, US2002/0192211A1, US 6,015,567, US 6,333,169, US 4,968,603, US 5,821,337, US 6,054,297, US 6,407,213, US 6,719,971, US 6,800,738, US2004/0236078A1, US 5,648,237, US 6,267,958, US 6,685,940, US 6,821,515, WO98/17797, US 6,127,526, US 6,333,398, US 6,797,814, US 6,339,142, US 6,417,335, US 6,489,447, WO99/31140, US2003/0147884A1, US2003/0170234A1, US2005/0002928A1, US 6,573,043, US2003/0152987A1, WO99/48527, US2002/0141993A1, WO01/00245, US2003/0086924, US2004/0013667A1, WO00/69460, WO01/00238, WO01/15730, US 6,627,196B1, US6,632,979B1, WO01/00244, US2002/0090662A1, WO01/89566, US2002/0064785, US2003/0134344, WO 04/24866, US2004/0082047, US2003/0175845A1, WO03/087131, US2003/0228663, WO2004/008099A2, US2004/0106161, WO2004/048525, US2004/0258685A1, US 5,985,553, US 5,747,261, US 4,935,341, US 5,401,638, US 5,604,107, WO 87/07646, WO 89/10412, WO 91/05264, EP 412,116 B1, EP 494,135 B1, US 5,824,311, EP 444,181 B1, EP 1,006,194 A2, US 2002/0155527A1, WO 91/02062, US 5,571,894, US 5,939,531, EP 502,812 B1, WO 93/03741, EP 554,441 B1, EP 656,367 A1, US 5,288,477, US 5,514,554, US 5,587,458, WO 93/12220, WO 93/16185, US 5,877,305, WO 93/21319, WO 93/21232, US 5,856,089, WO 94/22478, US 5,910,486, US 6,028,059, WO 96/07321, US 5,804,396, US 5,846,749, EP 711,565, WO 96/16673, US 5,783,404, US 5,977,322, US 6,512,097, WO 97/00271, US 6,270,765, US 6,395,272, US 5,837,243, WO 96/40789, US 5,783,186, US 6,458,356, WO 97/20858, WO 97/38731, US 6,214,388, US 5,925,519, WO 98/02463, US 5,922,845, WO 98/18489, WO 98/33914, US 5,994,071, WO 98/45479, US 6,358,682 B1, US 2003/0059790, WO 99/55367, WO 01/20033, US 2002/0076695 A1, WO 00/78347, WO 01/09187, WO 01/21192, WO 01/32155, WO 01/53354, WO 01/56604, WO 01/76630, WO02/05791, WO 02/11677, US 6,582,919, US2002/0192652A1, US 2003/0211530A1, WO 02/44413, US 2002/0142328, US 6,602,670 B2, WO 02/45653, WO 02/055106, US 2003/0152572, US 2003/0165840, WO 02/087619, WO 03/006509, WO03/012072, WO 03/028638, US 2003/0068318, WO 03/041736, EP 1,357,132, US 2003/0202973, US 2004/0138160, US 5,705,157, US 6,123,939, EP 616,812 B1, US 2003/0103973, US 2003/0108545, US 6,403,630 B1, WO 00/61145, WO 00/61185, US 6,333,348 B1, WO 01/05425, WO 01/64246, US 2003/0022918, US 2002/0051785 A1, US 6,767,541, WO 01/76586, US 2003/0144252, WO 01/87336, US 2002/0031515 A1, WO 01/87334, WO 02/05791, WO 02/09754, US 2003/0157097, US 2002/0076408, WO 02/055106, WO 02/070008, WO 02/089842 and WO 03/86467.

Patients treated with the HER2 antibody Trastuzumab/Herceptin™ are selected for therapy based on HER2 protein overexpression/ gene amplification; see, for example, WO99/31140 (Paton et al.), US2003/0170234A1 (Hellmann, S.), and US2003/0147884 (Paton et al.); as well as WO01/89566, US2002/0064785, and US2003/0134344 (Mass et al.). See, also, US2003/0152987, Cohen et al., concerning immunohistochemistry (IHC) and fluorescence in situ hybridization (FISH) for detecting HER2 overexpression and amplification. WO2004/053497 and US2004/024815A1 (Bacus et al.), as well as US 2003/0190689 (Crosby and Smith), refer to determining or predicting response to Trastuzumab therapy. US2004/013297A1 (Bacus et al.) concerns determining or predicting response to ABX0303 EGFR antibody therapy. WO2004/000094 (Bacus et al.) is directed to determining response to GW572016, a small molecule, EGFR-HER2 tyrosine kinase inhibitor. WO2004/063709, Amler et al., refers to biomarkers and methods for determining sensitivity to EGFR inhibitor, erlotinib HC1. US2004/0209290, Cobleigh et al., concerns gene expression markers for breast cancer prognosis.

Patients to be treated with a HER2 dimerization inhibitor (like pertuzumab as described herein above in more detail) can be selected for therapy based on HER activation or dimerization.

Patent publications concerning pertuzumab and selection of patients for therapy therewith include: WO01/00245 (Adams et al.); US2003/0086924 (Sliwkowski, M.); US2004/0013667A1 (Sliwkowski, M.); as well as WO2004/008099A2, and US2004/0106161(Bossenmaier et al.).

Herceptin™/Trastuzumab is indicated in the art for the treatment of patients with metastatic breast cancer whose tumors overexpress HER2 protein or have HER 2 gene amplification:

a) As monotherapy for the treatment of those patients who have received at least two chemotherapy regimens for their metastatic disease. Prior chemotherapy must have included at least an anthracycline and a taxane unless patients are unsuitable for these treatments. Hormone receptor positive patients must also have received hormonal therapy, unless patients are unsuitable for these treatments,

b) In combination with paclitaxel for the treatment of those patients who have not received chemotherapy for their metastatic disease and for whom an anthracycline is not suitable and

c) In combination with docetaxel for the treatment of those patients who have not received chemotherapy for their metastatic disease.

HerceptinTM/Trastuzumab can also be used as adjuvant treatment in early breast cancer. HerceptinTM/ Trastuzumab is also approved for the treatment of patients with HER2-positive early breast cancer following surgery, chemotherapy (neoadjuvant (i.e. before surgery) or adjuvant), and radiotherapy (if applicable). In addition Herceptin in combination with capecitabine or 5-fluorouracil and cisplatin is indicated for the treatment of patients with HER2 positive locally advance or metastatic adenocarcinoma of the stomach or gastroesophageal junction who have not received prior anti-cancer treatment for their metastatic disease. The efficacy and safety of neoadjuvant pertuzumab and trastuzumab therapy has been assessed in a phase 2 trial (NEOSPHERE); Gianni (2012) Lancet Oncol 13, 25-32.

In the art, the treatment of breast cancer patients with Herceptin™/Trastuzumab is, for example, recommended and routine for patients having HER2-positive cancer. HER2-positive cancer is present if a high HER2 (protein) expression level detected by immunohistochemical methods (e.g. HER2 (+++)) or HER2 gene amplification detected by in-situ-hybridization (e.g. ISH positive, like a HER2 gene copy number higher than 4 copies of the HER2 gene per tumor cell or ratio of $\geq 2.0$ for the number of HER2 gene copies to the number of signals for CEP17.) or both is found in samples obtained from the patients such as breast tissue biopsies or breast tissue resections or in tissue derived from metastatic sites.

WO 2011/109789, WO 2011/066342, WO 2009/089149 and WO2006/133396 disclose the therapeutic use of PD-L1 inhibitors. Moreover, WO 2010/077634 discloses anti-PD-L1 antibodies and their therapeutic use.

The present invention relates to a method of determining the need of a cancer patient for a PD-L1 inhibitor cotherapy, (i) wherein therapy comprising a modulator of the HER2/neu (ErbB2) signaling pathway and a chemotherapeutic agent is contemplated for the patient or (ii) wherein the patient is undergoing therapy comprising a modulator of the HER2/neu (ErbB2) signaling pathway and a chemotherapeutic agent, said method comprising the steps of

a) measuring in vitro in a sample from said patient the expression level of Estrogen receptor (ER) and of programmed death ligand 1 (PD-L1),

b) determining a patient as being in need of a PD-L1 inhibitor cotherapy if a low or absent ER expression level and an expression level of programmed death ligand 1 (PD-L1) that is increased in comparison to a control is measured in step (a).

Accordingly, the present invention provides a method for determining a cancer patient's need for PD-L1 modulator cotherapy in combination with a modulator of the HER2/neu (ErbB2) signaling pathway and a chemotherapeutic agent, the method comprising the steps of

- testing a tumor sample of a patient for whom therapy comprising a modulator of the HER2/neu (ErbB2) signaling pathway and a chemotherapeutic agent is contemplated or who is undergoing said therapy;
- determining the expression level of Estrogen receptor (ER) and of programmed death ligand 1 (PD-L1) in said tumor sample,

whereby a low or absent ER expression level and an expression level of programmed death ligand 1 (PD-L1) that is increased in comparison to the control is indicative of a successful use of PD-L1 modulator cotherapy in said patient.

As demonstrated in the appended example, it has been surprisingly found in this invention that Estrogen receptor (ER) negative (ER(-)) cancer patients (cancer patients with a low or even absent ER expression level) undergoing therapy with a modulator of the HER2/neu (ErbB2) signaling pathway (like Herceptin™/Trastuzumab) and a chemotherapeutic agent (like dodetaxel/Taxotere®) show a significantly worse pathological complete response (pCR) to the therapy compared to Estrogen receptor (ER) positive (ER(+)) cancer patients, if the expression level of programmed death ligand 1 (PD-L1) is increased in a sample of the ER negative (ER(-)) cancer patients as compared to a control. The terms "programmed death ligand 1", "CD274" and "PD-L1" are used interchangeably herein. The ER negative (ER(-)) cancer patients with increased expression level of programmed death ligand 1 (PD-L1) as compared to a control will therefore benefit from additional cotherapy with a PD-L1 inhibitor. It is expected that the pathological complete response rate (pCR) in this patient group will increase, if these patients receive cotherapy with a PD-L1 inhibitor in addition to therapy with a modulator of the HER2/neu (ErbB2) signaling pathway (like Herceptin™/Trastuzumab) and a chemotherapeutic agent (like dodetaxel/Taxotere®). In other words, the ER negative (ER(-)) cancer patients are to receive a programmed death ligand 1 (PD-L1) inhibitor in addition to a modulator of the HER2/neu (ErbB2) signaling pathway (like Trastuzumab) and a chemotherapeutic agent (like dodetaxel/Taxotere®), if the expression level of programmed death ligand 1 (PD-L1) is increased in a sample from the patient in comparison to a control. In the following, ER negative cancer patients or (biological/tumor) samples derived from ER negative cancer patients are denoted herein as "ER(-)". Likewise ER positive cancer patients or (biological/tumor) samples derived from ER positive cancer patients are denoted herein as "ER(+)".

In accordance with the above, the present invention relates to a method of treating a cancer in a cancer patient for whom therapy comprising a modulator of the HER2/neu (ErbB2) signaling pathway and a chemotherapeutic agent is contemplated, the method comprising selecting a cancer patient whose cancer is determined to have a low or absent ER expression level and to have an increased expression level of programmed death ligand 1 (PD-L1) in comparison to a control, and administering to the patient an effective amount of a modulator of the HER2/neu (ErbB2) signaling pathway, of a chemotherapeutic agent and of a programmed death ligand 1 (PD-L1) inhibitor. Likewise, the present invention relates to a method of treating a cancer in a cancer patient who is undergoing therapy comprising a modulator of the HER2/neu (ErbB2) signaling pathway and a chemotherapeutic agent, the method comprising selecting a cancer patient whose cancer is determined to have a low or absent ER expression level and to have an increased expression level of programmed death ligand 1 (PD-L1) in comparison to a control, and administering to the patient an effective amount of a programmed death ligand 1 (PD-L1) inhibitor. Herein contemplated is, accordingly, a pharmaceutical composition comprising a modulator of the HER2/neu (ErbB2) signaling pathway, and an inhibitor of programmed death ligand 1 (PD-L1) for use in the treatment of cancer, whereby said cancer is determined to have a low or absent ER expression level and to have an increased expression level of programmed death ligand 1 (PD-L1) in comparison to a control.

In accordance with the above, the herein provided method for determining the need of a cancer patient for a PD-L1 inhibitor cotherapy, may comprise an additional step prior to step a), wherein said step is or comprises obtaining a sample from said cancer patient. Accordingly, the present invention provides a method of determining the need of a cancer patient for a PD-L1 inhibitor cotherapy, (i) wherein therapy comprising a modulator of the HER2/neu (ErbB2) signaling pathway and a chemotherapeutic agent is contemplated for the patient or (ii) wherein the patient is undergoing therapy comprising a modulator of the HER2/neu (ErbB2) signaling pathway and a chemotherapeutic agent, said method comprising a step of obtaining a sample from said cancer patient, the method further comprising the steps

a) measuring in vitro in a sample from said patient the expression level of Estrogen receptor (ER) and of programmed death ligand 1 (PD-L1),
b) determining a patient as being in need of a PD-L1 inhibitor cotherapy if a low or absent ER expression level and an expression level of programmed death ligand 1 (PD-L1) that is increased in comparison to a control is measured in step (a).

Furthermore, it has been found herein and is demonstrated in the appended example, that a patient's need of PD-L1 inhibitor cotherapy can be determined even more reliably, if the expression level of interferon-gamma (IFNγ) is measured in the sample of the patient in addition to the expression level of programmed death ligand 1 (PD-L1). It is shown herein that patients with low or absent ER expression have a significantly worse pathologic complete response to therapy with a modulator of the HER2/neu (ErbB2) signaling pathway and a chemotherapeutic agent, if the expression level of programmed death ligand 1 (PD-L1) is increased and if the expression level of interferon-gamma (IFNγ) is decreased.

Accordingly, the methods provided herein preferably further comprise measuring the expression level of interferon-gamma (IFNγ) in the sample from the patient, whereby a patient is determined to be in need of a PD-L1 inhibitor cotherapy, if the expression level of interferon-gamma (IFNγ) is decreased in comparison to a control. In accordance with the above, the present invention relates in a preferred aspect to a method of determining the need of a cancer patient for a PD-L1 inhibitor cotherapy, (i) wherein therapy comprising a modulator of the HER2/neu (ErbB2) signaling pathway and a chemotherapeutic agent is contemplated for the patient or (ii) wherein the patient is undergoing therapy comprising a modulator of the HER2/neu (ErbB2) signaling pathway and a chemotherapeutic agent, said method comprising the steps of

a) measuring in vitro in a sample from said patient the expression level of Estrogen receptor (ER), the expression level of programmed death ligand 1 (PD-L1), and the expression level of interferon-gamma (IFNγ)
b) determining a patient as being in need of a PD-L1 inhibitor cotherapy if a low or absent ER expression level, an expression level of programmed death ligand 1 (PD-L1) that is increased in comparison to a control, and an expression level of interferon-gamma (IFNγ) that is decreased in comparison to a control is measured in step (a).

Accordingly, an expression level of interferon-gamma (IFNγ) that is decreased in comparison to a control is indicative of a successful use of PD-L1 inhibitor cotherapy in said patient. The herein provided pharmaceutical composition is, in accordance with the above, for use in the treatment of cancer, whereby said cancer is determined to have a low or absent ER expression level, the cancer is determined to have an increased expression level of programmed death ligand 1 (PD-L1) in comparison to a control and the cancer is determined to have a decreased expression level of interferon-gamma (IFNγ) in comparison to the control. Accordingly, a pharmaceutical composition is provided herein comprising a modulator of the HER2/neu (ErbB2) signaling pathway, and an inhibitor of programmed death ligand 1 (PD-L1) for use in the treatment of cancer, whereby said cancer is determined to have a low or absent ER expression level and to have an increased expression level of programmed death ligand 1 (PD-L1) in comparison to a control and to have a decreased expression level of interferon-gamma (IFNγ) in comparison to the control.

The term "cancer patient" as used herein refers to a patient that is suspected to suffer from cancer, suffering from cancer or being prone to suffer from cancer. The cancer to be treated in accordance with the present invention can be a solid cancer, such as breast cancer or gastric cancer. Further, the cancer may be ovarian cancer or colorectal cancer. The cancer is preferably a "HER2-positive" cancer.

Preferably, the cancer is breast cancer, like early breast cancer. The breast cancer may be early stage breast cancer or metastatic breast cancer. Accordingly, the cancer patient (to be treated) is suspected to suffer from solid cancer, is suffering from solid cancer or is being prone to suffer from solid cancer, whereby the solid cancer can be breast cancer or gastric cancer. Preferably, the cancer is breast cancer, like early stage breast cancer. The patient is preferably a human.

As mentioned above, the expression level of Estrogen receptor (ER) and of programmed death ligand (PD-L1), and optionally of interferon-gamma (IFN-γ) can be measured in vitro in a sample from the patient. Preferably, the herein provided methods comprise measuring of interferon-gamma (IFN-γ) in vitro in a sample from the patient. Preferably, the sample to be assessed/analyzed herein is a tumor tissue sample. A patient (or a patient group) is determined as being in need of a PD-L1 inhibitor cotherapy if a low or absent ER expression level and an expression level of programmed death ligand 1 (PD-L1) that is increased in comparison to a control and, optionally, an expression level of interferon-gamma (IFNγ) that is decreased in comparison to the control, is measured in vitro in said sample.

The term "ER" is an abreviation of "Estrogen receptor". Likewise, the terms "PD-L1" and "IFN-γ" are abreviations of the terms "programmed death ligand" and "interferon-gamma", respectively. Accordingly, the term "ER" can be used interchangeably herein with "Estrogen receptor". Likewise, the terms "PD-L1" and "IFN-γ" can be used interchangeably herein with the terms "programmed death ligand" and "interferon-gamma", respectively.

Preferably, the (tumor/biological) sample of the patient and/or the cancer to be treated is characterized by or associated with a low or absent estrogen receptor (ER) expression level. Preferably, the sample of the patient is a tumor sample. The ER expression level can be ER negative (ER(-)). The term "ER(-)" can be used herein interchangeably with the term "ER negative".

"ER negative" expression level can be determined by routine and standard procedures as described, for example, in the Guideline on Hormone Receptor Testing in Breast Cancer S. Nofech-Mozes, E. Vella, S. Dhesy-Thind, and W. Hanna (A Quality Initiative of the Program in Evidence-Based Care (PEBC), Cancer Care Ontario (CCO); Report Date: April 8, 2011). The Guidelines (and references cited therein) are incorporated by reference in its entirety herein. These Guidelines are available at world wide web at
cancercare.on.ca) and
PEBC Pathology & Laboratory Medicine page at:
https://www.cancercare.on.ca/toolbox/ qualityguidelines/clin-program/pathlabebs/

Routine and standard procedures for determining the "ER negative" expression level are described in these Guideline and also in the following references:

Nofech-Mozes S, Vella ET, Dhesy-Thind S, Hagerty KL, Mangu PB, Temin S, et al. Systematic review on hormone receptor testing in breast cancer. Applied Immunohistochem Mol Morphol. 2012 May;20(3):214-63. doi: 10.1097/PAI.0b013e318234aa12. Epub 2011 Nov 11.

Nofech-Mozes S, Vella ET, Dhesy-Thind S, Hanna WM. Cancer Care Ontario guideline recommendations for hormone receptor testing in breast cancer. Clin Oncol (R Coll Radiol). Epub 2012 May 17.

"ER negative" expression may be determined by IHC (immunohistochemistry), if, for example the expression level of ER is low or absent and/or if the progesterone receptor (PR) expression level is low or absent. The abbreviation "PR" is used herein interchangeably with the term "progesterone receptor". A sample or patients may be assessed as "ER negative" herein according to the following staining pattern (by IHC):
Only nuclear (not cytoplasmic) staining should be scored.

There are three categories for staining:

Positive: ≥10% staining for ER or PR
Low positive: 1% to 9% staining for ER or PR
Negative: < 1% staining for ER and PR

Accordingly, a sample or patients may particularly be assessed as "ER negative" herein if the sample shows the following staining pattern by IHC: < 1% staining for ER and PR.

Samples or patients may be assessed as "ER positive" herein if the sample shows a "positive" staining by IHC: ≥1% staining for ER or PR (i.e. more than 1% of the cells examined/assessed have estrogen receptors or progesterone receptors/show staining for estrogen receptors by IHC (immunohistochemistry).

Preferably, a sample or patients is assessed as "ER negative" herein if the sample shows the following staining pattern by IHC: : < 1% staining for ER (i.e. less than 1% of the cells examined/assessed have estrogen receptors/show staining for estrogen receptor(s) by IHC (immunohistochemistry). Most preferably, a sample or patients is/are assessed as "ER negative" if the nuclei in a tumor tissue sample show < 1% staining for ER staining by IHC. Accordingly, from the three categories provided herein above, the assessment of "ER negative" is based on < 1% staining for ER by IHC.

Likewise, "ER negative" expression can be determined by further methods routinely employed in the art. For example, "ER negative" may be determined if the mRNA/RNA expression level is low or absent. Routine methods to be used comprise, but are not limited to: Allred score, IRS, Remmele score or any other suitable biochemical detection method. A person skilled in the art is aware that the cut-off for such methods has to match the cut-off as defined above via IHC. Nucleic acid sequences and amino acid sequences of Progesterone receptor (PR), Estrogen receptor (ER), of programmed death ligand 1 (PD-L1), and/or of interferon-gamma (IFNγ) to be used herein are well known and can be retrieved from databases like NCBI. Examplary sequences are provided herein (see for example SEQ ID NO: 38-51).

The methods and sample types used for establishing a cut-off value of a marker (like programmed death ligand 1 (PD-L1) and/or interferon-gamma (IFN-γ)) and for measuring the sample obtained from an individual or patient to be analyzed match each other or are the same. Cut-off values, i.e. values above which overexpression (e.g. increased expression of programmed death ligand 1 (PD-L1) in comparison to a control) is acknowledged can be obtained in a control group. Cut-off values, i.e. values below which decreased expression (e.g. decreased expression of interferon-gamma (IFN-γ) in comparison to a control) is acknowledged can be obtained in a control group.

The control group on which the cut-off value is based is chosen to match the group of individuals/patients under investigation, with other words, if the method of the present invention is used to determine the need for PD-L1 cotherapy in patients with breast cancer or gastric cancer, respectively, the control group is also patients with breast cancer or gastric cancer, respectively. The control group used to establish the cut-off values for both, PDL-1 and IFN-γ, respectively), comprises at least 40, or at least 50, or at least 100 individuals/patients. An expression level or corresponding value above the cut-off is considered to represent overexpression and a value at or below the cut-off is considered as decreased expression.

In one embodiment, the "IFN-γ" expression level in a tumor tissue sample from an individual/patient is compared to a cut-off value. A value above the cut-off is considered to represent overexpression of IFN-γ and a value at or below the cut-off is considered as decreased expression of IFN-γ. In one embodiment the decreased expression is acknowledged if the expression level for IFN-γ is at or below the value of the highest quintile, quartile or tertile, respectively, as established in the control group. In one embodiment the cut-off for IFN-γ is the highest tertile. In one embodiment the cut-off value is a value between the $70^{th}$ and the $80^{th}$ percentile. In one embodiment the cut-off value for IFN-γ is the $73^{rd}$ percentile, i.e a value above this cut-off is considered to represent overexpression of IFN-γ and a value at or below the $73^{rd}$ percentile is considered as decreased expression of IFN-γ. In one embodiment, individuals/patients are determined as being in need of a PD-L1 cotherapy, if IFN-γ expression in a sample (like a tumor tissue sample) is decreased (i.e. below or at the IFN-γ cut-off value) In one embodiment individuals/patients are determined as not being in need of a PDL-1 cotherapy, if IFN-γ is overexpressed (i.e. above the IFN-γ cut-off value as described above).

In one embodiment the PD-L1 expression level, in a tumor tissue sample from an individual/patient is compared to a cut-off value. A value above the cut-off is considered to represent overexpression of PD-L1 and a value at or below the cut-off is considered as decreased expression of PD-L1.In one embodiment overexpression for PDL-1 is acknowledged if the expression level for PDL-1 is above a cut-off value between the $50^{th}$ percentile and the $75^{th}$ percentile, as established in a control group. In one embodiment overexpression for PDL-1 is acknowledged if the expression level for PDL-1 is above a cut-off value between the $50^{th}$ percentile and the $70^{th}$ percentile, of the control group. In one embodiment individuals/patients are determined as being in need of a PDL-1 cotherapy, if PDL-1 is overexpressed (i.e. the PDL-1 expression level determined is above the PDL-1 cut-off value).

In one further embodiment overexpression for PDL-1 is established in the sub-group of individuals/patients having a decreased expression level of IFN-γ in a tumor tissue sample. In one embodiment overexpression for PDL-1 is acknowledged if the expression level for PDL-1 is above a cut-off value between the $40^{th}$ percentile and the $65^{th}$ percentile, as established in this sub-group. In one embodiment overexpression for PDL-1 is acknowledged if the expression level for PDL-1 is above a cut-off value between the $50^{th}$ percentile and the $60^{th}$ percentile, as established in this sub-group. In one embodiment individuals/patients are determined as being in need of a PDL-1 cotherapy, if the PDL-1 expression level in the sub-group with decreased expression of IFN-γ is above the $54^{th}$ percentile.

In one embodiment, individuals/patients are determined as being in need of a PDL-1 cotherapy, if IFN-γ expression in a tumor tissue sample is decreased (i.e. below or at the IFN-γ cut-off value) and PDL-1 is overexpressed (i.e. above the PDL-1 cut-off value).

The term "expression level of programmed death ligand 1 (PD-L1) that is increased in comparison to a control" can be used interchangeably herein with "expression level of programmed death ligand 1 (PD-L1) above the PDL-1 cut-off value" as defined and explained herein above.

The term "expression level of interferon-gamma (IFNγ) that is decreased in comparison to a control" " can be used interchangeably herein with "expression level of interferon-gamma (IFNγ) below or at the IFNγ cut-off value".

The present invention relates to the following aspects.

The present invention relates to a method of determining the need of a cancer patient for a PD-L1 inhibitor cotherapy, (i) wherein therapy comprising a modulator of the HER2/neu (ErbB2) signaling pathway and a chemotherapeutic agent is contemplated for the patient or (ii) wherein the patient is undergoing therapy comprising a modulator of the HER2/neu (ErbB2) signaling pathway and a chemotherapeutic agent, the method comprising the steps of

a) measuring in vitro in a sample from said patient the expression level of Estrogen receptor (ER), of programmed death ligand 1 (PD-L1), and of interferon-gamma (IFNγ);

b) determining a patient as being in need of a PD-L1 inhibitor cotherapy if a low or absent ER expression level (like ER(-)/ER-negative), an expression level of programmed death ligand 1 (PD-L1) above the PDL-1 cut-off value and an expression level of interferon-gamma (IFNγ) below or at the IPNγ cut-off value is measured in step (a).

The present invention relates to a method of treating a cancer in a cancer patient for whom therapy comprising a modulator of the HER2/neu (ErbB2) signaling pathway and a chemotherapeutic agent is contemplated, the method comprising selecting a cancer patient whose cancer is determined to have a low or absent ER expression level (like ER(-)/ER-negative) and to have an expression level of programmed death ligand 1 (PD-L1) above the PDL-1 cut-off value and to have an expression level of interferon-gamma (IFNγ) below or at the IFNγ cut-off value, and administering to the patient an effective amount of a modulator of the HER2/neu (ErbB2) signaling pathway, of a chemotherapeutic agent and of a programmed death ligand 1 (PD-L1) inhibitor.

The present invention relates to a method of treating a cancer in a cancer patient who is undergoing therapy comprising a modulator of the HER2/neu (ErbB2) signaling pathway and a chemotherapeutic agent, the method comprising selecting a cancer patient whose cancer is determined to have a low or absent ER expression level (like ER(-)/ER-negative) and to have an expression level of programmed death ligand 1 (PD-L1) above the PDL-1 cut-off value and to have an expression level of interferon-gamma (IFNγ) below or at the IFNγ cut-off value, and administering to the patient an effective amount of a programmed death ligand 1 (PD-L1) inhibitor.

The present invention relates to a pharmaceutical composition comprising a modulator of the HER2/neu (ErbB2) signaling pathway, and an inhibitor of programmed death ligand 1 (PD-L1) for use in the treatment of cancer, whereby said cancer is determined to have a low or absent ER expression level (like ER(-)/ER-negative) and to have an expression level of programmed death ligand 1 (PD-L1) above the PDL-1 cut-off value and to have an expression level of interferon-gamma (IFNγ) below or at the IFNγ cut-off value.

All explanations and definitions given herein for "PD-L1 inhibitor", "PD-L1 inhibitor cotherapy", "cancer", "cancer patient", "modulator of the HER2/neu (ErbB2) signaling pathway", "chemotherapeutic agent", "sample", "expression level" and the like apply, mutatis mutandis, to the above aspects of the present invention.

The expression level of Estrogen receptor (ER), of programmed death ligand 1 (PD-L1), and of interferon-gamma (IFNγ) in a sample from the patient may be measured in vitro simultaneously or subsequently in any combination. For example, the expression level of Estrogen receptor (ER), of programmed death ligand 1 (PD-L1), and of interfeion-gamma (IFNγ) may be measured simultaneously. The expression level of Estrogen receptor (ER) may be measured first, followed by the measurement of programmed death ligand 1 (PD-L1) and of interferon-gamma (IFNγ). The expression level of programmed death ligand 1 (PD-L1) may be measured first, followed by the (simultaneous or subsequent) measurement of Estrogen receptor (ER) and of interferon-gamma (IFNγ).The expression level of interferon-gamma (IFNγ).may be measured first, followed by the (simultaneous or subsequent) measurement of Estrogen receptor (ER) and of programmed death ligand 1 (PD-L1). Any order/combination of the measurement of the expression level of Estrogen receptor (ER), of programmed death ligand 1 (PD-L1), and of interferon-gamma (IFNγ) in a sample from the patient is envisaged and comprised herein.

Herein contemplated is a determination of a patient as being in need of a PD-L1 inhibitor cotherapy if, in a first step (1) a low or absent ER expression level (like ER(-)/ER-negative) is measured, and if, in a second step (2) an expression level of interferon-gamma (IFNγ) below or at the IPNγ cut-off value is measured and if, in a third step (3) an expression level of programmed death ligand 1 (PD-L1) above the PDL-1 cut-off value is measured.

The present invention relates to the following aspects:

The present invention relates to a method of determining the need of a cancer patient for a PD-L1 inhibitor cotherapy, (i) wherein therapy comprising a modulator of the HER2/neu (ErbB2) signaling pathway and a chemotherapeutic agent is contemplated for the patient or (ii) wherein the patient is undergoing therapy comprising a modulator of the HER2/neu (ErbB2) signaling pathway and a chemotherapeutic agent, the method comprising the steps of

a) measuring in vitro in a sample from said patient the expression level of Estrogen receptor (ER), of programmed death ligand 1 (PD-L1), and of interferon-gamma (IFNγ);
b) determining a patient as being in need of a PD-L1 inhibitor cotherapy if, in a first step (1) a low or absent ER expression level (like ER(-)/ER-negative) is measured, and if in a second step (2) an expression level of interferon-gamma (IFNγ) below or at the IFNγ cut-off value is measured and if in a third step (3) an expression level of programmed death ligand 1 (PD-L1) above the PDL-1 cut-off value is measured.

The present invention relates to a method of treating a cancer in a cancer patient for whom therapy comprising a modulator of the HER2/neu (ErbB2) signaling pathway and a chemotherapeutic agent is contemplated, the method comprising selecting a cancer patient whose cancer is determined to have in a first step (1) a low or absent ER expression level (like ER(-)/ER-negative) and in a second step (2) to have an expression level of interferon-gamma (IFNγ) below or at the IFNγ cut-off value, and in a third step (3) to have an expression level of programmed death ligand 1 (PD-L1) above the PDL-1 cut-off value, and administering to the patient an effective amount of a modulator of the HER2/neu (ErbB2) signaling pathway, of a chemotherapeutic agent and of a programmed death ligand 1 (PD-L1) inhibitor.

The present invention relates to a method of treating a cancer in a cancer patient who is undergoing therapy comprising a modulator of the HER2/neu (ErbB2) signaling pathway and a chemotherapeutic agent, the method comprising selecting a cancer patient whose cancer is determined to have in have in a first step (1) a low or absent ER expression level (like ER(-)/ER-negative) and in a second step (2) to have an expression level of interferon-gamma (IFNγ) below or at the IFNγ cut-off value, and in a third step (3) to have an expression level of programmed death ligand 1 (PD-L1) above the PDL-1 cut-off value, and administering to the patient an effective amount of a programmed death ligand 1 (PD-L1) inhibitor.

The present invention relates to a pharmaceutical composition comprising a modulator of the HER2/neu (ErbB2) signaling pathway, and an inhibitor of programmed death ligand 1 (PD-L1) for use in the treatment of cancer, whereby said cancer is determined to have a low or absent ER expression level (like ER(-)/ER-negative), to have an expression level of interferon-gamma (IFNγ) below or at the IFNγ cut-off value, to have an expression level of programmed death ligand 1 (PD-L1) above the PDL-1 cut-off value.

All explananations and definitions given herein for "PD-L1 inhibitor", "PD-L1 inhibitor cotherapy", "cancer", "cancer patient", "modulator of the HER2/neu (ErbB2) signaling pathway", "chemotherapeutic agent", "sample", "expression level" and the like apply, mutatis mutandis, to the above aspects of the present invention.

The following relates to a an exemplary cut-off value allowing determining a patient as being in need of a PD-L1 inhibitor cotherapy in accordance with the present invention. It can be easily determined by routine techniques (such as Affymetrix) whether the expression level of PD-L1 and/or IFN-gamma in a sample from a patient is below or above such cut-off values.

If a gene expression analysis gives a result for IFN-gamma expression higher or equal to 4.8 no combination treatment (HER2-targeted and PDL1-targeted) is recommended and no further PDL1 assessment is necessary. If a gene expression analysis gives a result for IFN-gamma lower than 4.8 a parallel assessment of PDL-1 is necessary. If PDL-1 gene expression analysis then gives a result of higher or equal to 5.3 a combination treatment (HER2-targeted and PDL1-targeted) is recommended. This exemplary protocol is illustrated in **Figure 19.**

In this context Affymetrix can be performed as follows: Total RNA from tumor cells was extracted FFPE tumor sections using Light Cycler Pertuzumab FFPET RNA Kit (Roche Diagnostics). RNA was processed for hybridization using the WT-Ovation FFPE System V2 (Nugen) and hybridized to Affymetrix GeneChip® Human Genome U133 Plus 2.0 Arrays. Hybridized arrays were washed and stained on Affymetrix Fluidics Station 450 and scanned with an Affymetrix GeneChip® Scanner 3000 7G.

As mentioned the expression level of PD-L1 and/or IFN-gamma in a sample from a patient can be determined by routine techniques, such as Affymetrix. The following relates an exemplary protocol for such a determination (also termed herein Gene Expression Profiling):
The tumor biopsy samples can be profiled for gene expression on AFFYMETRIX HG-U133Plus 2 whole Human Genome microarray platform. Roche HighPure RNA extraction, NuGen amplification and standard AFFYMETRIX hybridization and scanning protocols can be used. These protocols etc. are incorporated herein by reference. All array scans usually pass standard AFFYMETRIX QC.

Robust Multiarray algorithm (RMA) can be used for preprocessing of raw signals (Irizarry et al, 2003. World wide web at .ncbi.nlm.nih.gov/pubmed/12925520; incorporated herein by reference). All probe sets available for the genes of interest can be retrieved as reported below. Fir gene CD274, when several probe sets were available to represent this gene, the probe set with the highest average expression value (defined as an arithmetical average of expression of a given probe set) was selected to represent the gene:

*CD274 (PDL1)*

223834_at selected for PDL1

227458_at

The selected probe set corresponds to the last exon / 3'UTR of the gene and captures all known RefSEq mRNAs (see **Figure 6**)

*IFNG*

210354_at

This probe set also represents the last exon / 3'UTR of the gene and captures all known RefSEq mRNAs (see **Figure 7**)

In accordance with the above, the expression level of Interferon-gamma may be measured prior to the expression level of Estrogen receptor (ER) and/or prior to the expression level of programmed death ligand 1 (PD-L1). The step of measuring the expression level of Estrogen receptor (ER) and of programmed death ligand 1 (PD-L1) may even be absent.

As shown in the appended Example, PD-L1 cotherapy can, for example, not be recommended if the expression level of interferon-gamma (IFN$\gamma$) is higher or equal to (about) 4.8 as determined by routine methods like Affymetrix.

Accordingly, the present invention provides a method of determining the need of a cancer patient for a PD-L1 inhibitor cotherapy, wherein therapy comprising a modulator of the HER2/neu (ErbB2) signaling pathway and a chemotherapeutic agent is contemplated for the patient or wherein the patient is undergoing therapy comprising a modulator of the HER2/neu (ErbB2) signaling pathway and a chemotherapeutic agent, the method comprising the steps

(a) measuring in vitro in a sample from said patient the expression level of interferon-gamma (IFN$\gamma$)
(b) determining a patient as being not in need of a PD-L1 inhibitor cotherapy if the expression level of interferon-gamma (IFN$\gamma$) is higher or equal to (about) 4.8 as determined by routine methods like Affymetrix in step (a).

If the expression level of interferon-gamma (IFN$\gamma$) is lower than (about) 4.8 as determined by routine methods like Affymetrix, the expression level of programmed death ligand 1 (PD-L1) and, optionally, Estrogen receptor (ER) can be measured in vitro in a sample from said patient.

Accordingly, the present invention provides a method of determining the need of a cancer patient for a PD-L1 inhibitor cotherapy, wherein therapy comprising a modulator of the HER2/neu (ErbB2) signaling pathway and a chemotherapeutic agent is contemplated for the patient or wherein the patient is undergoing therapy comprising a modulator of the HER2/neu (ErbB2) signaling pathway and a chemotherapeutic agent, the method comprising the steps

(a) measuring in vitro in a sample from said patient the expression level of interferon-gamma (IFN$\gamma$), Estrogen receptor (ER) and of programmed death ligand 1 (PD-L1),
(b) determining a patient as being in need of a PD-L1 inhibitor cotherapy if the expression level of interferon-gamma (IFN$\gamma$) is lower than (about) 4.8 as determined by routine methods like Affymetrix, and if a low or absent ER expression level and, optionally, an expression level of programmed death ligand 1 (PD-L1) that is increased in comparison to a control is measured in step (a).

A patient can be determined in accordance with the present invention to be in need of PD-L1 inhibitor cotherapy if the expression level of programmed death ligand 1 (PD-L1) measured in the sample from the patient is increased in comparison to a control. For example, the expression level of programmed death ligand 1 (PD-L1) can be higher or equal to (about) 5.3 determined by routine methods like Affymetrix.

All explananations and definitions given herein for "PD-L1 inhibitor", "PD-L1 inhibitor cotherapy", "cancer", "cancer patient", "modulator of the HER2/neu (ErbB2) signaling pathway", "chemotherapeutic agent", "sample", "expression level" and the like as given herein apply, mutatis mutandis, in this context.

Accordingly, the present invention relates to a method of treating a cancer in a cancer patient for whom therapy comprising a modulator of the HER2/neu (ErbB2) signaling pathway and a chemotherapeutic agent is contemplated, the method comprising selecting a cancer patient whose cancer is determined to have a low or absent ER expression level and to have an increased expression level of programmed death ligand 1 (PD-L1) in comparison to a control, and an expression level of interferon-gamma (IFN$\gamma$) that is lower than (about) 4.8 as determined by routine methods like Affymetrix, and administering to the patient an effective amount of a modulator of the HER2/neu (ErbB2) signaling pathway, of a chemotherapeutic agent and of a programmed death ligand 1 (PD-L1) inhibitor.

Furthermore, the present invention relates to a method of treating a cancer in a cancer patient who is undergoing therapy comprising a modulator of the HER2/neu (ErbB2) signaling pathway and a chemotherapeutic agent, the method comprising selecting a cancer patient whose cancer is determined to have a low or absent ER expression level and to have an increased expression level of programmed death ligand 1 (PD-L1) in comparison to a control, and to have an expression level of interferon-gamma (IFNγ) that is lower than (about) 4.8 as determined by routine methods like Affymetrix, and administering to the patient an effective amount of a programmed death ligand 1 (PD-L1) inhibitor.

A pharmaceutical composition is provided comprising a modulator of the HER2/neu (ErbB2) signaling pathway, and an inhibitor of programmed death ligand 1 (PD-L1) for use in the treatment of cancer, whereby said cancer is determined to have a low or absent ER expression level and to have an increased expression level of programmed death ligand 1 (PD-L1) in comparison to a control, and an expression level of interferon-gamma (IFNγ) that is lower than (about) 4.8 as determined by routine methods like Affymetrix.

The pharmaceutical composition for use in the treatment of cancer may further comprise a chemotherapeutic agent.

In accordance with the above, the herein provided methods may comprise a step of measuring the expression level of Interferon-gamma (IFNγ) in said sample and determining a patient as being in need of a PD-L1 inhibitor cotherapy if an expression level of interferon- gamma (IFNγ) that is decreased in comparison to the control is measured. For example, a "decreased expression level" of interferon- gamma (IFNγ) may be an expression level lower than (about) 4.8 as determined by routine methods like Affymetrix. Accordingly, the cancer that is determined to have a decreased expression level of interferon-gamma (IFNγ) in comparison to the control may be determined to have an expression level of interferon-gamma (IFNγ) that is lower than (about) 4.8 as determined by routine methods like Affymetrix,

It is envisaged herein that the expression level may be reflected in the activity of the gene product/protein. Accordingly, also the activity of ER, PD-L1 and/or IFN-γ can be measured and evaluated in addition or in the alternative to the expression level in accordance with the present invention. A person skilled in the art is aware of corresponding means and methods for detecting and evaluating the ER, PD-L1 and IFN-γ expression level and/or activity. Exemplary methods to be used include but are not limited to molecular assessments such as Western Blots, Northern Blots, Real-Time PCR and the like. Such methods are described herein in detail.

The expression level of ER, PD-L1 and/or IFN-γ may be the mRNA expression level of ER, PD-L1 and/or IFN-γ. If the gene product is an RNA, in particular an mRNA (e.g. unspliced, partially spliced or spliced mRNA), determination can be performed by taking advantage of northern blotting techniques, in situ hybridization, hybridization on microarrays or DNA chips equipped with one or more probes or probe sets specific for mRNA transcripts or PCR techniques, like, quantitative PCR techniques, such as Real time PCR. These and other suitable methods for binding (specific) mRNA are well known in the art and are, for example, described in Sambrook and Russell (2001, loc. cit.). A skilled person is capable of determining the amount of the component, in particular said gene products, by taking advantage of a correlation, preferably a linear correlation, between the intensity of a detection signal and the amount of the gene product to be determined.

The expression level may be the protein expression level of ER, PD-L1 and/or IFN-γ. Quantification of the protein expression level can be performed by taking advantage of the well known techniques such as western blotting techniques, immunoassays, gel- or blot-based methods, IHC, mass spectrometry, flow cytometry, FACS and the like. Generally, a person skilled in the art is aware of methods for the quantitation of (a) polypeptide(s)/protein(s). Amounts of purified polypeptide in solution can be determined by physical methods, e.g. photometry. Methods of quantifying a particular polypeptide in a mixture may rely on specific binding, e.g of antibodies. Specific detection and quantitation methods exploiting the specificity of antibodies comprise for example immunohistochemistry (*in situ*). Western blotting combines separation of a mixture of proteins by electrophoresis and specific detection with antibodies. Electrophoresis may be multi-dimensional such as 2D electrophoresis. Usually, polypeptides are separated in 2D electrophoresis by their apparent molecular weight along one dimension and by their isoelectric point along the other direction. Alternatively, protein quantitation methods may involve but are not limited to mass spectrometry or enzyme-linked immunosorbant assay methods.

Also the use of high throughput screening (HTS) is envisaged in the context of the present invention. Suitable (HTS) approaches are known in the art. A person skilled in the art is readily in the position to adapt such protocols or known HTS approaches to the performance of the methods of the present invention. Such assays are usually performed in liquid phase, wherein for each cell/tissue/cell culture to be tested at least one reaction batch is made. Typical containers to be used are micro titer plates having for example, 384, 1536, or 3456 wells (i.e. multiples of the "original" 96 reaction vessels). Robotics, data processing and control software, and sensitive detectors, are further commonly used components of a HTS device. Often robot system are used to transport micro titer plates from station to station for addition and mixing of sample(s) and reagent(s), incubating the reagents and final readout (detection). Usually, HTS can be used in the simultaneous preparation, incubation and analysis of many plates. The assay can be performed in a single reaction (which is usually preferred), may, however, also comprise washing and/or transfer steps. Detection can be performed taking advantage of radioactivity, luminescence or fluorescence, like fluorescence-resonance-energy transfer (FRET) and fluorescence polarisation (FP) and the like. The biological samples described herein can also be used in such a context. In particular cellular assays and in vivo assays can be employed in HTS. Cellular assays may also comprise cellular extracts, i.e. extracts from cells, tissues and the like. However, preferred herein is the use of cell(s) or tissue(s) as biological sample (in particular a sample obtained from a patient/subject suffering or being prone to suffer from cancer), whereas in vivo assays are particularly useful in the validation of modulators/inhbitors/chemotherapeutic agents to be used herein. Depending on the results of a first assay, follow up assays can be performed by rerunning the experiment to collect further data on a narrowed set (e.g. samples found "positive" in the first assay), confirming and refining observations.

As used in context of the methods of the present invention, a non-limiting example of a "control" is preferably a control from a patient who is not in need of a PD-L1 inhibitor cotherapy, for example a sample/cell/tissue obtained from one or more healthy subjects or one or more patients that suffer from a cancer/tumor and are known to be not in need of a PD-L1 inhibitor cotherapy treatment. For example, such a control (sample) may be from a patient who does not benefit from additional PD-L1 inhibitor cotherapy. Another non-limiting example of a "control" is an "internal standard", for example a mixture of purified or synthetically produced proteins and/or peptides or RNA, where the amounts of each protein/peptide/RNA is gauged by using the control described above.

A further non-limiting example of a "control" may be a "healthy" control, for example a sample/cell/tissue obtained from a healthy subject or patient that is not suffering from a cancer/tumor or a cell obtained from such a subject. In accordance with the above, the reference or control expression level of ER, PD-L1 and/or IFN-γ is that determined in (a sample of) the corresponding healthy control subject/patient, i.e. it is the "normal" status of ER, PD-L1 and/or IFN-γ. The control may also be a sample/cell/tissue obtained from the individual or patient suspected of suffering from the cancer provided that the sample/cell/tissue does not contain tumor or cancer cells. In a further alternative, the "control" may be a sample/cell/tissue obtained from an individual or patient suffering from the cancer, that has been obtained prior to the development or diagnosis of said cancer.

The sample to be assessed in accordance with the herein provided methods may comprise non-diseased cells and/or diseased cells, i.e. non-cancerous cells and/or cancerous cells. However the content of cancerous cells among non cancerous cells should be higher than for example 50%. The sample may also (or even solely) comprise cancer/tumor cell(s), such as breast cancer/tumor cell(s). The term "sample" shall generally mean any biological sample obtained from a patient's tumor. The sample may be a tissue resection or a tissue biopsy. The sample may also be a metastatic lesion or a section of a metastatic lesion or a blood sample known or suspected to comprise circulating tumor cells. In accordance with the above, the biological sample may comprise cancer cells and to a certain extent i.e. less than for example 50% non-cancer cells (other cells). The skilled pathologist is able to differentiate cancer cells from normal tissue cells. Methods for obtaining tissue biopsies, tissue resections and body fluids and the like from mammals, such as humans, are well known in the art.

As explained above, the cancer patient who is determined to be in need of PD-L1 inhibitor cotherapy in accordance with the present invention is undergoing therapy comprising a modulator of the HER2/neu (ErbB2) signaling pathway and a chemotherapeutic agent or such a therapy comprising a modulator of the HER2/neu (ErbB2) signaling pathway and a chemotherapeutic agent is contemplated for the patient. Therapy comprising a modulator of the HER2/neu (ErbB2) signaling pathway and a chemotherapeutic agent is indicated for patients with "HER2-positive cancer", like a patient that is suspected to suffer from a HER2-positive cancer, suffering from a HER2-positive cancer or being prone to suffer from a HER2-positive cancer. Preferably, the cancer to be treated is in accordance with the present invention a "HER2-positive cancer", particularly a "HER2-positive breast cancer". A "HER2-positive cancer" can be a "HER2-positive breast cancer" or a "HER2-positive gastric cancer". Further, the HER2-positive cancer may be ovarian cancer, lung cancer, colorectal cancer, kidney cancer, bone cancer, bone marrow cancer, bladder cancer, skin cancer, prostate cancer, esophagus cancer, salivary gland cancer, pancreas cancer, liver cancer, head and neck cancer, CNS (especially brain) cancer, cervix cancer, cartilage cancer, colon cancer, genitourinary cancer, gastrointestinal tract cancer, pancreas cancer, synovium cancer, testis cancer, thymus cancer, thyroid cancer and uterine cancer.

The term "HER2-positive cancer" as used herein refers to a cancer/tumorous tissue etc. which comprises cancer cells which have higher than normal levels of HER2. For the purpose of the present invention, "HER2-positive cancer" has an immunohistochemistry (IHC) score of at least 2+ and/or an *in situ* hybridization (ISH) amplification ratio ≥2.0 (i.e. is ISH-positive). Accordingly, HER2-positive cancer is present if a high HER2 (protein) expression level detected e.g. by immunohistochemical methods and/or HER2 gene amplification detected by in-situ-hybridization (ISH positive, like a HER2 gene copy number higher than 4 copies of the HER2 gene per tumor cell or ratio of ≥ 2.0 for the number of HER2 gene copies to the number of signals for CEP17.) is found in samples obtained from the patients such as breast tissue biopsies or breast tissue resections or in tissue derived from metastatic sites. In one embodiment "HER2-positive cancer" has an immunohistochemistry (IHC) score of HER2(3+) and/or is ISH positive.

The expression level of HER2 may be detected by an immunohistochemical method, whereas said HER2 gene amplification status can be measured with in situ hybridization methods, like fluorescence in situ hybridization techniques (FISH). Corresponding assays and kits are well known in the art, for protein expression assays as well as for the detection of gene amplifications. Alternatively other methods like qRT-PCR might be used to detect levels of HER2 gene expression.

The expression level of HER2 can, inter alia, be detected by an immunohistochemical method. Such methods are well known in the art and corresponding commercial kits are available. Exemplary kits which may be used in accordance with the present invention are, inter alia, HerceptTest™ produced and distributed by the company Dako or the test called Ventana Pathway™. The level of HER2 protein expression may be assessed by using the reagents provided with and following the protocol of the HercepTest™. A skilled person will be aware of further means and methods for determining the expression level of HER2 by immunohistochemical methods; see for example WO 2005/117553. Therefore, the expression level of HER2 can be easily and reproducibly determined by a person skilled in the art without undue burden. However, to ensure accurate and reproducible results, the testing must be performed in a specialized laboratory, which can ensure validation of the testing procedures.

The expression level of HER2 can be classified in a low expression level, an intermediate expression level and a high expression level. It is preferred in context of this invention that HER2-positive disease is defined by a strong expression level of HER2 (e.g. HER2(3+) by IHC), for example determined in a sample of a cancer patient.

The recommended scoring system to evaluate the IHC staining patterns which reflect the expression levels of HER2 designated herein HER2(0), HER2(+), HER2(++) and HER2(+++), is as follows:

| Staining Intensity Score | Staining Pattern | HER2 overexpression assessment |
|---|---|---|
| 0 | No staining is observed or membrane staining is observed in < 10 % of the tumor cells | negative |
| 1+ | A faint/barely perceptible membrane staining is detected in > 10 % of the tumor cells. the cells are only stained in part of their membrane. | negative |
| 2+ | A weak to moderate complete staining is detected in > 10 % of the tumor cells. | weak to moderate overexpression. |
| 3+ | A strong complete membrane staining is detected in > 10 % of the tumor cells. | strong overexpression. |

The above IHC staining patterns are routinely used in determining HER2-positive breast cancer. The terms HER2(+), HER2(++) and HER2(+++) used herein are equivalent to the terms HER2(1+), HER2(2+) and HER2(3+). A "low protein expression level" used in context of this invention corresponds to a 0 or 1+ score ("negative assessment" according to the table shown herein above), an "weak to moderate protein expression level" corresponds to a 2+ score ("weak to moderate overexpression", see the table above) and a "high protein expression level" corresponds to a 3+ score ("strong overexpression", see the table above). As described herein above in detail, the evaluation of the protein expression level (i.e. the scoring system as shown in the table) is based on results obtained by immunohistochemical methods. As a standard or routinely, the HER-2 status is, accordingly, performed by immunohistochemistry with one of two FDA-approved commercial kits available; namely the Dako Herceptest™ and the Ventana Pathway™. These are semi-quantitative assays which stratify expression levels into 0 (<20,000 receptors per cell, no expression visible by IHC staining), 1+ (~100,000 receptors per cell, partial membrane staining, < 10% of cells overexpressing HER-2), 2+ (~500,000 receptors per cell, light to moderate complete membrane staining, > 10% of cells overexpressing HER-2), and 3+ (~2,000,000 receptors per cell, strong complete membrane staining, > 10% of cells overexpressing HER-2).

Alternatively, further methods for the evaluation of the protein expression level of HER2 may be used, e.g. Western Blots, ELISA-based detection systems and so on.

A HER2-positive cancer may also be diagnosed by assessing the gene amplification status of HER2. HER2-positive cancer is, accordingly, diagnosed if this assessment by ISH is positive. In accordance with this assessment, a HER2-positive cancer may, inter alia, relate to an average HER2 gene copy number higher than 4 copies of the HER2 gene per tumor cell (for those test systems without an internal centromere control probe) or to a HER2/CEP17 ratio of >=2.0 (for those test systems using an internal chromosome 17 centromere control probe). In other words, the HER2-positive cancer may, inter alia, relate to a HER2 gene copy number greater than 4. The amplification level of the HER2 gene may easily be identified by in situ hybridization (ISH) like fluorescent in situ hybridization (FISH), chromogenic in situ hybridization (CISH) and silver in situ hybridization (SISH). These methods are known to the skilled artisan. The principles of these methods can be deduced from standard text books. Commercial kits for the determination of the HER2 gene amplification status by in situ hybridization are available.

The below IHC staining patterns are recommended for determining HER2-positive gastric cancer (see Dako Herceptest package insert).

of Hercep Test™ stained biopsies a cluster of at least 5 stained tumor cells is recommended. A cluster of at least 5 stained tumor cells consists of 5 connected HER2 stained tumor cells.

**Table 9: Interpretation and scoring of HER2 immunohistochemical staining**

| Score | Surgical Specimen - Staining Pattern | Biopsy Specimen - Pattern | HER2 Overexpression Assessment |
|---|---|---|---|
| 0 | No reactivity or membranous reactivity in < 10% of tumor cells | No reactivity or no membranous reactivity in any (or < 5 clustered) tumor cell | Negative |
| 1+ | Faint/barely perceptible membranous reactivity in ≥ 10% of tumor cells, cells are reactive only in part of their membrane | Tumor cell cluster (≥ 5 cells) with a faint/barely perceptible membranous reactivity irrespective of percentage of tumor cells stained | Negative |
| 2+ | Weak to moderate complete, basolateral or lateral membranous reactivity in ≥ 10% of tumor cells | Tumor cell cluster (≥ 5 cells) with a weak to moderate complete, basolateral or lateral membranous reactivity irrespective of percentage of tumor cells stained | Equivocal |
| 3+ | Strong complete, basolateral or lateral membranous reactivity in ≥ 10% of tumor cells | Tumor cell cluster (≥ 5 cells) with a strong complete, basolateral or lateral membranous reactivity irrespective of percentage of tumor cells stained | Positive |
| Guidelines based on Hofmann et al.(40). | | | |

A more refined IHC staining patterns for determining HER2-positive gastric cancer is as follows:

| Staining Intensity Score | Surgical specimen - staining pattern | Biopsy specimen - staining pattern | HER2 Overexpression Assessment |
|---|---|---|---|
| 0 | No reactivity or no membranous reactivity in < 10% of tumour cells | No reactivity or no membranous reactivity in any tumour cell | Negative |
| 1+ | Faint / barely perceptible membranous reactivity in ≥ 10% of tumour cells; cells are reactive only in part of their membrane | Tumour cell cluster (≥ 5 cells) with a faint / barely perceptible membranous reactivity irrespective of percentage of tumour cells stained | Negative |
| 2+ | Weak to moderate complete, basolateral or lateral membranous reactivity in ≥ 10% of tumour cells | Tumour cell cluster (≥ 5 cells) with a weak to moderate complete, basolateral or lateral membranous reactivity irrespective of percentage of tumour cells stained | Equivocal |
| 3+ | Strong complete, basolateral or lateral membranous reactivity in ≥ 10% of tumour cells | Tumour cell cluster (≥ 5 cells) with a strong complete, basolateral or lateral membranous reactivity irrespective of percentage of tumour cells stained | Positive |

As indicated above, the HER2 positive cancer to be treated in accordance with the present invention may be breast cancer, such early stage breast cancer. The term "early-stage breast cancer" as used herein refers to breast cancer that has not spread beyond the breast or the axilliary lymph nodes. Such cancer can be generally treated with neoadjuvant or adjuvant therapy. The term "neoadjuvant therapy" as used herein refers to systemic therapy given prior to surgery. The term "adjuvant therapy" refers to systemic therapy given after surgery. In accordanc with the above, treatment may be neoadjuvant or adjuvant therapy of early-stage breast cancer.

In accordance with the above, the sample to be assessed can be (obtained) from a patient with HER2-positive cancer as defined above. For example, the sample may be obtained from a tumorous tissue, (a) tumor(s) and, accordingly, is (a) tumor cell(s) or (a) tumor tissue(s) suspected of being HER2-positive tumour, like a breast tumor and the like. A person skilled in the art is in the position to identify such tumors and/or individuals/patients suffering from corresponding cancer using standard techniques known in the art and methods disclosed herein. Generally, said tumor cell or cancer cell may be obtained from any biological source/organism, particularly any biological source/organism, suffering from the above-mentioned cancer. In context of this invention particular useful cells are, preferably, human cells. These cells can be obtained from e.g. biopsies or from biological samples. The tumor/cancer/tumor cell/cancer cell is a solid tumor/ cancer/tumor cell/cancer cell. In accordance with the above, the cancer/tumor cell may be a breast cancer/tumor cell or said sample comprises a cancer/tumor cell, such as a breast cancer/tumor cell. In line with the above, said tumor/cancer may be a breast tumor/cancer. The modulator of the HER2/neu (ErbB2) signaling pathway may be an inhibitor of HER2, for example, a HER dimerization/signaling inhibitor. The HER dimerization inhibitor may be a HER2 dimerization inhibitor. The HER dimerization inhibitor may inhibit HER heterodimerization or HER homodimerization. The HER dimerization inhibitor may be an anti-HER antibody. The term "antibody" herein is used in the broadest sense and specifically covers intact monoclonal antibodies, polyclonal antibodies, multispecific antibodies (e.g., bispecific antibodies) formed from at least two intact antibodies, and antibody fragments, so long as they exhibit the desired biological activity. Also human and humanized as well as CDR-grafted antibodies are comprised.

The HER antibody may bind to a HER receptor selected from the group consisting of EGFR, HER2 and HER3. Preferably, the antibody binds to HER2. The anti HER2 antibody may bind to domain II of HER2 extracellular domain. The antibody may bind to a junction between domains I, II and III of HER2 extracellular domain. The anti HER2 antibody may be Pertuzumab.

For the purposes herein, "Pertuzumab" and "rhuMAb 2C4", which are used interchangeably, refer to an antibody comprising the variable light and variable heavy domains (amino acid sequences thereof shown in SEQ ID Nos. 5 and 6, respectively, as depicted in Figure 2). The variable light and variable heavy domains of variant 574/Pertuzumab are also shown in Figure 2 (amino acid sequences thereof shown in SEQ ID Nos. 7 and 8, respectively, as depicted in Figure 2). Where Pertuzumab is an intact antibody, it preferably comprises an IgG1 antibody; in one embodiment comprising the light chain amino acid sequence in it preferably comprises the light chain and heavy chain amino acid sequences, respectively, as shown in Figure 3A/3B and 5A/5B (Fig. 5A/5B show the light chain and heavy chain amino acid sequences of a variant Pertuzumab). The heavy chain amino acid sequences of Pertuzumab as shown in Fig. 3B may optionally comprise an additional amino acid "K" at position 449 at the C-terminus. The antibody is optionally produced by recombinant Chinese Hamster Ovary (CHO) cells. The terms "Pertuzumab" and "rhuMAb 2C4" herein cover biosimilar versions of the drug with the United States Adopted Name (USAN) or International Nonproprietary Name (INN): Pertuzumab. Again, corresponding sequences are shown in Figures 2 to 5.

The modulator of the HER2/neu (ErbB2) signaling pathway may be an inhibitor of HER shedding, for example a HER2 shedding inhibitor. The inhibitor of HER shedding may inhibit HER heterodimerization or HER homodimerization. Said inhibitor of HER shedding may be an anti-HER antibody.

The term "antibody" herein is used in the broadest sense and specifically covers intact monoclonal antibodies, polyclonal antibodies, multispecific antibodies (e.g., bispecific antibodies) formed from at least two intact antibodies, and antibody fragments, so long as they exhibit the desired biological activity. Also human and humanized as well as CDR-grafted antibodies are comprised.

The anti-HER antibody may bind to a HER receptor selected from the group consisting of EGFR, HER2 and HER3. Preferably, the antibody binds to HER2. The HER2 antibody may bind to sub-domain IV of the HER2 extracellular domain. Preferably, the HER2 antibody is Herceptin™ /Trastuzumab.

For the purposes herein, "Herceptin™"/"Trastuzumab" and "rhuMAb4D5-8", which are used interchangeably, refer to an antibody comprising the variable light domains and variable heavy domains (amino acid sequences thereof are shown in Figure 4, respectively; the domain is indicated by arrows). Where Trastuzumab is an intact antibody, it preferably comprises an IgG1 antibody; in one embodiment comprising the light chain amino and the heavy chain amino acid sequence as shown in Figure 4. The antibody is optionally produced by Chinese Hamster Ovary (CHO) cells. The terms "Trastuzumab" and "rhuMAb4D5-8" herein cover biosimilar versions of the drug with the United States Adopted Name (USAN) or International Nonproprietary Name (INN): Trastuzumab.

The inhibitor of programmed death ligand 1 (PD-L1) may be an antibody specifically binding to PD-L1 (anti-PD-L1 antibody). Again, the term "antibody" is used in the broadest sense and specifically covers intact monoclonal antibodies, polyclonal antibodies, multispecific antibodies (e.g., bispecific antibodies) formed from at least two intact antibodies, and antibody fragments, so long as they exhibit the desired biological activity. Also human and humanized as well as CDR-grafted antibodies are comprised.

Exemplary anti-PD-L1 antibodies are disclosed in WO 2010/077634 which is incorporated herein in its entirety. Corresponding exemplary anti-PD-L1 antibodies to be used in accordance with the present invention are described below.

The anti-PD-L1 antibody may comprise a heavy chain variable region polypeptide comprising an HVR-H1, HVR-H2 and HVR-H3 sequence, wherein:

(a) the HVR-H1 sequence is GFTFSX1SWIH (SEQ ID NO:1);

(b) the HVR-H2 sequence is AWIX2PYGGSX3YYADSVKG (SEQ ID NO:2);
(c) the HVR-H3 sequence is RHWPGGFDY (SEQ ID NO:3);

further wherein: X1 is D or G; X2 is S or L; X3 is T or S. X1 may be D; X2 may be S and X3 may be T.

The polypeptide may further comprise variable region heavy chain framework sequences juxtaposed between the HVRs according to the formula: (HC-FR1)-(HVR-H1)-(HC-FR2)-(HVR-H2)-(HC-FR3)-(HVR-H3)-(HC-FR4). The framework sequences may be derived from human consensus framework sequences. The framework sequences may be VH subgroup III consensus framework. One or more of the framework sequences may be the following:

HC-FR1 is EVQLVESGGGLVQPGGSLRLSCAAS (SEQ ID NO:4)
HC-FR2 is WVRQAPGKGLEWV (SEQ ID NO:5)
HC-FR3 is RFTISADTSKNTAYLQMNSLRAEDTAVYYCAR (SEQ ID NO:6)
HC-FR4 is WGQGTLVTVSA (SEQ ID NO:7).

The heavy chain polypeptide may be in combination with a variable region light chain comprising an HVR-L1, HVR-L2 and HVR-L3, wherein:

(a) the HVR-L1 sequence is RASQX4X5X6TX7X8A (SEQ ID NOs:8);
(b) the HVR-L2 sequence is SASX9LX10S, and (SEQ ID NOs:9);
(c) the HVR-L3 sequence is QQX11X12X13X14PX15T (SEQ ID NOs:10); further wherein: X4 is D or V; X5 is V or I; X6 is S or N; X7 is A or F; X8 is V or L; X9 is F or T; X10 is Y or A; X11 is Y, G, F, or S; X12 is L, Y, F or W; X13 is Y, N, A, T, G, F or I; X14 is H, V, P, T or I; X15 is A, W, R, P or T.

X4 may be D; X5 may be V; X6 may be S; X7 may be A; X8 may be V; X9 may be F; X10 may be Y; X11 may be Y; X12 may be L; X13 may be Y; X14 may be H; X15 may be A.

The polypeptide may further comprise variable region light chain framework sequences juxtaposed between the HVRs according to the formula: (LC-FR1)-(HVR-L1)-(LC-FR2)-(HVR-L2)-(LC-FR3)-(HVR-L3)-(LC-FR4).The framework sequences may be derived from human consensus framework sequences. The framework sequences may be VL kappa I consensus framework. One or more of the framework sequences may be the following:

LC-FR1 is DIQMTQSPSSLSASVGDRVTITC (SEQ ID NO:11);
LC-FR2 is WYQQKPGKAPKLLIY (SEQ ID NO:12);
LC-FR3 is GVPSRFSGSGSGTDFTLTISSLQPEDFATYYC (SEQ ID NO:13);
LC-FR4 is FGQGTKVEIKR (SEQ ID NO:14).

Theanti-PD-L1 antibody (or an antigen binding fragment thereof) may comprise a heavy chain and a light chain variable region sequence, wherein:

(a) the heavy chain comprises an HVR-H1, HVR-H2 and HVR-H3, wherein further:

(i) the HVR-H1 sequence is GFTFSX1SWIH (SEQ ID NO:1);
(ii) the HVR-H2 sequence is AWIX2PYGGSX3YYADSVKG (SEQ ID NO:2);
(iii) the HVR-H3 sequence is RHWPGGFDY, and (SEQ ID NO:3);

(b) the light chain comprises an HVR-L1, HVR-L2 and HVR-L3, wherein further:

(iv) the HVR-L1 sequence is RASQX4X5X6TX7X8A (SEQ ID NOs:8);
(v) the HVR-L2 sequence is SASX9LX10S (SEQ ID NOs:9);
(vi) the HVR-L3 sequence is QQX11X12X13X14PX15T (SEQ ID NOs:10);

wherein: X1 is D or G; X2 is S or L; X3 is T or S; X4 may be D or V; X5 may be V or I; X6 may be S or N; X7 may be A or F; X8 may be V or L; X9 may be F or T; X10 may be Y or A; X11 may be Y, G, F, or S; X12 may be L, Y, F or W; X13 may be Y, N, A, T, G, F or I; X14 may be H, V, P, T or I; X15 may be A, W, R, P or T.

X1 may be D; X2 may be S and X3 may be T. Furthermore, the positions may be as follows: X4 = D, X5 = V, X6 = S, X7 = A and X8 = V, X9 = F, and X10 = Y, X11 = Y, X12 = L, X13 = Y, X14 = H and/or X15 = A. Furthermore, the positions may be as follows: X1 = D, X2 = S and X3 = T, X4 = D, X5 = V, X6 = S, X7 = A and X8 = V, X9 = F, and X10 = Y, X11 = Y, X12 = L, X13 = Y, X14 = H and X15 = A.

The antibody (an antigen binding fragment thereof) may further comprise

(a) variable region heavy chain framework sequences juxtaposed between the HVRs according to the formula: (HC-FR1)-(HVR-H1)-(HC-FR2)-(HVR-H2)-(HC-FR3)-(HVR-H3)-(HC-FR4), and
(b) variable region light chain framework sequences juxtaposed between the HVRs according to the formula: (LC-FR1)-(HVR-L1)-(LC-FR2)-(HVR-L2)-(LC-FR3)-(HVR-L3)-(LC-FR4). The framework sequences may be derived from human consensus framework sequences.

The variable region heavy chain framework sequences may be VH subgroup III consensus framework. One or more of the framework sequences may be the following:

HC-FR1 is EVQLVESGGGLVQPGGSLRLSCAAS (SEQ ID NO:4);
HC-FR2 is WVRQAPGKGLEWV (SEQ ID NO:5);
HC-FR3 is RFTISADTSKNTAYLQMNSLRAEDTAVYYCAR (SEQ ID NO:6);
HC-FR4 is WGQGTLVTVSA (SEQ ID NO:7).

The variable region light chain framework sequences may be VL kappa I consensus framework. One or more of the framework sequences may be the following:

LC-FR1 is DIQMTQSPSSLSASVGDRVTITC (SEQ ID NO: 11);
LC-FR2 is WYQQKPGKAPKLLIY (SEQ ID NO:12);
LC-FR3 is GVPSRFSGSGSGTDFTLTISSLQPEDFATYYC, and (SEQ ID NO:13);
LC-FR4 is FGQGTKVEIKR (SEQ ID NO:14).

The antibody (or antigen binding fragment thereof) may be or may comprise

(a) the variable heavy chain framework sequences are the following:

(i) HC-FR1 is EVQLVESGGGLVQPGGSLRLSCAAS (SEQ ID NO:4);
(ii) HC-FR2 is WVRQAPGKGLEWV (SEQ ID NO:5);
(iii) HC-FR3 is RFTISADTSKNTAYLQMNSLRAEDTAVYYCAR (SEQ ID NO:6);
(iv) HC-FR4 is WGQGTLVTVSA; and (SEQ ID NO:7);

(b) the variable light chain framework sequences are the following:

(i) LC-FR1 is DIQMTQSPSSLSASVGDRVTITC (SEQ ID NO:11);
(ii) LC-FR2 is WYQQKPGKAPKLLIY (SEQ ID NO:12);
(iii) LC-FR3 is GVPSRFSGSGSGTDFTLTISSLQPEDFATYYC (SEQ ID NO:13);
(iv) LC-FR4 is FGQGTKVEIKR (SEQ ID NO:14).

The antibody (or fragment thereof) may further comprise a human constant region. The constant region may selected from the group consisting of IgG1, IgG2, IgG3 and IgG4. The constant region may be IgG1. The antibody (or fragment thereof) may further comprise murine constant region. The constant region may be selected from the group consisting of IgG1, IgG2A, IgG2B and IgG3. The constant region may be IgG2A.

The antibody (or fragment thereof) may have reduced or minimal effector function. The minimal effector function may result from an effector-less Fc mutation. The effector-less Fc mutation may be N297A. The effector-less Fc mutation may be D265A/N297A. The minimal effector function may result from aglycosylation.

The antibody (or fragment thereof) may comprise a heavy chain and a light chain variable region sequence, wherein:

(a) the heavy chain comprises an HVR-H1, HVR-H2 and an HVR-H3, having at least 85% overall sequence identity to GFTFSDSWIH (SEQ ID NO:15), AWISPYGGSTYYADSVKG (SEQ ID NO:16) and RHWPGGFDY (SEQ ID NO:3), respectively, and
(b) the light chain comprises an HVR-L1, HVR-L2 and an HVR-L3, having at least 85% overall sequence identity to RASQDVSTAVA (SEQ ID NO:17), SASFLYS (SEQ ID NO:18) and QQYLYHPAT (SEQ ID NO:19), respectively.

The sequence identity may be at least 90%.

The antibody (or fragment thereof) may further comprise:

(a) variable region heavy chain (VH) framework sequences juxtaposed between the HVRs according to the formula: (HC-FR1)-(HVR-H1)-(HC-FR2)-(HVR-H2)-(HC-FR3)-(HVR-H3)-(HC-FR4), and
(b) variable region light chain (VL) framework sequences juxtaposed between the HVRs according to the formula: (LC-FR1)-(HVR-L1)-(LC-FR2)-(HVR-L2)-(LC-FR3)-(HVR-L3)-(LC-FR4).

The antibody (or fragment thereof) may further comprise a VH and VL framework region derived from a human consensus sequence. The VH framework sequence may be derived from a Kabat subgroup I, II, or III sequence. The VH framework sequence may be a Kabat subgroup III consensus framework sequence. The VH framework sequences may be the following:

HC-FR1 is EVQLVESGGGLVQPGGSLRLSCAAS (SEQ ID NO:4);
HC-FR2 is WVRQAPGKGLEWV (SEQ ID NO:5);
HC-FR3 is RFTISADTSKNTAYLQMNSLRAEDTAVYYCAR (SEQ ID NO:6);
HC-FR4 is WGQGTLVTVSA (SEQ ID NO:7).

The VL framework sequence may be derived from a Kabat kappa I, II, III or IV subgroup sequence. The VL framework sequence may be a Kabat kappa I consensus framework sequence.
The VL framework sequences may be the following:

LC-FR1 is DIQMTQSPSSLSASVGDRVTITC (SEQ ID NO:11);
LC-FR2 is WYQQKPGKAPKLLIY (SEQ ID NO:12);
LC-FR3 is GVPSRFSGSGSGTDFTLTISSLQPEDFATYYC (SEQ ID NO:13);
LC-FR4 is FGQGTKVEIKR (SEQ ID NO:14).

The antibody (or fragment thereof) may comprise a heavy chain and a light chain variable region sequence, wherein:

(a) the heavy chain sequence has at least 85% sequence identity to the heavy chain sequence: EVQLVESGGGLVQPGGSLRLSCAASGFTFSDSWIHWVRQAPG KGLEWVAWISPYGGSTYYADSVKGRFTISADTSKNTAYLQMNSLRAEDTAVYYC ARRHWPGGFDYWGQGTLVTVSA (SEQ ID NO:20), and
(b) the light chain sequence has at least 85% sequence identity to the light chain sequence: DIQMTQSPSSLSAS-VGDRVTITCRASQDVSTAVAWYQQKPGK APKLLIYSASFLYSGVPSRFSGSGSGTDFTLTISSLQPEDFATYYC-QQYLYH PATFGQGTKVEIKR (SEQ ID NO:21).

The sequence identity may be at least 90%.
The antibody (or fragment thereof) may comprise a heavy chain and light chain variable region sequence, wherein:

(a) the heavy chain comprises the sequence: EVQLVESGGGLVQPGGSLRLS CAASGFTFSDSWIHWVRQAPGKGLEWVAWISPYGGSTYYADSVKGRFTISADTS KNTAYLQMNSLRAEDTAVYYCARRHWPGGFDYWGQGTLVTVSA (SEQ ID NO:20), and
(b) the light chain comprises the sequence: DIQMTQSPSSLSASVGDRVTITC RASQDVSTAVAWYQQKPGKAPKLLIYSASFLYSGVPSRFSGSGSGTDFTLTISSLQ PEDFATYYCQQYLYH PATFGQGTKVEIKR (SEQ ID NO:21).

Moreover, the anti-PD-L1 antibody may be encoded by a nucleic acid. Accordingly, herein described is an isolated nucleic acid encoding the above polypeptide /antibody (or fragment thereof).
Provided herein is an isolated nucleic acid encoding a light chain or a heavy chain variable sequence of an anti-PD-L1 antibody or antigen binding fragment, wherein:

(a) the heavy chain further comprises and HVR-H1, HVR-H2 and an HVR-H3 sequence having at least 85% sequence identity to GFTFSDSWIH (SEQ ID NO:15), AWISPYGGSTYYADSVKG (SEQ ID NO:16) and RHWPGGFDY (SEQ ID NO:3), respectively, or
(b) the light chain further comprises an HVR-L1, HVR-L2 and an HVR-L3 sequence having at least 85% sequence identity to RASQDVSTAVA (SEQ ID NO:17), SASFLYS (SEQ ID NO:18) and QQYLYHPAT (SEQ ID NO:19), respectively.

The sequence identity may be 90%. The anti-PD-L1 antibody may further comprise a VL and a VH framework region derived from a human consensus sequence. The VH sequence may be derived from a Kabat subgroup I, II, or III sequence. The VL sequence may be derived from a Kabat kappa I, II, III or IV subgroup sequence. The anti-PD-L1 antibody may comprise a constant region derived from a murine antibody. The anti-PD-L1 antibody may comprise a constant region derived from a human antibody. The constant region may be IgG1. The antibody encoded by the nucleic acid may have reduced or minimal effector function. The minimal effector function may result from an effector-less Fc mutation. The effector-less Fc mutation may be N297A.

Further provided herein is a vector comprising the nucleic acid, a host cell comprising the vector. The host cell may be eukaryotic. The host cell may be mammalian. The host cell may be a Chinese Hamster Ovary (CHO) cell. The host cell may be prokaryotic. The host cell may be E. coli. Also provided herein is a process for making an anti-PD-L1 antibody comprising culturing the above host cell under conditions suitable for the expression of the vector encoding the anti-PD-L1 antibody or antigen binding fragment, and recovering the antibody or fragment.

The following describes in more detail the herein provided means and methods for treating a cancer and/or a cancer patient.

Herein contemplated is, accordingly, a pharmaceutical composition comprising a modulator of the HER2/neu (ErbB2) signaling pathway (like Trastuzumab), and an inhibitor of programmed death ligand 1 (PD-L1) (like the anti-PD-L1 antibody described herein) for use in the treatment of cancer, whereby said cancer is determined to have a low or absent ER expression level and to have an increased expression level of programmed death ligand 1 (PD-L1) in comparison to a control. The cancer may be determined to have a decreased expression level of interferon-gamma (IFNγ) in comparison to the control. The pharmaceutical composition may further comprise a chemotherapeutic agent (like taxol or a taxol derivative, such as dodetaxel (Taxotere®)).

In accordance with the above, the present invention provides a method for treating cancer comprising administering an effective amount of a modulator of the HER2/neu (ErbB2) signaling pathway, a chemotherapeutic agent and an inhibitor of programmed death ligand 1 (PD-L1) to a subject in need thereof. The cancer may be determined to have a decreased expression level of interferon-gamma (IFNγ) in comparison to the control.

Herein provided is a modulator of the HER2/neu (ErbB2) signaling pathway, and an inhibitor of programmed death ligand 1 (PD-L1) for use in the treatment of cancer, whereby said cancer is determined to have a low or absent ER expression level and to have an increased expression level of programmed death ligand 1 (PD-L1) in comparison to a control. Moreover, herein provided is a modulator of the HER2/neu (ErbB2) signaling pathway, an inhibitor of programmed death ligand 1 (PD-L1) and a chemotherapeutic agent (like taxol or a taxol derivative, such as dodetaxel (Taxotere®)) for use in the treatment of cancer, whereby said cancer is determined to have a low or absent ER expression level and to have an increased expression level of programmed death ligand 1 (PD-L1) in comparison to a control. The cancer may be determined to have a decreased expression level of interferon-gamma (IFNγ) in comparison to the control.

As discussed above, the present invention provides a method of treating a cancer in a cancer patient for whom therapy comprising a modulator of the HER2/neu (ErbB2) signaling pathway and a chemotherapeutic agent is contemplated, the method comprising selecting a cancer patient whose cancer is determined to have a low or absent ER expression level and to have an increased expression level of programmed death ligand 1 (PD-L1) in comparison to a control, and administering to the patient an effective amount of a modulator of the HER2/neu (ErbB2) signaling pathway, of a chemotherapeutic agent and of a programmed death ligand 1 (PD-L1) inhibitor. Likewise, the present invention provides a method of treating a cancer in a cancer patient who is undergoing therapy comprising a modulator of the HER2/neu (ErbB2) signaling pathway and a chemotherapeutic agent, the method comprising selecting a cancer patient whose cancer is determined to have a low or absent ER expression level and to have an increased expression level of programmed death ligand 1 (PD-L1) in comparison to a control, and administering to the patient an effective amount of a programmed death ligand 1 (PD-L1) inhibitor.

The explanations and definitions given herein above in relation to "cancer", "cancer patient", "PD-L1 inhibitor", "PD-L1 inhibitor therapy", "modulator of the HER2/neu (ErbB2) signaling pathway", "chemotherapeutic agent", "low or absent ER expression level" "increased expression level of programmed death ligand 1 (PD-L1)", "decreased expression level of interferon-gamma (IFN-γ) and the like apply, mutatis mutandis, in the context of the herein .

The terms "treatment", "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effect attributed to the disease. The term "treatment" as used herein covers any treatment of a disease in a patient and includes: (a) preventing a disease related in a patient which may be predisposed to the disease; (b) inhibiting the disease, i.e. arresting its development; or (c) relieving the disease, i.e. causing regression of the disease.

A "patient" for the purposes of the present invention includes both humans and other animals, particularly mammals, and other organisms. Thus, the methods are applicable to both human therapy and veterinary applications. Preferably, the patient is human.

The below explanations relate in more detail to the treatment/therapy of these patients/this patient group in accordance with the present invention.

The pharmaceutical composition will be formulated and dosed in a fashion consistent with good medical practice, taking into account the clinical condition of the individual patient, the site of delivery of the pharmaceutical composition, the method of administration, the scheduling of administration, and other factors known to practitioners. The "effective amount" of the pharmaceutical composition for purposes herein is thus determined by such considerations.

The skilled person knows that the effective amount of one of the herein described PD-L1 inhibitor(s), modulator(s) of the HER2/neu (ErbB2) signaling pathway and chemotherapeutic agent(s) in a pharmaceutical composition administered to an individual will, inter alia, depend on the nature of the compound. For example, if said compound is a (poly)peptide or protein the total pharmaceutically effective amount of pharmaceutical composition administered parenterally per dose will be in the range of about 1 μg protein /kg/day to 10 mg protein /kg/day of patient body weight, although, as noted above, this will be subject to therapeutic discretion. More preferably, this dose is at least 0.01 mg protein /kg/day, and most preferably for humans between about 0.01 and 1 mg protein /kg/day.

The following administration may be employed in respect of Trastuzumab:

Posology and method of administration

HER2 testing is mandatory prior to initiation of therapy. Herceptin treatment should only be initiated by a physician experienced in the administration of cytotoxic chemotherapy.

MBC

Three-weekly schedule

The recommended initial loading dose is 8 mg/kg body weight. The recommended maintenance dose at three-weekly intervals is 6 mg/kg body weight, beginning three weeks after the loading dose.

Weekly schedule

The recommended initial loading dose of Herceptin is 4 mg/kg body weight. The recommended weekly maintenance dose of Herceptin is 2 mg/kg body weight, beginning one week after the loading dose.

Administration in combination with paclitaxel or docetaxel

In the pivotal trials (H0648g, M77001), paclitaxel or docetaxel was administered the day following the first dose of Herceptin (for dose, see the Summary of Product Characteristics for paclitaxel or docetaxel) and immediately after the subsequent doses of Herceptin if the preceding dose of Herceptin was well tolerated.

Administration in combination with an aromatase inhibitor

In the pivotal trial (BO16216) Herceptin and anastrozole were administered from day 1. There were no restrictions on the relative timing of Herceptin and anastrozole at administration (for dose, see the Summary of Product Characteristics for anastrozole or other aromatase inhibitors).

EBC

Three-weekly and weekly schedule

As a three-weekly regimen the recommended initial loading dose of Herceptin is 8 mg/kg body weight. The recommended maintenance dose of Herceptin at three-weekly intervals is 6 mg/kg body weight, beginning three weeks after the loading dose.

As a weekly regimen (initial loading dose of 4 mg/kg followed by 2 mg/kg every week) concomitantly with paclitaxel following chemotherapy with doxorubicin and cyclophosphamide. (See section 5.1 for chemotherapy combination dosing).

MGC

Three-weekly schedule

The recommended initial loading dose is 8 mg/kg body weight. The recommended maintenance dose at three-weekly intervals is 6 mg/kg body weight, beginning three weeks after the loading dose.

Breast Cancer (MBC and EBC) and Gastric Cancer (MGC)

Duration of treatment

Patients with MBC or MGC should be treated with Herceptin until progression of disease. Patients with EBC should be treated with Herceptin for 1 year or until disease recurrence, whatever occurs first.

Dose reduction

No reductions in the dose of Herceptin were made during clinical trials. Patients may continue therapy during periods of reversible, chemotherapy-induced myelosuppression but they should be monitored carefully for complications of neutropenia during this time. Refer to the Summary of Product Characteristics for paclitaxel, docetaxel or aromatase inhibitor for information on dose reduction or delays.

Missed doses

If the patient misses a dose of Herceptin by one week or less, then the usual maintenance dose (weekly regimen: 2 mg/kg; three-weekly regimen: 6 mg/kg) should be given as soon as possible. Do not wait until the next planned cycle. Subsequent maintenance doses (weekly regimen: 2 mg/ kg; three-weekly regimen: 6 mg/kg respectively) should then be given according to the previous schedule.

If the patient misses a dose of Herceptin by more than one week, a re-loading dose of Herceptin should be given over approximately 90 minutes (weekly regimen: 4 mg/kg; three-weekly regimen: 8 mg/kg). Subsequent Herceptin maintenance doses (weekly regimen: 2 mg/kg; three-weekly regimen 6 mg/kg respectively) should then be given (weekly regimen: every week; three-weekly regimen every 3 weeks) from that point.

Special patient populations

Clinical data show that the disposition of Herceptin is not altered based on age or serum creatinine In clinical trials, elderly patients did not receive reduced doses of Herceptin. Dedicated pharmacokinetic studies in the elderly and those with renal or hepatic impairment have not been carried out. However in a population pharmacokinetic analysis, age and renal impairment were not shown to affect trastuzumab disposition.

Method of administration

Herceptin loading dose should be administered as a 90-minute intravenous infusion. Do not administer as an intravenous push or bolus. Herceptin intravenous infusion should be administered by a health-care provider prepared to manage anaphylaxis and an emergency kit should be available. Patients should be observed for at least six hours after the start of the first infusion and for two hours after the start of the subsequent infusions for symptoms like fever and chills or other infusion-related symptoms (see sections 4.4 and 4.8). Interruption or slowing the rate of the infusion may help control such symptoms. The infusion may be resumed when symptoms abate.

If the initial loading dose was well tolerated, the subsequent doses can be administered as a 30-minute infusion.Pharmaceutical compositions of the invention may be administered parenterally.

Pharmaceutical compositions of the invention preferably comprise a pharmaceutically acceptable carrier. By "pharmaceutically acceptable carrier" is meant a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion. The administration of the herein provided compositions may, inter alia, comprise an administration twice daily, every day, every other day, every third day, every forth day, every fifth day, once a week, once every second week, once every third week, once every month, etc.

The pharmaceutical composition is also suitably administered by sustained release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Biopolymers 22:547-556 (1983)), poly (2-hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al., Id.) or poly-D-(-)-3-hydroxybutyric acid (EP 133,988). Sustained release pharmaceutical composition also include liposomally entrapped compound. Liposomes containing the pharmaceutical composition are prepared by methods known per se: DE 3,218,121; Epstein et al., Proc. Natl. Acad. Sci. (USA) 82:3688-3692 (1985); Hwang et al., Proc. Natl. Acad. Sci. (USA) 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal therapy.

For parenteral administration, the pharmaceutical composition is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation.

Generally, the formulations are prepared by contacting the components of the pharmaceutical composition uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) (poly)peptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The components of the pharmaceutical composition to be used for therapeutic administration must be sterile. Sterility is readily accomplished by filtration through sterile filtration membranes (e.g., 0.2 micron membranes). Therapeutic components of the pharmaceutical composition generally are placed into a container having a sterile access port, for example, an intravenous solution bag or vial having a stopper pierceable by a hypodermic injection needle.

The components of the pharmaceutical composition ordinarily will be stored in unit or multidose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized compound(s) using bacteriostatic Water-for-Injection.

The herein provided treatment of cancer comprising a the modulator of the HER2/neu (ErbB2) signaling pathway, an inhibitor of programmed death ligand 1 (PD-L1) and a chemotherapeutic agent (like taxol or a taxol derivative, such as dodetaxel (Taxotere®)) may be performed by way of the simultaneous, sequential or separate administration of the individual components of said treatment. For example, one or more of the modulator(s) of the HER2/neu (ErbB2) signaling pathway as defined herein (like Trastuzumab) may be administered simultaneously with one or more of the herein defined inhibitor(s) of programmed death ligand 1 (PD-L1) (like the herein provided and described anti-PD-L1 antibodies). Also sequential administration of the modulator(s) of the HER2/neu (ErbB2) signaling pathway as defined herein (like Trastuzumab) may be administered simultaneously with one or more of the herein defined inhibitor(s) of programmed death ligand 1 (PD-L1) (like the herein provided and described anti-PD-L1 antibodies) to be used in accordance with the present invention is envisaged herein. The herein defined modulators of the HER2/neu (ErbB2) signaling pathway as defined herein (like Trastuzumab) and the one or more of the herein defined inhibitor of programmed death ligand 1 (PD-L1) (like the herein provided and described anti-PD-L1 antibodies) may also be administered separately. For example, one or more of the modulator(s) of the HER2/neu (ErbB2) signaling pathway as defined herein (like Trastuzumab) may be administered in a first step followed by administration in a second step with one or more of the inhibitor(s) of programmed death ligand 1 (PD-L1) (like the herein provided and described anti-PD-L1 antibodies) and vice versa. Likewise, the chemotherapeutic agent may be administered simultaneously, sequentially or separately. Any combination of simultaneous, sequential or separate administration of the modulator(s) of the HER2/neu (ErbB2) signaling pathway, inhibitor(s) of programmed death ligand 1 (PD-L1) and chemotherapeutic agent(s) (like taxol or a taxol derivative, such as dodetaxel (Taxotere®)) is envisaged herien.

The herein provided treatment of cancer comprising a the modulator of the HER2/neu (ErbB2) signaling pathway, an inhibitor of programmed death ligand 1 (PD-L1) and a chemotherapeutic agent (like taxol or a taxol derivative, such as dodetaxel (Taxotere®)) can be applied as a sole therapy. It may, however, also be applied with one or more additional therapies (i.e. in a further cotherapy with), for example, conventional therapies like surgery, radiotherapy and/or one or more additional chemotherapeutic agents.

Surgery may comprise the step of partial or complete tumour resection, prior to, during or after the administration of the herein provided cancer treatment comprising a modulator of the HER2/neu (ErbB2) signaling pathway, an inhibitor of programmed death ligand 1 (PD-L1) and a chemotherapeutic agent (like taxol or a taxol derivative, such as dodetaxel (Taxotere®)). The herein provided modulator of the HER2/neu (ErbB2) signaling pathway, inhibitor of programmed death ligand 1 (PD-L1) and chemotherapeutic agent (like taxol or a taxol derivative, such as dodetaxel (Taxotere®)) may be administered in a neoadjuvant or adjuvant setting (in particular neoadjuvant or adjuvant treatment of cancer).

The modulator of the HER2/neu (ErbB2) signaling pathway, the chemotherapeutic agent and the inhibitor of programmed death ligand 1 (PD-L1) can be administered in a neoadjuvant setting. The modulator of the HER2/neu (ErbB2) signaling pathway, the chemotherapeutic agent and the inhibitor of programmed death ligand 1 (PD-L1) can be administered in an adjuvant setting or in a metastatic setting.

Accordingly, the herein provided modulator of the HER2/neu (ErbB2) signaling pathway, an inhibitor of programmed death ligand 1 (PD-L1) and a chemotherapeutic agent (like taxol or a taxol derivative, such as dodetaxel (Taxotere®)) may be administered to a patient in need of such a treatment during or after a surgical intervention/resection of the cancerous tissue. Therefore, the present invention is useful in neoadjuvant therapy, i.e. the treatment with the herein provided therapy given to a patient/patient group in need thereof prior to surgery. It is also useful in adjuvant therapy (i.e. after surgery).

The chemotherapeutic agent to be used herein is preferably a taxane (the term "taxol" is used interchangeably herein with "taxane") or a taxane derivate (taxol derivative), like dodetaxel (Taxotere®) or paclitaxel. The use of dodetaxel/(Taxotere®) is particularly preferred herein.

The (additional) chemotherapeutic agent(s) may be one or more of the following exemplary, non-limiting, drugs or agents:

Cisplatin, Vinorelbin, Carboplatin, Paclitaxel, Gemcitabin, Docetaxel, Bevacizumab, Pemetrexed, Etoposid, Irinotecan, Ifosfamid, Topotecan,
(an) anti-angiogenic agent(s) like a VEGF blocker (such as bevacizumab/Avastin or sutent (sunitinib malate-SU-11248)), linomide, inhibitors of integrin $\alpha v\beta 3$ function, angiostatin, razoxin, thalidomide, and including vascular targeting agents (for example combretastatin phosphate or N-acetylcolchinol-O-phosphate));
(an) cytostatic agent(s) such as antioestrogens (for example tamoxifen, toremifene, raloxifene, droloxifene, iodoxyfene), progestogens (for example megestrol acetate), aromatase inhibitors (for example anastrozole, letrazole, vorazole, exemestane), antiprogestogens, antiandrogens (for example flutamide, nilutamide, bicalutamide, cyproterone acetate), LHRH agonists and antagonists (for example goserelin acetate, luprolide), inhibitors of testosterone $5\alpha$-dihydroreductase (for example finasteride), anti-invasion agents (for example metalloproteinase inhibitors like marimastat and inhibitors of urokinase plasminogen activator receptor function) and inhibitors of growth factor function, (such growth factors include for example platelet derived growth factor and hepatocyte growth factor such inhibitors include growth factor antibodies, growth factor receptor antibodies, tyrosine kinase inhibitors and serine/threonine kinase inhibitors);
biological response modifiers (for example interferon); (an) anti-metabolite agent(s) (for example gemcitabine); (an) anti-hormonal compound(s) such as (an) anti-estrogen(s); antibodies (for example edrecolomab); adjuvant (anti-) hormonal therapy/therapies (i.e. therapy with (an) adjuvant (anti-) hormone drug(s), such as tamoxifen; gene therapy approaches (like antisense therapies); and/or immunotherapy approaches.

The chemotherapy may also (additionally) include the use of one or more of antiproliferative/antineoplastic drugs and combinations thereof, as used in medical oncology, such as (an) tyrosine kinase inhibitor(s), (a) raf inhibitor(s), (a) ras inhibitor(s), (a) dual tyrosine kinase inhibitor(s), taxol, (an) taxane(s) (like paclitaxel or docetaxel), (an) anthracycline(s), like doxorubicin or epirubicin, , aromatase inhibitors (such as anastrozole or letrozole) and/or vinorelbine; cyclophosphamide, methotrexate or fluorouracil (which is also known as 5-FU) can be used in such cotherapy individually or in form of a cotherapy comprising these three drugs ("CMF therapy"), optionally in combination with any of the other herein provided additional therapies. Particular examples of chemotherapeutic agents for use with a combination treatment of the present invention are pemetrexed, raltitrexed, etoposide, vinorelbine, paclitaxel, docetaxel, cisplatin, oxaliplatin, carboplatin, gemcitabine, irinotecan (CPT-1 1), 5-fluorouracil (5-FU, (including capecitabine)), doxorubicin, cyclophosphamide, temozolomide, hydroxyurea, (iii) antiproliferative/antineoplastic drugs and combinations thereof, as used in medical oncology, such as antimetabolites (for example antifolates like methotrexate, fluoropyrimidines like 5-fluorouracil, purine and adenosine analogues, cytosine arabinoside); antitumour antibiotics (for example anthracyclines like doxorubicin, daunomycin, epirubicin and idarubicin, mitomycin-C, dactinomycin, mithramycin); platinum derivatives (for example cisplatin, carboplatin); alkylating agents (for example nitrogen mustard, melphalan, chlorambucil, busulphan, cyclophosphamide, ifosfamide, nitrosoureas, thiotepa); antimitotic agents (for example vinca alkaloids like vincristine and taxoids like taxol, taxotere); topoisomerase inhibitors (for example epipodophyllotoxins like etoposide and teniposide, amsacrine, topotecan, and also irinotecan); also enzymes (for example asparaginase); and thymidylate synthase inhibitors (for example raltitrexed); and additional types of chemotherapeutic agents.

Inhibitors/Modulators/chemotherapeutic agents for use in accordance with the present invention are described herein and refer generally to known and/or commercially available Inhibitors/Modulators/chemotherapeutic. However, also the use of inhibitors yet to be generated or known compounds to be tested for their inhibiting activity is envisaged in context of the present invention.

In a further aspect, the present invention relates to the use of (a) nucleic acid(s) or antibody(antibodies) capable of detecting the expression level of ER, PD-L1 and, optionally, IPNγ for determining a patient's need for PD-L1 inhibitor cotherapy in combination with a modulator of the HER2/neu (ErbB2) signaling pathway and a chemotherapeutic agent. The respective explanations of said terms have been given above and apply here mutatis mutandis.

Preferably, the nucleic acid (e.g. oligonucleotide(s)) is (are) about 15 to 100 nucleotides in length. A person skilled in the art is, based on his general knowledge and the teaching provided herein, easily in the position to identify and/or prepare (a) an oligo- or polynucleotide capable of detecting the expression level of ER, PD-L1 and, optionally, IFNγ. In particular these nucleic acid(s) (e.g. oligo- or polynucleotides) may be used as probe(s) in the methods described herein, for example in the measurement of the expression level.. A skilled person will know, for example, computer programs which may be useful for the identification of corresponding probes to be used herein. For example, a nucleic acid encoding estrogen receptor (or a part of the nucleic acid) (e.g. SEQ ID NO: 38), a nucleic acid encoding PD-L1 (or a part of the nucleic acid) (e.g. SEQ ID NO: 42) and, optionally, a nucleic acid encoding IPNγ (or a part of the nucleic acid) (e.g. SEQ ID NO: 44 may be used in this context for identifying specific probes for detecting the expression level of ER, PD-L1 and IFNγ, respectively. Exemplary nucleic acid sequences encoding ER, PD-L1 and IPNγ are available on corresponding databases, such as the NCBI database (world wide web at ncbi.nlm.nih.gov/sites/entrez).

Furthermore, a composition is provided herein which is a diagnostic composition further comprising, optionally, means for detection/determining/evaluating the expression level of ER, PD-L1 and IFNγ. Such means for detection, are, for example, the above-described nucleotides and/or antibodies. Accordingly, the present invention relates to such means (e.g. such nucleotides and/or antibodies) for the preparation of a diagnostic composition for determining a patient in need of a PD-L1 inhibitor cotherapy.

In an alternative aspect, the present invention relates to such means for detection (e.g the above-described nucleic acids and/or antibodies and/or the "binding molecules" described below in context of the kit to be used in accordance with the present invention) for use in determining a patient in need of a PD-L1 inhibitor cotherapy. Preferably, the present invention relates to (an) antibody/antibodies for use in determining a patient in need of a PD-L1 inhibitor cotherapy.

Furthermore, the present invention also relates to a kit useful for carrying out the herein provided methods, the kit comprising (a) nucleic acid or (an) antibody capable of detecting the expression level of ER, PD-L1 and, optionally, IFNγ. Also envisaged herein is the use of the herein described kit for carrying out the herein provided methods. Said kit useful for carrying out the methods and uses described herein may comprise oligonucleotides or polynucleotides capable of determining the expression level of ER, PD-L1 and, optionally, IFNγ. For example, said kit may comprise (a) compound(s) required for specifically measuring the expression level of ER, PD-L1 and, optionally, IFNγ.. Moreover, the present invention also relates to the use of (a) compound(s) required for specifically measuring the expression level of ER, PD-L1 and, optionally, IFNγ, for the preparation of a kit for carrying out the methods or uses of this invention. On the basis of the teaching of this invention, the skilled person knows which compound(s) is (are) required for specifically measuring the expression level of ER, PD-L1 and, optionally, IFNγ. For example, such compound(s) may be (a) "binding molecule(s)". Particularly, such compound(s) may be (a) (nucleotide) probe(s), (a) primer(s) (pair(s)), (an) antibody(ies) and/or (an) aptamer(s) specific for a (gene) product of the ER gene/coding sequence, PD-L1 gene/coding sequence and, optionally, IFNγ/coding sequence. The kit (to be prepared in context) of this invention may be a diagnostic kit.

The kit (to be prepared in context) of this invention or the methods and uses of the invention may further comprise or be provided with (an) instruction manual(s). For example, said instruction manual(s) may guide the skilled person (how) to determine the (reference/control) expression level of ER, PD-L1 and, optionally, IFNγ. or (how) to determine a patient's need of PD-L1 inhibitor therapy. Particularly, said instruction manual(s) may comprise guidance to use or apply the herein provided methods or uses. The kit (to be prepared in context) of this invention may further comprise substances/chemicals and/or equipment suitable/required for carrying out the methods and uses of this invention. For example, such substances/chemicals and/or equipment are solvents, diluents and/or buffers for stabilizing and/or storing (a) compound(s) required for specifically measuring the expression level of ER, PD-L1 and, optionally, IFNγ.

As used herein, the terms "comprising" and "including" or grammatical variants thereof are to be taken as specifying the stated features, integers, steps or components but do not preclude the addition of one or more additional features, integers, steps, components or groups thereof. This term encompasses the terms "consisting of" and "consisting essentially of." Thus, the terms "comprising"/"including"/"having" mean that any further component (or likewise features, integers, steps and the like) can be present.

The term "consisting of' means that no further component (or likewise features, integers, steps and the like) can be present.

The term "consisting essentially of" or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed composition, device or method. Thus, the term "consisting essentially of" means that specific further components (or likewise features, integers, steps and the like) can be present, namely those not materially affecting the essential characteristics of the composition, device or method. In other words, the term "consisting essentially of" (which can be interchangeably used herein with the term "comprising substantially"), allows the presence of other components in the composition, device or method in addition to the mandatory components (or likewise features, integers, steps and the like), provided that the essential characteristics of the device or method are not materially affected by the presence of other components.

The term "method" refers to manners, means, techniques and procedures for accomplishing a given task including, but not limited to, those manners, means, techniques and procedures either known to, or readily developed from known manners, means, techniques and procedures by practitioners of the chemical, biological and biophysical arts.

As used herein, the term "isolated" refers to a composition that has been removed from its in-vivo location (e.g. aquatic organism or moss). Preferably the isolated compositions of the present invention are substantially free from other substances (e.g., other proteins that do not comprise anti-adhesive effects) that are present in their in-vivo location (i.e. purified or semipurified

As used herein the term "about" refers to ± 10%.

The present invention also relates to the following items:

1. A method of determining the need of a cancer patient for a PD-L1 inhibitor cotherapy, (i) wherein therapy comprising a modulator of the HER2/neu (ErbB2) signaling pathway and a chemotherapeutic agent is contemplated for the patient or (ii) wherein the patient is undergoing therapy comprising a modulator of the HER2/neu (ErbB2) signaling pathway and a chemotherapeutic agent, said method comprising the steps of

    a) measuring in vitro in a sample from said patient the expression level of Estrogen receptor (ER) and of programmed death ligand 1 (PD-L1),
    b) determining a patient as being in need of a PD-L1 inhibitor cotherapy if a low or absent ER expression level and an expression level of programmed death ligand 1 (PD-L1) that is increased in comparison to a control is measured in step (a).

2. A method of treating a cancer in a cancer patient for whom therapy comprising a modulator of the HER2/neu (ErbB2) signaling pathway and a chemotherapeutic agent is contemplated, the method comprising selecting a cancer patient whose cancer is determined to have a low or absent ER expression level and to have an increased expression level of programmed death ligand 1 (PD-L1) in comparison to a control, and administering to the patient an effective amount of a modulator of the HER2/neu (ErbB2) signaling pathway, of a chemotherapeutic agent and of a programmed death ligand 1 (PD-L1) inhibitor.

3. A method of treating a cancer in a cancer patient who is undergoing therapy comprising a modulator of the HER2/neu (ErbB2) signaling pathway and a chemotherapeutic agent, the method comprising selecting a cancer patient whose cancer is determined to have a low or absent ER expression level and to have an increased expression level of programmed death ligand 1 (PD-L1) in comparison to a control, and administering to the patient an effective amount of a programmed death ligand 1 (PD-L1) inhibitor.

4. A pharmaceutical composition comprising a modulator of the HER2/neu (ErbB2) signaling pathway, and an inhibitor of programmed death ligand 1 (PD-L1) for use in the treatment of cancer, whereby said cancer is determined to have a low or absent ER expression level and to have an increased expression level of programmed death ligand 1 (PD-L1) in comparison to a control.

5. The pharmaceutical composition for use in the treatment of canccer of item 4, further comprising a chemotherapeutic agent.

6. The method of any one of items 1 to 3, further comprising measuring in vitro in a sample from said patient the expression level of interferon-gamma (IFNγ) and determining a patient as being in need of a PD-L1 inhibitor cotherapy if an expression level of interferon-gamma (IFNγ) that is decreased in comparison to a control is measured.

7. The method of any one of items 1, 2, 3 and 6; or the pharmaceutical composition of item 4 and 5, wherein the ER expression level is ER(-).

8. The method of any one of items 1, 2, 3, 6 and 7; or the pharmaceutical composition of any one of item 4, 5 and 7, wherein said modulator of the HER2/neu (ErbB2) signaling pathway is the HER2 antibody Herceptin/Trastuzumab.

9. The method of any one of items 1, 2, 3, 6, 7 and 8; or the pharmaceutical composition of any one of items 5, 7 and 8, wherein said chemotherapeutic agent is taxol or a taxol derivative.

10. The method of any one of items 1, 2, 3, 6, 7 and 8 to 9; or the pharmaceutical composition of any one of items 4, 5 and 7 to 9, wherein said inhibitor of programmed death ligand 1 (PD-L1) is an antibody specifically binding to PD-L1 (anti-PD-L1 antibody).

11. The method of any one of items 1, 2, 3, and 6 to 10; or the pharmaceutical composition of any one of items 4, 5 and 7 to 10, wherein said cancer is a solid cancer.

12. The method of item 11; or the pharmaceutical composition of item 11, wherein said solid cancer is breast cancer orgastric cancer

13. The method of any one of items 1, 2, 3, and 6 to 12, ; or the pharmaceutical composition of any one of items 4, 5 and 7 to 12, wherein the expression level of PD-L1 is the mRNA expression level.

14. Use of a nucleic acid or antibody capable of detecting the expression level of ER, PD-L1 and, optionally, IFNγ for determining a patient's need for PD-L1 inhibitor cotherapy in combination with a modulator of the HER2/neu (ErbB2) signaling pathway and a chemotherapeutic agent.

15. The method of any one of items 1, 2, 3 and 6 to 14; or the pharmaceutical composition of any one of items 4, 5 and 7 to 14, wherein said modulator of the HER2/neu (ErbB2) signaling pathway, said chemotherapeutic agent and said inhibitor of programmed death ligand 1 (PD-L1) are to be administered in a neoadjuvant setting.

The present invention is further described by reference to the following non-limiting figures and examples. Unless otherwise indicated, established methods of recombinant gene technology were used as described, for example, in Sambrook, Russell "Molecular Cloning, A Laboratory Manual", Cold Spring Harbor Laboratory, N.Y. (2001)) which is incorporated herein by reference in its entirety.

The Figures show:

**Figure 1.**
Figure 1 provides a schematic of the HER2 protein structure, and amino acid sequences for Domains I-IV, respectively) of the extracellular domain thereof.

**Figure 2.**
Figures 2A and 2B depict alignments of the amino acid sequences of the variable light ($V_L$) (Fig. 2A) and variable heavy ($V_H$) (Fig. 2B) domains of murine monoclonal antibody 2C4 (SEQ ID Nos. 5 and 6, respectively); $V_L$ and $V_H$ domains of variant 574/Pertuzumab (SEQ ID Nos. 7 and 8, respectively), and human $V_L$ and $V_H$ consensus frameworks (hum κ1, light kappa subgroup I; humIII, heavy subgroup III) (SEQ ID Nos. 9 and 10, respectively). Asterisks identify differences between variable domains of Pertuzumab and murine monoclonal antibody 2C4 or between variable domains of Pertuzumab and the human framework. Complementarity Determining Regions (CDRs) are in brackets.

**Figure 3.**
Figures 3A and 3B show the amino acid sequences of Pertuzumab light chain (Fig. 3A) and heavy chain (Fig. 3B). CDRs are shown in bold. Calculated molecular mass of the light chain and heavy chain are 23,526.22 Da and 49,216.56 Da (cysteines in reduced form). The carbohydrate moiety is attached to Asn 299 of the heavy chain.

**Figure 4.**
Figures 4A and 4B show the amino acid sequences of Trastuzumab light chain (Fig. 4A) and heavy chain (Fig. 4B), respectively. Boundaries of the variable light and variable heavy domains are indicated by arrows.

**Figure 5.**

Figures 5A and 5B depict a variant Pertuzumab light chain sequence (Fig. 5A) and a variant Pertuzumab heavy chain sequence (Fig. 5B), respectively.

The Example illustrates the invention.

**Example 1: Cancer patients undergoing HER2 targeted therapy and chemotherapy benefit from PD-L1 inhibitor cotherapy, if the expression level of ER is low or absent (ER negative) and if PD-L1 expression level is increased**

Estimation of gene expression was performed with the help of R Bioconductor package 'affy', R version 2.15.0. All exploratory analyses and predictive models were made using SAS JMP ver. 10.0
48 HER2+, ER+ and 39 HER2+, ER- breast cancer biopsies were obtained from NeoSphere clinical trial. The samples had been taken at diagnosis from patients afterwards treated with Docetaxel and Trastuzumab in a neo-adjuvant setting. The distribution of main clinical covariates at base line, as well as of clinical response (as assessed at the surgery) in the involved population is as follows:

ER negative samples:

Patient Age (see **Figure 17**)

Quantiles

| | | |
|---|---|---|
| 100.0% | maximum | 72 |
| 99.5% | | 72 |
| 97.5% | | 71.55 |
| 90.0% | | 64 |
| 75.0% | quartile | 54 |
| 50.0% | median | 50.5 |
| 25.0% | quartile | 44.25 |
| 10.0% | | 39 |
| 2.5% | | 34.675 |
| 0.5% | | 34 |
| 0.0% | minimum | 34 |

Cancer Type

| Level | Count | Prob |
|---|---|---|
| IBC | 2 | 0.04167 |
| LABC | 22 | 0.45833 |
| OPERABLE | 24 | 0.50000 |
| Total | 48 | 1.00000 |

pT (pathologic staging of Tumor)

| Level | Count | Prob |
|---|---|---|
| T2 | 18 | 0.37500 |
| T3 | 15 | 0.31250 |
| T4 | 15 | 0.31250 |
| Total | 48 | 1.00000 |

pN (pathologic staging of nodes)

| Level | Count | Prob |
|---|---|---|
| N0 | 12 | 0.25000 |
| N1 | 36 | 0.75000 |
| Total | 48 | 1.00000 |

G (Grade)

| Level | Count | Prob |
|---|---|---|
| G1 | 1 | 0.02083 |
| G2 | 15 | 0.31250 |
| G3 | 16 | 0.33333 |
| NA | 16 | 0.33333 |
| Total | 48 | 1.00000 |

ER positive samples:

Patient Age (see **Figure 18**)

Quantiles

| | | |
|---|---|---|
| 100.0% | maximum | 74 |
| 99.5% | | 74 |
| 97.5% | | 74 |
| 90.0% | | 65 |
| 75.0% | quartile | 57 |
| 50.0% | median | 50 |
| 25.0% | quartile | 43 |
| 10.0% | | 40 |
| 2.5% | | 32 |
| 0.5% | | 32 |
| 0.0% | minimum | 32 |

Cancer Type

| Level | Count | Prob |
|---|---|---|
| IBC | 5 | 0.12821 |
| LABC | 8 | 0.20513 |
| OPERABLE | 26 | 0.66667 |
| Total | 39 | 1.00000 |

pT

| Level | Count | Prob |
|---|---|---|
| T2 | 15 | 0.38462 |
| T3 | 16 | 0.41026 |
| T4 | 8 | 0.20513 |
| Total | 39 | 1.00000 |

pN

| Level | Count | Prob |
|---|---|---|
| N0 | 11 | 0.28205 |
| N1 | 28 | 0.71795 |
| Total | 39 | 1.00000 |

G

| Level | Count | Prob |
|---|---|---|
| G2 | 13 | 0.33333 |
| G3 | 10 | 0.25641 |
| NA | 16 | 0.41026 |
| Total | 39 | 1.00000 |

Contingency Analysis of pathological complete response (pCR) By estrogen receptor status (ER)

| Count Row % | pCR = NO | pCR = YES | |
|---|---|---|---|
| ER = ER- | 27<br>56.25 | 21<br>43.75 | 48 |
| ER = ER+ | 33<br>84.62 | 6<br>15.38 | 39 |
| | 60 | 27 | 87 |

Gene Expression Profiling

The tumor biopsy samples were profiled for gene expression on AFFYMETRIX HG-U133Plus 2 whole Human Genome microarray platform. Roche HighPure RNA extraction, NuGen amplification and standard AFFYMETRIX hybridization and scanning protocols were used. All array scans passed standard AFFYMETRIX QC.

Robust Multiarray algorithm (RMA) was used for preprocessing of raw signals (Irizarry et al, 2003. http://www.ncbi.nlm.nih.gov/pubmed/12925520). All probe sets available for the genes of interest were retrieved as reported below. Fir gene CD274, when several probe sets were available to represent this gene, the probe set with the probe set with the highest average expression value (defined as an arithmetical average of expression of a given probe set) was selected to represent the gene:

*CD274 (PDL1)*

223834_at selected for PDL1

227458_at

The selected probe set corresponds to the last exon / 3'UTR of the gene and captures all known RefSEq mRNAs (see **Figure 6**)

*IFNG*

210354_at

This probe set also represents the last exon / 3'UTR of the gene and captures all known RefSEq mRNAs (see **Figure 7**)

**Figure 8** shows joint distribution of the expression of the above genes in the samples of both ER- and ER- populations. Symbol types correspond to the final pCR status (solid: pCR acheeved, open - pCR not achieved).

More details on distribution of CD274 and IFNG expression across ER and pCR strata can be found in **Appendix I.**

For every ER subpopulation, a logistic regression model was constructed that relates expression of the selected genes with clinical response adjusted for patient age, cancer type, and nodal status:

$$\text{Response} \sim \text{Patient.Age} + \text{Cancer.Type} + \text{pN} + \text{CD274} + \text{IFNG}$$

*1. ER- population.*

Summarized model output is given below. Odds ratios are (OR) provided per unit change of biomarker value. As the expression values are given on log2 scale, one unit change would correspond to 2-fold overexpression. For details see Appendix.

| Term | ER- population | |
|---|---|---|
| | OR (95% CI) | LR test p-value |
| CD274 | 5.2 (1.5 ; 26.7) | 0.008 |
| IFNG | 0.30 (0.10 ; 0.74) | 0.007 |
| Patient Age | | 0.24 |
| Cancer Type | | 0.91 |
| pN | | 0.87 |

The final model for predicting probability for a particular patient to respond to the treatment includes expression of CD274 and IFNG and looks like:

$$p(pCR) = -3.737 + 1.607{*}CD274 - 1.069{*}IFNG$$

*2. ER+ population.*

Summarized model output is given below. Odds ratios are (OR) provided per unit change of biomarker value. As the expression values are given on log2 scale, one unit change would correspond to 2-fold overexpression. For details see Appendix.

| | ER+ population | |
|---|---|---|
| Term | OR (95% CI) | LR test p-value |
| CD274 | | 0.93 |
| IFNG | | 0.23 |
| Patient Age | | 0.34 |
| Cancer Type | | 0.39 |
| pN | | 0.92 |

The role of PDL1 expression is evident in ER- subpopulation of HER2+ breast cancer patients that underwent combinational treatment with Trastuzumab and chemotherapy in the neoadjuvant setting. Namely, overexpression of PDL1 at diagnosis corresponds to a lower rate of response to neoadjuvant therapy (i.e. a lower rate of response to combinational treatment with Trastuzumab and chemotherapy). This holds irrespective of patient age, cancer type, or lymph node status. A baseline assessment of gene expression of either of the two biomarkers, PDL1 and INFG, respectively, allows to identify if a patient is likely to experience a greater benefit if a PDL-1 targeted therapy is added to Trastuzumab and chemotherapy.

The following relates to a cut-off value allowing determining a patient as being in need of a PD-L1 inhibitor cotherapy in accordance with the present invention.

If a gene expression analysis gives a result for IFNG expression higher or equal to 4.8 no combination treatment (HER2-targeted and PDL1-targeted) is recommended and no further PDL1 assessment would be necessary. If a gene expression analysis gives a result for IFNG lower than 4.8 a parallel assessment of PDL-1 is necessary. If PDL-1 gene expression analysis then gives a result of higher or equal to 5.3 a combination treatment (HER2-targeted and PDL1-targeted) is recommended (see **Figure 19**).

**Appendix I**

ER- subpopulation

*Oneway Analysis of CD274 Expression By pCR ER=ERneg (see Figure 9 A)*

*t Test*

YES-NO

Assuming unequal variances

| | | | |
|---|---|---|---|
| Difference | -0.32948 | t Ratio | -1.94171 |
| Std Err Dif | 0.16969 | DF | 45.11513 |
| Upper CL Dif | 0.01226 | Prob > \|t\| | 0.0584 |
| Lower CL Dif | -0.67122 | Prob > t | 0.9708 |
| Confidence | 0.95 | Prob < t | 0.0292* |

The results are also shown in **Figure 9B.**

*Oneway Analysis of IFNG Expression By pCR ER=ERneg*

The results are shown in **Figure 10A.**

*t Test*

YES-NO

Assuming unequal variances

| | | | |
|---|---|---|---|
| Difference | 0.58405 | t Ratio | 2.044225 |
| Std Err Dif | 0.28571 | DF | 30.21429 |
| Upper CL Dif | 1.16737 | Prob > \|t\| | 0.0497* |
| Lower CL Dif | 0.00073 | Prob > t | 0.0249* |
| Confidence | 0.95 | Prob < t | 0.9751 |

The results are shown in **Figure 10B.**

ER+ subpopulation

*Oneway Analysis of CD274 Expression By pCR ER=ERpos*

The results are shown in **Figure 11A.**

*t Test*

YES-NO

Assuming unequal variances

| | | | |
|---|---|---|---|
| Difference | 0.25169 | t Ratio | 0.898709 |
| Std Err Dif | 0.28006 | DF | 6.542171 |
| Upper CL Dif | 0.92345 | Prob > \|t\| | 0.4007 |
| Lower CL Dif | -0.42006. | Prob > t | 0.2003 |
| Confidence | 0.95 | Prob < t | 0.7997 |

The results are shown in **Figure 11B.**

*Oneway Analysis of IFNG Expression By pCR ER=ERpos*

The results are shown in **Figure 12A.**

*t Test*

YES-NO

Assuming unequal variances

| | | | |
|---|---|---|---|
| Difference | 0.5931 | t Ratio | 1.501336 |
| Std Err Dif | 0.3951 | DF | 7.109044 |
| Upper CL Dif | 1.5244 | Prob > \|t\| | 0.1763 |
| Lower CL Dif | -0.3382 | Prob > t | 0.0882 |
| Confidence | 0.95 | Prob < t | 0.9118 |

The results are shown in **Figure 12B.**

## Appendix II

Nominal Logistic Fit for pCR ER=ERneg
Converged in Gradient, 5 iterations

Whole Model Test

| Model | -LogLikelihood | DF | ChiSquare | Prob>ChiSq |
|---|---|---|---|---|
| Difference | 6.784783 | 6 | 13.56957 | 0.0348* |
| Full | 26.110299 | | | |
| Reduced | 32.895082 | | | |

| | |
|---|---|
| RSquare (U) | 0.2063 |
| AICc | 69.0206 |
| BIC | 79.319 |
| Observations (or Sum Wgts) | 48 |

| Measure | Training | Definition |
|---|---|---|
| Entropy RSquare | 0.2063 | 1-Loglike(model)/Loglike(0) |
| Generalized RSquare | 0.3301 | (1-(L(0)/L(model))^(2/n))/(1-L(0)^(2/n)) |
| Mean -Log p | 0.5440 | ∑-Log(ρ[j])/n |
| RMSE | 0.4278 | $\sqrt{\sum(y[j]-\rho[j])^2/n}$ |
| Mean Abs Dev | 0.3665 | ∑ \|y[j]-ρ[j]\|/n |
| Misclassification Rate | 0.2292 | ∑(ρ[j]≠ρMax)/n |
| N | 48 | n |

Lack Of Fit

| Source | DF | -LogLikelihood | ChiSquare |
|---|---|---|---|
| Lack Of Fit | 41 | 26.110299 | 52.2206 |
| Saturated | 47 | 0.000000 | Prob>ChiSq |
| Fitted | 6 | 26.110299 | 0.1125 |

Parameter Estimates

| Term | Estimate | Std Error | ChiSquare | Prob>ChiS q | Lower 95% | Upper 95% |
|---|---|---|---|---|---|---|
| Intercept | - 5.9688255 | 4.1632695 | 2.06 | 0.1517 | -15.115329 | 1.70408281 |
| Patient Age | 0.04906238 | 0.0425045 | 1.33 | 0.2484 | -0.0324034 | 0.13829525 |
| Cancer Type[IBC] | - 0.0943023 | 1.0982289 | 0.01 | 0.9316 | -2.5407977 | 2.23824618 |
| Cancer Type[LABC] | - 0.1514945 | 0.6544424 | 0.05 | 0.8169 | -1.5051269 | 1.21757158 |
| pN[N0] | 0.08157636 | 0.4979574 | 0.03 | 0.8699 | -0.8986622 | 1.09707358 |
| CD274 Expression | 1.64979222 | 0.7194762 | 5.26 | 0.0218* | 0.39533833 | 3.2836052 |
| IFNG Expression | - 1.1882978 | 0.5122023 | 5.38 | 0.0203* | -2.3323039 | -0.2889168 |

For log odds of NO/YES

Effect Likelihood Ratio Tests

| Source | Nparm | DF | L-R ChiSquare | Prob>ChiSq |
|---|---|---|---|---|
| Patient Age | 1 | 1 | 1.38574446 | 0.2391 |
| Cancer Type | 2 | 2 | 0.19781033 | 0.9058 |
| pN | 1 | 1 | 0.02690704 | 0.8697 |
| CD274 Expression | 1 | 1 | 7.09800433 | 0.0077* |
| IFNG Expression | 1 | 1 | 7.15387723 | 0.0075* |

Odds Ratios

For pCR odds of NO versus YES

Tests and confidence intervals on odds ratios are likelihood ratio based.

Unit Odds Ratios

Per unit change in regressor

| Term | Odds Ratio | Lower 95% | Upper 95% | Reciprocal |
|---|---|---|---|---|
| Patient Age | 1.050286 | 0.968116 | 1.148315 | 0.9521217 |
| CD274 Expression | 5.205898 | 1.484886 | 26.67176 | 0.1920898 |
| IFNG Expression | 0.30474 | 0.097072 | 0.749074 | 3.2814908 |

Odds Ratios for Cancer Type

| Level1 | /Level2 | Odds Ratio | Prob>Chisq | Lower 95% | Upper 95% |
|---|---|---|---|---|---|
| LABC | IBC | 0.9444125 | 0.9722 | 0.0282989 | 35.902054 |
| OPERABLE | IBC | 1.405087 | 0.8471 | 0.0357479 | 68.159191 |
| OPERABLE | LABC | 1.4877895 | 0.6568 | 0.2518769 | 9.0216463 |
| IBC | LABC | 1.0588593 | 0.9722 | 0.0278536 | 35.337072 |
| IBC | OPERABLE | 0.7116997 | 0.8471 | 0.0146715 | 27.973694 |
| LABC | OPERABLE | 0.6721381 | 0.6568 | 0.1108445 | 3.9701934 |

Odds Ratios for pN

| Level1 | /Level2 | Odds Ratio | Prob>Chisq | Lower 95% | Upper 95% |
|---|---|---|---|---|---|
| N1 | N0 | 0.8494615 | 0.8697 | 0.1114536 | 6.033483 |
| N0 | N1 | 1.1772165 | 0.8697 | 0.1657417 | 8.9723459 |

Receiver Operating Characteristic
(see **Figure 13**)
Using pCR='YES' to be the positive level

AUC

0.79718

Confusion Matrix
Actual
Predicted

| Training | NO | YES |
|---|---|---|
| NO | 22 | 5 |
| YES | 6 | 15 |

Lift Curve
(see **Figure 14**)
pCR

- NO

- YES

Prediction Profiler
(see **Figure 15**)
Nominal Logistic Fit for pCR ER=ERpos
Converged in Gradient, 19 iterations

Whole Model Test

| Model | -LogLikelihood | DF | ChiSquare | Prob>ChiSq |
|---|---|---|---|---|
| Difference | 2.400597 | 6 | 4.801193 | 0.5696 |
| Full | 14.343001 | | | |
| Reduced | 16.743598 | | | |

| | |
|---|---|
| RSquare (U) | 0.1434 |
| AICc | 46.2989 |
| BIC | 54.3309 |
| Observations (or Sum Wgts) | 39 |

| Measure | Training | Definition |
|---|---|---|
| Entropy RSquare | 0.1434 | 1-Loglike(model)/Loglike(0) |
| Generalized RSquare | 0.2010 | (1-(L(0)/L(model))^(2/n))/(1-L(0)^(2/n)) |
| Mean -Log p | 0.3678 | $\sum$-Log($\rho$[j])/n |
| RMSE | 0.3462 | $\sqrt{\sum(y[j]-\rho[j])^2}$/n |
| Mean Abs Dev | 0.2351 | $\sum$\|y[j]-$\rho$[j]\|/n |
| Misclassification Rate | 0.1795 | $\sum(\rho[j]\neq\rho Max)$/n |
| N | 39 | n |

Lack Of Fit

| Source | DF | -LogLikelihood | ChiSquare |
|---|---|---|---|
| Lack Of Fit | 32 | 14.343001 | 28.686 |
| Saturated | 38 | 0.000000 | Prob>ChiSq |
| Fitted | 6 | 14.343001 | 0.6351 |

Parameter Estimates

| Term | | Estimate | Std Error | ChiSquare | Prob>Chi Sq | Lower 95% | Upper 95% |
|---|---|---|---|---|---|---|---|
| Intercept | Unstable | 7.20306909 | 3597.5107 | 0.00 | 0.9984 | - 7043.7884 | 7058.1945 |
| Patient Age | | 0.0578149 | 0.0628112 | 0.85 | 0.3573 | - 0.0560483 | 0.19608254 |
| Cancer Type[IBC] | Unstable | 12.0092513 | 7195.0139 | 0.00 | 0.9987 | - 14089.959 | 14113.9773 |
| Cancer Type [LABC] | Unstable | -6.5864683 | 3597.507 | 0.00 | 0.9985 | - 7057.5706 | 7044.39766 |
| pN[N0] | | -0.0542869 | 0.5572904 | 0.01 | 0.9224 | - 1.1698378 | 1.117206 |
| CD274 Expression | | 0.08485271 | 0.9859164 | 0.01 | 0.9314 | - 1.8704698 | 2.14104768 |
| IFNG Expression | | -0.7334678 | 0.6191817 | 1.40 | 0.2362 | - 2.0476903 | 0.45985303 |

For log odds of NO/YES

Effect Likelihood Ratio Tests

| Source | Nparm | DF | L-R ChiSquare | Prob>ChiSq |
|---|---|---|---|---|
| Patient Age | 1 | 1 | 0.92588732 | 0.3359 |
| Cancer Type | 2 | 2 | 1.89140212 | 0.3884 |
| pN | 1 | 1 | 0.00946444 | 0.9225 |
| CD274 Expression | 1 | 1 | 0.00742213 | 0.9313 |
| IFNG Expression | 1 | 1 | 1.45693945 | 0.2274 |

Odds Ratios

For pCR odds of NO versus YES

Tests and confidence intervals on odds ratios are likelihood ratio based.

Unit Odds Ratios

Per unit change in regressor

| Term | Odds Ratio | Lower 95% | Upper 95% | Reciprocal |
|---|---|---|---|---|
| Patient Age | 1.059519 | 0.945493 | 1.216627 | 0.9438246 |
| CD274 Expression | 1.088557 | 0.154051 | 8.508347 | 0.9186476 |
| IFNG Expression | 0.480241 | 0.129033 | 1.583841 | 2.0822891 |

Odds Ratios for Cancer Type

| Level1 | /Level2 | Odds Ratio | Prob>Chisq | Lower 95% | Upper 95% |
|---|---|---|---|---|---|
| LABC | IBC | 8.3942e-9 | 0.2128 | 0 | 5.1523961 |
| OPERABLE | IBC | 2.6876e-8 | 0.4499 | 0 | 20.868673 |
| OPERABLE | LABC | 3.2017112 | 0.3193 | 0.2999262 | 36.429388 |
| IBC | LABC | 119129251 | 0.2128 | 0.1940845 | . |
| IBC | OPERABLE | 37207993 | 0.4499 | 0.0479187 | . |
| LABC | OPERABLE | 0.312333 | 0.3193 | 0.0274504 | 3.3341535 |

Odds Ratios for pN

| Level1 | /Level2 | Odds Ratio | Prob>Chisq | Lower 95% | Upper 95% |
|---|---|---|---|---|---|
| N1 | N0 | 1.1146872 | 0.9225 | 0.1070551 | 10.377869 |
| NO | N1 | 0.8971126 | 0.9225 | 0.0963589 | 9.3409878 |

Receiver Operating Characteristic
(see **Figure 16**)
Using pCR='YES' to be the positive level

AUC

0.77273

Confusion Matrix
Actual
Predicted

| Training | NO | YES |
|---|---|---|
| NO | 32 | 1 |
| YES | 6 | 0 |

The present invention refers to the following nucleotide and amino acid sequences:
The sequences provided herein are, inter alia, available in the NCBI database and disclosed in WO 2010/077634 and can be retrieved from world wide web at ncbi.nlm.nih.gov/sites/entrez?db=gene; Theses sequences also relate to annotated and modified sequences. The present invention also provides techniques and methods wherein homologous sequences, and variants of the concise sequences provided herein are used.

SEQ ID NO:s 1-21 define the anti-PD-Ll antibody to be used in accordance with the present invention. SEQ ID NO:s 1-21 are shown in the sequence listing.

SEQ ID No. 22 to 37 show sequences of amino acid sequences for Domains I-IV of the HER2 protein (SEQ ID NO. 22-25, see also **Figure 1**) and sequences of anti-HER2-antibodies. (SEQ ID No. 26 to 37; see also **Figures 2-5**).

SEQ ID No. 26:
Amino acid sequence of the variable light ($V_L$) (Fig. 2A) domain of murine monoclonal antibody 2C4 (SEQ ID Nos. 5 and 6, respectively) as shown in Figure 2.

SEQ ID No. 27:
Amino acid sequence of the variable heavy ($V_H$) (Fig. 2B) domain of murine monoclonal antibody 2C4 as shown in Figure 2.

SEQ ID No. 28:
Amino acid sequence of the variable light ($V_L$) (Fig. 2A) domain of variant 574/Pertuzumab as shown in Figure 2.

SEQ ID No. 29:
Amino acid sequence of the variable heavy ($V_H$) (Fig. 2B) domain of variant 574/Pertuzumab as shown in Figure 2.

SEQ ID No. 30:
human $V_L$ consensus frameworks (hum κ1, light kappa subgroup I; humIII, heavy subgroup III) as shown in Figure 2.

SEQ ID No. 31:
human $V_H$ consensus frameworks (hum κ1, light kappa subgroup I; humIII, heavy subgroup III) as shown in Figure 2.

SEQ ID No. 32:
Amino acid sequences of Pertuzumab light chain as shown in Figure 3A.

SEQ ID No. 33:
Amino acid sequences of Pertuzumab heavy chain as shown in Figure 3B.

SEQ ID No. 34:
Amino acid sequence of Trastuzumab light chain domain as shown in Fig. 4A. Boundaries of the variable light domain are indicated by arrows.

SEQ ID No. 35:
Amino acid sequence of Trastuzumab heavy chain as shown in Fig. 4B. Boundaries of the variable heavy domain are indicated by arrows.

SEQ ID No. 36:
Amino acid sequence of variant Pertuzumab light chain sequence (Fig. 5A).

SEQ ID No. 37:
Amino acid sequence of variant Pertuzumab heavy chain sequence (Fig. 5B).

SEQ ID NO. 38
Nucleotide sequence encoding homo sapiens Progesterone Receptor (PR)
NCBI Reference Sequence: NC_000011.9
>gi|224589802:c101000544-100900355 Homo sapiens chromosome 11, GRCh37.p10 Primary Assembly

SEQ ID No. 39:
Amino acid sequence of homo sapiens Progesterone Receptor (PR)
PRGR_HUMAN Length: 933 December 07, 2012 15:10 Type: P Check: 6067 ..

SEQ ID NO. 40:
Nucleotide sequence encoding homo sapiens Estrogen Receptor (ER)
(NM_000125.3)

SEQ ID NO. 41:
Nucleotide sequence encoding homo sapiens Estrogen Receptor (ER)
NCBI Reference Sequence: NC_000006.11
>gi|224589818:152011631-152424409 Homo sapiens chromosome 6, GRCh37.p10 Primary Assembly

SEQ ID No. 42:
Amino acid sequence of homo sapiens Estrogen Receptor (ER)
>ENST00000206249_6

SEQ ID No. 43:
Nucleotide sequence encoding homo sapiens programmed death ligand 1(PD-L1)
NCBI Reference Sequence: NC_000009.11
>gi|224589821:5450503-5470567 Homo sapiens chromosome 9, GRCh37.p10 Primary Assembly

SEQ ID NO. 44
Nucleotide sequence encoding homo sapiens programmed death ligand 1(PD-L1) (CD274), transcript variant 1, mRNA
NCBI Reference Sequence: NM_014143.3
>gi|292658763|ref|NM_014143.3| Homo sapiens CD274 molecule (CD274), transcript variant 1, mRNA

SEQ ID No.45:
Amino acid sequence of homo sapiens programmed death ligand 1(PD-L1) (programmed cell death 1 ligand 1 isoform a precursor [Homo sapiens])
NCBI Reference Sequence: NP_054862.1
>gi|7661534|ref|NP_054862.1| programmed cell death 1 ligand 1 isoform a precursor [Homo sapiens]

SEQ ID No. 46:
Nucleotide sequence encoding homo sapiens programmed death ligand 1(PD-L1) (CD274), transcript variant 2, mRNA
NCBI Reference Sequence: NM_001267706.1
>gi|390979638|ref|NM_001267706.1| Homo sapiens CD274 molecule (CD274), transcript variant 2, mRNA

SEQ ID No. 47:
Amino acid sequence of homo sapiens programmed death ligand 1(PD-L1) (programmed cell death 1 ligand 1 isoform b precursor [Homo sapiens])
NCBI Reference Sequence: NP_001254635.1
>gi|390979639|ref|NP_001254635.1| programmed cell death 1 ligand 1 isoform b precursor [Homo sapiens]

SEQ ID No. 48:
Nucleotide sequence encoding homo sapiens programmed death ligand 1(PD-L1) (Homo sapiens CD274 molecule (CD274), transcript variant 3, non-coding RNA)
NCBI Reference Sequence: NR_052005.1
>gi|390979640|ref|NR_052005.1| Homo sapiens CD274 molecule (CD274), transcript variant 3, non-coding RNA

SEQ ID No. 49:
Nucleotide sequence encoding homo sapiens interferon gamma (Homo sapiens chromosome 12, GRCh37.p10 Primary Assembly)
NCBI Reference Sequence: NC_000012.11
>gi|224589803:c68553521-68548550 Homo sapiens chromosome 12, GRCh37.p10 Primary Assembly

SEQ ID No. 50:
Nucleotide sequence encoding homo sapiens interferon gamma, mRNA
NCBI Reference Sequence: NM_000619.2
>gi|56786137|ref|NM_000619.2| Homo sapiens interferon, gamma (IFNG), mRNA

SEQ ID No. 51:
Amino acid sequence of homo sapiens interferon gamma, interferon gamma precursor [Homo sapiens]
NCBI Reference Sequence: NP_000610.2
>gi|56786138|ref|NP_000610.2| interferon gamma precursor [Homo sapiens]

All references cited herein are fully incorporated by reference. Having now fully described the invention, it will be understood by a person skilled in the art that the invention may be practiced within a wide and equivalent range of conditions, parameters and the like, without affecting the spirit or scope of the invention or any embodiment thereof.
The invention further discloses the following items:

1. A method of determining the need of a cancer patient for a PD-L1 inhibitor cotherapy, (i) wherein therapy comprising a modulator of the HER2/neu (ErbB2) signaling pathway and a chemotherapeutic agent is contemplated for the patient or (ii) wherein the patient is undergoing therapy comprising a modulator of the HER2/neu (ErbB2) signaling pathway and a chemotherapeutic agent, the method comprising the steps of

a) measuring in vitro in a sample from said patient the expression level of Estrogen receptor (ER) and of programmed death ligand 1 (PD-L1),
b) determining a patient as being in need of a PD-L1 inhibitor cotherapy if a low or absent ER expression level and an expression level of programmed death ligand 1 (PD-L1) that is increased in comparison to a control is measured in step (a).

2. A method of treating a cancer in a cancer patient for whom therapy comprising a modulator of the HER2/neu (ErbB2) signaling pathway and a chemotherapeutic agent is contemplated, the method comprising selecting a cancer patient whose cancer is determined to have a low or absent ER expression level and to have an increased expression level of programmed death ligand 1 (PD-L1) in comparison to a control, and administering to the patient an effective amount of a modulator of the HER2/neu (ErbB2) signaling pathway, of a chemotherapeutic agent and of a programmed death ligand 1 (PD-L1) inhibitor.

3. A method of treating a cancer in a cancer patient who is undergoing therapy comprising a modulator of the HER2/neu (ErbB2) signaling pathway and a chemotherapeutic agent, the method comprising selecting a cancer patient whose cancer is determined to have a low or absent ER expression level and to have an increased expression level of programmed death ligand 1 (PD-L1) in comparison to a control, and administering to the patient an effective amount of a programmed death ligand 1 (PD-L1) inhibitor.

4. A pharmaceutical composition comprising a modulator of the HER2/neu (ErbB2) signaling pathway, and an inhibitor of programmed death ligand 1 (PD-L1) for use in the treatment of cancer, whereby said cancer is determined to have a low or absent ER expression level and to have an increased expression level of programmed death ligand 1 (PD-L1) in comparison to a control.

5. The pharmaceutical composition for use in the treatment of cancer of item 4, further comprising a chemotherapeutic agent.

6. The method of any one of items 1 to 3, further comprising measuring in vitro in a sample from said patient the expression level of interferon-gamma (IFNγ) and determining a patient as being in need of a PD-L1 inhibitor cotherapy if an expression level of interferon-gamma (IFNγ) that is decreased in comparison to a control is measured.

7. The pharmaceutical composition of item 4 or 5, whereby said cancer is determined to have a decreased expression level of interferon-gamma (IFNγ) in comparison to the control.

8. The method of any one of items 1, 2, 3 and 6; or the pharmaceutical composition of any one of items 4, 5 and 7, wherein the ER expression level is ER(-).

9. The method of any one of items 1, 2, 3, 6 and 8; or the pharmaceutical composition of any one of items 4, 5, 7 and 8, wherein said modulator of the HER2/neu (ErbB2) signaling pathway is an inhibitor of HER shedding.

10. The method of item 9, or the pharmaceutical composition of item 9, wherein said inhibitor of HER shedding is a HER2 shedding inhibitor.

11. The method of item 9 or 10, or the pharmaceutical composition of item 9 or 10, wherein said inhibitor of HER shedding inhibits HER heterodimerization or HER homodimerization.

12. The method of any one of items 9 to 11; or the pharmaceutical composition of any one of items 9 to 11, wherein said inhibitor of HER shedding is a HER antibody.

13. The method of item 12; or the pharmaceutical composition of item 12, wherein said HER antibody binds to a HER receptor selected from the group consisting of EGFR, HER2 and HER3.

14. The method of item 13; or the pharmaceutical composition of item 13, wherein said antibody binds to HER2.

15. The method of item 14; or the pharmaceutical composition of item 14, wherein said HER2 antibody binds to sub-domain IV of the HER2 extracellular domain.

16. The method of any one of items 12 to 15; or the pharmaceutical composition of any one of item 12 to 15, wherein said HER2 antibody is Herceptin/Trastuzumab.

17. The method of any one of items 1, 2, 3, 6 and 8; or the pharmaceutical composition of any one of items 4, 5, 7 and 8, wherein said modulator of the HER2/neu (ErbB2) signaling pathway is a HER dimerization/signaling inhibitor.

18. The method of item 17; or the pharmaceutical composition of item 17, wherein said HER dimerization inhibitor is a HER2 dimerization inhibitor.

19. The method of item 17 or 18; or the pharmaceutical composition of item 17 or 18, wherein said HER dimerization inhibitor inhibits HER heterodimerization or HER homodimerization.

20. The method of any one of items 17 to 19; or the pharmaceutical composition of any one of items 17 to 19, wherein said HER dimerization inhibitor is a anti HER antibody.

21. The method of item 20; or the pharmaceutical composition of item 20, wherein said HER antibody binds to a HER receptor selected from the group consisting of EGFR, HER2 and HER3.

22. The method of item 21; or the pharmaceutical composition of item 21, wherein said antibody binds to HER2.

23. The method of item 22 or the pharmaceutical composition of item 22, wherein said anti HER2 antibody binds to domain II of HER2 extracellular domain.

24. The method of item 23; or the pharmaceutical composition of item 23, wherein said antibody binds to a junction between domains I, II and III of HER2 extracellular domain.

25. The method of any one of items 20 to 24; or the pharmaceutical composition of any one of items 20 to 24, wherein said anti HER2 antibody is Pertuzumab.

26. The method of any one of items 1, 2, 3, 6 and 8 to 25; or the pharmaceutical composition of any one of items 5 and 7 to 25, wherein said chemotherapeutic agent is taxol or a taxol derivative.

27. The method of item 26; or the pharmaceutical composition of item 26, wherein said taxol derivative is dodetaxel.

28. The method of any one of items 1, 2, 3, 6 and 8 to 27; or the pharmaceutical composition of any one of items 4, 5 and 7 to 27, wherein said inhibitor of programmed death ligand 1 (PD-L1) is an antibody specifically binding to PD-L1 (anti-PD-Ll antibody).

29. The method of item 28; or the pharmaceutical composition of item 28, wherein said antibody comprises an heavy chain variable region polypeptide comprising an HVR-H1, HVR-H2 and HVR-H3 sequence, wherein:

(a) the HVR-H1 sequence is GFTFSX1SWIH (SEQ ID NO:1);
(b) the HVR-H2 sequence is AWIX2PYGGSX3YYADSVKG (SEQ ID NO:2);
(c) the HVR-H3 sequence is RHWPGGFDY (SEQ ID NO:3); further wherein: X1 is D or G; X2 is S or L; X3 is T or S.

30. The method of item 29; or the pharmaceutical composition of item 29, wherein X1 is D; X2 is S and X3 is T.

31. The method of item 29; or the pharmaceutical composition of item 29, wherein said polypeptide further comprises variable region heavy chain framework sequences juxtaposed between the HVRs according to the formula: (HC-FR1)-(HVR-H1)-(HC-FR2)-(HVR-H2)-(HC-FR3)-(HVR-H3)-(HC-FR4).

32. The method of item 31; or the pharmaceutical composition of item 31, wherein the framework sequences are derived from human consensus framework sequences.

33. The method of item 32; or the pharmaceutical composition of item 32, wherein the framework sequences are VH subgroup III consensus framework.

34. The method of item 33; or the pharmaceutical composition of item 33, wherein one or more of the framework sequences is the following: HC-FR1 is EVQLVESGGGLVQPGGSLRLSCAAS (SEQ ID NO:4) HC-FR2 is WVRQAPGKGLEWV (SEQ ID NO:5) HC-FR3 is RFTISADTSKNTAYLQMNSLRAEDTAVYYCAR (SEQ ID NO:6) HC-FR4 is WGQGTLVTVSA (SEQ ID NO:7).

35. The method of item 29; or the pharmaceutical composition of item 29, wherein said heavy chain polypeptide is in combination with a variable region light chain comprising an HVR-L1, HVR-L2 and HVR-L3, wherein:

(a) the HVR-L1 sequence is RASQX4X5X6TX7X8A (SEQ ID NOs:8);
(b) the HVR-L2 sequence is SASX9LX10S, and (SEQ ID NOs:9);
(c) the HVR-L3 sequence is QQX11X12X13X14PX15T (SEQ ID NOs:10);

further wherein: X4 is D or V; X5 is V or I; X6 is S or N; X7 is A or F; X8 is V or L; X9 is F or T; X10 is Y or A; X11 is Y, G, F, or S; X12 is L, Y, F or W; X13 is Y, N, A, T, G, F or I; X14 is H, V, P, T or I; X15 is A, W, R, P or T.

36. The method of item 35; or the pharmaceutical composition of item 35, wherein X4 is D; X5 is V; X6 is S; X7 is A; X8 is V; X9 is F; X10 is Y; X11 is Y; X12 is L; X13 is Y; X14 is H; X15 is A.

37. The method of item 35; or the pharmaceutical composition of item 35, wherein said polypeptide further comprises variable region light chain framework sequences juxtaposed between the HVRs according to the formula: (LC-FR1)-(HVR-L1)-(LC-FR2)-(HVR-L2)-(LC-FR3)-(HVR-L3)-(LC-FR4).

38. The method of item 37; or the pharmaceutical composition of item 37 wherein the framework sequences are derived from human consensus framework sequences.

39. The method of item 37; or the pharmaceutical composition of item 37, wherein the framework sequences are VL kappa I consensus framework.

40. The method of item 39; or the pharmaceutical composition of item 39, wherein one or more of the framework sequences is the following:

LC-FR1 is DIQMTQSPSSLSASVGDRVTITC (SEQ ID NO:11);
LC-FR2 is WYQQKPGKAPKLLIY (SEQ ID NO:12);
LC-FR3 is GVPSRFSGSGSGTDFTLTISSLQPEDFATYYC (SEQ ID NO:13);
LC-FR4 is FGQGTKVEIKR (SEQ ID NO:14).

41. The method of item 29; or the pharmaceutical composition of item 29, wherein said anti-PD-Ll antibody comprises a heavy chain and a light chain variable region sequence, wherein:

(a) the heavy chain comprises an HVR-H1, HVR-H2 and HVR-H3, wherein further:

(i) the HVR-H1 sequence is GFTFSX1SWIH (SEQ ID NO:1);
(ii) the HVR-H2 sequence is AWIX2PYGGSX3YYADSVKG (SEQ ID NO:2);
(iii) the HVR-H3 sequence is RHWPGGFDY, and (SEQ ID NO:3);

(b) the light chain comprises an HVR-L1, HVR-L2 and HVR-L3, wherein further:

(iv) the HVR-L1 sequence is RASQX4X5X6TX7X8A (SEQ ID NOs:8);
(v) the HVR-L2 sequence is SASX9LX10S (SEQ ID NOs:9);
(vi) the HVR-L3 sequence is QQX11X12X13X14PX15T (SEQ ID N0s:10);

wherein: X1 is D or G; X2 is S or L; X3 is T or S; X4 may be D or V; X5 may be V or I; X6 may be S or N; X7 may be A or F; X8 may be V or L; X9 may be F or T; X10 may be Y or A; X11 may be Y, G, F, or S; X12 may be L, Y, F or W; X13 may be Y, N, A, T, G, F or I; X14 may be H, V, P, T or I; X15 may be A, W, R, P or T.

42. The method of item 41; or the pharmaceutical composition of item 41, wherein X1 is D; X2 is S and X3 is T.

43. The method of item 41; or the pharmaceutical composition of item 41, wherein X4 = D, X5 = V, X6 = S, X7 = A and X8 = V, X9 = F, and X10 = Y, X11 = Y, X12 = L, X13 = Y, X14 = H and X15 = A.

44. The method of item 41; or the pharmaceutical composition of item 41, wherein X1 = D, X2 = S and X3 = T, X4 = D, X5 = V, X6 = S, X7 = A and X8 = V, X9 = F, and X10 = Y, X11 = Y, X12 = L, X13 = Y, X14 = Hand X15 = A.

45. The method of any one of items 41 to 44; or the pharmaceutical composition of any one of items 41 to 44, wherein the antibody further comprises

(a) variable region heavy chain framework sequences juxtaposed between the HVRs according to the formula: (HC-FR1)-(HVR-H1)-(HC-FR2)-(HVR-H2)-(HC-FR3)-(HVR-H3)-(HC-FR4), and
(b) variable region light chain framework sequences juxtaposed between the HVRs according to the formula: (LC-FR1)-(HVR-L1)-(LC-FR2)-(HVR-L2)-(LC-FR3)-(HVR- L3)-(LC-FR4).

46. The method of item 45; or the pharmaceutical composition of item 45, wherein the framework sequences are derived from human consensus framework sequences.

47. The method of item 46; or the pharmaceutical composition of item 46,wherein the variable region heavy chain framework sequences are VH subgroup III consensus framework.

48. The method of item 47; or the pharmaceutical composition of item 47, wherein one or more of the framework sequences is the following:

HC-FR1 is EVQLVESGGGLVQPGGSLRLSCAAS (SEQ ID NO:4);
HC-FR2 is WVRQAPGKGLEWV (SEQ ID NO:5);
HC-FR3 is RFTISADTSKNTAYLQMNSLRAEDTAVYYCAR (SEQ ID NO:6);
HC-FR4 is WGQGTLVTVSA (SEQ ID NO:7).

49. The method of item 46; or the pharmaceutical composition of item 46, wherein the variable region light chain framework sequences are VL kappa I consensus framework.

50. The method of item 49; or the pharmaceutical composition of item 49, wherein one or more of the framework sequences is the following:

LC-FR1 is DIQMTQSPSSLSASVGDRVTITC (SEQ ID NO:11);
LC-FR2 is WYQQKPGKAPKLLIY (SEQ ID NO:12);
LC-FR3 is GVPSRFSGSGSGTDFTLTISSLQPEDFATYYC, and (SEQ ID NO: 13);
LC-FR4 is FGQGTKVEIKR (SEQ ID NO:14).

51. The method of item 46; or the pharmaceutical composition of item 46, wherein:

(a) the variable heavy chain framework sequences are the following:

(i) HC-FR1 is EVQLVESGGGLVQPGGSLRLSCAAS (SEQ ID NO:4);
(ii) HC-FR2 is WVRQAPGKGLEWV (SEQ ID NO:5);
(iii) HC-FR3 is RFTISADTSKNTAYLQMNSLRAEDTAVYYCAR (SEQ ID NO:6);
(iv) HC-FR4 is WGQGTLVTVSA; and (SEQ ID NO:7);

(b) the variable light chain framework sequences are the following:

(i) LC-FR1 is DIQMTQSPSSLSASVGDRVTITC (SEQ ID NO:11);
(ii) LC-FR2 is WYQQKPGKAPKLLIY (SEQ ID NO:12);
(iii) LC-FR3 is GVPSRFSGSGSGTDFTLTISSLQPEDFATYYC (SEQ ID NO:13);
(iv) LC-FR4 is FGQGTKVEIKR (SEQ ID NO:14).

52. The method of item 51; or the pharmaceutical composition of item 51, wherein the antibody further comprises a human constant region.

53. The method of item 52; or the pharmaceutical composition of item 52, wherein the constant region is selected from the group consisting of IgG1, IgG2, IgG3 and IgG4.

54. The method of item 53; or the pharmaceutical composition of item 53, wherein the constant region is IgG1.

55. The method of item 51; or the pharmaceutical composition of item 51, wherein the antibody further comprises murine constant region.

56. The method of item 55; or the pharmaceutical composition of item 55, wherein the constant region is selected from the group consisting of IgG1, IgG2A, IgG2B and IgG3.

57. The method of item 56; or the pharmaceutical composition of item 56, wherein the constant region is IgG2A.

58. The method of item 53 or 56; or the pharmaceutical composition of item 53 or 56, wherein said antibody has reduced or minimal effector function.

59. The method of item 58; or the pharmaceutical composition of item 58, wherein the minimal effector function results from an effector-less Fc mutation.

60. The method of item 59; or the pharmaceutical composition of item 59, wherein the effector-less Fc mutation is N297A.

61. The method of item 59; or the pharmaceutical composition of item 59, wherein the effector-less Fc mutation is D265A/N297A.

62. Method of item 58; or the pharmaceutical composition of item 58, wherein the minimal effector function results from aglycosylation.

63. The method of item 29; or the pharmaceutical composition of item 29, wherein said antibody comprises a heavy chain and a light chain variable region sequence, wherein:

(a) the heavy chain comprises an HVR-H1, HVR-H2 and an HVR-H3, having at least 85% overall sequence identity to GFTFSDSWIH (SEQ ID NO:15), AWISPYGGSTYYADSVKG (SEQ ID NO: 16) and RHWPGGFDY (SEQ ID NO:3), respectively, and
(b) the light chain comprises an HVR-L1, HVR-L2 and an HVR-L3, having at least 85% overall sequence identity to RASQDVSTAVA (SEQ ID NO:17), SASFLYS (SEQ ID NO:18) and QQYLYHPAT (SEQ ID NO:19), respectively.

64. The method of item 63; or the pharmaceutical composition of item 63, wherein said sequence identity is at least 90%.

65. The method of item 64; or the pharmaceutical composition of item 64, wherein said antibody further comprises:

(a) variable region heavy chain (VH) framework sequences juxtaposed between the HVRs according to the formula: (HC-FR1)-(HVR-H1)-(HC-FR2)-(HVR-H2)-(HC-FR3)-(HVR-H3)-(HC-FR4), and
(b) variable region light chain (VL) framework sequences juxtaposed between the HVRs according to the formula: (LC-FR1)-(HVR-L1)-(LC-FR2)-(HVR-L2)-(LC-FR3)-(HVR-L3)-(LC-FR4).

66. The method of item 65; or the pharmaceutical composition of item 65, wherein said antibody further comprises a VH and VL framework region derived from a human consensus sequence.

67. The method of item 66; or the pharmaceutical composition of item 66, wherein the VH framework sequence is derived from a Kabat subgroup I, II, or III sequence.

68. The method of item 67; or the pharmaceutical composition of item 67, wherein the VH framework sequence is a Kabat subgroup III consensus framework sequence.

69. The method of item 68; or the pharmaceutical composition of item 68, wherein the VH framework sequences are the following:

HC-FR1 is EVQLVESGGGLVQPGGSLRLSCAAS (SEQ ID NO:4);
HC-FR2 is WVRQAPGKGLEWV (SEQ ID NO:5);
HC-FR3 is RFTISADTSKNTAYLQMNSLRAEDTAVYYCAR (SEQ ID NO:6);
HC-FR4 is WGQGTLVTVSA (SEQ ID NO:7).

70. The method of item 66; or the pharmaceutical composition of item 66, wherein the VL framework sequence is derived from a Kabat kappa I, II, III or IV subgroup sequence.

71. The method of item 70; or the pharmaceutical composition of item 70, wherein the the VL framework sequence is a Kabat kappa I consensus framework sequence.

72. The method of item 71; or the pharmaceutical composition of item 71, wherein the VL framework sequences are the following:

LC-FR1 is DIQMTQSPSSLSASVGDRVTITC (SEQ ID NO:11);
LC-FR2 is WYQQKPGKAPKLLIY (SEQ ID NO:12);
LC-FR3 is GVPSRFSGSGSGTDFTLTISSLQPEDFATYYC (SEQ ID NO:13);
LC-FR4 is FGQGTKVEIKR (SEQ ID NO:14).

73. The method of item 29; or the pharmaceutical composition of item 29, wherein said antibody comprises a heavy chain and a light chain variable region sequence, wherein:

(a) the heavy chain sequence has at least 85% sequence identity to the heavy chain sequence: EVQL VESGGGL VQPGGSLRLSCAASGFTFSDSWIHWVRQAPG KGLEWVAWISPYGGSTYYADSVKGRFTISADTSKNTAYLQMNSLRAEDTAV YYCARRHWPGGFDYWGQGTLVTVSA (SEQ ID NO:20), and
(b) the light chain sequence has at least 85% sequence identity to the light chain sequence: DIQMTQSPSSLSASVGDRVTITCRASQDVSTAVAWYQQKPGK APKLLIYSASFLYSGVPSRFSGSGSGTDFTLTISSLQPEDFATYYCQQYLYH PATFGQGTKVEIKR (SEQ ID NO:21).

74. The method of item 73; or the pharmaceutical composition of item 73, wherein the sequence identity is at least 90%.

75. The method of item 29; or the pharmaceutical composition of item 29, wherein said antibody comprises a heavy chain and light chain variable region sequence, wherein:

(a) the heavy chain comprises the sequence: EVQLVESGGGLVQPGGSLRLS CAASGFTFSDSWIHWVRQAPGKGLEWVAWISPYGGSTYYADSVKGRFTISA DTSKNTAYLQMNSLRAEDTAVYYCARRHWPGGFDYWGQGTLVTVSA (SEQ ID NO:20), and
(b) the light chain comprises the sequence: DIQMTQSPSSLSASVGDRVTITC RASQDVSTAVAWYQQKPGKAPKLLIYSASFLYSGVPSRFSGSGSGTDFTLTIS SLQPEDFATYYCQQYLYH PATFGQGTKVEIKR (SEQ ID NO:21).

76. The method of any one of items 1, 2, 3, 6 and 8 to 75; or the pharmaceutical composition of any one of items 4, 5 and 7 to 75, wherein said cancer is a solid cancer.

77. The method of item 76; or the pharmaceutical composition of item 76, wherein said solid cancer is breast cancer or gastric cancer.

78. The method of item 76; or the pharmaceutical composition of item 76, wherein said solid cancer is breast cancer.

79. The method of any one of items 1, 2, 3, 6 and 8 to 78; or the pharmaceutical composition of any one of items 4, 5 and 7 to 78, wherein the expression level of PD-L1 is higher or equal to 5.3 determined by routine methods like Affymetrix.

80. The method of any one of items 1, 2, 3, 6 and 8 to 79, ; or the pharmaceutical composition of any one of items 4, 5 and 7 to 79, wherein the expression level of PD-L1 is the mRNA expression level.

81. The method of item 80; or the pharmaceutical composition of item 80, wherein the mRNA expression level of PD-L1 is assessed by in situ hybridization, micro-arrays, or RealTime PCR.

82. The method of any one of items 1, 2, 3, 6 and 8 to 79; or the pharmaceutical composition of any one of items 4, 5 and 7 to 79, wherein the expression level of PD-L1 is the protein expression level.

83. The method of item 82; or the pharmaceutical composition of item 82, wherein said protein expression level of PD-L1 is assessed by immunoassay, gel- or blot-based methods, IHC, mass spectrometry, flow cytometry, or FACS.

84. The method of any one of items 1, 2, 3, 6 and 8 to 83; or the pharmaceutical composition of any one of items 4, 5 and 7 to 83, wherein the patient to be treated is a human.

85. Use of a nucleic acid or antibody capable of detecting the expression level of ER, PD-L1 and, optionally, IFNγ for determining a patient's need for PD-L1 inhibitor cotherapy in combination with a modulator of the HER2/neu (ErbB2) signaling pathway and a chemotherapeutic agent.

86. A kit useful for carrying out the method of any one of items 1, 2, 3 6 and 8 to 84, comprising a nucleic acid or an antibody capable of detecting the expression level of ER, PD-L1 and, optionally, IFNγ.

87. The method of any one of items 1, 2, 3, 6 and 8 to 84; or the pharmaceutical composition of any one of items 4, 5 and 7 to 84, wherein said modulator of the HER2/neu (ErbB2) signaling pathway, said chemotherapeutic agent and said inhibitor of programmed death ligand 1 (PD-L1) are to be administered in a neoadjuvant setting or adjuvant setting or metastatic setting.

88. A method for treating cancer comprising administering an effective amount of a modulator of the HER2/neu (ErbB2) signaling pathway, a chemotherapeutic agent and an inhibitor of programmed death ligand 1 (PD-L1) to a subject in need thereof.

## Claims

1. A programmed death ligand 1 (PD-L1) inhibitor for use in a method of treating a cancer in a cancer patient who is undergoing therapy comprising a modulator of the HER2/neu (ErbB2) signaling pathway and a chemotherapeutic agent, or for use in a method of treating a cancer in a cancer patient wherein therapy comprising a modulator of the HER2/neu (ErbB2) signaling pathway and a chemotherapeutic agent is contemplated for the patient, the method comprising selecting a cancer patient whose cancer is determined to have a low or absent ER expression level and to have an increased expression level of programmed death ligand 1 (PD-L1) in comparison to a control, and administering to the patient an effective amount of the programmed death ligand 1 (PD-L1) inhibitor.

2. The programmed death ligand 1 (PD-L1) inhibitor for use in the method of claim 1, further comprising measuring in vitro in a sample from said patient the expression level of interferon-gamma (IFN$\gamma$) and determining a patient as being in need of a PD-L1 inhibitor cotherapy if an expression level of interferon-gamma (IFN$\gamma$) that is decreased in comparison to a control is measured.

3. The programmed death ligand 1 (PD-L1) inhibitor for use in the method of claim 1 or 2, wherein the ER expression level is ER(-).

4. The programmed death ligand 1 (PD-L1) inhibitor for use in the method of any one of claims 1 to 3, wherein said modulator of the HER2/neu (ErbB2) signaling pathway is an inhibitor of HER shedding.

5. The programmed death ligand 1 (PD-L1) inhibitor for use in the method of claim 4, wherein said inhibitor of HER shedding is a HER antibody.

6. The programmed death ligand 1 (PD-L1) inhibitor for use in the method of claim 5, wherein said HER antibody binds to a HER receptor selected from the group consisting of EGFR, HER2 and HER3.

7. The programmed death ligand 1 (PD-L1) inhibitor for use in the method of claim 5 or 6, wherein said HER2 antibody is Herceptin/Trastuzumab.

8. The programmed death ligand 1 (PD-L1) inhibitor for use in the method of any one of claims 1 to 7, wherein said chemotherapeutic agent is taxol or a taxol derivative.

9. The programmed death ligand 1 (PD-L1) inhibitor for use in the method of claim 8, wherein said taxol derivative is dodetaxel.

10. The programmed death ligand 1 (PD-L1) inhibitor for use in the method of any one of claims 1 to 9, wherein said inhibitor of programmed death ligand 1 (PD-L1) is an antibody specifically binding to PD-L1 (anti-PD-LI antibody).

11. The programmed death ligand 1 (PD-L1) inhibitor for use in the method of claim 10, wherein said antibody comprises an heavy chain variable region polypeptide comprising an HVR-H1, HVR-H2 and HVR-H3 sequence, wherein:

(a) the HVR-H1 sequence is GFTFSX1SWIH (SEQ ID NO:1);
(b) the HVR-H2 sequence is AWIX2PYGGSX3YYADSVKG (SEQ ID NO:2);
(c) the HVR-H3 sequence is RHWPGGFDY (SEQ ID NO:3);

further wherein: X1 is D or G; X2 is S or L; X3 is T or S.

12. The programmed death ligand 1 (PD-L1) inhibitor for use in the method of claim 11, wherein said heavy chain polypeptide is in combination with a variable region light chain comprising an HVR-L1, HVR-L2 and HVR-L3, wherein:

(a) the HVR-L1 sequence is RASQX4X5X6TX7X8A (SEQ ID NOs:8);
(b) the HVR-L2 sequence is SASX9LX10S, and (SEQ ID NOs:9);
(c) the HVR-L3 sequence is QQX11X12X13X14PX15T (SEQ ID NOs:10);

further wherein: X4 is D or V; X5 is V or I; X6 is S or N; X7 is A or F; X8 is V or L; X9 is F or T; X10 is Y or A; X11 is Y, G, F, or S; X12 is L, Y, F or W; X13 is Y, N, A, T, G, F or I; X14 is H, V, P, T or I; X15 is A, W, R, P or T.

13. The programmed death ligand 1 (PD-L1) inhibitor for use in the method of claim 11, wherein said antibody comprises a heavy chain and a light chain variable region sequence, wherein:

(a) the heavy chain comprises an HVR-H1, HVR-H2 and an HVR-H3, having at least 85% overall sequence identity to GFTFSDSWIH (SEQ ID NO:15), AWISPYGGSTYYADSVKG (SEQ ID NO:16) and RHWPGGFDY (SEQ ID NO:3), respectively, and
(b) the light chain comprises an HVR-L1, HVR-L2 and an HVR-L3, having at least 85% overall sequence identity to RASQDVSTAVA (SEQ ID NO: 17), SASFLYS (SEQ ID NO: 18) and QQYLYHPAT (SEQ ID NO: 19), respectively.

14. The programmed death ligand 1 (PD-L1) inhibitor for use in the method of any one of claims 1 to 13, wherein said cancer is a solid cancer.

15. The programmed death ligand 1 (PD-L1) inhibitor for use in the method of claim 14, wherein said solid cancer is breast cancer or gastric cancer.

16. Use of a nucleic acid or antibody capable of detecting the expression level of ER, PD-L1 and, optionally, IFNγ for determining a patient's need for PD-L1 inhibitor cotherapy in combination with a modulator of the HER2/neu (ErbB2) signaling pathway and a chemotherapeutic agent.

17. A kit useful for carrying out a method, the kit comprising a nucleic acid or an antibody capable of detecting the expression level of ER, PD-L1 and, optionally, IPNγ,
wherein the method is a method of determining the need of a cancer patient for a PD-L1 inhibitor cotherapy, (i) wherein therapy comprising a modulator of the HER2/neu (ErbB2) signaling pathway and a chemotherapeutic agent is contemplated for the patient or (ii) wherein the patient is undergoing therapy comprising a modulator of the HER2/neu (ErbB2) signaling pathway and a chemotherapeutic agent, the method comprising the steps of

a) measuring in vitro in a sample from said patient the expression level of Estrogen receptor (ER) and of programmed death ligand 1 (PD-L1),
b) determining a patient as being in need of a PD-L1 inhibitor cotherapy if

a low or absent ER expression level and an expression level of programmed death ligand 1 (PD-L1) that is increased in comparison to a control is measured in step (a).

**18.** The programmed death ligand 1 (PD-L1) inhibitor for use in the method of any one of claims 1 to 15, wherein said modulator of the HER2/neu (ErbB2) signaling pathway, said chemotherapeutic agent and said inhibitor of programmed death ligand 1 (PD-L1) are to be administered in a neoadjuvant setting or adjuvant setting or metastatic setting.

Figure 1.

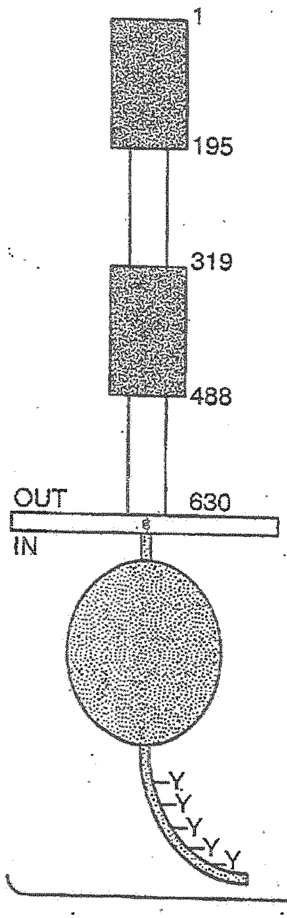


Domain I (L1)
TQVCTGTDMKLRLPASPETHLDMLRHLYQGCQVVQGNLELTYLPTNASLSFLQDIQEVQGYV
LIAHNQVRQVPLQRLRIVRGTQLFEDNYALAVLDNGDPLNNTTPVTGASPGGLRELQLRSLT
EILKGGVLIQRNPQLCYQDTILWKDIFHKNNQLALTLIDTNRSRACHPCSPMCKGSRCWGES
SEDCQSLTR

Domain II (CR1)
TVCAGGCARCKGPLPTDCCHEQCAAGCTGPKHSDCLACLHFNHSGICELHCPALVTYNTDTF
ESMPNPEGRYTFGASCVTACPYNYLSTDVGSCTLVCPLHNQEVTAEDGTQRCEKCSKPCARV

Domain III (L2)
CYGLGMEHLREVRAVTSANIQEFAGCKKIFGSLAFLPESFDGDPASNTAPLQPEQLQVFETLE
EITGYLYISAWPDSLPDLSVFQNLQVIRGRILHNGAYSLTLQGLGISWLGLRSLRELGSGLAL
IHHNTHLCFVHTVPWDQLFRNPHQALLHTANRPEDECVGEGLA

Domain IV (CR2)
CHQLCARGHCWGPGPTQCVNCSQFLRGQECVEECRVLQGLPREYVNARHCLPCHPECQPQNGS
VTCFGPEADQCVACAHYKDPPFCVARCPSGVKPDLSYMPIWKFPDEEGACQPCPINCTHSCVD
LDDKGCPAEQRASPLT

Transmembrane
Juxtamembrane

Tyrosine Kinase

Regulatory Region

FIG. 1

Figure 2.

Variable Light

```
                 10        20          30          40
2C4       DTVMTQSHKIMSTSVGDRVSITC [KASQDVSIGVA] WYQQRP
           **    **** *       *                     *
574       DIQMTQSPSSLSASVGDRVTITC [KASQDVSIGVA] WYQQKP
                                    *   ** ***
hum. κI   DIQMTQSPSSLSASVGDRVTITC [RASQSISNYLA] WYQQKP

                    50          60        70       80
2C4       GQSPKLLIY [SASYRYT] GVPDRFTGSGSGTDFTFTISSVQA
           **                    *  *          *    * *
574       GKAPKLLIY [SASYRYT] GVPSRFSGSGSGTDFTLTISSLQP
                      * *****
hum κI    GKAPKLLIY [AASSLES] GVPSRFSGSGSGTDFTLTISSLQP

                   90        100
2C4       EDLAVYYC [QQYYIYPYT] FGGGTKLEIK (SEQ ID NO:5)
            * *                   *   *
574       EDFATYYC [QQYYIYPYT] FGQGTKVEIK (SEQ ID NO:7)
                      *** *
hum κI    EDFATYYC [QQYNSLPWT] FGQGTKVEIK (SEQ ID NO:9)
```

FIG. 2A

Variable Heavy

```
                 10        20          30          40
2C4       EVQLQQSGPELVKPGTSVKISCKAS [GFTFTDYTMD] WVKQS
              **  **  *  * ***  *                 * *
574       EVQLVESGGGLVQPGGSLRLSCAAS [GFTFTDYTMD] WVRQA
                                        ** * *
hum III   EVQLVESGGGLVQPGGSLRLSCAAS [GFTFSSYAMS] WVRQA

                    50  a       60          70        80
2C4       HGKSLEWIG [DVNPNSGGSIYNQRFKG] KASLTVDRSSRIVYM
          *  *   **                     *** *    **** *
574       PGKGLEWVA [DVNPNSGGSIYNQRFKG] RFTLSVDRSKNTLYL
                     ****** *** ****       * * *
hum III   PGKGLEWVA [VISGDGGSTYYADSVKG] RFTISRDNSKNTLYL

           abc       90           100ab          110
2C4       ELRSLTFEDTAVYYCAR [NLGPSFYFDY] WGQGTTLTVSS (SEQ ID NO:6)
          ***   **                           **
574       QMNSLRAEDTAVYYCAR [NLGPSFYFDY] WGQGTLVTVSS (SEQ ID NO:8)
                             ********
hum III   QMNSLRAEDTAVYYCAR [GRVGYSLYDY] WGQGTLVTVSS (SEQ ID NO:10)
```

FIG. 2B

**Figure 3.**

### Amino Acid Sequence for Pertuzumab Light Chain

1 10 20 30 40 50 60
DIQMTQSPSSLSASVGDRVTITC**KASQDVSIGVA**WYQQKPGKAPKLLIY**SASYRYT**GVPS

70 80 90 100 110 120
RFSGSGSGTDFTLTISSLQPEDFATYYC**QQYYIYPYT**FGQGTKVEIKRTVAAPSVFIFPP

130 140 150 160 170 180
SDEQLKSGTASVVCLLNNFYPREAKVQWKVDNALQSGNSQESVTEQDSKDSTYSLSSTLT

190 200 210
LSKADYEKHKVYACEVTHQGLSSPVTKSFNRGEC

FIG. 3A

### Amino Acid Sequence for Pertuzumab Heavy Chain

1 10 20 30 40 50 60
EVQLVESGGGLVQPGGSLRLSCAAS**GFTFTDYTMD**WVRQAPGKGLEWVA**DVNPNSGGSIY**

70 80 90 100 110 120
**NQRFK**GRFTLSVDRSKNTLYLQMNSLRAEDTAVYYCARN**LGPSFYFDY**WGQGTLVTVSSA

130 140 150 160 170 180
STKGPSVFPLAPSSKSTSGGTAALGCLVKDYFPEPVTVSWNSGALTSGVHTFPAVLQSSG

190 200 210 220 230 240
LYSLSSVVTVPSSSLGTQTYICNVNHKPSNTKVDKKVEPKSCDKTHTCPPCPAPELLGGP

250 260 270 280 290 300
*
SVFLFPPKPKDTLMISRTPEVTCVVVDVSHEDPEVKFNWYVDGVEVHNAKTKPREEQYNS

310 320 330 340 350 360
TYRVVSVLTVLHQDWLNGKEYKCKVSNKALPAPIEKTISKAKGQPREPQVYTLPPSREEM

370 380 390 400 410 420
TKNQVSLTCLVKGFYPSDIAVEWESNGQPENNYKTTPPVLDSDGSFFLYSKLTVDKSRWQ

430 440 448
QGNVFSCSVMHEALHNHYTQKSLSLSPG

FIG. 3B

Figure 4.

**Light Chain**

```
1                           15                            30                            45
D I Q M T Q S P S S L S A S V G D R V T I T C R A S Q D V N T A V A W Y Q Q K P G K A P K
46                          60                            75                            90
L L I Y S A S F L Y S G V P S R F S G S R S G T D F T L T I S S L Q P E D F A T Y Y C Q Q
91                          105                           120                           135
H Y T T P P T F G Q G T K V E I K R T V A A P S V F I F P P S D E Q L K S G T A S V V C L
136                         150                           165                           180
L N N F Y P R E A K V Q W K V D N A L Q S G N S Q E S V T E Q D S K D S T Y S L S S T L T
181                         195                           210     214
L S K A D Y E K H K V Y A C E V T H Q G L S S P V T K S F N R G E C
```

FIG. 4A

Figure 4 (cont.).

Heavy Chain

```
1                                             15                                            30                                            45
E V Q L V E S G G G L V Q P G G S L R L S C A A S G F N I K D T Y I H W V R Q A P G K G L
46                                            60                                            75                                            90
E W V A R I Y P T N G Y T R Y A D S V K G R F T I S A D T S K N T A Y L Q M N S L R A E D
91                                            105                                           120                                           135
T A V Y Y C S R W G G D G F Y A M D Y W G Q G T L V T V S S A S T K G P S V F P L A P S S
136                                           150                                           165                                           180
K S T S G G T A A L G C L V K D Y F P E P V T V S W N S G A L T S G V H T F P A V L Q S S
181                                           195                                           210                                           225
G L Y S L S S V V T V P S S S L G T Q T Y I C N V N H K P S N T K V D K K V E P K S C D K
226                                           240                                           255                                           270
T H T C P P C P A P E L L G G P S V F L F P P K P K D T L M I S R T P E V T C V V V D V S
271                                           285                                           300                                           315
H E D P E V K F N W Y V D G V E V H N A K T K P R E E Q Y N S T Y R V V S V L T V L H Q D
316                                           330                                           345                                           360
W L N G K E Y K C K V S N K A L P A P I E K T I S K A K G Q P R E P Q V Y T L P P S R E E
361                                           375                                           390                                           405
M T K N Q V S L T C L V K G F Y P S D I A V E W E S N G Q P E N N Y K T T P P V L D S D G
406                                           420                                           435                                         449
S F F L Y S K L T V D K S R W Q Q G N V F S C S V M H E A L H N H Y T Q K S L S L S P G
```

FIG. 4B

Figure 5.

```
1                           15                            30                            45
V H S D I Q M T Q S P S S L S A S V G D R V T I T C K A S Q D V S I G V A W Y Q Q K P G K
46                          60                            75                            90
A P K L L I Y S A S Y R Y T G V P S R F S G S G S G T D F T L T I S S L Q P E D F A T Y Y
91                          105                           120                           135
C Q Q Y Y I Y P Y T F G Q G T K V E I K R T V A A P S V F I F P P S D E Q L K S G T A S V
136                         150                           165                           180
V C L L N N F Y P R E A K V Q W K V D N A L Q S G N S Q E S V T E Q D S K D S T Y S L S S
181                         195                           210             217
T L T L S K A D Y E K H K V Y A C E V T H Q G L S S P V T K S F N R G E C
```

FIG. 5A

Figure 5 (cont.).

```
1                                          15                                         30                                        45
E V Q L V E S G G G L V Q P G G S L R L S C A A S G F T F T D Y T M D W V R Q A P G K G L
46                                         60                                         75                                        90
E W V A D V N P N S G G S I Y N Q R F K G R F T L S V D R S K N T L Y L Q M N S L R A E D
91                                         105                                        120                                       135
T A V Y Y C A R N L G P S F Y F D Y W G Q G T L V T V S S A S T K G P S V F P L A P S S K
136                                        150                                        165                                       180
S T S G G T A A L G C L V K D Y F P E P V T V S W N S G A L T S G V H T F P A V L Q S S G
181                                        195                                        210                                       225
L Y S L S S V V T V P S S S L G T Q T Y I C N V N H K P S N T K V D K K V E P K S C D K T
226                                        240                                        255                                       270
H T C P P C P A P E L L G G P S V F L F P P K P K D T L M I S R T P E V T C V V V D V S H
271                                        285                                        300                                       315
E D P E V K F N W Y V D G V E V H N A K T K P R E E Q Y N S T Y R V V S V L T V L H Q D W
316                                        330                                        345                                       360
L N G K E Y K C K V S N K A L P A P I E K T I S K A K G Q P R E P Q V Y T L P P S R E E M
361                                        375                                        390                                       405
T K N Q V S L T C L V K G F Y P S D I A V E W E S N G Q P E N N Y K T T P P V L D S D G S
406                                        420                                        435                                    449
F F L Y S K L T V D K S R W Q Q G N V F S C S V M H E A L H N H Y T Q K S L S L S P G K
```

FIG. 5B

Figure 6.

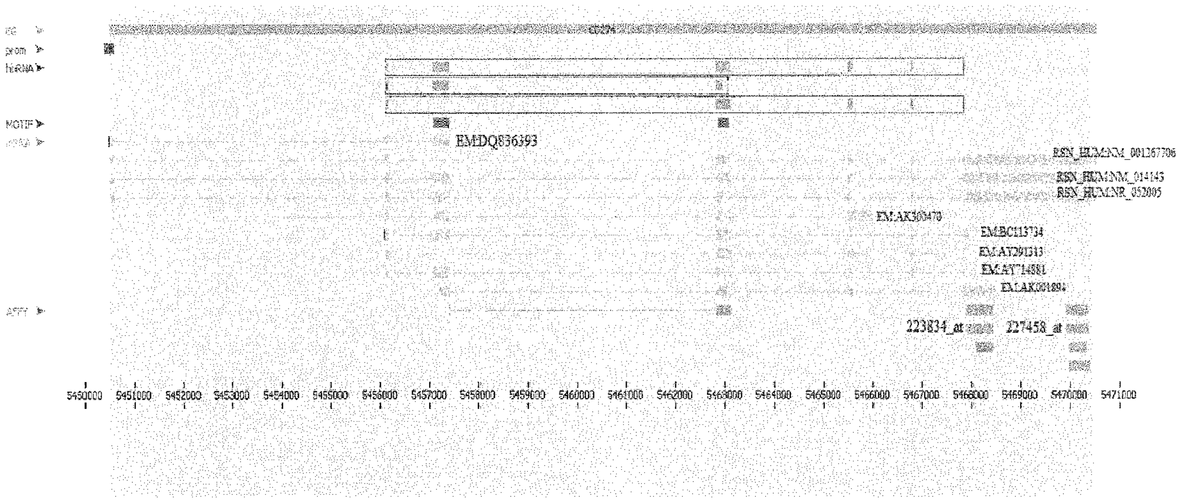
prom
hnRNA
MOTIF
EMDQ836393
RSN_HUM:NM_001267706
RSN_HUM:NM_014143
RSN_HUM:NR_052005
EM:AK300470
EM:BC113734
EM:AY291313
EM:AY714881
EM:AK001894
223834_at
227458_at
5450000
5451000
5452000
5453000
5454000
5455000
5456000
5457000
5458000
5459000
5460000
5461000
5462000
5463000
5464000
5465000
5466000
5467000
5468000
5469000
5470000
5471000

Figure 7.

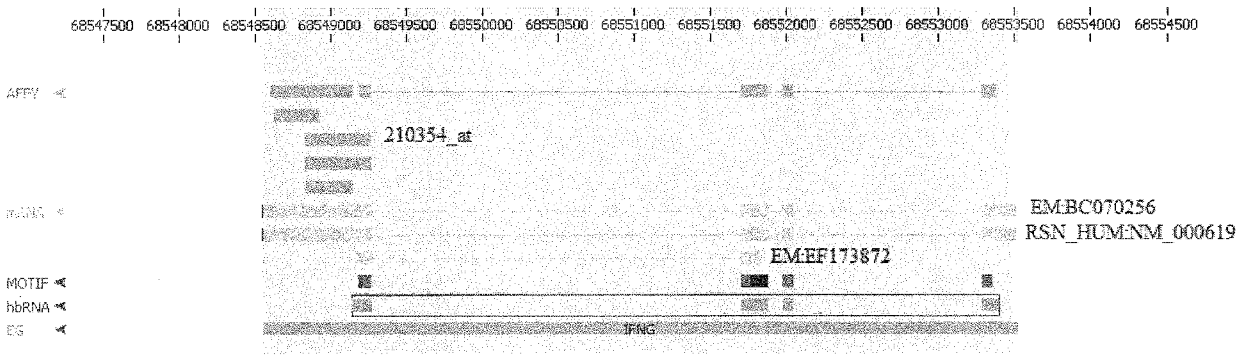
68547500 68548000 68548500 68549000 68549500 68550000 68550500 68551000 68551500 68552000 68552500 68553000 68553500 68554000 68554500
AFFY
210354_at
mRNA
EM:BC070256
RSN_HUM:NM_000619
EM:EF173872
MOTIF
hbRNA
EG
IFNG

Figure 8.

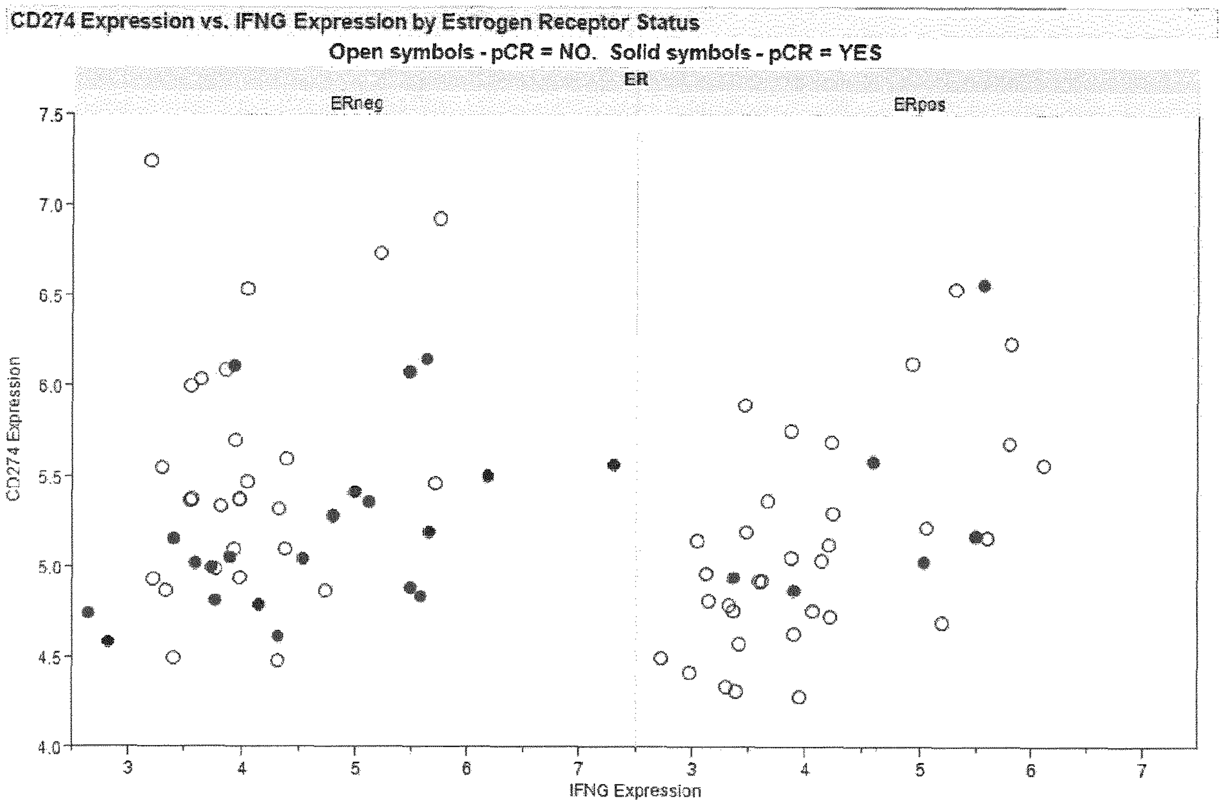
CD274 Expression vs. IFNG Expression by Estrogen Receptor Status
Open symbols - pCR = NO. Solid symbols - pCR = YES
ER
ERneg
ERpos
CD274 Expression
IFNG Expression
7.5
7.0
6.5
6.0
5.5
5.0
4.5
4.0
3
4
5
6
7

Figure 9.

A.

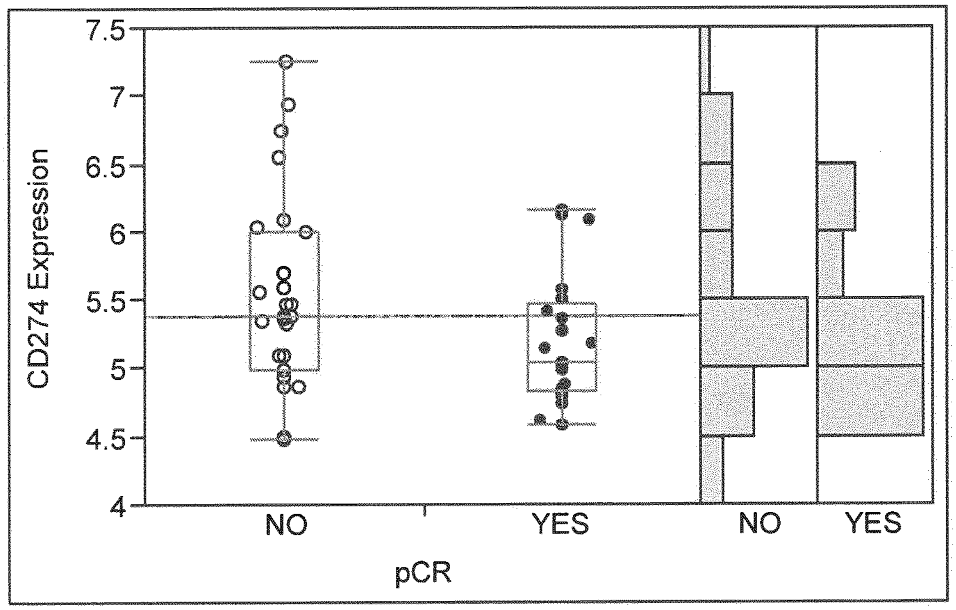
7.5
7
6.5
6
5.5
5
4.5
4
CD274 Expression
NO
YES
pCR
NO
YES

B.

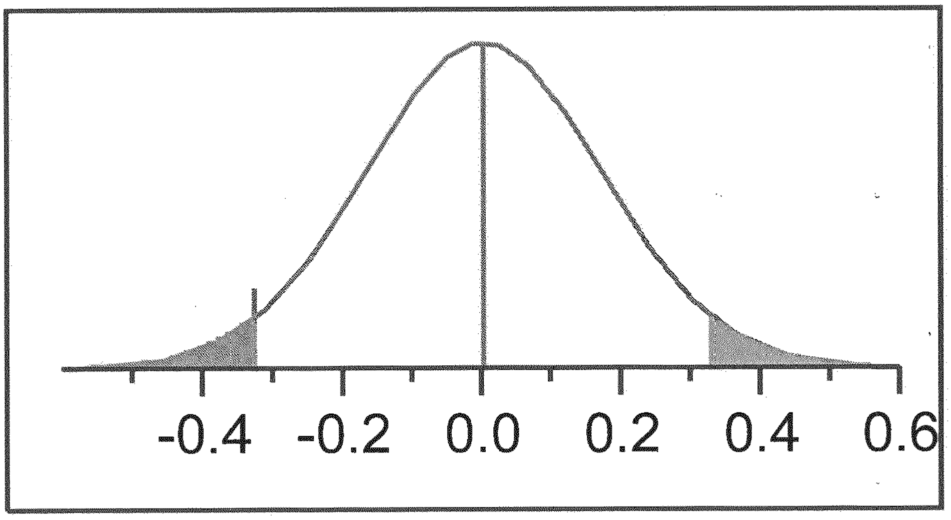
-0.4
-0.2
0.0
0.2
0.4
0.6

Figure 10.

A.

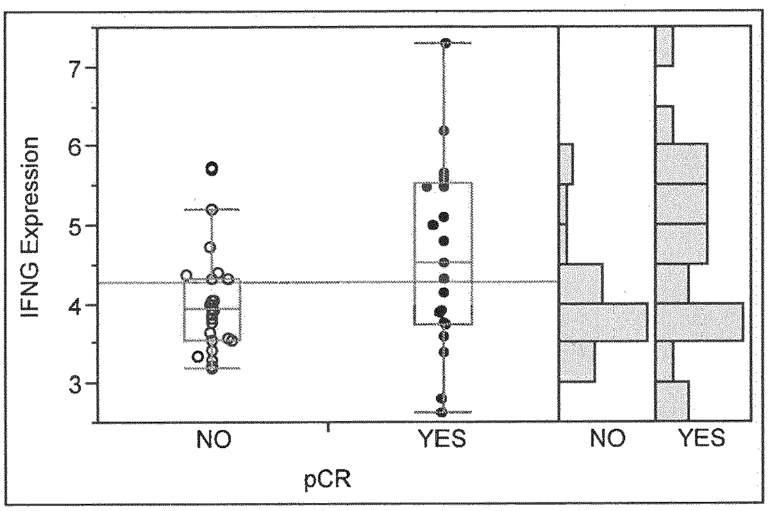


B.

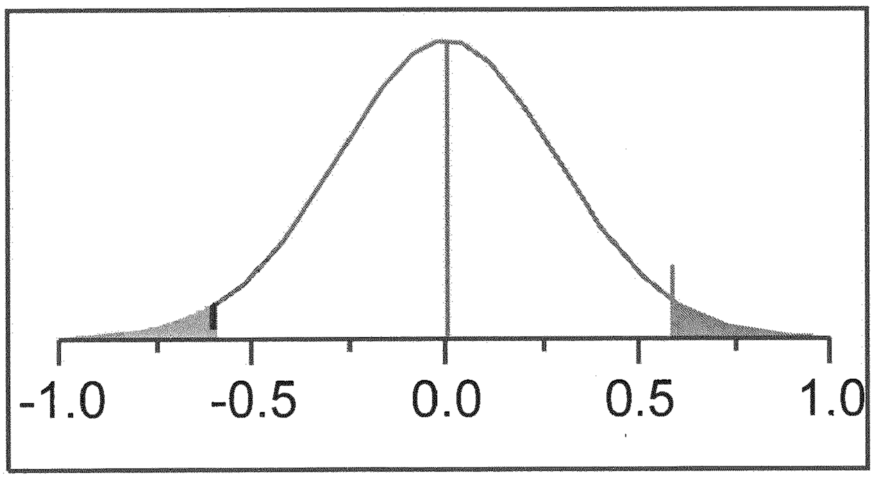

**Figure 11.**

**A.**

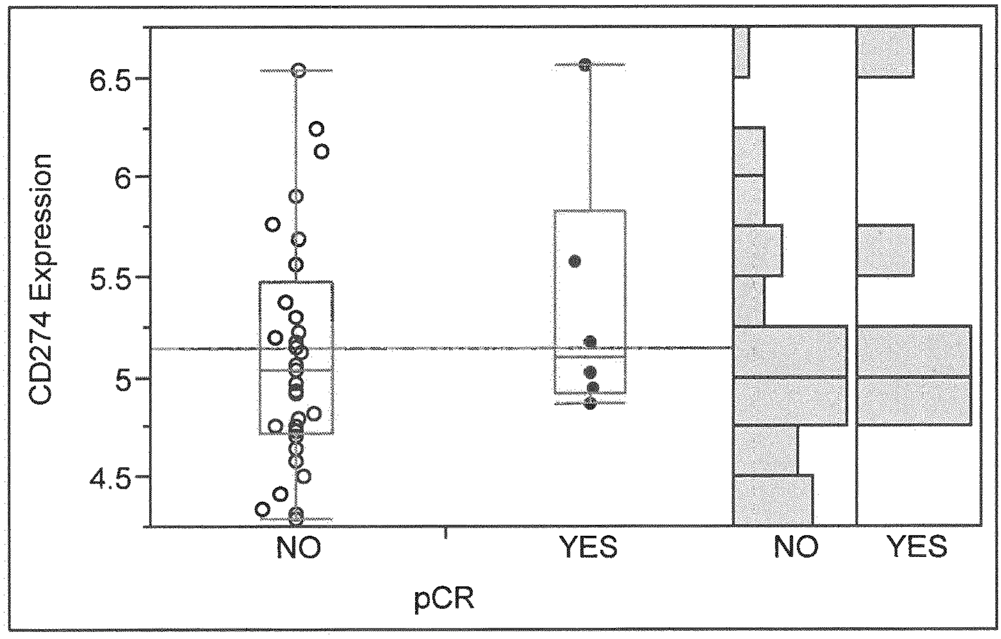


**B.**

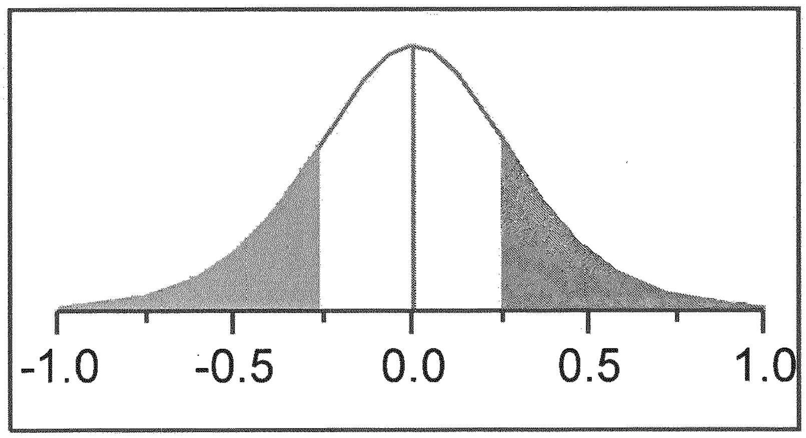

Figure 12.

A.

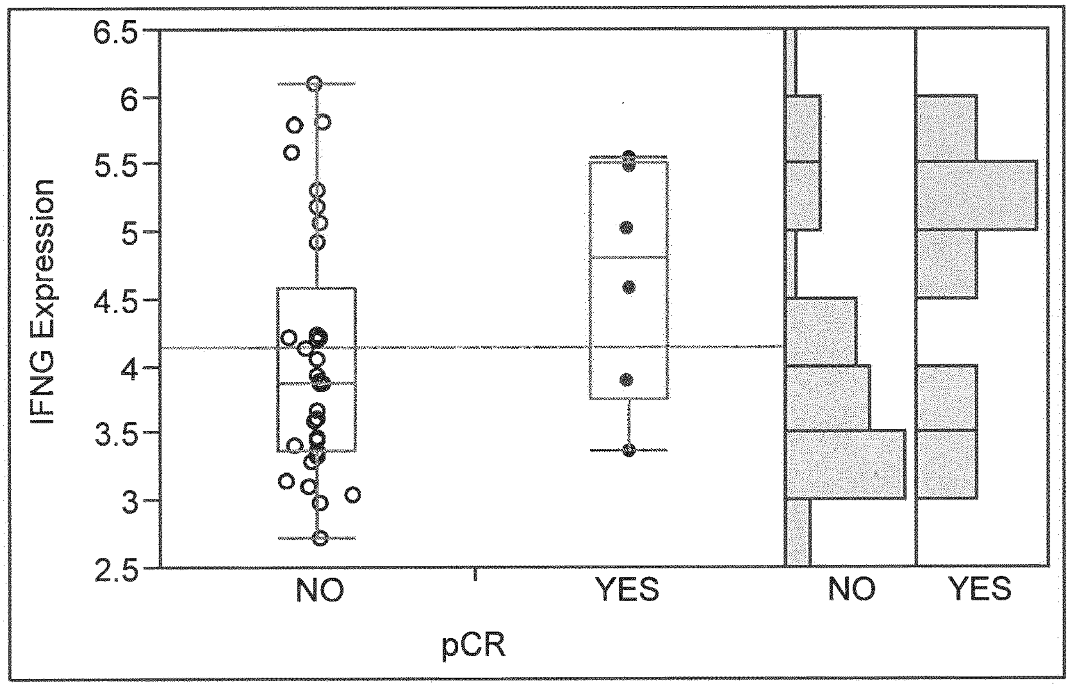
6.5
6
5.5
5
4.5
4
3.5
3
2.5
IFNG Expression
NO
YES
pCR
NO
YES

B.

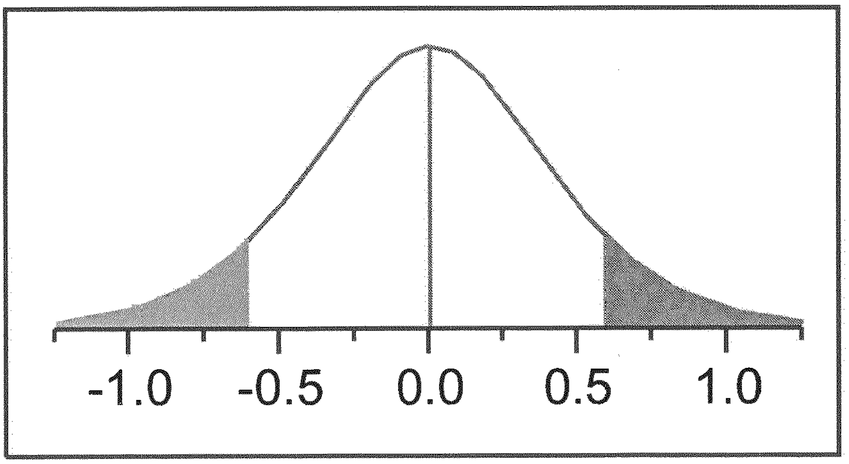
-1.0
-0.5
0.0
0.5
1.0

**Figure 13.**

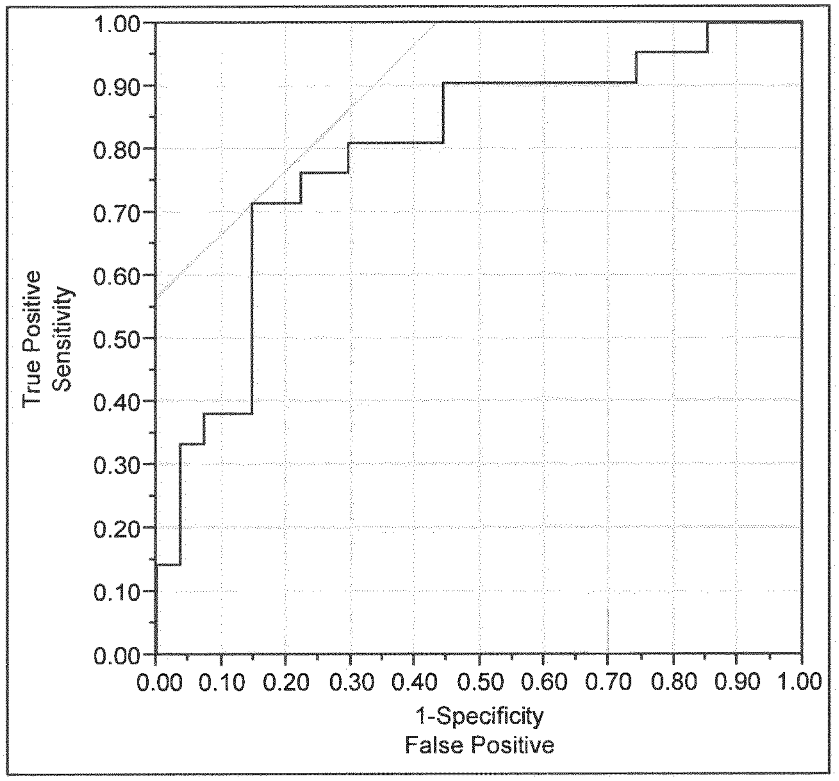


**Figure 14.**

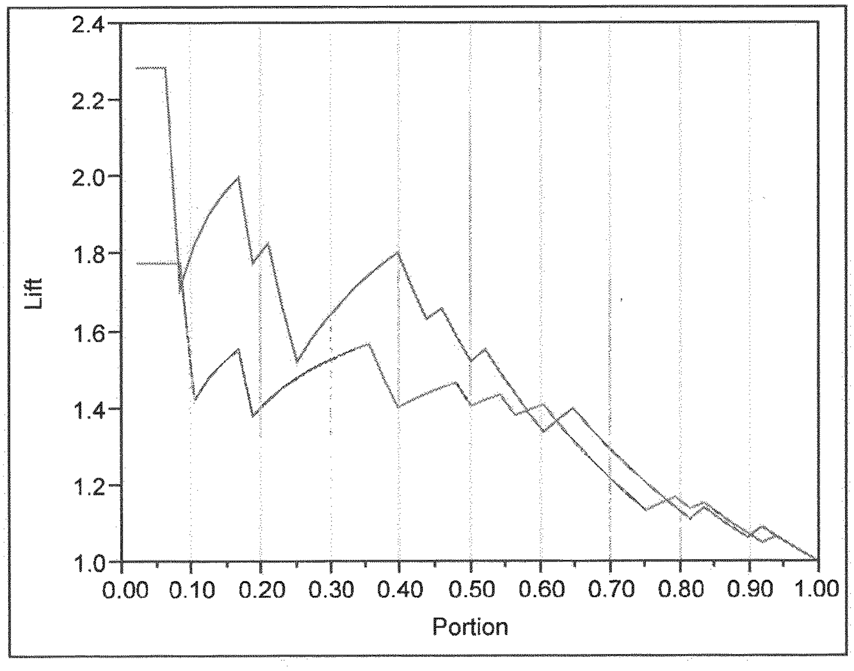

**Figure 15.**

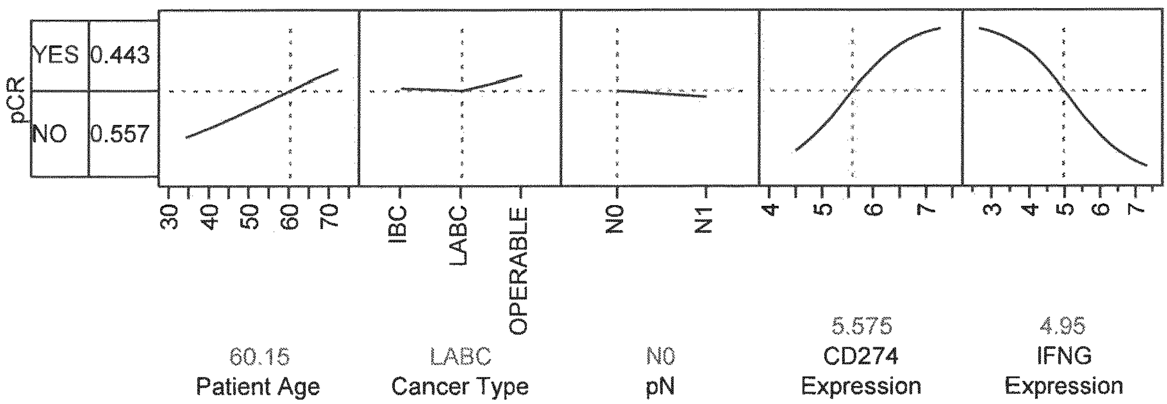


**Figure 16.**

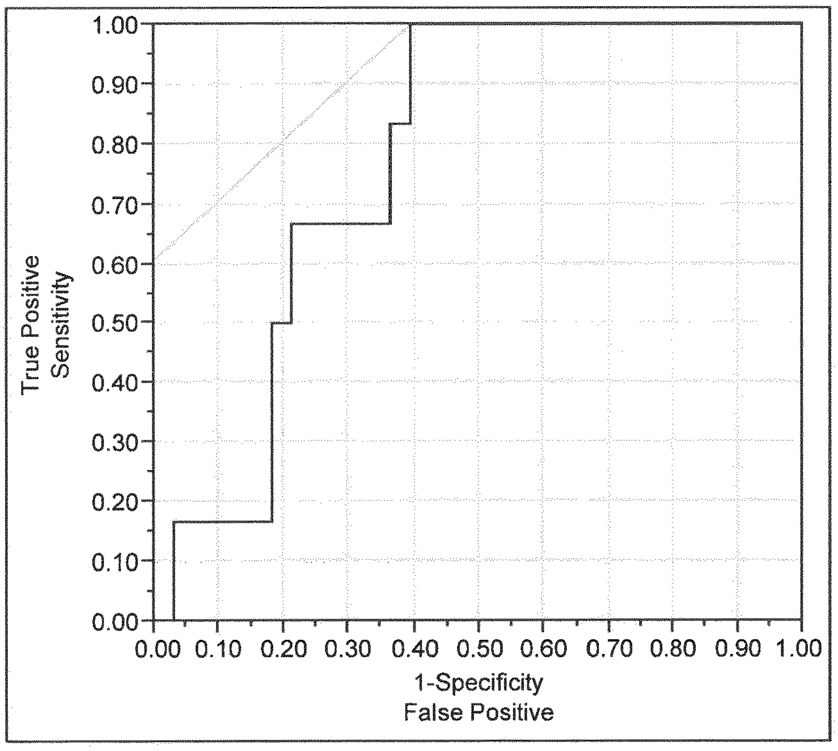

Figure 17.

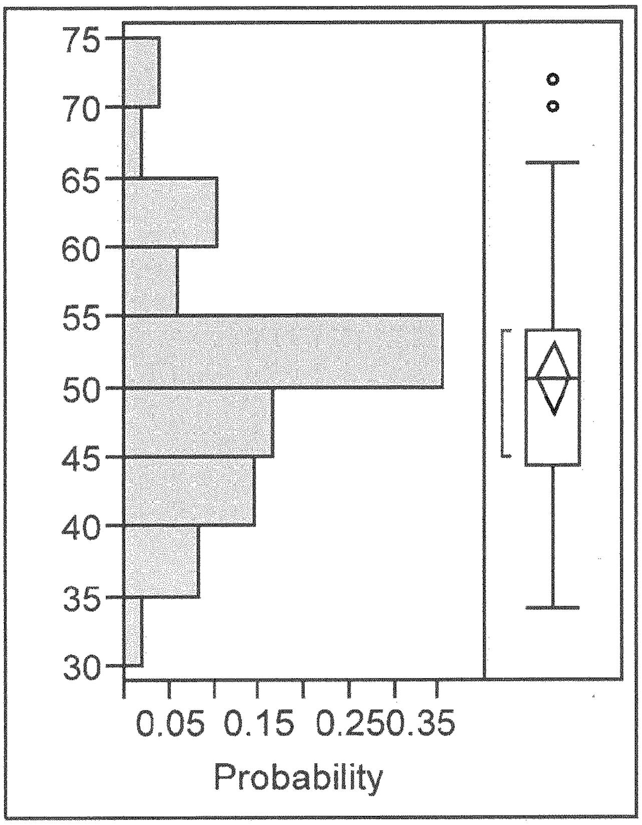


Figure 18.

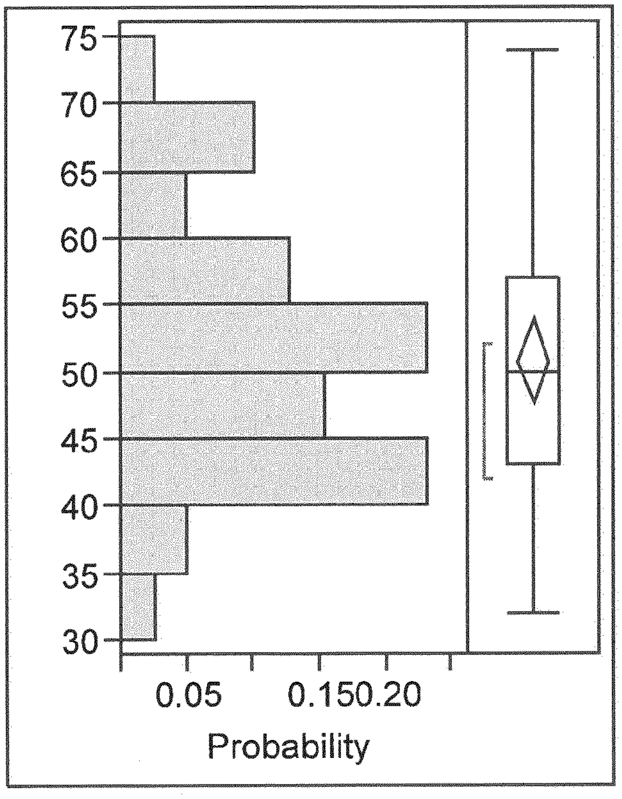

**Figure 19.**

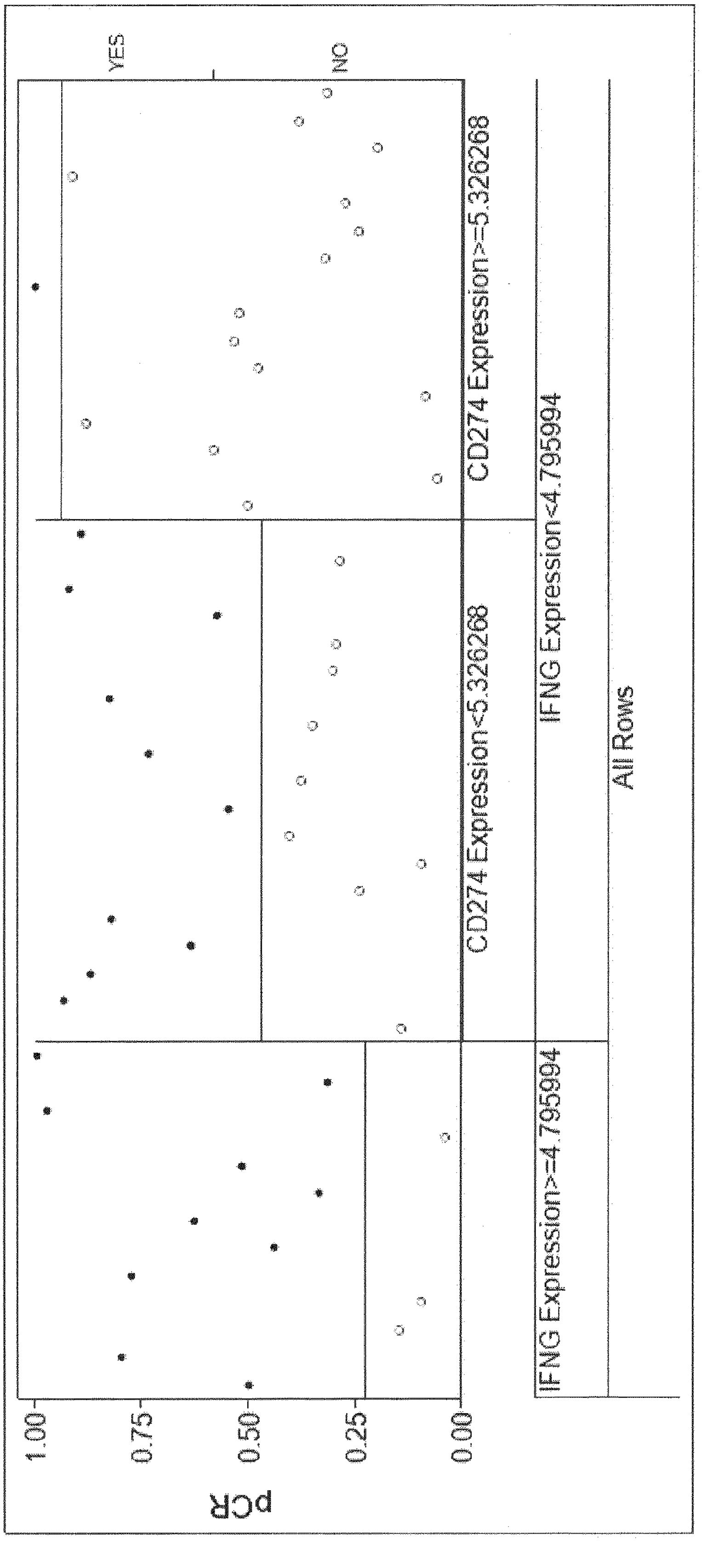
pCR
1.00
0.75
0.50
0.25
0.00
YES
NO
CD274 Expression>=5.326268
CD274 Expression<5.326268
IFNG Expression<4.795994
IFNG Expression>=4.795994
All Rows

Europäisches Patentamt
European Patent Office
Office européen des brevets

# EUROPEAN SEARCH REPORT

Application Number
EP 18 20 4426

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | US 2011/251259 A1 (DEFOUGEROLLES ANTONIN [US] ET AL) 13 October 2011 (2011-10-13)<br>* claims *<br>* paragraphs [0007], [0015], [0038], [0051] *<br>* pages 304, 324 * | 10,14-17<br><br>1-18 | INV.<br>G01N33/74<br>G01N33/68 |
| X,D<br><br><br>Y | WO 2011/109789 A2 (UNIV JOHNS HOPKINS [US]; BEDI ATUL [US]; RAVI RAJANI [US]) 9 September 2011 (2011-09-09)<br>* claims *<br>* paragraph [0104] *<br>* paragraph [0143] *<br>* paragraph [0158] - paragraph [0159] * | 10,14,15<br><br><br>1-18 | |
| X,D<br><br><br>Y | WO 2011/066342 A2 (AMPLIMMUNE INC [US]; LANGERMANN SOLOMON [US]) 3 June 2011 (2011-06-03)<br>* claims *<br>* page 61, line 22 - page 63, line 10 * | 10,14,15<br><br><br>1-18 | |
| X,D | WO 2010/077634 A1 (GENENTECH INC [US]; IRVING BRYAN [US]; CHEUNG JEANNE [US]; CHIU HENRY) 8 July 2010 (2010-07-08)<br>* claims 13-48 *<br>* page 105, line 32 - page 106, line 1 *<br>* page 107, line 9 - page 108, line 9 *<br>* page 7, line 27 - line 31 * | 1-18 | TECHNICAL FIELDS SEARCHED (IPC)<br>G01N |
| X,D<br><br><br>Y | WO 2009/089149 A1 (UNIV JOHNS HOPKINS [US]; CHEN LIEPING [US]) 16 July 2009 (2009-07-16)<br>* claims *<br>* page 3, line 16 - page 4, line 3 *<br>-/-- | 10,14,15<br><br><br>1-18 | |

2

The present search report has been drawn up for all claims

| Place of search | Date of completion of the search | Examiner |
|---|---|---|
| The Hague | 5 April 2019 | Routledge, Brian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 18 20 4426

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br>Y | WO 2006/133396 A2 (DANA FARBER CANCER INST INC [US]; BRIGHAM & WOMENS HOSPITAL [US]; UNIV) 14 December 2006 (2006-12-14)<br>* claims 1, 12, 14, 15 *<br>* example 14 *<br>* page 35, line 13 - page 37, line 5 *<br>----- | 10,14,15<br>1-18 | |

TECHNICAL FIELDS SEARCHED (IPC)

The present search report has been drawn up for all claims

| Place of search | Date of completion of the search | Examiner |
|---|---|---|
| The Hague | 5 April 2019 | Routledge, Brian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 18 20 4426

This annex lists the patent family members relating to the patent documents cited in the above-mentioned European search report. The members are as contained in the European Patent Office EDP file on
The European Patent Office is in no way liable for these particulars which are merely given for the purpose of information.

05-04-2019

| Patent document cited in search report | Publication date | Patent family member(s) | Publication date |
|---|---|---|---|
| US 2011251259 A1 | 13-10-2011 | AU 2011237630 A1 | 20-09-2012 |
| | | CA 2792561 A1 | 13-10-2011 |
| | | EP 2555778 A1 | 13-02-2013 |
| | | JP 2013523162 A | 17-06-2013 |
| | | JP 2017038600 A | 23-02-2017 |
| | | JP 2019047826 A | 28-03-2019 |
| | | US 2011251259 A1 | 13-10-2011 |
| | | US 2014148497 A1 | 29-05-2014 |
| | | US 2017067060 A1 | 09-03-2017 |
| | | US 2019048352 A1 | 14-02-2019 |
| | | WO 2011127180 A1 | 13-10-2011 |
| WO 2011109789 A2 | 09-09-2011 | CA 2791383 A1 | 09-09-2011 |
| | | DK 2542590 T3 | 28-08-2017 |
| | | EP 2542590 A2 | 09-01-2013 |
| | | EP 3260470 A1 | 27-12-2017 |
| | | ES 2638521 T3 | 23-10-2017 |
| | | JP 6066732 B2 | 25-01-2017 |
| | | JP 6378262 B2 | 22-08-2018 |
| | | JP 2013521311 A | 10-06-2013 |
| | | JP 2017043600 A | 02-03-2017 |
| | | JP 2018172439 A | 08-11-2018 |
| | | PL 2542590 T3 | 30-11-2017 |
| | | PT 2542590 T | 31-08-2017 |
| | | US 2013039911 A1 | 14-02-2013 |
| | | US 2015183881 A1 | 02-07-2015 |
| | | US 2016340430 A1 | 24-11-2016 |
| | | US 2017158770 A1 | 08-06-2017 |
| | | WO 2011109789 A2 | 09-09-2011 |
| WO 2011066342 A2 | 03-06-2011 | EP 2504028 A2 | 03-10-2012 |
| | | JP 2013512251 A | 11-04-2013 |
| | | US 2013017199 A1 | 17-01-2013 |
| | | WO 2011066342 A2 | 03-06-2011 |
| WO 2010077634 A1 | 08-07-2010 | AR 074563 A1 | 26-01-2011 |
| | | AU 2009333580 A1 | 08-07-2010 |
| | | AU 2016203867 A1 | 30-06-2016 |
| | | AU 2018203226 A1 | 31-05-2018 |
| | | BR PI0917592 A2 | 01-12-2015 |
| | | CA 2740806 A1 | 08-07-2010 |
| | | CN 102245640 A | 16-11-2011 |
| | | CN 104479018 A | 01-04-2015 |
| | | CN 108997498 A | 14-12-2018 |
| | | CO 6390023 A2 | 29-02-2012 |
| | | CR 20110316 A | 18-07-2011 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 18 20 4426

This annex lists the patent family members relating to the patent documents cited in the above-mentioned European search report. The members are as contained in the European Patent Office EDP file on
The European Patent Office is in no way liable for these particulars which are merely given for the purpose of information.

05-04-2019

| Patent document cited in search report | Publication date | Patent family member(s) | Publication date |
|---|---|---|---|
| | | CR 20160570 A | 13-01-2017 |
| | | CY 1118943 T1 | 10-01-2018 |
| | | DK 2376535 T3 | 12-06-2017 |
| | | EC SP11011115 A | 29-07-2011 |
| | | EP 2376535 A1 | 19-10-2011 |
| | | EP 3255060 A1 | 13-12-2017 |
| | | EP 3447073 A1 | 27-02-2019 |
| | | ES 2628095 T3 | 01-08-2017 |
| | | HK 1163130 A1 | 17-07-2015 |
| | | HK 1207387 A1 | 29-01-2016 |
| | | HR P20170908 T1 | 22-09-2017 |
| | | HU S1700049 I1 | 28-12-2017 |
| | | IL 213353 A | 30-04-2017 |
| | | JP 5681638 B2 | 11-03-2015 |
| | | JP 6178349 B2 | 09-08-2017 |
| | | JP 2012511329 A | 24-05-2012 |
| | | JP 2015091260 A | 14-05-2015 |
| | | JP 2017136085 A | 10-08-2017 |
| | | KR 20110092300 A | 17-08-2011 |
| | | KR 20170113681 A | 12-10-2017 |
| | | KR 20180089573 A | 08-08-2018 |
| | | LT 2376535 T | 26-06-2017 |
| | | LT PA2017041 I1 | 10-01-2018 |
| | | LU C00051 I1 | 30-11-2017 |
| | | MA 32948 B1 | 02-01-2012 |
| | | MX 342591 B | 05-10-2016 |
| | | MX 356367 B | 25-05-2018 |
| | | NO 2018006 I1 | 26-02-2018 |
| | | NZ 592119 A | 26-07-2013 |
| | | PE 03412012 A1 | 24-04-2012 |
| | | PE 17222014 A1 | 02-12-2014 |
| | | PL 2376535 T3 | 29-09-2017 |
| | | PT 2376535 T | 23-06-2017 |
| | | RU 2011128399 A | 20-01-2013 |
| | | RU 2017132160 A | 08-02-2019 |
| | | SG 172059 A1 | 28-07-2011 |
| | | SG 196798 A1 | 13-02-2014 |
| | | SG 10201708690S A | 28-12-2017 |
| | | TW 201032822 A | 16-09-2010 |
| | | TW 201417828 A | 16-05-2014 |
| | | TW 201712034 A | 01-04-2017 |
| | | UA 109108 C2 | 27-07-2015 |
| | | US 2010203056 A1 | 12-08-2010 |
| | | US 2013045200 A1 | 21-02-2013 |
| | | US 2013045201 A1 | 21-02-2013 |
| | | US 2013045202 A1 | 21-02-2013 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 18 20 4426

This annex lists the patent family members relating to the patent documents cited in the above-mentioned European search report. The members are as contained in the European Patent Office EDP file on
The European Patent Office is in no way liable for these particulars which are merely given for the purpose of information.

05-04-2019

| Patent document cited in search report | Publication date | Patent family member(s) | Publication date |
|---|---|---|---|
| | | US 2014065135 A1 | 06-03-2014 |
| | | US 2015322153 A1 | 12-11-2015 |
| | | US 2016222117 A1 | 04-08-2016 |
| | | US 2017107287 A1 | 20-04-2017 |
| | | US 2019016807 A1 | 17-01-2019 |
| | | WO 2010077634 A1 | 08-07-2010 |
| | | ZA 201102417 B | 26-06-2013 |
| WO 2009089149 A1 | 16-07-2009 | US 2010285039 A1 | 11-11-2010 |
| | | WO 2009089149 A1 | 16-07-2009 |
| WO 2006133396 A2 | 14-12-2006 | AU 2006254902 A1 | 14-12-2006 |
| | | BR PI0611766 A2 | 20-12-2011 |
| | | CA 2611861 A1 | 14-12-2006 |
| | | CA 2981431 A1 | 14-12-2006 |
| | | CN 101355965 A | 28-01-2009 |
| | | CN 103830725 A | 04-06-2014 |
| | | CN 104436190 A | 25-03-2015 |
| | | CY 1118612 T1 | 12-07-2017 |
| | | DK 1907000 T3 | 28-01-2013 |
| | | DK 2397156 T3 | 09-01-2017 |
| | | EP 1907000 A2 | 09-04-2008 |
| | | EP 2397155 A1 | 21-12-2011 |
| | | EP 2397156 A1 | 21-12-2011 |
| | | EP 3130350 A1 | 15-02-2017 |
| | | ES 2397355 T3 | 06-03-2013 |
| | | ES 2608316 T3 | 07-04-2017 |
| | | HK 1115326 A1 | 12-04-2013 |
| | | HU E030877 T2 | 28-06-2017 |
| | | IL 187999 A | 26-02-2015 |
| | | IL 230396 A | 31-01-2017 |
| | | IL 236805 A | 28-02-2018 |
| | | IL 236806 A | 29-06-2017 |
| | | JP 5753819 B2 | 22-07-2015 |
| | | JP 5788632 B2 | 07-10-2015 |
| | | JP 5882954 B2 | 09-03-2016 |
| | | JP 2008543774 A | 04-12-2008 |
| | | JP 2012229213 A | 22-11-2012 |
| | | JP 2013231054 A | 14-11-2013 |
| | | JP 2015187143 A | 29-10-2015 |
| | | JP 2017075168 A | 20-04-2017 |
| | | JP 2018109045 A | 12-07-2018 |
| | | KR 20080104254 A | 02-12-2008 |
| | | KR 20140043477 A | 09-04-2014 |
| | | KR 20140136030 A | 27-11-2014 |
| | | KR 20160029859 A | 15-03-2016 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 18 20 4426

This annex lists the patent family members relating to the patent documents cited in the above-mentioned European search report. The members are as contained in the European Patent Office EDP file on
The European Patent Office is in no way liable for these particulars which are merely given for the purpose of information.

05-04-2019

| Patent document cited in search report | Publication date | Patent family member(s) | Publication date |
|---|---|---|---|
| | | LT 2397156 T | 27-02-2017 |
| | | MX 349137 B | 13-07-2017 |
| | | MX 351401 B | 13-10-2017 |
| | | NZ 564243 A | 31-03-2011 |
| | | NZ 590308 A | 24-02-2012 |
| | | NZ 593388 A | 31-08-2012 |
| | | NZ 601439 A | 30-11-2012 |
| | | PL 2397156 T3 | 31-07-2017 |
| | | PT 2397156 T | 23-12-2016 |
| | | RU 2011133335 A | 20-02-2013 |
| | | RU 2016131170 A | 01-02-2018 |
| | | SG 10201702670V A | 29-06-2017 |
| | | SI 1907000 T1 | 29-03-2013 |
| | | US 2007122378 A1 | 31-05-2007 |
| | | US 2014178370 A1 | 26-06-2014 |
| | | US 2016362492 A1 | 15-12-2016 |
| | | WO 2006133396 A2 | 14-12-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4968603 A
- WO P9422478 A
- US 5824311 A
- US 5677171 A
- US 5821337 A
- WO 9321319 A
- WO 01000245 A
- WO 09400136 A
- US 5783186 A
- US 5183884 A
- US 5480968 A
- EP 599274 A
- US 5641869 A
- US 6339142 B
- US 20060018899 A
- US 5720937 A
- US 5720954 A
- US 5725856 A
- US 5770195 A
- US 5772997 A
- US 6165464 A
- US 6387371 B
- US 6399063 B
- US 20020192211 A1
- US 6015567 A
- US 6333169 B
- US 6054297 A
- US 6407213 B
- US 6719971 B
- US 6800738 B
- US 20040236078 A1
- US 5648237 A
- US 6267958 B
- US 6685940 B
- US 6821515 B
- WO 9817797 A
- US 6127526 A
- US 6333398 B
- US 6797814 B
- US 6417335 B
- US 6489447 B
- WO 9931140 A
- US 20030147884 A1
- US 20030170234 A1
- US 20050002928 A1
- US 6573043 B
- US 20030152987 A1
- WO 9948527 A
- US 20020141993 A1
- WO 0100245 A
- US 20030086924 A
- US 20040013667 A1
- WO 0069460 A
- WO 0100238 A
- WO 0115730 A
- US 6627196 B1
- US 6632979 B1
- WO 0100244 A
- US 20020090662 A1
- WO 0189566 A
- US 20020064785 A
- US 20030134344 A
- WO 0424866 A
- US 20040082047 A
- US 20030175845 A1
- WO 03087131 A
- US 20030228663 A
- WO 2004008099 A2
- US 20040106161 A
- WO 2004048525 A
- US 20040258685 A1
- US 5985553 A
- US 5747261 A
- US 4935341 A
- US 5401638 A
- US 5604107 A
- WO 8707646 A
- WO 8910412 A
- WO 9105264 A
- EP 412116 B1
- EP 494135 B1
- EP 444181 B1
- EP 1006194 A2
- US 20020155527 A1
- WO 9102062 A
- US 5571894 A
- US 5939531 A
- EP 502812 B1
- WO 9303741 A
- EP 554441 B1
- EP 656367 A1
- US 5288477 A
- US 5514554 A
- US 5587458 A
- WO 9312220 A
- WO 9316185 A
- US 5877305 A
- WO 9321232 A

- US 5856089 A
- WO 9422478 A
- US 5910486 A
- US 6028059 A
- WO 9607321 A
- US 5804396 A
- US 5846749 A
- EP 711565 A
- WO 9616673 A
- US 5783404 A
- US 5977322 A
- US 6512097 B
- WO 9700271 A
- US 6270765 B
- US 6395272 B
- US 5837243 A
- WO 9640789 A
- US 6458356 B
- WO 9720858 A
- WO 9738731 A
- US 6214388 B
- US 5925519 A
- WO 9802463 A
- US 5922845 A
- WO 9818489 A
- WO 9833914 A
- US 5994071 A
- WO 9845479 A
- US 6358682 B1
- US 20030059790 A
- WO 9955367 A
- WO 0120033 A
- US 20020076695 A1
- WO 0078347 A
- WO 0109187 A
- WO 0121192 A
- WO 0132155 A
- WO 0153354 A
- WO 0156604 A
- WO 0176630 A
- WO 0205791 A
- WO 0211677 A
- US 6582919 B
- US 20020192652 A1
- US 20030211530 A1
- WO 0244413 A
- US 20020142328 A
- US 6602670 B2
- WO 0245653 A
- WO 02055106 A
- US 20030152572 A
- US 20030165840 A
- WO 02087619 A
- WO 03006509 A
- WO 03012072 A
- WO 03028638 A
- US 20030068318 A
- WO 03041736 A
- EP 1357132 A
- US 20030202973 A
- US 20040138160 A
- US 5705157 A
- US 6123939 A
- EP 616812 B1
- US 20030103973 A
- US 20030108545 A
- US 6403630 B1
- WO 0061145 A
- WO 0061185 A
- US 6333348 B1
- WO 0105425 A
- WO 0164246 A
- US 20030022918 A
- US 20020051785 A1
- US 6767541 B
- WO 0176586 A
- US 20030144252 A
- WO 0187336 A
- US 20020031515 A1
- WO 0187334 A
- WO 0209754 A
- US 20030157097 A
- US 20020076408 A
- WO 02070008 A
- WO 02089842 A
- WO 0386467 A
- US 20030147884 A, Paton
- US 20030152987 A, Cohen
- WO 2004053497 A
- US 2004024815 A1, Bacus
- US 20030190689 A, Crosby and Smith
- US 2004013297 A1, Bacus
- WO 2004000094 A, Bacus
- WO 2004063709 A
- US 20040209290 A, Cobleigh
- WO 2011109789 A
- WO 2011066342 A
- WO 2009089149 A
- WO 2006133396 A
- WO 2010077634 A
- WO 2005117553 A
- US 3773919 A
- EP 58481 A
- EP 133988 A
- DE 3218121
- EP 52322 A
- EP 36676 A
- EP 88046 A
- EP 143949 A
- EP 142641 A
- JP 58118008 A
- US 4485045 A
- US 4544545 A
- EP 102324 A

**Non-patent literature cited in the description**


- **BASELGA ; MENDELSOHN.** *Pharmac. Ther.,* 1994, vol. 64, 127-154
- **MASUI et al.** *Cancer Research,* 1984, vol. 44, 1002-1007
- **WU et al.** *J. Clin. Invest.,* 1995, vol. 95, 1897-1905
- **SLAMON et al.** *Science,* 1987, vol. 235, 177-182
- **SLAMON et al.** *Science,* 1989, vol. 244, 707-712
- **KING et al.** *Science,* 1985, vol. 229, 974
- **YOKOTA et al.** *Lancet,* 1986, vol. 1, 765-767
- **FUKUSHIGE et al.** *Mol Cell Biol.,* 1986, vol. 6, 955-958
- **GUERIN et al.** *Oncogene Res.,* 1988, vol. 3, 21-31
- **COHEN et al.** *Oncogene,* 1989, vol. 4, 81-88
- **YONEMURA et al.** *Cancer Res.,* 1991, vol. 51, 1034
- **BORST et al.** *Gynecol. Oncol.,* 1990, vol. 38, 364
- **WEINER et al.** *Cancer Res.,* 1990, vol. 50, 421-425
- **KERN et al.** *Cancer Res.,* 1990, vol. 50, 5184
- **PARK et al.** *Cancer Res.,* 1989, vol. 49, 6605
- **ZHAU et al.** *Mol. Carcinog.,* 1990, vol. 3, 254-257
- **AASLAND et al.** *Br. J. Cancer,* 1988, vol. 57, 358-363
- **WILLIAMS et al.** *Pathobiology,* 1991, vol. 59, 46-52
- **MCCANN et al.** *Cancer,* 1990, vol. 65, 88-92
- **GU et al.** *Cancer Lett.,* 1996, vol. 99, 185-9
- **ROSS et al.** *Hum. Pathol.,* 1997, vol. 28, 827-33
- **ROSS et al.** *Cancer,* 1997, vol. 79, 2162-70
- **SADASIVAN et al.** *J. Urol.,* 1993, vol. 150, 126-31
- **DREBIN et al.** *Cell,* 1985, vol. 41, 695-706
- **MYERS et al.** *Meth. Enzym.,* 1991, vol. 198, 277-290
- **DREBIN et al.** *Oncogene,* 1988, vol. 2, 273-277
- **HUDZIAK et al.** *Mol. Cell. Biol.,* 1989, vol. 9 (3), 1165-1172
- **FENDLY et al.** *Cancer Research,* 1990, vol. 50, 1550-1558
- **KOTTS et al.** *In Vitro,* 1990, vol. 26 (3), 59A
- **SARUP et al.** *Growth Regulation,* 1991, vol. 1, 72-82
- **SHEPARD et al.** *J. Clin. Immunol.,* 1991, vol. 11 (3), 117-127
- **KUMAR et al.** *Mol. Cell. Biol.,* 1991, vol. 11 (2), 979-986
- **LEWIS et al.** *Cancer Immunol. Immunother.,* 1993, vol. 37, 255-263
- **PIETRAS et al.** *Oncogene,* 1994, vol. 9, 1829-1838
- **VITETTA et al.** *Cancer Research,* 1994, vol. 54, 5301-5309
- **SLIWKOWSKI et al.** *J. Biol. Chem.,* 1994, vol. 269 (20), 14661-14665
- **SCOTT et al.** *J. Biol. Chem.,* 1991, vol. 266, 14300-5
- **D'SOUZA et al.** *Proc. Natl. Acad. Sci.,* 1994, vol. 91, 7202-7206
- **LEWIS et al.** *Cancer Research,* 1996, vol. 56, 1457-1465
- **SCHAEFER et al.** *Oncogene,* 1997, vol. 15, 1385-1394
- **BASELGA et al.** *J. Clin. Oncol.,* 1996, vol. 14, 737-744
- *Food and Drug Administration,* 25 September 1998
- **FRANKLIN, M.C.** *Cancer Cell,* 2004, vol. 5, 317-328
- **TAGLIABUE et al.** *Int. J. Cancer,* 1991, vol. 47, 933-937
- **MCKENZIE et al.** *Oncogene,* 1989, vol. 4, 543-548
- **MAIER et al.** *Cancer Res.,* 1991, vol. 51, 5361-5369
- **BACUS et al.** *Molecular Carcinogenesis,* 1990, vol. 3, 350-362
- **STANCOVSKI et al.** *PNAS (USA),* 1991, vol. 88, 8691-8695
- **BACUS et al.** *Cancer Research,* 1992, vol. 52, 2580-2589
- **XU et al.** *Int. J. Cancer,* 1993, vol. 53, 401-408
- **KASPRZYK et al.** *Cancer Research,* 1992, vol. 52, 2771-2776
- **HANCOCK et al.** *Cancer Res.,* 1991, vol. 51, 4575-4580
- **SHAWVER et al.** *Cancer Res.,* 1994, vol. 54, 1367-1373
- **ARTEAGA et al.** *Cancer Res.,* 1994, vol. 54, 3758-3765
- **HARWERTH et al.** *J. Biol. Chem.,* 1992, vol. 267, 15160-15167
- **KLAPPER et al.** *Oncogene,* 1997, vol. 14, 2099-2109
- **KRAUS et al.** *PNAS (USA),* 1989, vol. 86, 9193-9197
- **PLOWMAN et al.** *Proc. Natl. Acad. Sci. USA,* 1993, vol. 90, 1746-1750
- **PLOWMAN et al.** *Nature,* 1993, vol. 366, 473-475
- **EARP et al.** *Breast Cancer Research and Treatment,* 1995, vol. 35, 115-132
- **GROENEN et al.** *Growth Factors,* 1994, vol. 11, 235-257

- **HOLMES et al.** *Science,* 1992, vol. 256, 1205-1210
- **LEMKE.** *G. Molec. & Cell. Neurosci.,* 1996, vol. 7, 247-262
- **LEE et al.** *Pharm. Rev.,* 1995, vol. 47, 51-85
- **CHANG et al.** *Nature,* 1997, vol. 387, 509-512
- **CARRAWAY et al.** *Nature,* 1997, vol. 387, 512-516
- **ZHANG et al.** *PNAS,* 1997, vol. 94 (18), 9562-7
- **HARARI et al.** *Oncogene,* 1999, vol. 18, 2681-89
- **LEVI et al.** *Journal of Neuroscience,* 1995, vol. 15, 1329-1340
- **MORRISSEY et al.** *Proc. Natl. Acad. Sci. USA,* 1995, vol. 92, 1431-1435
- **LEWIS et al.** *Cancer Res.,* 1996, vol. 56, 1457-1465
- **CARRAWAY ; CANTLEY.** *Cell,* 1994, vol. 78, 5-8
- **REID et al.** Effects of Cell Culture Process Changes on Humanized Antibody Characteristics. *Poster presented at Well Characterized Biotech Pharmaceuticals conference,* January 2003
- **HARRIS et al.** The Ideal Chromatographic Antibody Characterization Method. *IBC Antibody Production Conference,* February 2002
- **ROUSE et al.** Poster presented at WCBP. *Glycoprotein Characterization by High Resolution Mass Spectrometry and Its Application to Biopharmaceutical Development,* 06 January 2004
- **JILL PORTER.** Strategic Use of Comparability Studies and Assays for Well Characterized Biologicals. *IBC Meeting,* September 2000
- **GIANNI.** *Lancet Oncol,* 2012, vol. 13, 25-32
- **S. NOFECH-MOZES ; E. VELLA ; S. DHESY-THIND.** Guideline on Hormone Receptor Testing. *Breast Cancer*
- **W. HANNA.** A Quality Initiative of the Program in Evidence-Based Care (PEBC). *Cancer Care Ontario (CCO),* 08 April 2011
- **NOFECH-MOZES S ; VELLA ET ; DHESY-THIND S ; HAGERTY KL ; MANGU PB ; TEMIN S et al.** Systematic review on hormone receptor testing in breast cancer. *Applied Immunohistochem Mol Morphol,* May 2012, vol. 20 (3), 214-63
- **NOFECH-MOZES S ; VELLA ET ; DHESY-THIND S ; HANNA WM.** Cancer Care Ontario guideline recommendations for hormone receptor testing in breast cancer. *Clin Oncol (R Coll Radiol)*
- **SIDMAN, U. et al.** *Biopolymers,* 1983, vol. 22, 547-556
- **R. LANGER et al.** *J. Biomed. Mater. Res.,* 1981, vol. 15, 167-277
- **R. LANGER.** *Chem. Tech.,* 1982, vol. 12, 98-105
- **EPSTEIN et al.** *Proc. Natl. Acad. Sci.,* 1985, vol. 82, 3688-3692
- **HWANG et al.** *Proc. Natl. Acad. Sci.,* 1980, vol. 77, 4030-4034
- **SAMBROOK ; RUSSELL.** Molecular Cloning, A Laboratory Manual. Cold Spring Harbor Laboratory, 2001